# EUROPEAN PATENT APPLICATION

(11) **EP 4 393 919 A1**
(43) Date of publication of application: **03.07.2024**
(21) Application number: 22307048.3
(22) Date of filing: 27.12.2022
(51) Int. Cl.: C07D 413/10, C07D 413/14, C07D 417/10, C07D 471/02, C07D 491/02, A61K 31/438, A61P 35/00, A61K 31/421, A61K 31/427, A61K 31/437

(54) **LSD1 MODULATORS**

(71) Applicant: Exscientia Al Limited, Oxford OX4 4GE (GB)
(72) Inventor: The designation of the inventor has not yet been filed
(74) Representative: Keltie LLP

(57) **Abstract**

Disclosed are compounds, compositions and methods for treating disease, syndromes, conditions and disorders that are affected by the modulation of LSD1. Such compounds are represented by formula (I) wherein the variables are defined herein.

## Description

### TECHNICAL FIELD

The present disclosure relates to novel compounds capable of modulating lysine specific demethylase-1 (LSD1) monoamine oxidase activity. Such oxidative activity may be inhibited by the compounds described herein. The present invention further describes the synthesis of the compounds and their uses as medicaments in diseases or disorders where LSD1 modulation may be beneficial.

### BACKGROUND

Gene expression can be modulated at multiple levels in the cell. For example, DNA promoter methylation is associated with suppression of gene expression. Various modifications of this type have already been approved for clinical use such as Vidaza. Further modifications involve methylation of histones which form the protein scaffold that DNA is normally coiled around. Histones are critical for organising DNA and the regulated coiling and uncoiling is crucial in controlling gene expression as coiled DNA is not usually accessible for gene transcription. Histones can be modified by acetylation, lysine methylation, ubiquinylation, sumoylation many of which modify the accessibility of the histone to the associated DNA.

A group of enzymes known as histone lysine methyl transferase and histone lysine demethylase are involved in histone lysine modifications. In particular, histone demethylase LSD1 (KDM1A) belongs to the broad family of flavin adenine dinucleotide (FAD) dependent monoamine oxidases and catalyses demethylase activity. Lysine specific demethylase-1 (LSD1) catalyses demethylation at histone H3 on lysine 4 (H3K4me1/2) resulting in a transcriptional repressive state. However, LSD1 can also demethylate histone H3 on lysine 9 methyl 1/2 (H3K9me1/2) to act as a transcriptional activator in certain cellular context. Therefore, depending on which lysine is demethylated by LSD1, gene expression can either be repressed or activated (Maiques-Diaz A., Somervaille T.C. LSD1: biologic roles and therapeutic targeting. Epigenomics. 2016;8(8):1103-1116.).

The LSD1 protein structure is comprised of three domains. The SWIRM and Tower domains function as scaffolding for multiprotein complex formation. The amine oxidase domain (AO) contains the active site, wherein a catalytic lysine residue (K661) acts to deprotonate a methylated histone lysine (e.g. H3K4me2), prompting hydride transfer to FAD (Kong, X., et al. Catalytic mechanism investigation of lysine-specific demethylase 1 (LSD1): A computational study. PLoS One 2011, 6 (9), e25444).

Elevated levels of LSD1 have been found in diverse cancers and shows close relationship with many cellular effects such as epithelial-mesenchymal transition (EMT), cell proliferation and differentiation, stem cell biology, and malignant transformation (Abdel-Magid AF. Lysine-specific demethylase 1 (LSD-1) inhibitors as potential treatment for different types of cancers. ACS Medicinal Chemistry Letters. 2017; 8:1134-5). LSD1 inactivation also enhances anti-tumor immunity and inhibits checkpoint blockade (Sheng W., et al. LSD1 ablation stimulates anti-tumor immunity and enables checkpoint blockade. Cell. 2018; 174:549-63). LSD1 dysfunction is also associated with the development of ALL (acute lymphoblastic leukemia) and AML (acute myeloid leukemia) (Mould D. P., et. al. Reversible inhibitors of LSD1 as therapeutic agents in acute myeloid leukemia: clinical significance and progress to date. Medicinal Research Reviews. 2015; 35:586-618)

Further, high expression levels of LSD1 have also been found in breast cancer, neuroblastoma, colorectal cancer, and prostate cancer. Aberrant dysregulation of LSD1 causes differentiation arrest in both hematological and solid tumors, making it a potential target for cancer therapy (P. Troyer, Medical Epigenetics (Second Edition, 2021, LSD-1 functions in activation and repression of transcription).

Numerous LSD1 inhibitors have been reported to date, some of them such as TCP, ORY-1001, GSK-2879552, IMG-7289, INCB059872, CC-90011, and ORY-2001 (Vafidemstat) have been approved or currently undergo clinical assessment for cancer therapy, particularly for small lung cancer cells (SCLC) and acute myeloid leukaemia (AML). However, blood brain barrier (BBB) penetration of these medications remains poor. Therefore, the above mentioned therapies remain unavailable for the treatment of cancers affecting the brain or the central nervous system such as brain tumours (e.g. glioblastoma) and CNS leukaemia.

To date there is no effective and approved medical treatment available with good BBB penetration properties which is based on the inhibition of LSD1.

The present invention has been devised with the above observations in mind.

### SUMMARY OF THE INVENTION

Generally, provided herein are compounds and pharmaceutical compositions capable of inhibiting LSD1. Also provided are method of treatment and therapeutic / medical uses involving compounds or pharmaceutical compositions of this disclosure for inhibiting LSD1 and, particularly, for treating diseases, disorders or conditions associated with LSD1.

In a first aspect of this disclosure, there is provided a compound of formula (I), or a pharmaceutically acceptable salt, solvate, prodrug, or pharmaceutically active metabolite thereof: 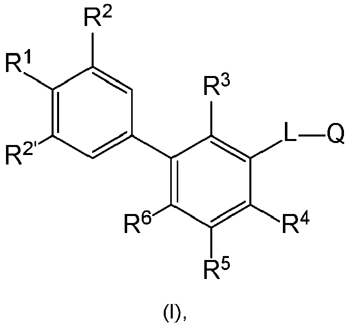 wherein
R¹ is nitro or cyano;
R² and R^{2'} are each independently selected from C₁₋₃ alkyl, hydrogen or halogen;
R³ is selected from hydrogen, C₁₋₃ alkyl, C₁₋₃ haloalkyl, C₃-C₆ cycloalkyl, C₃-C₆ cycloalkenyl, C₃-C₆ heterocycloalkyl, C₄-C₅ heterocycloalkenyl, C₃-C₅ heterocycloalkyl C₁-C₃ alkyl, C₅-C₆ aryl, C₃-C₆ heteroaryl, C₁₋C₄ alkoxyl, C₃-C₆ cycloalkyl-C₁-C₃ alkoxyl, C₁₋C₄ haloalkoxyl, C₁-C₃ haloalkyl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, cyano, wherein each alkyl, cycloalkyl, cycloalkenyl, heterocycloalkyl, heterocycloalkenyl, heterocycloalkyl alkyl, aryl, heteroaryl, alkoxyl, cycloalkyl alkoxyl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, amino;
R⁴ is selected from hydrogen or halogen;
R⁵ is selected from the group consisting of hydrogen, C₁₋₃ alkyl, C₃-C₆ cycloalkyl, C₃-C₆ cycloalkenyl, C₃-C₆ aryl, C₃-C₆ heteroaryl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, wherein each alkyl, cycloalkyl, cycloalkenyl, aryl, heteroaryl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, or amino;
R⁶ is selected from the group consisting of hydrogen, C₁₋C₄ alkoxyl, C₁₋C₄ alkoxyl, amino, alkylamino, aminoalkyl, dialkylamino, C₈₋C₁₁ spirocycloalkyl, C₅-C₁₀ heterospirocycloalkyl, C₅-C₁₁ monocyclic or bicyclic aryl, monocyclic or bicyclic heteroaryl, C₂-C₆ cyclic amine, C₂-C₅ heterocyclic amine, C₃-C₆ unsaturated or aromatic cyclic amine, C₃-C₅ unsaturated or aromatic heterocyclic amine, C₇₋C₁₀ spirocyclic amine, or C₄₋C₉ heterospirocyclic amine, wherein each is optionally substituted with 1 to 3 substituents selected from the group consisting of hydroxyl, amino, C₁-C₆ alkyl, C₁-C₆ alkoxyl, halogen, C₁-C₃ haloalkyl, optionally substituted C₃-C₆ cycloalkyl, optionally substituted C₂-C₅ heterocycloalkyl, or optionally substituted C₃-C₆ heteroaryl, wherein said optionally substituted cycloalkyl, heterocycloalkyl and heteroaryl may be substituted with 1 or 2 substituents selected from the group consisting of C₁-C₃ alkyl, C₁-C₃ alkoxyl, hydroxyl, halogen or amine;
L is a linker selected from the group consisting of a bond, -CH₂-, -CH₂-CH₂-, -CH(CH₃)-, -C(CH₃)₂-, -C≡C-, -CH₂-O-, -C(O)-, -O-, -O-CH₂-, -NH-CH₂-, -N(CH₃)-CH₂-, --NH-C(O)-, -N(CH₃)-C(O)-, -CH₂-NH-CH₂-
Q is a monocyclic or bicyclic ring system selected from the group consisting of C₅-C₉ cycloalkyl, C₄-C₈ heterocycloalkyl, C₈₋C₁₁ spirocycloalkyl, C₅-C₁₀ heterospirocycloalkyl, C₃-C₈ cyclic amine, C₃-C₈ heterocyclic amine, C₇₋C₁₀ spirocyclic amine, or C₄₋C₉ heterospirocyclic amine, wherein each cycloalkyl or heterocycloalkyl group may be saturated or unsaturated, said bicyclic ring may be a fused or bridged bicycle, and wherein Q is optionally substituted with 1 to 3 R⁷, wherein each R⁷ is independently selected from hydrogen, C₁-C₆ alkyl, C₁-C₆ haloalkyl, C₁-C₆ alkylcarbonyl, hydroxyl, oxo, halogen, C₁-C₆ alkylamine or amino.

In embodiments, each heteroaryl or heterocyclic substituent may include from 1 to 4, 1 to 3, or 1 or 2 heteroatoms independently selected from O, S and N.

In embodiments each heteroaryl or heterocyclic substituent may be connected to the remainder of the compound through a carbon atom, or through a heteroatom. In embodiments, the heteroaryl or heterocyclic substituent is connected to the remainder of the compound through a carbon atom, or through a N atom.

Suitably, one of R² and R^{2'} may be hydrogen and the other may be F; one of R² and R^{2'} may be hydrogen and the other may be CI; or both R² and R^{2'} may be hydrogen. In some particular embodiments one of R² and R^{2'} may be methyl and the other may be hydrogen, For Cl.

Suitably, R³ may be selected from hydrogen, halogen, C₃-C₆ cycloalkyl, C₃-C₆ heterocycloalkyl, C₅-C₆ aryl, C₃-C₆ heteroaryl, or C₂-C₅ heterocyclic amine. In various embodiments, R³ may be hydrogen or CI; R³ may be an optionally substituted 5 or 6 membered heteroaryl; R³ may be an optionally substituted 4 to 6 membered heterocycloalkyl; or R³ may be an optionally substituted 3 to 5 membered cycloalkyl; wherein each hetero moiety may include 1 or 2 heteroatoms independently selected from O, S and N; and/or wherein any optional substituents are independently selected from 1 or 2 substituents of the group consisting of methyl, F and Cl. In embodiments, R3 is optionally substituted oxazole.

In various embodiments R⁴ may be hydrogen; R⁴ may be F and R⁵ and R⁶ may each be hydrogen; R⁴ may be F and R⁵ and R⁶ may each be hydrogen; or R⁴, R⁵ and R⁶ may all be hydrogen.

In embodiments R⁵ may be hydrogen; R⁵ may be F; R⁵ may be a 4 or 5 membered heterocycloalkyl; R⁵ may be C₁-C₃ alkyl; R⁵ may be 3, 4 or 5 membered cycloalkyl; or R⁵ may be optionally substituted C₃-C₆ cycloalkyl, C₃-C₆ cycloalkenyl, C₃-C₆ heterocycloalkyl, C₃-C₆ aryl or C₃-C₆ heteroaryl. Particularly, R⁵ can be selected from hydrogen, F, methyl or pyrrolidine.

In embodiments R⁶ may be hydrogen; R⁶ may be For CI; R⁶ may be an optionally substituted 4 or 5 membered heterocycloalkyl; R⁶ may be an optionally substituted 8, 9 or 10 membered bicyclic or spirocyclic heterocycloalkyl; R⁶ may be an optionally substituted 5 or 6 membered heteroaryl; R⁶ may be an optionally substituted 4, 5 or 6 membered cycloalkyl or spirocycloalkyl; R⁶ may be a C₁-C₃ haloalkoxy; or R⁶ may be a C₂-C₈ alkylamine or dialkylamine, wherein each hetero moiety may include 1 or 2 heteroatoms independently selected from O, S and N; and/or wherein any optional substituents are independently selected from 1 or 2 substituents from the group consisting of C₁-C₃ alkyl, C₁-C₃ haloalkyl, C₁-C₃ alkoxyl, F, Cl and hydroxyl. Particularly, R⁶ can be selected from hydrogen, F, methyl or pyrollidine.

In various embodiments L may suitably be selected from -CH₂- or -CH₂-CH₂-.

Suitably, Q may be selected from the group consisting of optionally substituted 5 to 7 membered heterocycloalkyl and optionally substituted 4 to 7 membered mono or fused or bridged bicycloalkylamine; wherein each hetero moiety may include 1 or 2 heteroatoms independently selected from O and N; and/or wherein any optional substituents are independently selected from 1 to 3 substituents from the group consisting of C₁-C₃ alkyl, C₁-C₃ haloalkyl, C₁-C₃ alkoxyl, C₁-C₃ alkylamine, C₁-C₃ dialkylamine, C₁-C₃ carbonyl, amine, amino-C₁-C₃ alkyl, oxo, F, Cl and hydroxyl.

In embodiments, R⁷ may be independently selected from C₁-C₃ alkyl, C₁-C₃ haloalkyl, hydroxyl, oxo, F, C₁-C₃ alkylamino, C₁-C₃ dialkylamino or amino; or R⁷ may be independently selected from methyl, ethyl, amine and F; optionally wherein there are 1, 2 or 3 R⁷ groups.

In some beneficial embodiments, R³ may be selected from the group consisting of the following structures: 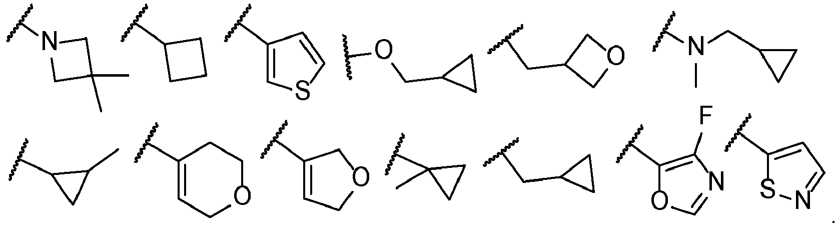 In some beneficial embodiments, R⁵ may be selected from the group consisting of hydrogen and the following structures:

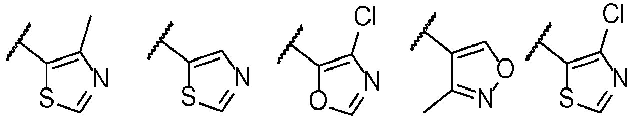

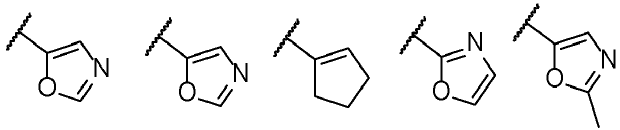

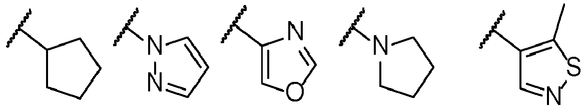

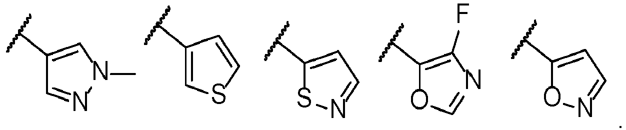

In some beneficial embodiments, R⁶ may be selected from the group consisting of hydrogen and the following structures:

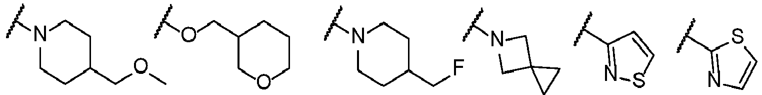

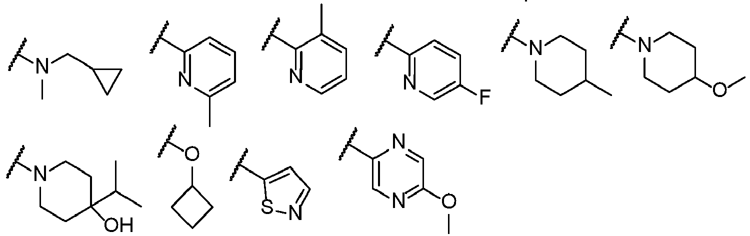

In some beneficial embodiments, Q may be selected from the group consisting of the following structures: 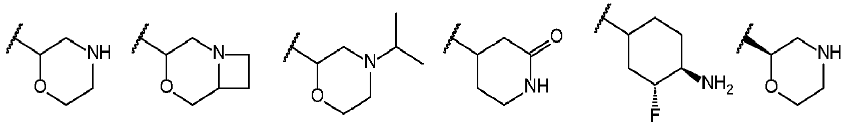 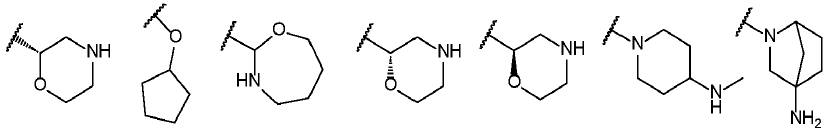 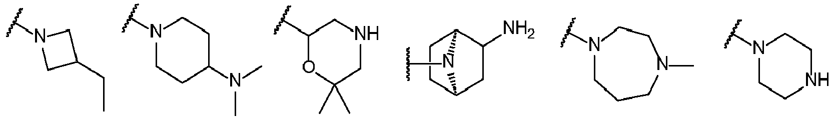 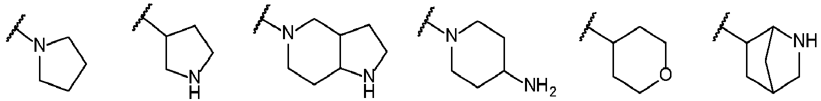 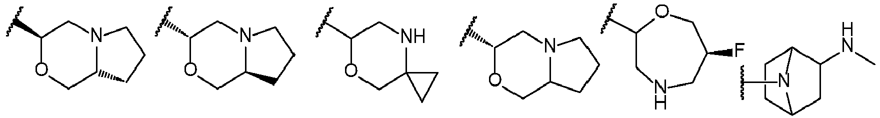 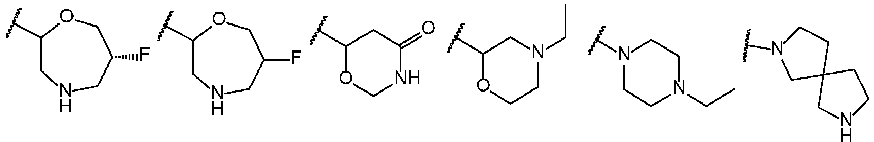 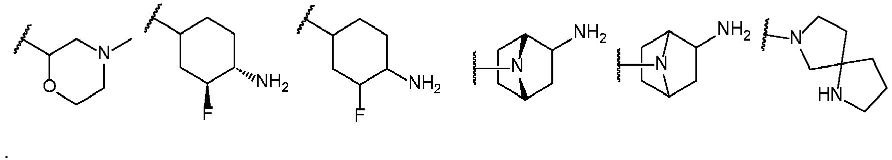 Particularly beneficially, Q may be selected from the group consisting of the following structures: 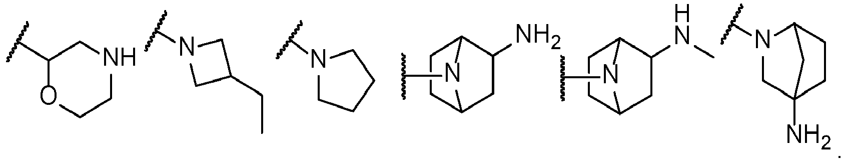 The disclosure also provides a compound selected from any one of: the group of compounds shown in Table 1; the group of compounds of Table 1 having an IC50 against LSD1 ≤ 1,000 nM; the group of compounds of Table 1 having an IC50 against LSD1 ≤ 250 nM; the group of compounds of Table 1 having an IC50 against LSD1 ≤ 100 nM; the group of compounds of Table 1 having an IC50 against LSD1 ≤ 50 nM; the group of compounds of Table 1 having an IC50 against LSD1 ≤ 25 nM; or the group of compounds of Table 1 having an IC50 against LSD1 ≤ 10 nM; or a pharmaceutically acceptable salt, solvate, prodrug, or pharmaceutically active metabolite thereof.

The disclosure provides a compound selected from any one of Examples 1 to 351.

According to a second aspect of the disclosure, there is provided a pharmaceutical composition comprising one or more compound according to the first aspect of the disclosure, or a pharmaceutically acceptable salt, solvate, prodrug, or pharmaceutically active metabolite thereof, or combinations thereof, and one or more pharmaceutically acceptable carrier.

In a third aspect, the disclosure provides a compound according to the first aspect or the pharmaceutical composition according to the second aspect for use in medicine. In particular, the compound or pharmaceutical composition may be for use is in the treatment of diseases, conditions or disorders associated with LSD1, such as cancers, oncologic diseases, autoimmune disorders and/or inflammatory diseases.

In a fourth aspect, the disclosure provides a method of treating a disease, disorder or condition in a subject in need thereof, the method comprising administering to the subject a therapeutically effective amount of a compound or pharmaceutical composition according to the disclosure.

In embodiments of the uses and methods of this disclosure, the disease, condition or disorder may be selected from the group consisting of: breast, prostate, head and neck, brain, laryngeal, oral and thyroid cancers (e.g. papillary thyroid carcinoma), hematological cancers (e.g. non-Hodgkin lymphoma, B cell lymphoma; chronic myelogenous leukemia), sarcomas, lung cancers, gastrointestinal cancers, genitourinary tract cancers, liver cancers, bone cancers, nervous system cancers, gynecological cancers and skin cancers. In embodiments, the diseases, conditions or disorders selected from the group consisting of: glioblastoma and acute myeloid leukemia (AML).

According to various embodiments, a compound or pharmaceutical composition may be administered orally, topically, by inhalation, by intranasal administration, or systemically by intravenous, intraperitoneal, subcutaneous, or intramuscular injection. In some embodiments, the compound of pharmaceutical composition of the disclosure may be administered in combination with one or more additional therapeutic agent. When administered in combination with one or more additional therapeutic agent, the administering may be simultaneously, sequentially or separately from the one or more additional therapeutic agent.

Suitably, the compound or pharmaceutical composition of the disclosure may be administered to a subject to achieve an desirable, effective amount of the compound in the blood or in the plasma of the subject. For example, the effective amount may be between about 500 nM and about 10 µM.

Within the scope of this disclosure it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. More particularly, it is specifically intended that any embodiment of any aspect may form an embodiment of any other aspect, and all such combinations are encompassed within the scope of the invention. The applicant reserves the right to change any originally filed claim or file any new claim accordingly, including the right to amend any originally filed claim to depend from and/or incorporate any feature of any other claim although not originally claimed in that manner.

### DETAILED DESCRIPTION

Described herein are compounds and compositions (e.g. organic molecules, research tools, pharmaceutical formulations and therapeutics); uses for the compounds and compositions of the disclosure (*in vitro* and *in vivo*); as well as corresponding methods, whether diagnostic, therapeutic or for research applications. The chemical synthesis and biological testing of the compounds of the disclosure are also described. Beneficially, the compounds, compositions, uses and methods have utility in research towards and/or the treatment of diseases or disorders in animals, such as humans. Diseases or disorders which may benefit from LSD1 modulation include cancer and/or oncologic disease, for example, brain tumours (e.g. glioblastoma), CNS leukaemia, cancers of hematopoietic origin or solid tumors, including chronic myelogenous leukemia, myeloid leukemia (e.g. AML - Acute myeloid leukemia), small cell lung cancer, non-Hodgkin lymphoma and other B cell lymphomas.

However, the compounds may also or alternatively be useful as lead molecules for the selection, screening and development of further derivatives that may have one or more improved beneficial drug property, as desired. Such further selection and screening may be carried out using the proprietary computational evolutionary algorithm described e.g. in the Applicant's earlier published patent application WO 2011/061548, which is hereby incorporated by reference in its entirety.

The disclosure also encompasses salts, solvates and functional derivatives of the compounds described herein. These compounds may be useful in the treatment of diseases or disorders which may benefit from LSD1 cancer and/or oncologic diseases.

Unless defined otherwise, all technical and scientific terms used herein have the same meaning as commonly understood by one of ordinary skill in the art (e.g. in organic, physical or theoretical chemistry; biochemistry and molecular biology).

Unless otherwise indicated, the practice of the present invention employs conventional techniques in chemistry and chemical methods, biochemistry, molecular biology, pharmaceutical formulation, and delivery and treatment regimens for patients, which are within the capabilities of a person of ordinary skill in the art. Such techniques are also described in the literature cited herein. All documents cited in this disclosure are herein incorporated by reference in their entirety.

Prior to setting forth the detailed description of the invention, a number of definitions are provided that will assist in the understanding of the disclosure.

In accordance with this disclosure, the terms 'molecule' or 'molecules' are used interchangeably with the terms 'compound' or 'compounds', and sometimes the term 'chemical structure'. The term 'drug' is typically used in the context of a pharmaceutical, pharmaceutical composition, medicament or the like, which has a known or predicted physiological or *in vitro* activity of medical significance; but such characteristics and qualities are not excluded in a molecule or compound of the disclosure. The term 'drug' is therefore used interchangeably with the alternative terms and phrases 'therapeutic (agent)', 'pharmaceutical (agent)', and 'active (agent)'. Therapeutics according to the disclosure also encompass compositions and pharmaceutical formulations comprising the compounds of the disclosure.

The term "compound," as used herein is meant to include all stereoisomers, geometric isomers, tautomers, and isotopically enriched variants of the structures depicted. Compounds herein identified by name or structure as one particular tautomeric form are intended to include other tautomeric forms unless otherwise specified. The term "tautomer," as used herein refers to compounds whose structures differ markedly in arrangement of atoms, but which exist in easy and rapid equilibrium, and it is to be understood that compounds provided herein may be depicted as different tautomers, and when compounds have tautomeric forms, all tautomeric forms are intended to be within the scope of the disclosure, and the naming of the compounds does not exclude any tautomer.

It will be appreciated that certain compounds provided herein may contain one or more centers of asymmetry and may therefore be prepared and isolated in a mixture of isomers such as a racemic mixture, or in an enantiomerically pure form.

Compounds provided herein may also contain unnatural proportions of atomic isotopes at one or more of the atoms that constitute such compounds. That is, an atom, in particular when mentioned in relation to a compound according to formula (I) comprises all isotopes and isotopic mixtures of that atom, either naturally occurring or synthetically produced, either with natural abundance or in an isotopically enriched form. For example, when hydrogen is mentioned, it is understood to refer to ¹H, ²H, ³H or mixtures thereof; when carbon is mentioned, it is understood to refer to ¹¹C, ¹²C, ¹³C, ¹⁴C or mixtures thereof; when nitrogen is mentioned, it is understood to refer to ¹³N, ¹⁴N, ¹⁵N or mixtures thereof; when oxygen is mentioned, it is understood to refer to ¹⁴O, ¹⁵O, ¹⁶O, ¹⁷O, ¹⁸O or mixtures thereof; and when fluoro is mentioned, it is understood to refer to ¹⁸F, ¹⁹F or mixtures thereof; unless expressly noted otherwise. For example, in deuteroalkyl and deuteroalkoxy groups, where one or more hydrogen atoms are specifically replaced with deuterium (²H). As some of the aforementioned isotopes are radioactive, the compounds provided herein therefore also comprise compounds with one or more isotopes of one or more atoms, and mixtures thereof, including radioactive compounds, wherein one or more non-radioactive atoms has been replaced by one of its radioactive enriched isotopes. Radiolabeled compounds are useful as therapeutic agents, e.g., cancer therapeutic agents, research reagents, e.g., assay reagents, and diagnostic agents, e.g., in vivo imaging agents. All isotopic variations of the compounds provided herein, whether radioactive or not, are intended to be encompassed within the scope of the present disclosure.

Prodrugs and solvates of the compounds of the disclosure are also encompassed within the scope of the disclosure. The term 'prodrug' means a compound (e.g. a drug precursor) that is transformed *in vivo* to yield a compound of the disclosure or a pharmaceutically acceptable salt, solvate or ester of the compound. The transformation may occur by various mechanisms (e.g. by metabolic or chemical processes), such as by hydrolysis of a hydrolysable bond, e.g. in blood (see Higuchi & Stella (1987), "Pro-drugs as Novel Delivery Systems", vol. 14 of the A.C.S. Symposium Series; (1987), *"*Bioreversible Carriers in Drug Design", Roche, ed., American Pharmaceutical Association and Pergamon Press*).* The compositions and medicaments of the disclosure therefore may comprise prodrugs of the compounds of the disclosure. In some aspects and embodiments, the compounds of the disclosure are themselves prodrugs which may be metabolised *in vivo* to give the therapeutically effective compound. For example, a sulfoxide prodrug may be metabolized *in vivo* to the therapeutically active sulfone (see Basarab G.S. et al., (2008), Bioorg Med Chem Lett, 18(16),4716-4722; Gibhard L. et al., (2008), Antimicrobial Agents and Chemotherapy, 62(12),00232-18).

In the context of the present disclosure, the terms 'individual', 'subject', or 'patient' are used interchangeably to indicate an animal that may be suffering from a medical (pathological) condition and may be responsive to a molecule, pharmaceutical drug, medical treatment or therapeutic treatment regimen of the disclosure. The animal is suitably a mammal, such as a human, cow, sheep, pig, dog, cat, bat, mouse or rat. In particular, the subject may be a human.

As used herein, terms "treat" or "treatment" refer to therapeutic or palliative measures. Beneficial or desired clinical results include, but are not limited to, alleviation, in whole or in part, of symptoms associated with a disease or disorder or condition, diminishment of the extent of disease, stabilized (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment

The term "preventing" as used herein means the prevention of the onset, recurrence or spread, in whole or in part, of the disease or condition as described herein, or a symptom thereof.

The term "halo" refers to one of the halogens, group 17 of the periodic table. In particular, the term refers to fluorine, chlorine, bromine and iodine. Preferably, the term refers to fluorine or chlorine.

The term 'alkyl' refers to a monovalent, optionally substituted, saturated aliphatic hydrocarbon radical. Any number of carbon atoms may be present, but typically the number of carbon atoms in the alkyl group may be from 1 to about 20, from 1 to about 12, from 1 to about 6 or from 1 to about 4. Usefully, the number of carbon atoms is indicated, for example, a C1-12 alkyl (or C₁₋₁₂ alkyl) refers to any alkyl group containing 1 to 12 carbon atoms in the chain. An alkyl group may be a straight chain (i.e. linear), branched chain, or cyclic. 'Lower alkyl' refers to an alkyl of 1 to 6 carbon atoms in the chain, and may have from 1 to 4 carbon atoms, or 1 to 2 carbon atoms. Thus, representative examples of lower alkyl radicals include methyl, ethyl, n-propyl, n-butyl, n-pentyl, n-hexyl, isopropyl, isobutyl, isopentyl, amyl (C₅H₁₁), sec-butyl, tert-butyl, sec-amyl, tert-pentyl, 2-ethylbutyl, 2,3-dimethylbutyl, and the like. `Higher alkyl' refers to alkyls of 7 carbons and above, including n-heptyl, n-octyl, n-nonyl, n-decyl, n-dodecyl, n-tetradecyl, n-hexadecyl, n-octadecyl, n-eicosyl, and the like, along with branched variations thereof. A linear carbon chain of say 4 to 6 carbons would refer to the chain length not including any carbons residing on a branch, whereas in a branched chain it would refer to the total number. Optional substituents for alkyl and other groups are described below.

The term 'substituted' means that one or more hydrogen atoms (attached to a carbon or heteroatom) is replaced with a selection from the indicated group of substituents, provided that the designated atom's normal valency under the existing circumstances is not exceeded. The group may be optionally substituted with particular substituents at positions that do not significantly interfere with the preparation of compounds falling within the scope of this invention and on the understanding that the substitution(s) does not significantly adversely affect the biological activity or structural stability of the compound. Combinations of substituents are permissible only if such combinations result in stable compounds. By `stable compound' or `stable structure', it is meant a compound that is sufficiently robust to survive isolation to a useful degree of purity from a reaction mixture and/or formulation into an efficacious therapeutic agent. By 'optionally substituted' it is meant that the group concerned is either unsubstituted, or at least one hydrogen atom is replaced with one of the specified substituent groups, radicals or moieties.

Any radical / group / moiety described herein that may be substituted (or optionally substituted) may be substituted with one or more (e.g. one, two, three, four or five) substituents, which are independently selected from the designated group of substituents. Thus, substituents may be selected from the group: halogen (or 'halo', e.g. F, Cl and Br), hydroxyl (-OH), amino or aminyl (-NH₂), thiol (-SH), cyano (-CN), (lower) alkyl, (lower) alkoxy, (lower) alkenyl, (lower) alkynyl, aryl, heteroaryl, (lower) alkylthio, oxo, haloalkyl, hydroxyalkyl, nitro (-NO₂), phosphate, azido (-N₃), alkoxycarbonyl, carboxy, alkylcarboxy, alkylamino, dialkylamino, aminoalkyl, alkylaminoalkyl, dialkylaminoalkyl, thioalkyl, alkylsulfonyl, arylsulfinyl, alkylaminosulfonyl, arylaminosulfonyl, alkylsulfonylamino, arylsulfonylamino, carbamoyl, alkylcarbamoyl, dialkylcarbamoyl, arylcarbamoyl, alkylcarbonylamino, arylcarbonylamino, cycloalkyl, heterocycloalkyl, unless otherwise indicated. Alternatively, where the substituents are on an aryl or other cyclic ring system, two adjacent atoms may be substituted with a methylenedioxy or ethylenedioxy group. More suitably, the substituents are selected from: halogen, hydroxy, amino, thiol, cyano, (C₁-C₆)alkyl, (C₁-C₆)alkoxy, (C₁-C₆)alkenyl, (C₁-C₆)alkynyl, aryl, aryl(C₁-C₆)alkyl, aryl(C₁-C₆)alkoxy, heteroaryl, (C₁-C₆)alkylthio, oxo, halo(C₁-C₆)alkyl, hydroxy(C₁-C₆)alkyl, nitro, phosphate, azido, (C₁-C₆)alkoxycarbonyl, carboxy, (C₁-C₆)alkylcarboxy, (C₁-C₆)alkylamino, di(C₁-C₆)alkylamino, amino(C₁-C₆)alkyl, (C₁-C₆)alkylamino(C₁-C₆)alkyl, di(C₁-C₆)alkylamino(C₁-C₆)alkyl, thio(C₁-C₆)alkyl, (C₁-C₆)alkylsulfonyl, arylsulfinyl, (C₁-C₆)alkylaminosulfonyl, arylaminosulfonyl, (C₁-C₆)alkylsulfonylamino, arylsulfonylamino, carbamoyl, (C₁-C₆)alkylcarbamoyl, di(C₁-C₆)alkylcarbamoyl, arylcarbamoyl, (C₁-C₆)alkylcarbonylamino, arylcarbonylamino, (C₁-C₆)cycloalkyl, and heterocycloalkyl. Still more suitably, the substituents are selected from one or more of: fluoro, chloro, bromo, hydroxy, (C₁-C₆)alkyl, (C₁-C₆)haloalkyl, (C₁-C₆)alkoxy, (C₅-C₆)aryl, a 5- or 6-membered heteroaryl, (C₄-C₆)cycloalkyl, a 4- to 6-membered heterocycloalkyl, cyano, (C₁-C₆)alkylthio, amino, -NH(alkyl), -NH((C₁-C₆)cycloalkyl), -N((C₁-C₆)alkyl)₂, -OC(O)-(C₁-C₆)alkyl, -OC(O)-(C₅-C₆)aryl, -OC(O)-(C₁-C₆)cycloalkyl, carboxy and -C(O)O-(C₁-C₆)alkyl. Most suitably, the substituents are selected from one or more of: fluoro, chloro, bromo, hydroxy, amino, (C₁-C₆)alkyl and (C₁-C₆)alkoxy, wherein alkyl and alkoxy are optionally substituted by one or more chloro. Particularly preferred substituents are: chloro, methyl, ethyl, methoxy and ethoxy.

The term 'halo' refers to a monovalent halogen radical chosen from chloro, bromo, iodo, and fluoro. A 'halogenated' compound is one substituted with one or more halo substituent. Preferred halo groups are F, CI and Br, and most preferred is CI.

As used herein, the term 'cyano' refers to a -CN group. As used herein, the term 'hydroxyl' or 'hydroxo' refers to an -OH group. As used herein, the term 'amino' or 'aminyl' refers to an - NH₂ group. As used herein, the term 'oxo' refers to an '=O' group attached to a carbon atom.

The term 'C₁-C₆ haloalkyl' refers to a hydrocarbon chain substituted with at least one halogen atom independently chosen at each occurrence, for example fluorine, chlorine, bromine and iodine. The halogen atom may be present at any position on the hydrocarbon chain. Similarly, a C1-C3 haloalkyl group is linear or branched hydrocarbon chain containing 1, 2, or 3 carbon atoms substituted with at least one halogen atom. For example, C₁-C₃ haloalkyl may refer to chloromethyl, fluoromethyl, trifluoromethyl, chloroethyl e.g. 1-chloroethyl and 2-chloroethyl, trichloroethyl e.g.1,2,2-trichloroethyl, 2,2,2-trichloroethyl, fluoroethyl e.g.1-fluoromethyl and 2-fluoroethyl, trifluoroethyl e.g. 1,2,2-trifluoroethyl and 2,2,2-trifluoroethyl, chloropropyl, trichloropropyl, fluoropropyl, trifluoropropyl.

As used herein, the term 'geminal' refers to substituent atoms or groups attached to the same atom in a molecule. As used herein, the term 'vicinal' refers to substituent atoms or groups attached to adjacent atoms in a molecule. The stereochemical relationship between the substituent atoms or groups can be cis, trans, undefined, or unresolved.

When used herein, the term 'independently', in reference to the substitution of a parent moiety with one or more substituents, means that the parent moiety may be substituted with any of the listed substituents, either individually or in combination, and any number of chemically possible substituents may be used. In any of the embodiments, where a group is substituted, it may contain up to 5, up to 4, up to 3, or 1 and 2 substituents. As a non-limiting example, useful substituents include: phenyl or pyridine, independently substituted with one or more lower alkyl, lower alkoxy or halo substituents, such as: chlorophenyl, dichlorophenyl, trichlorophenyl, tolyl, xylyl, 2-chloro-3-methylphenyl, 2,3-dichloro- 4-methylphenyl, etc.

'Alkylene' or 'alkylenyl' means a difunctional group obtained by removal of a hydrogen atom from an alkyl group as defined above. Non-limiting examples of alkylene include methylene, ethylene and propylene. 'Lower alkylene' means an alkylene having from 1 to 6 carbon atoms in the chain, and may be straight or branched. Alkylene groups are optionally substituted.

The term 'alkenyl' refers to a monovalent, optionally substituted, unsaturated aliphatic hydrocarbon radical. Therefore, an alkenyl has at least one carbon-carbon double bond (C=C). The number of carbon atoms in the alkenyl group may be indicated, such as from 2 to about 20. For example, a C2-12 alkenyl (or C₂₋₁₂ alkenyl) refers to an alkenyl group containing 2 to 12 carbon atoms in the structure. Alkenyl groups may be straight (i.e. linear), branched chain, or cyclic. 'Lower alkenyl' refers to an alkenyl of 1 to 6 carbon atoms, and may have from 1 to 4 carbon atoms, or 1 to 2 carbon atoms. Representative examples of lower alkenyl radicals include ethenyl, 1-propenyl, 1-butenyl, 1-pentenyl, 1-hexenyl, isopropenyl, isobutenyl, and the like. Higher alkenyl refers to alkenyls of seven carbons and above, such as 1-heptenyl, 1-octenyl, 1-nonenyl, 1-decenyl, 1-dodecenyl, 1-tetradecenyl, 1-hexadecenyl, 1-octadecenyl, 1-eicosenyl, and the like, along with branched variations thereof. Optional substituents include are described elsewhere.

'Alkenylene' means a difunctional group obtained by removal of a hydrogen from an alkenyl group that is defined above. Non-limiting examples of alkenylene include -CH=CH-, -C(CH₃)=CH-, and -CH=CHCH₂-.

'Alkynyl' and 'lower alkynyl' is defined similarly to the term 'alkenyl', except that it includes at least one carbon-carbon triple bond.

The term 'alkoxy' refers to a monovalent radical of the formula RO-, where R is any alkyl, alkenyl or alkynyl as defined herein. Alkoxy groups may be optionally substituted by any of the optional substituents described herein. 'Lower alkoxy' has the formula RO-, where the R group is a lower alkyl, alkenyl or alkynyl. Representative alkoxy radicals include methoxy, ethoxy, n-propoxy, n-butoxy, n-pentyloxy, n-hexyloxy, isopropoxy, isobutoxy, isopentyloxy, amyloxy, sec-butoxy, tert-butoxy, tert-pentyloxy, and the like. Preferred alkoxy groups are methoxy and ethoxy.

The term 'aryl' as used herein refers to a substituted or unsubstituted aromatic carbocyclic radical containing from 5 to about 15 carbon atoms; and preferably 5 or 6 carbon atoms. An aryl group may have only one individual carbon ring, or may comprise one or more fused rings in which at least one ring is aromatic in nature. A 'phenyl' is a radical formed by removal of a hydrogen atom from a benzene ring, and may be substituted or unsubstituted. A 'phenoxy' group, therefore, is a radical of the formula RO-, wherein R is a phenyl radical. 'Benzyl' is a radical of the formula R-CH₂-, wherein R is phenyl, and 'benzyloxy' is a radical of the formula RO-, wherein R is benzyl. Non-limiting examples of aryl radicals include, phenyl, naphthyl, benzyl, biphenyl, furanyl, pyridinyl, indanyl, anthraquinolyl, tetrahydronaphthyl, a benzoic acid radical, a furan-2-carboxylic acid radical, and the like.

A 'heteroaryl' group is herein defined as a substituted or unsubstituted 'aryl' group in which one or more carbon atoms in the ring structure has been replaced with a heteroatom, such as nitrogen, oxygen or sulphur. Generally, the heteroaryl group contains one or two heteroatoms. A preferred heteroatom is N. Exemplary heteroaryl groups include: furan, benzofuran, isobenzofuran, pyrrole, indole, isoindole, thiophene, benzothiophene, benzo[c]thiophene, imidazole, benzimidazole, purine, pyrazole, indazole, oxazole, benzoxazole, isoxazole, benzisoxazole, thiazole, benzothiazole, pyridine, quinoline, isoquinoline, pyrazine, quinoxaline, acridine, pyrimidine, quinazoline, pyridazine and cinnoline.

The terms 'heterocycle' or 'heterocyclic' group as used herein refer to a monovalent radical of from about 4- to about 15- ring atoms, and preferably 4-, 5- or 6,7- ring members. Generally, the heterocyclic group contains one, two or three heteroatoms, selected independently from nitrogen, oxygen and sulphur. A preferred heteroatom is N. A heterocyclic group may have only one individual ring, or may comprise one or more fused rings in which at least one ring contains a heteroatom. It may be fully saturated or partially saturated, and may be substituted or unsubstituted as in the case or aryl and heteroaryl groups. Representative examples of unsaturated 5-membered heterocycles with only one heteroatom include 2- or 3-pyrrolyl, 2- or 3-furanyl, and 2- or 3-thiophenyl. Corresponding partially saturated or fully saturated radicals include 3-pyrrolin-2-yl, 2- or 3-pyrrolindinyl, 2- or 3-tetrahydrofuranyl, and 2- or 3-tetrahydrothiophenyl. Representative unsaturated 5-membered heterocyclic radicals having two heteroatoms include imidazolyl, oxazolyl, thiazolyl, pyrazolyl, and the like. The corresponding fully saturated and partially saturated radicals are also included. Representative examples of unsaturated 6-membered heterocycles with only one heteroatom include 2-, 3-, or 4-pyridinyl, 2H-pyranyl, and 4H-pryanyl. Corresponding partially saturated or fully saturated radicals include 2-, 3-, or 4-piperidinyl, 2-, 3-, or 4-tetrahydropyranyl and the like. Representative unsaturated 6-membered heterocyclic radicals having two heteroatoms include 3- or 4-pyridazinyl, 2-, 4-, or 5-pyrimidinyl, 2-pyrazinyl, morpholino, and the like. The corresponding fully saturated and partially saturated radicals are also included, e.g. 2-piperazine. The heterocyclic radical is bonded through an available carbon atom or heteroatom in the heterocyclic ring directly to the entity or through a linker such as an alkylene such as methylene or ethylene.

The term 'pharmaceutically acceptable' indicates that the compound, or salt or composition thereof is compatible chemically and/or toxicologically with the other ingredients comprising a formulation and/or the subject being treated therewith.

Unless defined otherwise, 'room temperature' is intended to mean a temperature of from about 18 to 28°C, typically between about 18 and 25°C, and more typically between about 18 and 22°C. As used herein, the phrase 'room temperature' may be shortened to 'rt' or 'RT'.

### Molecules and Compounds

In some embodiments, the compounds of the disclosure may be biaryl compounds.

Disclosed herein is a compound having the structural formula (I), or a pharmaceutically acceptable salt, solvate, prodrug, or pharmaceutically active metabolite thereof: 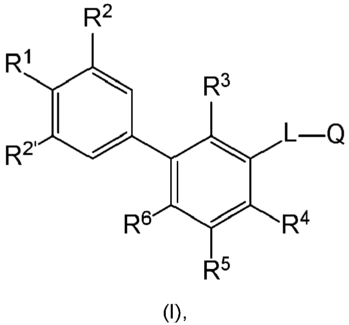 wherein
R¹ is nitro or cyano;
R² and R^{2'} are each independently selected from C₁₋₃ alkyl, hydrogen or halogen;
R³ is selected from hydrogen, C₁₋₃ alkyl, C₁₋₃ haloalkyl, C₃-C₆ cycloalkyl, C₃-C₆ cycloalkenyl, C₃-C₆ heterocycloalkyl, C₄-C₅ heterocycloalkenyl, C₃-C₅ heterocycloalkyl C₁-C₃ alkyl, C₅-C₆ aryl, C₃-C₆ heteroaryl, C₁₋C₄ alkoxyl, C₃-C₆ cycloalkyl-C₁-C₃ alkoxyl, C₁₋C₄ haloalkoxyl, C₁-C₃ haloalkyl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, cyano, wherein each alkyl, cycloalkyl, cycloalkenyl, heterocycloalkyl, heterocycloalkenyl, heterocycloalkyl alkyl, aryl, heteroaryl, alkoxyl, cycloalkyl alkoxyl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, amino;
R⁴ is selected from hydrogen or halogen;
R⁵ is selected from the group consisting of hydrogen, C₁₋₃ alkyl, C₃-C₆ cycloalkyl, C₃-C₆ cycloalkenyl, C₃-C₆ aryl, C₃-C₆ heteroaryl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, wherein each alkyl, cycloalkyl, cycloalkenyl, aryl, heteroaryl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, or amino;
R⁶ is selected from the group consisting of hydrogen, C₁₋C₄ alkoxyl, C₁₋C₄ alkoxyl, amino, alkylamino, aminoalkyl, dialkylamino, C₈₋C₁₁ spirocycloalkyl, C₅-C₁₀ heterospirocycloalkyl, C₅-C₁₁ monocyclic or bicyclic aryl, monocyclic or bicyclic heteroaryl, C₂-C₆ cyclic amine, C₂-C₅ heterocyclic amine, C₃-C₆ unsaturated or aromatic cyclic amine, C₃-C₅ unsaturated or aromatic heterocyclic amine, C₇₋C₁₀ spirocyclic amine, or C₄₋C₉ heterospirocyclic amine, wherein each is optionally substituted with 1 to 3 substituents selected from the group consisting of hydroxyl, amino, C₁-C₆ alkyl, C₁-C₆ alkoxyl, halogen, C₁-C₃ haloalkyl, optionally substituted C₃-C₆ cycloalkyl, optionally substituted C₂-C₅ heterocycloalkyl, or optionally substituted C₃-C₆ heteroaryl, wherein said optionally substituted cycloalkyl, heterocycloalkyl and heteroaryl may be substituted with 1 or 2 substituents selected from the group consisting of C₁-C₃ alkyl, C₁-C₃ alkoxyl, hydroxyl, halogen or amine;
L is a linker selected from the group consisting of a bond, -CH₂-, -CH₂-CH₂-, -CH(CH₃)-, -C(CH₃)₂-, -C=C-, -CH₂-O-, -C(O)-, -O-, -O-CH₂-, -NH-CH₂-, -N(CH₃)-CH₂-, --NH-C(O)-, -N(CH₃)-C(O)-, -CH₂-NH-CH₂-
Q is a monocyclic or bicyclic ring system selected from the group consisting of C₅-C₉ cycloalkyl, C₄-C₈ heterocycloalkyl, C₈₋C₁₁ spirocycloalkyl, C₅-C₁₀ heterospirocycloalkyl, C₃-C₈ cyclic amine, C₃-C₈ heterocyclic amine, C₇₋C₁₀ spirocyclic amine, or C₄₋C₉ heterospirocyclic amine, wherein each cycloalkyl or heterocycloalkyl group may be saturated or unsaturated, said bicyclic ring may be a fused or bridged bicycle, and wherein Q is optionally substituted with 1 to 3 R⁷, wherein each R⁷ is independently selected from hydrogen, C₁-C₆ alkyl, C₁-C₆ haloalkyl, C₁-C₆ alkylcarbonyl, hydroxyl, oxo, halogen, C₁-C₆ alkylamine or amino.

In embodiments, each hetero substituent may include from 1 to 4, 1 to 3, 2 or 1 heteroatoms independently selected from O, S and N.

In embodiments each heteroaryl or heterocyclic substituent may be connected to the remainder of the compound through a carbon atom, or through a heteroatom. In embodiments, the heteroaryl or heterocyclic substituent is connected to the remainder of the compound through a carbon atom, or through a N atom.

Suitably, one of R² and R^{2'} may be hydrogen and the other is F; alternatively, one of R² and R^{2'} may be hydrogen and the other may be Cl; alternatively, both R² and R^{2'} may be hydrogen. In some embodiments, one of R² and R^{2'} may be methyl and the other may be hydrogen, For Cl.

Suitably, one of R² and R^{2'} may be hydrogen and the other may be F; one of R² and R^{2'} may be hydrogen and the other may be F and R¹ is cyano (CN). In embodiments, one of R² and R^{2'} is methyl and the other is hydrogen.

In embodiments of the disclosure, R³ may be selected from C₃-C₆ cycloalkyl, C₃-C₆ heterocycloalkyl, C₅-C₆ aryl, C₃-C₆ heteroaryl, or C₂-C₅ heterocyclic amine.

Alternatively R³ may be an optionally substituted 5 or 6 membered heteroaryl; alternatively R³ may be an optionally substituted 4 to 6 membered heterocycloalkyl; or R³ may be an optionally substituted 3 to 5 membered cycloalkyl. In any such embodiments, each hetero moiety may include 1 or 2 heteroatoms independently selected from O, S and N. Suitably, optional substituents are independently selected from 1 or 2 substituents of the group consisting of methyl, F and CI.

Suitably, R³ may be 5 membered heteroaryl comprising N and S.

Suitably, R³ may be 5 membered heteroaryl comprising N and O and which is substituted with F.

In various aspects and embodiments, R³ may suitably be selected from the group consisting of the following structures:

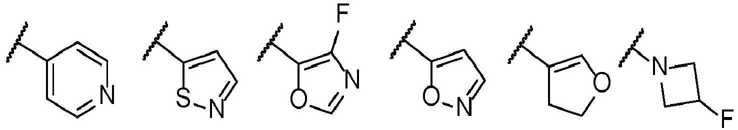

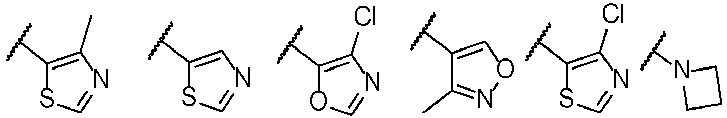

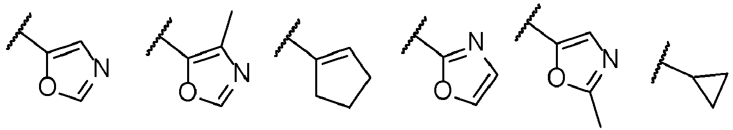

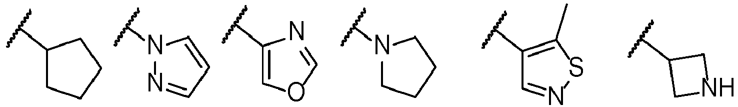

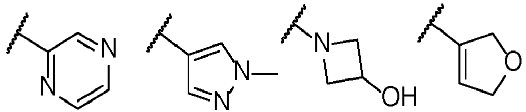

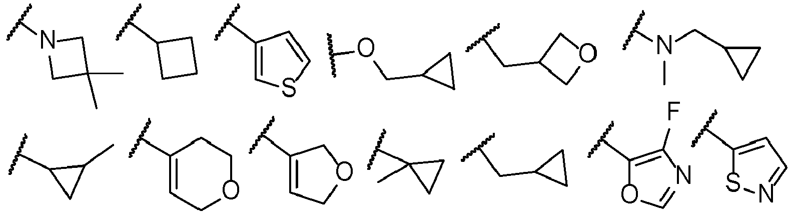

More suitably, R³ may more suitably be selected from the group consisting of the following structures:

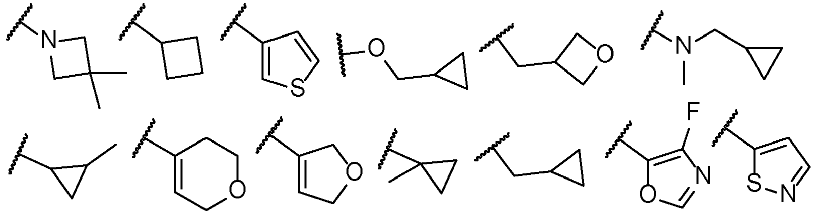

Alternatively, in various embodiments, R³ may be selected from hydrogen or halogen; for example, R³ may be hydrogen or Cl.

In any embodiments, R⁴ may be hydrogen; alternatively R⁴ may be F and R⁵ and R⁶ may each be hydrogen; alternatively R⁴ may be F and R⁵ and R⁶ may each be hydrogen; or R⁴, R⁵ and R⁶ may all be hydrogen.

In various embodiments, R⁵ may be a 4 or 5 membered heterocycloalkyl; R⁵ may be C₁-C₃ alkyl; R⁵ may be a 3, 4 or 5 membered cycloalkyl; or R⁵ may be an optionally substituted C₃-C₆ cycloalkyl, C₃-C₆ cycloalkenyl, C₃-C₆ heterocycloalkyl, C₃-C₆ aryl or C₃-C₆ heteroaryl.

More suitably, R⁵ may be selected from the group consisting of hydrogen and the following structures:

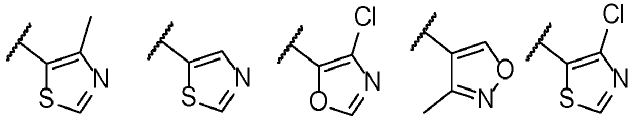

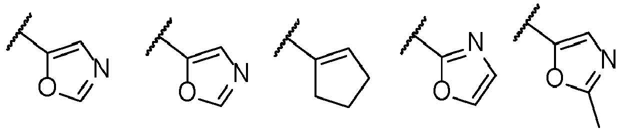

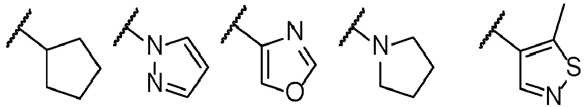

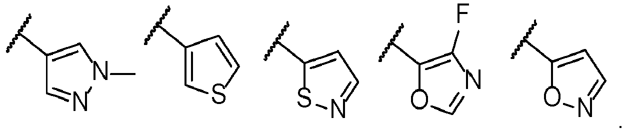

In particularly beneficial embodiments, R⁵ may be selected from hydrogen, F, methyl or pyrrolidine. In alternative beneficial embodiments, R⁵ may be hydrogen or R⁵ may be F.

In any embodiments, R⁶ may be an optionally substituted 4 or 5 membered heterocycloalkyl; R⁶ may be an optionally substituted 8, 9 or 10 membered bicyclic or spirocyclic heterocycloalkyl; R⁶ may be an optionally substituted 5 or 6 membered heteroaryl; R⁶ may be an optionally substituted 4, 5 or 6 membered cycloalkyl or spirocycloalkyl; R⁶ may be a C₁-C₃ haloalkoxy; or R⁶ may be a C₂-C₈ alkylamine or dialkylamine. Typically, each hetero moiety may include 1 or 2 heteroatoms independently selected from O, S and N. Suitably, any optional substituents may be independently selected from 1 or 2 substituents from the group consisting of C₁-C₃ alkyl, C₁-C₃ haloalkyl, C₁-C₃ alkoxyl, F, Cl and hydroxyl.

More suitably, in various aspects and embodiments, R⁶ is selected from the group consisting of hydrogen and the following structures:

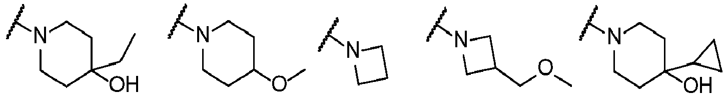

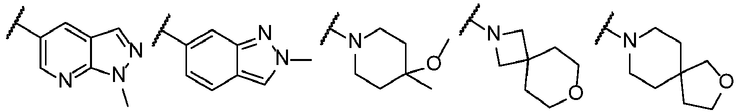

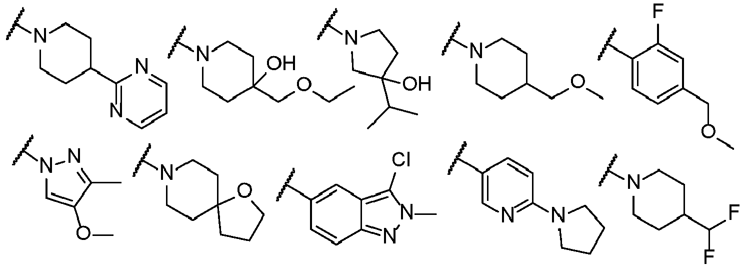

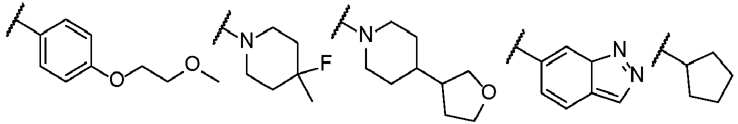

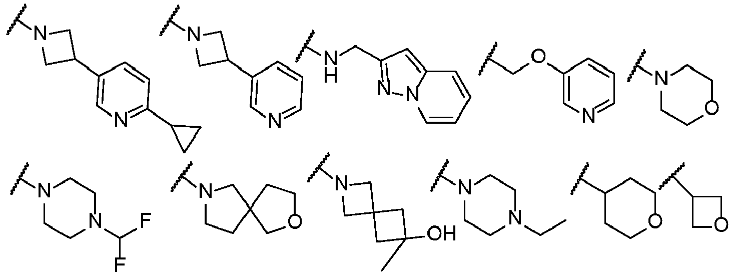

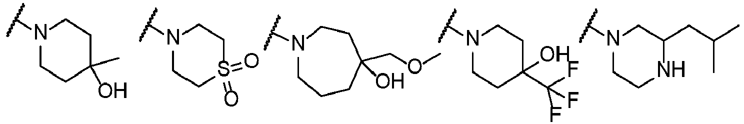

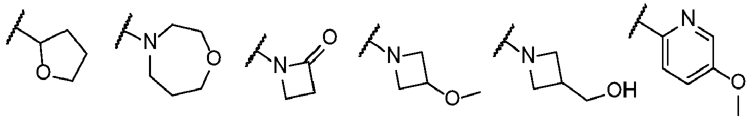

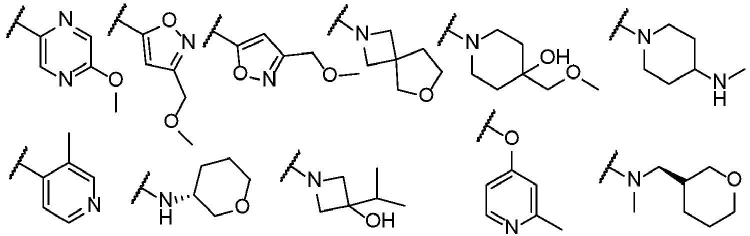

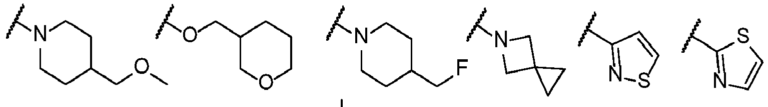

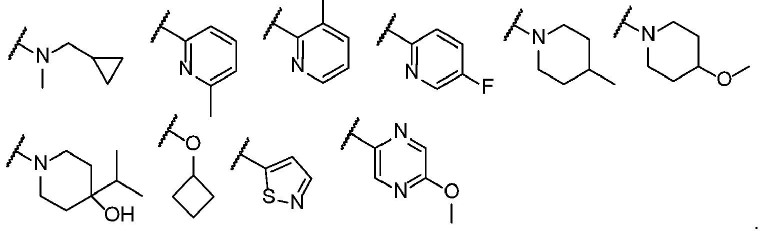

More particularly, in various aspects and embodiments, R⁶ may be selected from the group consisting of hydrogen and the following structures: 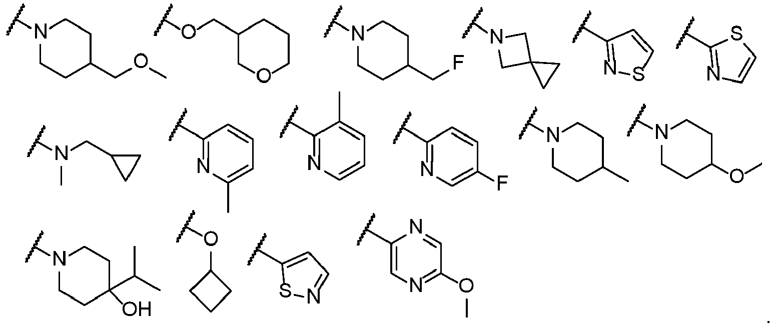 In some particularly beneficial embodiments, R⁶ is selected from hydrogen, F, methyl or pyrollidine. In other embodiments, R⁶ may be hydrogen; or R⁶ may be selected from F or CI;

In various alternative aspects and embodiments R⁶ is hydrogen. For example R⁶ is hydrogen and R⁵ is selected from the group of structures shown in the various embodiments above.

In any embodiments, L is suitably -CH₂- or -CH₂-CH₂-.

Q may be selected from the group consisting of optionally substituted 5 to 7 membered heterocycloalkyl and optionally substituted 4 to 7 membered mono or fused or bridged bicycloalkylamine. In any such embodiments, each heterocyclic moiety may include 1 or 2 heteroatoms independently selected from O and N. Further, optional substituents of any such Q moiety, where present, may be independently selected from 1 to 3 substituents from the group consisting of C₁-C₃ alkyl, C₁-C₃ haloalkyl, C₁-C₃ alkoxyl, C₁-C₃ alkylamine, C₁-C₃ dialkylamine, C₁-C₃ carbonyl, amine, amino-C₁-C₃ alkyl, oxo, F, Cl and hydroxyl.

Suitably, Q is selected from the group consisting of the following structures:

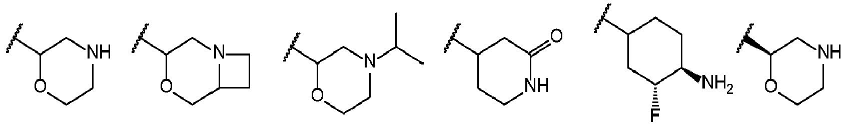

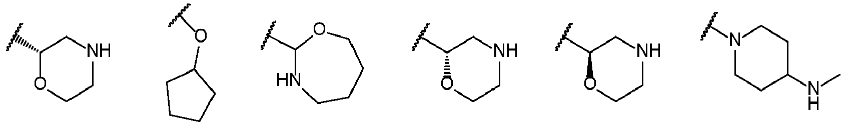

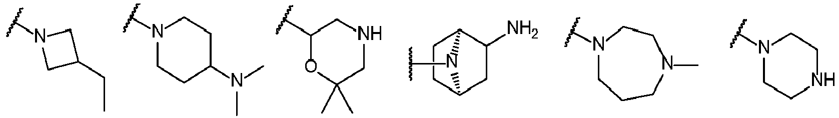

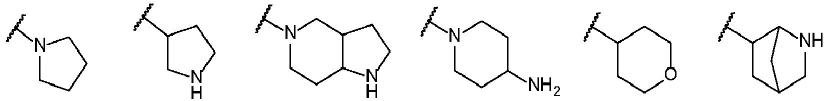

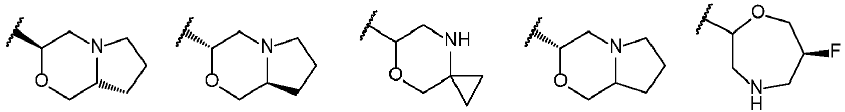

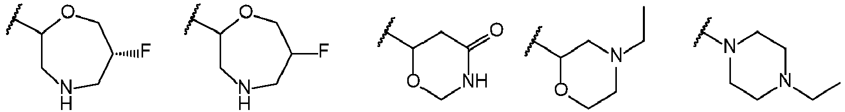

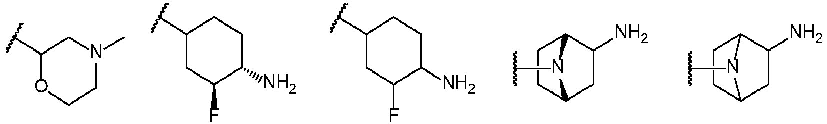

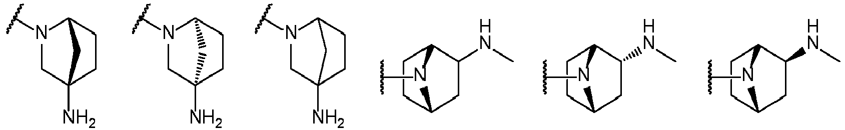

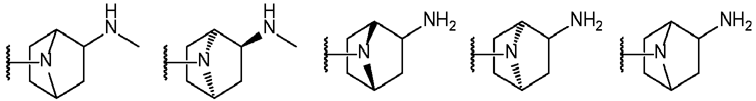

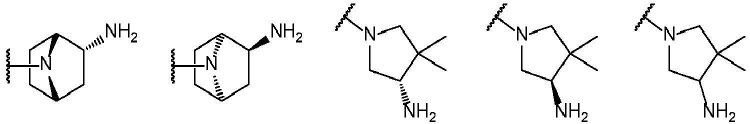

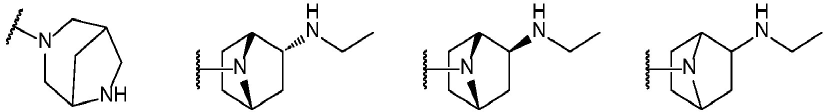

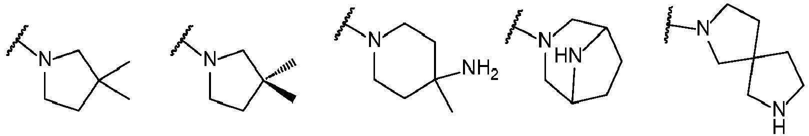

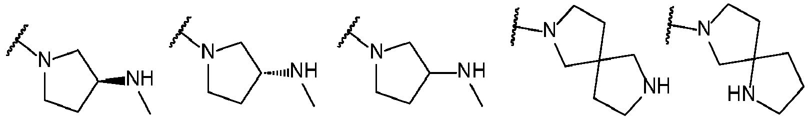

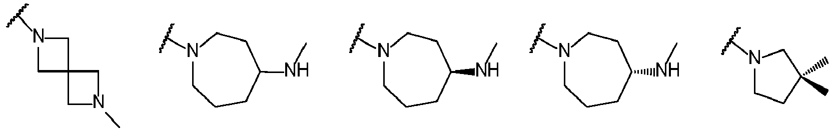

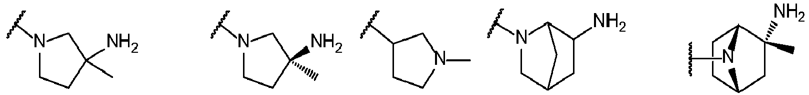

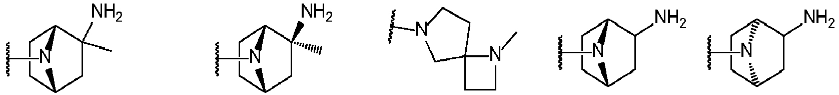

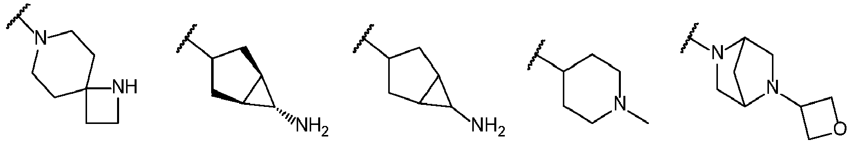

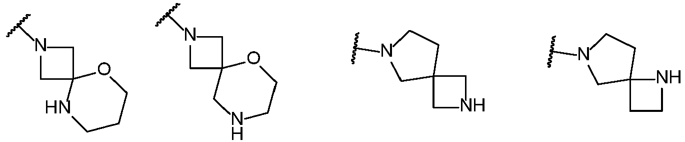

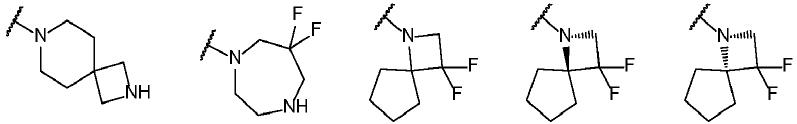

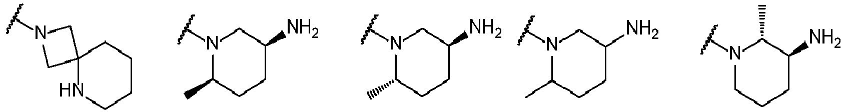

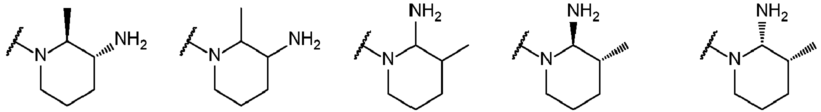

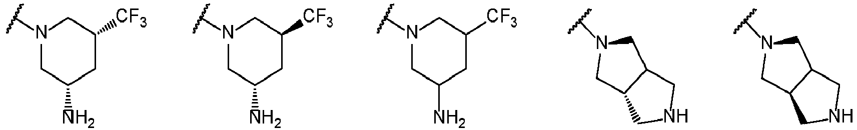

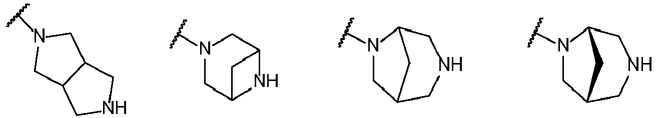

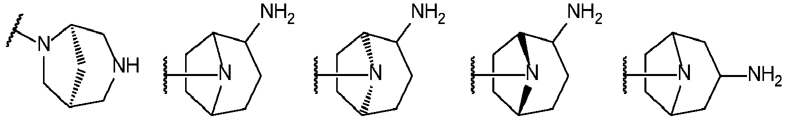

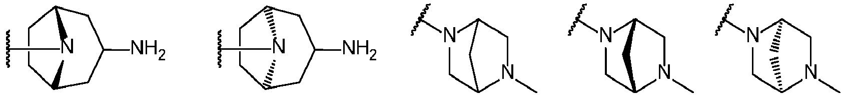

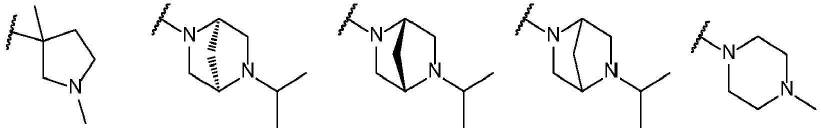

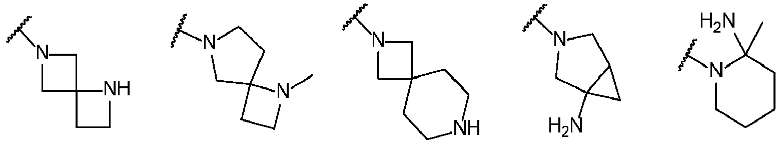

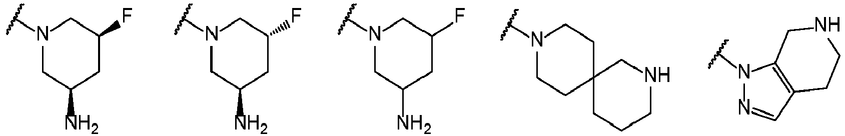

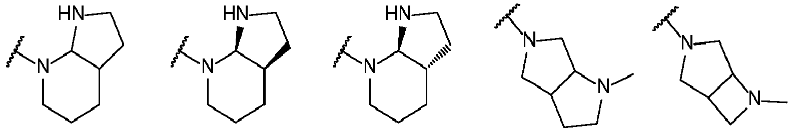

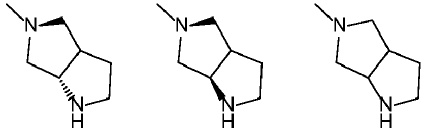

More suitably, Q is selected from the group consisting of the following structures: 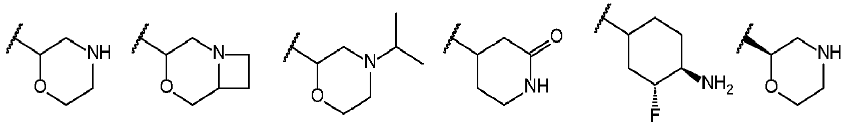 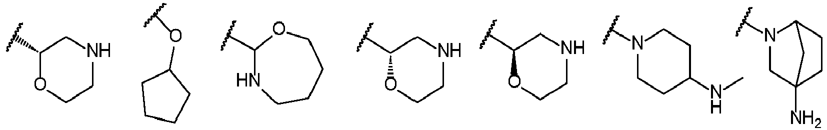 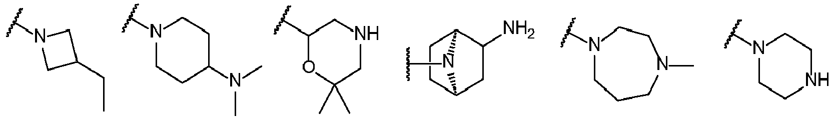 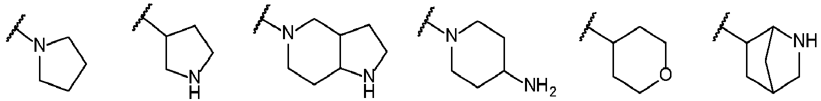 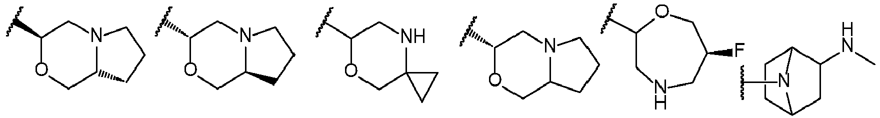 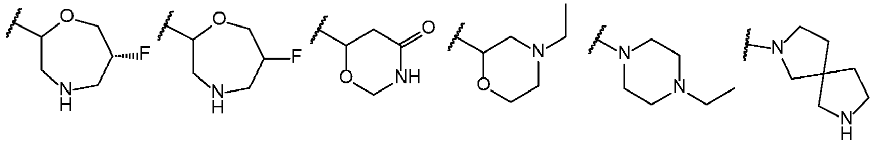 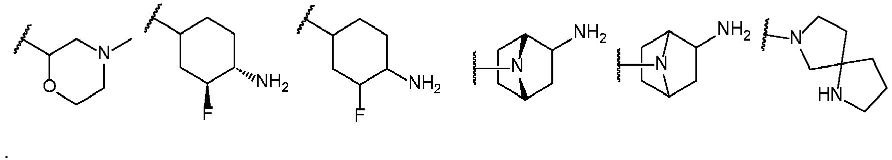 Advantageously, Q is selected from the group consisting of the following structures: 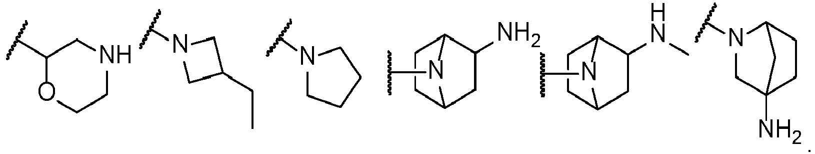 Beneficially, R⁷ may be independently selected from C₁-C₃ alkyl, C₁-C₃ haloalkyl, hydroxyl, oxo, F, C₁-C₃ alkylamino, C₁-C₃ dialkylamino or amino. Alternatively, R⁷ may be independently selected from methyl, ethyl, amine and F. In embodiments, there may be 1, 2 or 3 R⁷ groups in the compounds of this disclosure.

In particularly suitably compounds according to structure (I), the compound may be defined wherein: R¹ is cyano; and/or R² and R^{2'} are each independently selected from hydrogen or F; and/or R³ is selected from hydrogen, propyl, i-propyl, CHF₂, cyclopropyl, -CH₂-cyclopropyl, -CH₂-oxetane, isothiazole, oxazole, isooxazole, pyridyl, methoxyl, -O-CH₂-cyclopropyl, -O-CH₂-CF₃, - O-CF₃, CF₃, Cl, amino, - N(CH₃)(CH₂-isopropyl), pyrrolidine, cyano; and/or R⁴ is hydrogen or F; and/or R⁵ is selected from hydrogen, methyl, cyclopropyl, cyclopentenyl, F, amino, NH-CH₂-CH₂-N(CH₃)₂; and/or R⁶ is selected from hydrogen, -O-cyclobutyl, -O-CH₂-oxane, -O-CH₂-CH₂-CHF₂, -O-CH₂-CH₂-CF₃, -O-CH₂-CF₃, -CH₂₋O-CHF₂, amino, -N(CH₃)(CH-(CH₃)₂, benzimdazole, pyridine; and/or L is a linker selected from a bond, -CH₂-, -C≡C-, -CH₂-NH-CH₂-, and/or Q is C₄-C₈ heterocycloalkyl which may comprise a further heteroatom selected from N or O; or Q is morpholinyl. In any such embodiments, each heteroaryl, heterocycloalkyl and/or cycloalkyl group is optionally substituted. In some particular embodiments, such groups may be optionally substituted with one or more groups selected from F, Cl, methyl, hydroxyl and amino.

In embodiments, the compound of formula (I) may be selected from any of the compounds shown in Table 1 below.

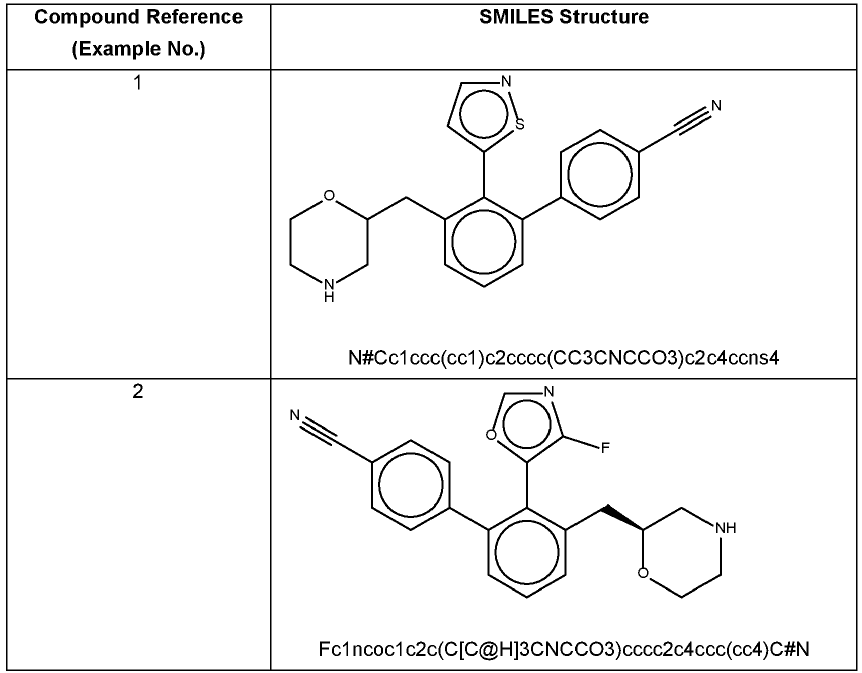

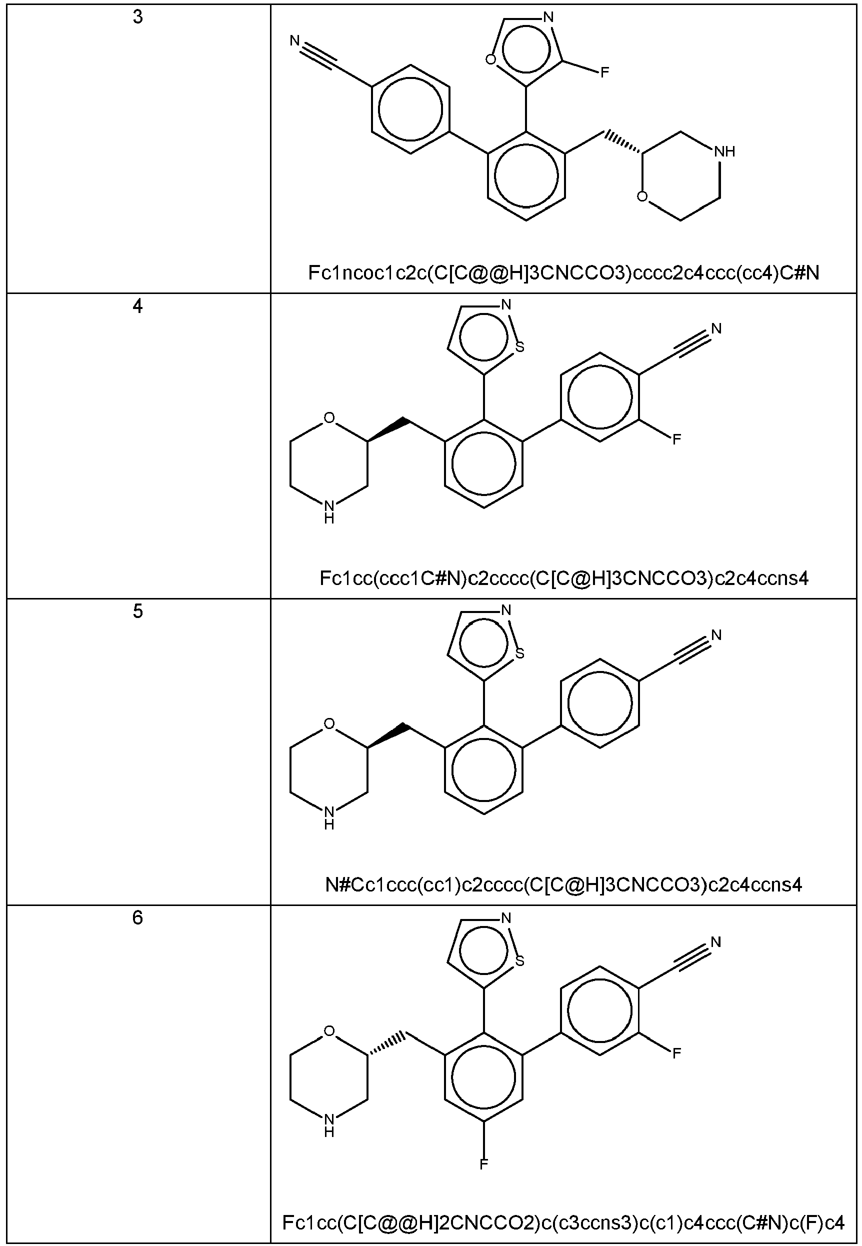

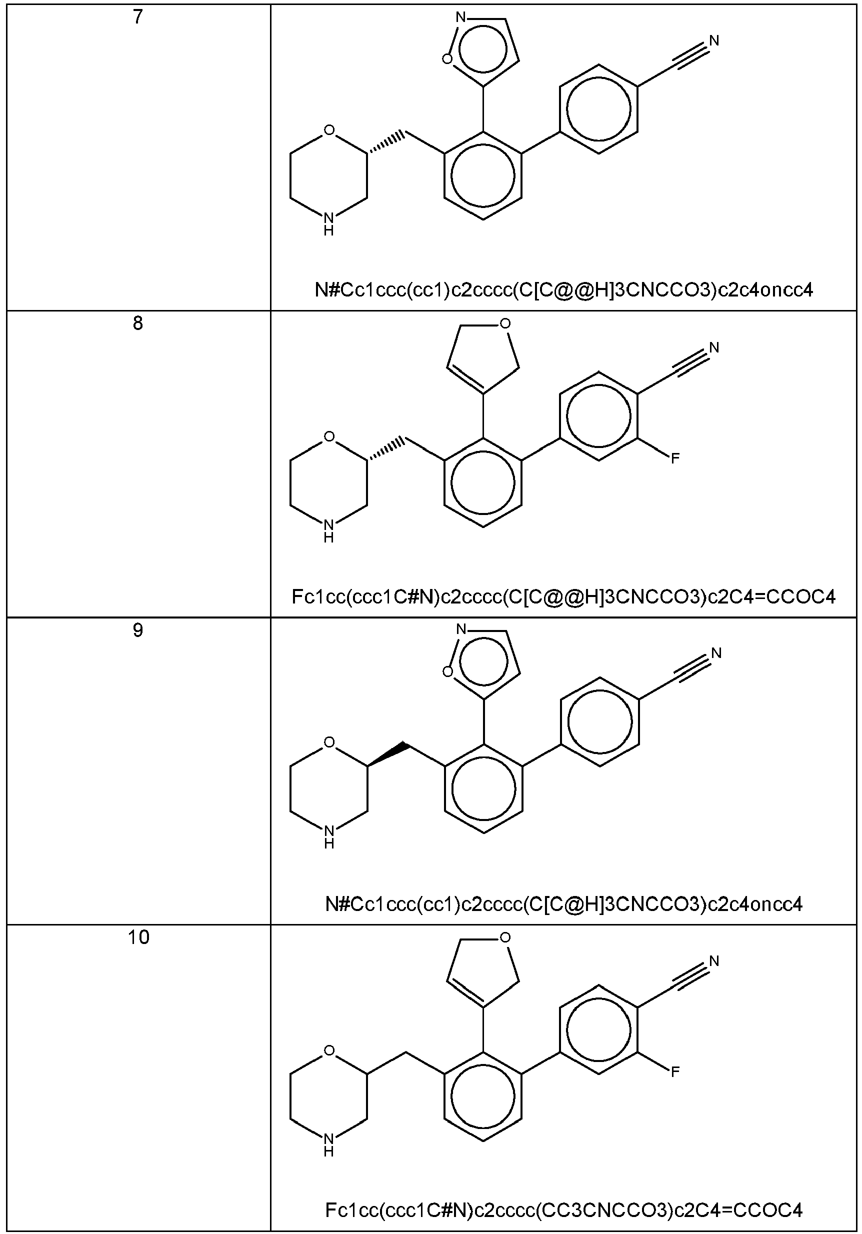

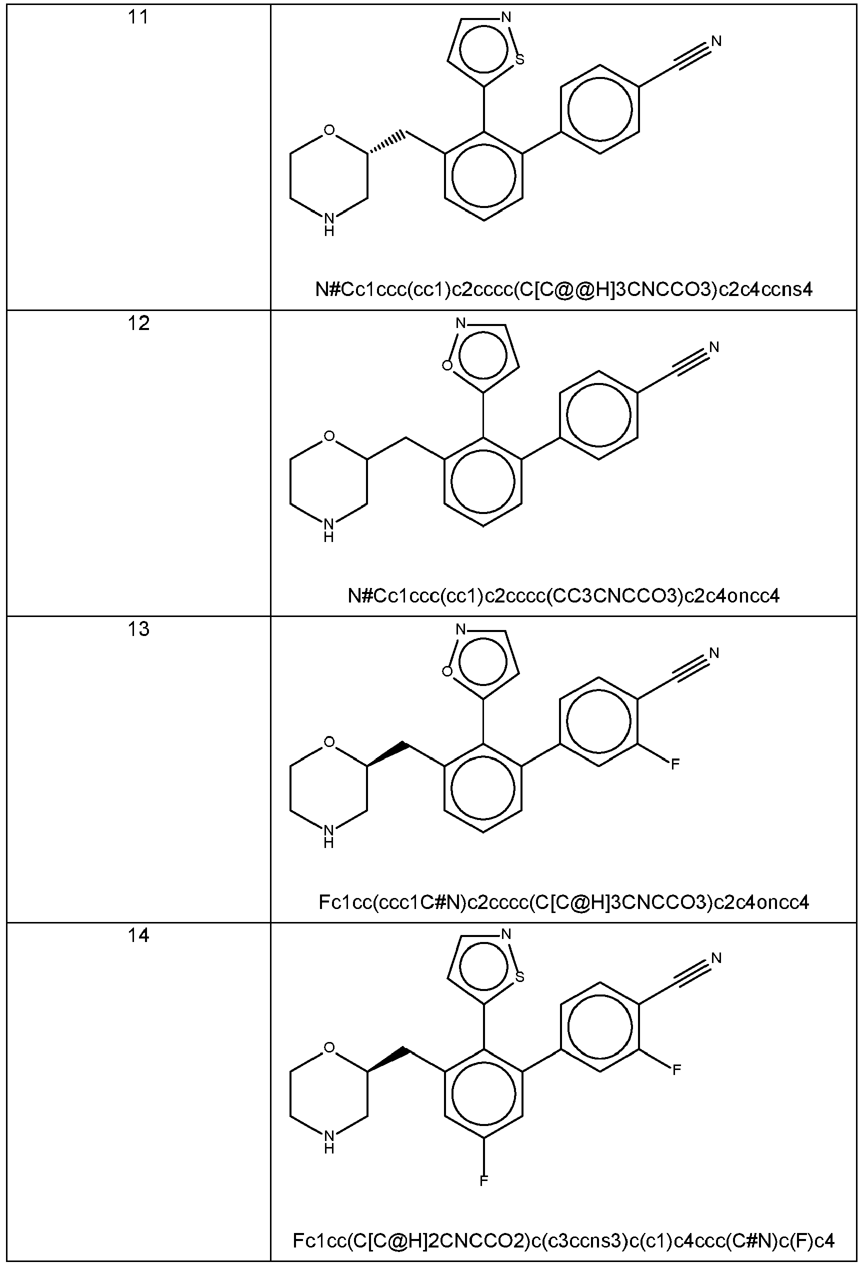

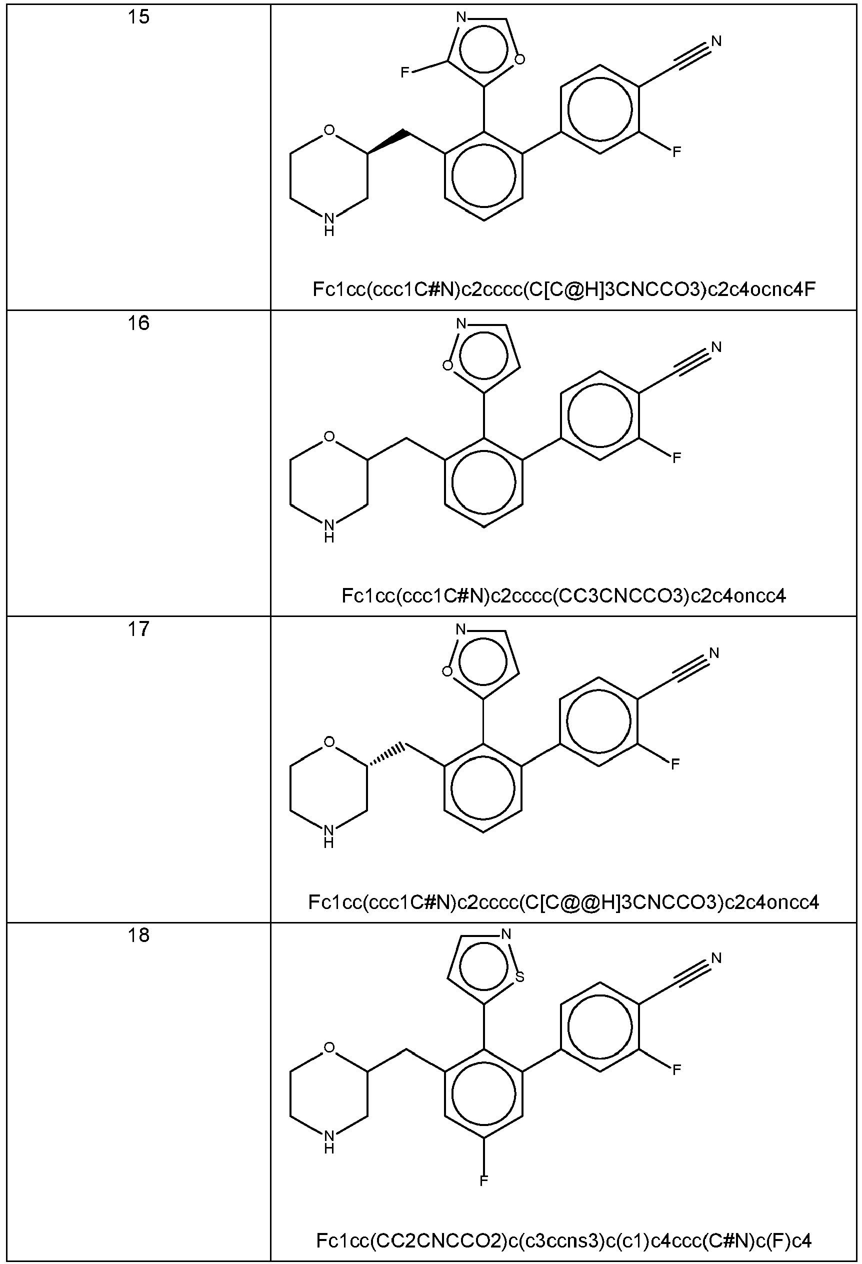

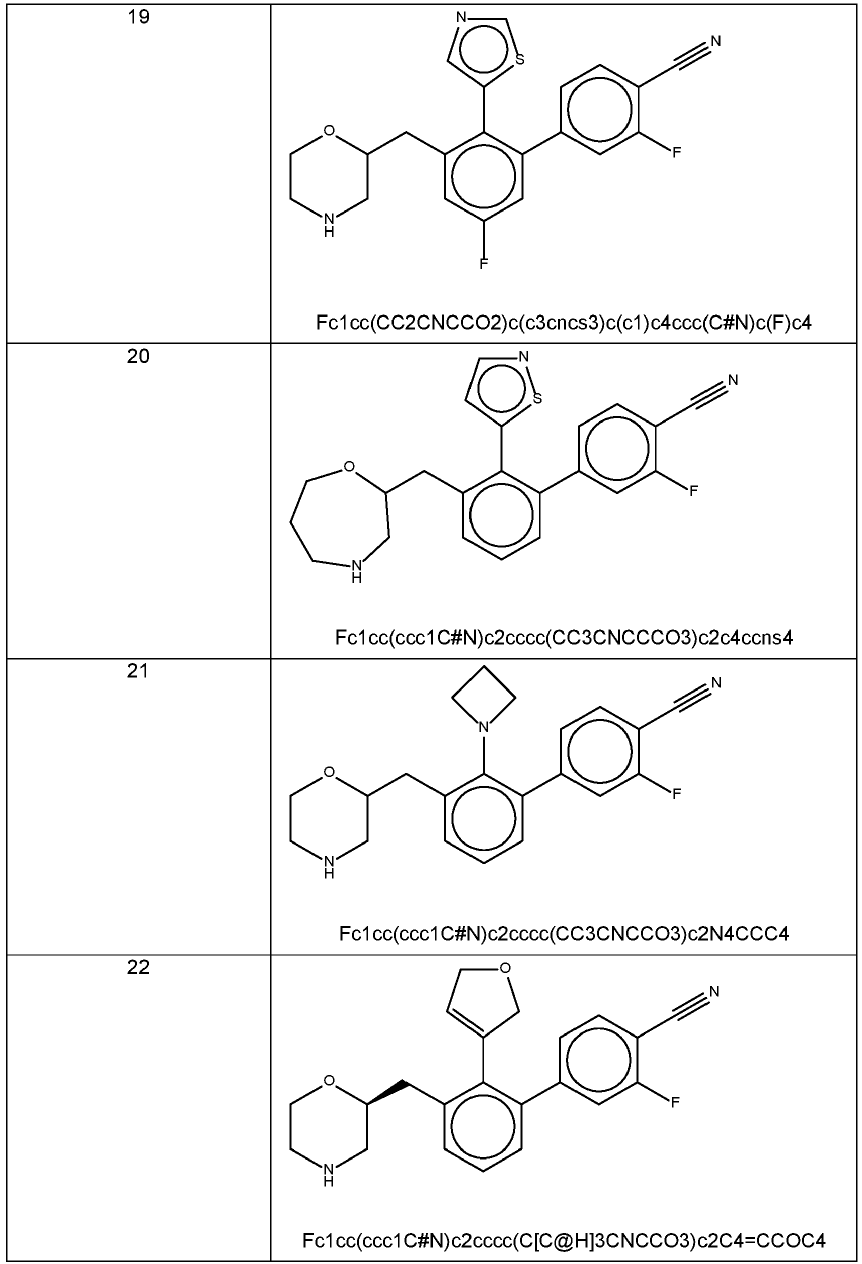

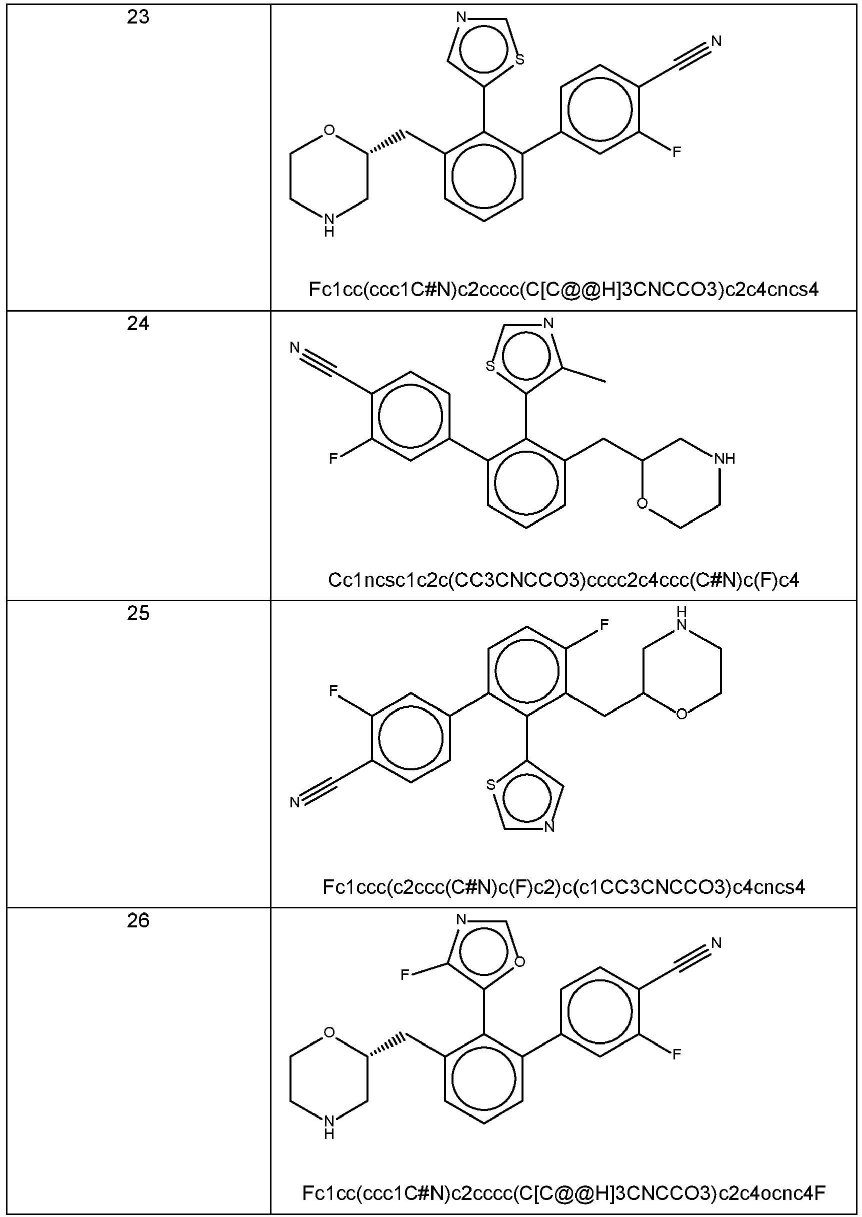

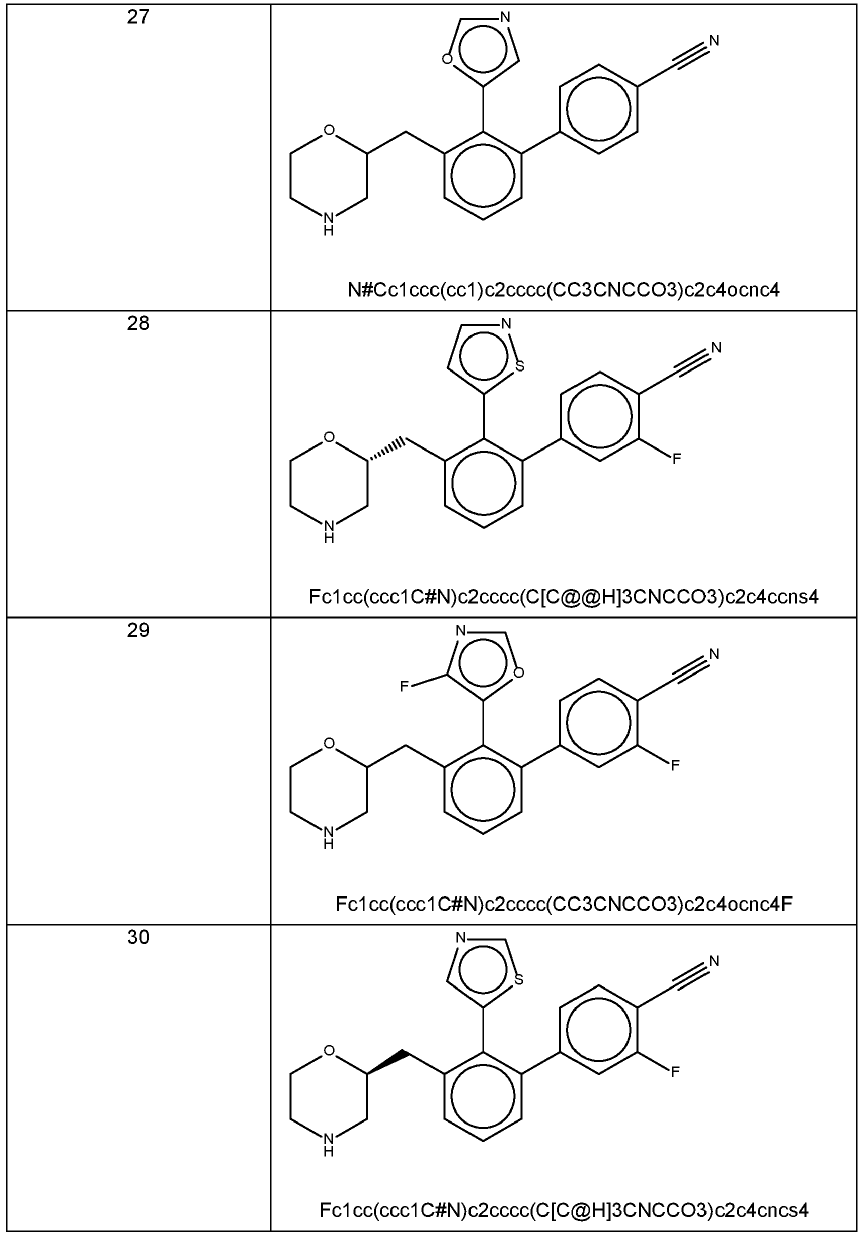

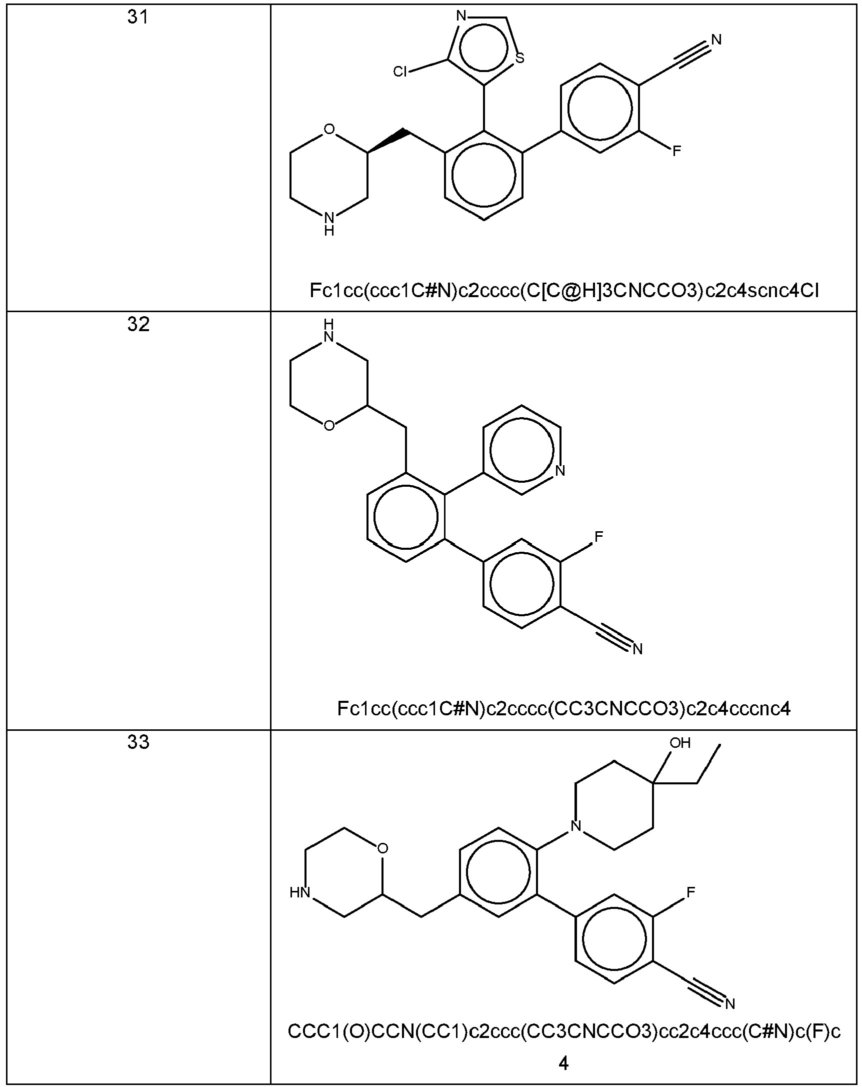

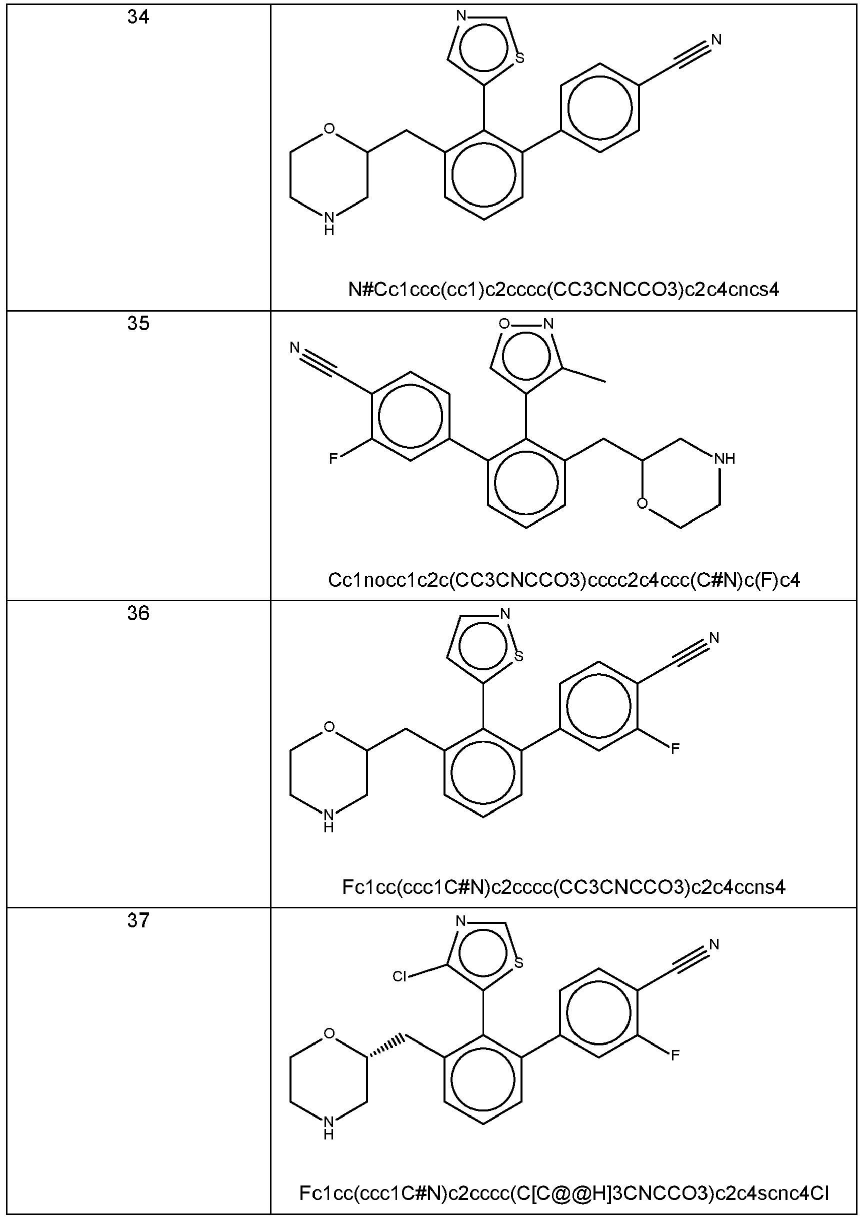

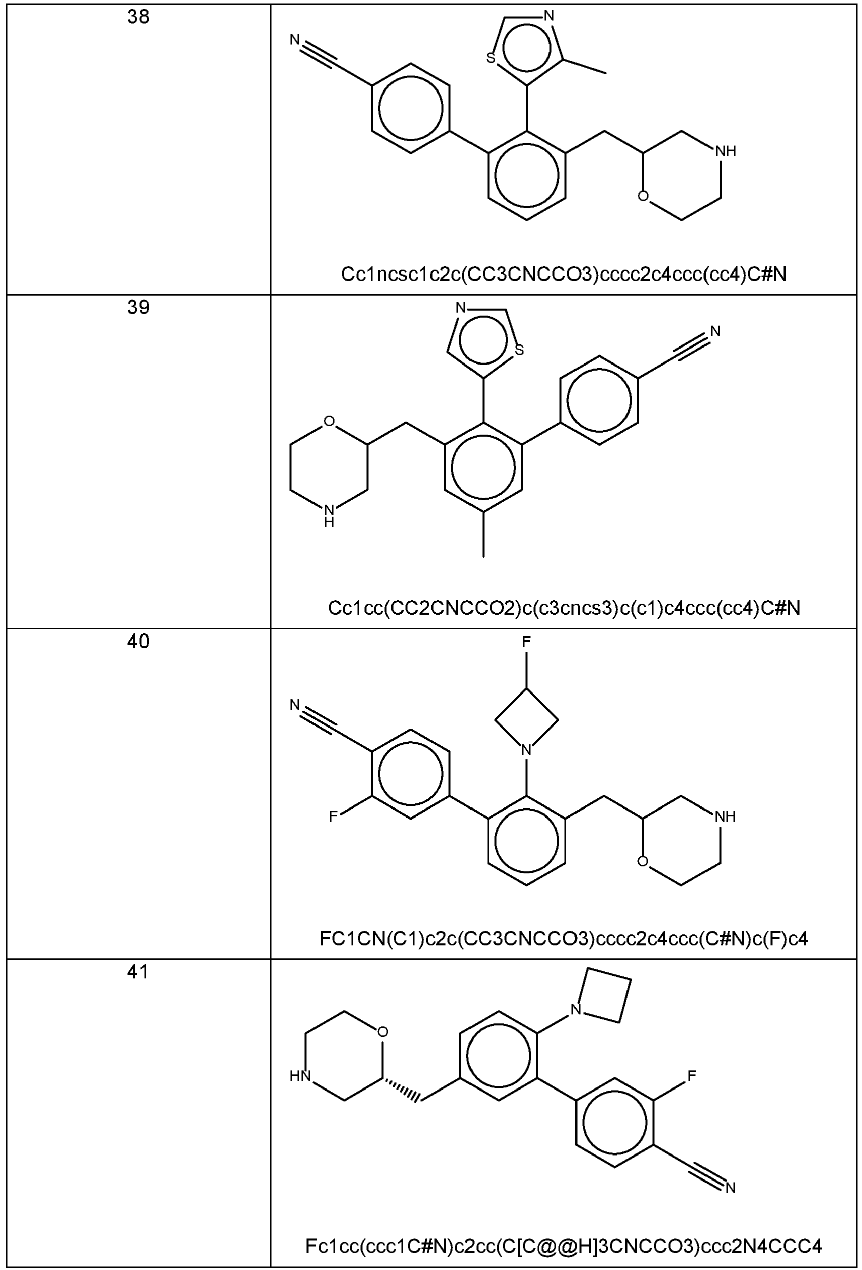

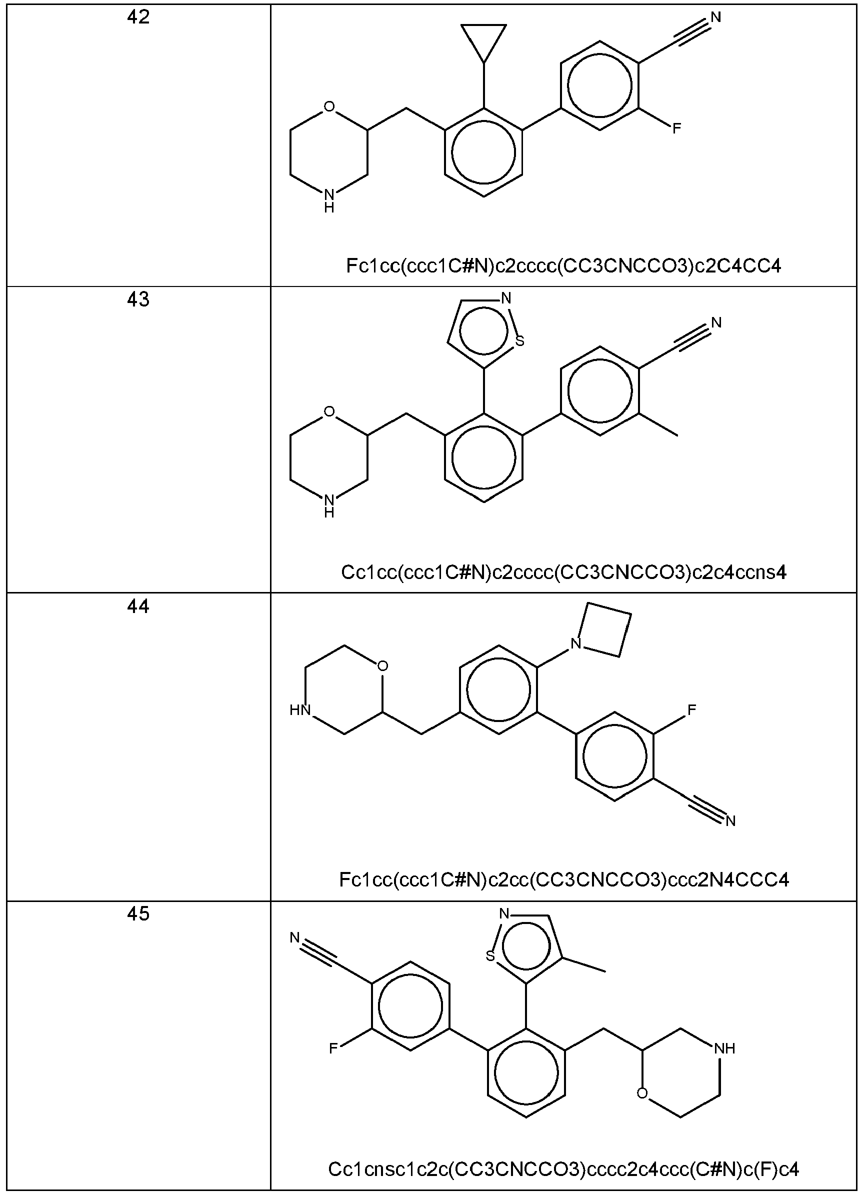

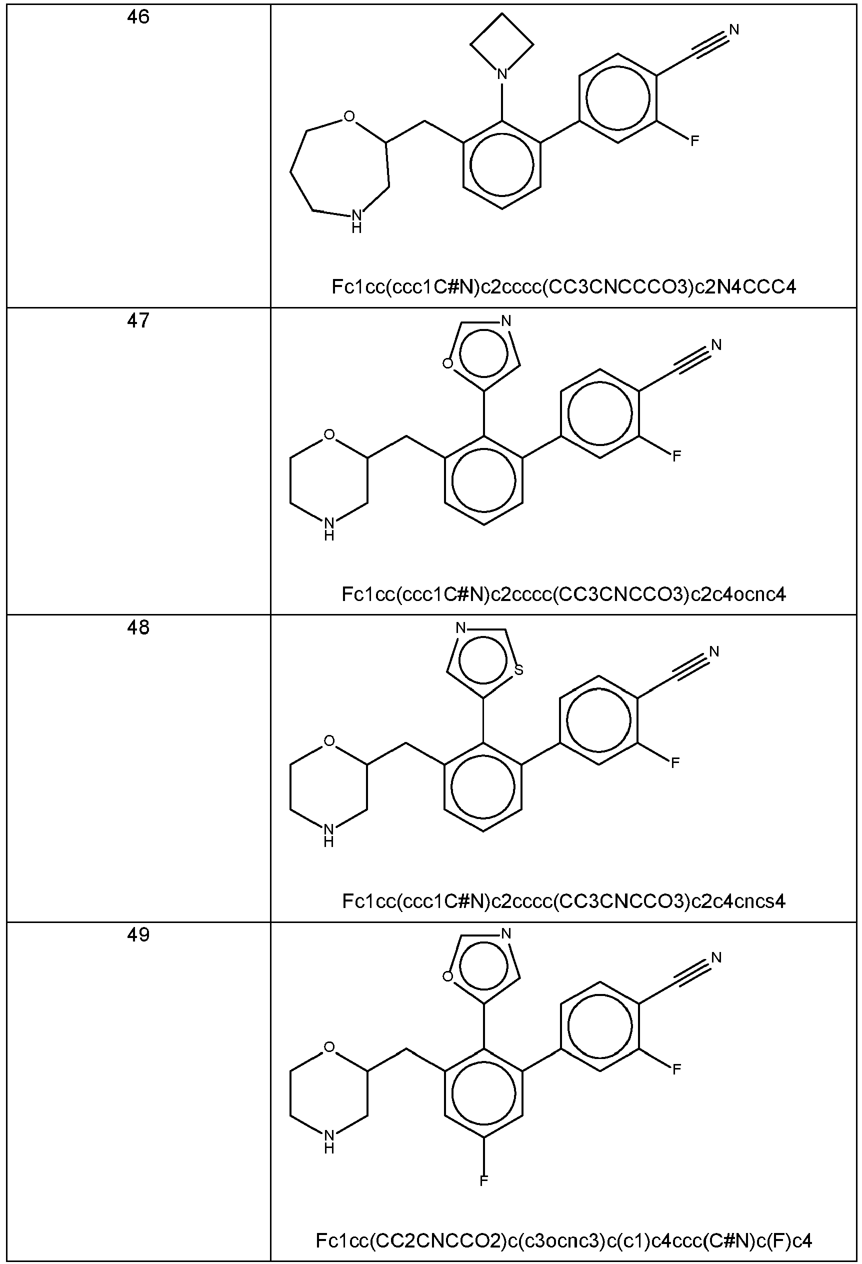

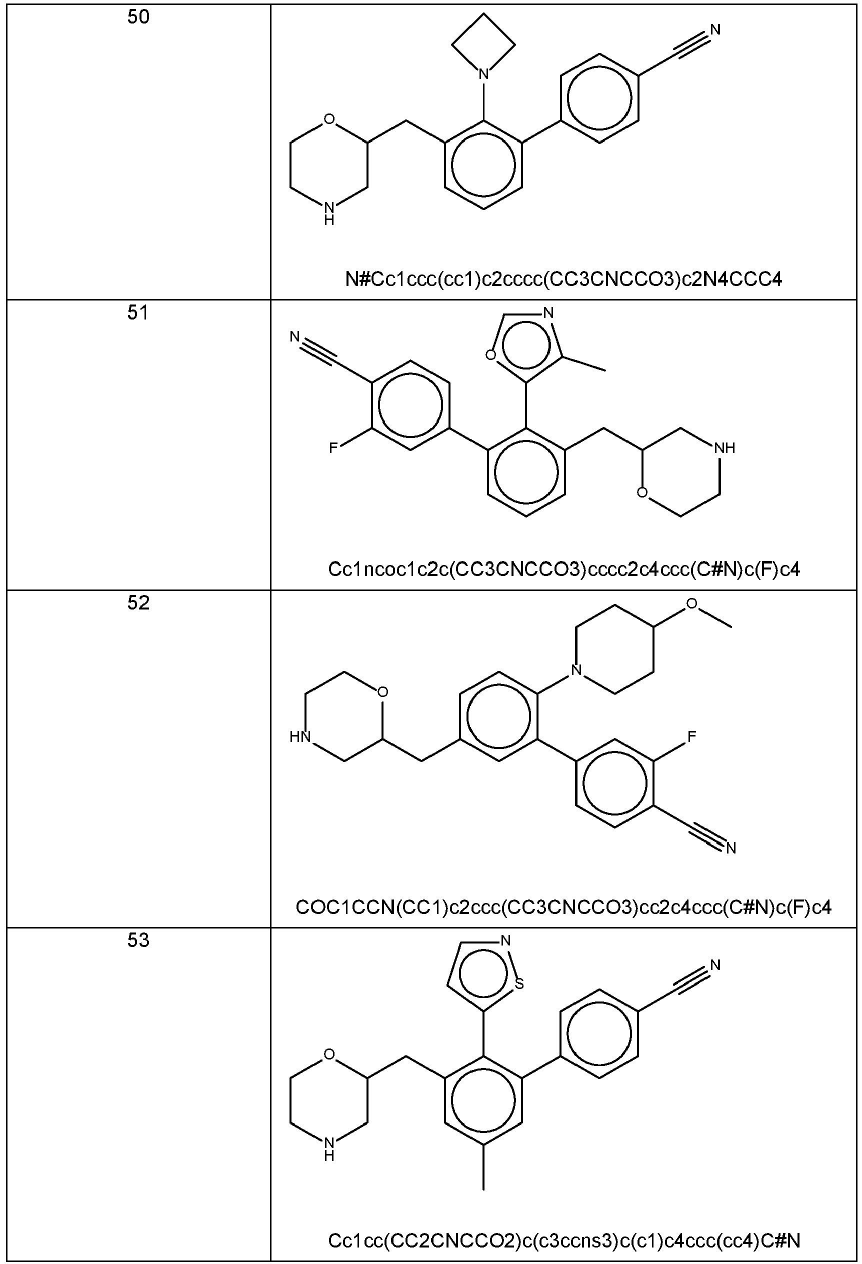

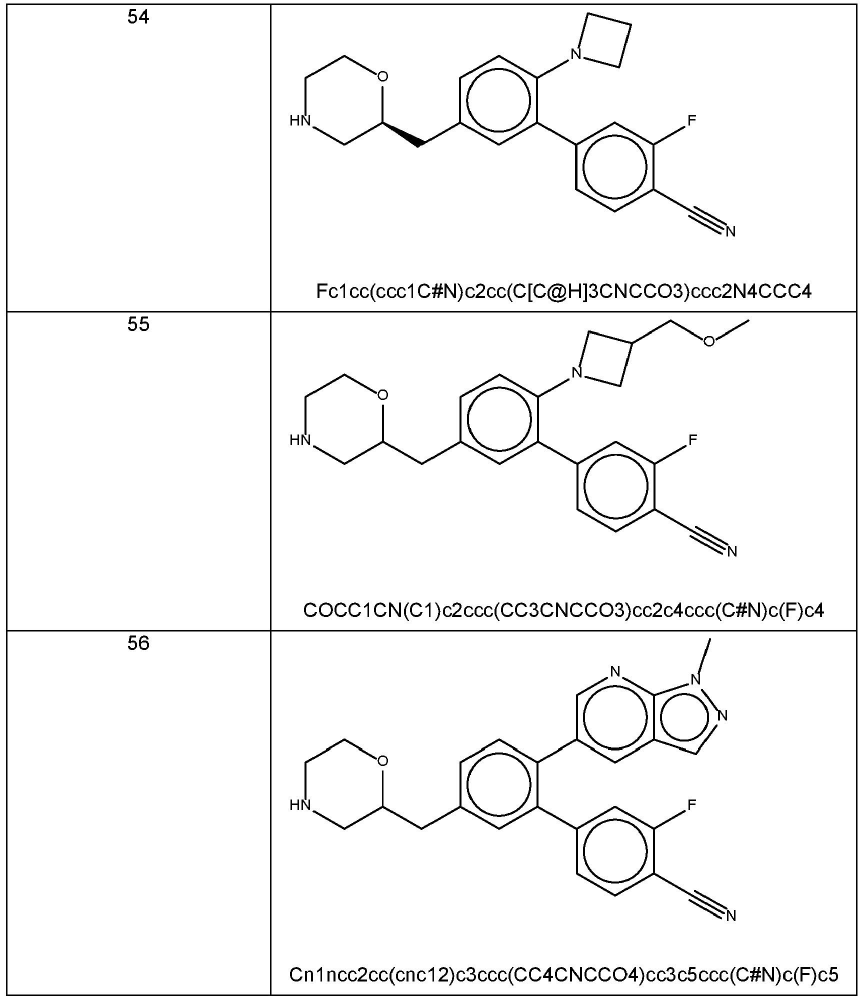

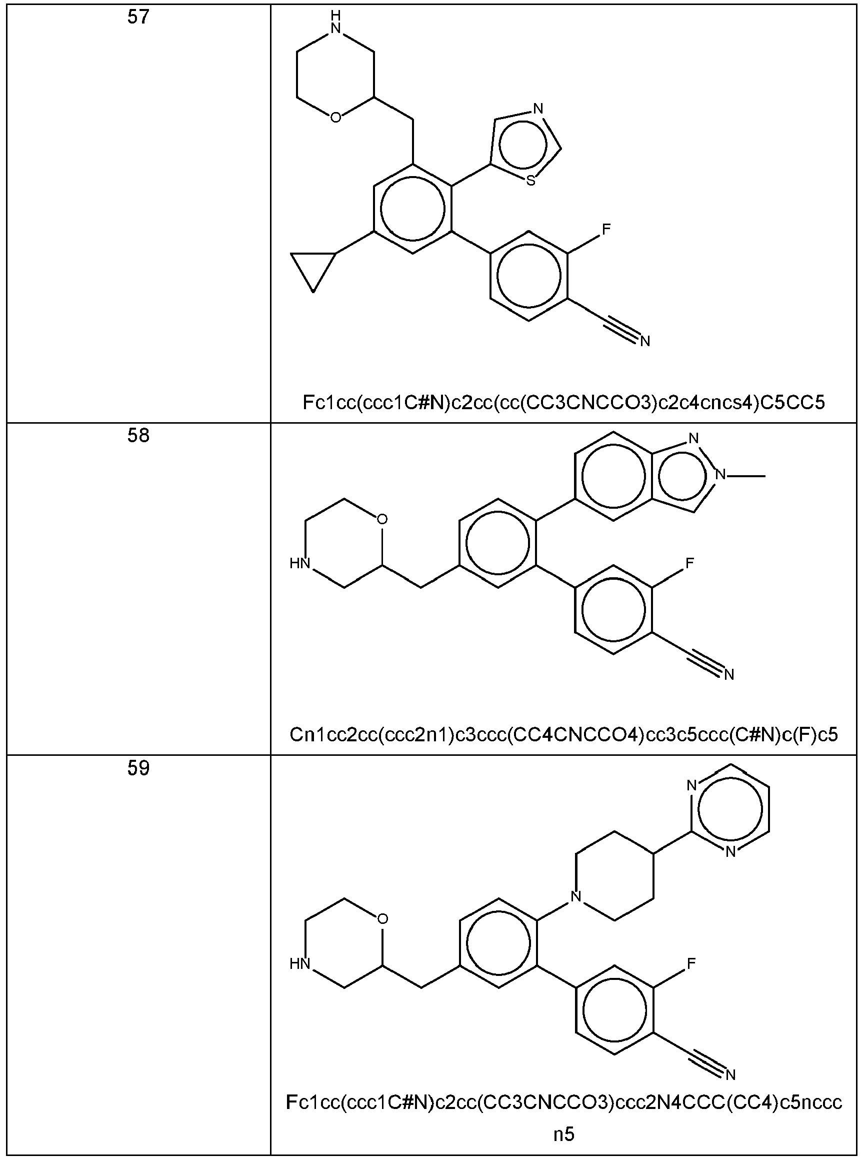

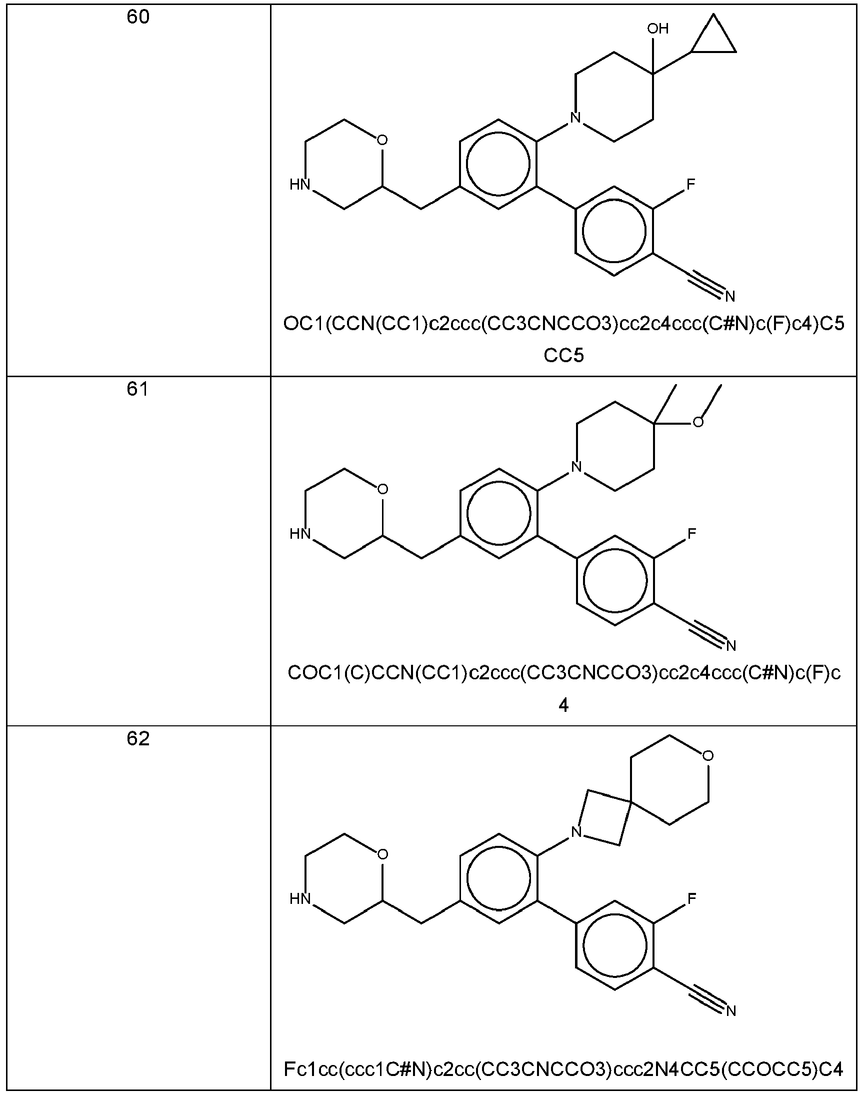

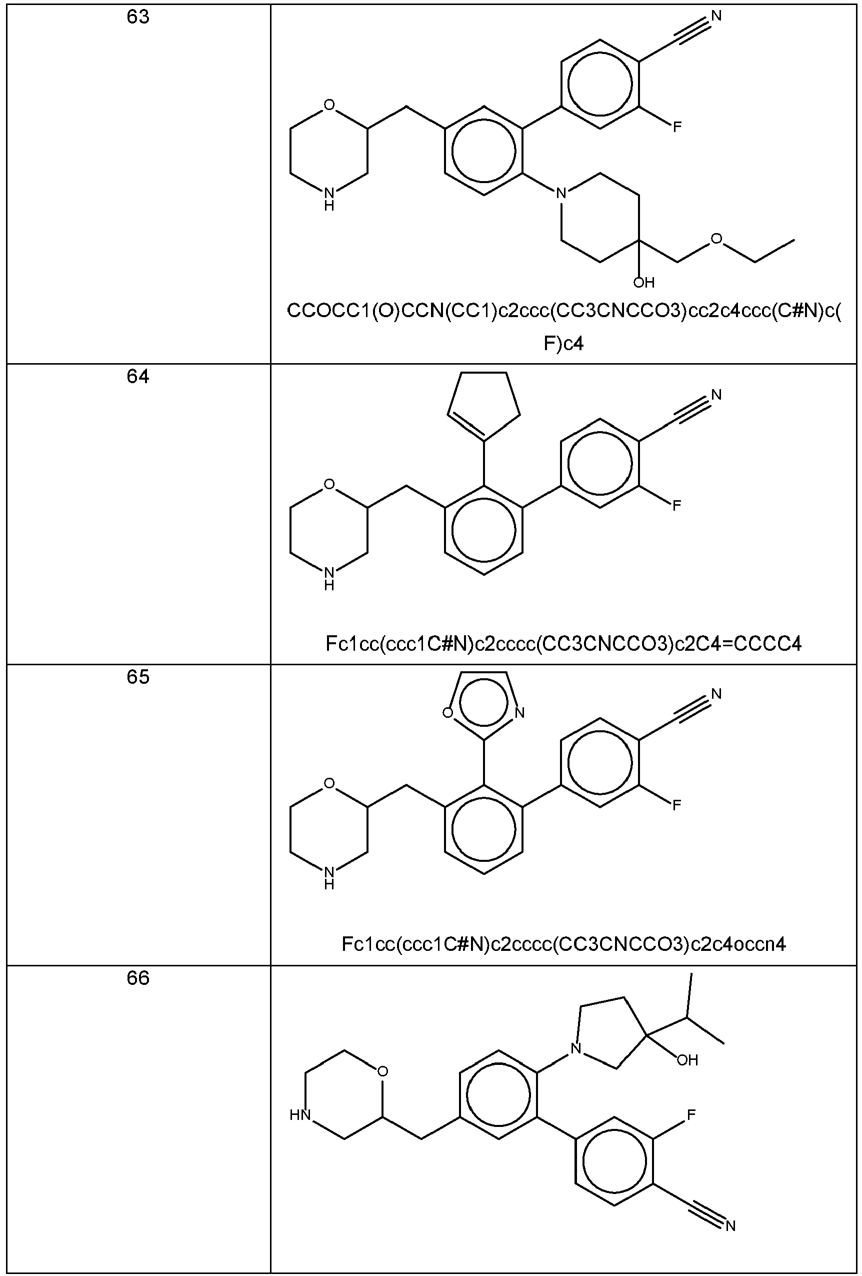

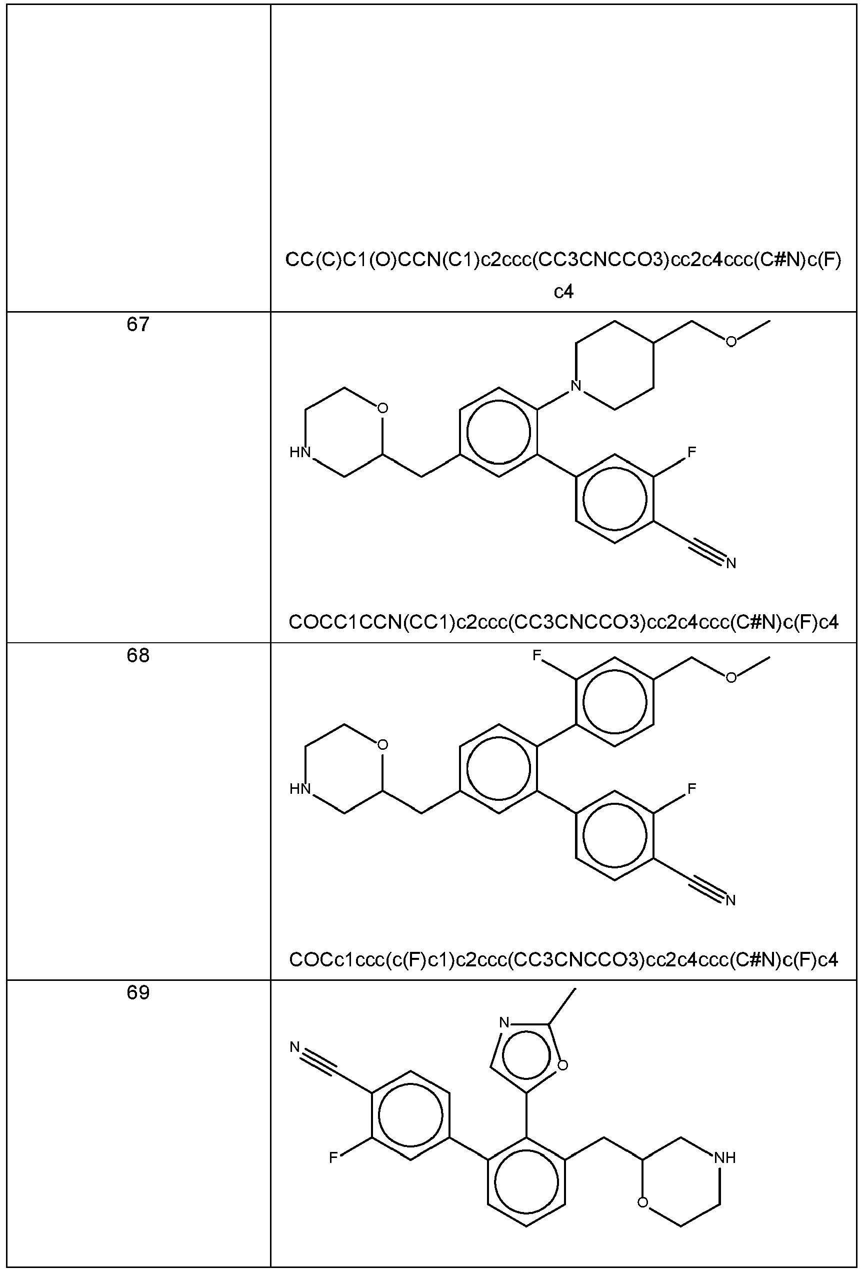

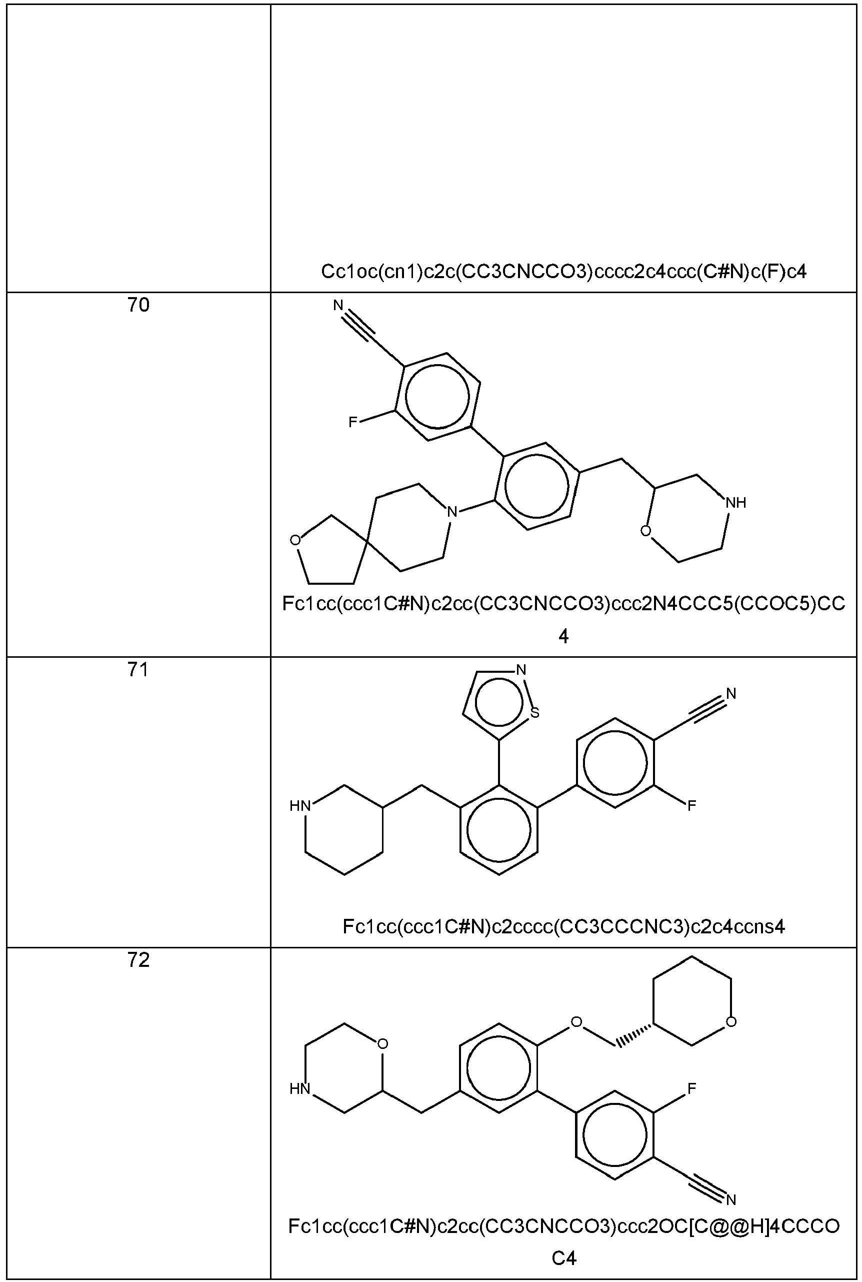

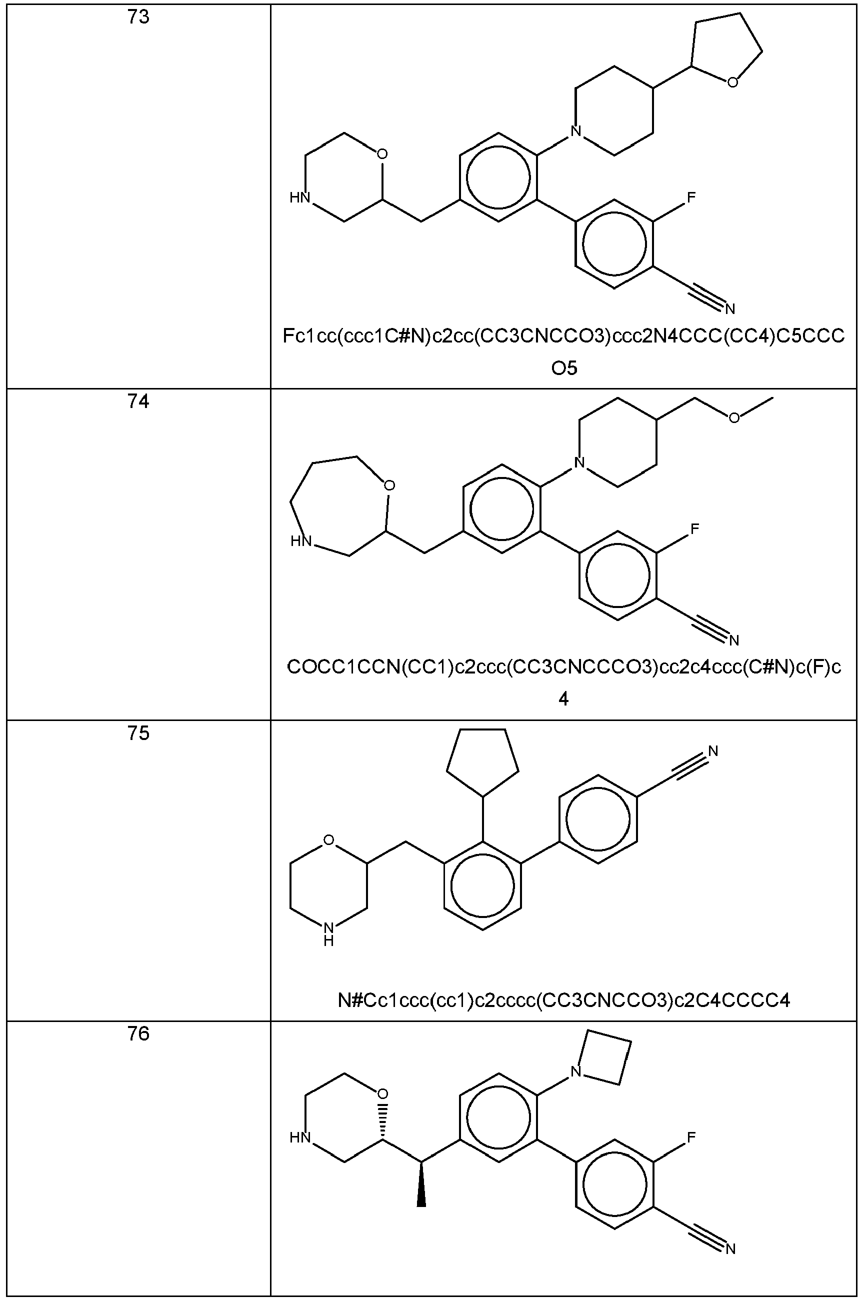

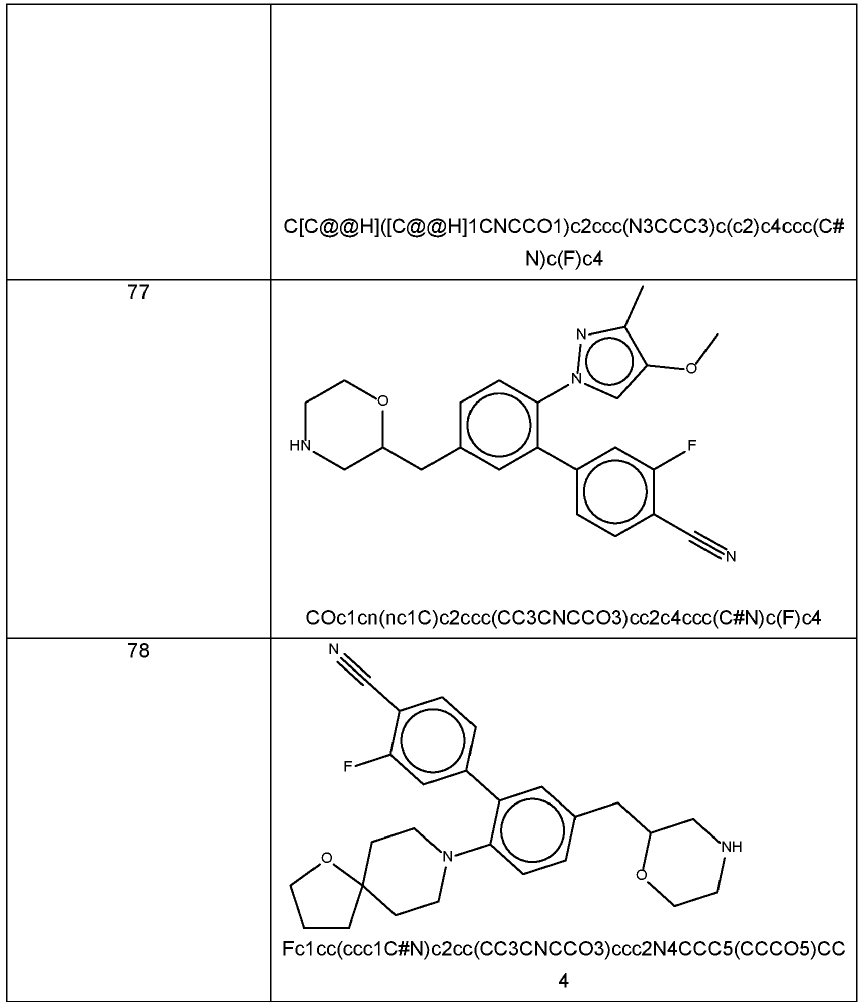

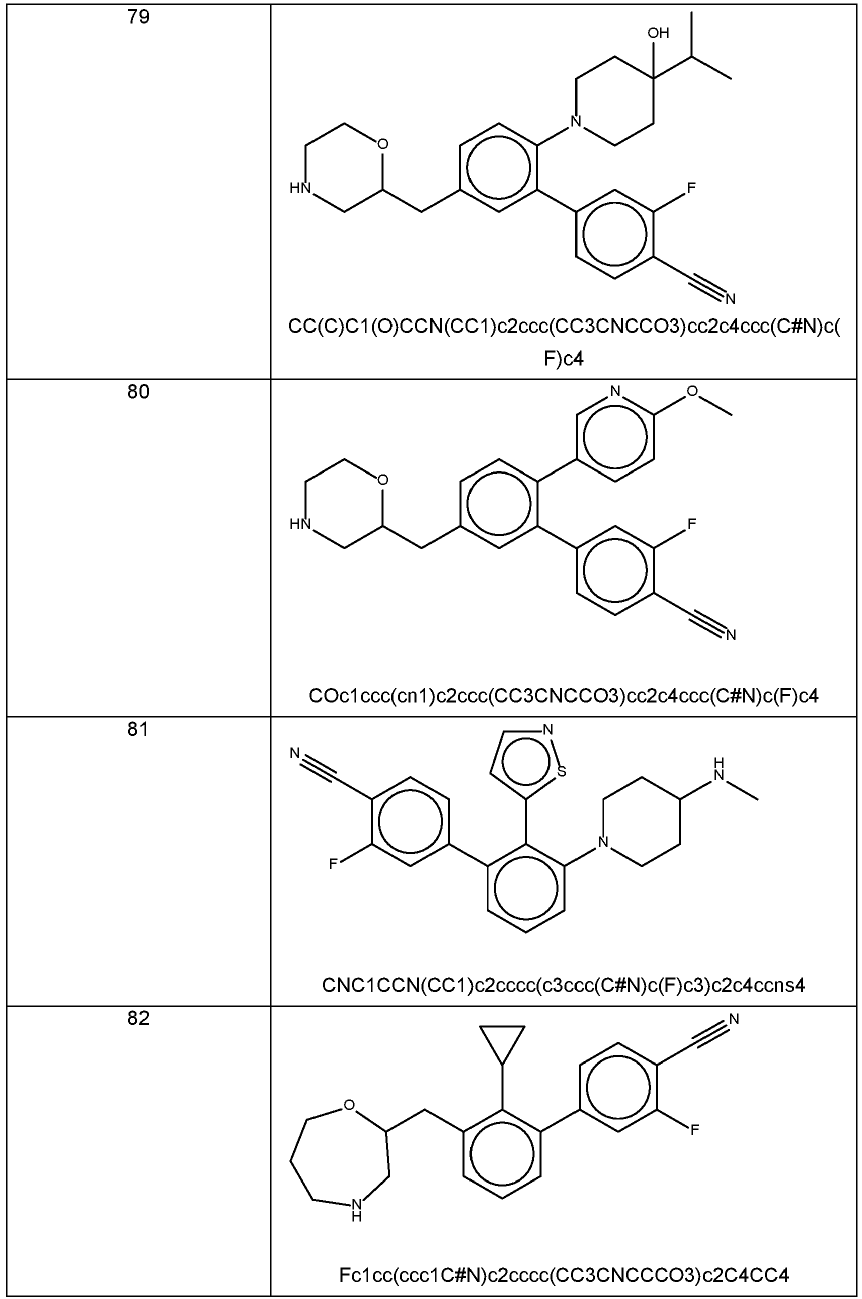

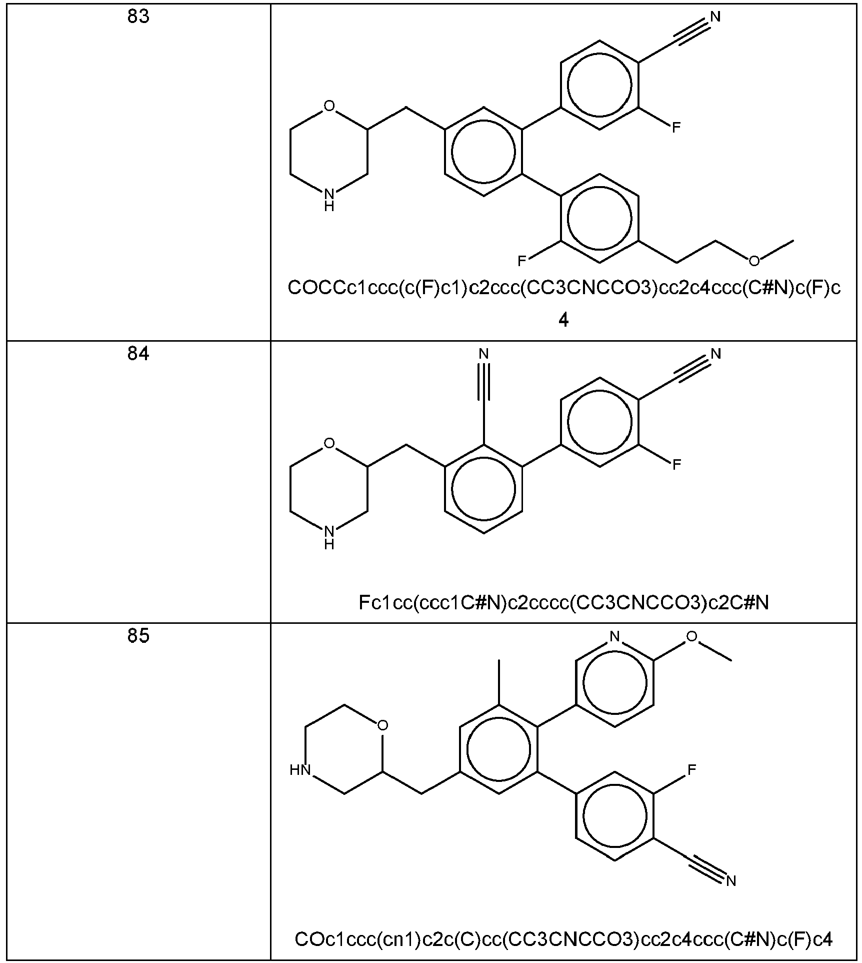

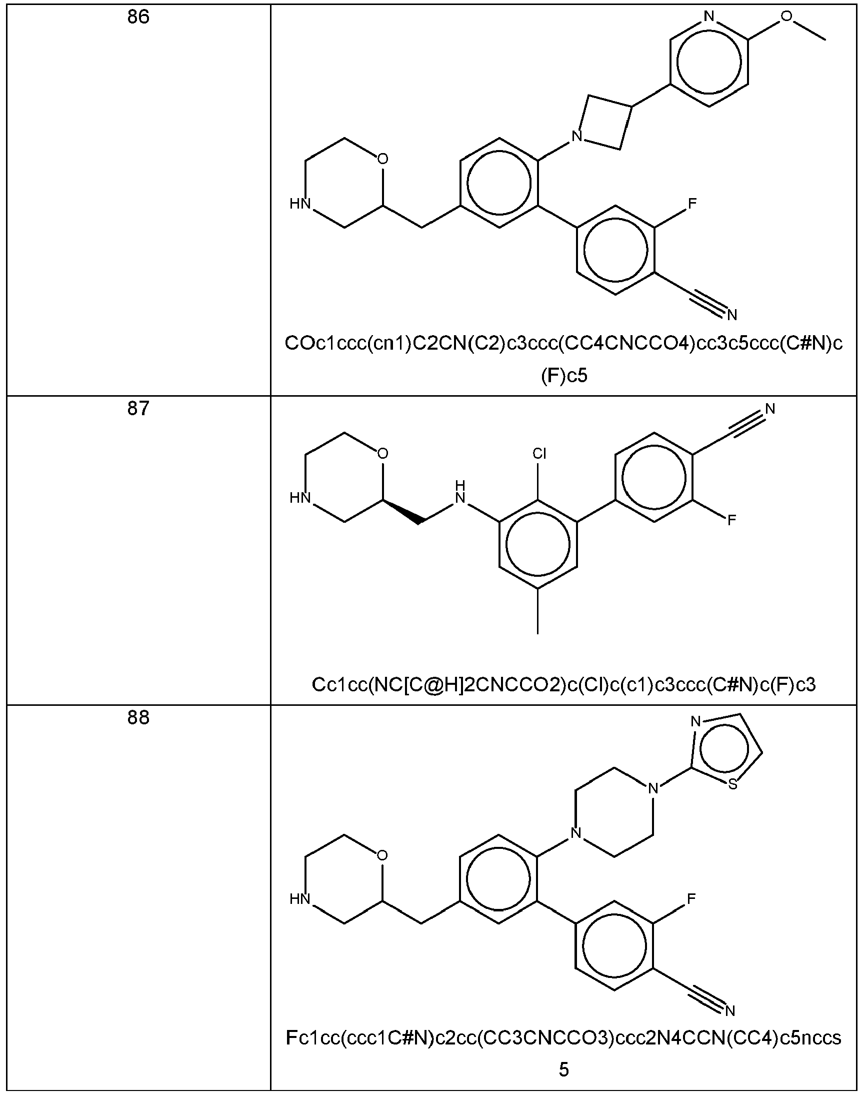

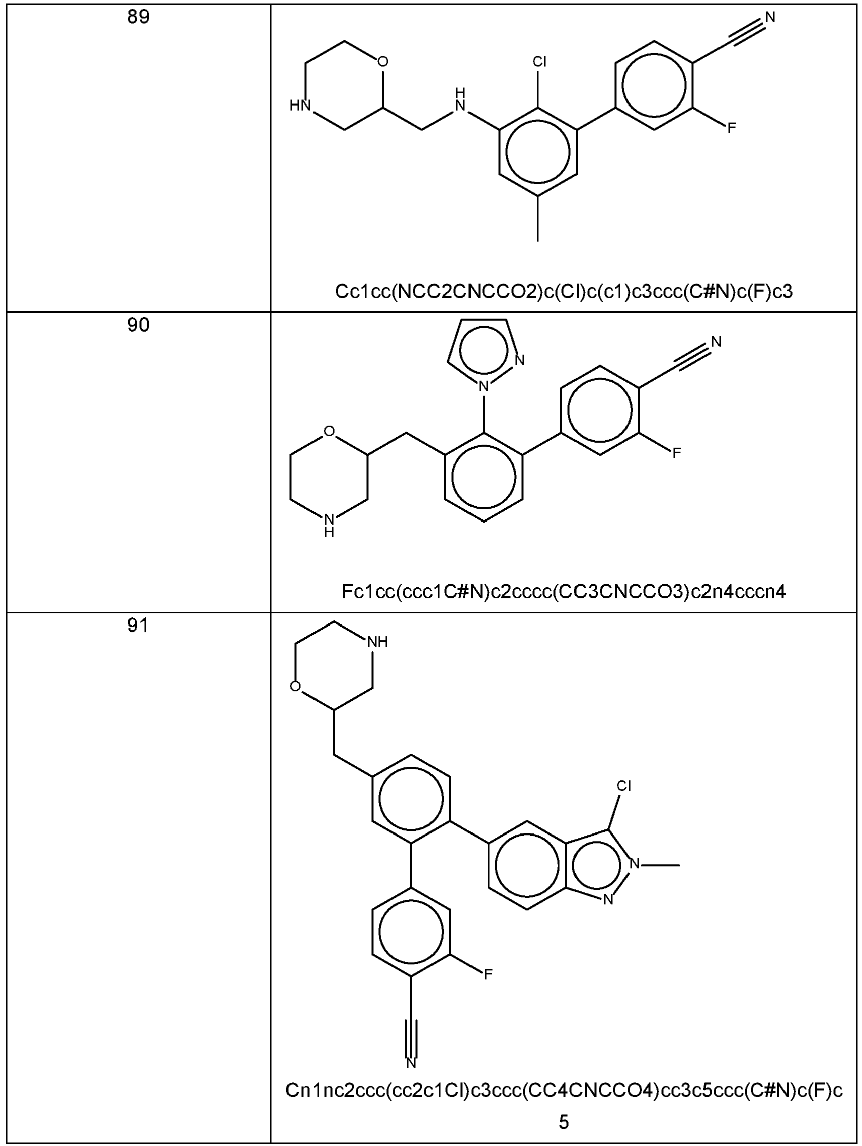

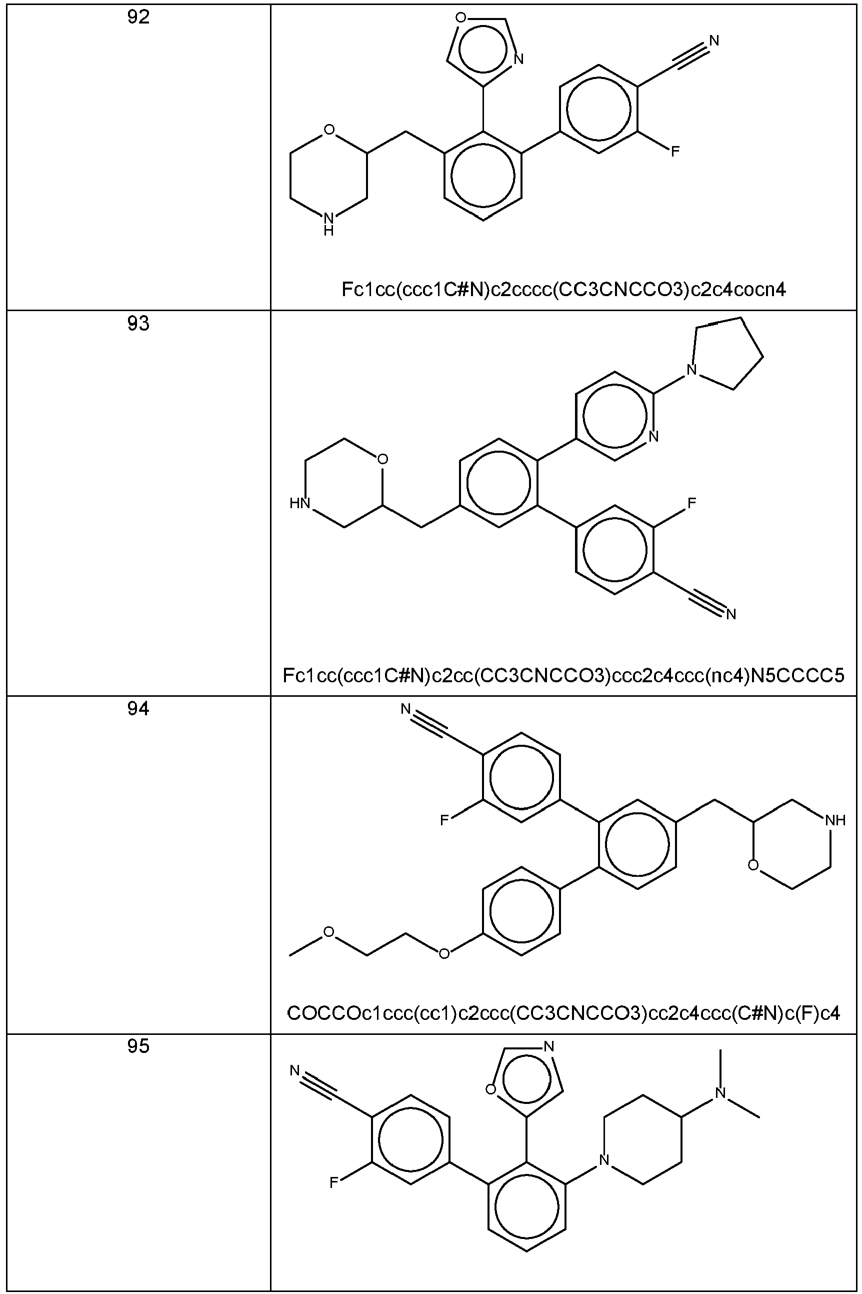

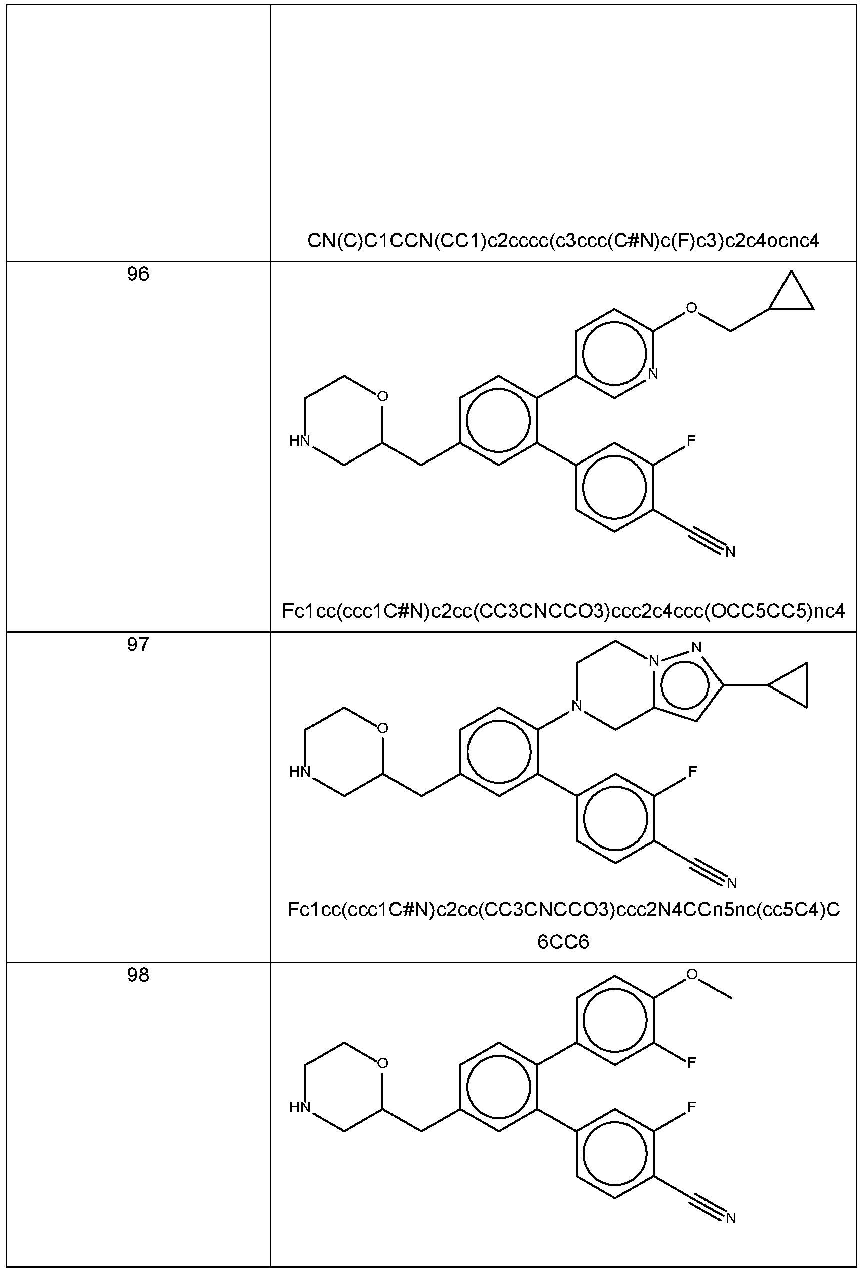

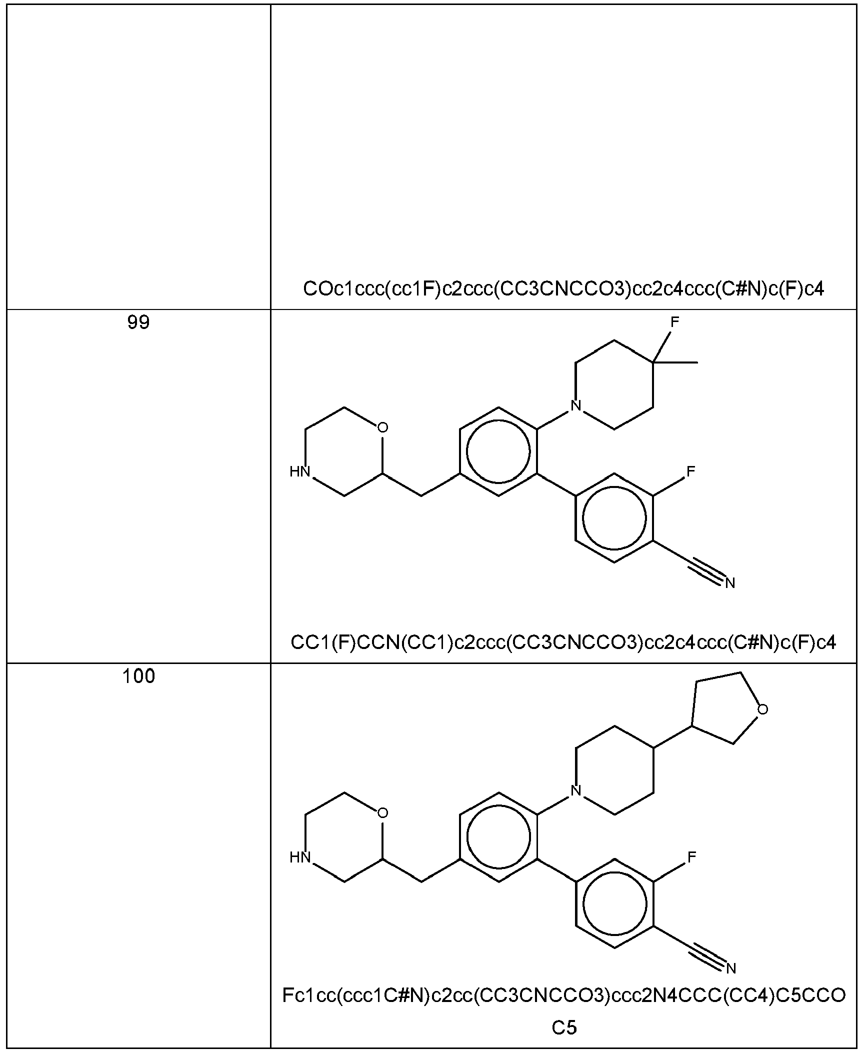

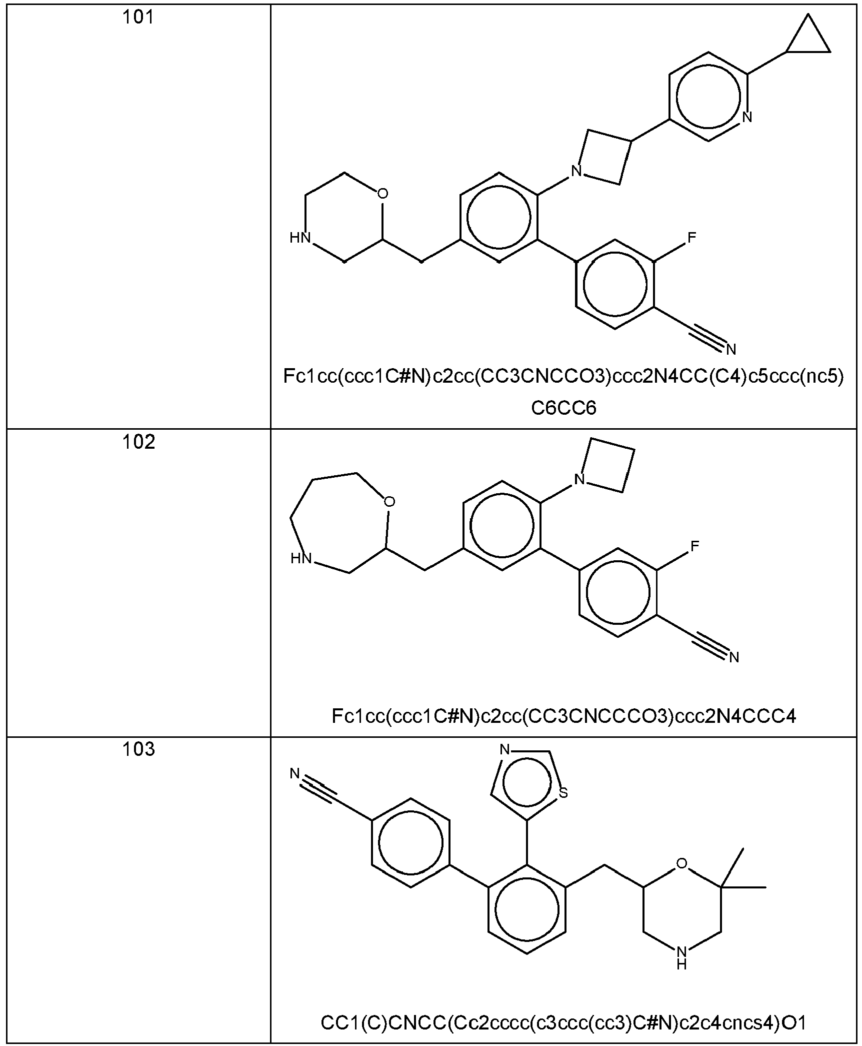

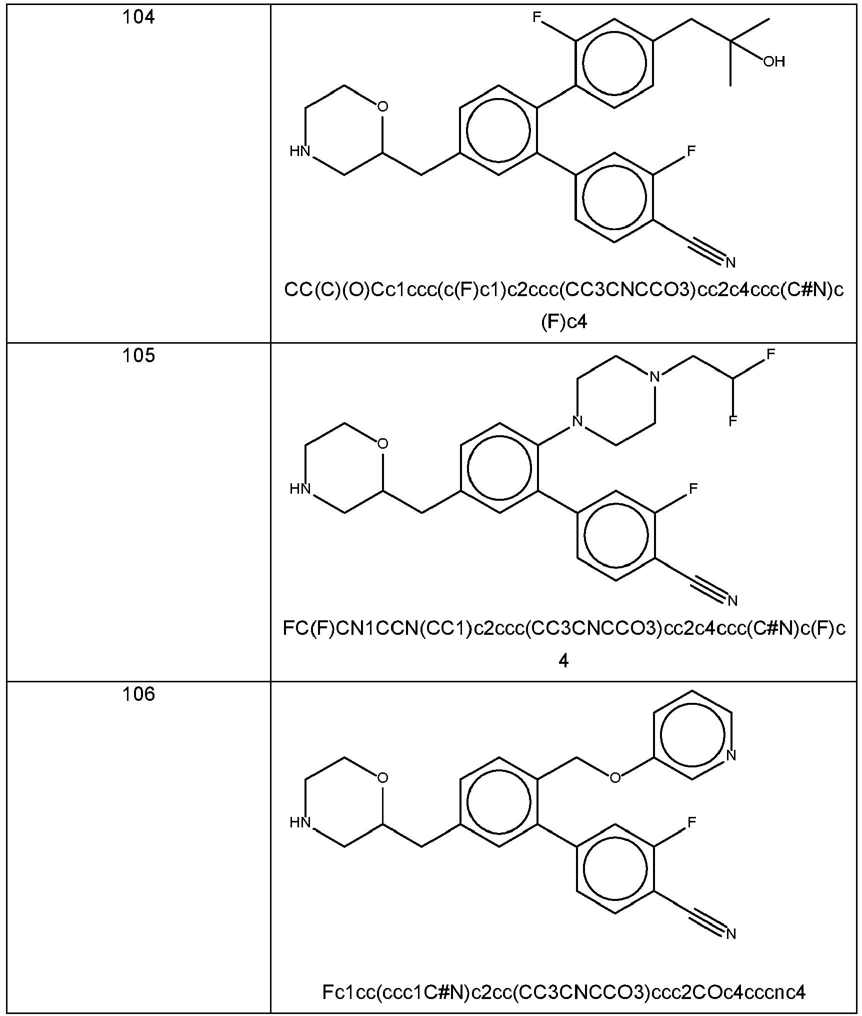

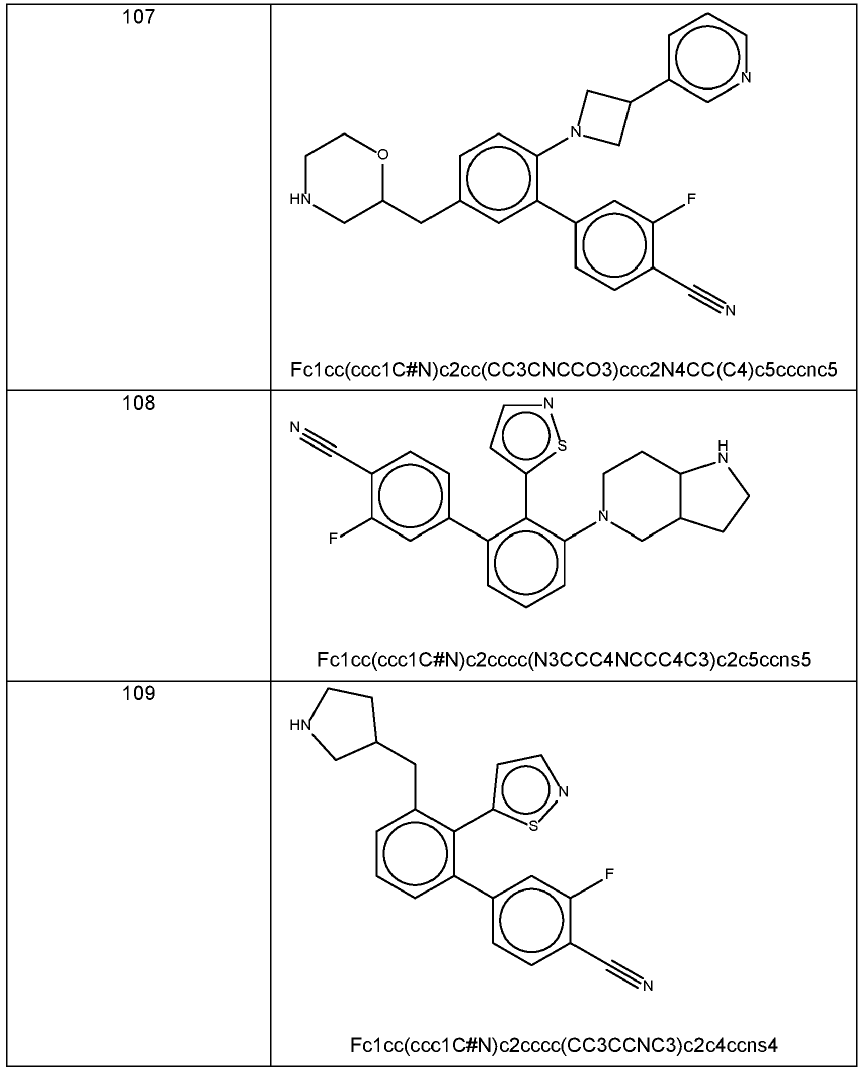

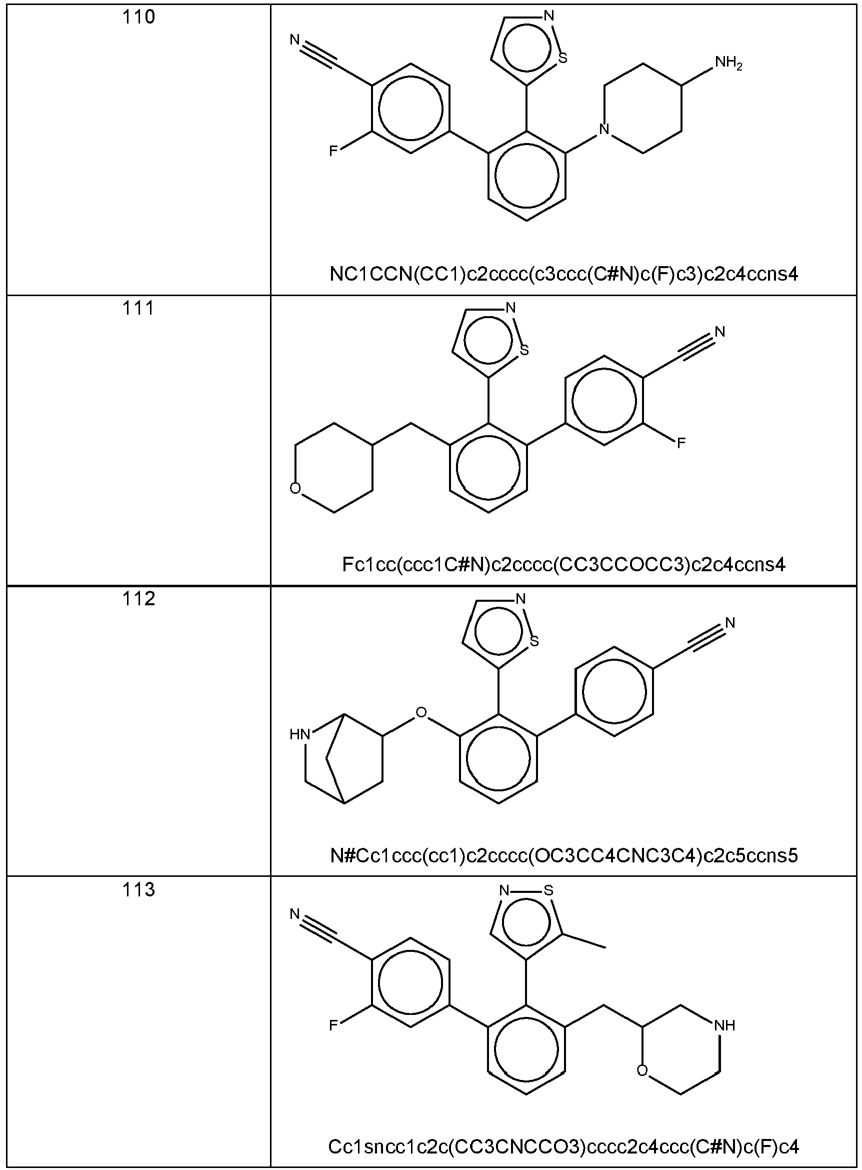

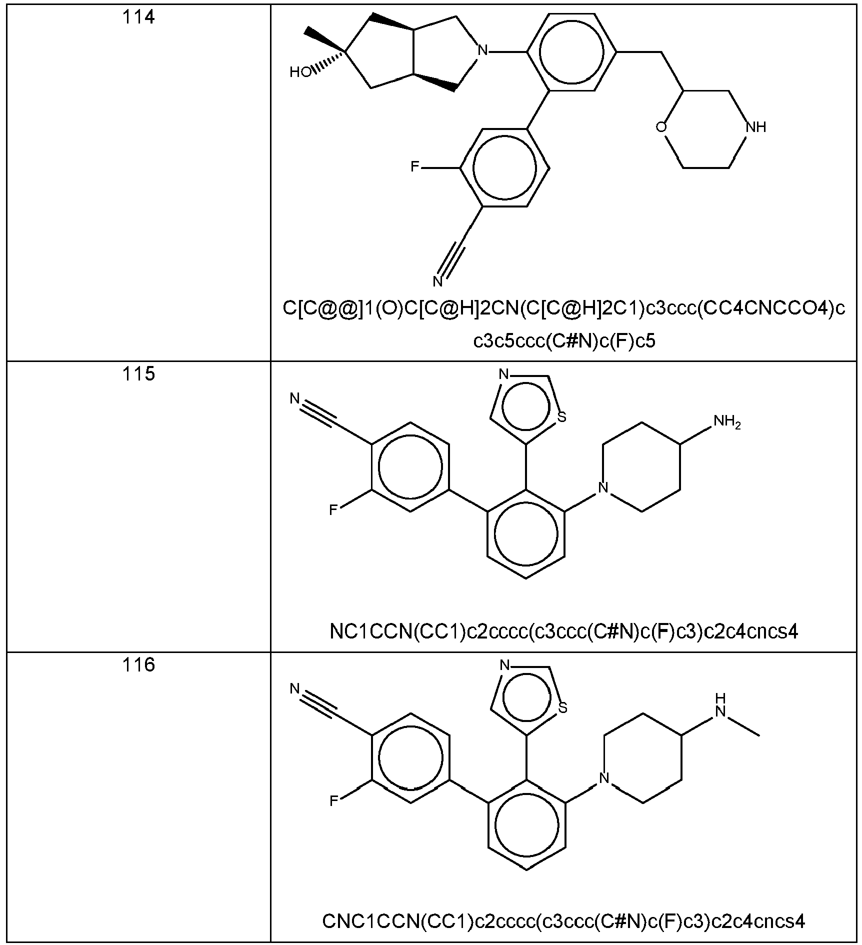

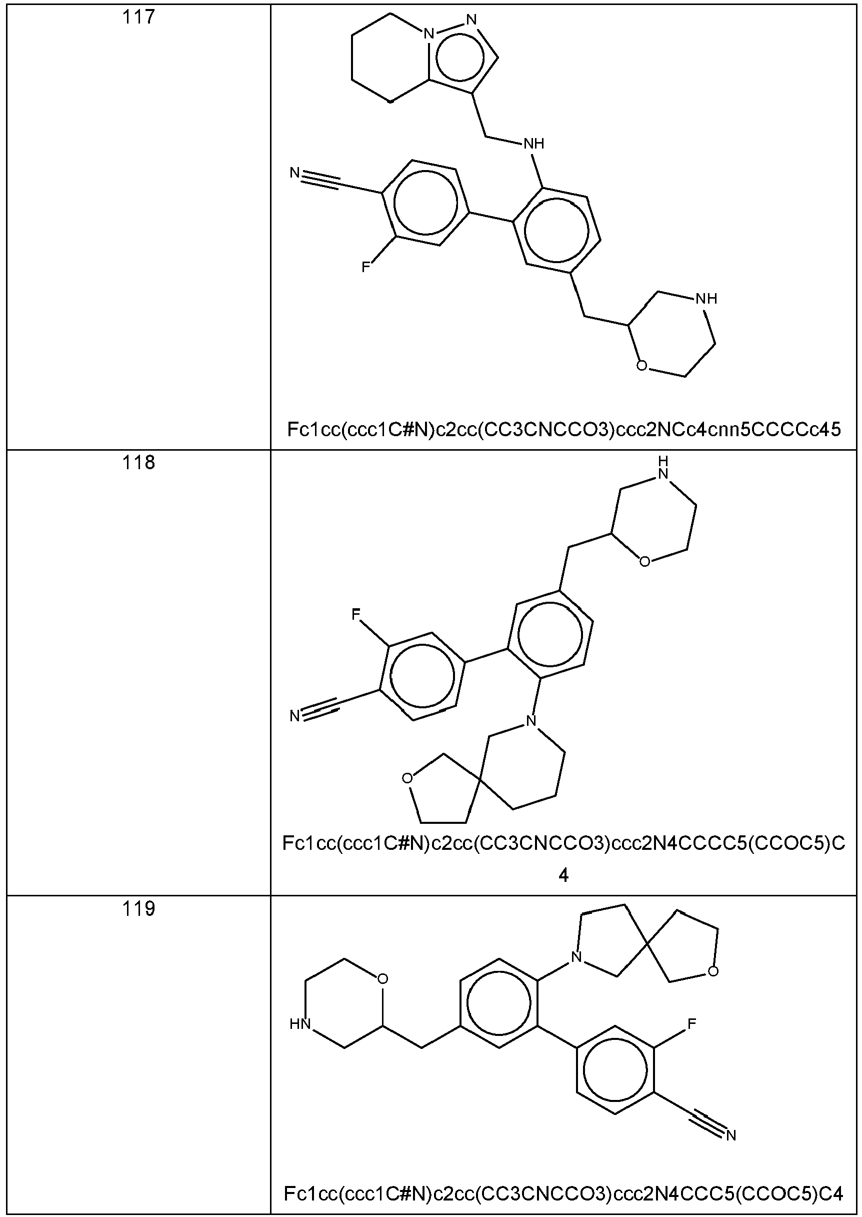

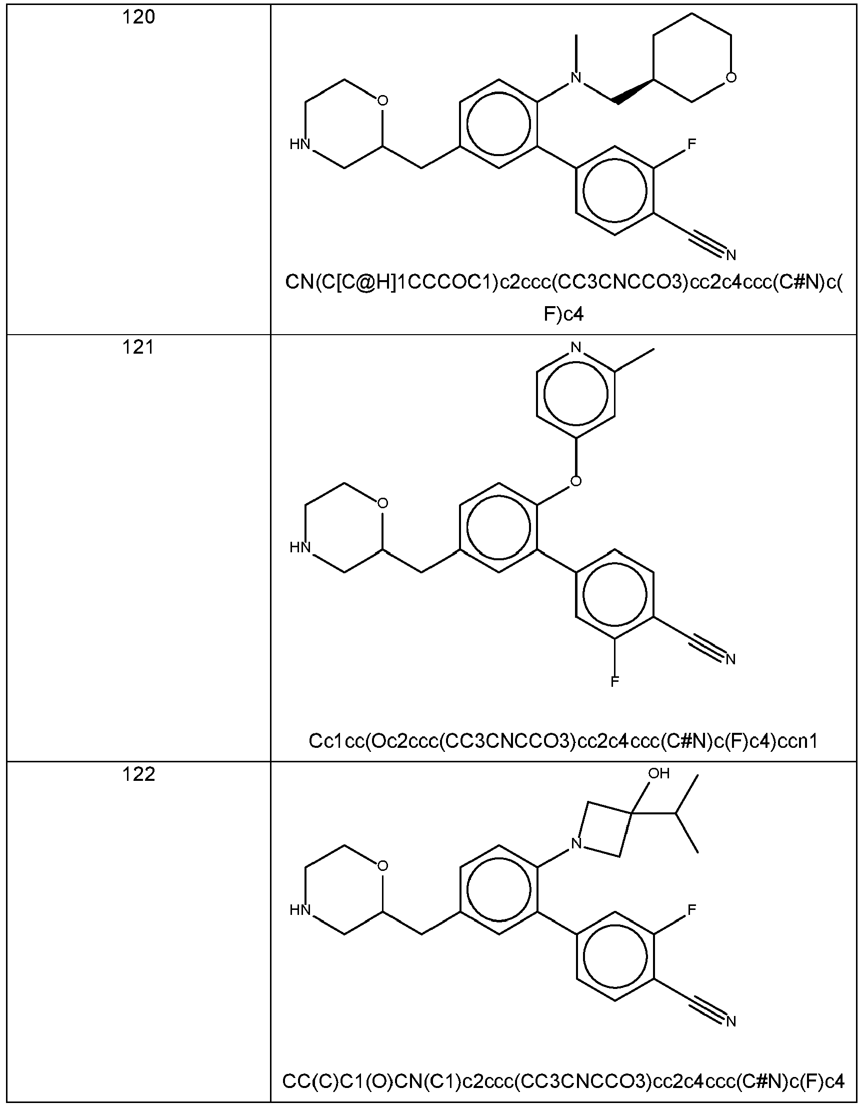

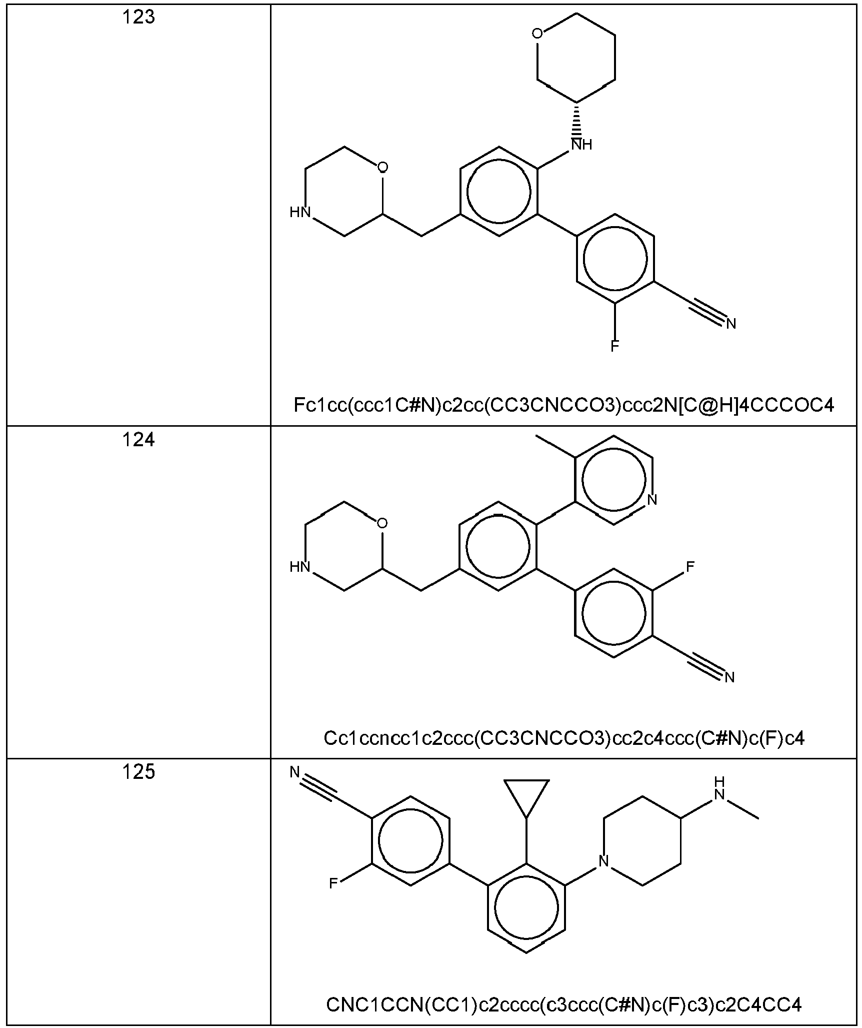

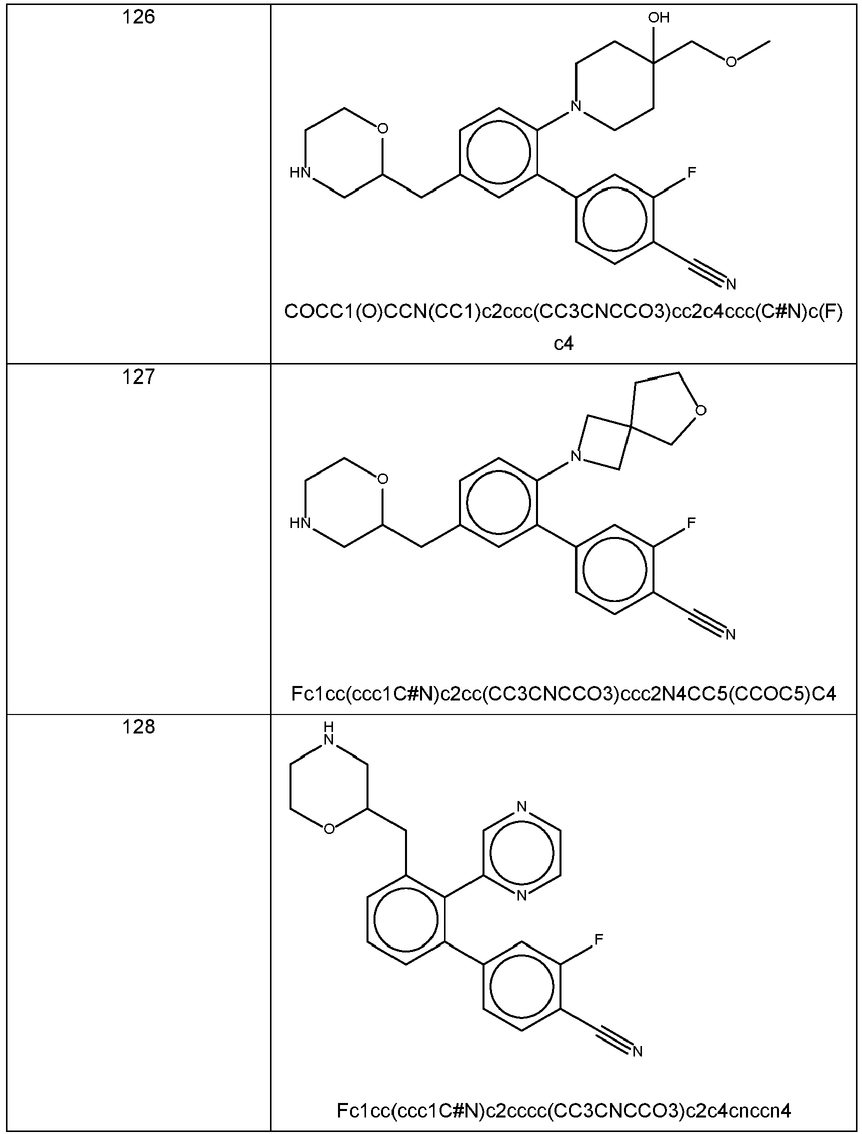

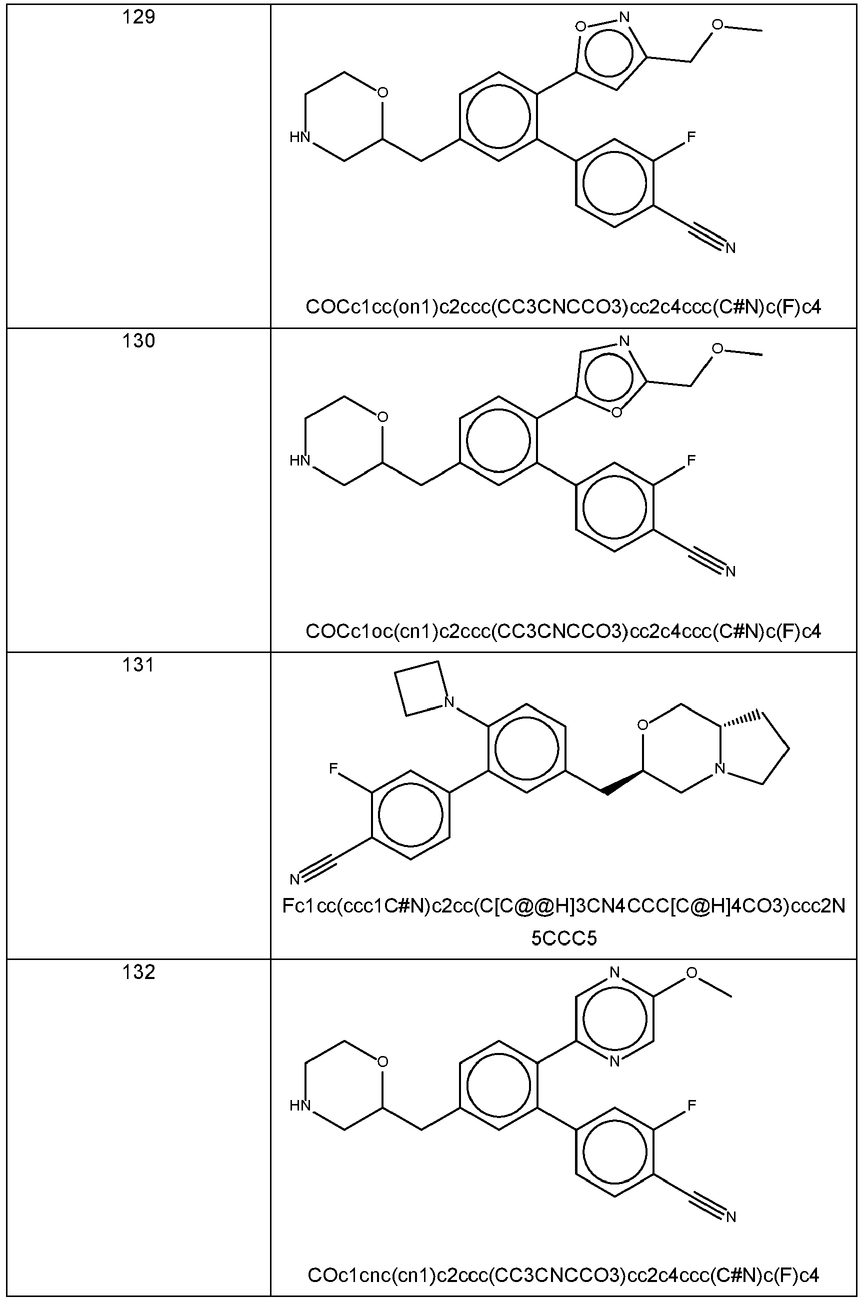

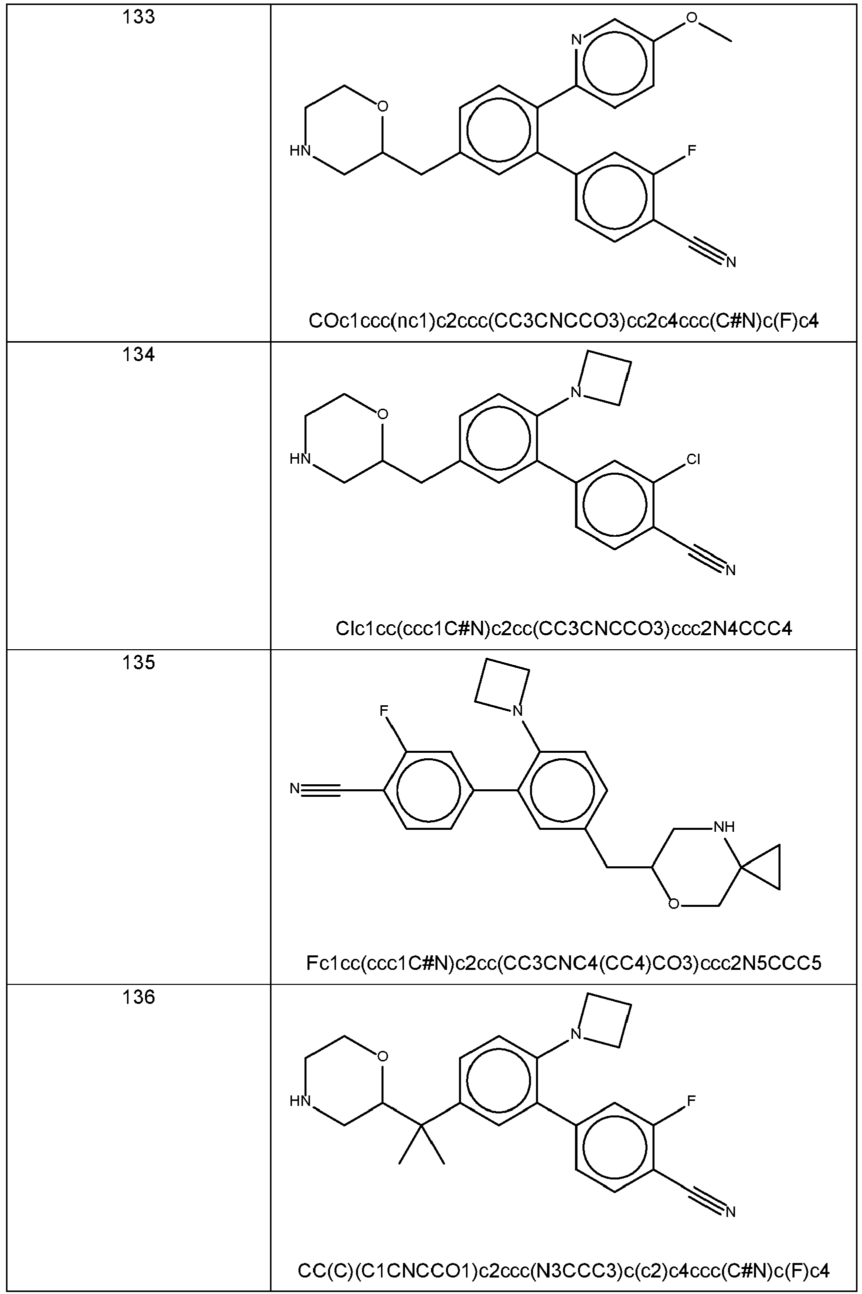

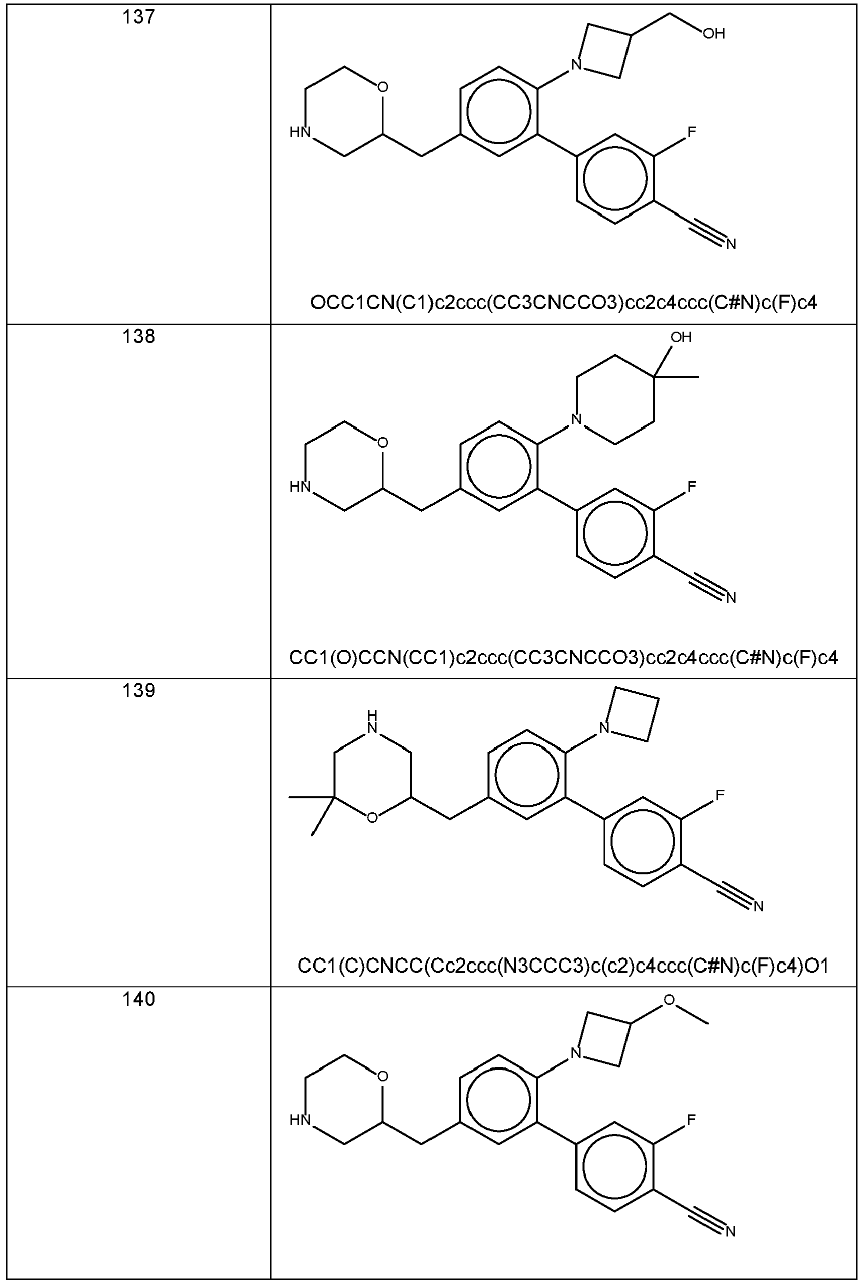

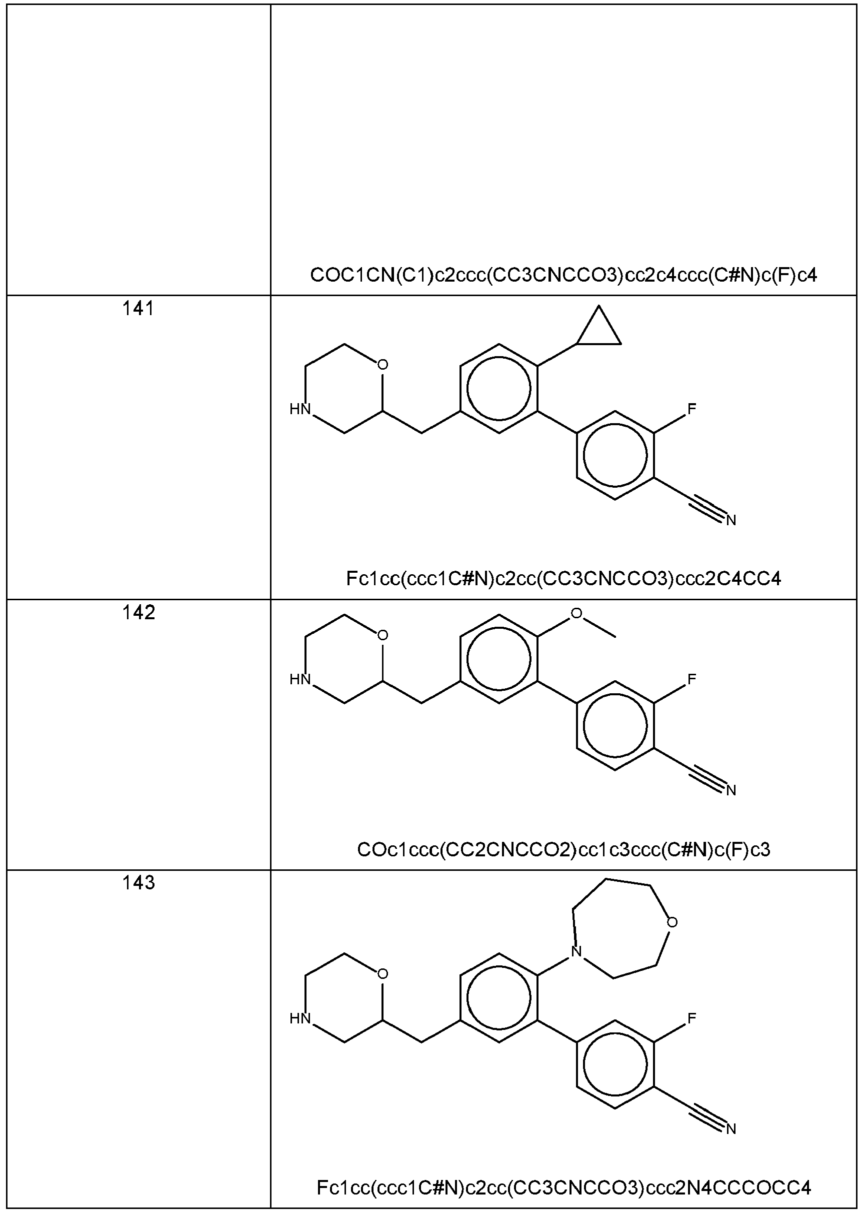

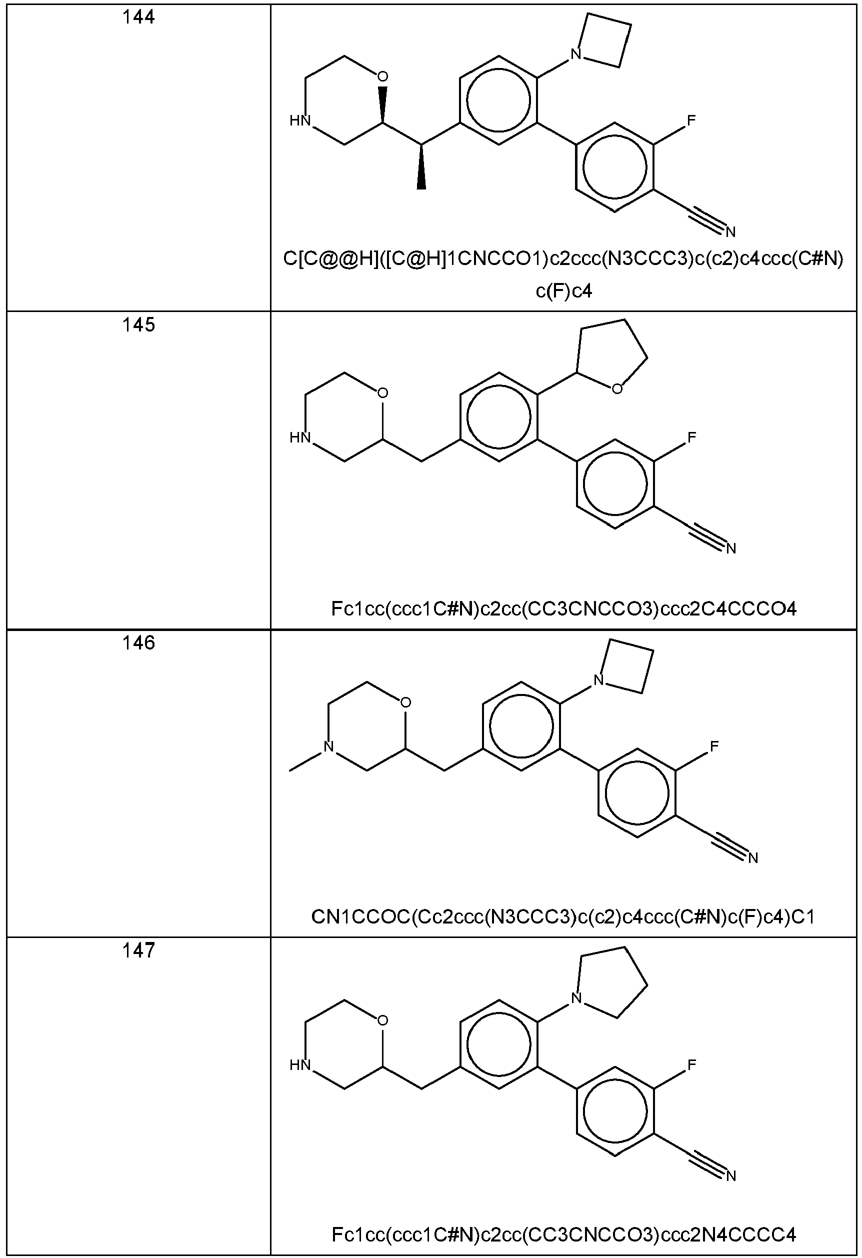

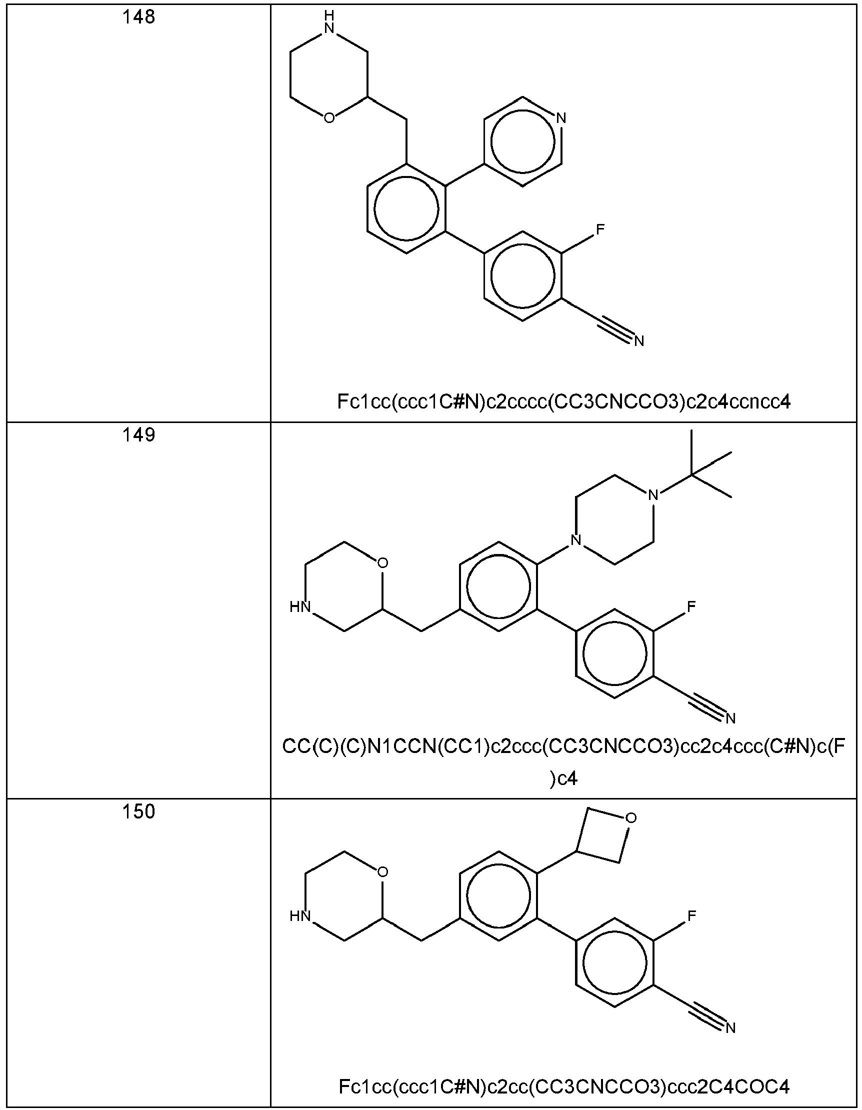

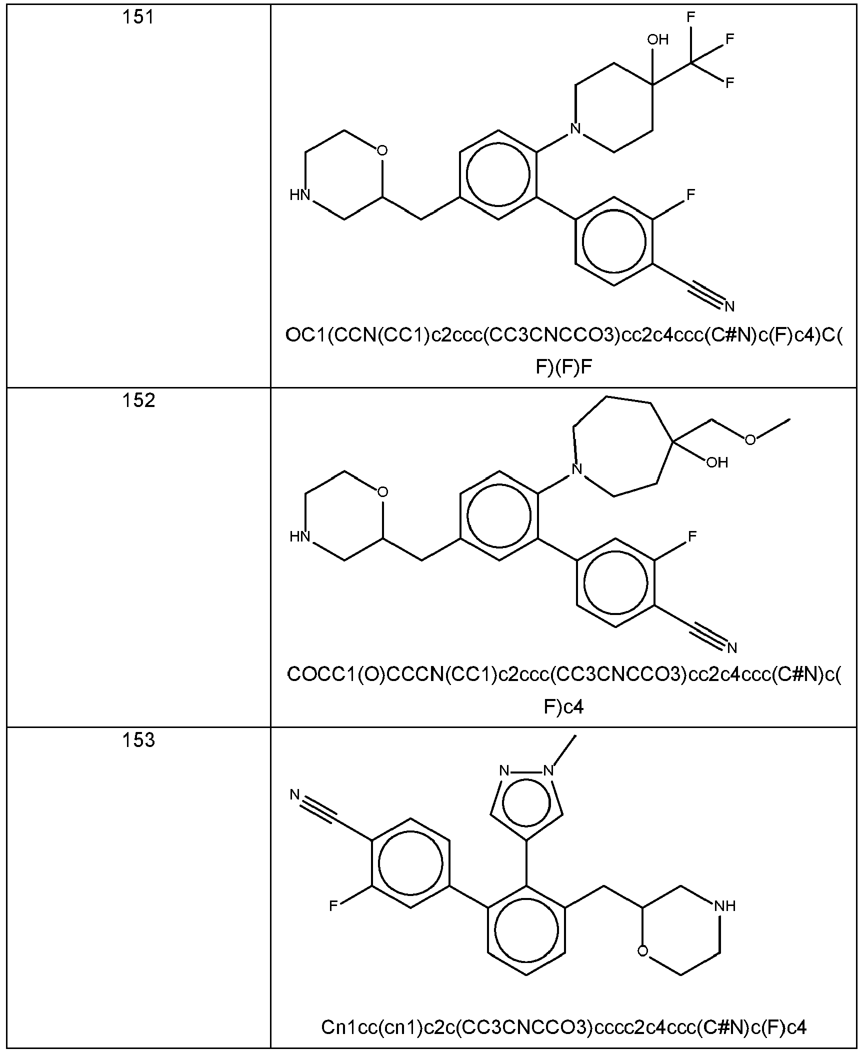

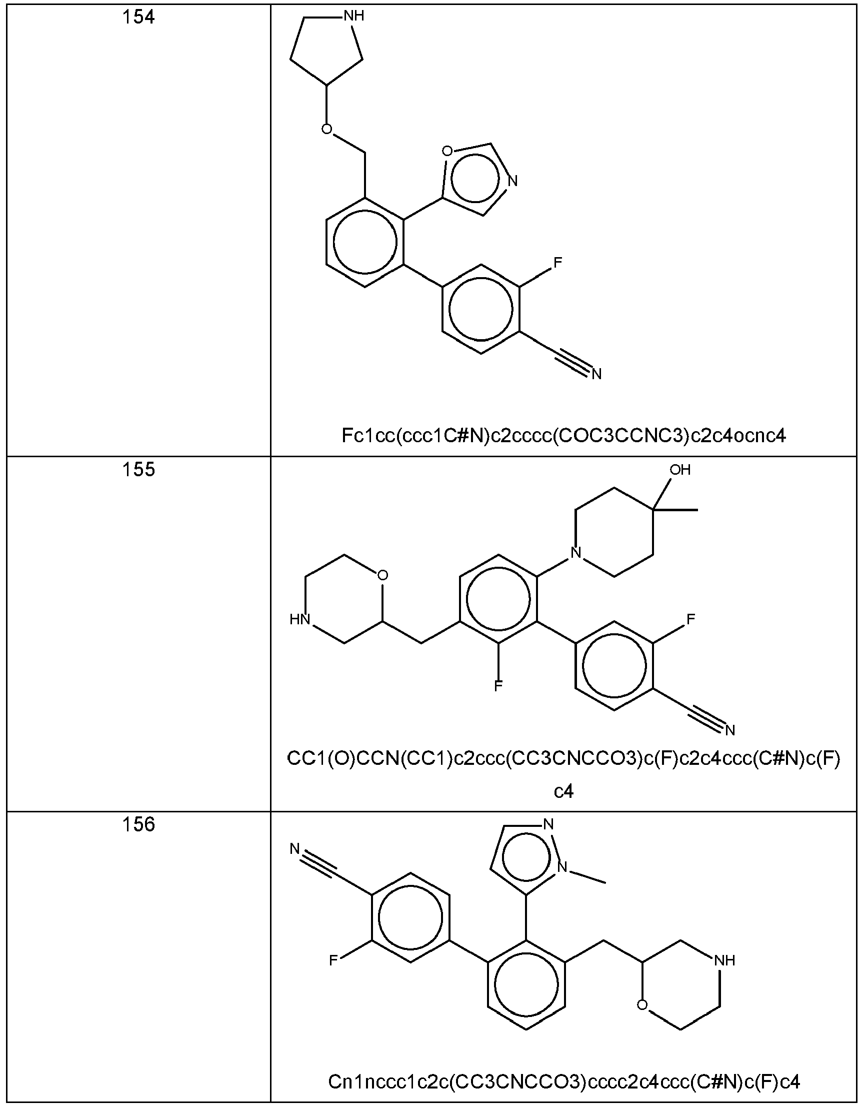

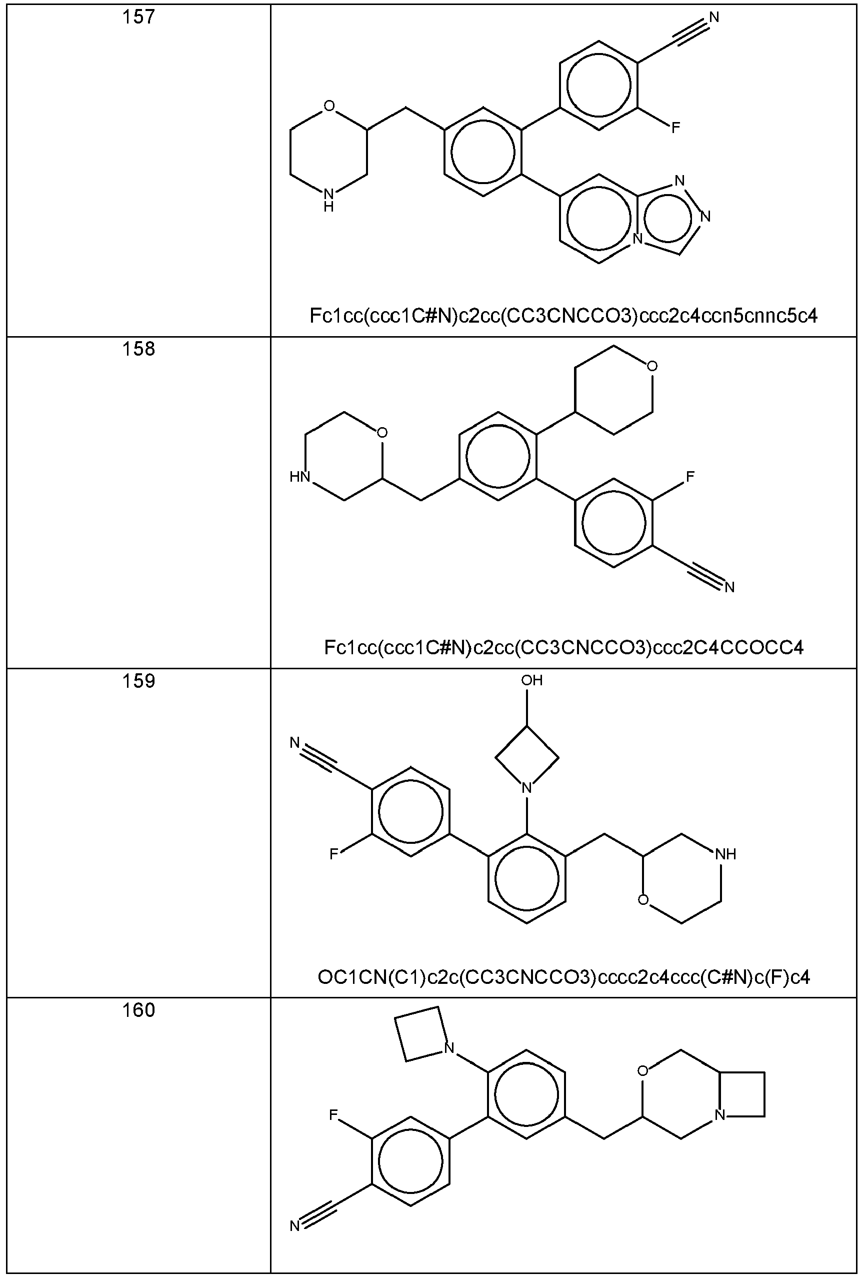

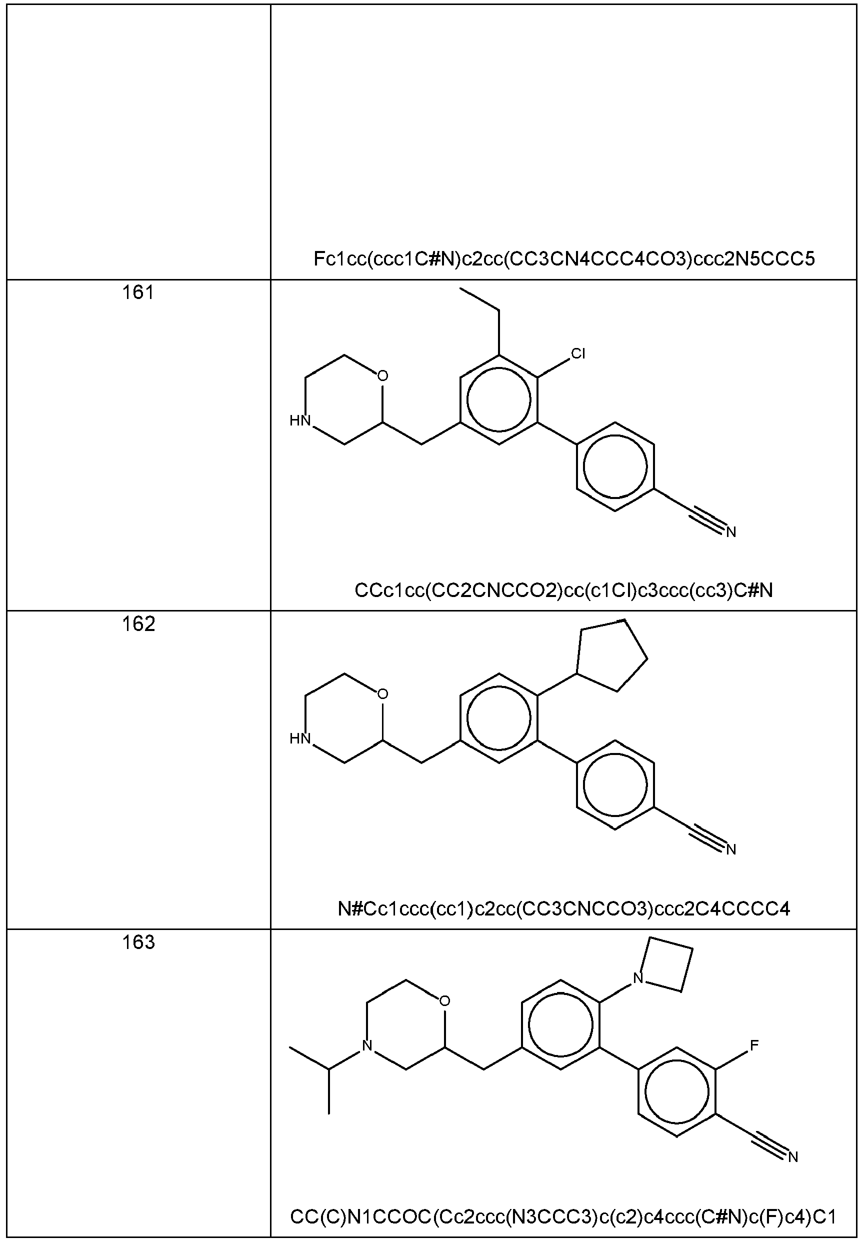

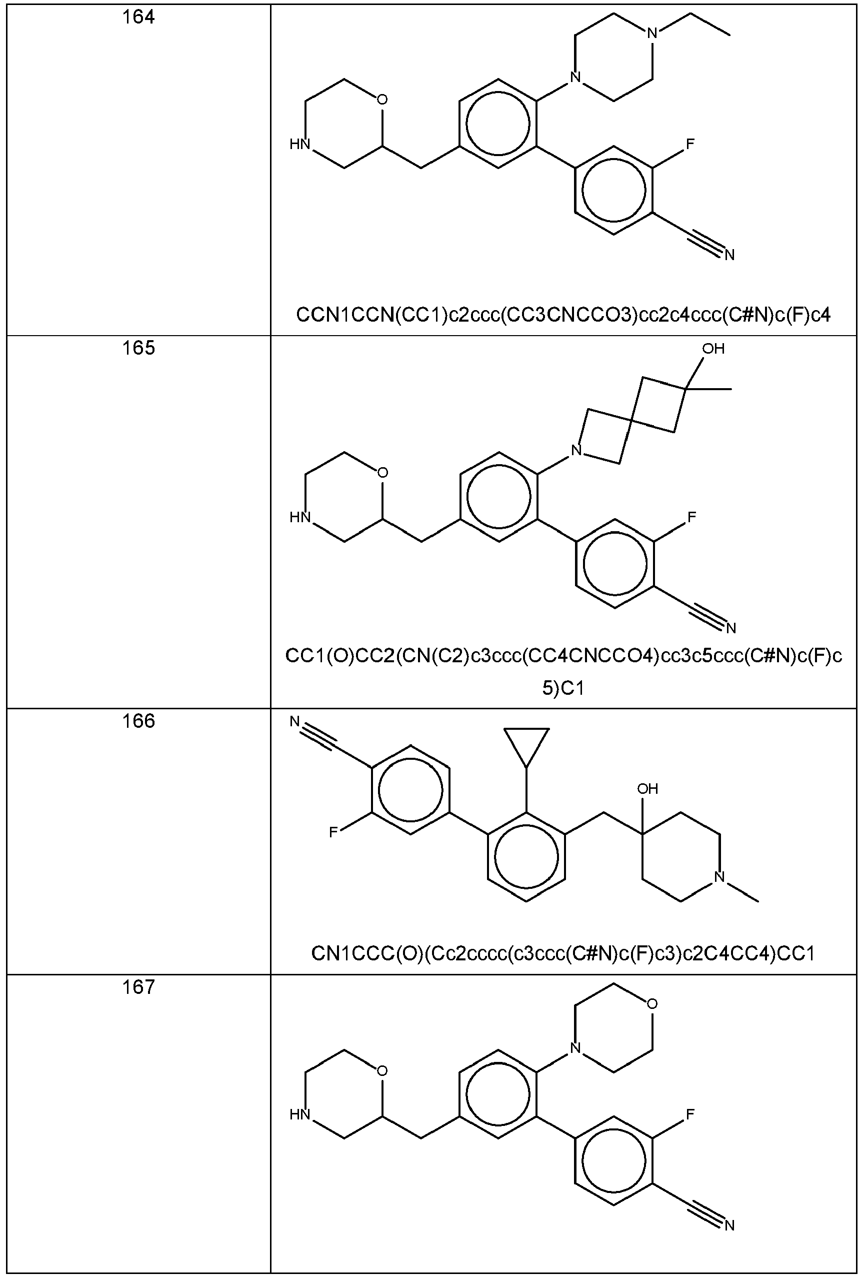

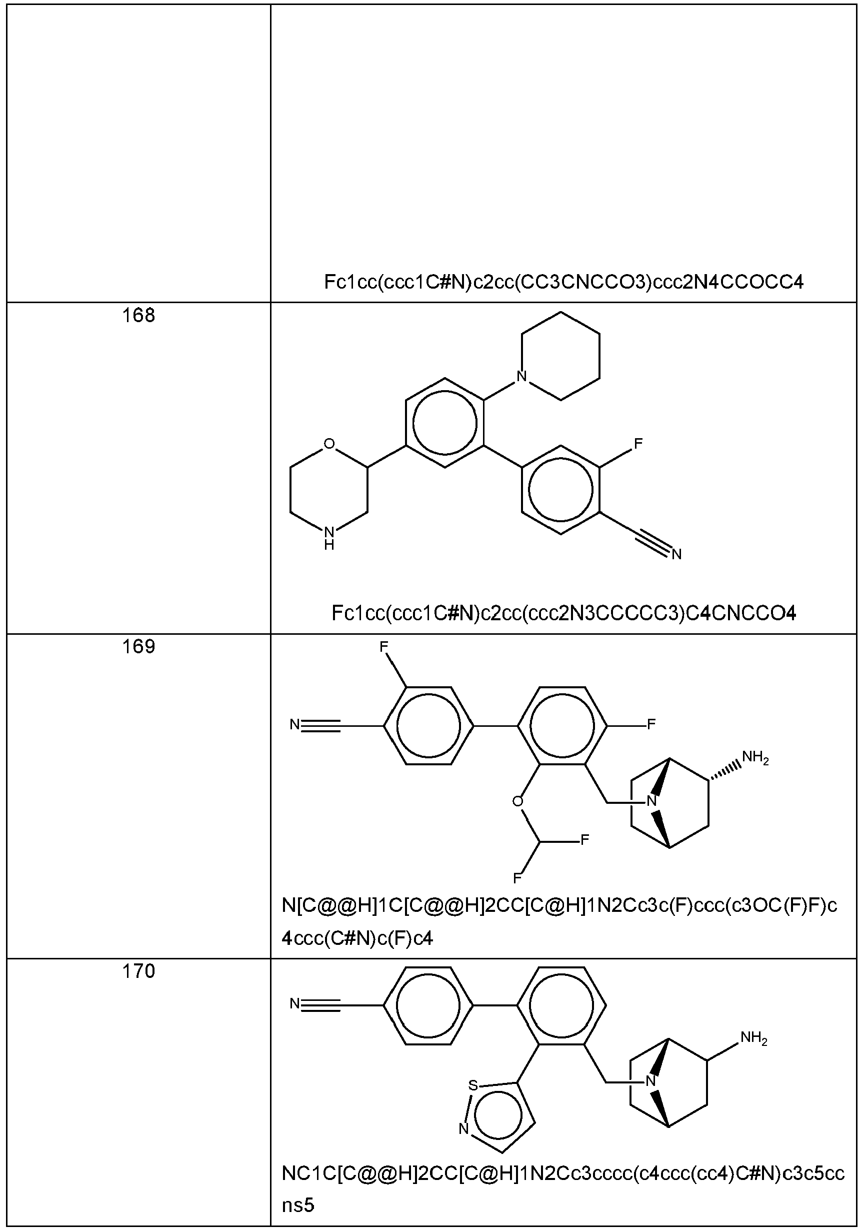

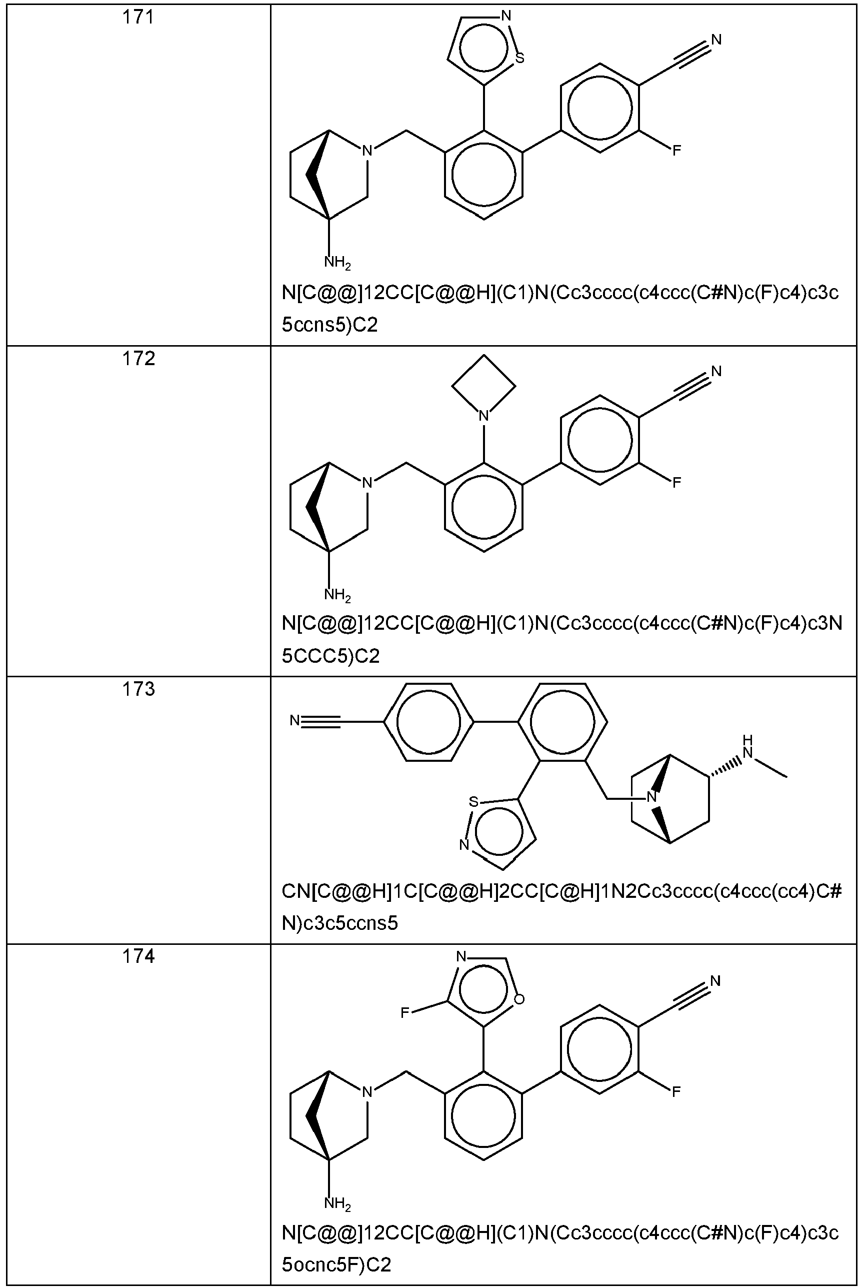

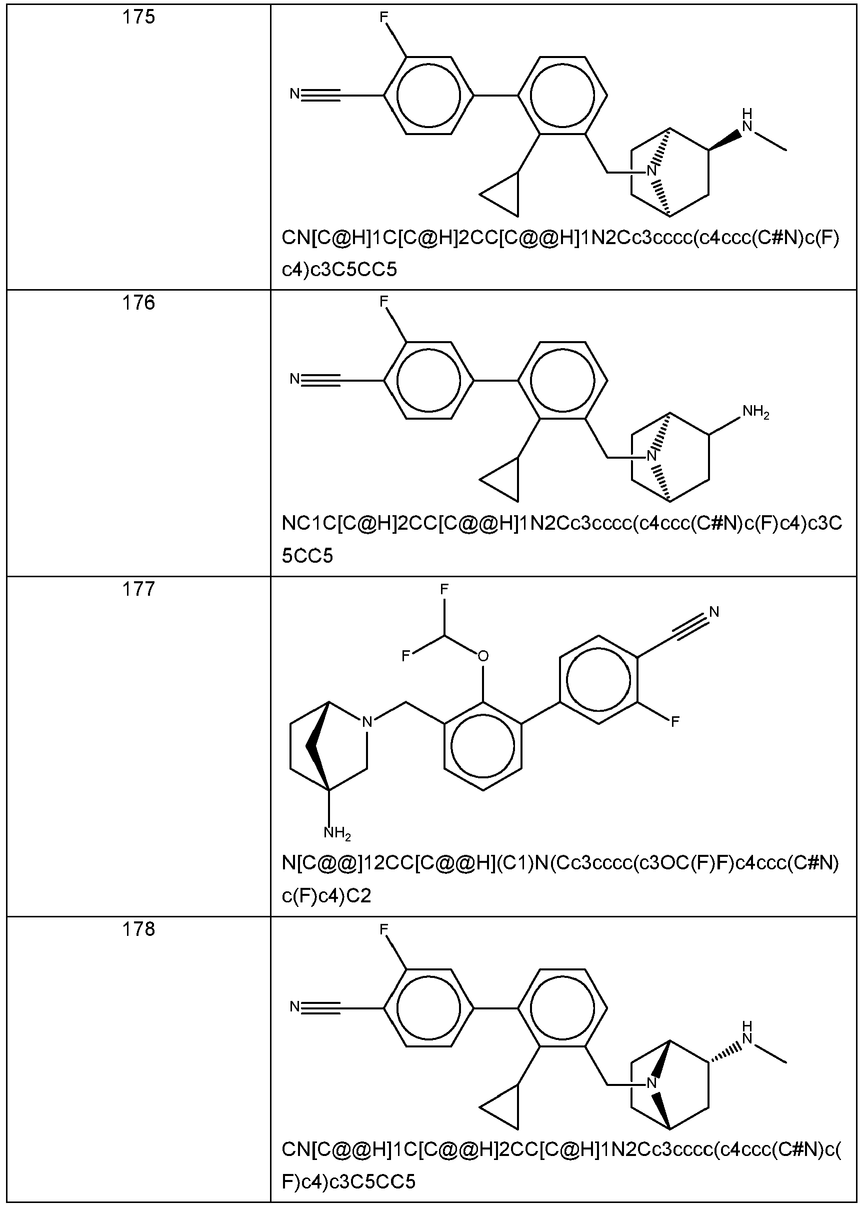

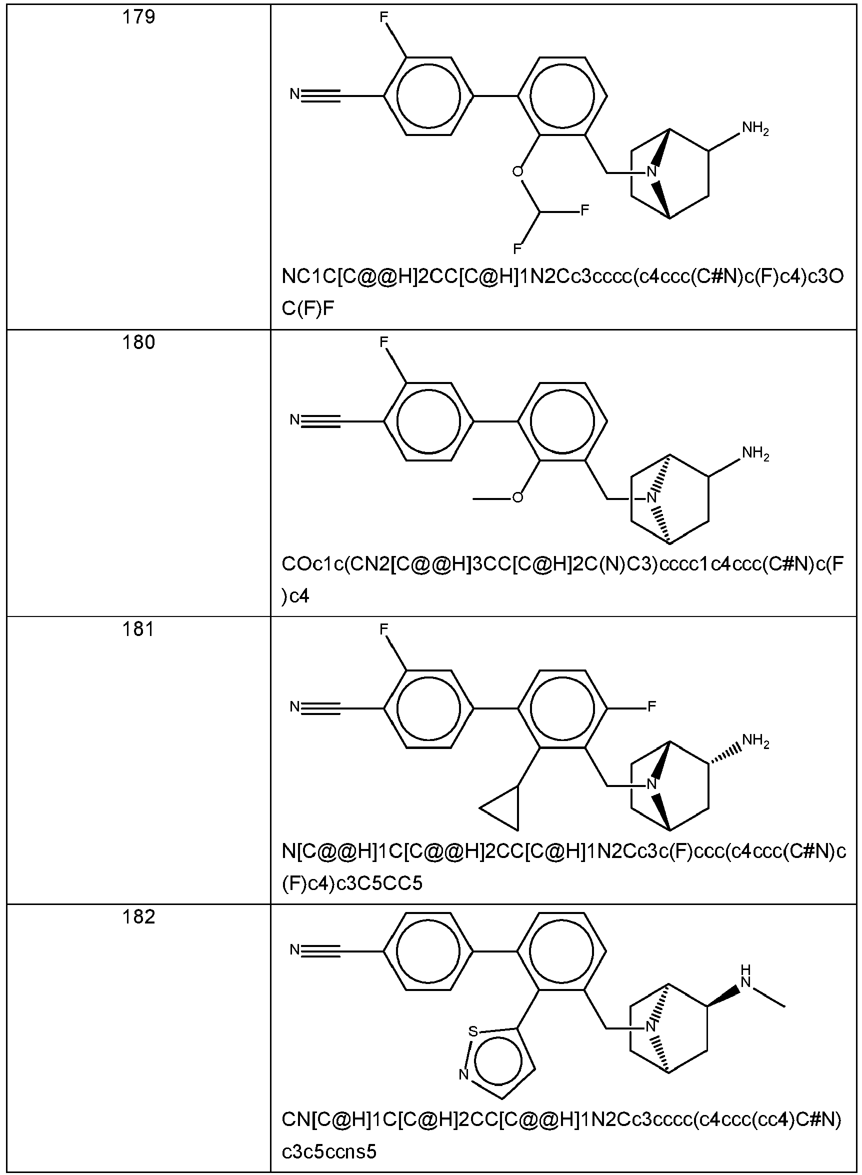

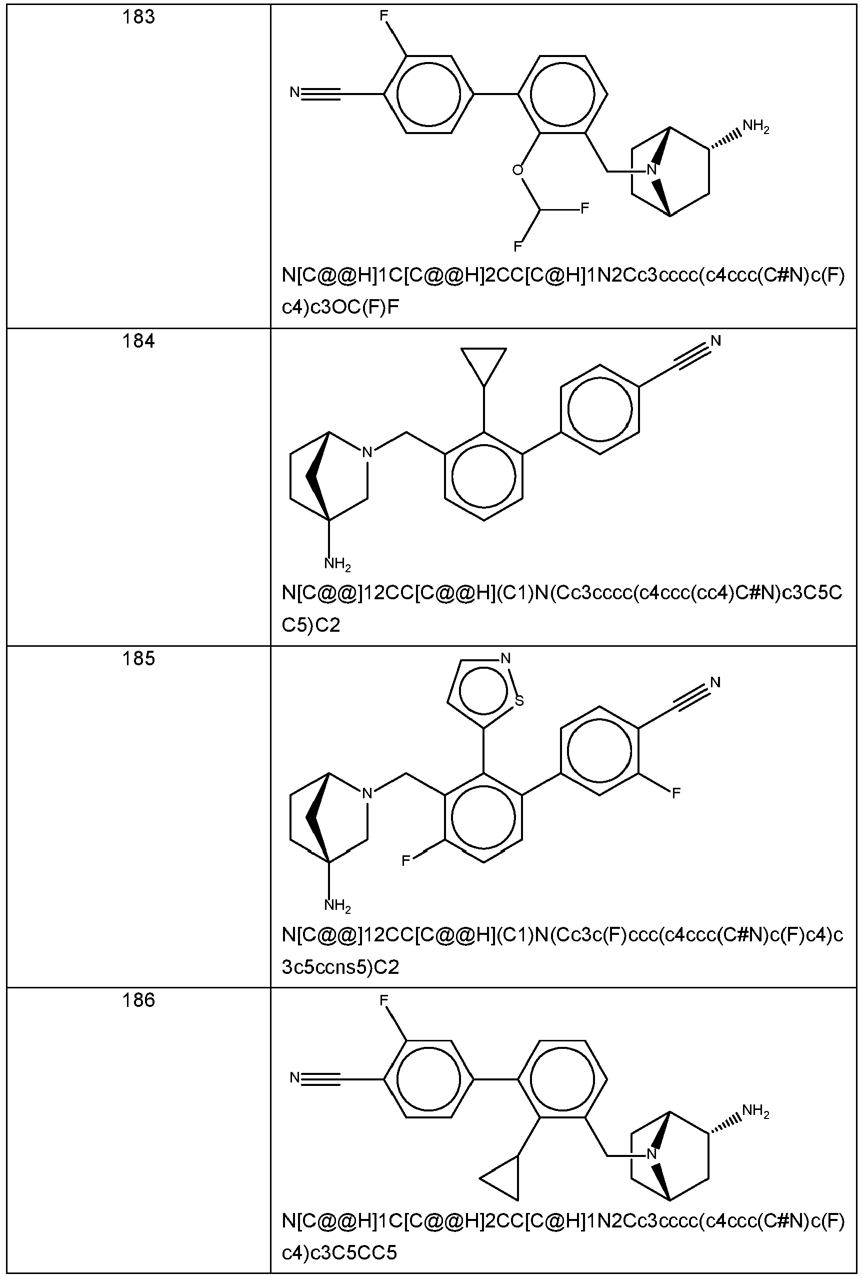

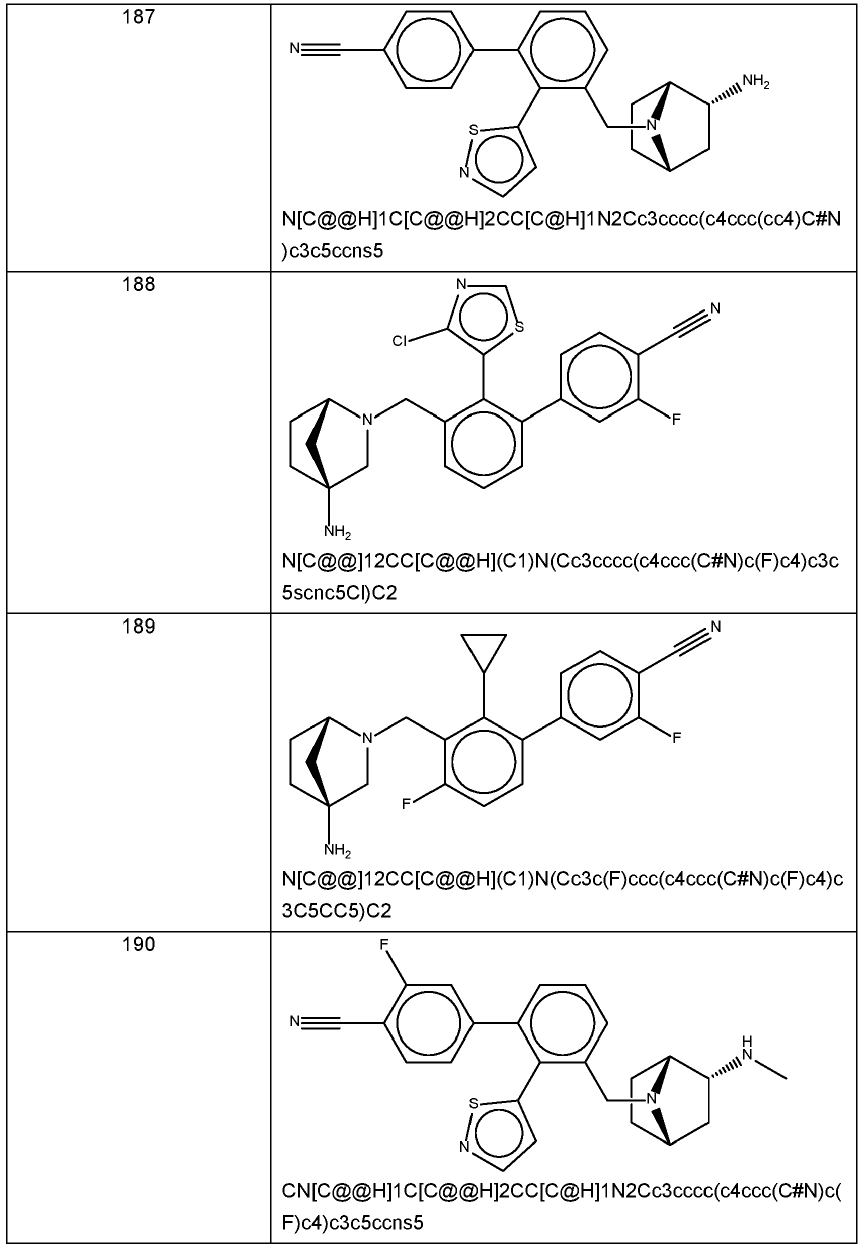

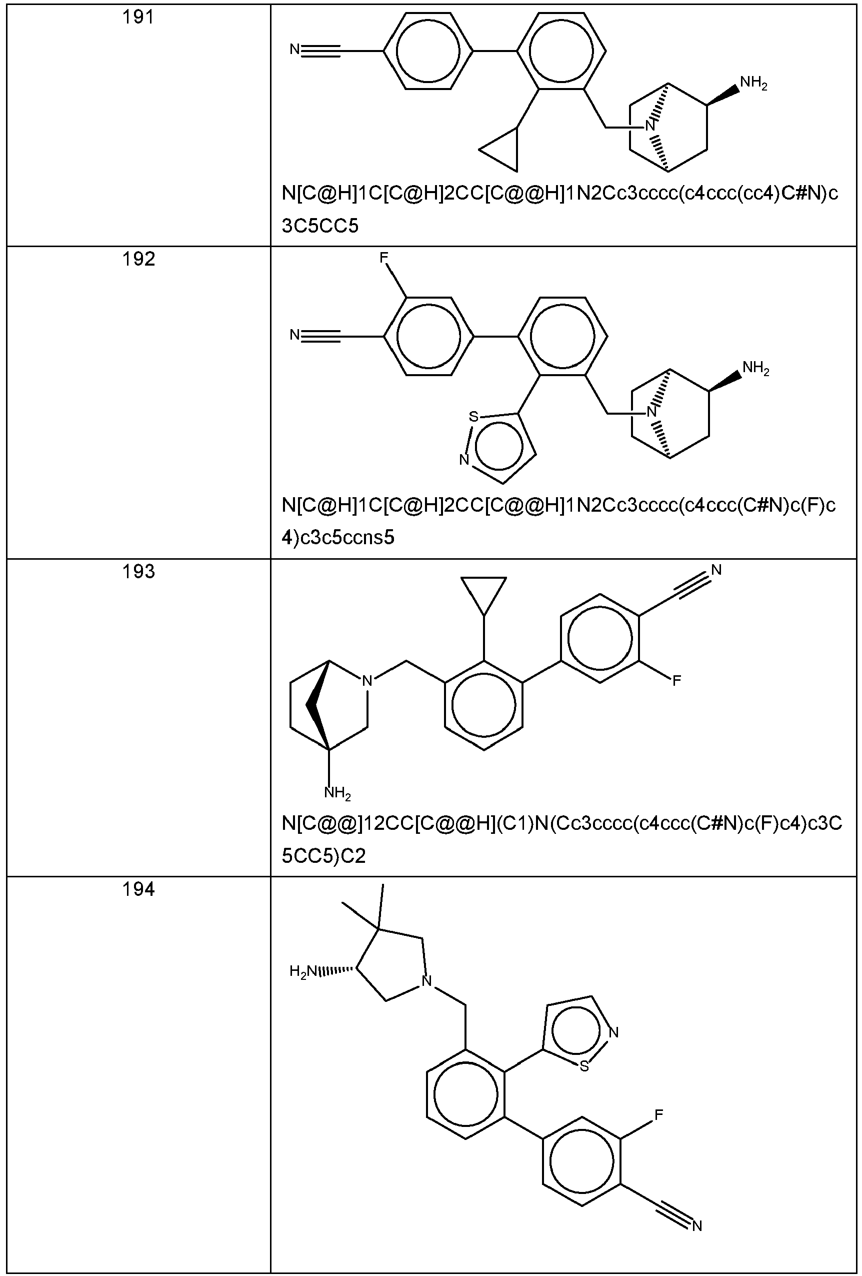

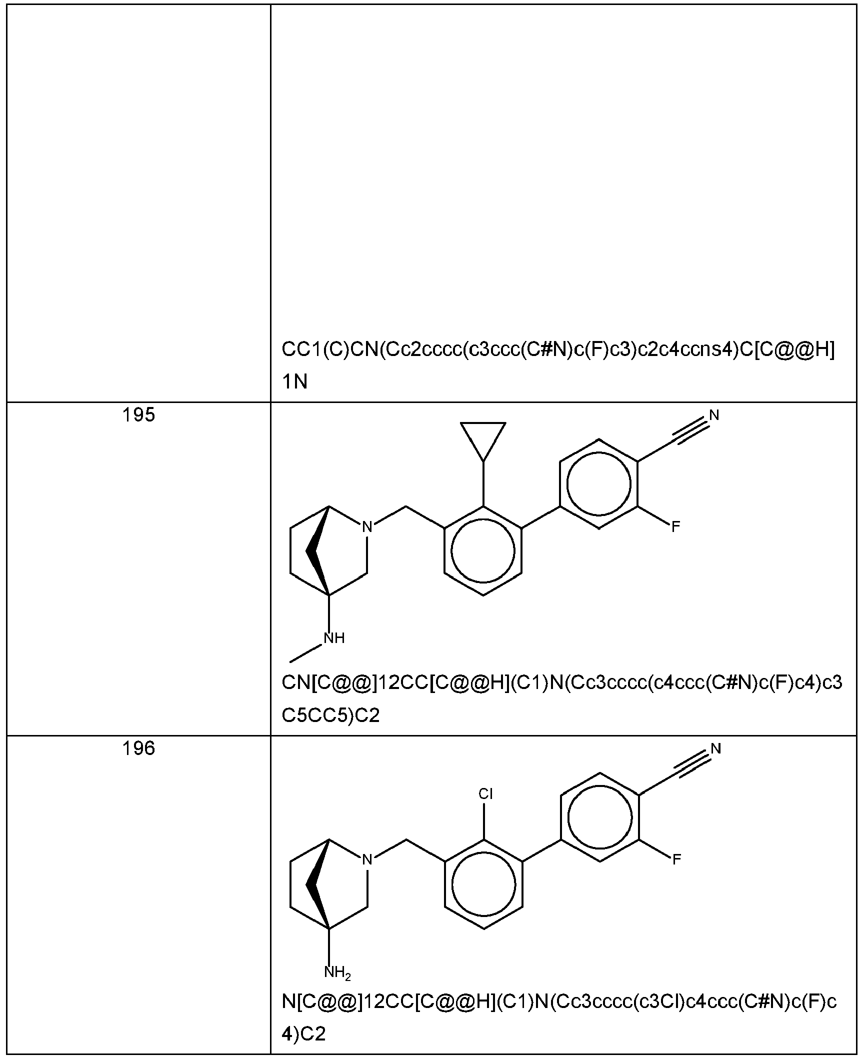

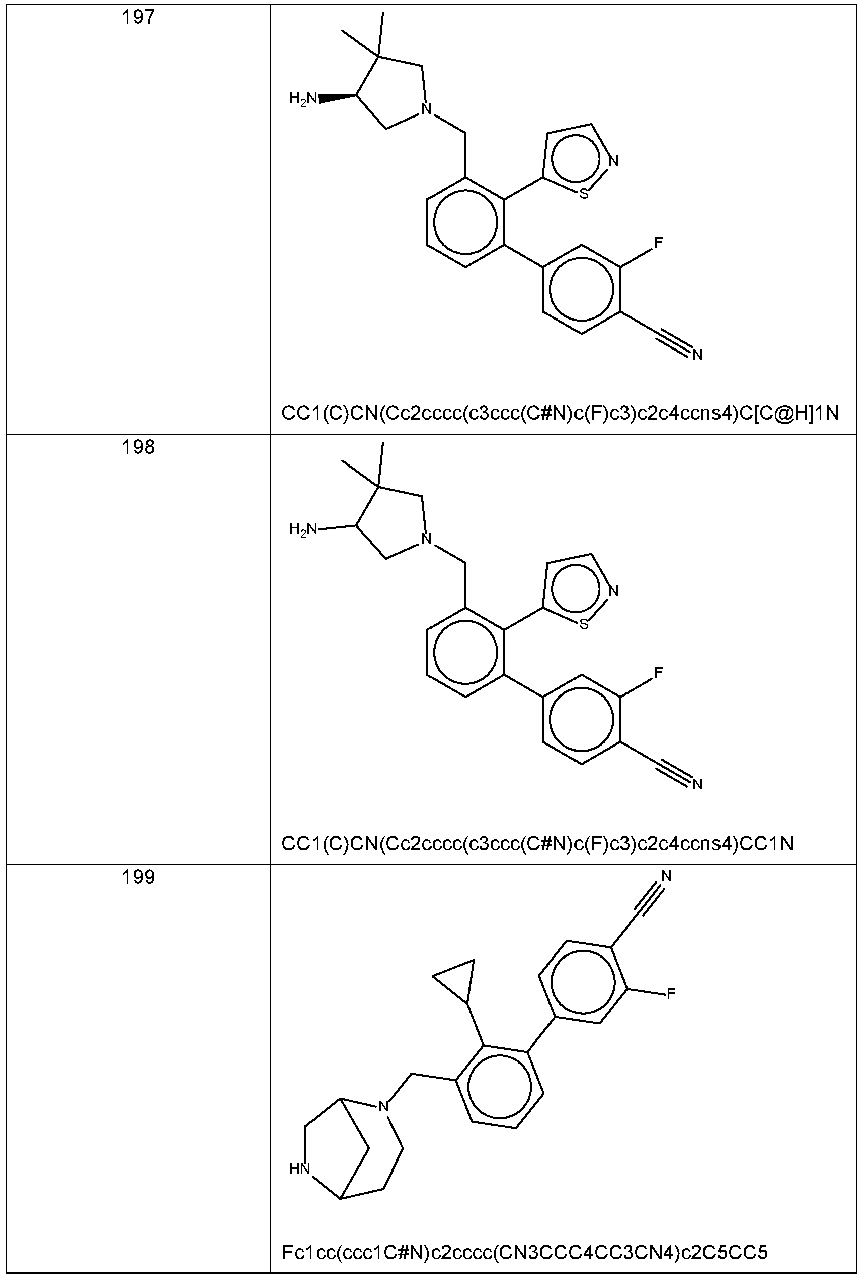

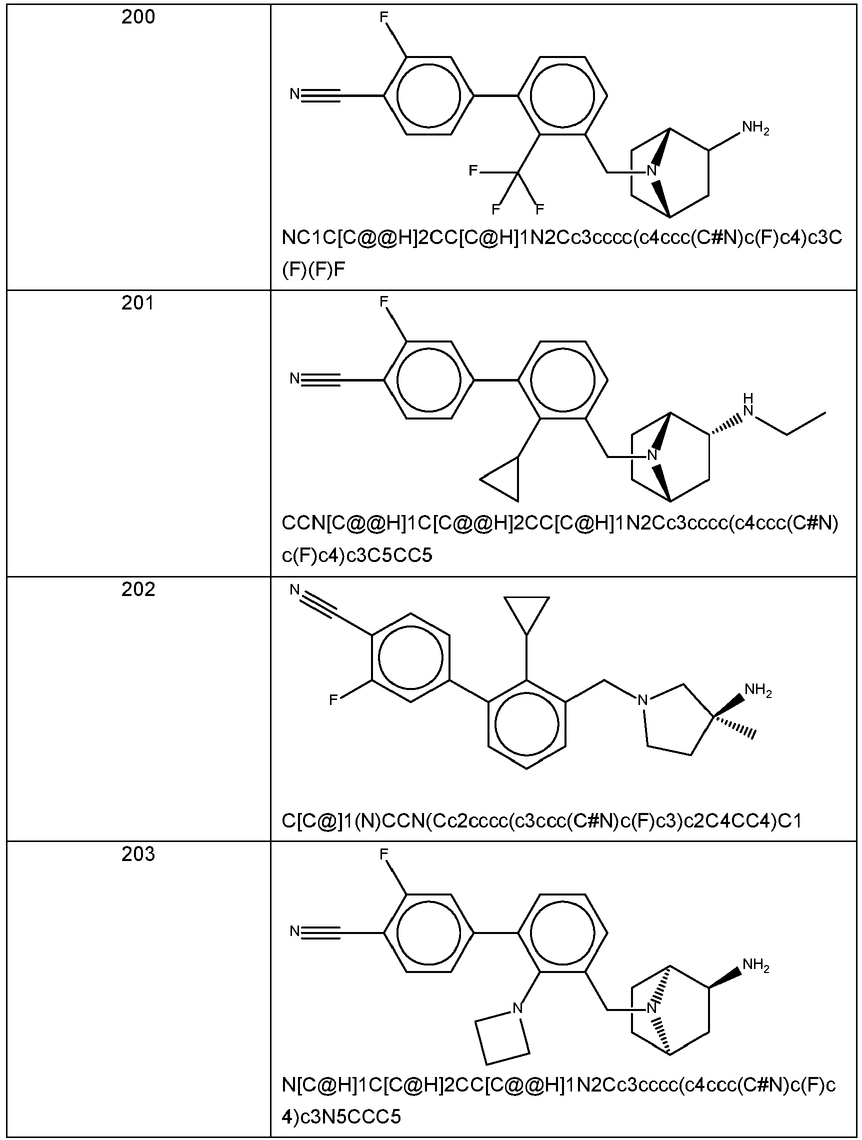

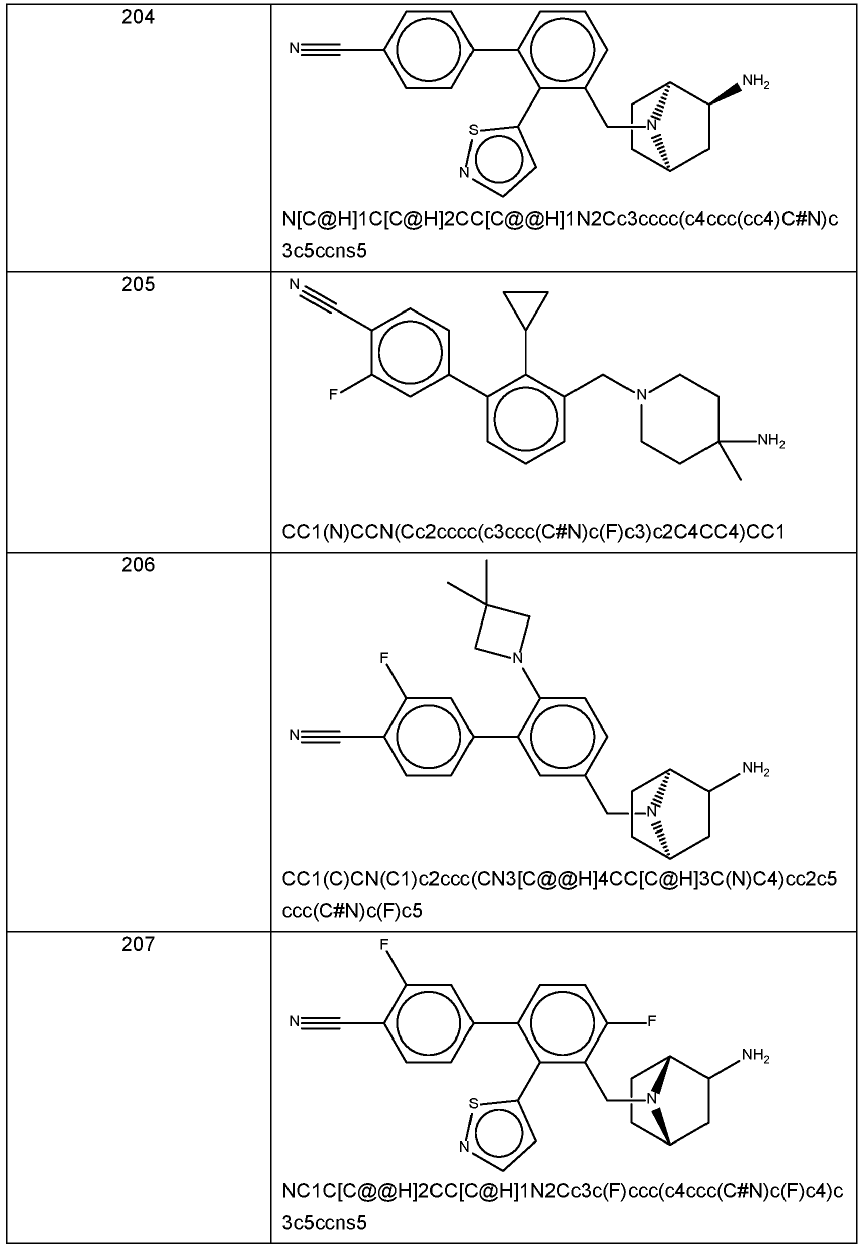

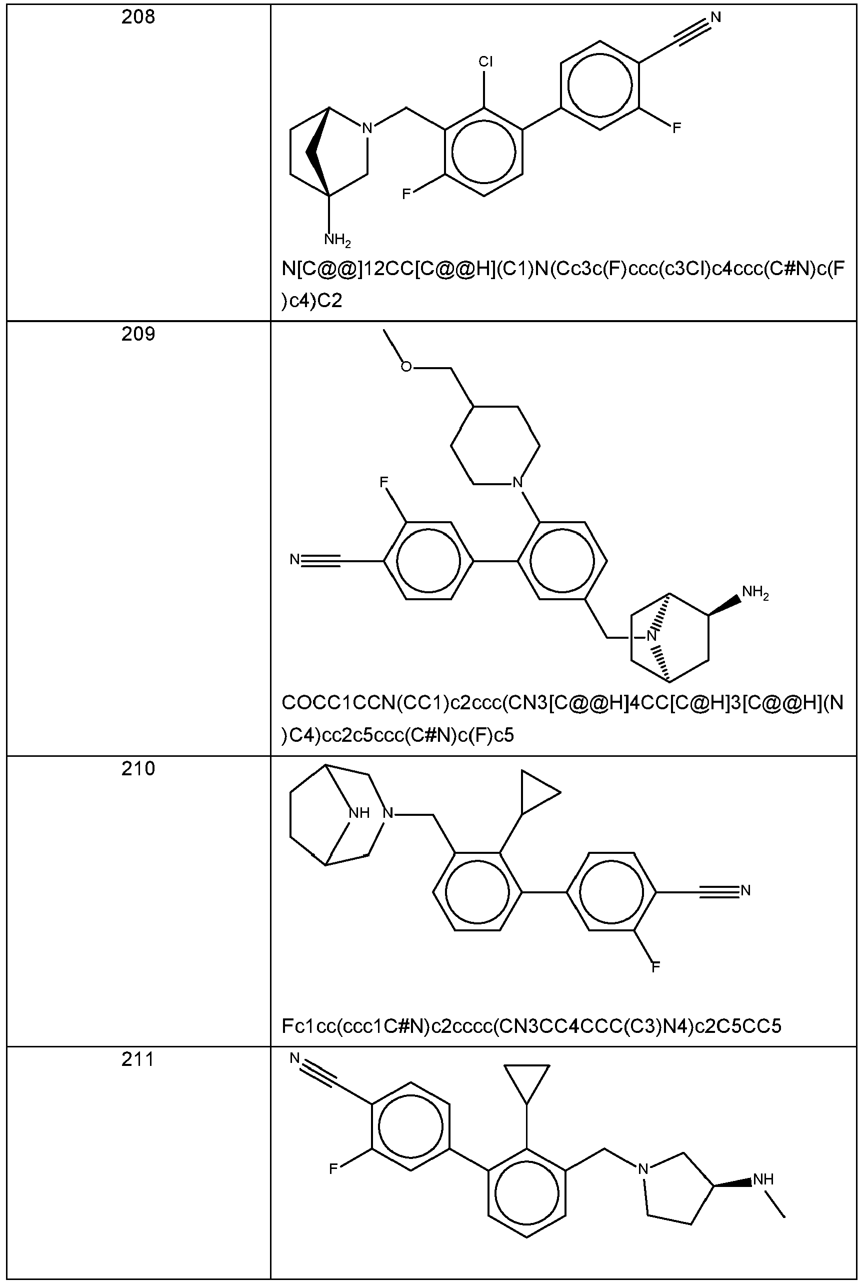

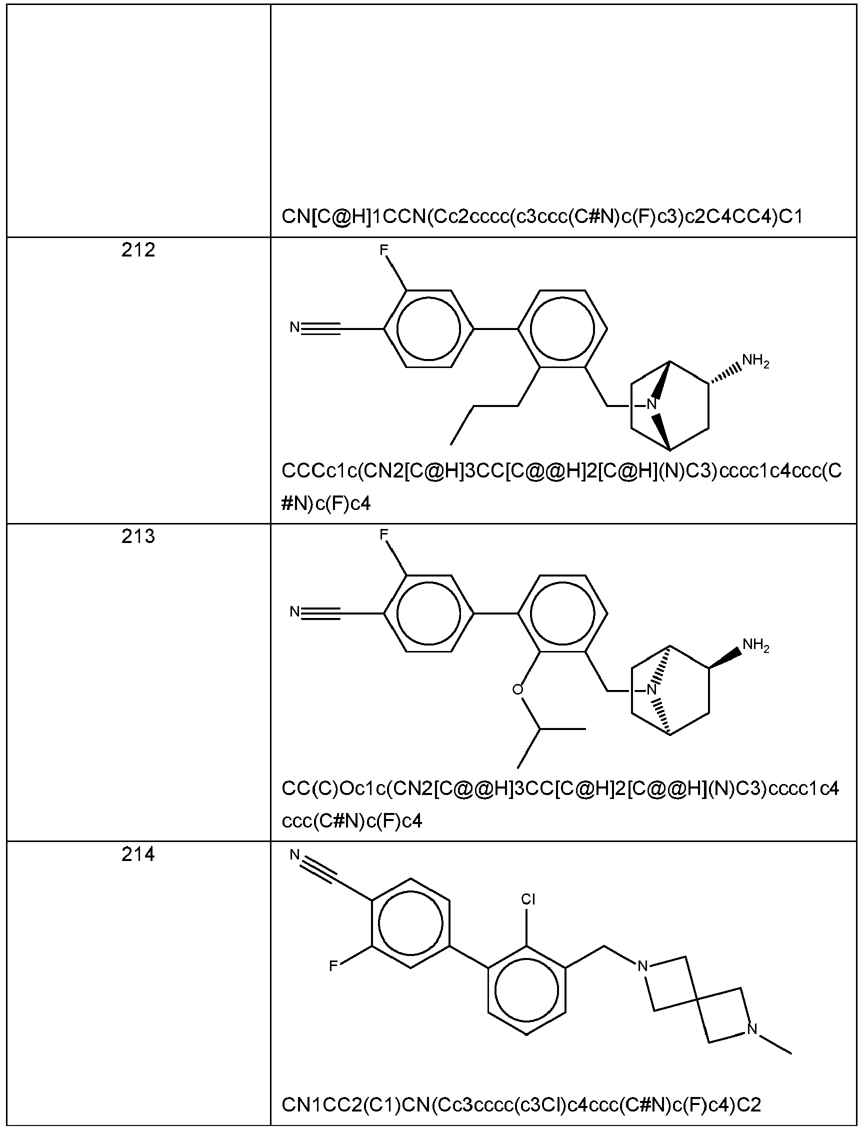

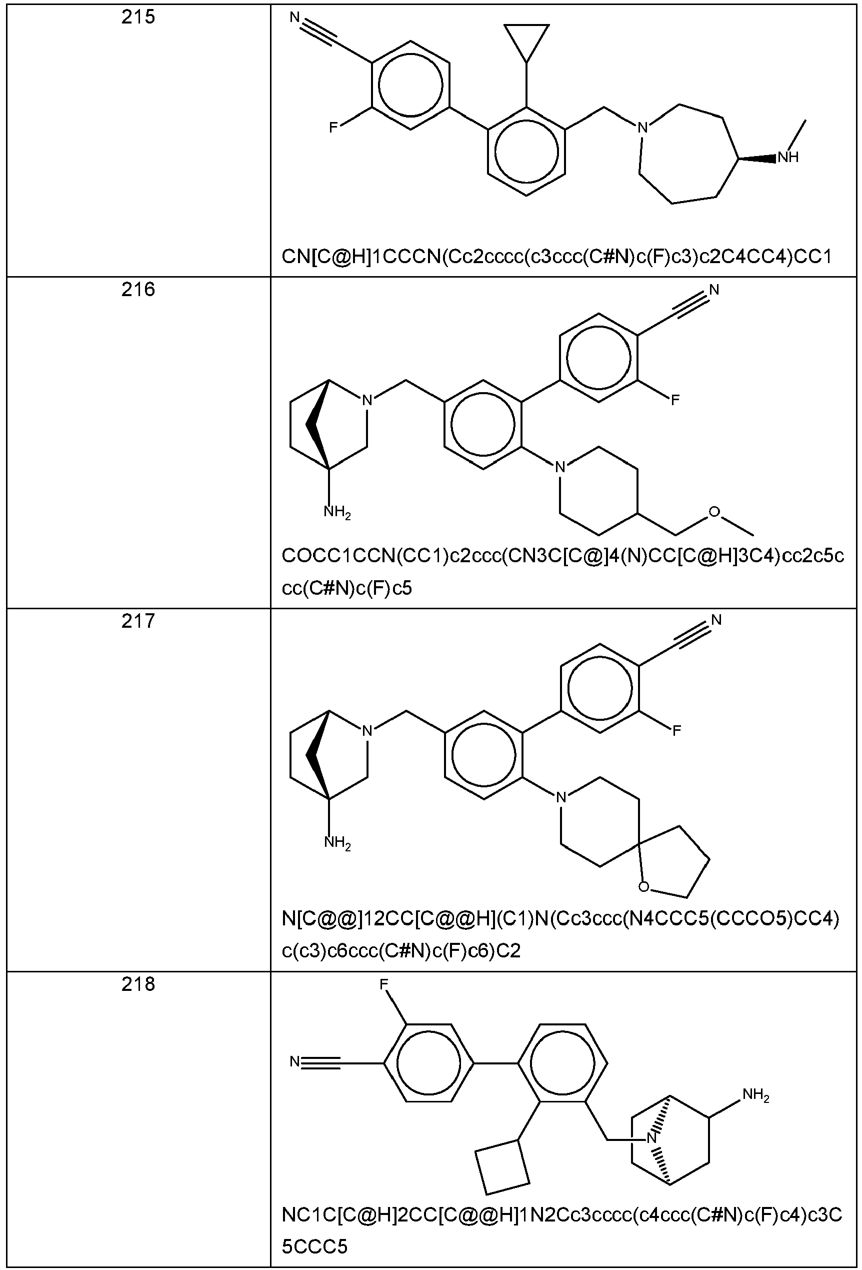

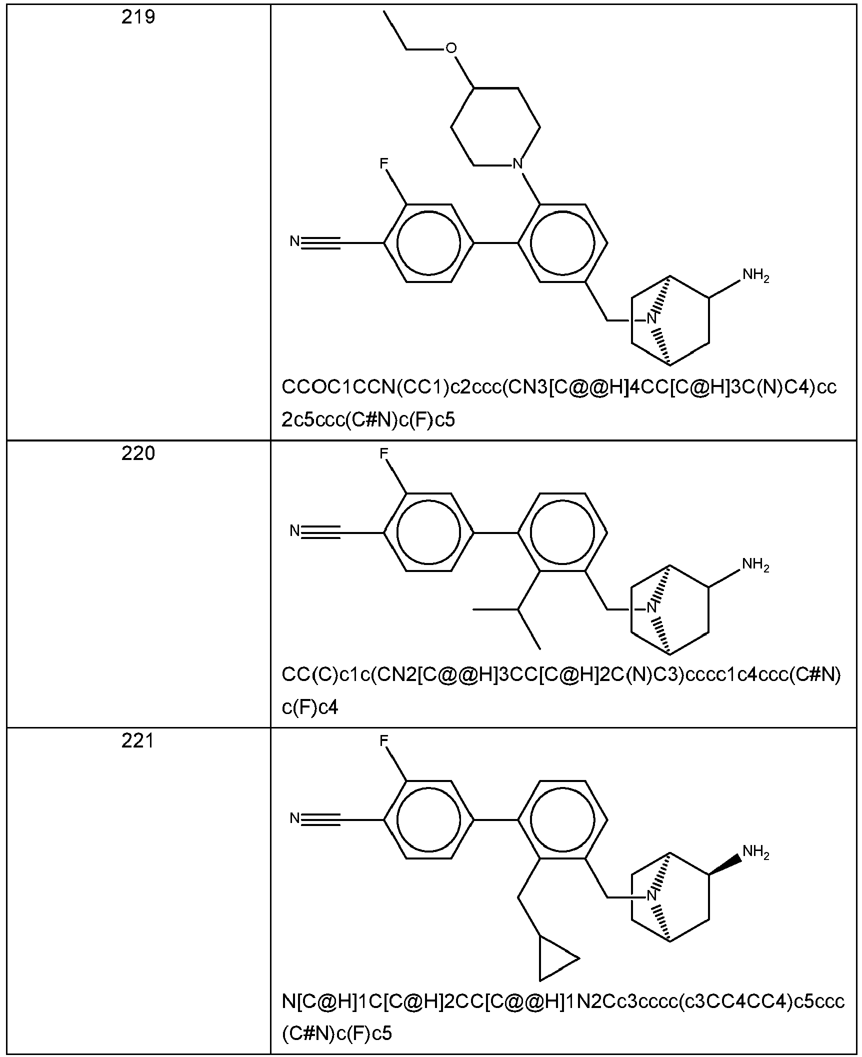

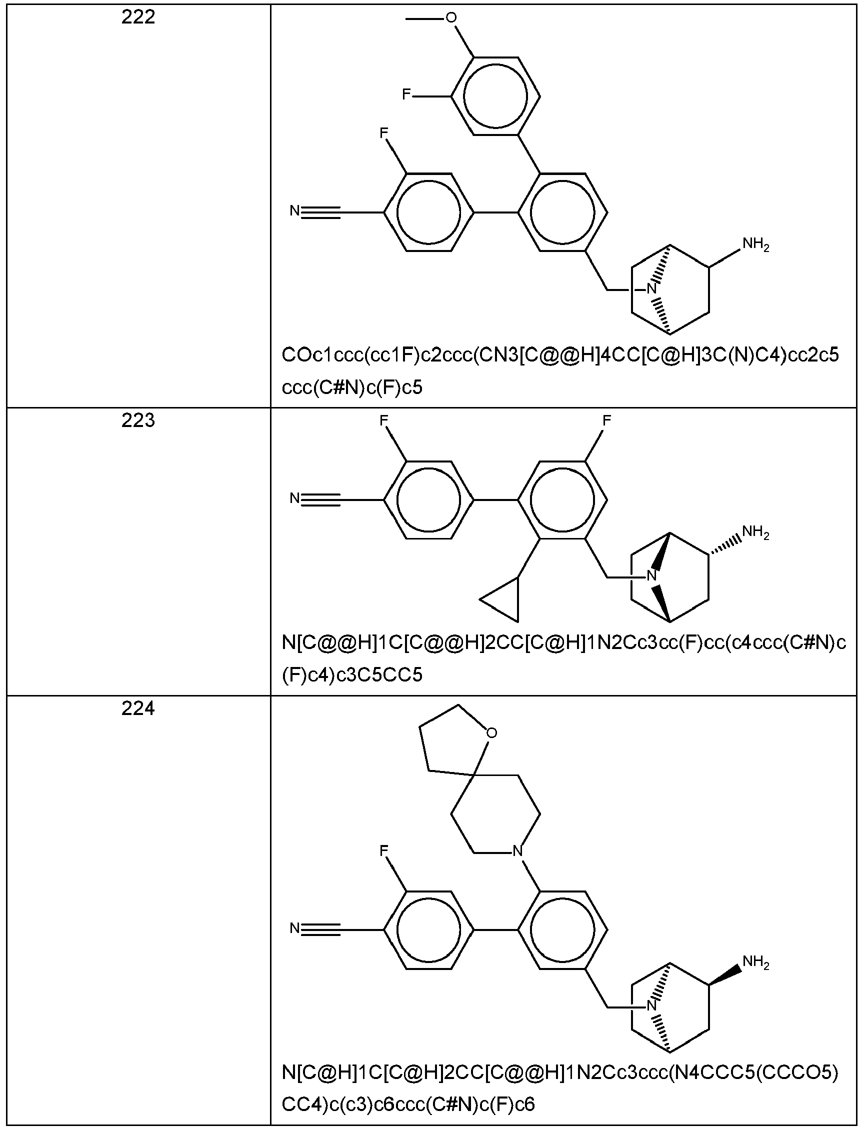

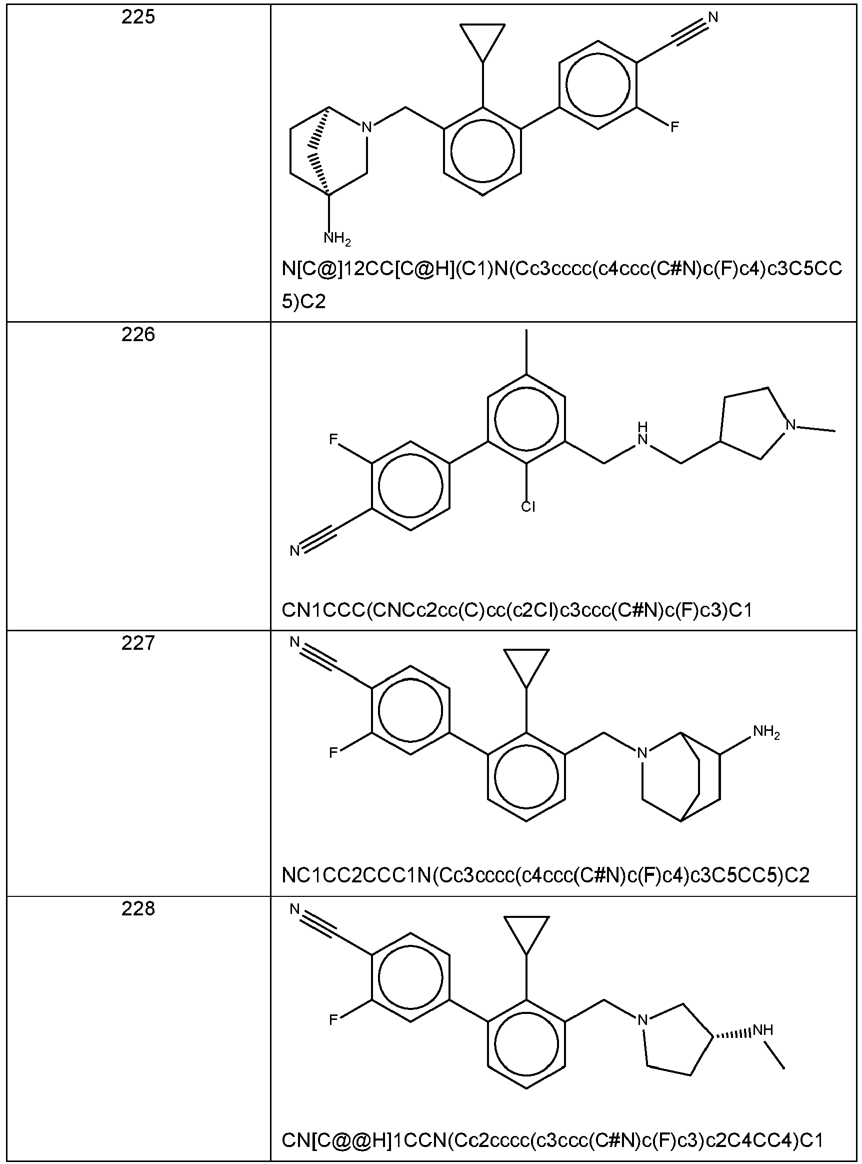

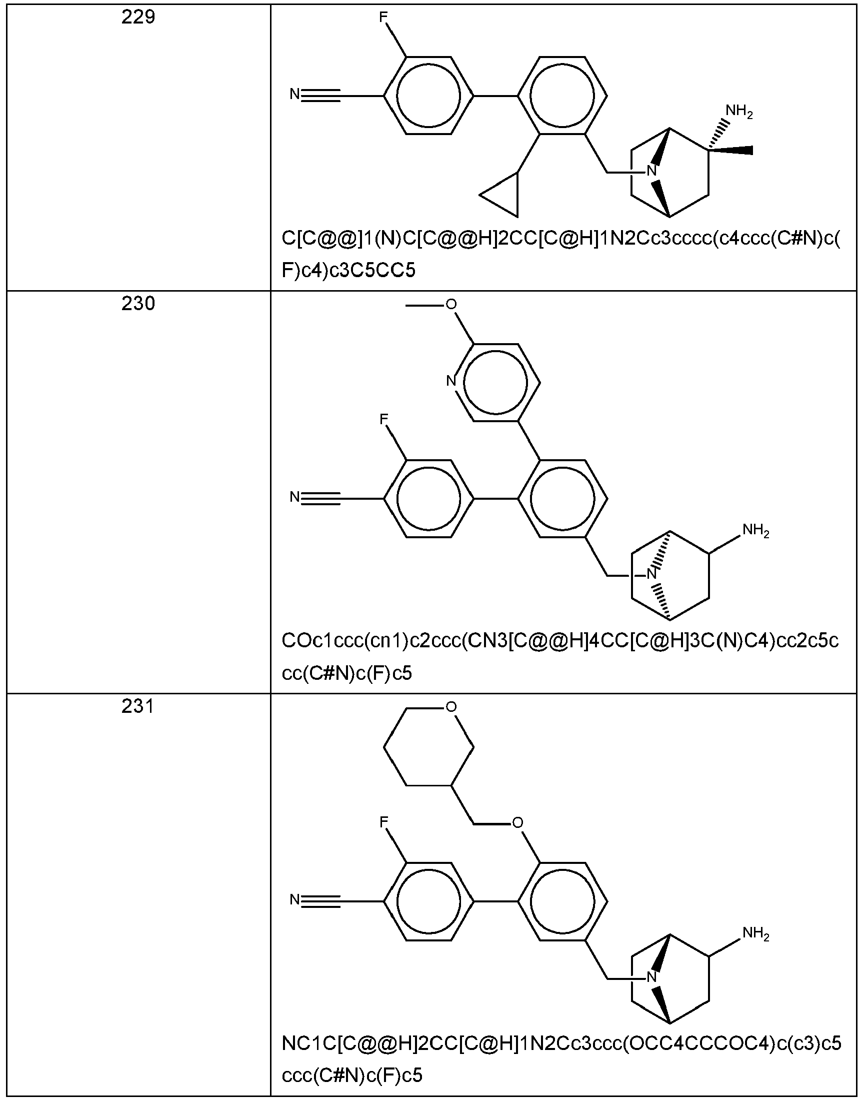

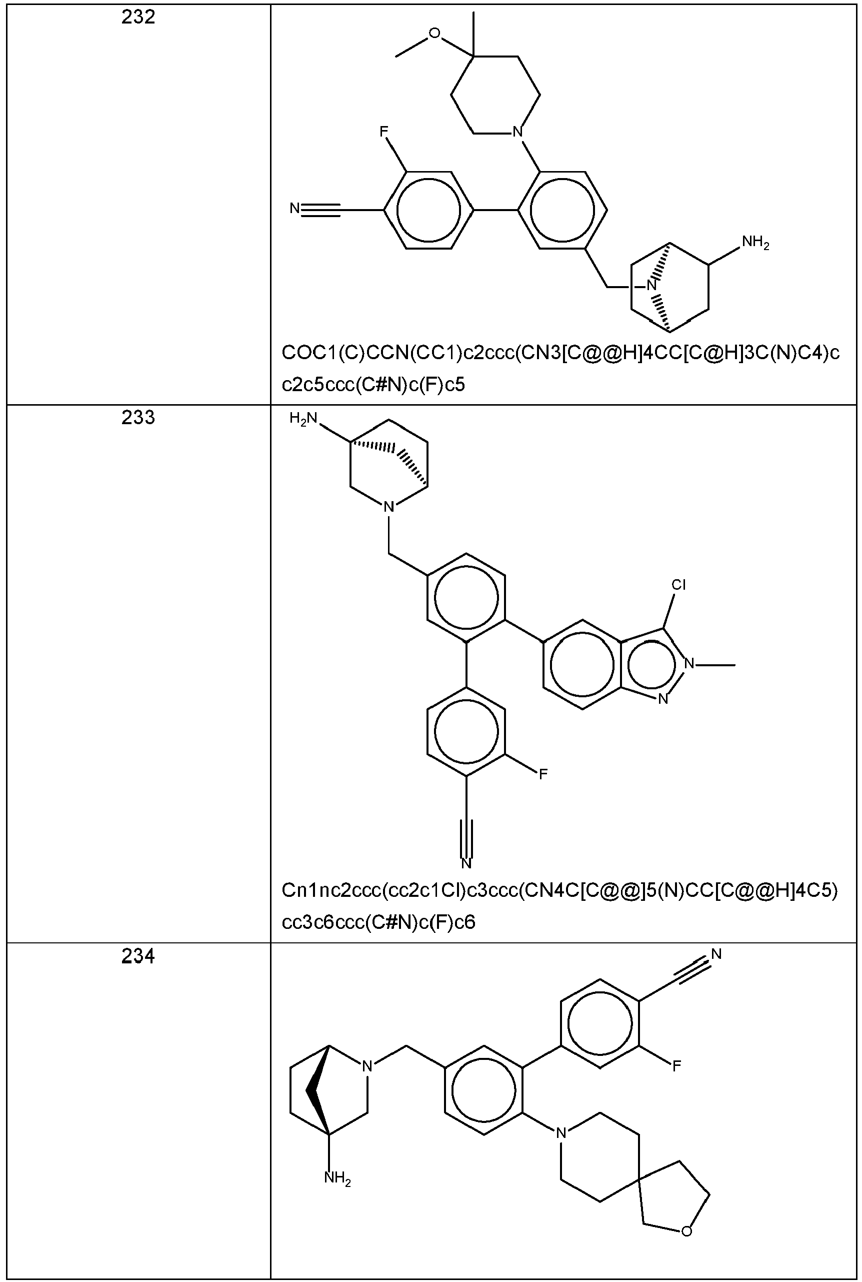

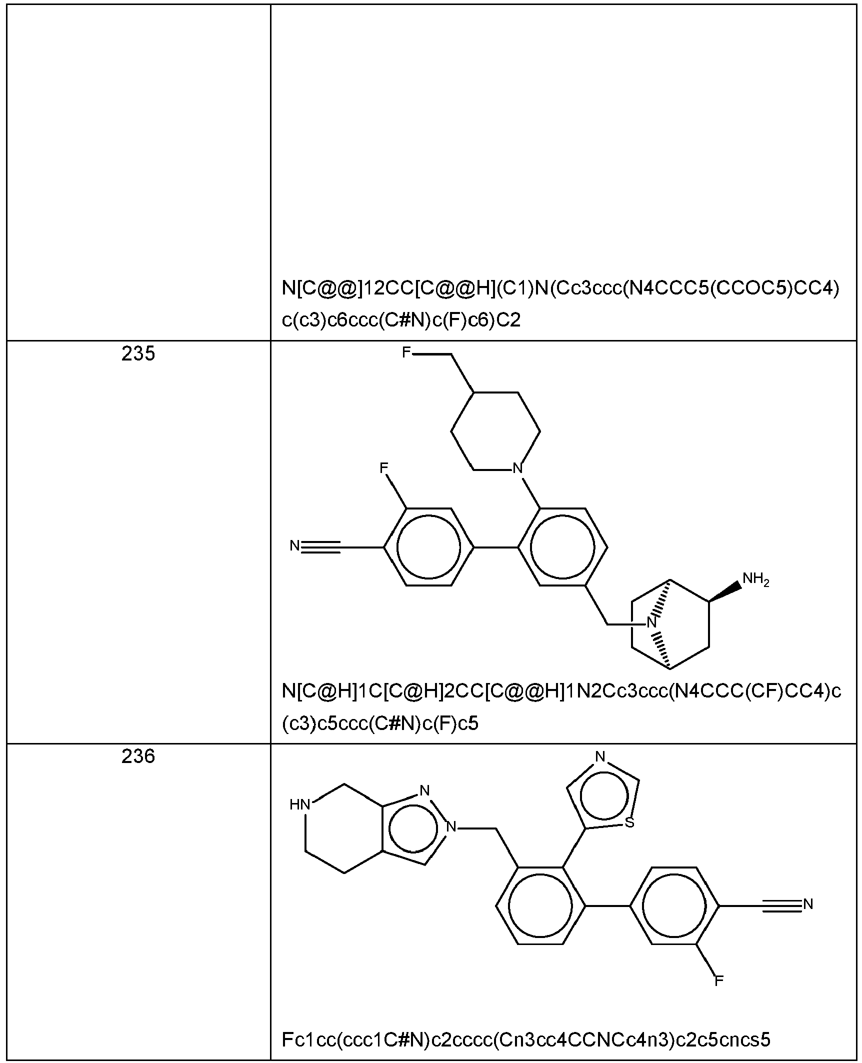

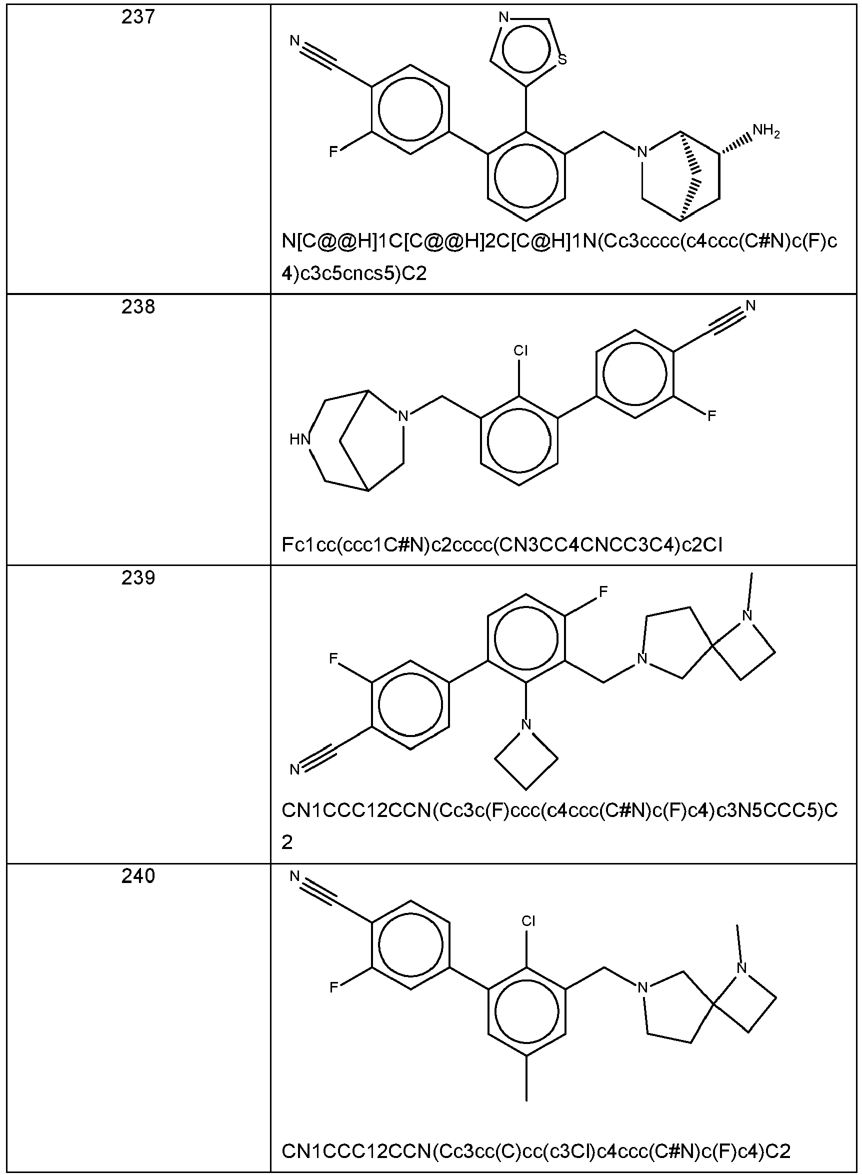

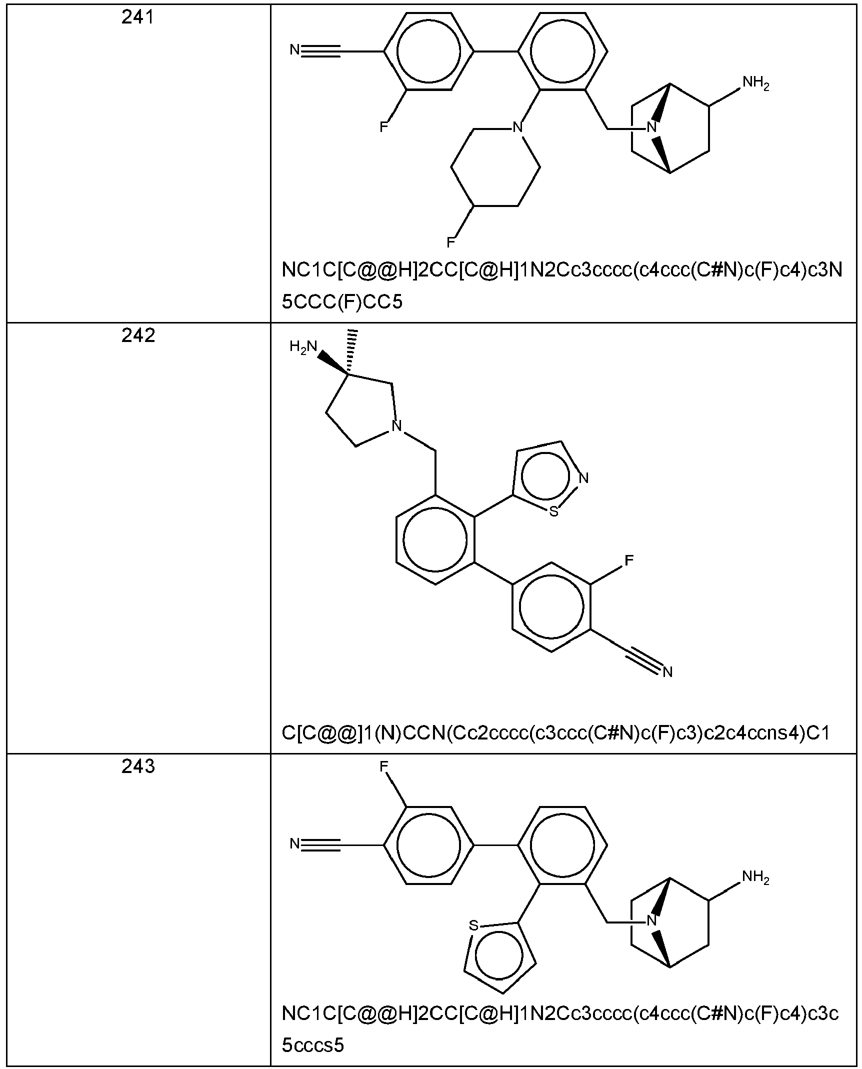

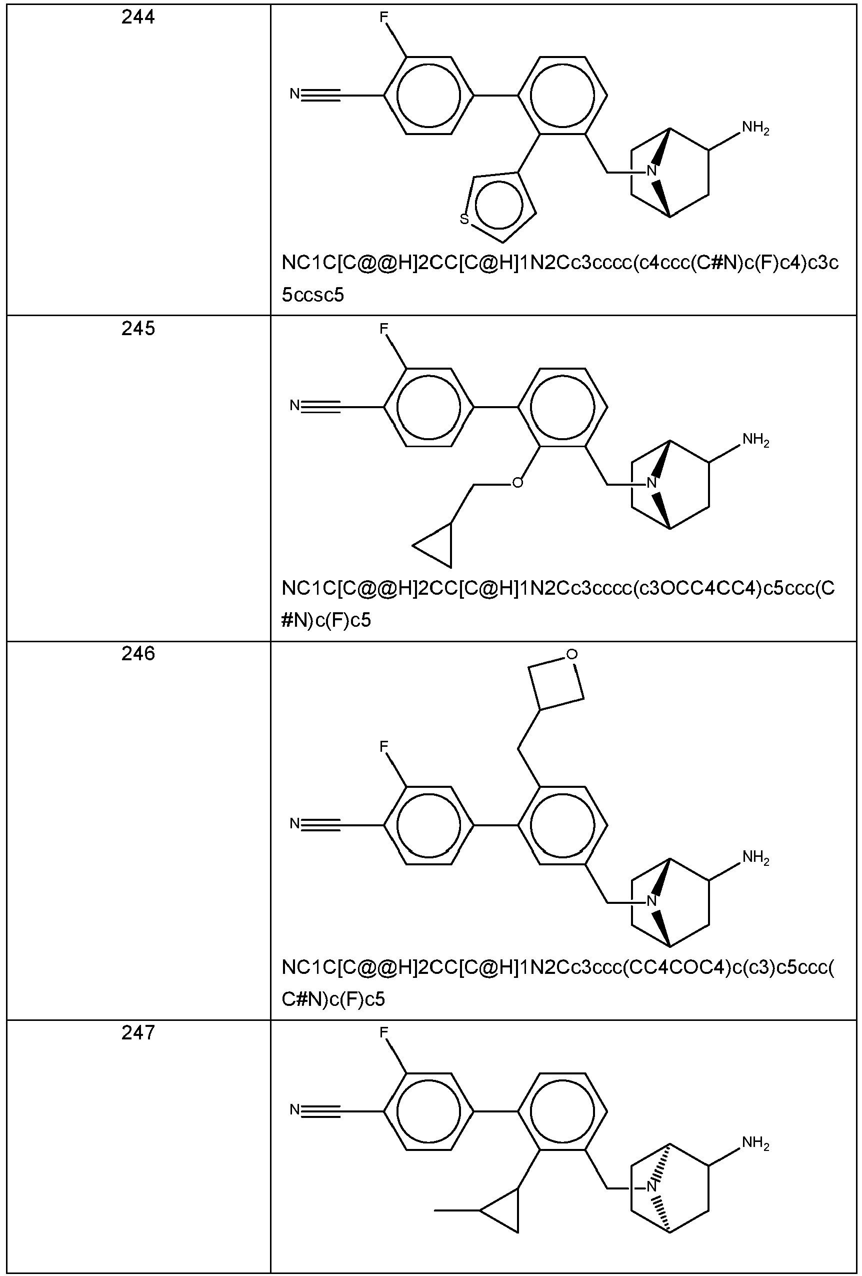

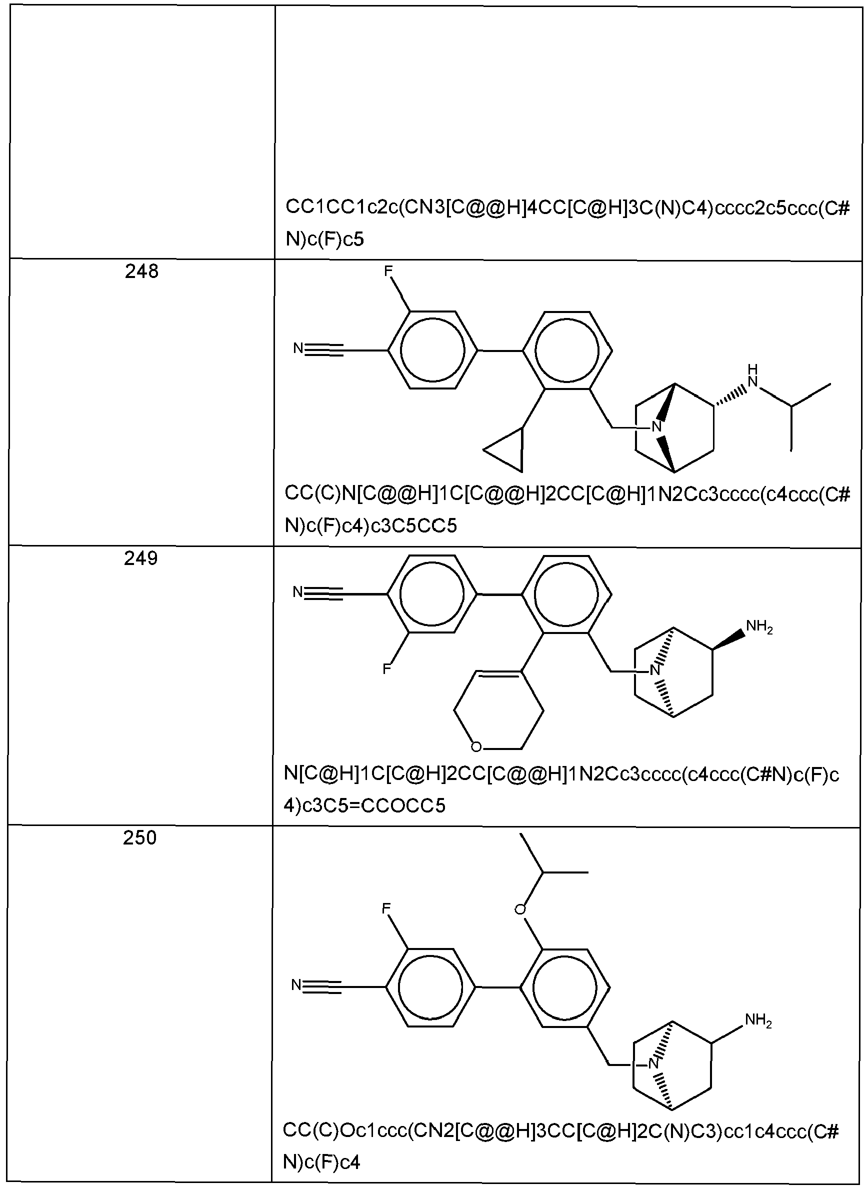

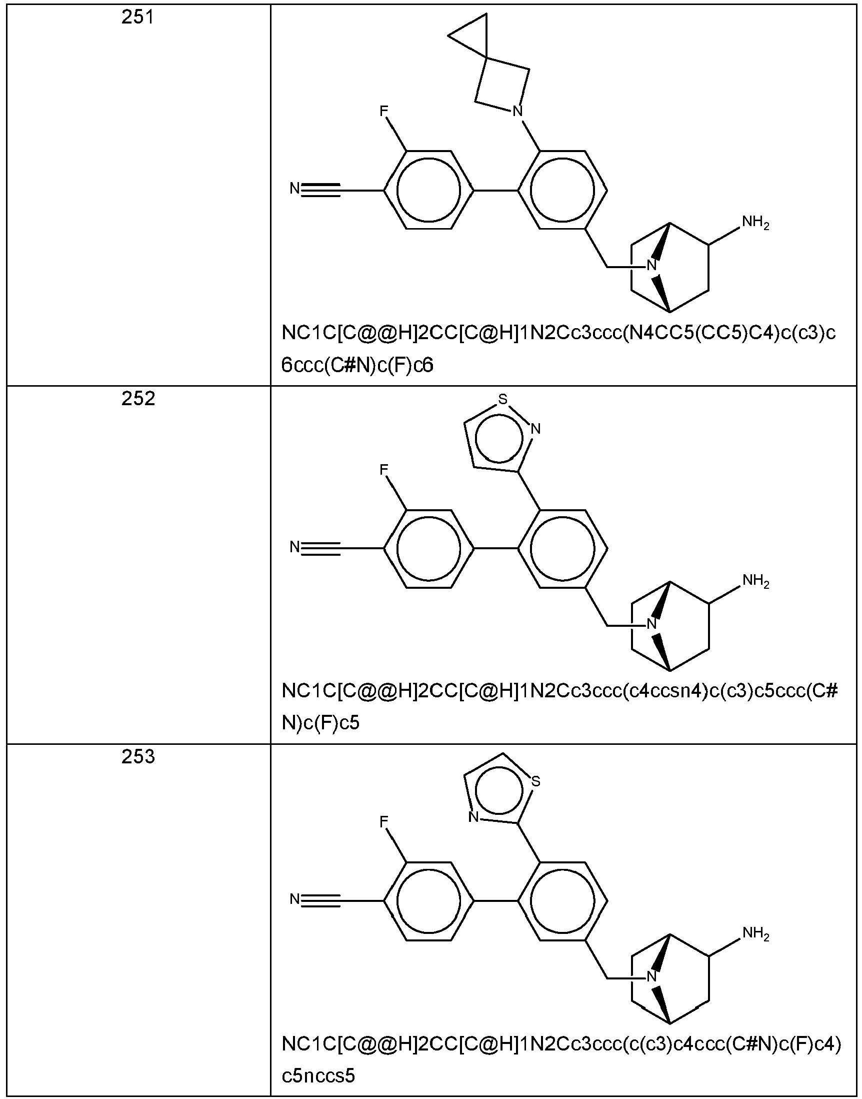

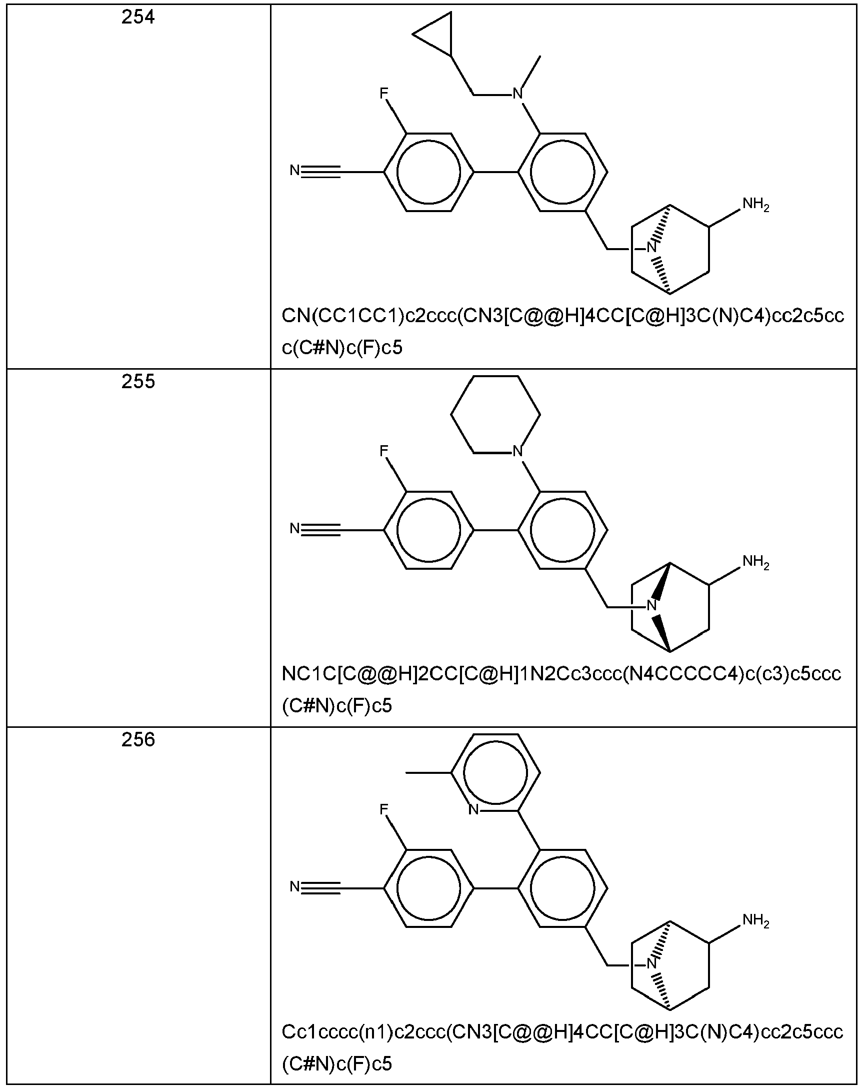

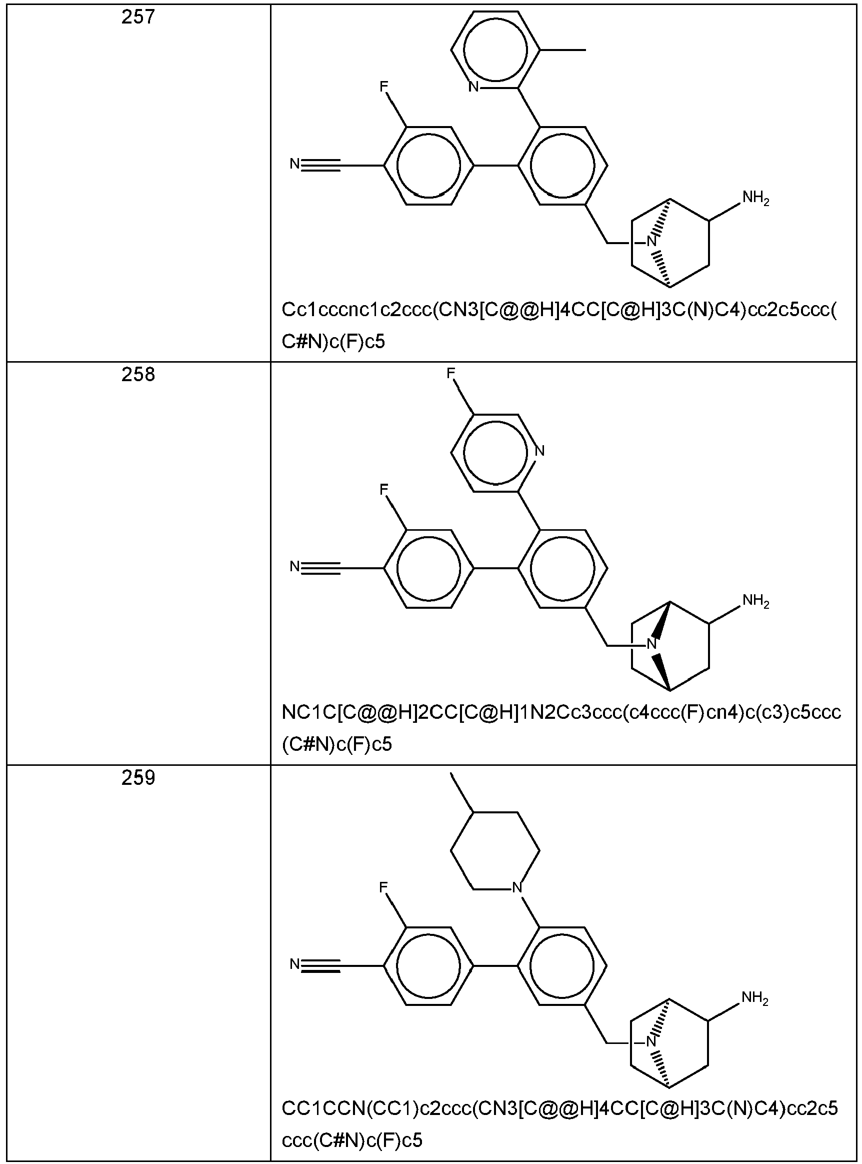

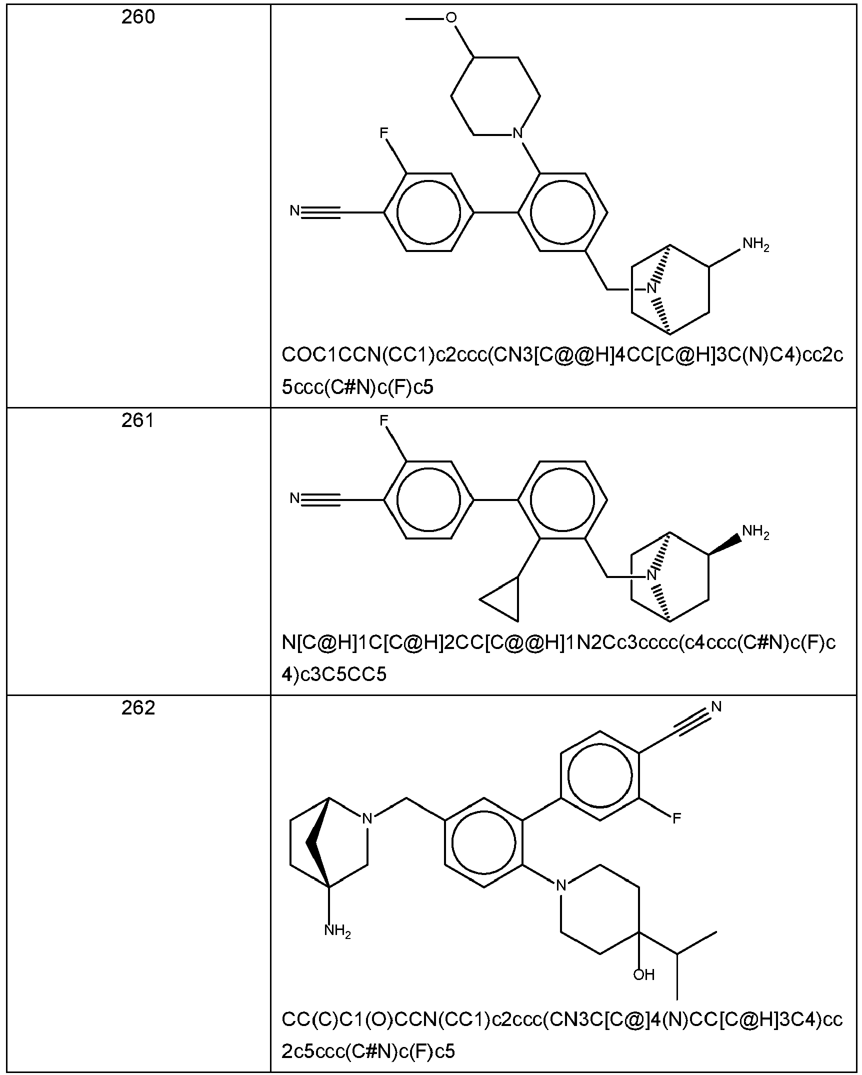

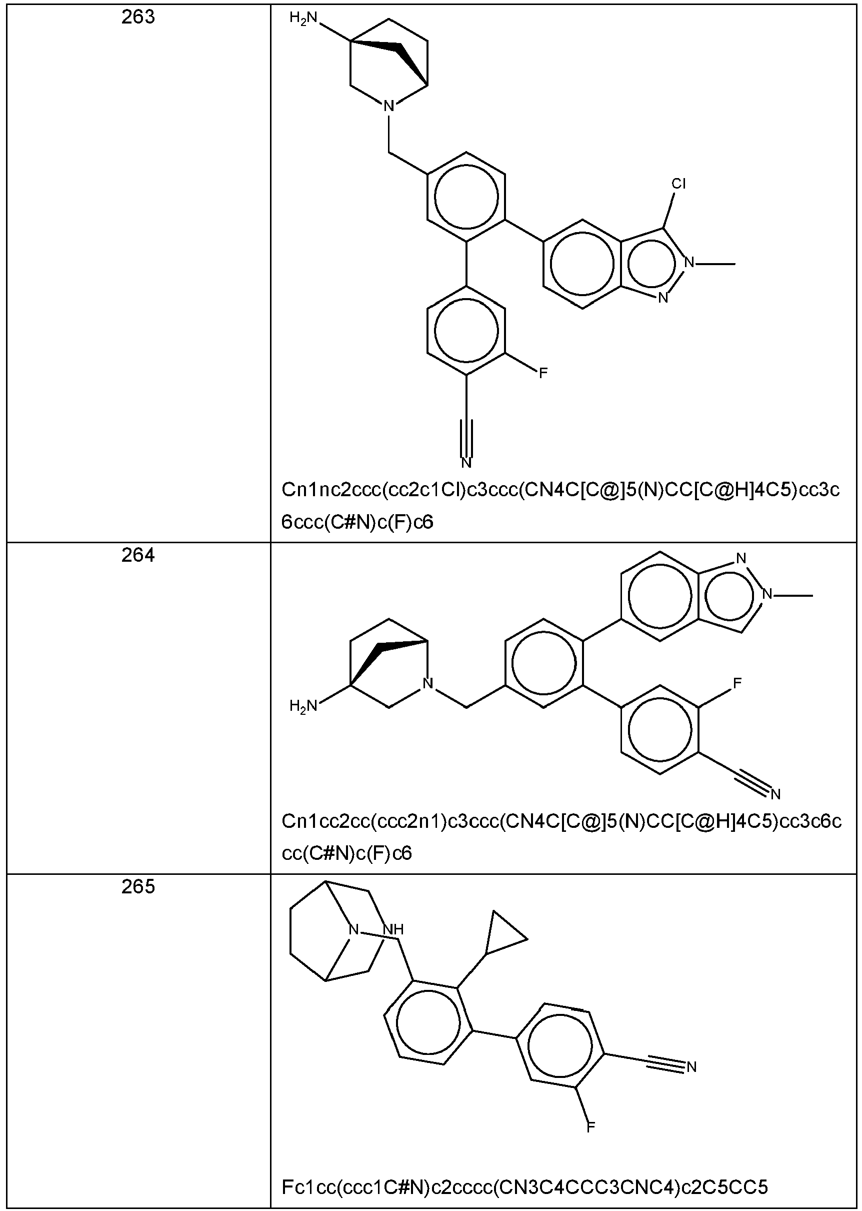

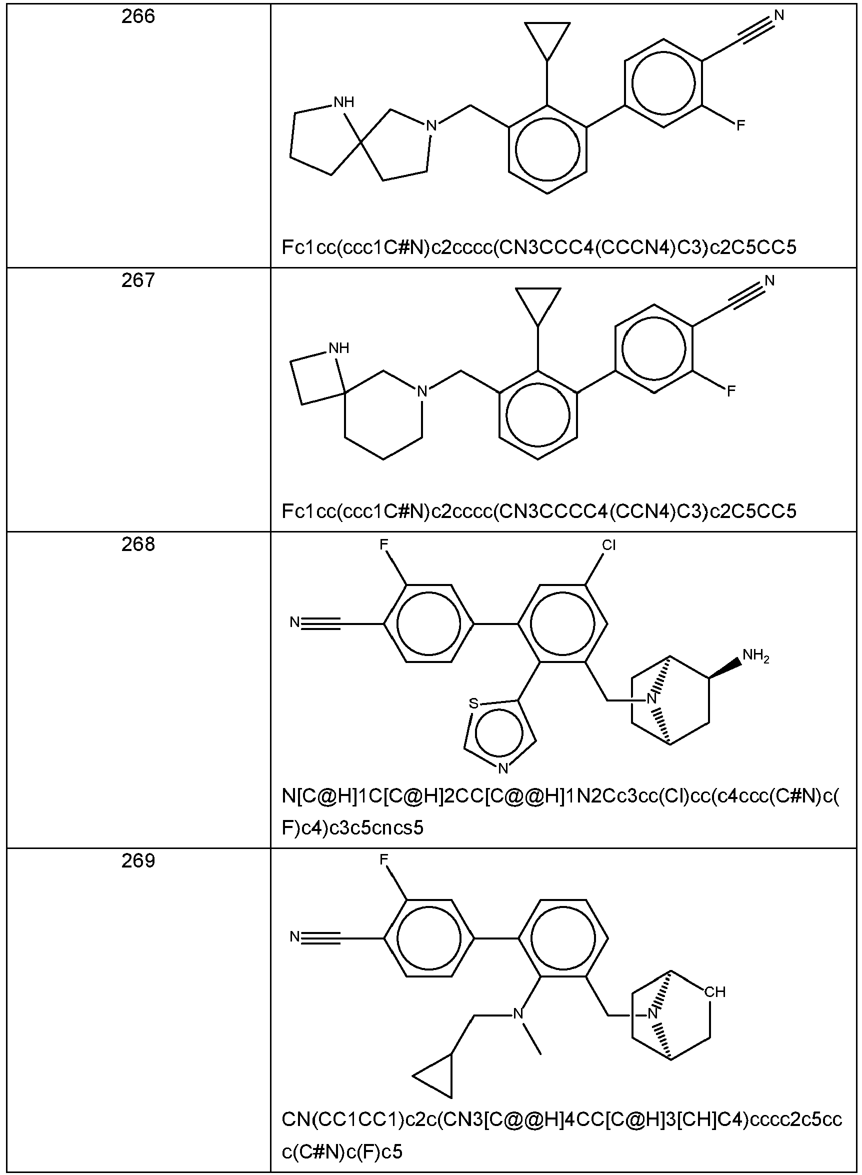

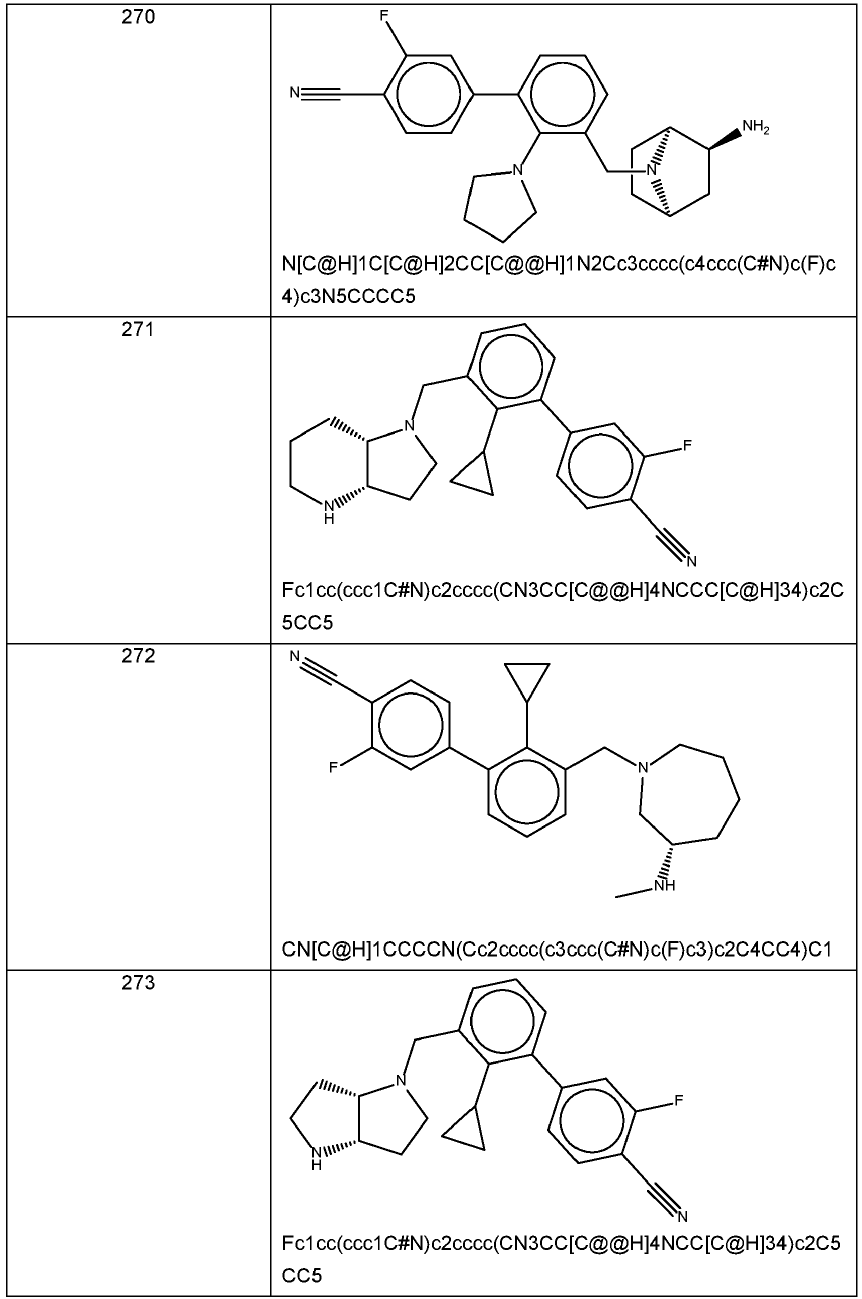

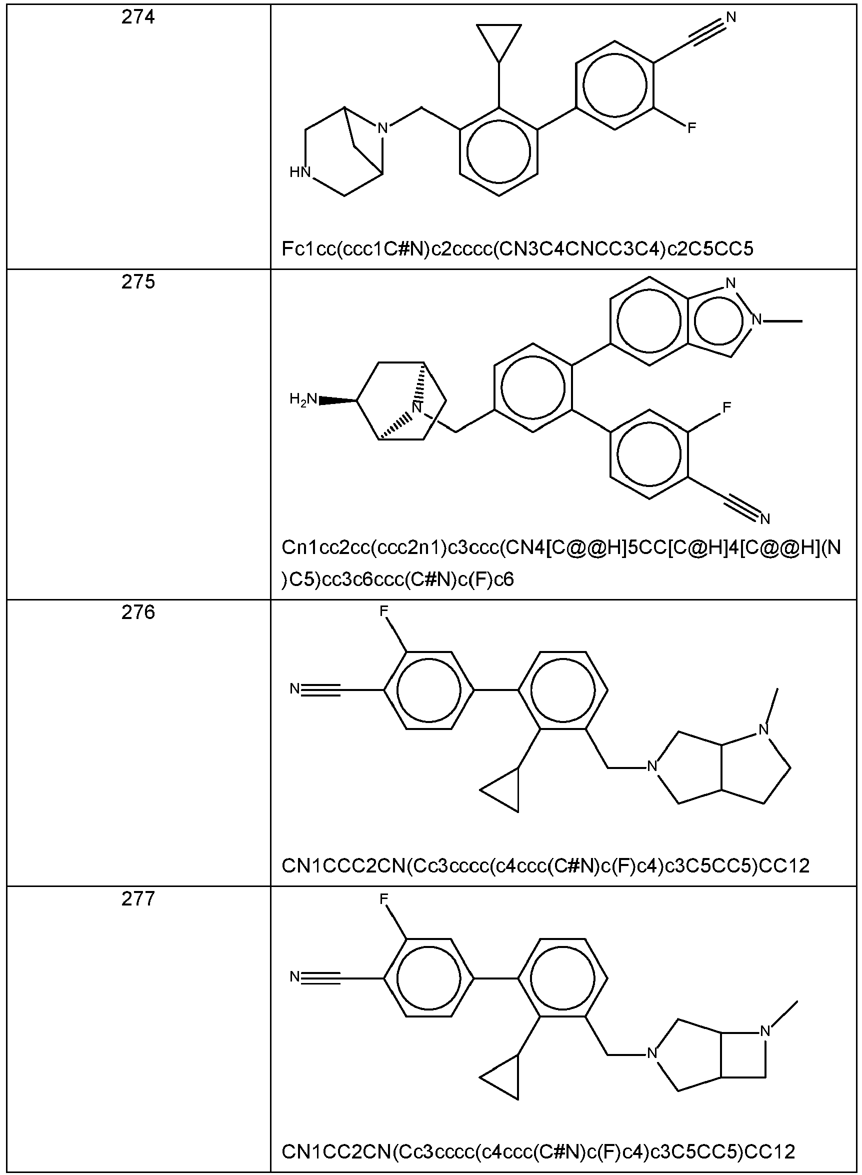

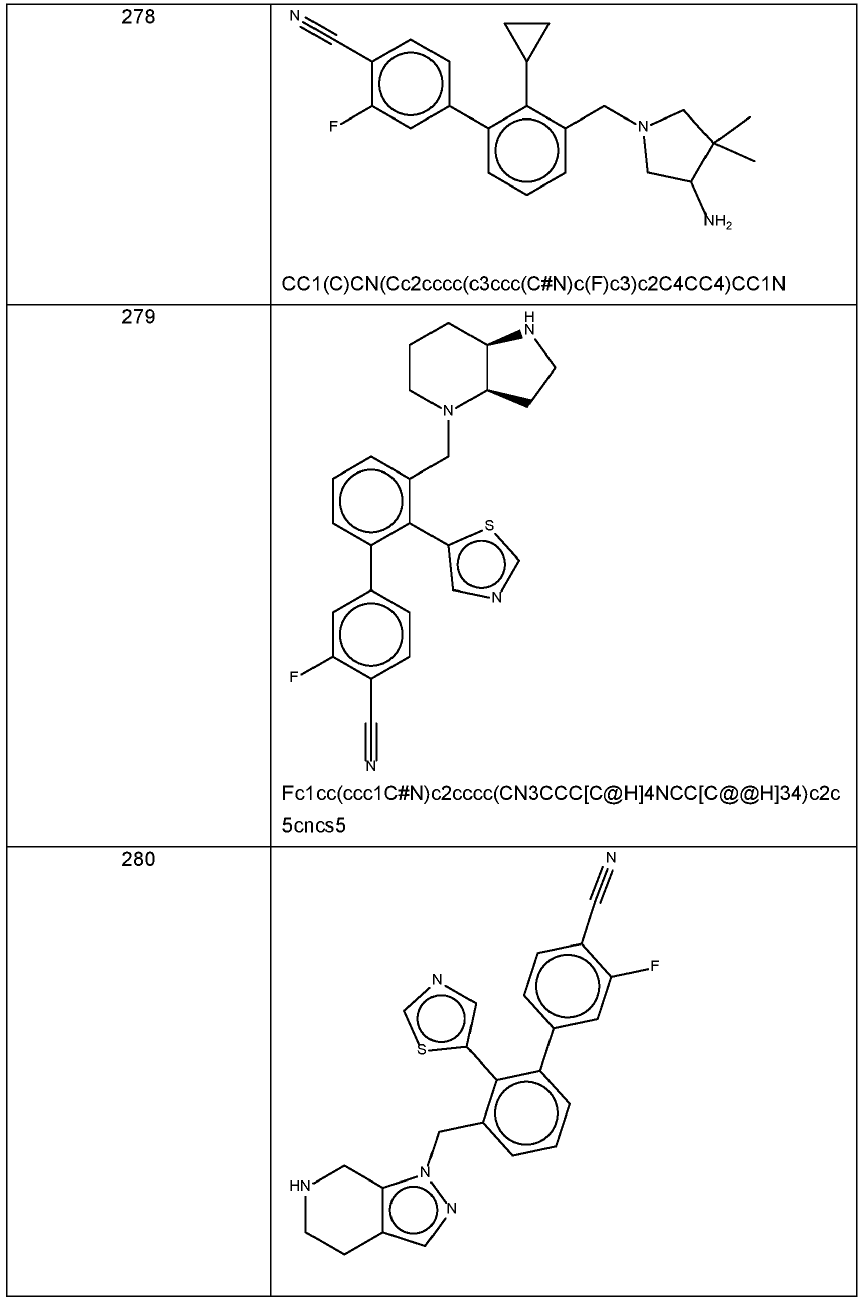

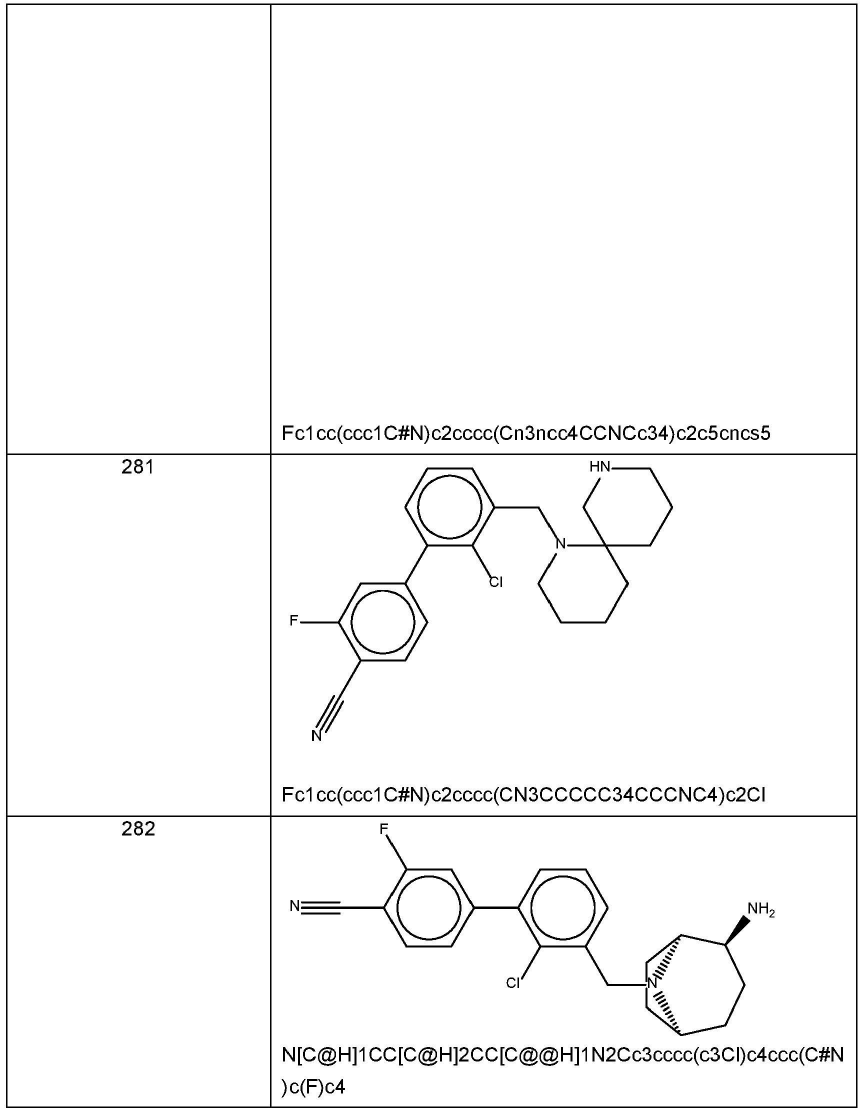

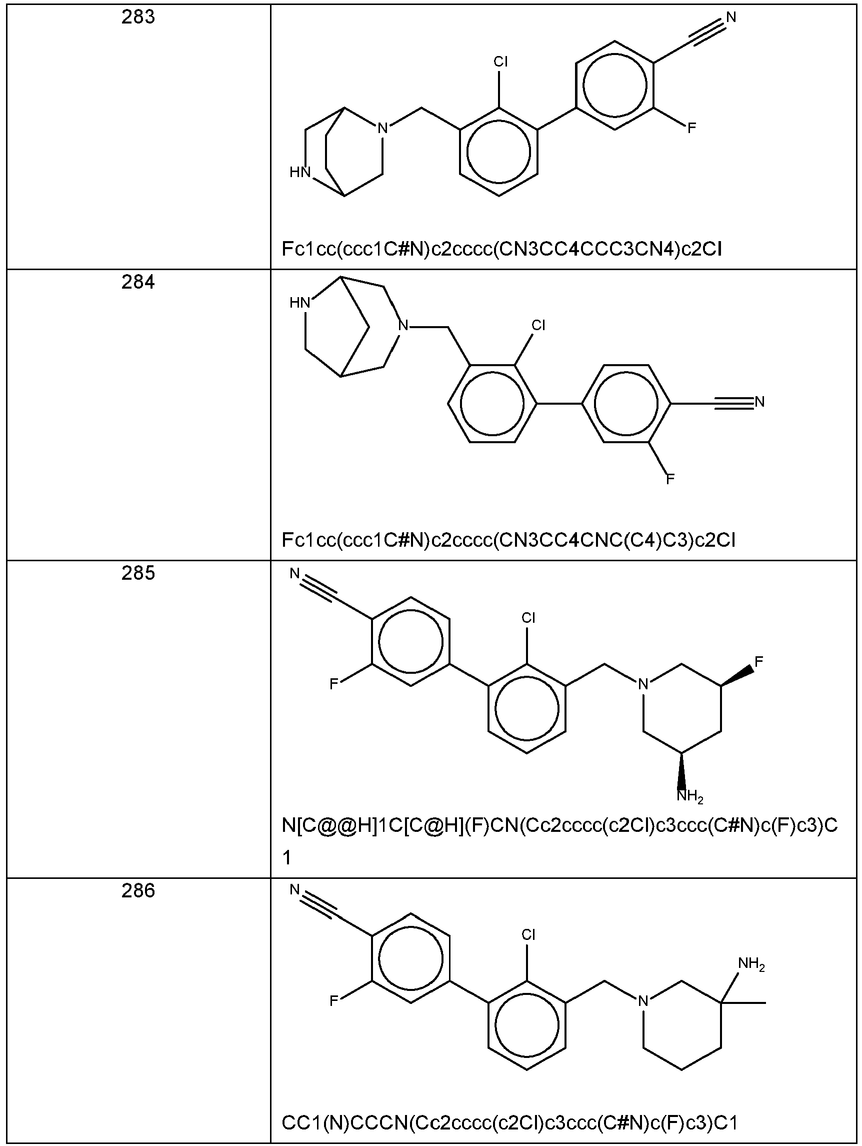

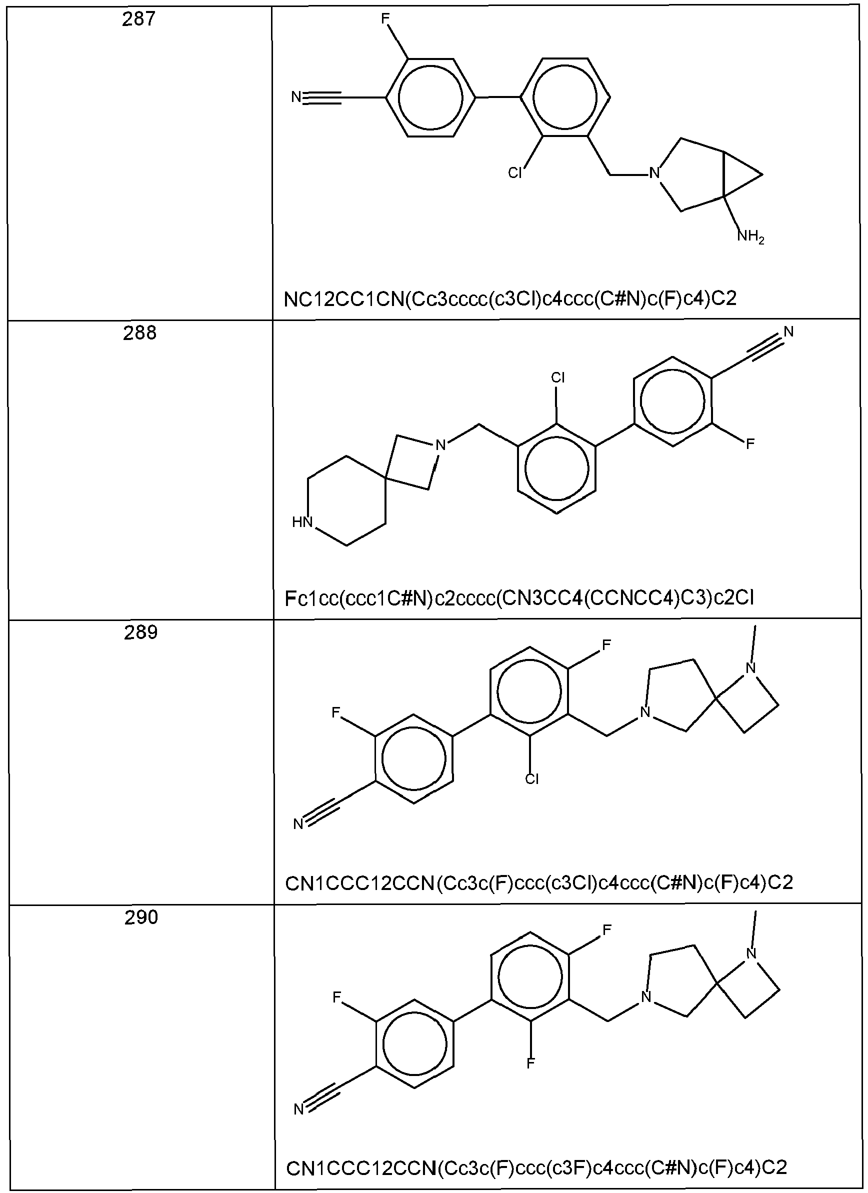

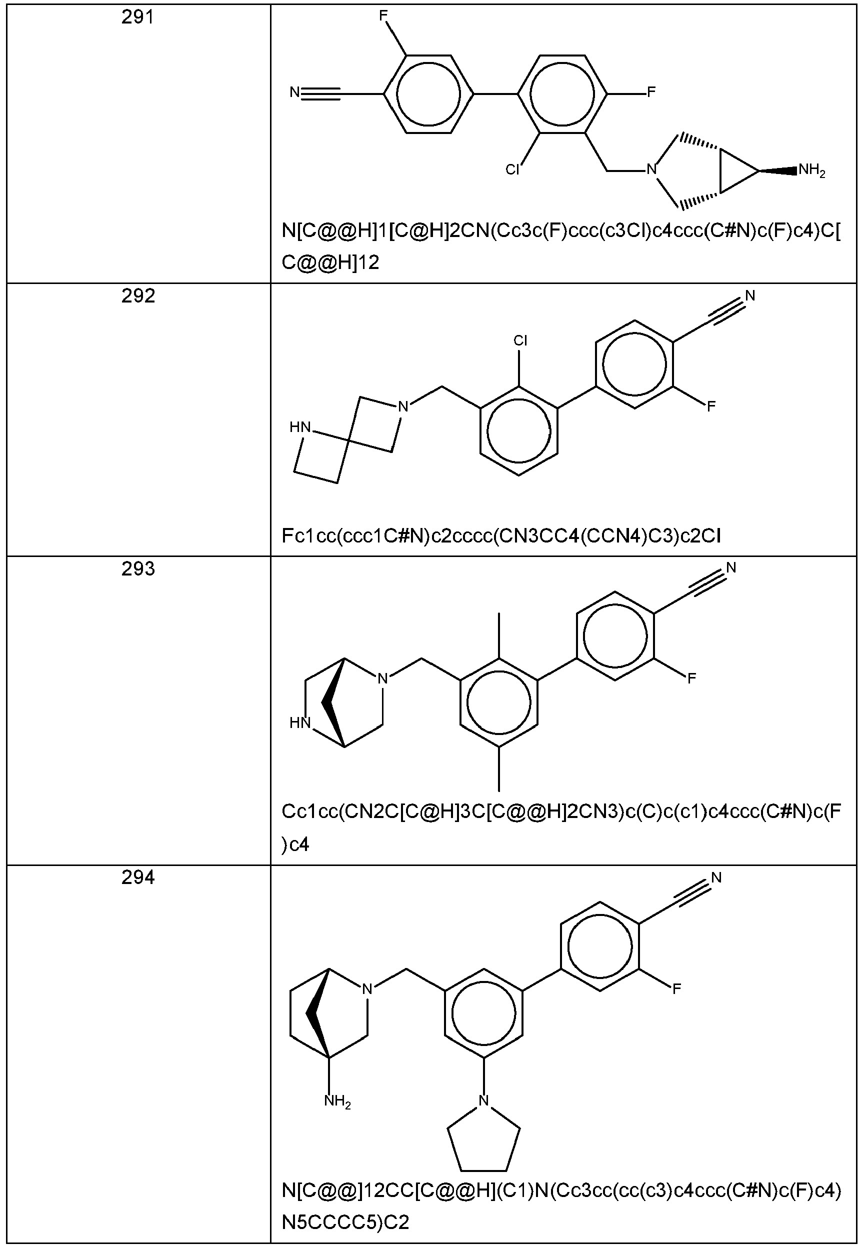

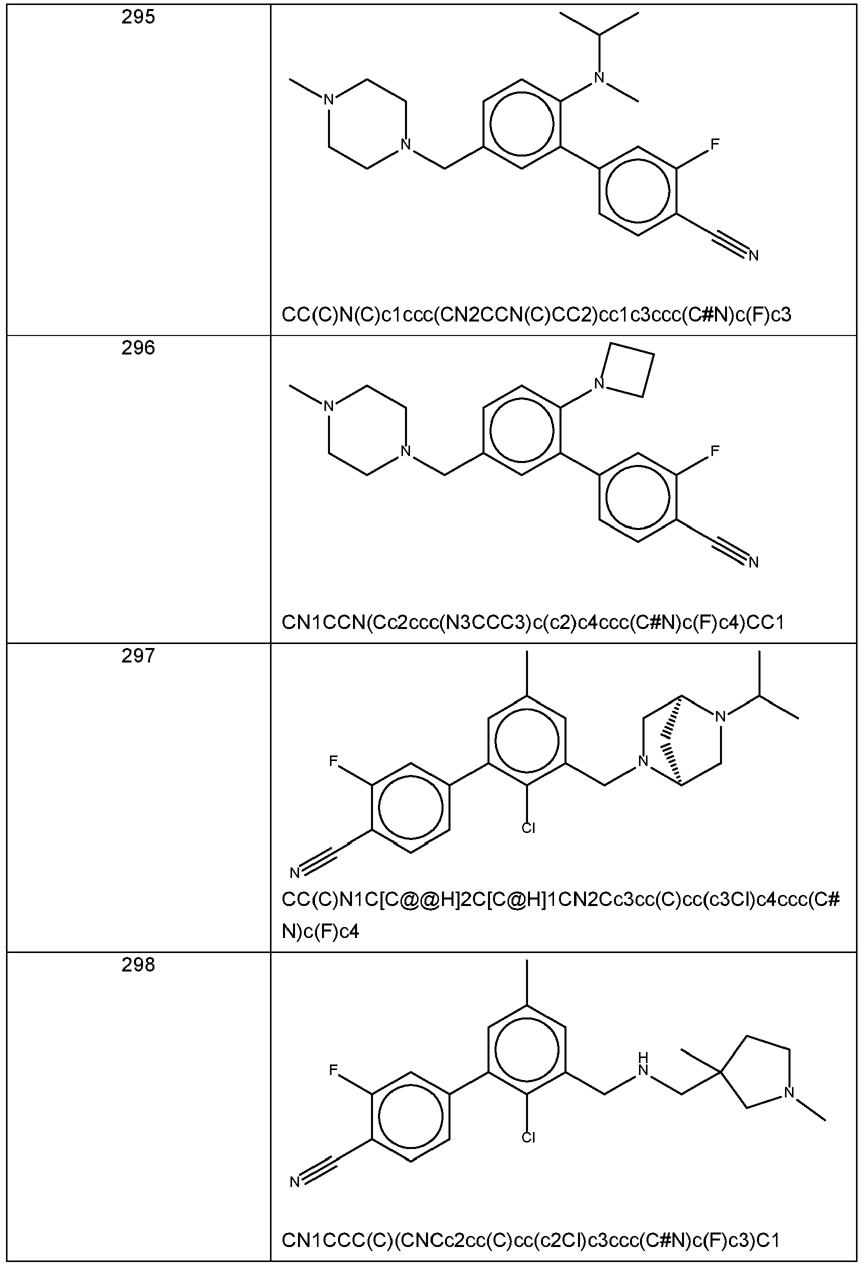

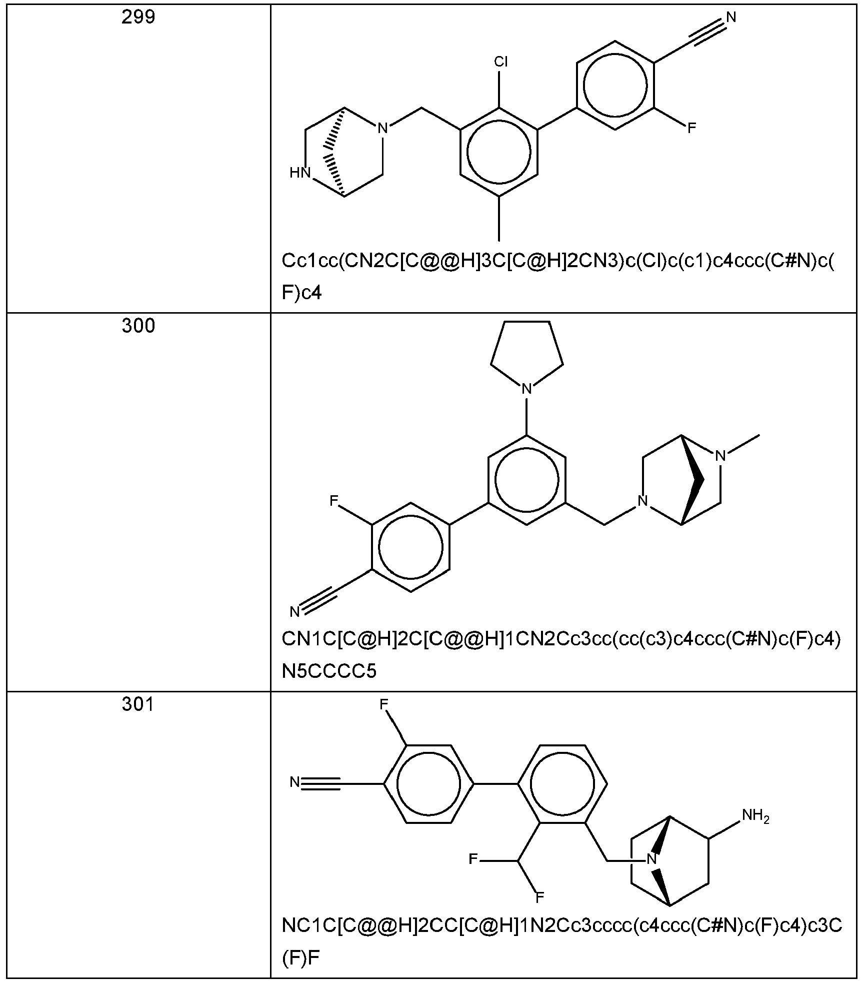

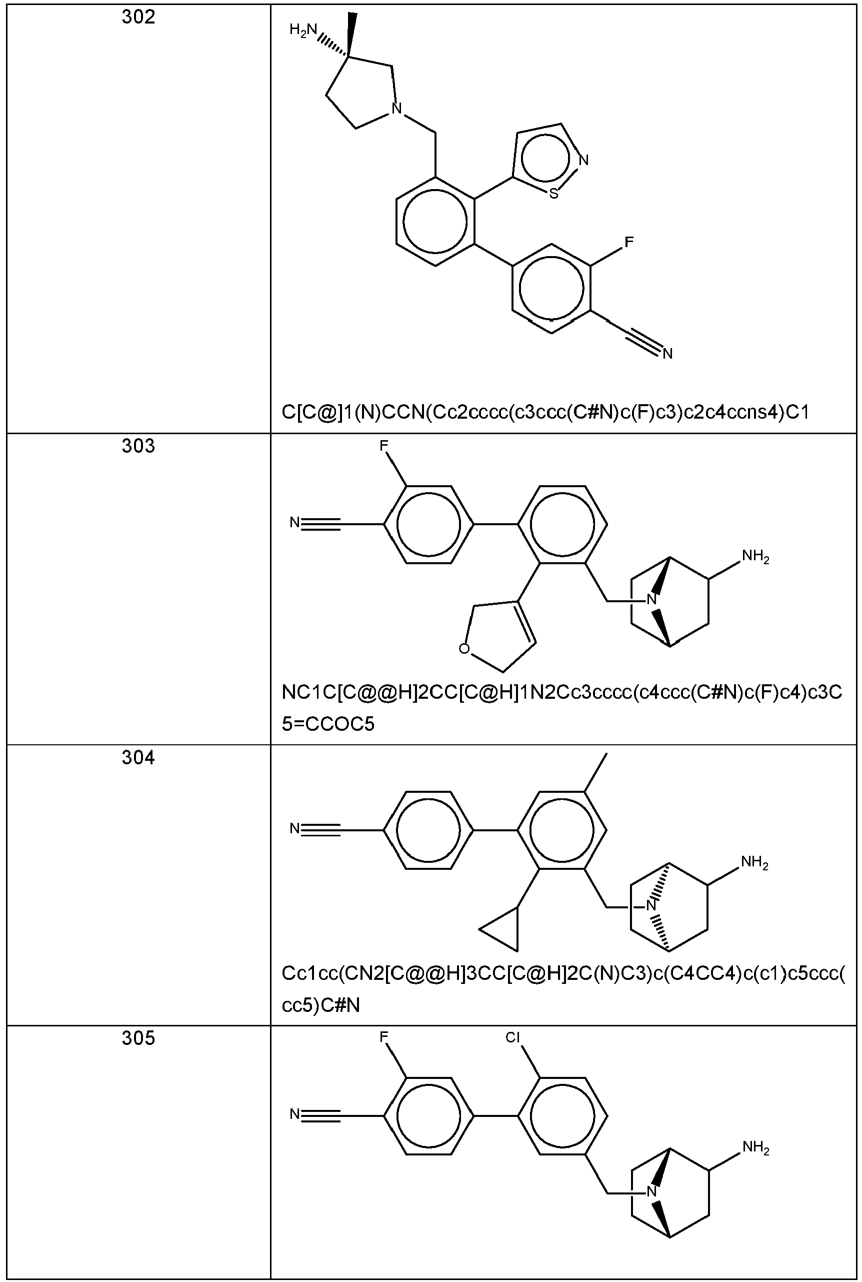

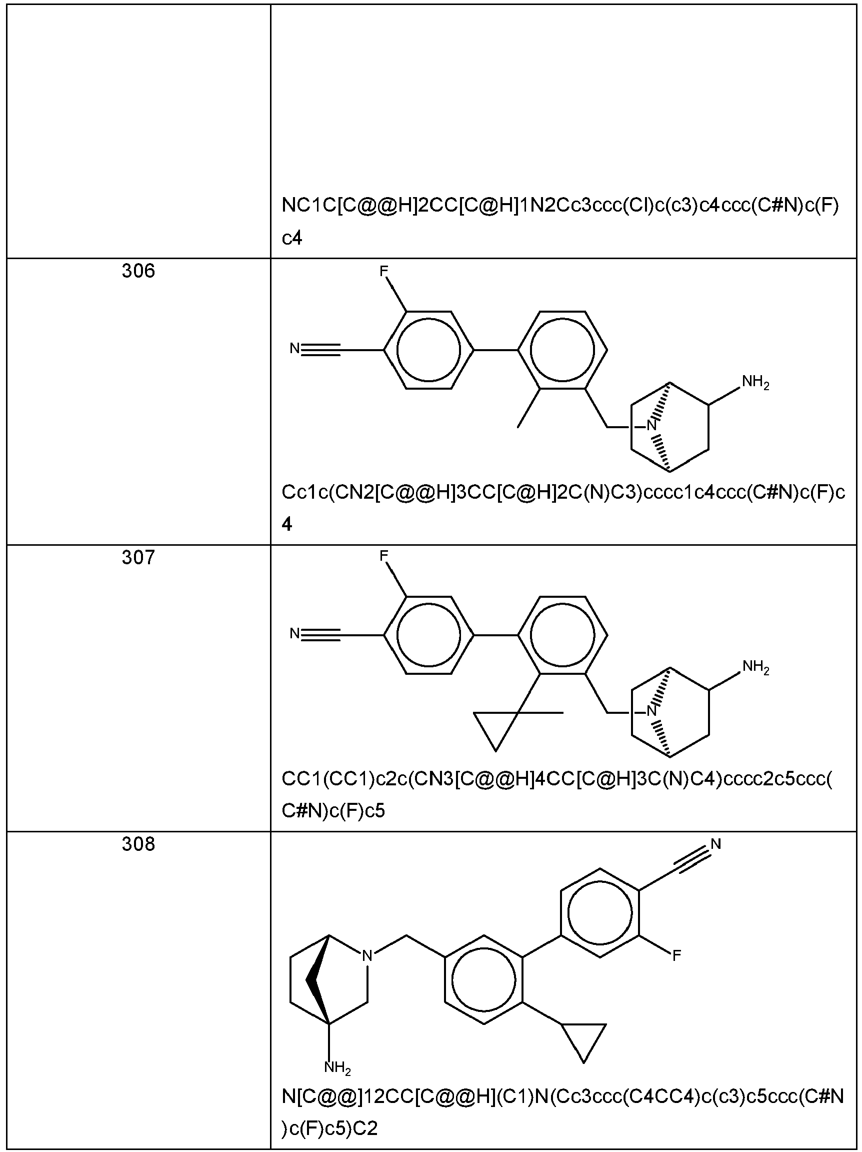

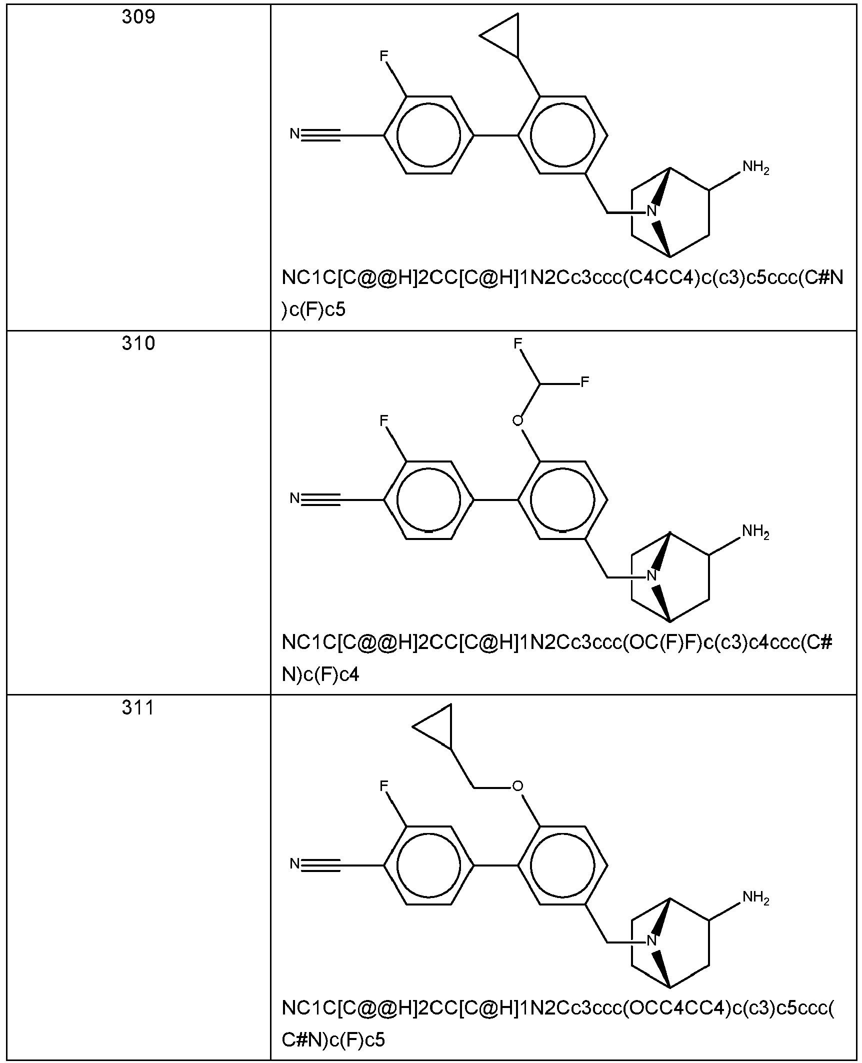

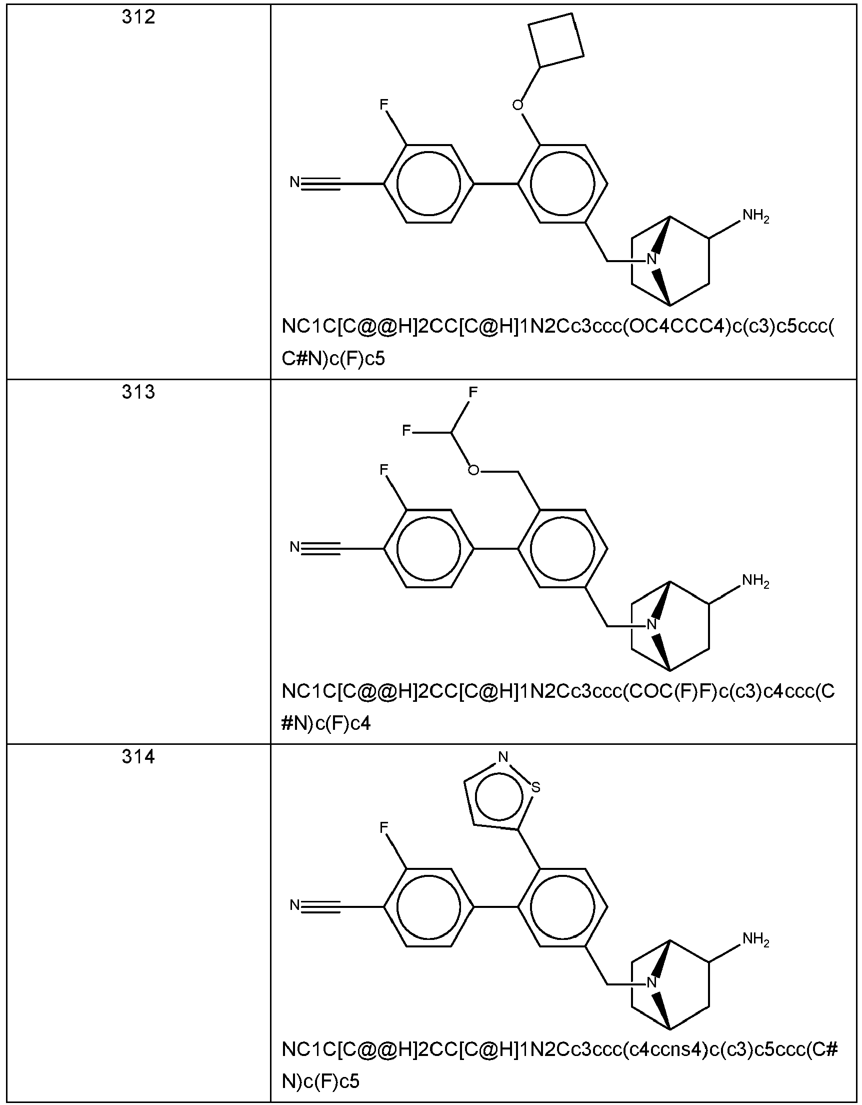

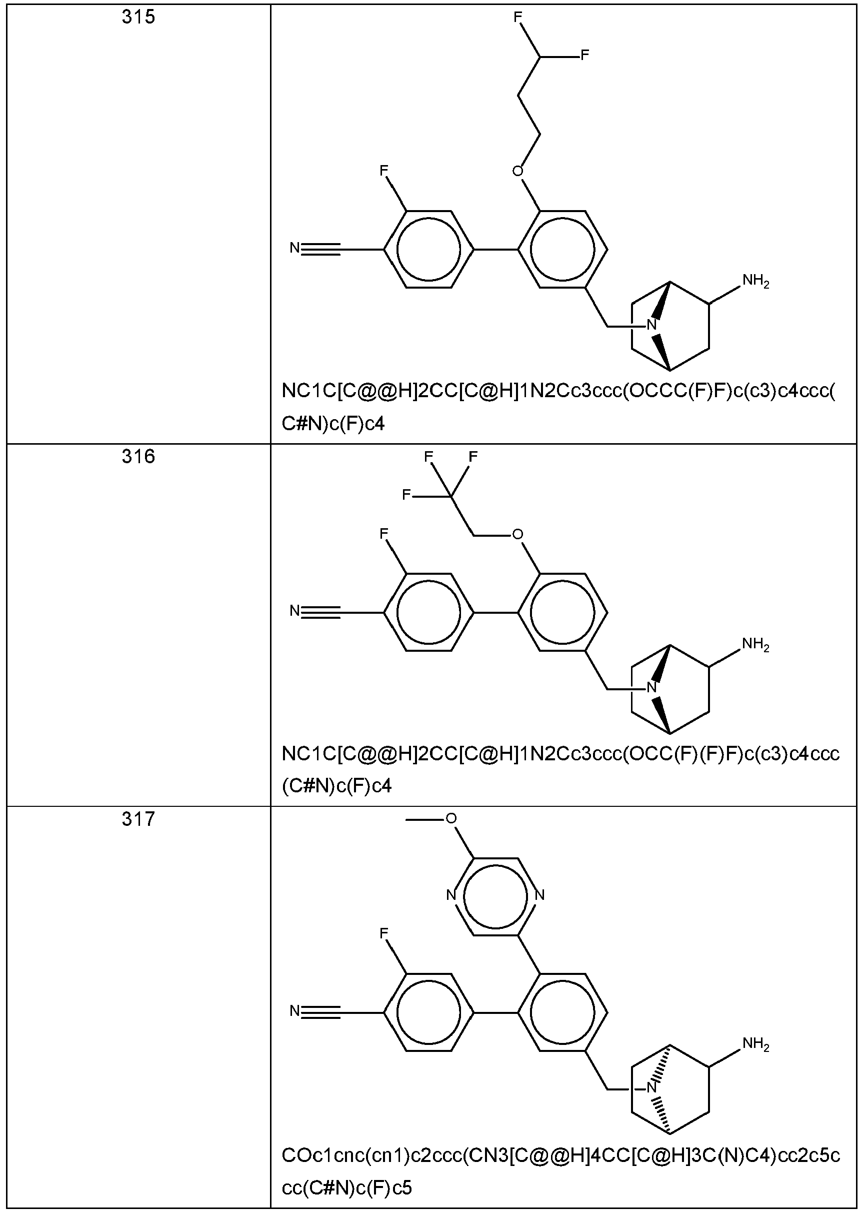

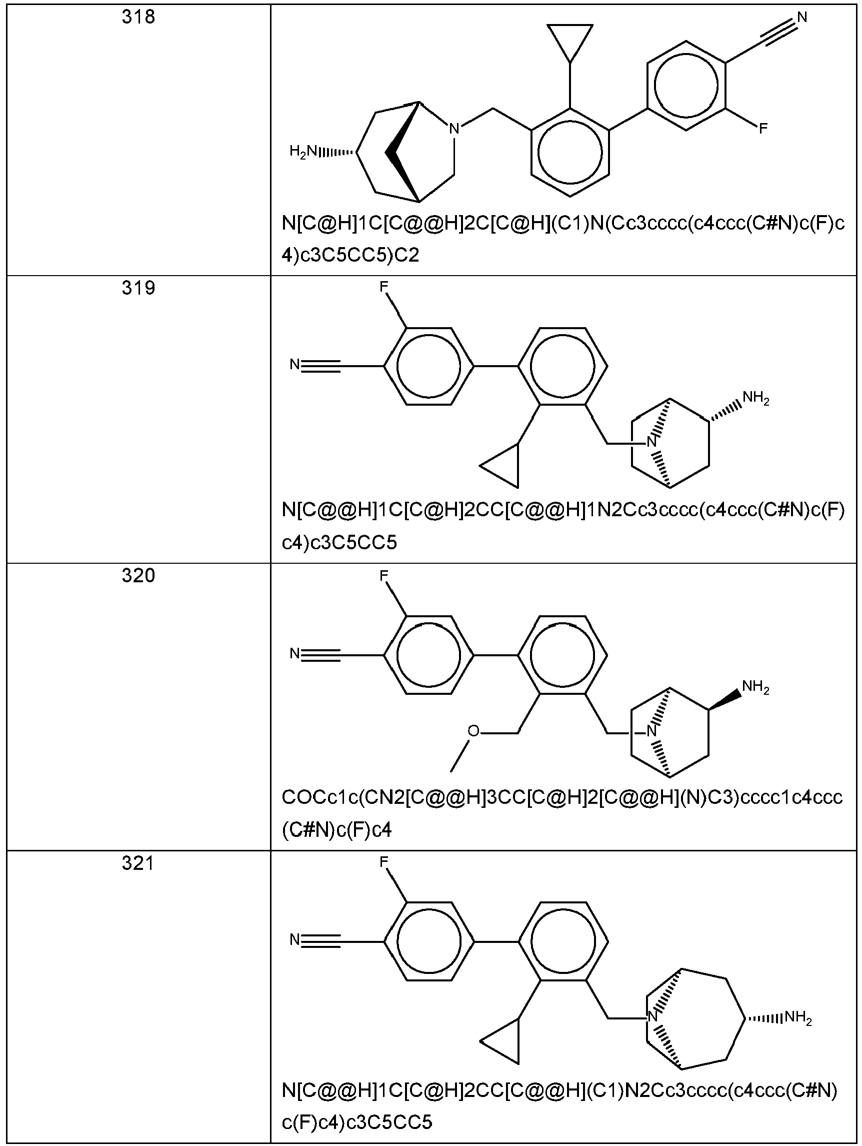

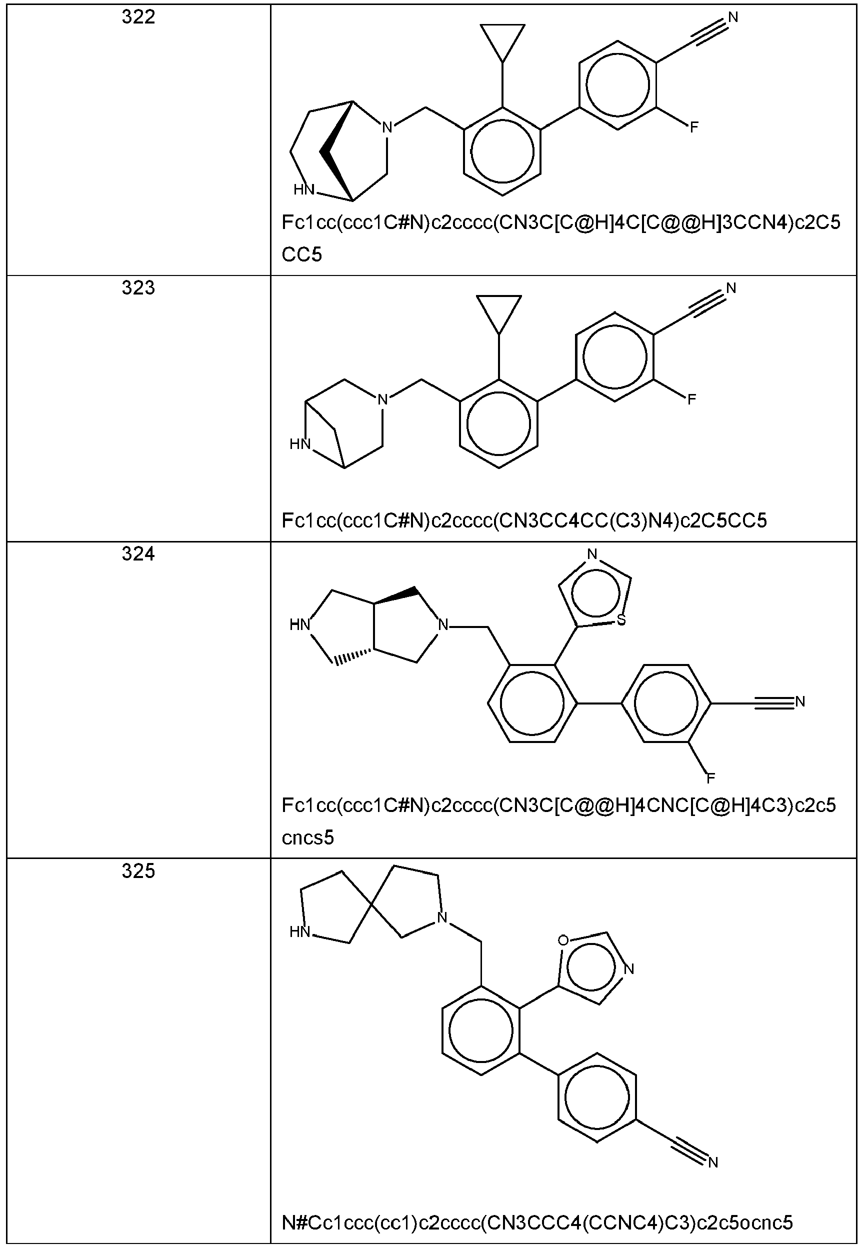

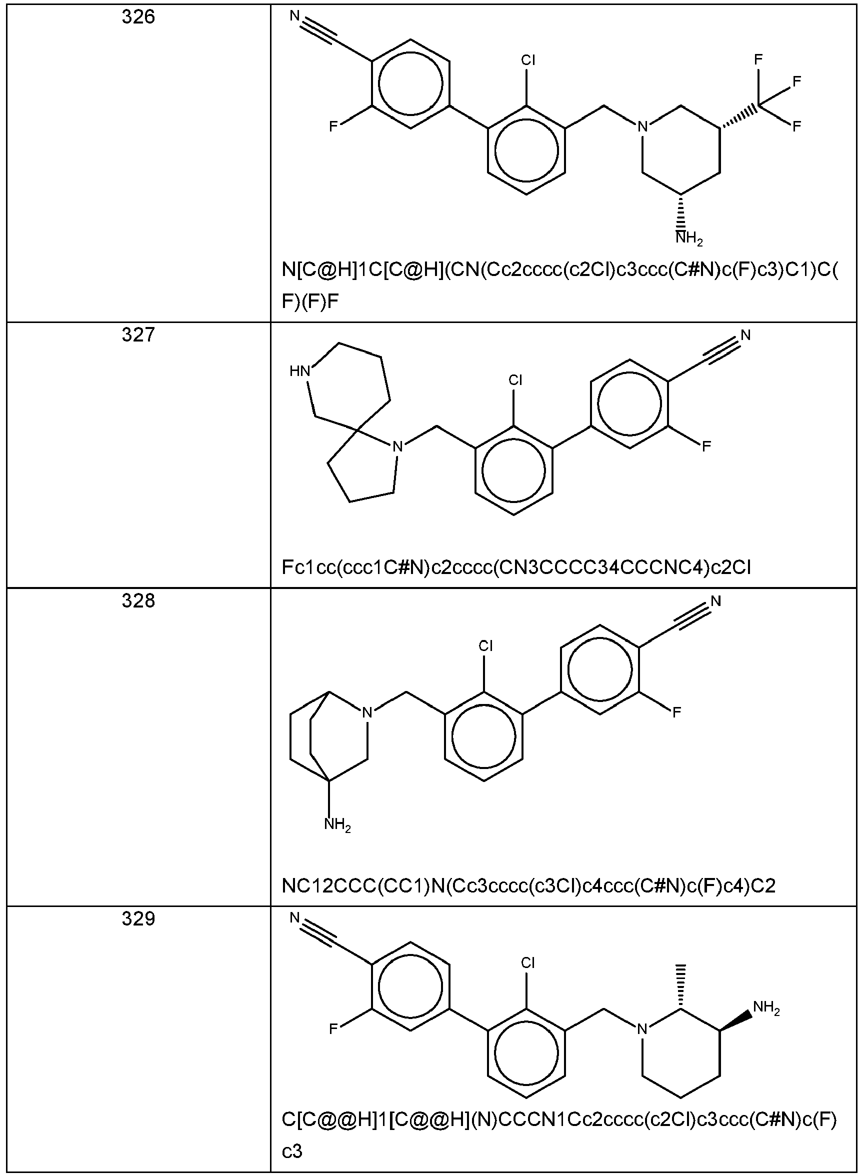

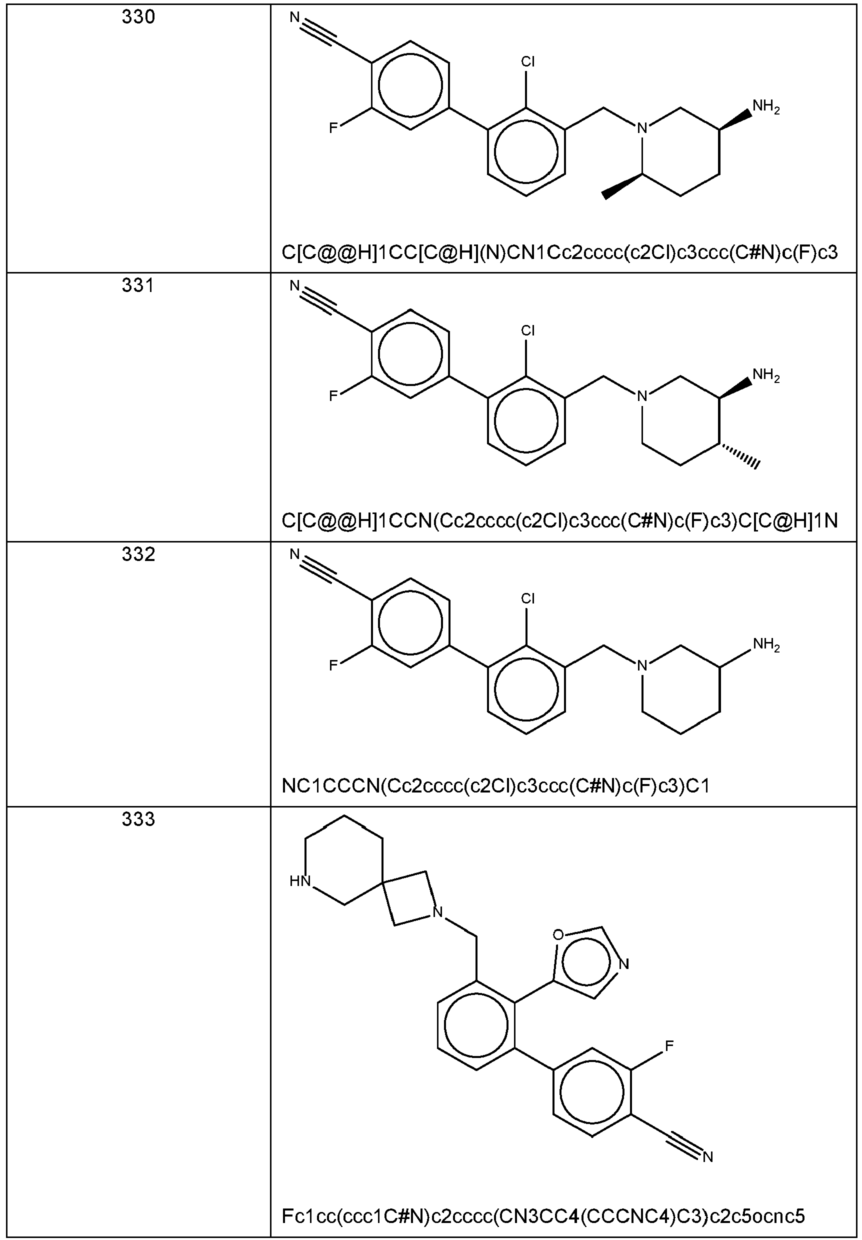

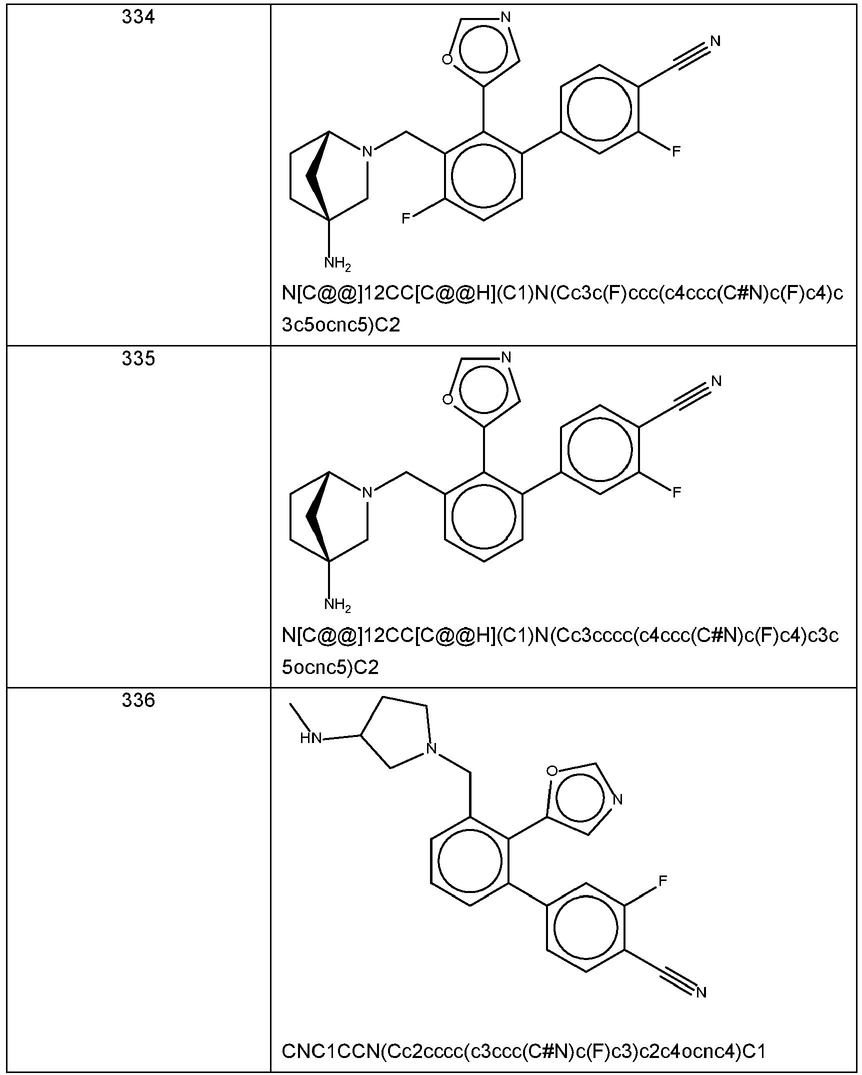

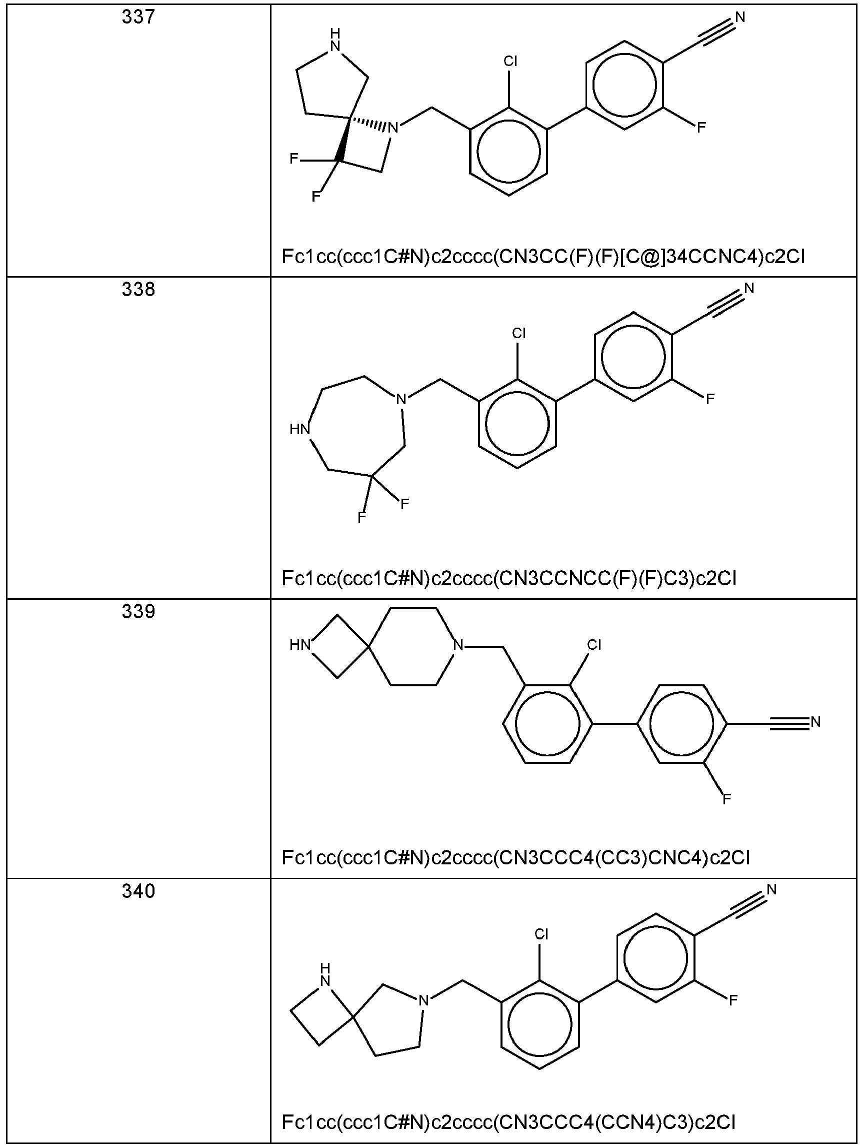

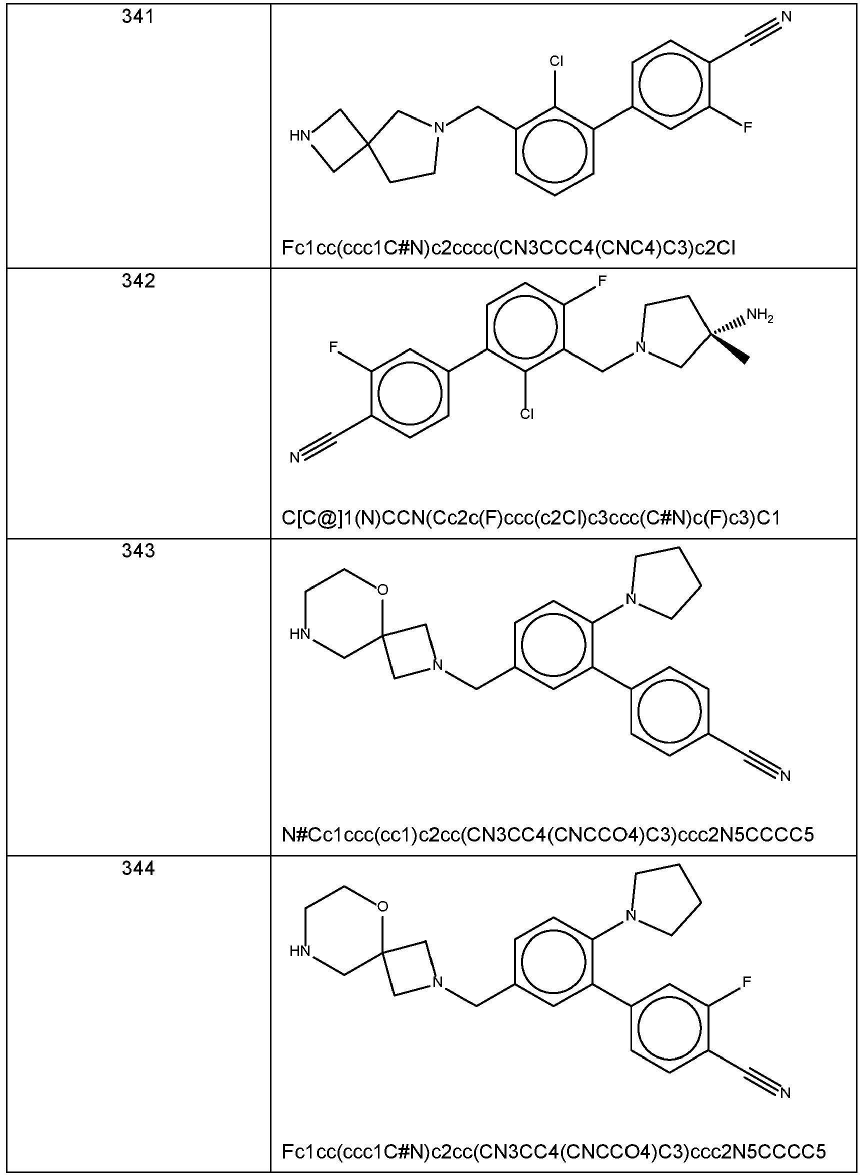

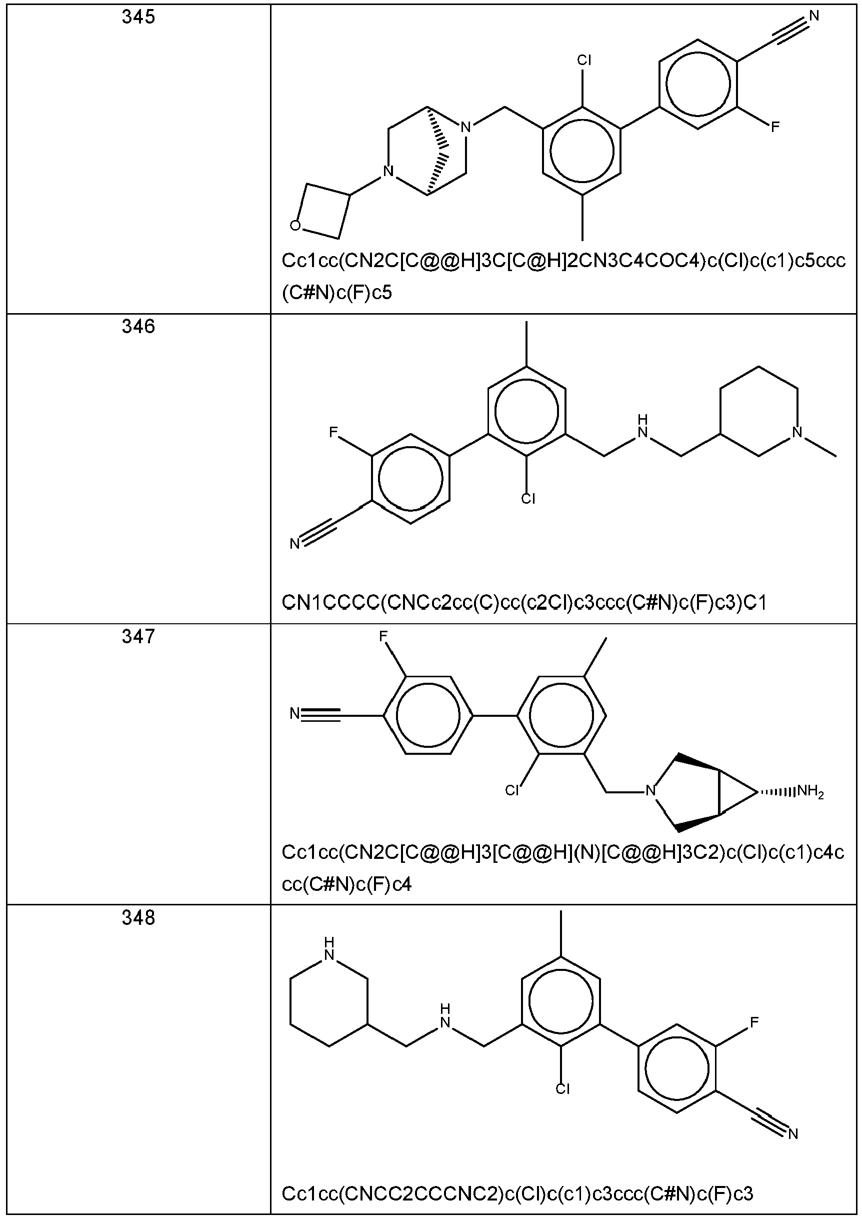

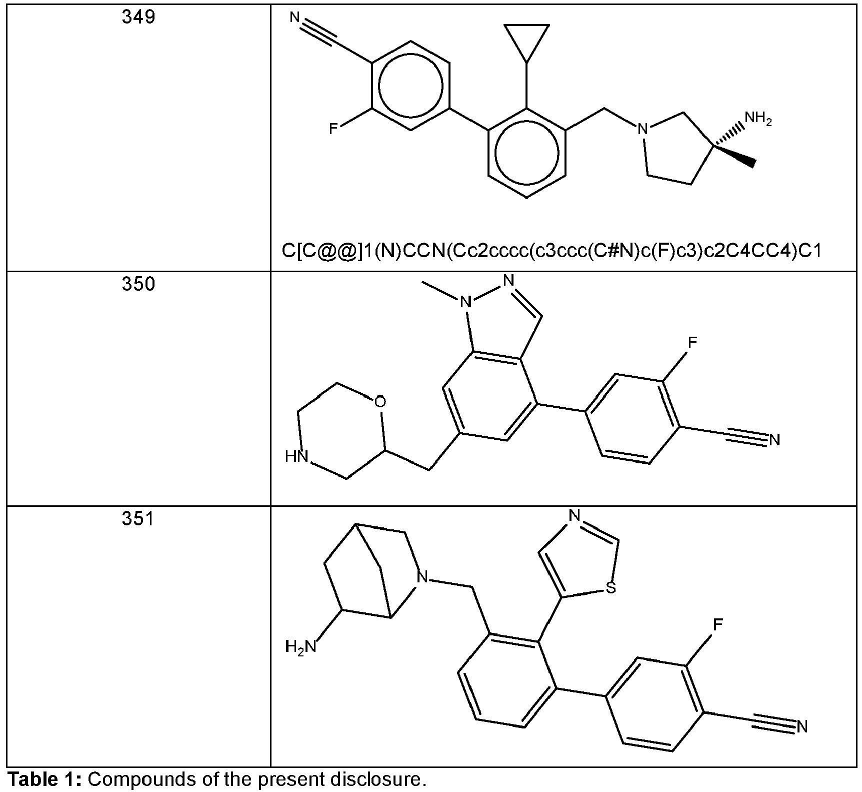

The term 'active agent' is typically used to refer to a compound according to the disclosure which has inhibition activity against LSD1; especially under physiological conditions. However, it is often the case that the active agent may be difficult to administer or deliver to the physiological site of relevance, e.g. due to solubility, half-life or many other chemical or biological reasons. Therefore, it is known to use 'prodrugs' of the active agent in order to overcome physiochemical, biological or other barriers in drug efficiency and/or toxicity. Moreover, prodrug strategy may be used to increase the selectivity of drugs for their intended target. In accordance with the disclosure, therefore, prodrugs may be beneficial in targeting the active agent to the biological sites of interest while advantageously bypassing e.g. the stomach (or lungs), where problematic of inconvenient side-effects may be manifested due to localised inhibition of LSD1 activity.

An active agent may be formed from a compound or prodrug of the disclosure by metabolism of the drug *in vivo,* and/or by chemical or enzymatic cleavage of the prodrug *in vivo.* Typically, a prodrug may be a pharmacologically inactive compound that requires chemical or enzymatic transformation to become an effective, active agent inside the body in which it is intended to have its therapeutic effect. On the other hand, since a prodrug may, in some embodiments, have very close structural similarity to the active agent, in some such embodiments, the prodrug may also have activity against the LSD1 target. This may be particularly the case where the active agent is formed from a compound of prodrug of the disclosure by metabolism or a minor chemical transformation, such that the metabolite is closely related to the parent compound / prodrug. Accordingly, prodrugs of the disclosure may be active inhibitors of LSD1. Suitably, however, such prodrugs may be characterised by having lower inhibition activity against LSD1 than the drug / active agent that is derived from the prodrug of the disclosure.

On the other hand, where the therapeutic effect is derived from the release of the active agent from a larger chemical entity, then the eventual active agent / compound / drug may have significant structural differences compared to the prodrug from which is was derived. In such cases, the prodrug can effectively 'mask' the form(s) of the active agent, and in such cases the prodrug may be completely (or essentially) completely inactive under physiological conditions.

### Dosage Forms, Medicaments and Pharmaceuticals

The compounds, molecules or agents of the disclosure may be used to treat (e.g. cure, alleviate or prevent) one or more diseases, infections or disorders. Thus, in accordance with the disclosure, the compounds and molecules may be manufactured into medicaments or may be incorporated or formulated into pharmaceutical compositions.

The molecules, compounds and compositions of the disclosure may be administered by any convenient route, for example, methods of administration include intradermal, intramuscular, intraperitoneal, intravenous, subcutaneous, intranasal, epidural, oral, sublingual, intranasal, intravaginal, transdermal, rectally, by inhalation, or topically to the skin. Delivery systems are also known to include, for example, encapsulation in liposomes, microgels, microparticles, microcapsules, capsules, etc. Any other suitable delivery system known in the art is also envisioned in use. Administration can be systemic or local. The mode of administration may be left to the discretion of the practitioner.

The dosage administered will, of course, vary depending upon known factors, such as the pharmacodynamic properties of the particular active agent; the chosen mode and route of administration; the age, health and weight of the recipient; the nature of the disease or disorder to be treated; the extent of the symptoms; any simultaneous or concurrent treatments; the frequency of treatment; and the effect desired. In general, a daily dosage of active agent of between about 0.001 and about 1,000 mg/kg of body weight can be expected. For some applications, the dosage may suitably be within the range of about 0.01 to about 100 mg/kg; between about 0.1 to about 25 mg/kg, or between about 0.5 and 10 mg/kg.

Depending on known factors, such as those noted above, the required dosage of the active agent may be administered in a single daily dose, or the total daily dosage may be administered in divided doses of e.g. two, three, or four times daily. Suitably, the therapeutic treatment regime according to the disclosure is devised for a single daily dose or for a divided daily dose of two doses.

Dosage forms of the pharmaceutical compositions of the disclosure suitable for administration may contain from about 1 mg to about 2,000 mg of the active ingredient per unit. Typically, the daily dosage of compounds may be at least about 10 mg and at most about 1,500 mg per human dose; such as between about 25 and 1,250 mg or suitably between about 50 and 1,000 mg. Typically, the daily dosage of compounds may be at most about 1000 mg. In such compositions the compound of the invention will ordinarily be present in an amount of about 0.5-95% by weight based on the total weight of the composition.

The 'effective amount' or 'therapeutically effective amount' is meant to describe an amount of compound or a composition of the disclosure that is effective in curing, inhibiting, alleviating, reducing or preventing the adverse effects of the diseases or disorders to be treated, or the amount necessary to achieve a physiological or biochemically-detectable effect. Thus, at the effective amount, the compound or agent is able to produce the desired therapeutic, ameliorative, inhibitory or preventative effect in relation to disease or disorder. Beneficially, an effective amount of the compound or composition of the disclosure may have the effect of inhibiting LSD1. Diseases or disorders which may benefit from LSD1 inhibition include, for example, autoimmune disorders, inflammatory diseases, cancers and/or oncologic diseases, such as rheumatoid arthritis, multiple sclerosis, psoriasis, Sjogren's syndrome and systemic lupus erythematosus or vasculitic conditions, cancers of hematopoietic origin or solid tumors, including chronic myelogenous leukemia, myeloid leukemia, non-Hodgkin lymphoma and other B cell lymphomas. Further exemplary diseases, disorder or conditions include: lymphoma, including diffuse large B-cell lymphoma (DLBCL), mantle cell lymphoma, non-Hodgkin lymphoma, relapsed or refractory NHL and recurrent follicular lymphoma, Hodgkin lymphoma; leukemia, including acute lymphoblastic leukemia (ALL), acute myelogenous leukemia (AML), acute promyelocytic leukemia (APL), chronic lymphocytic leukemia (CLL), chronic myelogenous leukemia (CML); myeloproliferative disease, including primary myelofibrosis (PMF), polycythemia vera (PV), essential thrombocytosis (ET); myelodysplasia syndrome (MDS), and multiple myeloma; sarcoma including chondrosarcoma, Ewing's sarcoma, osteosarcoma, rhabdomyosarcoma, angiosarcoma, fibrosarcoma, liposarcoma, myxoma, rhabdomyoma, fibroma, lipoma, harmatoma, and teratoma; lung cancer, including non-small cell lung cancer (NSCLC), small cell lung cancer, bronchogenic carcinoma, squamous cell, undifferentiated small cell, undifferentiated large cell, adenocarcinoma, alveolar (bronchiolar) carcinoma, bronchial adenoma, chondromatous hamartoma, and mesothelioma; gastrointestinal cancer, including esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma), and colorectal cancer; genitourinary tract cancer, including cancers of the kidney (adenocarcinoma, Wilm's tumor, nephroblastomal), bladder and urethra (squamous cell carcinoma, transitional cell carcinoma, adenocarcinoma), prostate (adenocarcinoma, sarcoma), and testis (seminoma, teratoma, embryonal carcinoma, teratocarcinoma, choriocarcinoma, sarcoma, interstitial cell carcinoma, fibroma, fibroadenoma, adenomatoid tumors, lipoma); liver cancer, including hepatoma (hepatocellular carcinoma), cholangiocarcinoma, hepatoblastoma, angiosarcoma, hepatocellular adenoma, and hemangioma; bone cancer, including osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma, and giant cell tumors; nervous system cancer, including cancers of the skull (osteoma, hemangioma, granuloma, xanthoma, osteitis deformans), meninges (meningioma, meningiosarcoma, gliomatosis), brain (astrocytoma, meduoblastoma, glioma, ependymoma, germinoma (pinealoma), glioblastoma multiform, oligodendroglioma, schwannoma, retinoblastoma, congenital tumors), and spinal cord (neurofibroma, meningioma, glioma, sarcoma), as well as neuroblastoma and Lhermitte-Duclos disease; gynecological cancer, including cancers of the uterus (endometrial carcinoma), cervix (cervical carcinoma, pre -tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), and fallopian tubes (carcinoma); and skin cancer, including melanoma, basal cell carcinoma, squamous cell carcinoma, Kaposi's sarcoma, moles dysplastic nevi, lipoma, angioma, dermatofibroma, and keloids.

For therapeutic applications, the effective amount or therapeutically effective amount of a compound / active agent of the disclosure may be at least about 50 nM or at least about 100 nM; typically at least about 200 nM or at least about 300 nM in the blood of the subject. The effective amount or therapeutically effective amount may be at most about 5 µM, at most about 3 µM, suitably at most about 2 µM and typically at most about 1 µM in the blood of the subject. For example, the therapeutically effective amount may be at most about 500 nM, such as between about 100 nM and 500 nM. In some embodiments the amount of therapeutic compound is measured in serum of the subject and the above concentrations may then apply to serum concentration of the compounds of the disclosure.

When administered to a subject, a compound of the disclosure is suitably administered as a component of a composition that comprises a pharmaceutically acceptable carrier or vehicle. One or more additional pharmaceutical acceptable carrier (such as diluents, adjuvants, excipients or vehicles) may be combined with the compound of the disclosure in a pharmaceutical composition. Suitable pharmaceutical carriers are described in "*Remington's Pharmaceutical Sciences*" by E. W. Martin. Pharmaceutical formulations and compositions of the disclosure are formulated to conform to regulatory standards and according to the chosen route of administration.

Acceptable pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilising, thickening, lubricating and colouring agents may be used. When administered to a subject, the pharmaceutically acceptable vehicles are generally sterile. Water is a suitable vehicle when the compound is to be administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or buffering agents.

The medicaments and pharmaceutical compositions of the disclosure can take the form of solutions, suspensions, emulsion, tablets, pills, pellets, powders, gels, capsules (for example, capsules containing liquids or powders), modified-release formulations (such as slow or sustained-release formulations), suppositories, emulsions, aerosols, sprays, suspensions, or any other form suitable for use. Other examples of suitable pharmaceutical vehicles are described in Remington's Pharmaceutical Sciences, Alfonso R. Gennaro ed., Mack Publishing Co. Easton, Pa., 19th ed., 1995, see for example pages 1447-1676.

Suitably, the therapeutic compositions or medicaments of the disclosure are formulated in accordance with routine procedures as a pharmaceutical composition adapted for oral administration (more suitably for humans). Compositions for oral delivery may be in the form of tablets, lozenges, aqueous or oily suspensions, granules, powders, emulsions, capsules, syrups, or elixirs, for example. Thus, in one embodiment, the pharmaceutically acceptable vehicle is a capsule, tablet or pill.

Orally administered compositions may contain one or more agents, for example, sweetening agents such as fructose, aspartame or saccharin; flavouring agents such as peppermint, oil of wintergreen, or cherry; colouring agents; and preserving agents, to provide a pharmaceutically palatable preparation. When the composition is in the form of a tablet or pill, the compositions may be coated to delay disintegration and absorption in the gastrointestinal tract, so as to provide a sustained release of active agent over an extended period of time. Selectively permeable membranes surrounding an osmotically active driving compound are also suitable for orally administered compositions. In these dosage forms, fluid from the environment surrounding the capsule is imbibed by the driving compound, which swells to displace the agent or agent composition through an aperture. These dosage forms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, etc. Such vehicles are preferably of pharmaceutical grade. For oral formulations, the location of release may be the stomach, the small intestine (the duodenum, the jejunem, or the ileum), or the large intestine. One skilled in the art is able to prepare formulations that will not dissolve in the stomach yet will release the material in the duodenum or elsewhere in the intestine. Suitably, the release will avoid the deleterious effects of the stomach environment, either by protection of the compound (or composition) or by release of the compound (or composition) beyond the stomach environment, such as in the intestine. To ensure full gastric resistance a coating impermeable to at least pH 5.0 would be essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac, which may be used as mixed films.

While it can be beneficial to provide therapeutic compositions and/or compounds of the disclosure in a form suitable for oral administration, for example, to improve patient compliance and for ease of administration, in some embodiments compounds or compositions of the disclosure may cause undesirable side-effects, such as intestinal inflammation which may lead to premature termination of a therapeutic treatment regime. Thus, in some embodiments, the therapeutic treatment regime is adapted to accommodate `treatment holidays', e.g. one or more days of non-administration. For example, treatment regimens and therapeutic methods of the disclosure may comprise a repetitive process comprising administration of the therapeutic composition or compound for a number of consecutive days, followed by a treatment holiday of one or more consecutive days. For example, a treatment regime of the disclosure may comprise a repetitive cycle of administration of the therapeutic composition or compound for between 1 and 49 consecutive days, between 2 and 42 days, between 3 and 35 days, between 4 and 28 days, between 5 and 21 days, between 6 and 14 days, or between 7 and 10 days; followed by a treatment holiday of between 1 and 14 consecutive days, between 1 and 12 days, between 1 and 10 days, or between 1 and 7 days (e.g. 1, 2, 3, 4, 5, 6 or 7 days).

To aid dissolution of the therapeutic agent into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. Potential nonionic detergents that could be included in the formulation as surfactants include: lauromacrogol 400, polyoxyl 40 stearate, polyoxyethylene hydrogenated castor oil 10, 50 and 60, glycerol monostearate, polysorbate 20, 40, 60, 65 and 80, sucrose fatty acid ester, methyl cellulose and carboxymethyl cellulose. These surfactants, when used, could be present in the formulation of the compound or derivative either alone or as a mixture in different ratios.

Typically, compositions for intravenous administration comprise sterile isotonic aqueous buffer. Where necessary, the compositions may also include a solubilising agent.

Another suitable route of administration for the therapeutic compositions of the disclosure is via pulmonary or nasal delivery.

Additives may be included to enhance cellular uptake of the therapeutic agent of the disclosure, such as the fatty acids oleic acid, linoleic acid and linolenic acid.

The therapeutic agents of the disclosure may also be formulated into compositions for topical application to the skin of a subject.

Where the invention provides more than one active compound / agent for use in combination, generally, the agents may be formulated separately or in a single dosage form, depending on the prescribed most suitable administration regime for each of the agents concerned. When the therapeutic agents are formulated separately, the pharmaceutical compositions of the invention may be used in a treatment regime involving simultaneous, separate or sequential administration with the other one or more therapeutic agent. The other therapeutic agent(s) may comprise a compound of the disclosure or a therapeutic agent known in the art).

The compounds and/or pharmaceutical compositions of the disclosure may be formulated and suitable for administration of the compound to the central nervous system (CNS) and/or for crossing the blood-brain barrier (BBB).

The invention will now be described by way of the following non-limiting examples.

### EXAMPLES

### Chemical Synthesis

The following Tables 2 and 3 identify specific compounds of this disclosure and the reaction steps (procedure) used for their synthesis, as well as compound yield and characterisation data. Exemplary reaction steps and conditions are illustrated further below.

**Table 2: Compounds of the disclosure and procedures used in their synthesis (Series 1).**

| **SERIES 1** | | | | |
|---|---|---|---|---|
| Example 44 | Procedure: A1+B2+D1 | SM1: BB-2 | SM2: azetidine | Yield: 50% |
| 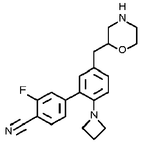 | 1H NMR (DMSO-d6, 600 MHz) δ 7.9-8.0 (m, 1H), 7.50 (dd, 1H, J=1.4, 10.6 Hz), 7.42 (dd, 1H, J=1.5, 8.0 Hz), 7.12 (dd, 1H, J=2.0, 8.3 Hz), 6.95 (d, 1H, J=1.9 Hz), 6.56 (d, 1H, J=8.2 Hz), 3.6-3.8 (m, 1H), 3.43 (t, 5H, J=7.3 Hz), 3.3-3.4 (m, 1H), 2.5-2.7 (m, 6H), 2.35 (dd, 1H, J=10.0, 12.2 Hz), 2.0-2.1 (m, 2H). m/z: 352 [M+H]+ | | | |
| 2'-(azetidin-1-yl)-3-fluoro-5'-[(morpholin-2-yl)methyl]-[1,1'-biphenyl]-4-carbonitrile | | | | |

| Example 102 | Procedure: A1+B2+D1 | SM1: BB-2H | SM2: azetidine | Yield: 100% |
|---|---|---|---|---|
| 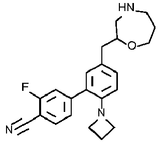 | 1H NMR (600 MHz, DMSO-d6): δ ppm 7.89 - 7.95 (m, 1 H) 7.46 - 7.54 (m, 1 H) 7.39 - 7.45 (m, 1 H) 7.10 - 7.17 (m, 1 H) 6.92 - 7.01 (m, 1 H) 6.52-6.59 (m, 1 H) 3.78 (m, J=12.20, 5.30, 5.30 Hz, 1 H) 3.49 - 3.59 (m, 2 H) 3.43 (t, J=7.26 Hz, 4 H) 3.32 - 3.39 (m, 1 H) 2.83 - 2.91 (m, 2 H) 2.51 - 2.69 (m, 3 H) 2.36 - 2.45 (m, 1 H) 2.00 - 2.15 (m, 2 H) 1.57 - 1.79 (m, 2 H). m/z: 366 [M+H]+ | | | |
| 2'-(azetidin-1-yl)-3-fluoro-5'-[(1,4-oxazepan-2-yl)methyl]-[1,1'-biphenyl]-4-carbonitrile | | | | |

| Example 147 | Procedure: A1+B2+D1 | SM1: BB-2 | SM2: pyrrolidine | Yield: 60% |
|---|---|---|---|---|
| 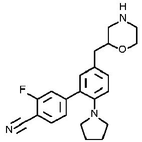 | 1H NMR (DMSO-d6, 500 MHz): δ (ppm) 7.91 (t, J = 7.6 Hz, 1H), 7.53 (dd, J = 10.8, 1.2 Hz, 1H), 7.43 (dd, J = 8.1, 1.5 Hz, 1H), 7.11 (dd, J = 8.3, 2.2 Hz, 1H), 7.00 (d, J = 2.2 Hz, 1H), 6.88 (d, J = 8.3 Hz, 1H), 3.61-3.72 (m, 1H), 3.41-3.47 (m, 1H), 3.32-3.38 (m, 2H), 3.17 (brs, 1H), 2.78-2.90 (m, 4H), 2.70 (dd, J = 12.0, 2.0 Hz, 1H), 2.51-2.65 (m, 4H), 2.34 (dd, J = 12.1, 9.9 Hz, 1H), 1.67-1.81 (m, 4H). m/z: 366 [M+H]+ | | | |
| 3-fluoro-5'-[(morpholin-2-yl)methyl]-2'-(pyrrolidin-1 -yl)-[1,1'-biphenyl]-4-carbonitrile | | | | |

| Example 146 | Procedure: A1+B2 | SM1: BB-2D | SM2: azetidine | Yield: 44% |
|---|---|---|---|---|
| 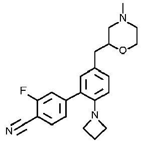 | 1H NMR (DMSO-d6, 600 MHz): δ (ppm) 7.92 (dd, J = 7.8, 7.3 Hz, 1H), 7.51 (dd, J = 10.6, 1.4 Hz, 1H), 7.42 (dd, J = 8.1, 1.5 Hz, 1H), 7.13 (dd, J = 8.2, 2.1 Hz, 1H), 6.96 (d, J = 2.1 Hz, 1H), 6.56 (d, J = 8.4 Hz, 1H), 3.67-3.75 (m, 1H), 3.51-3.60 (m, 1H), 3.38-3.47 (m, 5H), 2.53-2.68 (m, 4H), 2.12 (s, 3H), 2.08 (quin, J = 7.3 Hz, 2H), 1.92 (td, J = 11.3, 3.3 Hz, 1H), 1.69 (dd, J = 11.0, 10.0 Hz, 1H). m/z: 366 [M+H]+ | | | |
| 2'-(azetidin-1-yl)-3-fluoro-5'-[(4-methylmorpholin-2-yl)methyl]-[1,1'-biphenyl]-4-carbonitrile | | | | |

| Example 145 | Procedure: L2+B2+D2 | SM1: BB-2 | SM2: oxolane | Yield: 64% |
|---|---|---|---|---|
| 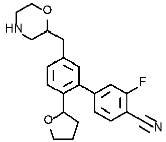 | 1H NMR (500 MHz, DMSO-d6) δ ppm 7.94 - 8.03 (m, 1 H) 7.53 (dt, J=10.45, 1.25 Hz, 1 H) 7.47 (d, J=7.82 Hz, 1 H) 7.37 (dt, J=8.10, 2.00 Hz, 1 H) 7.27 - 7.33 (m, 1 H) 7.01 - 7.08 (m, 1 H) 4.66 (td, J=7.46, 3.91 Hz, 1 H) 3.89 - 4.01 (m, 1 H) 3.60 - 3.74 (m, 2 H) 3.46 - 3.56 (m, 1 H) 3.33 - 3.40 (m, 1 H) 2.77 - 2.88 (m, 1 H) 2.55 - 2.76 (m, 5 H) 2.30 - 2.41 (m, 1 H) 1.97 - 2.07 (m, 1 H) 1.76 - 1.97 (m, 2 H) 1.58 - 1.71 (m, 1 H). m/z: 367 [M+H]+ | | | |
| 3-fluoro-5'-[( (morpholin-2-yl)methyl]--2'-(oxolan-2-yl)-[1,1'-biph enyl]-4-carbonitrile | | | | |

| Example 54 | Procedure: A1+B2+Chiral Separation+D1 | SM1: BB-2 | SM2: azetidine | Yield: 68% |
|---|---|---|---|---|
| 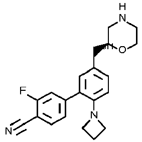 | 1H NMR (DMSO-d6, 500 MHz): δ (ppm) 7.92 (t, J = 7.6 Hz, 1H), 7.50 (dd, J = 10.8, 1.2 Hz, 1H), 7.42 (dd, J = 8.1, 1.5 Hz, 1H), 7.12 (dd, J = 8.3, 2.0 Hz, 1H), 6.95 (d, J = 2.0 Hz, 1H), 6.56 (d, J = 8.3 Hz, 1H), 3.61-3.76 (m, 1H), 3.40-3.51 (m, 5H), 3.33-3.40 (m, 1H), 2.70 (dd, J = 12.2, 2.0 Hz, 1H), 2.52-2.65 (m, 5H), 2.34 (dd, J = 12.1, 9.9 Hz, 1H), 2.02-2.14 (m, 2H). m/z: 352 [M+H]+ | | | |
| 2'-(azetidin-1-yl -3-fluoro-5'-{[(2S)-mor pholin-2-yl]methyl}-[1,1'-biphenyl]-4-carbonitrile | | | | |

| Example 41 | Procedure: A1+B2+Chiral Separation+D1 | SM1: BB-2 | SM2: azetidine | Yield: 96% |
|---|---|---|---|---|
| 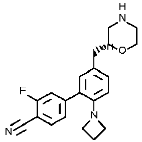 | 1H NMR (500 MHz, J=1 0.51, 1.47 Hz, 1 6.90 - 6.99 (m, 1 H) 3.39 - 3.49 (m, 5 H) J=11.98, 1.96 Hz, 1 H) DMSO-d6) δ ppm 7.85 H) 7.42 (dd, J=8.07, 1.47 6.56 (d, J=8.31 Hz, 1 H) 3.33 - 3.39 (m, 1 H) 2.73 H) 2.52 - 2.66 (m, 4 H) 2.02 - 2.18 (m, 2 H). m/z: - 7.98 (m, 1 H) 7.50 Hz, 1 H) 7.08 - 7.16 (m, 3.67 (m, J=10.30, 2.00 - 3.01 (m, 1 H) 2.71 2.35 (dd, J=12.23, 10.03 352 [M+H]+ (dd, 1 H) Hz, 1 H) (dd, Hz, 1 | | | |
| 2'-(azetidin-1-yl )-3-fluoro-5'-{[(2R)-mor pholin-2-yl]methyl}-[1,1'-biphenyl]-4-carbonitrile | | | | |

| Example 21 | Procedure: A1+B2+D1 | SM1: BB-1 | SM2: azetidine | Yield: 42% |
|---|---|---|---|---|
| 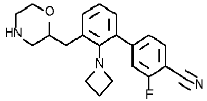 | 1H NMR (DMSO-d6, 500 MHz): δ (ppm) 7.89 (t, J = 7.5 Hz, 1H), 7.43 (dd, J = 10.6, 1.3 Hz, 1H) 7.29 (dd, J = 8.1, 1.5 Hz, 1H), 7.10 (dd, J = 7.5, 1.3 Hz, 1H), 6.94 (dd, J = 7.6, 1.5 Hz, 1H), 6.76 (t, J = 7.6 Hz, 1H), 3.69 (br d, J = 10.5 Hz, 1H), 3.48-3.64 (m, 5H), 3.41 (td, J = 10.7, 3.5 Hz, 1H), 2.78 (dd, J = 14.8, 6.7 Hz, 1H), 2.57-2.69 (m, 4H), 2.50 (br s, 1H), 2.34 (dd, J = 12.2, 10.0 Hz, 1H), 1.97 (quin, J= 7.5 Hz, 2H). m/z: 352 [M+H]+ | | | |
| 2'-(azetidin-1--yl)-3-fluoro-3'-[(morp holin-2-yl)methyl]-[1,1'-bi phenyl]-4-carbonitrile | | | | |

| Example 144 | Procedure: G1+G0+I1+G3+A1+ B2+D1 | SM1: 2-(4-bromo-3-chlorophenyl)propanal | SM2: trimethyl(oxo)-λ⁶-sulfanylium iodide | Yield: 51% |
|---|---|---|---|---|
| 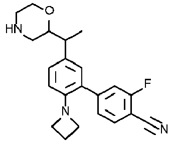 | 1H NMR (DMSO-d6, 600 MHz): δ (ppm) 7.92 (t, J = 7.5 Hz, 1H), 7.50 (dd, J = 10.7, 1.3 Hz, 1H), 7.42 (dd, J = 8.1, 1.5 Hz, 1H), 7.14 (dd, J = 8.5, 2.1 Hz, 1H), 6.94 (d, J = 2.1 Hz, 1H), 6.56 (d, J = 8.4 Hz, 1H), 3.63 (br d, J =11.0 Hz, 1H), 3.44 (t, J = 7.3 Hz, 4H), 3.31-3.39 (m, 2H), 2.78 (br d, J = 10.4 Hz, 1H), 2.19-2.45 (m, 1H), 2.08 (q, J = 8.2 Hz, 2H), 1.12 (d, J = 7.2 Hz, 3H). m/z: 366 [M+H]+ | | | |
| 2'-(azetidin-1--yl)-3-fluoro-5'-[1-(mor pholin-2-yl)ethyl]-[1,1'-bip phenyl]-4-carbonitrile | | | | |

| Example 168 | Procedure: A1+B2+D1 | SM1: BB-2 | SM2: piperidine | Yield: 11% |
|---|---|---|---|---|
| 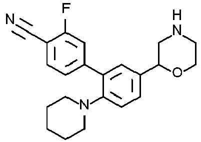 | 1H NMR (DMSO-d6, 1H), 7.78-7.83 (m, Hz, 1H), 7.29 (d, J = 1.8 Hz, 1H), 4.10 (dd, 3.41 (brd, J = 12.6 2.72 (br 600 MHz): δ (ppm) 8.98 1H), 7.70 (dd, J = 8.1, 1.3 2.1 Hz, 1H), 7.13 (d, J = J = 12.7, 3.2 Hz, 1H), Hz, 1H), 3.25 (brd, J = s, 4H), 1.42 (br s, 6H). (brs, 2H), 7.99 (t, J = Hz, 1H), 7.37 (dd, J = 8.4 Hz, 1H), 4.66 (dd, J 3.84 (td, J = 12.4, 2.0 Hz, 12.5 Hz, 1H), 3.04-3.15 (m, m/z: 366 [M+H]+ 7.6 Hz, 8.3, 2.0 = 11.2, 1H), 2H), | | | |
| 3-fluoro-5' '-(morpholin-2-yl)-2'-( piperidin-1-yl)-[1,1'-biphe nyl]-4-carbonitrile | | | | |

| Example 167 | Procedure: A1+B2+D2 | SM1: BB-2 | SM2: morpholine | Yield: 90% |
|---|---|---|---|---|
| 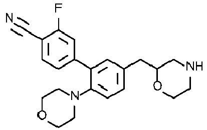 | 1H NMR (DMSO-d6, 7.80 (dd, J = 11.0, 8.3, 2.2 Hz, 1H), 7.1 J = 12.7, 3.4 Hz, 3.47-3.55 (m, 4H), 500 MHz): δ (ppm) 9.04 .2 Hz, 1H), 7.72 (dd, J = 9 (d, J = 2.0 Hz, 1H), 7.1 1H), 3.80-3.89 (m, 1H), 3.66 3.16 (br t, J = 11.4 Hz, 2H), (m, 7H). m/z: 382 [M+H]+ (br s, 2H), 7.93-8.03 (m, 8.1, 1.5 Hz, 1H), 7.26 ( 0 (d, J = 8.1 Hz, 1H), 3. (td, J = 12.5, 2.2 Hz, 2.88-3.01 (m, 1H), 2.6 1H), dd, J = 93 (dd, 1H), 6-2.84 | | | |
| 3-fluoro-5'-[( morpholin-2-yl)methyl]-2 '-(morpholin-4-yl)-[1,1'-bi phenyl]-4-carbonitrile | | | | |

| Example 143 | Procedure: A1+B2+D2 | SM1: BB-2 | SM2: 1,4-oxazepane | Yield: 90% |
|---|---|---|---|---|
| 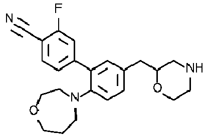 | 1H NMR (500 MHz, H) 7.69 (dd, J=10.88, 7.24 (m, 1 H) 7.14 - H) 3.83 - 3.89 (m, 1 (m, 2 H) 3.10 - 3.19 (d, J=6.36 DMSO-d6) δ ppm 8.64 - 1.34 Hz, 1 H) 7.58 (dd, 7.17 (m, 1 H) 7.12 (d, J= H) 3.65 - 3.73 (m, 1 H) (m, 2 H) 2.98 - 3.06 (m, Hz, 3 H) 1.54 - 1.68 (m, 2 9.44 (m, 2 H) 7.89 - 8.0 J=8.07, 1.47 Hz, 1 H) 2.20 Hz, 1 H) 3.91 - 3.9 3.60 - 3.64 (m, 2 H) 3.48 4 H) 2.87 - 2.97 (m, 1 H) H). m/z: 396 [M+H]+ 3 (m, 1 7.18 - 5 (m, 1 - 3.53 2.74 | | | |
| 3-fluoro-5'-[(mo rpholin-2-yl)methyl]-2'-( 1,4-oxazepan-4-yl)-[1,1' -biphenyl]-4-carbonitrile | | | | |

| Example 164 | Procedure: A1+B2+D2 | SM1: BB-2 | SM2: 1-ethylpiperazine | Yield: 62% |
|---|---|---|---|---|
| 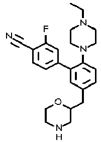 | 1H NMR (600 MHz, DMSO-d6, 300K) δ ppm 7.96 (t, J=7.5 Hz, 1 H), 7.79 (dd, J=11.2, 1.3 Hz, 1 H), 7.67 (dd, J=8.1, 1.5 Hz, 1 H), 7.20 (dd, J=8.3, 2.1 Hz, 1 H), 7.13 (d, J=2.1 Hz, 1 H), 7.05 (d, J=8.2 Hz, 1 H), 3.67 (br d, J=11.0 Hz, 1 H), 3.43 - 3.57 (m, 1 H), 3.35 (td, J=10.7, 3.0 Hz, 1 H), 2.54 - 2.83 (m, 10H), 2.10 - 2.45 (m, 7 H), 0.96 (t, J=7.2 Hz, 3 H). m/z: 409 [M+H]+ | | | |
| 2'-(4-ethylpiperazin-1 -yl)-3-fluoro-5'-[(morpholin-2-yl)methyl]-[1,1'-biphenyl]-4-carbonitrile | | | | |

| Example 163 | Procedure: A1+B2+D1+G6 | SM1: BB-2 | SM2: azetidine | Yield: 32% |
|---|---|---|---|---|
| 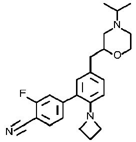 | 1H NMR (DMSO-d6, 500 MHz): δ (ppm) 7.92 (t, J = 7.6 Hz, 1H), 7.51 (dd, J = 10.8, 1.2 Hz, 1H), 7.42 (dd, J = 8.1, 1.5 Hz, 1H), 7.13 (dd, J = 8.3, 2.0 Hz, 1H), 6.97 (d, J = 2.0 Hz, 1H), 6.56 (d, J = 8.3 Hz, 1H), 3.64-3.82 (m, 1H), 3.48-3.57 (m, 1H), 3.43 (t, J = 7.3 Hz, 4H), 3.38 (td, J = 11.1, 2.3 Hz, 1H), 2.51-2.69 (m, 5H), 2.15 (td, J = 11.2, 3.1 Hz, 1H), 2.04-2.11 (m, 2H), 1.91-1.98 (m, 1H), 0.90-0.96 (m, 6H). m/z: 394 [M+H]+ | | | |
| 2'-(azetidin-1-yl)-3-fluoro-5'-{[4-(propan-2-yl)morpholin-2-yl]methyl}-[1,1'-biphenyl]-4-carbonitrile | | | | |

| Example 50 | Procedure: A1+B2+D1 | SM1: BB-1 | SM2: azetidine | Yield: 27% |
|---|---|---|---|---|
| 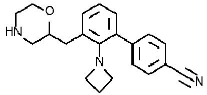 | 1H NMR (500 MHz, DMSO-d6) Shift 7.79-7.84 (m, 2H), 7.44-7.49 (m, 2H), 7.07 (dd, J=1.47, 7.58 Hz, 1H), 6.90 (dd, J=1.47, 7.58 Hz, 1H), 6.75 (t, J=7.58 Hz, 1H), 3.69 (brd, J=10.51 Hz, 1H), 3.58-3.66 (m, 1H), 3.44-3.54 (m, 4H), 3.39-3.44 (m, 1H), 2.78 (dd, J=6.72, 14.79 Hz, 1H), 2.57-2.68 (m, 4H),2.36-2.99 (brs 1H), 2.34 (dd, J=9.90, 12.10 Hz, 1H), 1.90-2.01 (m, 2H). m/z: 334 [M+H]+ | | | |
| 2'-(azetidin-1-yl)-3'-[(morpholin-2-yl)methyl]-[1,1'-biphenyl]-4-carbonitrile | | | | |

| Example 162 | Procedure: B1+M1+B0+H0+G0 +I1+G3+B2+D2 | SM1: 4-bromo-2-chloro-1-iodobenzene | SM2: (cyclopent-1-en-1-yl)boronic acid | Yield: 83% |
|---|---|---|---|---|
| 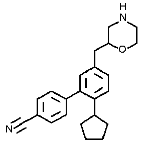 | 1H NMR (DMSO-d6, 500 MHz): δ (ppm) 7.90 (d, J = 8.3 Hz, 2H), 7.49 (d, J = 8.3 Hz, 2H), 7.36 (d, J = 8.1 Hz, 1H), 7.23 (dd, J = 8.1, 2.0 Hz, 1H), 6.97 (d, J = 2.0 Hz, 1H), 3.67 (dd, J = 10.1,2.1 Hz, 1H), 3.44-3.61 (m, 1H), 3.36 (td, J = 10.7, 3.5 Hz, 2H), 2.77-2.90 (m, 1H), 2.73 (dd, J = 12.2, 2.2 Hz, 1H), 2.55-2.68 (m, 4H), 2.37 (dd, J = 12.2, 10.0 Hz, 1H), 1.66-1.88 (m, 4H), 1.41-1.60 (m, 4H). m/z: 347 [M+H]+ | | | |
| 2'-cyclopentyl-5'-[(morpholin-2-yl)methyl]-[1,1'-biphenyl]-4-carbonitrile | | | | |

| Example 161 | Procedure: B1+D2 | SM1: BB-2C | SM2: 4-boranylbenzonitrile | Yield: 54% |
|---|---|---|---|---|
| 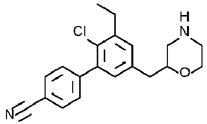 | 1H NMR (DMSO-d6, 600 MHz): δ (ppm) 9.01 (br s, 2H), 7.93-7.96 (m, 2H), 7.58-7.64 (m, 2H), 7.30 (d, J = 2.1 Hz, 1H), 7.13 (d, J = 2.1 Hz, 1H), 3.93 (dd, J = 12.6, 3.4 Hz, 1H), 3.86-3.91 (m, 1H), 3.66 (td, J = 12.5, 2.3 Hz, 1H), 3.11-3.21 (m, 2H), 2.94 (td, J = 12.5, 3.9 Hz, 1H), 2.74-2.82 (m, 5H), 1.21 (t, J = 7.5 Hz, 3H). m/z: 341 [M+H]+ | | | |
| 2'-chloro-3'-ethyl-5'-[(morpholin-2-yl)methyl]-[1,1'-biphenyl]-4-carbonitrile | | | | |

| Example 142 | Procedure: G7+B2+D1 | SM1: BB-2 | SM2: sodium methanolate | Yield: 45% |
|---|---|---|---|---|
| 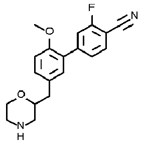 | 1H NMR (500 MHz, DMSO-d6) δ (ppm) 7.94 (dd, J = 8.1, 7.3 Hz, 1H), 7.65 (dd, J = 11.0, 1.5 Hz, 1H), 7.54 (dd, J = 8.1, 1.5 Hz, 1H), 7.22-7.27 (m, 2H), 7.08 (d, J = 8.6 Hz, 1H), 3.78 (s, 3H), 3.62-3.71 (m, 1H), 3.42-3.55 (m, 1H), 3.35 (td, J = 10.6, 3.8 Hz, 1H), 2.55-2.74 (m, 5H), 2.18-2.39 (m, 2H). m/z: 327 [M+H]+ | | | |
| 3-fluoro-2'-methoxy-5'-[(morpholin-2-yl)methyl]-[1,1'-biphenyl]-4-carbonitrile | | | | |

| Example 141 | Procedure: B7+D1 | SM1: BB-2 | SM2: potassium cyclopropyltrifluorobor anuide | Yield: 70% |
|---|---|---|---|---|
| 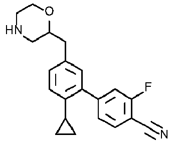 | 1H NMR (DMSO-d6, 500 MHz): δ (ppm) 7.95-8.06 (m, 1H), 7.60 (dd, J = 10.5, 1.5 Hz, 1H), 7.47 (dd, J = 7.9, 1.6 Hz, 1H), 7.19 (dd, J = 7.9, 1.8 Hz, 1H), 7.08 (d, J = 2.0 Hz, 1H), 6.98 (d, J = 8.1 Hz, 1H), 3.66 (dt, J = 10.6, 1.8 Hz, 1H), 3.43-3.56 (m, 1H), 3.33-3.38 (m, 1H), 2.53-2.79 (m, 6H), 2.36 (dd, J = 12.1, 9.9 Hz, 1H), 1.78 (tt, J = 8.4, 5.3 Hz, 1H), 0.74-0.91 (m, 2H), 0.53-0.68 (m, 2H). m/z: 337 [M+H]+ | | | |
| 2'-cyclopropyl-3-fluoro-5'-[(morpholin-2-yl)methyl]-[1,1'-biphenyl]-4-carbonitrile | | | | |

| Example 140 | Procedure: A1+B2+D1 | SM1: BB-2 | SM2: 3-methoxyazetidine | Yield: 98% |
|---|---|---|---|---|
| 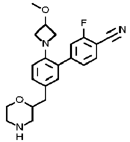 | 1H NMR (DMSO-d6, 500 MHz): δ (ppm) 7.94 (t, J = 7.5 Hz, 1H), 7.50 (dd, J = 10.5, 1.2 Hz, 1H), 7.41 (dd, J = 8.1, 1.5 Hz, 1H), 7.13 (dd, J = 8.3, 2.0 Hz, 1H), 6.97 (d, J = 2.0 Hz, 1H), 6.71-8.05 (m, 2H), 6.59 (d, J = 8.3 Hz, 1H), 4.04-4.12 (m, 1H), 3.61-3.70 (m, 3H), 3.40-3.48 (m, 1H), 3.33-3.38 (m, 1H), 3.26 (dt, J = 8.1, 3.9 Hz, 2H), 3.12 (s, 3H), 2.70 (dd, J = 12.0, 2.0 Hz, 1H), 2.52-2.65 (m, 4H), 2.35 (dd, J = 12.2, 10.0 Hz, 1H). m/z: 382 [M+H]+ | | | |
| 3-fluoro-2'-(3-methoxyazetidin-1-yl)-5'-[(morpholin-2-yl)methyl]-[1,1'-biphenyl]-4-carbonitrile | | | | |

| Example 139 | Procedure: A1+B2+D1 | SM1: BB-2F | SM2: azetidine | Yield: 72% |
|---|---|---|---|---|
| 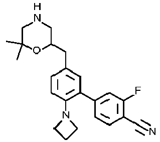 | 1H NMR (DMSO-d6, 600 MHz): δ (ppm) 7.87-7.99 (m, 1H), 7.49 (dd, J = 10.6, 1.4 Hz, 1H), 7.41 (dd, J = 8.0, 1.5 Hz, 1H), 7.12 (dd, J = 8.3, 2.0 Hz, 1H), 6.95 (d, J = 2.1 Hz, 1H), 6.55 (d, J = 8.4 Hz, 1H), 3.65-3.82 (m, 1H), 3.43 (t, J = 7.3 Hz, 4H), 2.64 (dd, J = 12.1,2.0 Hz, 1H), 2.57 (dd, J = 13.7, 6.1 Hz, 1H), 2.50-2.52 (m, 1H), 2.42 (dd, J = 13.9, 7.0 Hz, 1H), 2.34 (d, J = 12.0 Hz, 1H), 2.21-2.31 (m, 1H), 2.16 (dd, J = 12.0, 10.6 Hz, 1H), 2.03-2.11 (m, 2H), 1.19 (s, 3H), 1.03 (s, 3H). m/z: 380.1 [M+H]+ | | | |
| 2'-(azetidin-1-yl)-5'-[(6,6-dimethylmorpholin-2-yl)methyl]-3-fluoro-[1,1'-biphenyl]-4-carbonitrile | | | | |

| Example 160 | Procedure: J+G1+N1+A1+F3+B 2 | SM1: (azetidin-2-yl)methanol | SM2: 1-bromo-4-(bromomethyl)-2-chlorobenzene | Yield: 22% |
|---|---|---|---|---|
| 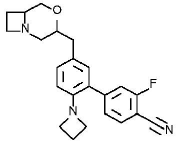 | 1H NMR (500 MHz, DMSO-d6) δ 7.93 (t, J=7.50 Hz, 1H), 7.52 (dd, J=1.34, 10.64 Hz, 1H), 7.43 (dd, J=1.47, 8.07 Hz, 1H), 7.15 (dd, J=1.96, 8.31 Hz, 1H), 6.99 (d, J=1.96 Hz, 1H), 6.57 (d, J=8.31 Hz, 1H), 3.86 (br dd, J=5.62, 11.49 Hz, 3H), 3.70 (t, J=11.13 Hz, 1H), 3.52 (br d, J=14.18 Hz, 1H), 3.44 (t, J=7.34 Hz, 4H), 3.24-3.29 (m, 1H), 2.56-2.63 (m, 1H), 2.51-2.54 (m, 1H), 2.35-2.48 (m, 2H), 2.26 (br d, J=8.31 Hz, 1H), 2.09 (quin, J=7.21 Hz, 2H), 1.39-1.50 (m, 1H). m/z: 378 [M+H]+ | | | |
| 2'-(azetidin-1-yl)-3-fluoro-5'-({4-oxa-1-azabicyclo[4.2.0]octan-3-yl}methyl)-[1,1'-biphenyl]-4-carbonitrile | | | | |

| Example 138 | Procedure: I1+I2+A4+E1+B1+D 3+D2 | SM1: 4-methylpiperidin-4-ol | SM2: BB-2J (step3) | Yield: 72% |
|---|---|---|---|---|
| 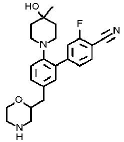 | 1H NMR (DMSO-d6, 600 MHz): δ (ppm) 7.93-7.99 (m, 1H), 7.79 (dd, J = 11.1, 1.2 Hz, 1H), 7.69 (dd, J = 8.1, 1.5 Hz, 1H), 7.20 (dd, J = 8.2, 2.1 Hz, 1H), 7.13 (d, J = 2.1 Hz, 1H), 7.08 (d, J = 8.2 Hz, 1H), 4.07-4.32 (m, 1H), 3.87 (s, 1H), 3.81 (dd, J = 11.8, 2.9 Hz, 1H), 3.61-3.69 (m, 1H), 3.50 (td, J = 11.8, 2.0 Hz, 1H), 3.33 (br s, 1H), 2.52-3.00 (m, 10H), 1.30-1.51 (m, 4H), 1.10 (s, 3H. m/z: 410 [M+H]+ | | | |
| 3-fluoro-2'-(4-hydroxy-4-methylpiperidin-1-yl)-5'-[(morpholin-2-yl)methyl]-[1,1'-biphenyl]-4-carbonitrile | | | | |

| Example 137 | Procedure: A2+F1+B2+D1 | SM1: BB-2 | SM2: methyl azetidine-3-carboxylate hydrochloride | Yield: 30% |
|---|---|---|---|---|
| 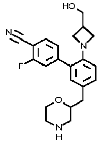 | 1H NMR (600 MHz, DMSO-d6)sample +0.5 eq of tartrate: δ 7.93 (t, J=7.48 Hz, 1H), 7.50 (d, J=1.32 Hz, 1H), 7.48 (d, J=1.32 Hz, 1H), 7.40 (dd, J=1.47, 8.07 Hz, 1H), 7.13 (dd, J=2.05, 8.36 Hz, 1H), 6.97 (d, J=1.91 Hz, 1H), 6.57 (d, J=8.36 Hz, 1H), 5.22-10.27 (br, 1H), 4.34-4.75 (br, 1H), 3.85 (s, 1H), 3.80 (br dd, J=2.71, 11.81 Hz, 1H), 3.56-3.65 (m, 2H), 3.45-3.52 (m, 4H), 3.40-3.44 (m, 2H), 3.13-3.26 (m, 2H), 2.82-2.96 (m, 2H), 2.69-2.80 (m, 1H), 2.51-2.67 (m, 4H). m/z: 382 [M+H]+ | | | |
| 3-fluoro-2'-[3-(hydroxymethyl)azetidin-1-yl]-5'-[(morpholin-2-yl)methyl]-[1,1'-biphenyl]-4-carbonitrile | | | | |

| Example 136 | Procedure: A1+B2+D1 | SM1: BB-2E | SM2: azetidine | Yield: 61% |
|---|---|---|---|---|
| 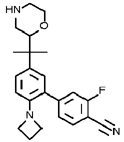 | 1H NMR (DMSO-d6, 600 MHz): δ (ppm) 7.92 (t, J = 7.6 Hz, 1H), 7.51 (dd, J = 10.6, 1.4 Hz, 1H), 7.42 (dd, J = 7.9, 1.5 Hz, 1H), 7.28 (dd, J = 8.5, 2.3 Hz, 1H), 7.04 (d, J = 2.3 Hz, 1H), 6.58 (d, J = 8.5 Hz, 1H), 3.76 (dd, J = 11.0, 2.9 Hz, 1H), 3.44 (t, J = 7.3 Hz, 6H), 2.57-2.64 (m, 1H), 2.50 (dt, J = 3.7, 1.8 Hz, 3H), 2.28 (dd, J = 12.0, 10.6 Hz, 1H), 2.08 (quin, J = 7.3 Hz, 2H), 1.16-1.33 (m, 6H). m/z: 380 [M+H]+ | | | |
| 2'-(azetidin-1-yl)-3-fluoro-5'-[2-(morpholin-2-yl)propan-2-yl]-[1,1'-biphenyl]-4-carbonitrile | | | | |

| Example 135 | Procedure: A1+B2+D1 | SM1: BB-2A | SM2: azetidine | Yield: 60% |
|---|---|---|---|---|
| 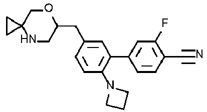 | 1H NMR (DMSO-d6, 600 MHz): δ (ppm) 7.85-7.96 (m, 1H), 7.50 (dd, J = 10.6, 1.4 Hz, 1H), 7.42 (dd, J = 8.0, 1.5 Hz, 1H), 7.14 (dd, J = 8.2, 2.1 Hz, 1H), 6.98 (d, J = 1.9 Hz, 1H), 6.56 (d, J = 8.4 Hz, 1H), 3.71 (d, J = 11.2 Hz, 1H), 3.43 (t, J = 7.3 Hz, 5H), 3.04 (d, J = 11.2 Hz, 1H), 2.43-2.74 (m, 5H), 2.03-2.17 (m, 2H), 0.28-0.49 (m, 4H). m/z: 378.1 [M+H]+ | | | |
| 2'-(azetidin-1-yl)-3-fluoro-5'-({7-oxa-4-azaspiro[2.5]octan-6-yl}methyl)-[1,1'-biphenyl]-4-carbonitrile | | | | |

| Example 107 | Procedure: A4+B2+D1 | SM1: BB-2 | SM2: 3-(azetidin-3- yl)pyridine dihydrochloride | Yield: 74% |
|---|---|---|---|---|
| 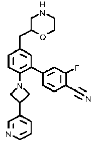 | 1H NMR (DMSO-d6, 600 MHz): δ (ppm) 8.48 (d, J = 2.2 Hz, 1H), 8.43 (dd, J = 4.7, 1.6 Hz, 1H), 7.90-7.98 (m, 1H), 7.75 (dt, J = 8.0, 1.9 Hz, 1H), 7.56 (dd, J = 10.6, 1.2 Hz, 1H), 7.47 (dd, J = 8.1, 1.5 Hz, 1H), 7.31-7.39 (m, 1H), 7.18 (dd, J = 8.4, 2.1 Hz, 1H), 7.01 (d, J = 1.9 Hz, 1H), 6.68 (d, J = 8.4 Hz, 1H), 3.86-3.93 (m, 2H), 3.85 (s, 1H), 3.73-3.82 (m, 2H), 3.59-3.68 (m, 1H), 3.44-3.53 (m, 3H), 2.83-2.97 (m, 2H), 2.76 (td, J = 12.1, 3.4 Hz, 1H), 2.65 (qd, J = 14.1, 6.5 Hz, 2H), 2.54 (dd, J = 12.1, 10.6 Hz, 1H). m/z: 429 [M+H]+ | | | |
| 3-fluoro-5'-[(morpholin-2-yl)methyl]-2'-[3-(pyridin-3-yl)azetidin-1-yl]-[1,1'-biphenyl]-4-carbonitrile | | | | |

| Example 42 | Procedure: B4+B2+D1 | SM1: BB-1 | SM2: cyclopropylboronic acid | Yield: 48% |
|---|---|---|---|---|
| 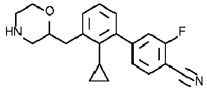 | 1H NMR (DMSO-d6, 500 MHz): δ (ppm) 7.95 (t, J = 7.3 Hz, 1H), 7.60 (dd, J = 10.8, 1.5 Hz, 1H), 7.45 (dd, J = 8.1, 1.5 Hz, 1H), 7.25-7.32 (m, 2H), 7.13 (dd, J = 7.5, 1.6 Hz, 1H), 3.64-3.78 (m, 2H), 3.34-3.47 (m, 1H), 2.91-3.02 (m, 2H), 2.79 (dd, J = 12.1, 2.1 Hz, 1H), 2.61-2.69 (m, 2H), 2.44-2.48 (m, 1H), 2.29 (br s, 1H), 1.94-2.05 (m, 1H), 0.68-0.79 (m, 2H), -0.10-0.03 (m, 2H). m/z: 337 [M+H]+ | | | |
| 2'-cyclopropyl-3-fluoro-3'-[(morpholin-2-yl)methyl]-[1,1'-biphenyl]-4-carbonitrile | | | | |

| Example 159 | Procedure: A1+D3+B2+D1 | SM1: BB-1 | SM2: 3-[(tert- butyldimethylsilyl)oxy] azetidine | Yield: |
|---|---|---|---|---|
| 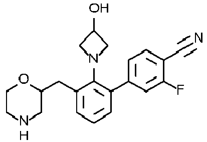 | 1H NMR (600 MHz, DMSO-d6) δ ppm 7.85 - 7.96 (m, 1 H) 7.38 - 7.47 (m, 1 H) 7.28 - 7.32 (m, 1 H) 7.07 - 7.15 (m, 1 H) 6.94 - 6.99 (m, 1 H) 6.78 (t, J=7.48 Hz, 1 H) 4.92 - 5.82 (m, 1 H) 4.10 - 4.22 (m, 1 H) 3.74 - 3.83 (m, 4 H) 3.53 - 3.57 (m, 1 H) 3.27 - 3.30 (m, 2 H) 3.08 - 3.18 (m, 1 H) 2.87 - 2.92 (m, 1 H) 2.68 - 2.86 (m, 4 H) 2.52 - 2.57 (m, 1 H). m/z: 368 [M+H]+ | | | |
| 3-fluoro-2'-(3-hydroxyazetidin-1-yl)-3'-[(morpholin-2-yl)methyl]-[1,1'-biphenyl]-4-carbonitrile | | | | |

| Example 76 | Procedure: G1+G0+I1+G3+A1+ B2+D1 | SM1: 2-(4-bromo-3-chlorophenyl)propanal | SM2: trimethyl(oxo)-λ⁶-sulfanylium iodide | Yield: 42% |
|---|---|---|---|---|
| 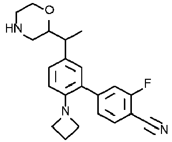 | 1H NMR (DMSO-d6, 600 MHz): δ (ppm) 7.92 (t, J = 7.4 Hz, 1H), 7.51 (dd, J = 10.6, 1.4 Hz, 1H), 7.42 (dd, J = 8.0, 1.5 Hz, 1H), 7.13 (dd, J = 8.4, 2.1 Hz, 1H), 6.95 (d, J = 2.1 Hz, 1H), 6.57 (d, J = 8.4 Hz, 1H), 3.74 (brd, J = 10.9 Hz, 1H), 3.44 (t, J = 7.3 Hz, 4H), 3.37-3.42 (m, 1H), 3.25-3.29 (m, 1H), 2.51-2.63 (m, 3H), 2.34-2.44 (m, 1H), 2.22 (dd, J = 12.2, 10.0 Hz, 1H), 2.13-2.35 (m, 1H), 2.08 (quin, J = 7.3 Hz, 2H), 1.20 (d, J = 6.9 Hz, 3H). m/z: 366 [M+H]+ | | | |
| 2'-(azetidin-1-yl)-3-fluoro-5'-[1-(morpholin-2-yl)ethyl]-[1,1'-biphenyl]-4-carbonitrile | | | | |

| Example 55 | Procedure: A2+B2+D1 | SM1: BB-2 | SM2: 3-(methoxymethyl)azeti dine hydrochloride | Yield: 67% |
|---|---|---|---|---|
| 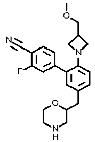 | 1H NMR (600 MHz, DMSO-d6) δ ppm 7.87 - 8.00 (m, 1 H) 7.46 - 7.52 (m, 1 H) 7.38 - 7.43 (m, 1 H) 7.10 - 7.18 (m, 1 H) 6.97 (d, J=1.91 Hz, 1 H) 6.53 - 6.62 (m, 1 H) 3.80 (br dd, J=11.74, 2.79 Hz, 1 H) 3.60 - 3.63 (m, 1 H) 3.49 - 3.53 (m, 3 H) 3.36 - 3.38 (m, 2 H) 3.30 - 3.31 (m, 1 H) 3.17 - 3.20 (m, 5 H) 2.87 - 2.95 (m, 2 H) 2.74 - 2.80 (m, 1 H) 2.69 - 2.73 (m, 1 H) 2.59 - 2.67 (m, 2 H) 2.52 - 2.58 (m, 1 H). m/z: 396 [M+H]+ | | | |
| 3-fluoro-2'-[3-(methoxymethyl)azetidin-1-yl]-5'-[(morpholin-2-yl)methyl]-[1,1'-biphenyl]-4-carbonitrile | | | | |

| Example 158 | Procedure: B1+M1+B2+D2 | SM1: BB-2 | SM2: 2-(3,6-dihydro-2H-pyran-4-yl)-4,4,5,5-tetramethyl-1,3,2-dioxaborolane | Yield: 72% |
|---|---|---|---|---|
| 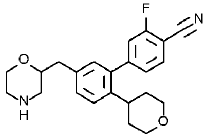 | 1H NMR (600 MHz, CD3SOCD3, 300 K) δ (ppm) = 7.99 (t, J = 7.5 Hz, 1H), 7.51 (dd, J = 1.3, 10.3 Hz, 1H), 7.39 (d, J = 8.1 Hz, 1H), 7.35 (dd, J = 1.5, 7.9 Hz, 1H), 7.29 (dd, J = 1.6, 8.1 Hz, 1H), 7.03 (d, J = 1.6 Hz, 1H), 3.88 (s, 1H), 3.87 - 3.78 (m, 2H), 3.73 - 3.61 (m, 1H), 3.55 - 3.44 (m, 1H), 3.25 - 3.13 (m, 2H), 3.01 - 2.52 (m, 4H), 1.79 - 1.62 (m, 2H), 1.58 - 1.49 (m, 2H). m/z: 381.1 [M+H]+ | | | |
| 3-fluoro-5'-[(morpholin-2-yl)methyl]-2'-(oxan-4-yl)-[1,1'-biphenyl]-4-carbonitrile | | | | |

| Example 77 | Procedure: D1+Q1+B2+D1 | SM1: tert-butyl 4-methoxy-3-methyl-1H-pyrazole-1-carboxylate | SM2: tert-butyl 2-{[3-chloro-4-(4,4,5,5-tetramethyl-1,3,2-dioxaborolan-2-yl)phenyl]methyl}morp holine-4-carboxylate | Yield: 73% |
|---|---|---|---|---|
| 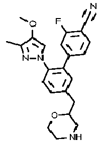 | 1H NMR (500 MHz, CD3SOCD3, 300 K) δ (ppm) = 7.93 - 7.83 (m, 1H), 7.49 - 7.39 (m, 3H), 7.33 (s, 1H), 7.22 (dd, J = 1.5, 10.8 Hz, 1H), 7.01 (dd, J = 1.5, 8.1 Hz, 1H), 3.86 (s, 1H), 3.80 (brdd, J = 2.4, 11.7 Hz, 1H), 3.76 - 3.67 (m, 1H), 3.56 (s, 4H), 3.54 - 3.45 (m, 3H), 2.96 (br d, J = 11.2 Hz, 1H), 2.89 - 2.71 (m, 4H), 2.58 (br t, J = 11.2 Hz, 1H), 2.01 (s, 3H). m/z: 407.1 [M+H]+ | | | |
| 3-fluoro-2'-(4-methoxy-3-methyl-1H-pyrazol-1-yl)-5'-[(morpholin-2-yl)methyl]-[1,1'-biphenyl]-4-carbonitrile | | | | |

| Example 133 | Procedure: B1+B2+D1 | SM1: BB-3 | SM2: 2-bromo-5-methoxypyridine | Yield: 85% |
|---|---|---|---|---|
| 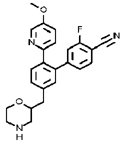 | 1H NMR (500 MHz, DMSO-d6) δ 8.20 (d, J=2.93 Hz, 1H), 7.79 (t, J=7.40 Hz, 1H), 7.54 (d, J=7.82 Hz, 1H), 7.40 (d, J=7.86 Hz, 1H), 7.26-7.34 (m, 3H), 7.15 (d, J=8.80 Hz, 1H), 7.01 (dd, J=1.47, 8.07 Hz, 1H), 3.88 (s, 1H), 3.80-3.84 (m, 4H), 3.75 (br d, J=7.58 Hz, 1H), 3.48-3.58 (m, 2H), 3.00 (br d, J=12.72 Hz, 1H), 2.90 (br d, J=12.72 Hz, 1H), 2.75-2.85 (m, 3H), 2.58-2.66 (m, 1H). m/z: 404.3 [M+H]+ | | | |
| 3-fluoro-2'-(5-methoxypyridin-2-yl)-5'-[(morpholin-2-yl)methyl]-[1,1'-biphenyl]-4-carbonitrile | | | | |

| Example 132 | Procedure: B1+B2+D1 | SM1: BB-3 | SM2: 2-bromo-5-methoxypyrazine | Yield: 76% |
|---|---|---|---|---|
| 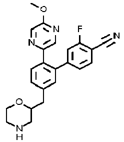 | 1H NMR (500 MHz, DMSO-d6)+ 0.5eq of tartrate: δ 8.20 (d, J=1.22 Hz, 1H), 8.11 (d, J=1.22 Hz, 1H), 7.80 (t, J=7.46 Hz, 1H), 7.60 (d, J=7.83 Hz, 1H), 7.43-7.47 (m, 1H), 7.32-7.38 (m, 2H), 7.04 (dd, J=1.34, 7.95 Hz, 1H), 3.90 (s, 4H), 3.83 (dd, J=2.69, 11.74 Hz, 1H), 3.76 (br s, 1H), 3.51-3.58 (m, 1H), 3.03 (brd, J=12.23 Hz, 1H), 2.92 (brd, J=11.98 Hz, 1H), 2.77-2.85 (m, 2H), 2.64 (t, J=11.49 Hz, 1H). m/z: 405 [M+H]+ | | | |
| 3-fluoro-2'-(5-methoxypyrazin-2-yl)-5'-[(morpholin-2-yl)methyl]-[1,1'-biphenyl]-4-carbonitrile | | | | |

| Example 80 | Procedure: B1+B2+D1 | SM1: BB-2 | SM2: 2-methoxy-5-(4,4,5,5-tetramethyl-1,3,2-dioxaborolan-2-yl)pyridine | Yield: 92% |
|---|---|---|---|---|
| 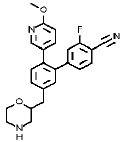 | 1H NMR (600 MHz, DMSO-d6) δ ppm 8.14 (s, 2 H) 7.90 - 8.00 (m, 1 H) 7.79 - 7.85 (m, 1 H) 7.41 (s, 2 H) 7.32 - 7.38 (m, 3 H) 7.09 (dd, J=8.07, 1.47 Hz, 1 H) 6.67 - 6.76 (m, 1 H) 3.84 - 3.88 (m, 1 H) 3.82 - 3.84 (m, 3 H) 3.76 - 3.81 (m, 1 H) 3.54 - 3.58 (m, 1 H) 3.06 - 3.10 (m, 1 H) 2.96 - 3.01 (m, 1 H) 2.80 - 2.88 (m, 3 H) 2.65 - 2.72 (m, 1 H). m/z: 404.1 [M+H]+ | | | |
| 3-fluoro-2'-(6-methoxypyridin-3-yl)-5'-[(morpholin-2-yl)methyl]-[1,1'-biphenyl]-4-carbonitrile | | | | |

| Example 157 | Procedure: B1+B2+D1 | SM1: BB-2 | SM2: 7-bromo-[1,2,4]triazolo[4,3- a]pyridine | Yield: 73% |
|---|---|---|---|---|
| 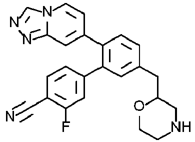 | 1H NMR (500 MHz, DMSO-d6) δ 9.20 (s, 1H), 10.63 - 8.43 (m, 3H), 8.38 (d, J = 7.3 Hz, 1H), 7.82 (t, J = 7.6 Hz, 1H), 7.68 (s, 1H), 7.58 (d, J = 7.8 Hz, 1H), 7.52 - 7.45 (m, 2H), 7.42 (s, 1H), 7.18 (dd, J = 8.1, 1.5 Hz, 1H), 6.51 (dd, J = 7.1, 1.5 Hz, 1H), 4.27 (s, 2H), 4.00 - 3.89 (m, 2H), 3.69 - 3.61 (m, 1H), 3.37 - 3.33 (m, 2H), 3.26 (br d, J = 12.7 Hz, 1H), 3.18 (br d, J = 12.5 Hz, 1H), 3.03 - 2.96 (m, 1H), 2.96 - 2.86 (m, 2H), 2.86 - 2.81 (m, 1H). m/z: 414 [M+H]+ | | | |
| 3-fluoro-5'-[(morpholin-2-yl)methyl]-2'-{[1,2,4]triazolo[4,3-a]pyridin-7-yl}-[1,1'-biphenyl]-4-carbonitrile | | | | |

| Example 131 | Procedure: J1+G1+N1+F3+A1+ B2 | SM1: [(2S)-pyrrolidin-2-yl]methanol | SM2: 2-chloroacetyl chloride | Yield: 33% |
|---|---|---|---|---|
| 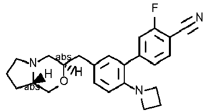 | 1H NMR (DMSO-d6, 600 MHz): δ (ppm) 7.92 (t, J = 7.6 Hz, 1H), 7.51 (dd, J = 10.6, 1.3 Hz, 1H), 7.42 (dd, J = 8.1, 1.5 Hz, 1H), 7.14 (dd, J = 8.3, 2.0 Hz, 1H), 6.97 (d, J = 2.1 Hz, 1H), 6.56 (d, J = 8.4 Hz, 1H), 3.85 (dd, J = 10.6, 2.9 Hz, 1H), 3.50-3.61 (m, 1H), 3.43 (t, J = 7.3 Hz, 4H), 3.11 (t, J = 10.3 Hz, 1H), 2.91 (td, J = 8.2, 2.1 Hz, 1H), 2.86 (dd, J = 10.9, 2.0 Hz, 1H), 2.53-2.73 (m, 2H), 2.08 (quin, J = 7.3 Hz, 2H), 2.00 (q, J = 8.4 Hz, 1H), 1.80-1.94 (m, 2H), 1.56-1.74 (m, 3H), 1.16 (qd, J = 10.6, 6.6 Hz, 1H). m/z: 392.1 [M+H]+ | | | |
| 5'-{[(3R,8aS)-hexahydro-1H-pyrrolo[2,1-c][1,4]oxazin-3-yl]methyl}-2'-(azetidin-1-yl)-3-fluoro-[1,1'-biphenyl]-4-carbonitrile | | | | |

| Example 65 | Procedure: O1+B2+D1 | SM1: BB-1 | SM2: 2-(tributylstannyl)-1,3-oxazole | Yield: 82% |
|---|---|---|---|---|
| 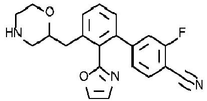 | 1H NMR (DMSO-d6, 500 MHz): δ (ppm) 8.08 (s, 1H), 7.79-7.94 (m, 1H), 7.60-7.68 (m, 1H), 7.54 (d, J = 7.1 Hz, 1H), 7.45-7.49 (m, 1H), 7.34 (s, 1H), 7.25 (dd, J = 10.6, 1.3 Hz, 1H), 7.04 (dd, J = 8.1, 1.5 Hz, 1H), 3.88 (s, 1H), 3.72 (dd, J = 11.7, 2.7 Hz, 1H), 3.31-3.54 (m, 2H), 2.62-2.91 (m, 5H), 2.43 (dd, J = 12.1, 10.6 Hz, 1H). m/z: 364.3 [M+H]+ | | | |
| 3-fluoro-3'-[(morpholin-2-yl)methyl]-2'-(1,3-oxazol-2-yl)-[1,1'-biphenyl]-4-carbonitrile | | | | |

| Example 350 CPD0019236 | Procedure: G1+B0+H0+G0+I1+ G3+B2+D1 | SM1: 6-bromo-4-chloro-1H-indazole | SM2: iodomethane | Yield: 98% |
|---|---|---|---|---|
| 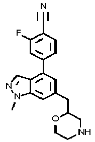 | 1H NMR (500 MHz, DMSO-d6, 300K) δ ppm 8.27 - 10.23 (m, 2 H), 8.21 (d, J=0.7 Hz, 1 H), 8.04 - 8.13 (m, 1 H), 7.87 (dd, J=10.5, 1.6 Hz, 1 H), 7.80 (dd, J=8.1, 1.6 Hz, 1 H), 7.63 (s, 1 H), 7.33 (s, 1 H), 4.26 (s, 1 H), 4.08 (s, 3 H), 3.96 (brdd, J=12.3, 3.3 Hz, 2 H), 3.58 - 3.74 (m, 1 H), 3.12 - 3.24 (m, 3 H), 2.92 - 3.08 (m, 3 H), 2.83 (t, J=11.9 Hz, 1 H). m/z: 351.3 [M+H]+ | | | |
| 2-fluoro-4-{1-methyl-6-[(morpholin-2-yl)methyl]-1H-indazol-4-yl}benzonitrile | | | | |

| Example 130 | Procedure: B1+G1+B2+D1 | SM1: BB-3 | SM2: (5-bromo-1,3-oxazol-2-yl)methanol | Yield: 75% |
|---|---|---|---|---|
| 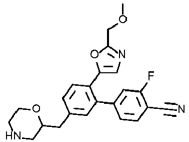 | 1H NMR (600 MHz, DMSO-d6) +é 0.5 eq of tartrate: δ 7.95 (t, J=7.58 Hz, 1H), 7.66 (d, J=7.92 Hz, 1H), 7.47 (dd, J=1.32, 10.27 Hz, 1H), 7.44 (dd, J=1.61, 7.92 Hz, 1H), 7.29 (d, J=1.47 Hz, 1H), 7.28 (dd, J=1.54, 8.00 Hz, 1H), 6.84 (s, 1H), 4.36-4.42 (m, 2H), 3.86 (s, 1H), 3.79 (dd, J=2.64, 11.59 Hz, 1H), 3.67-3.75 (m, 1H), 3.48 (dt, J=2.27, 11.70 Hz, 2H), 3.18-3.19 (m, 3H), 2.95 (brd, J=12.18 Hz, 1H), 2.72-2.87 (m, 4H), 2.56 (dd, J=10.64, 12.10 Hz, 1H). m/z: 408.3 [M+H]+ | | | |
| 3-fluoro-2'-[2-(methoxymethyl)-1,3-oxazol-5-yl]-5'-[(morpholin-2-yl)methyl]-[1,1'-biphenyl]-4-carbonitrile | | | | |

| Example 129 | Procedure: B1+F1+G1+B2+D1 | SM1: BB-3 | SM2: methyl 5-iodo-1,2-oxazole-3-carboxylate | Yield: 89% |
|---|---|---|---|---|
| 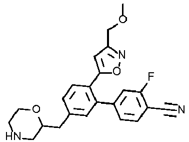 | 1H NMR (DMSO-d6, 400 MHz): δ (ppm) 7.96 (t, J = 8.0, 7.0 Hz, 1H), 7.76 (d, J = 8.0 Hz, 1H), 7.49 (dt, J = 8.8, 1.4 Hz, 2H), 7.37 (d, J = 1.7 Hz, 1H), 7.27 (dd, J = 8.0, 1.5 Hz, 1H), 6.31 (s, 1H), 4.43 (s, 2H), 3.89 (s, 2H), 3.81 (dd, 1H), 3.74 (m, 1H), 3.55 - 3.45 (td, 1H), 3.44 (s, 2H), 3.26 (s, 3H), 2.98 (d, J = 12.2 Hz, 1H), 2.94 - 2.72 (m, 4H), 2.65 - 2.55 (t, 1H).. m/z: 408 [M+H]+ | | | |
| 3-fluoro-2'-[3-(methoxymethyl)-1,2-oxazol-5-yl]-5'-[(morpholin-2-yl)methyl]-[1,1'-biphenyl]-4-carbonitrile | | | | |

| Example 106 | Procedure: G2+B0+M2+G0+I1+ G2+B2+D1 | SM1: (4-bromo-2-chlorophenyl)methanol | SM2: pyridin-3-ol | Yield: 62% |
|---|---|---|---|---|
| 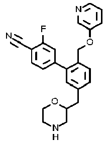 | 1H NMR (DMSO-d6, 600 MHz): +0.5Eq OF TARTRATE δ (ppm) 8.18-8.24 (m, 1H), 8.16 (dd, J = 4.3, 1.6 Hz, 1H), 7.91-8.00 (m, 1H), 7.58-7.61 (m, 1H), 7.53-7.58 (m, 1H), 7.42-7.47 (m, 1H), 7.35-7.38 (m, 1H), 7.27-7.33 (m, 2H), 7.20-7.26 (m, 1H), 4.87-5.18 (m, 2H), 3.85-3.93 (m, 1H), 3.75-3.83 (m, 1H), 3.65-3.76 (m, 1H), 3.43-3.56 (m, 2H), 2.94-3.01 (m, 1H), 2.86-2.92 (m, 1H), 2.71-2.83 (m, 3H), 2.54-2.64 (m, 1H). m/z: 404.3 [M+H]+ | | | |
| 3-fluoro-5'-[(morpholin-2-yl)methyl]-2'-[(pyridin-3-yloxy)methyl]-[1,1'-biphenyl]-4-carbonitrile | | | | |

| Example 47 | Procedure: C1+B2+D1 | SM1: BB-1 | SM2: 1,3-oxazole | Yield: 64% |
|---|---|---|---|---|
| 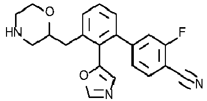 | 1H NMR (DMSO-d6, 500 MHz): δ (ppm) 8.33 (s, 1H), 7.80-7.90 (m, 1H), 7.58 (d, J = 7.8 Hz, 1H), 7.52 (d, J = 7.1 Hz, 1H), 7.41 (d, J = 7.6 Hz, 1H), 7.32 (dd, J = 10.5, 1.2 Hz, 1H), 7.10 (dd, J = 7.9, 1.6 Hz, 1H), 7.09 (s, 1H), 3.88 (s, 1H), 3.76 (br dd, J = 11.4, 2.3 Hz, 1H), 2.92-3.57 (m, 5H), 2.61-2.88 (m, 2H), 2.41-2.49 (m, 1H). m/z: 364 [M+H]+ | | | |
| 3-fluoro-3'-[(morpholin-2-yl)methyl]-2'-(1,3-oxazol-5-yl)-[1,1'-biphenyl]-4-carbonitrile | | | | |

| Example 82 | Procedure: B4+B2+D1 | SM1: BB-2I | SM2: cyclopropylboronic acid | Yield: 62% |
|---|---|---|---|---|
| 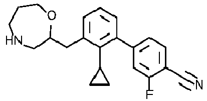 | 1H NMR (400 MHz, DMSO-d6) δ 7.97 (t, J = 8.0, 7.0 Hz, 1H), 7.60 (dd, J = 10.7, 1.5 Hz, 1H), 7.47 (dd, J = 8.0, 1.5 Hz, 1H), 7.37 - 7.25 (m, 2H), 7.16 (dd, J = 7.1, 2.0 Hz, 1H), 4.08 - 3.98 (m, 1H), 3.89 - 3.83 (m, 2H), 3.56 (m, J = 12.2, 7.1, 5.2 Hz, 2H), 3.20 (m, J = 11.2, 5.6 Hz, 2H), 3.04 (m, J = 14.2, 6.5 Hz, 2H), 2.88 (m, J = 13.5, 9.5 Hz, 1H), 2.11 - 1.96 (m, 1H), 1.90 (d, J = 9.5 Hz, 2H), 0.83 - 0.74 (m, 2H), 0.13 - -0.09 (m, 2H). m/z: 351.3 [M+H]+ | | | |
| 2'-cyclopropyl-3-fluoro-3'-[(1,4-oxazepan-2-yl)methyl]-[1,1'-biphenyl]-4-carbonitrile | | | | |

| Example 46 | Procedure: B1+B2+D1 | SM1: BB-2I | SM2: azetidine | Yield: 32% |
|---|---|---|---|---|
| 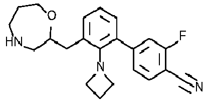 | 1H NMR (DMSO-d6, 500 MHz): - 0.6 eq of tartrate: δ (ppm) 7.85-7.96 (m, 1H), 7.40-7.47 (m, 1H), 7.31 (dd, J = 7.9, 1.3 Hz, 1H), 7.08-7.14 (m, 1H), 6.92-7.02 (m, 1H), 6.74-6.84 (m, 1H), 3.88-3.98 (m, 1H), 3.74-3.86 (m, 3H), 3.45-3.60 (m, 6H), 3.11-3.21 (m, 2H), 3.02-3.08 (m, 1H), 2.93-3.00 (m, 1H), 2.67-2.90 (m, 3H), 1.94-2.03 (m, 2H), 1.80-1.93 (m, 2H). m/z: 366.3 [M+H]+ | | | |
| 2'-(azetidin-1-yl)-3-fluoro-3'-[(1,4-oxazepan-2-yl)methyl]-[1,1'-biphenyl]-4-carbonitrile | | | | |

| Example 52 | Procedure: A1+B2+D1 | SM1: BB-2 | SM2: 4-methoxypiperidine | Yield: 52% |
|---|---|---|---|---|
| 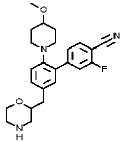 | 1H NMR (600 MHz, DMSO-d6) δ 8.00 - 7.94 (m, 1H), 7.79 (dd, J = 11.2, 1.3 Hz, 1H), 7.69 (dd, J = 8.1, 1.5 Hz, 1H), 7.20 (dd, J = 8.2, 2.1 Hz, 1H), 7.14 (d, J = 2.1 Hz, 1H), 7.06 (d, J = 8.2 Hz, 1H), 8.96 - 6.63 (m, 3H), 3.87 (s, 2H), 3.80 (dd, J = 11.8, 2.9 Hz, 1H), 3.69 - 3.63 (m, 1H), 3.49 (td, J = 11.8, 2.4 Hz, 1H), 3.43 - 3.33 (m, 2H), 3.21 (s, 3H), 3.23 - 3.17 (m, 1H), 2.95 (br d, J = 12.3 Hz, 1H), 2.92 - 2.86 (m, 3H), 2.78 (td, J = 12.2, 3.5 Hz, 1H), 2.71 - 2.64 (m, 2H), 2.60 - 2.54 (m, 3H), 1.82 - 1.70 (m, 2H), 1.41 - 1.28 (m, 2H). m/z: 410.4 [M+H]+ | | | |
| 3-fluoro-2'-(4-methoxypiperidin-1-yl)-5'-[(morpholin-2-yl)methyl]-[1,1'-biphenyl]-4-carbonitrile | | | | |

| Example 61 | Procedure: A2+B2+D1 | SM1: BB-2 | SM2: 4-methoxy-4-methylpiperidine hydrochloride | Yield: 62% |
|---|---|---|---|---|
| 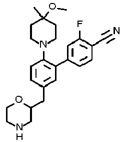 | 1H NMR (DMSO-d6, 500 MHz): δ (ppm) 7.96 (t, J = 7.7 Hz, 1H), 7.79 (dd, J = 11.1, 1.1 Hz, 1H), 7.71 (dd, J = 8.2, 1.3 Hz, 1H), 7.20 (dd, J = 8.3, 2.0 Hz, 1H), 7.13 (d, J = 2.0 Hz, 1H), 7.08 (d, J = 8.3 Hz, 1H), 6.34-8.10 (m, 2H), 3.85 (s, 2H), 3.78 (br dd, J = 11.6, 2.6 Hz, 1H), 3.59-3.67 (m, 1H), 3.47 (td, J = 11.6, 2.2 Hz, 1H), 3.24-3.40 (m, 3H), 3.06 (s, 3H), 2.83-2.94 (m, 2H), 2.62-2.79 (m, 7H), 2.53-2.57 (m, 1H), 1.60 (brd, J = 11.7 Hz, 2H), 1.31-1.45 (m, 2H), 1.08 (s, 3H). m/z: 424.4 [M+H]+ | | | |
| 3-fluoro-2'-(4-methoxy-4-methylpiperidin-1-yl)-5'-[(morpholin-2-yl)methyl]-[1,1'-biphenyl]-4-carbonitrile | | | | |

| Example 105 | Procedure: A1+B7+D1 | SM1: BB-2 | SM2: 1-(2,2-difluoroethyl)piperazine e | Yield: 83% |
|---|---|---|---|---|
| 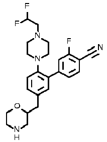 | 1H NMR (DMSO-d6, 600 MHz): δ (ppm) 7.93-8.02 (m, 1H), 7.78 (dd, J = 11.0, 1.2 Hz, 1H), 7.68 (dd, J = 8.2, 1.5 Hz, 1H), 7.23 (dd, J = 8.3, 2.1 Hz, 1H), 7.15 (d, J = 2.1 Hz, 1H), 7.08 (d, J = 8.4 Hz, 1H), 5.89-6.28 (m, 1H), 3.94 (s, 2H), 3.84 (dd, J = 12.1, 3.0 Hz, 1H), 3.67-3.74 (m, 2H), 3.54 (td, J = 12.0, 2.3 Hz, 2H), 2.95-3.04 (m, 2H), 2.83 (td, J = 12.3, 3.7 Hz, 1H), 2.57-2.78 (m, 9H), 2.46 (br s, 4H). m/z: 445.1 [M+H]+ | | | |
| 2'-[4-(2,2-difluoroethyl)piperazin-1-yl]-3-fluoro-5'-[(morpholin-2-yl)methyl]-[1,1'-biphenyl]-4-carbonitrile | | | | |

| Example 40 | Procedure: A1+B2+D1 | SM1: BB-1 | SM2: 3-fluoroazetidine | Yield: 80% |
|---|---|---|---|---|
| 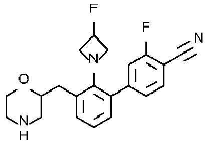 | 1H NMR (600 MHz, DMSO-d6) δ 7.93 (t, J = 7.6 Hz, 1H), 7.86 - 7.53 (m, 1H), 7.47 (dd, J = 10.4, 1.2 Hz, 1H), 7.32 (dd, J = 8.1, 1.5 Hz, 1H), 7.15 (dd, J = 7.5, 1.5 Hz, 1H), 6.99 (dd, J = 7.6, 1.5 Hz, 1H), 6.84 (t, J = 7.5 Hz, 1H), 5.22 - 5.04 (m, 1H), 3.85 (s, 5H), 3.70 - 3.59 (m, 2H), 3.54 (td, J = 11.7, 1.8 Hz, 1H), 3.38 - 3.11 (m, 1H), 2.93 - 2.66 (m, 5H), 2.55 (dd, J = 12.3, 10.5 Hz, 1H). m/z: 370 [M+H]+ | | | |
| 3-fluoro-2'-(3-fluoroazetidin-1-yl)-3'-[(morpholin-2-yl)methyl]-[1,1'-biphenyl]-4-carbonitrile | | | | |

| Example 16 | Procedure: B1+H2+N2+H1+R1. 1+R1.2+R1.3+I1+B2 +D2 | SM1: BB-1 | SM2: ethenylborane | Yield: 100% |
|---|---|---|---|---|
| 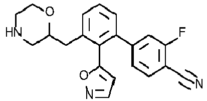 | 1H NMR (DMSO-d6, 500 MHz) δ 9.07 (br s, 2H), 8.58 (d, 1H, J=1.7 Hz), 7.86 (dd, 1H, J=7.1, 7.8 Hz), 7.6-7.7 (m, 1H), 7.55 (dd, 1H, J=1.0, 7.8 Hz), 7.47 (dd, 1H, J=1.1, 7.7 Hz), 7.32 (dd, 1H, J=1.5, 10.5 Hz), 7.12 (dd, 1H, J=1.5, 8.1 Hz), 6.50 (d, 1H, J=1.7 Hz), 3.90 (dd, 1H, J=3.2, 12.7 Hz), 3.7-3.8 (m, 1H), 3.60 (dt, 1H, J=2.3, 12.4 Hz), 3.0-3.2 (m, 2H), 2.9-3.0 (m, 1H), 2.6-2.8 (m, 3H). m/z: 364 [M+H]+ | | | |
| 3-fluoro-3'-[(morpholin-2-yl)methyl]-2'-(1,2-oxazol-5-yl)-[1,1'-biphenyl]-4-carbonitrile | | | | |

| Example 48 | Procedure: B1+B2+D1 | SM1: BB-1 | SM2: 5-(4,4,5,5-tetramethyl-1,3,2-dioxaborolan-2-yl)-1,3-thiazole | Yield: 89% |
|---|---|---|---|---|
| 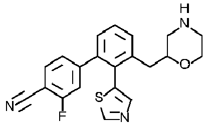 | 1H NMR (DMSO-d6, 500 MHz) δ 9.07 (s, 1H), 7.8-7.8 (m, 1H), 7.72 (s, 1H), 7.5-7.6 (m, 2H), 7.3-7.4 (m, 2H), 7.15 (dd, 1H, J=1.6, 7.9 Hz), 3.88 (s, 1H), 3.75 (br dd, 1H, J=2.4, 11.7 Hz), 3.5-3.6 (m, 1H), 3.41 (dt, 1H, J=2.3, 11.7 Hz), 3.0-3.3 (m, 3H), 2.7-2.9 (m, 3H), 2.64 (d, 2H, J=6.6 Hz), 2.45 (dd, 1H, J=10.6, 12.1 Hz), m/z: 380 [M+H]+ | | | |
| 3-fluoro-3'-[(morpholin-2-yl)methyl]-2'-(1,3-thiazol-5-yl)-[1,1'-biphenyl]-4-carbonitrile | | | | |

| Example 156 | Procedure: O2+B2+D2 | SM1: BB-1 | SM2: 1-methyl-5-(tributylstannyl)-1H-pyrazole | Yield: 71% |
|---|---|---|---|---|
| 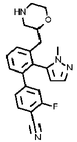 | 1H NMR (500 MHz, DMSO-d6) δ ppm 7.99 - 8.52 (m, 2 H) 7.74 - 7.88 (m, 1 H) 7.48 - 7.61 (m, 2 H) 7.38 - 7.45 (m, 2 H) 7.23 - 7.31 (m, 1 H) 7.11 - 7.18 (m, 1 H) 6.26 - 6.31 (m, 1 H) 3.69 - 3.75 (m, 1 H) 3.48 - 3.52 (m, 1 H) 3.37 - 3.39 (m, 1 H) 3.20 (d, J=19.07 Hz, 3 H) 3.12 - 3.17 (m, 1 H) 2.71 (brd, J=18.83 Hz, 4 H) 2.38 - 2.46 (m, 1 H). m/z: 377 [M+H]+ | | | |
| 3-fluoro-2'-(1-methyl-1H-pyrazol-5-yl)-3'-[(morpholin-2-yl)methyl]-[1,1'-biphenyl]-4-carbonitrile | | | | |

| Example 69 | Procedure: C1+B2+D1 | SM1: BB-1 | SM2: 2-methyl-1 ,3-oxazole | Yield: 76% |
|---|---|---|---|---|
| 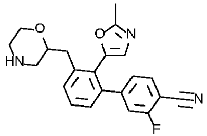 | 1H NMR (400 MHz, DMSO-d6) δ 9.46 - 8.05 (m, 2H), 7.86 (dd, J = 7.9, 7.2 Hz, 1H), 7.62 - 7.55 (m, 1H), 7.53 - 7.48 (m, 1H), 7.41 (dd, J = 7.6, 1.2 Hz, 1H), 7.34 (dd, J = 10.5, 1.4 Hz, 1H), 7.12 (dd, J = 8.0, 1.5 Hz, 1H), 6.86 (s, 1H), 4.12 (s, 1H), 3.89 (dd, J = 12.6, 3.1 Hz, 1H), 3.79 - 3.67 (m, 1H), 3.59 - 3.49 (m, 1H), 3.48 - 3.27 (m, 2H), 3.07 (br t, J = 12.3 Hz, 2H), 2.92 (td, J = 12.4, 3.8 Hz, 1H), 2.81 - 2.63 (m, 3H), 2.31 (s, 3H). m/z: 378.3 [M+H]+ | | | |
| 3-fluoro-2'-(2-methyl-1,3-oxazol-5-yl)-3'-[(morpholin-2-yl)methyl]-[1,1'-biphenyl]-4-carbonitrile | | | | |

| Example 51 | Procedure: C1+B2+D1 | SM1: BB-1 | SM2: 4-methyl-1 ,3-oxazole | Yield: 74% |
|---|---|---|---|---|
| 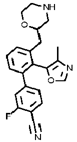 | 1H NMR (DMSO-d6, 500 MHz): δ (ppm) 8.32 (d, J = 13.7 Hz, 1H), 7.82-7.86 (m, 1H), 7.57-7.63 (m, 1H), 7.52 (brd, J = 7.3 Hz, 1H), 7.43 (d, J = 7.6 Hz, 1H), 7.25 (dd, J = 10.5, 1.5 Hz, 1H), 7.04 (br d, J = 8.1 Hz, 1H), 6.44-8.74 (m, 2H), 3.89 (s, 2H), 3.74 (brdd, J = 11.7, 2.2 Hz, 1H), 3.51-3.58 (m, 1H), 3.33-3.50 (m, 4H), 2.53-2.87 (m, 5H), 2.38-2.48 (m, 1H), 1.59 (d, J = 3.4 Hz, 3H). m/z: 378 [M+H]+ | | | |
| 3-fluoro-2'-(4-methyl-1,3-oxazol-5-yl)-3'-[(morpholin-2-yl)methyl]-[1,1'-biphenyl]-4-carbonitrile | | | | |

| Example 49 | Procedure: F3+E4+N1+D4+F3+ I1+B1+B2+D1 | SM1: methyl 2-bromo-3-chloro-5-fluorobenzoate | SM2: 4-[(4-methoxyphenyl)methy l]morpholin-3-one | Yield: 85% |
|---|---|---|---|---|
| 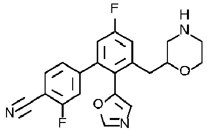 | 1H NMR (DMSO-d6, 500 MHz) +0.5 eq of tartrate: δ (ppm) 8.33 (s, 1H), 7.83-7.89 (m, 1H), 7.32-7.43 (m, 3H), 7.12 (dd, J = 8.1, 1.5 Hz, 1H), 7.09 (s, 1H), 3.88 (s, 1H), 3.75 (br dd, J = 11.6, 2.6 Hz, 1H), 3.51-3.57 (m, 1H), 3.39-3.44 (m, 1H), 2.76-2.87 (m, 2H), 2.63-2.74 (m, 3H), 2.42-2.48 (m, 1H). m/z: 382 [M+H]+ | | | |
| 3,5'-difluoro-3'-[(morpholin-2-yl)methyl]-2'-(1,3-oxazol-5-yl)-[1,1'-biphenyl]-4-carbonitrile | | | | |

| Example 128 | Procedure: B5+B2+D1 | SM1: BB-1 | SM2: (pyrazin-2-yl)boronic acid | Yield: 91% |
|---|---|---|---|---|
| 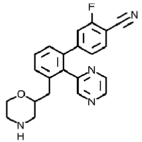 | 1H NMR (DMSO-d6, 600 MHz): δ (ppm) 8.67 (dd, J = 2.6, 1.5 Hz, 1H), 8.53 (d, J = 2.5 Hz, 1H), 8.35 (d, J = 1.5 Hz, 1H), 7.73 (t, J = 7.3 Hz, 1H), 7.57 (t, J = 7.6 Hz, 1H), 7.52 (d, J = 7.5 Hz, 1H), 7.41 (dd, J = 7.6, 1.2 Hz, 1H), 7.21 (dd, J = 10.4, 1.5 Hz, 1H), 6.93 (dd, J = 8.1, 1.5 Hz, 1H), 3.87 (s, 2H), 3.68 (br d, J = 11.7 Hz, 1H), 3.42-3.49 (m, 1H), 3.34 (br dd, J = 11.7, 9.4 Hz, 1H), 3.17-3.54 (m, 5H), 2.78 (brd, J = 12.2 Hz, 1H), 2.63-2.72 (m, 2H), 2.61 (brd, J = 6.3 Hz, 2H), 2.33-2.39 (m, 1H). m/z: 375.3 [M+H]+ | | | |
| 3-fluoro-3'-[(morpholin-2-yl)methyl]-2'-(pyrazin-2-yl)-[1,1'-biphenyl]-4-carbonitrile | | | | |

| Example 127 | Procedure: A2+B2+D1 | SM1: BB-2 | SM2: 6-oxa-2-azaspiro[3.4]octane; oxalic acid | Yield: 25% |
|---|---|---|---|---|
| 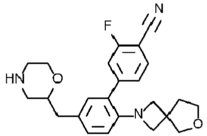 | 1H NMR (500 MHz, DMSO-d6) δ 7.94 (t, J = 7.6 Hz, 1H), 7.50 (dd, J = 10.8, 1.2 Hz, 1H), 7.42 (dd, J = 8.1, 1.2 Hz, 1H), 7.15 (dd, J = 8.3, 2.0 Hz, 1H), 6.99 (d, J = 2.0 Hz, 1H), 6.60 (d, J = 8.3 Hz, 1H), 3.85 (s, 1H), 3.80 (br dd, J = 11.6, 2.6 Hz, 1H), 3.66 (s, 2H), 3.62 (t, J = 7.0 Hz, 4H), 3.44 (s, 7H), 2.95 - 2.84 (m, 2H), 2.76 (td, J = 12.1, 3.4 Hz, 1H), 2.70 - 2.58 (m, 2H), 2.54 (t, J = 11.4 Hz, 1H), 1.99 (t, J = 7.0 Hz, 2H). m/z: 408 [M+H]+ | | | |
| 3-fluoro-5'-[(morpholin-2-yl)methyl]-2'-{6-oxa-2-azaspiro[3.4]octan-2-yl}-[1,1'-biphenyl]-4-carbonitrile | | | | |

| Example 33 | Procedure: A4+E1+B1 | SM1: BB-2J +D2 | SM2: 4-ethylpiperidin-4-ol | Yield: 70% |
|---|---|---|---|---|
| 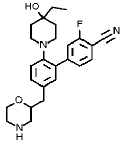 | 1H NMR (DMSO-d6, 500 MHz): δ (ppm) 7.96 (t, J = 7.6 Hz, 1H), 7.81 (dd, J = 11.2, 1.2 Hz, 1H), 7.70 (dd, J = 8.1, 1.5 Hz, 1H), 7.21 (dd, J = 8.1, 2.0 Hz, 1H), 7.14 (d, J = 2.0 Hz, 1H), 7.08 (d, J = 8.3 Hz, 1H), 6.15-8.75 (m, 3H), 3.90 (s, 2H), 3.82 (dd, J = 11.9, 2.8 Hz, 1H), 3.63-3.75 (m, 1H), 3.46-3.58 (m, 1H), 3.31-4.22 (m, 3H), 2.88-3.06 (m, 2H), 2.76-2.87 (m, 3H), 2.63-2.74 (m, 4H), 2.60 (t, J = 11.5 Hz, 1H), 1.26-1.44 (m, 6H), 0.81 (t, J = 7.5 Hz, 3H). m/z: 424.4 [M+H]+ | | | |
| 2'-(4-ethyl-4-hydroxypiperidin-1-yl)-3-fluoro-5'-[(morpholin-2-yl)methyl]-[1,1'-biphenyl]-4-carbonitrile | | | | |

| Example 67 | Procedure: A4+E1+B1+D1 | SM1: BB-2J | SM2: 4-(methoxymethyl)piperidine | Yield: 100% |
|---|---|---|---|---|
| 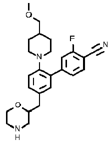 | 1H NMR (DMSO-d6, 500 MHz): δ (ppm) 7.96 (s, 1 H) 7.78 (dd, J=11.25, 1.22 Hz, 1 H) 7.62 - 7.71 (m, 1 H) 7.19 (s, 1 H) 7.12 - 7.15 (m, 1 H) 7.02 - 7.10 (m, 1 H) 6.67 (d, J=8.31 Hz, 2 H) 3.72 - 3.77 (m, 1 H) 3.55 - 3.62 (m, 1 H) 3.41 - 3.46 (m, 1 H) 3.21 (s, 3 H) 3.16 (d, J=6.11 Hz, 2 H) 2.91 - 2.98 (m, 2 H) 2.81 - 2.89 (m, 1 H) 2.67 - 2.81 (m, 2 H) 2.61 (br d, J=5.87 Hz, 2 H) 2.49 (br s, 3 H) 1.41 - 1.63 (m, 3 H) 0.99 - 1.15 (m, 2 H). m/z: 424.4 [M+H]+ | | | |
| 3-fluoro-2'-[4-(methoxymethyl)piperidin-1-yl]-5'-[(morpholin-2-yl)methyl]-[1,1'-biphenyl]-4-carbonitrile | | | | |

| Example 155 | Procedure: A3+B2+D1 | SM1: BB-2B | SM2: 4-methylpiperidin-4-ol | Yield: 85% |
|---|---|---|---|---|
| 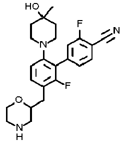 | 1H NMR (DMSO-d6, 500 MHz) δ 8.00 (t, 1H, J=7.6 Hz), 7.66 (d, 1H, J=10.8 Hz), 7.52 (d, 1H, J=8.1 Hz), 7.28 (t, 1H, J=8.6 Hz), 6.94 (d, 1H, J=8.3 Hz), 4.1-4.3 (m, 1H), 3.99 (s, 2H), 2.9-3.9 (m, 8H), 2.8-2.9 (m, 4H), 2.6-2.7 (m, 6H), 1.1-1.5 (m, 4H), 1.07 (s, 3H). m/z: 428 [M+H]+ | | | |
| 2',3-difluoro-6'-(4-hydroxy-4-methylpiperidin-1-yl)-3'-[(morpholin-2-yl)methyl]-[1,1'-biphenyl]-4-carbonitrile | | | | |

| Example 62 | Procedure: D1+B2 | SM1: BB-2 | SM2: 7-oxa-2-azaspiro[3.5]nonane hydrochloride | Yield: |
|---|---|---|---|---|
| 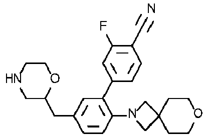 | 1H NMR (500 MHz, DMSO-d6) δ ppm 7.93 (t, J=7.58 Hz, 1 H) 7.57 - 7.83 (m, 2 H) 7.50 (dd, J=10.76, 1.22 Hz, 1 H) 7.42 (dd, J=8.07, 1.47 Hz, 1 H) 7.10 - 7.17 (m, 1 H) 6.98 (d, J=1.96 Hz, 1 H) 6.60 (d, J=8.31 Hz, 1 H) 3.75 - 3.81 (m, 1 H) 3.56 - 3.64 (m, 1 H) 3.45 (br t, J=5.01 Hz, 5 H) 3.25 (s, 4 H) 2.82 - 2.92 (m, 2 H) 2.71 - 2.79 (m, 1 H) 2.57 - 2.68 (m, 2 H) 2.52 - 2.55 (m, 1 H) 1.60 (t, J=5.14 Hz, 4 H). m/z: 422.4 [M+H]+ | | | |
| 3-fluoro-5'-[(morpholin-2-yl)methyl]-2'-{7-oxa-2-azaspiro[3.5]nonan-2-yl}-[1,1'-biphenyl]-4-carbonitrile | | | | |

| Example 126 | Procedure: A4+E1+B1+D2 | SM1: BB-2J | SM2: 4-(methoxymethyl)piperi din-4-ol | Yield: 44% |
|---|---|---|---|---|
| 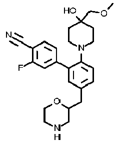 | 1H NMR (DMSO-d6, 600 MHz): δ (ppm) 7.95 (t, J = 7.6 Hz, 1H), 7.79 (dd, J = 11.1, 1.1 Hz, 1H), 7.68 (dd, J = 8.1, 1.5 Hz, 1H), 7.20 (dd, J = 8.2, 2.1 Hz, 1H), 7.13 (d, J = 2.1 Hz, 1H), 7.08 (d, J = 8.4 Hz, 1H), 6.06-8.53 (m, 2H), 4.14-4.41 (m, 1H), 3.84 (s, 2H), 3.79 (brdd, J = 11.7, 2.5 Hz, 1H), 3.61-3.66 (m, 1H), 3.45-3.51 (m, 1H), 3.32-3.43 (m, 3H), 3.27 (s, 3H), 3.11 (s, 2H), 2.52-2.94 (m, 10H), 1.42-1.53 (m, 2H), 1.27-1.36 (m, 2H). m/z: 440 [M+H]+ | | | |
| 3-fluoro-2'-[4-hydroxy-4-(methoxymethyl)piperidin-1-yl]-5'-[(morpholin-2-yl)methyl]-[1,1'-biphenyl]-4-carbonitrile | | | | |

| Example 79 | Procedure: A4+E1+B1+D1 | SM1: BB-2J | SM2: 4-(propan-2-yl)piperidin-4-ol | Yield: 74% |
|---|---|---|---|---|
| 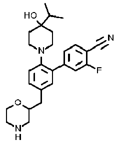 | 1H NMR (500 MHz, DMSO-d6) δ 7.96 (t, J = 7.6 Hz, 1H), 7.83 (dd, J = 11.2, 1.2 Hz, 1H), 7.69 (dd, J = 8.2, 1.3 Hz, 1H), 7.20 (dd, J = 8.3, 2.0 Hz, 1H), 7.14 (d, J = 2.0 Hz, 1H), 7.08 (d, J = 8.1 Hz, 1H), 3.86 (s, 1H), 3.80 (brdd, J = 11.6, 2.6 Hz, 1H), 3.70 - 3.60 (m, 1H), 3.57 - 3.11 (m, 4H), 3.00 - 2.53 (m, 11H), 1.58 - 1.42 (m, 1H), 1.41 - 1.28 (m, 4H), 0.82 (d, J = 6.8 Hz, 6H). m/z: 438.4 [M+H]+ | | | |
| 3-fluoro-2'-[4-hydroxy-4-(propan-2-yl)piperidin-1-yl]-5'-[(morpholin-2-yl)methyl]-[1,1'-biphenyl]-4-carbonitrile | | | | |

| Example 78 | Procedure: A2+B2+D1 | SM1: BB-2 | SM2: 1-oxa-8-azaspiro[4.5]decane | Yield: 59% |
|---|---|---|---|---|
| 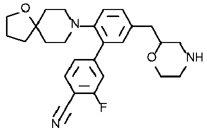 | 1H NMR (600 MHz, DMSO-d6) δ 7.97 (t, J = 7.6 Hz, 1H), 7.80 (dd, J = 11.1, 1.2 Hz, 1H), 7.70 (dd, J = 8.1, 1.5 Hz, 1H), 7.21 (dd, J = 8.2, 2.1 Hz, 1H), 7.14 (d, J = 1.9 Hz, 1H), 7.07 (d, J = 8.2 Hz, 1H), 3.92 (s, 1H), 3.83 (brdd, J = 12.1, 2.9 Hz, 1H), 3.75 - 3.63 (m, 3H), 3.57 - 3.49 (m, 1H), 3.45 - 3.09 (m, 5H), 3.04 - 2.91 (m, 2H), 2.87 - 2.75 (m, 3H), 2.74 - 2.55 (m, 5H), 1.87 - 1.77 (m, 2H), 1.69 - 1.58 (m, 2H), 1.55 - 1.40 (m, 4H). m/z: 436 [M+H]+ | | | |
| 3-fluoro-5'-[(morpholin-2-yl)methyl]-2'-{1-oxa-8-azaspiro[4.5]decan-8-yl}-[1,1'-biphenyl]-4-carbonitrile | | | | |

| Example 166 | Procedure: B1+F1+E4+N3+H0+G8+B2+D1 G8+B2+D1 | SM1: 2-bromo-3-chlorobenzaldehyde | SM2: cyclopropylboronic acid | Yield: 39% |
|---|---|---|---|---|
| 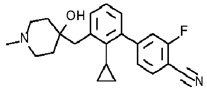 | 1H NMR (600 MHz, DMSO-d6, 300K) δ ppm 7.97 (t, J=7.5 Hz, 1 H), 7.58 (dd, J=10.6, 1.0 Hz, 1 H), 7.45 (dd, J=8.1, 1.3 Hz, 1 H), 7.31 - 7.36 (m, 1 H), 7.27 (t, J=7.6 Hz, 1 H), 7.16 (dd, J=7.5, 1.2 Hz, 1 H), 4.38 - 4.91 (m, 1 H), 3.94 (br s, 2 H), 3.11 (s, 2 H), 2.75 - 3.04 (m, 4 H), 2.57 (br s, 3 H), 2.03 (br s, 1 H), 1.55 - 1.86 (m, 4 H), 0.68 - 0.84 (m, 2 H), -0.11 - 0.02 (m, 2 H). m/z: 365 [M+H]+ | | | |
| 2'-cyclopropyl-3-fluoro-3'-[(4-hydroxy-1-methylpiperidin-4-yl)methyl]-[1,1'-biphenyl]-4-carbonitrile | | | | |

| Example 154 | Procedure: G1+C1+B2+D1 | SM1: 2-bromo-1-(bromomethyl)-3-chlorobenzene chlorobenzene | SM2: tert-butyl 3-hydroxypyrrolidine-1-carboxylate carboxylate | Yield: 3% |
|---|---|---|---|---|
| 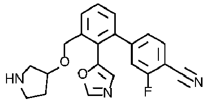 | 1H NMR (DMSO-d6, 500 MHz): δ (ppm) 8.66-9.01 (m, 2H), 8.32 (s, 1H), 7.87 (t, J = 7.6 Hz, 1H), 7.69-7.73 (m, 1H), 7.63-7.68 (m, 1H), 7.51 (dd, J = 7.6, 1.2 Hz, 1H), 7.33 (dd, J = 10.5, 1.2 Hz, 1H), 7.19 (s, 1H), 7.11 (dd, J = 8.1, 1.5 Hz, 1H), 4.39-4.52 (m, 2H), 4.22 (br t, J = 4.4 Hz, 1H), 3.10-3.28 (m, 4H), 1.89-2.07 (m, 2H). m/z: 364 [M+H]+ | | | |
| 3-fluoro-2'-(1,3-oxazol-5-yl)-3'-[(pyrrolidin-3-yloxy)methyl]-[1,1'-biphenyl]-4-carbonitrile | | | | |

| Example 98 | Procedure: B1+B2+D1 | SM1: BB-2 | SM2: (3-fluoro-4-methoxyphenyl)boronic acid methoxyphenyl)boroni c acid | Yield: 62% |
|---|---|---|---|---|
| 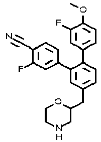 | 1H NMR (DMSO-d6, 500 MHz): δ (ppm) 11.02-13.51 (m, 1H), 8.16-9.59 (m, 1H), 7.83 (t, J = 7.5 Hz, 1H), 7.32-7.45 (m, 4H), 7.04-7.10 (m, 2H), 6.99 (dd, J = 12.3, 2.1 Hz, 1H), 6.82 (dd, J = 8.4, 1.1 Hz, 1H), 4.41-6.27 (m, 1H), 4.30 (s, 2H), 3.86-4.00 (m, 2H), 3.81 (s, 3H), 3.64 (br t, J = 11.7 Hz, 1H), 3.32-3.35 (m, 2H), 3.14-3.27 (m, 2H), 2.94-3.04 (m, 1H), 2.79-2.92 (m, 3H). m/z: 421.3 [M+H]+ | | | |
| 3-fluoro-2'-(3-fluoro-4-methoxyphenyl)-5'-[(morpholin-2-yl)methyl]-[1,1'-biphenyl]-4-carbonitrile | | | | |

| Example 58 | Procedure: B1+B2+D1 | SM1: BB-2 | SM2: 5-boranyl-2-methyl-2H-indazole | Yield: 96% |
|---|---|---|---|---|
| 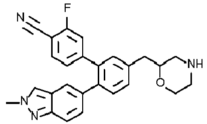 | 1H NMR (500 MHz, DMSO-d6) δ ppm 8.29 (s, 1 H) 7.84 - 8.15 (m, 2 H) 7.74 (s, 1 H) 7.51 (s, 1 H) 7.42 - 7.47 (m, 2 H) 7.40 (s, 1 H) 7.35 (s, 1 H) 7.29 - 7.34 (m, 1 H) 7.08 (dd, J=8.07, 1.22 Hz, 1 H) 6.86 (dd, J=8.93, 1.59 Hz, 1 H) 4.15 (s, 3 H) 3.81 - 3.86 (m, 1 H) 3.73 - 3.80 (m, 1 H) 3.51 - 3.56 (m, 1 H) 2.99 - 3.06 (m, 1 H) 2.88 - 2.95 (m, 1 H) 2.81 (br d, J=6.36 Hz, 3 H) 2.59 - 2.68 (m, 1 H). m/z: 427.3 [M+H]+ | | | |
| 3-fluoro-2'-(2-methyl-2H-indazol-5-yl)-5'-[(morpholin-2-yl)methyl]-[1,1'-biphenyl]-4-carbonitrile | | | | |

| Example 56 | Procedure: B1+B2+D1 | SM1: BB-2 | SM2: 1-methyl-5-(4,4,5,5-tetramethyl-1,3,2-dioxaborolan-2-yl)-1H-pyrazolo[3,4-b]pyridine | Yield: 58% |
|---|---|---|---|---|
| 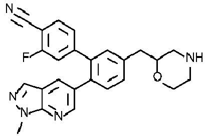 | 1H NMR (DMSO-d6, 600 MHz): δ (ppm) 8.22 (d, J = 2.1 Hz, 1H), 8.11 (s, 1H), 8.05 (d, J = 2.1 Hz, 1H), 7.75 (t, J = 7.4 Hz, 1H), 7.49 (d, J = 7.8 Hz, 1H), 7.45 (dd, J = 7.8, 1.7 Hz, 1H), 7.38-7.42 (m, 2H), 7.06 (dd, J = 8.1, 1.5 Hz, 1H), 6.22-8.51 (m, 2H), 4.03 (s, 3H), 3.87 (s, 2H), 3.81 (dd, J = 11.6, 2.6 Hz, 1H), 3.72-3.77 (m, 1H), 3.51 (td, J = 11.7, 2.3 Hz, 1H), 3.30-3.45 (m, 3H), 2.99 (brd, J = 12.0 Hz, 1H), 2.87 (brd, J = 12.3 Hz, 1H), 2.82 (d, J = 6.7 Hz, 2H), 2.75-2.80 (m, 1H), 2.61 (dd, J = 12.2, 10.6 Hz, 1H). m/z: 428.3 [M+H]+ | | | |
| 3-fluoro-2'-{1-methyl-1H-pyrazolo[3,4-b]pyridin-5-yl}-5'-[(morpholin-2-yl)methyl]-[1,1'-biphenyl]-4-carbonitrile | | | | |

| Example 83 | Procedure: B1+F3+G1+B2+D1 | SM1: BB-2 | SM2: [2-fluoro-4-(2-methoxy-2-oxoethyl)phenyl]boron ic acid | Yield: 86% |
|---|---|---|---|---|
| 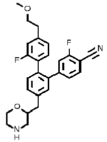 | 1H NMR (DMSO-d6, 600 MHz) δ 7.79 (dd, 1H, J=7.2, 7.8 Hz), 7.4-7.4 (m, 1H), 7.3-7.4 (m, 2H), 7.25 (dd, 1H, J=1.4, 10.6 Hz), 7.19 (t, 1H, J=7.9 Hz), 7.08 (ddd, 2H, J=1.6, 3.2, 8.0 Hz), 6.97 (dd, 1H, J=1.1, 11.2 Hz), 3.95 (br s, 2H), 3.8-3.9 (m, 2H), 3.5-3.6 (m, 4H), 3.3-3.4 (m, 3H), 3.2-3.2 (m, 3H), 3.0-3.1 (m, 1H), 2.9-3.0 (m, 1H), 2.8-2.9 (m, 5H), 2.6-2.7 (m, 1H). m/z: 449.3 [M+H]+ | | | |
| 3-fluoro-2'-[2-fluoro-4-(2-methoxyethyl)phenyl]-5'-[(morpholin-2-yl)methyl]-[1,1'-biphenyl]-4-carbonitrile | | | | |

| Example 86 | Procedure: P1+D5+A4+B2+D1 | SM1: 5-iodo-2-methoxypyridine | SM2: BB-2 (step 3) | Yield: 45% |
|---|---|---|---|---|
| 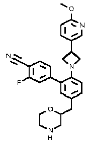 | 1H NMR (DMSO-d6, 600 MHz) δ 8.03 (d, 1H, J=2.5 Hz), 7.9-8.0 (m, 1H), 7.69 (dd, 1H, J=2.5, 8.7 Hz), 7.56 (dd, 1H, J=1.3, 10.6 Hz), 7.46 (dd, 1H, J=1.5, 8.0 Hz), 7.18 (dd, 1H, J=2.1, 8.4 Hz), 7.01 (d, 1H, J=1.9 Hz), 6.78 (d, 1H, J=8.7 Hz), 6.67 (d, 1H, J=8.4 Hz), 3.91 (s, 2H), 3.8-3.9 (m, 6H), 3.6-3.7 (m, 2H), 3.53 (dt, 1H, J=2.0, 11.9 Hz), 3.4-3.4 (m, 2H), 3.2-3.3 (m, 5H), 2.9-3.0 (m, 2H), 2.81 (dt, 1H, J=3.5, 12.1 Hz), 2.5-2.7 (m, 3H). m/z: 459 [M+H]+ | | | |
| 3-fluoro-2'-[3-(6-methoxypyridin-3-yl)azetidin-1-yl]-5'-[(morpholin-2-yl)methyl]-[1,1'-biphenyl]-4-carbonitrile | | | | |

| Example 101 | Procedure: B5+A4+B2+D1 | SM1: 5-(azetidin-3-yl)-2-bromopyridine | SM2: BB-2 (step 2) | Yield: 45% |
|---|---|---|---|---|
| 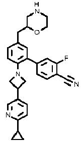 | 1H NMR (DMSO-d6, 600 MHz): δ (ppm) 8.28 (d, J = 2.2 Hz, 1H), 7.94 (t, J = 7.6 Hz, 1H), 7.58 (dd, J = 8.1, 2.4 Hz, 1H), 7.55 (dd, J = 10.6, 1.2 Hz, 1H), 7.46 (dd, J = 8.1, 1.5 Hz, 1H), 7.23 (d, J = 8.4 Hz, 1H), 7.18 (dd, J = 8.4, 1.9 Hz, 1H), 7.02 (d, J = 1.9 Hz, 1H), 6.68 (d, J = 8.4 Hz, 1H), 4.00 (br s, 2H), 3.81-3.91 (m, 3H), 3.66-3.76 (m, 2H), 3.52-3.60 (m, 1H), 3.41-3.44 (m, 2H), 3.31-3.34 (m, 5H), 3.04 (br t, J = 13.2 Hz, 2H), 2.83-2.91 (m, 1H), 2.62-2.73 (m, 3H), 2.01-2.08 (m, 1H), 0.82-0.94 (m, 4H). m/z: 469 [M+H]+ | | | |
| 2'-[3-(6-cyclopropylpyridin-3-yl)azetidin-1-yl]-3-fluoro-5'-[(morpholin-2-yl)methyl]-[1,1'-biphenyl]-4-carbonitrile | | | | |

| Example 92 | Procedure: B8+B2+D1 | SM1: BB-4 | SM2: 4-bromo-1,3-oxazole | Yield: 78% |
|---|---|---|---|---|
| 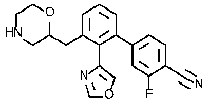 | 1H NMR (DMSO-d6, 600 MHz) δ 8.42 (d, 1H, J=1.0 Hz), 7.85 (d, 1H, J=0.9 Hz), 7.81 (dd, 1H, J=7.2, 7.9 Hz), 7.5-7.5 (m, 1H), 7.4-7.5 (m, 1H), 7.35 (dd, 1H, J=1.2, 7.6 Hz), 7.28 (dd, 1H, J=1.3, 10.6 Hz), 7.09 (dd, 1H, J=1.5, 8.1 Hz), 3.95 (br s, 1H), 3.81 (br dd, 1H, J=2.7, 12.1 Hz), 3.0-3.7 (m, 6H), 2.8-3.0 (m, 3H), 2.7-2.7 (m, 2H), 2.57 (br t, 1H, J=11.4 Hz), m/z: 364 [M+H]+ | | | |
| 3-fluoro-3'-[(morpholin-2-yl)methyl]-2'-(1,3-oxazol-4-yl)-[1,1'-biphenyl]-4-carbonitrile | | | | |

| Example 27 | Procedure: C1+B2+D1 | SM1: BB-1 | SM2: 1,3-oxazole | Yield: 84% |
|---|---|---|---|---|
| 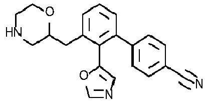 | 1H NMR (DMSO-d6, 600 MHz) δ 8.30 (s, 1H), 7.76 (d, 2H, J=8.5 Hz), 7.5-7.6 (m, 1H), 7.49 (dd, 1H, J=1.0, 7.8 Hz), 7.37 (dd, 1H, J=1.2, 7.6 Hz), 7.30 (d, 2H, J=8.5 Hz), 7.02 (s, 1H), 3.87 (s, 1H), 3.76 (dd, 1H, J=2.8, 11.7 Hz), 3.5-3.6 (m, 1H), 3.42 (dt, 2H, J=2.5, 11.7 Hz), 3.36 (br s, 2H), 2.8-2.9 (m, 2H), 2.6-2.8 (m, 3H), 2.4-2.5 (m, 1H). m/z: 346.3 [M+H]+ | | | |
| 3'-[(morpholin-2-yl)methyl]-2'-(1,3-oxazol-5-yl)-[1,1'-biphenyl]-4-carbonitrile | | | | |

| Example 34 | Procedure: C2+B2+D1 | SM1: BB-1 | SM2: 1,3-thiazole | Yield: 58% |
|---|---|---|---|---|
| 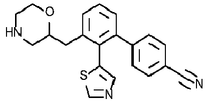 | 1H NMR (DMSO-d6, 500 MHz) δ 9.04 (s, 1H), 7.8-8.8 (m, 2H), 7.6-7.7 (m, 3H), 7.4-7.6 (m, 2H), 7.2-7.4 (m, 3H), 3.94 (s, 2H), 3.79 (dd, 1H, J=2.8, 11.9 Hz), 3.6-3.7 (m, 1H), 3.46 (dt, 1H, J=2.1, 11.9 Hz), 3.39 (s, 2H), 2.8-3.0 (m, 2H), 2.78 (dt, 1H, J=3.4, 12.2 Hz), 2.66 (d, 2H, J=6.6 Hz), 2.5-2.6 (m, 1H. m/z: 362 [M+H]+ | | | |
| 3'-[(morpholin-2-yl)methyl]-2'-(1,3-thiazol-5-yl)-[1,1'-biphenyl]-4-carbonitrile | | | | |

| Example 38 | Procedure: B3+B2+D1 | SM1: BB-1 | SM2: 4-methyl-5-(4,4,5,5-tetramethyl-1,3,2-dioxaborolan-2-yl)-1,3-thiazole | Yield: 86% |
|---|---|---|---|---|
| 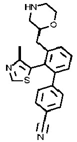 | 1H NMR (DMSO-d6, 600 MHz): δ (ppm) 8.97 (d, J = 5.1 Hz, 1H), 7.67-7.74 (m, 2H), 7.50-7.55 (m, 1H), 7.45-7.50 (m, 1H), 7.31 (d, J = 7.5 Hz, 1H), 7.26 (dd, J = 8.6, 2.3 Hz, 2H), 6.59-8.23 (m, 2H), 3.87 (s, 2H), 3.72 (br d, J = 11.7 Hz, 1H), 3.49 (qd, J = 7.8, 5.5 Hz, 1H), 3.35-3.42 (m, 1H), 2.91-3.32 (m, 3H), 2.80 (br dd, J = 11.3, 3.2 Hz, 1H), 2.63-2.77 (m, 3H), 2.51-2.60 (m, 1H), 2.38-2.46 (m, 1H), 1.86-1.97 (m, 3H). m/z: 376.2 [M+H]+ | | | |
| 2'-(4-methyl-1,3-thiazol-5-yl)-3'-[(morpholin-2-yl)methyl]-[1,1'-biphenyl]-4-carbonitrile | | | | |

| Example 24 | Procedure: B3+B2+D1 | SM1: BB-1 | SM2: 4-methyl-5-(4,4,5,5-tetramethyl-1,3,2-dioxaborolan-2-yl)-1,3-thiazole | Yield: 39% |
|---|---|---|---|---|
| 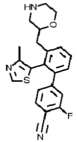 | 1H NMR (DMSO-d6, 500 MHz) δ 9.01 (s, 1H), 7.80 (t, 1H, J=7.5 Hz), 7.5-7.6 (m, 2H), 7.35 (d, 1H, J=7.1 Hz), 7.2-7.3 (m, 1H), 7.09 (dd, 1H, J=1.5, 8.1 Hz), 3.87 (s, 1H), 2.8-3.8 (m, 6H), 2.6-2.8 (m, 4H), 2.5-2.6 (m, 1H), 2.41 (dt, 1H, J=7.2, 11.2 Hz), 1.94 (s, 3H). m/z: 394 [M+H]+ | | | |
| 3-fluoro-2'-(4-methyl-1,3-thiazol-5-yl)-3'-[(morpholin-2-yl)methyl]-[1,1'-biphenyl]-4-carbonitrile | | | | |

| Example 124 | Procedure: B1+B2+D1 | SM1: BB-2 | SM2: 4-methyl-3-(4,4,5,5-tetramethyl-1,3,2-dioxaborolan-2-yl)pyridine | Yield: 74% |
|---|---|---|---|---|
| 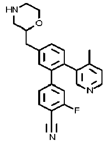 | 1H NMR (DMSO-d6, 500 MHz) δ 8.34 (d, 1H, J=4.9 Hz), 8.20 (d, 1H, J=8.1 Hz), 7.77 (t, 1H, J=7.6 Hz), 7.4-7.5 (m, 2H), 7.3-7.4 (m, 1H), 7.26 (d, 1H, J=10.5 Hz), 7.19 (brd, 1H, J=4.4 Hz), 7.06 (dd, 1H, J=1.5, 8.1 Hz), 3.89 (s, 1H), 3.7-3.9 (m, 3H), 3.5-3.6 (m, 2H), 3.1-3.4 (m, 3H), 3.02 (br d, 1H, J=12.2 Hz), 2.92 (br d, 1H, J=12.0 Hz), 2.8-2.9 (m, 3H), 2.64 (br t, 1H, J=11.5 Hz), 1.93 (d, 3H, J=7.1 Hz), m/z: 388 [M+H]+ | | | |
| 3-fluoro-2'-(4-methylpyridin-3-yl)-5'-[(morpholin-2-yl)methyl]-[1,1'-biphenyl]-4-carbonitrile | | | | |

| Example 85 | Procedure: F4+E4+N1+D4+F3+ I1+B0+B1+D1 | SM1: methyl 3-bromo-4-iodo-5-methylbenzoate | SM2: 4-[(4-methoxyphenyl)methy l]morpholin-3-one | Yield: 58% |
|---|---|---|---|---|
| 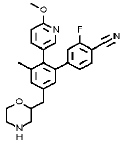 | 1H NMR (DMSO-d6, 600 MHz): δ (ppm) 7.82 (brd, J = 4.7 Hz, 1H), 7.73-7.78 (m, 1H), 7.45 (br t, J = 6.9 Hz, 1H), 7.29 (s, 1H), 7.25 (dd, J = 10.5, 1.1 Hz, 1H), 7.15 (d, J = 0.7 Hz, 1H), 7.04 (dd, J = 7.9, 1.5 Hz, 1H), 6.75 (d, J = 8.5 Hz, 1H), 6.30-9.07 (m, 2H), 3.95 (s, 2H), 3.86 (dd, J = 12.0, 2.9 Hz, 1H), 3.81 (s, 3H), 3.76-3.80 (m, 1H), 3.52-3.59 (m, 1H), 3.22-3.43 (m, 3H), 3.08 (br d, J = 12.2 Hz, 1H), 2.98 (br d, J = 12.3 Hz, 1H), 2.85 (td, J = 12.2, 3.4 Hz, 1H), 2.77 (br d, J = 6.6 Hz, 2H), 2.68 (br t, J = 11.4 Hz, 1H), 2.11 (s, 3H). m/z: 418 [M+H]+ | | | |
| 3-fluoro-2'-(6-methoxypyridin-3-yl)-3'-methyl-5'-[(morpholin-2-yl)methyl]-[1,1'-biphenyl]-4-carbonitrile | | | | |

| Example 94 | Procedure: B1 +B2+D1 | SM1: BB-2 | SM2: [4-(2-methoxyethoxy)phenyl]boronic acid l]boronic acid | Yield: 80% |
|---|---|---|---|---|
| 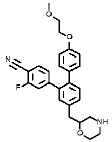 | 1H NMR (DMSO-d6, 500 MHz): δ (ppm) 12.49-12.81 (m, 1H), 8.63-8.98 (m, 2H), 7.81 (t, J = 7.6 Hz, 1H), 7.38 (s, 2H), 7.33 (s, 1H), 7.30 (dd, J = 10.6, 1.3 Hz, 1H), 7.07 (dd, J = 8.1, 1.5 Hz, 1H), 7.01 (d, J = 8.8 Hz, 2H), 6.86 (d, J = 8.8 Hz, 2H), 4.88-5.25 (m, 2H), 4.31 (s, 2H), 4.06 (dd, J = 5.4, 3.7 Hz, 2H), 3.96 (dd, J = 12.7, 2.9 Hz, 1H), 3.87-3.93 (m, 1H), 3.60-3.69 (m, 3H), 3.30 (s, 3H), 3.16-3.27 (m, 2H), 2.94-3.07 (m, 1H), 2.78-2.93 (m, 3H). m/z: 447 [M+H]+ | | | |
| 3-fluoro-2'-[4-(2-methoxyethoxy)phenyl]-5'-[(morpholin-2-yl)methyl]-[1,1'-biphenyl]-4-carbonitrile | | | | |

| Example 91 | Procedure: B1+B2+E1+D2 | SM1: BB-2 | SM2: (2-methyl-2H-indazol-5-yl)boronic acid indazol-5-yl)boronic acid | Yield: 82% |
|---|---|---|---|---|
| 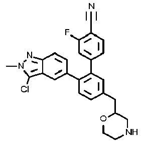 | 1H NMR (DMSO-d6, 500 MHz): δ (ppm) 9.21-9.42 (m, 2H), 7.77 (t, J = 7.5 Hz, 1H), 7.51 (d, J = 7.8 Hz, 1H), 7.41-7.47 (m, 3H), 7.35-7.40 (m, 2H), 7.10 (dd, J = 8.1, 1.5 Hz, 1H), 6.88 (dd, J = 8.8, 1.7 Hz, 1H), 4.12 (s, 3H), 4.00-4.03 (m, 1H), 3.94-3.99 (m, 1H), 3.72 (td, J = 12.3, 2.1 Hz, 1H), 3.24 (br d, J = 12.0 Hz, 1H), 3.13-3.20 (m, 1H), 2.78-3.01 (m, 4H). m/z: 461 [M+H]+ | | | |
| 2'-(3-chloro-2-methyl-2H-indazol-5-yl)-3-fluoro-5'-[(morpholin-2-yl)methyl]-[1,1'-biphenyl]-4-carbonitrile | | | | |

| Example 96 | Procedure: B2+B2+D1 | SM1: BB-2 | SM2: [6-(cyclopropylmethoxy) pyridin-3-yl]boronic acid | Yield: 66% |
|---|---|---|---|---|
| 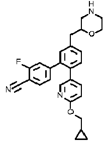 | 1H NMR (DMSO-d6, 600 MHz) δ 7.91 (d, 1H, J=2.5 Hz), 7.8-7.9 (m, 1H), 7.40 (d, 2H, J=0.9 Hz), 7.3-7.4 (m, 3H), 7.10 (dd, 1H, J=1.5, 8.1 Hz), 6.7-6.7 (m, 1H), 4.06 (d, 2H, J=7.0 Hz), 3.87 (s, 1H), 3.80 (br dd, 1H, J=2.6, 11.7 Hz), 3.7-3.8 (m, 1H), 3.50 (dt, 1H, J=2.0, 11.7 Hz), 3.1-3.4 (m, 2H), 2.99 (br d, 1H, J=12.3 Hz), 2.88 (br d, 1H, J=12.2 Hz), 2.7-2.8 (m, 3H), 2.6-2.6 (m, 1H), 1.1-1.2 (m, 1H), 0.5-0.6 (m, 2H), 0.2-0.3 (m, 2H). m/z: 444 [M+H]+ | | | |
| 2'-[6-(cyclopropylmethoxy)pyridin-3-yl]-3-fluoro-5'-[(morpholin-2-yl)methyl]-[1,1'-biphenyl]-4-carbonitrile | | | | |

| Example 93 | Procedure: B1+B2+D1 | SM1: BB-2 | SM2: [6-(pyrrolidin-1-yl)pyridin-3-yl]boronic acid | Yield: 47% |
|---|---|---|---|---|
| 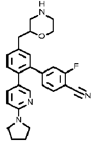 | 1H NMR (DMSO-d6, 400 MHz) δ (ppm) 7.90 - 7.80 (m, 2H), 7.41 - 7.33 (m, 3H), 7.30 (s, J = 1.5 Hz, 1H), 7.21 - 7.05 (m, 2H), 6.34 (d, J = 8.8, 0.8 Hz, 1H), 3.92 (s, 2H), 3.85 (dd, J = 11.8, 3.3 Hz, 1H), 3.77 (m, J = 10.7, 6.6 Hz, 2H), 3.62 - 3.48 (td, 2H), 3.41 - 3.28 (t, 4H), 3.05 (d, J = 12.3 Hz, 1H), 2.95 (d, J = 12.4 Hz, 1H), 2.81 (m, J = 11.4, 4.9 Hz, 3H), 2.71 - 2.60 (t, 1H), 1.99 - 1.87 (m, 4H).. m/z: 443 [M+H]+ | | | |
| 3-fluoro-5'-[(morpholin-2-yl)methyl]-2'-[6-(pyrrolidin-1-yl)pyridin-3-yl]-[1,1'-biphenyl]-4-carbonitrile | | | | |

| Example 104 | Procedure: B1+B2+D2 | SM1: BB-3 | SM2: 1-(4-bromo-3- fluorophenyl)-2-methylpropan-2-ol methylpropan-2-ol | Yield: 74% |
|---|---|---|---|---|
| 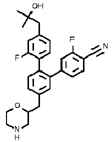 | 1H NMR (600 MHz, DMSO-d6) δ ppm 8.04 - 9.96 (m, 2 H) 7.79 (s, 1 H) 7.36 - 7.45 (m, 3 H) 7.18 - 7.23 (m, 1 H) 7.14 - 7.18 (m, 1 H) 7.09 - 7.13 (m, 1 H) 7.02 - 7.08 (m, 1 H) 6.89 - 6.96 (m, 1 H) 4.36 (s, 1 H) 3.87 - 4.01 (m, 2 H) 3.61 - 3.74 (m, 1 H) 3.21 - 3.26 (m, 1 H) 3.12 - 3.19 (m, 1 H) 2.94 - 3.03 (m, 1 H) 2.85 - 2.92 (m, 2 H) 2.75 - 2.85 (m, 1 H) 2.64 (s, 2 H) 0.95 - 1.13 (m, 6 H). m/z: 463 [M+H]+ | | | |
| 3-fluoro-2'-[2-fluoro-4-(2-hydroxy-2-methylpropyl)phenyl]-5'-[(morpholin-2-yl)methyl]-[1,1'-biphenyl]-4-carbonitrile | | | | |

| Example 75 | Procedure: M1+B2+D1 | SM1: BB-1 | SM2: (cyclopent-1-en-1-yl)boronic acid | Yield: 36% |
|---|---|---|---|---|
| 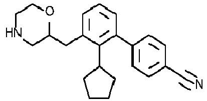 | 1H NMR (DMSO-d6, 500 MHz) δ 7.87 (d, 2H, J=8.3 Hz), 7.45 (d, 2H, J=8.1 Hz), 7.33 (brd, 1H, J=7.6 Hz), 7.20 (t, 1H, J=7.7 Hz), 6.9-7.0 (m, 1H), 3.89 (s, 1H), 2.6-3.9 (m, 14H), 1.3-2.0 (m, 8H). m/z: 345.3 [M+H]+ | | | |
| 2'-cyclopentyl-3'-[(morpholin-2-yl)methyl]-[1,1'-biphenyl]-4-carbonitrile | | | | |

| Example 153 | Procedure: C3+B2+B9+B2+D2 | SM1: BB-1 | SM2: 1-methyl-1H-pyrazole | Yield: 78% |
|---|---|---|---|---|
| 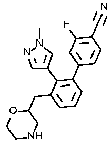 | 1H NMR (DMSO-d6, 500 MHz) δ 8.9-9.3 (m, 2H), 7.78 (t, 1H, J=7.5 Hz), 7.47 (s, 1H), 7.42 (d, 2H, J=4.6 Hz), 7.2-7.3 (m, 2H), 7.12 (s, 1H), 7.09 (dd, 1H, J=1.5, 8.1 Hz), 3.77 (s, 6H), 2.8-3.2 (m, 3H), 2.72 (d, 3H, J=6.4 Hz), m/z: 377 [M+H]+ | | | |
| 3-fluoro-2'-(1-methyl-1H-pyrazol-4-yl)-3'-[(morpholin-2-yl)methyl]-[1,1'-biphenyl]-4-carbonitrile | | | | |

| Example 84 | Procedure: K2+B2+D1 | SM1: BB-1 | | Yield: 81% |
|---|---|---|---|---|
| 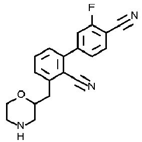 | 1H NMR (DMSO-d6, 500 MHz): δ (ppm) 8.11 (t, J = 7.2 Hz, 1H), 7.80-7.86 (m, 1H), 7.74-7.79 (m, 1H), 7.62-7.66 (m, 1H), 7.52-7.62 (m, 2H), 3.99 (s, 2H), 3.79-3.88 (m, 2H), 3.51 (br d, J = 2.0 Hz, 1H), 2.92-3.10 (m, 4H), 2.79-2.88 (m, 1H), 2.67-2.77 (m, 1H). m/z: 322.1 [M+H]+ | | | |
| 3'-fluoro-3-[(morpholin-2-yl)methyl]-[1,1'-biphenyl]-2,4'-dicarbonitrile | | | | |

| Example 39 | Procedure: K3+F4+E4+N1+D4+ F3+I1+C2+B1+D1 | SM1: ethyl 2-amino-3-bromo-5-methylbenzoate | SM2: 4-[(4-methoxyphenyl)methy l]morpholin-3-one | Yield: 87% |
|---|---|---|---|---|
| 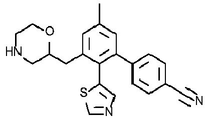 | 1H NMR (600 MHz, DMSO-d6) δ 9.02 (d, J = 0.7 Hz, 1H), 8.52 - 7.76 (m, 1H), 7.69 (d, J = 8.5 Hz, 2H), 7.64 (d, J = 0.6 Hz, 1H), 7.59 - 7.42 (m, 1H), 7.32 - 7.29 (m, 2H), 7.28 (d, J = 1.0 Hz, 1H), 7.14 - 7.11 (m, 1H), 3.74 (dd, J = 11.6, 2.8 Hz, 1H), 3.51 (br s, 1H), 3.42 - 3.38 (m, 1H), 2.85 - 2.79 (m, 1H), 2.78 - 2.74 (m, 1H), 2.73 - 2.66 (m, 1H), 2.64 - 2.57 (m, 2H), 2.45 - 2.40 (m, 1H), 2.38 (s, 3H). m/z: 376 [M+H]+ | | | |
| 5'-methyl-3'-[(morpholin-2-yl)methyl]-2'-(1,3-thiazol-5-yl)-[1,1'-biphenyl]-4-carbonitrile | | | | |

| Example 36 | Procedure: B6+B2+D1 | SM1: BB-4 | SM2: 5-bromo-1,2-thiazole | Yield: 91% |
|---|---|---|---|---|
| 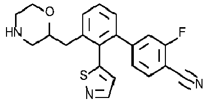 | 1H NMR (DMSO-d6, 500 MHz) δ 8.52 (d, 1H, J=1.7 Hz), 7.9-8.4 (m, 1H), 7.7-7.9 (m, 1H), 7.5-7.6 (m, 2H), 7.3-7.4 (m, 3H), 7.17 (dd, 1H, J=1.6, 7.9 Hz), 3.98 (s, 2H), 3.80 (br dd, 1H, J=2.7, 12.0 Hz), 3.6-3.7 (m, 1H), 3.47 (dt, 3H, J=2.1, 11.9 Hz), 2.94 (br d, 1H, J=12.2 Hz), 2.88 (br d, 1H, J=12.2 Hz), 2.80 (dt, 1H, J=3.4, 12.2 Hz), 2.6-2.7 (m, 2H), 2.54 (dd, 1H, J=1 0.8, 12.2 Hz), m/z: 360 [M+H]+ | | | |
| 3-fluoro-3'-[(morpholin-2-yl)methyl]-2'-(1,2-thiazol-5-yl)-[1,1'-biphenyl]-4-carbonitrile | | | | |

| Example 29 | Procedure: C1+E3+B2+D2 | SM1: BB-1 | SM2: 1,3-oxazole | Yield: 83% |
|---|---|---|---|---|
| 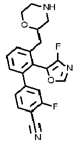 | 1H NMR (DMSO-d6, 500 MHz) δ 9.12 (br s, 2H), 8.35 (d, 1H, J=2.0 Hz), 7.8-8.0 (m, 1H), 7.6-7.7 (m, 1H), 7.5-7.6 (m, 1H), 7.47 (dd, 1H, J=1.1, 7.7 Hz), 7.36 (dd, 1H, J=1.3, 10.4 Hz), 7.10 (dd, 1H, J=1.6, 7.9 Hz), 3.87 (dd, 1H, J=3.4, 12.7 Hz), 3.7-3.8 (m, 1H), 3.6-3.6 (m, 1H), 3.12 (brd, 2H, J=12.5 Hz), 2.9-3.0 (m, 1H), 2.7-2.8 (m, 3H). m/z: 382 [M+H]+ | | | |
| 3-fluoro-2'-(4-fluoro-1,3-oxazol-5-yl)-3'-[(morpholin-2-yl)methyl]-[1,1'-biphenyl]-4-carbonitrile | | | | |

| Example 19 | Procedure: F4+E4+N1+D4+F3+ I1+B1+C1+D2 | SM1: methyl 2-bromo-3-chloro-5-fluorobenzoate | SM2: 4-[(4-methoxyphenyl)methy l]morpholin-3-one | Yield: 43% |
|---|---|---|---|---|
| 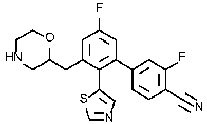 | 1H NMR (DMSO-d6, 500 MHz) δ 9.08 (s, 1H), 7.81 (t, 1H, J=7.5 Hz), 7.72 (s, 1H), 7.3-7.5 (m, 2H), 7.28 (dd, 1H, J=2.4, 8.8 Hz), 7.17 (d, 1H, J=7.8 Hz), 3.98 (s, 2H), 3.0-3.9 (m, 9H), 2.7-3.0 (m, 3H), 2.6-2.7 (m, 2H). m/z: 398 [M+H]+ | | | |
| 3,5'-difluoro-3'-[(morpholin-2-yl)methyl]-2'-(1,3-thiazol-5-yl)-[1,1'-biphenyl]-4-carbonitrile | | | | |

| Example 25 | Procedure: O2+H0+G0+I1+G3 C2+B2+D2 | SM1: 2-bromo-1-chloro-4-fluoro-3-iodobenzene | SM2: tributyl(prop-2-en-1-yl)stannane | Yield: 95% |
|---|---|---|---|---|
| 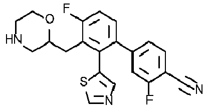 | 1H NMR (DMSO-d6, 500 MHz): δ (ppm) 9.22 (br s, 2H), 9.11 (s, 1H), 7.75-7.87 (m, 2H), 7.41-7.53 (m, 2H), 7.33 (dd, J = 10.5, 1.2 Hz, 1H), 7.13 (dd, J = 8.1, 1.5 Hz, 1H), 3.88 (dd, J = 12.7, 3.2 Hz, 1H), 3.77-3.84 (m, 1H), 3.55-3.63 (m, 1H), 3.10 (br d, J = 12.5 Hz, 2H), 2.86-2.97 (m, 1H), 2.61-2.81 (m, 3H). m/z: 398 [M+H]+ | | | |
| 3,4'-difluoro-3'-[(morpholin-2-yl)methyl]-2'-(1,3-thiazol-5-yl)-[1,1'-biphenyl]-4-carbonitrile | | | | |

| Example 123 | Procedure: A2+B2+D1 | SM1: BB-2 | SM2: (3S)-oxan-3-amine | Yield: 74% |
|---|---|---|---|---|
| 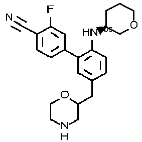 | 1H NMR (DMSO-d6, 600 MHz) δ 7.98 (t, 1H, J=7.6 Hz), 7.55 (dd, 1H, J=1.2, 10.6 Hz), 7.45 (dd, 1H, J=1.5, 8.1 Hz), 7.07 (dd, 1H, J=1.9, 8.4 Hz), 6.91 (d, 1H, J=2.1 Hz), 6.76 (d, 1H, J=8.5 Hz), 4.50 (d, 1H, J=8.4 Hz), 3.89 (s, 1H), 3.0-3.9 (m, 14H), 2.9-3.0 (m, 2H), 2.80 (dt, 1H, J=3.1, 12.2 Hz), 2.5-2.7 (m, 3H), 1.8-1.9 (m, 1H), 1.4-1.6 (m, 3H). m/z: 396.2 [M+H]+ | | | |
| 3-fluoro-5'-[(morpholin-2-yl)methyl]-2'-{[(3S)-oxan-3-yl]amino}-[11'-biphenyl]-4-carbonitrile | | | | |

| Example 122 | Procedure: A1+B2+D1 | SM1: BB-2 | SM2: 3-(propan-2-yl)azetidin-3-ol hydrochloride | Yield: 48% |
|---|---|---|---|---|
| 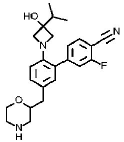 | 1H NMR (DMSO-d6, 500 MHz) δ 8.1-8.8 (m, 2H), 7.95 (t, 1H, J=7.6 Hz), 7.49 (dd, 1H, J=1.3, 10.6 Hz), 7.41 (dd, 1H, J=1.5, 8.1 Hz), 7.14 (dd, 1H, J=2.0, 8.3 Hz), 7.00 (d, 1H, J=2.0 Hz), 6.62 (d, 1H, J=8.6 Hz), 4.7-5.8 (m, 1H), 3.90 (s, 1H), 3.83 (dd, 1H, J=2.7, 12.0 Hz), 3.6-3.7 (m, 1H), 3.5-3.6 (m, 2H), 3.41 (d, 2H, J=8.1 Hz), 3.3-3.4 (m, 3H), 3.23 (dd, 2H, J=3.9, 8.1 Hz), 2.96 (br t, 2H, J=12.5 Hz), 2.80 (dt, 1H, J=3.4, 12.2 Hz), 2.5-2.7 (m, 3H), 1.71 (quin, 1H, J=6.8 Hz), 0.81 (d, 6H, J=6.8 Hz), m/z: 410.2 [M+H]+ | | | |
| 3-fluoro-2'-[3-hydroxy-3-(propan-2-yl)azetidin-1-yl]-5'-[(morpholin-2-yl)methyl]-[1,1'-biphenyl]-4-carbonitrile | | | | |

| Example 121 | Procedure: K1+G5+B2+D2 | SM1: BB-2 | SM2: 4-bromo-2-methylpyridine | Yield: 60% |
|---|---|---|---|---|
| 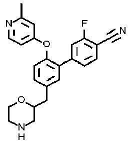 | 1H NMR (600 MHz, DMSO-d6, 300K) δ ppm 9.21 - 9.55 (m, 2 H), 8.53 (d, J=6.7 Hz, 1 H), 7.95 (dd, J=7.9, 7.2 Hz, 1 H), 7.65 (dd, J=10.6, 1.5 Hz, 1 H), 7.57 (d, J=2.1 Hz, 1 H), 7.47 - 7.54 (m, 2 H), 7.36 (d, J=8.4 Hz, 1 H), 7.23 (br s, 1 H), 7.15 (br d, J=4.0 Hz, 1 H), 3.98 - 4.05 (m, 1 H), 3.96 (dd, J=12.5, 3.3 Hz, 1 H), 3.73 (td, J=12.4, 2.3 Hz, 1 H), 3.11 - 3.26 (m, 2 H), 2.87 - 3.06 (m, 3 H), 2.81 (br d, J=10.7 Hz, 1 H), 2.56 (s, 3 H). m/z: 404 [M+H]+ | | | |
| 3-fluoro-2'-[(2-methylpyridin-4-yl)oxy]-5'-[(morpholin-2-yl)methyl]-[1,1'-biphenyl]-4-carbonitrile | | | | |

| Example 72 | Procedure: K1+G2+B2+D1 | SM1: BB-2 | SM2: [(3S)-oxan-3-yl]methanol | Yield: 90% |
|---|---|---|---|---|
| 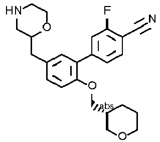 | 1H NMR (DMSO-d6, 600 MHz) δ 7.98 (dd, 1H, J=7.3, 7.9 Hz), 7.64 (dd, 1H, J=1.4, 11.1 Hz), 7.54 (dd, 1H, J=1.4, 8.1 Hz), 7.2-7.3 (m, 2H), 7.08 (d, 1H, J=8.4 Hz), 3.8-4.0 (m, 3H), 3.81 (dd, 1H, J=2.9, 12.0 Hz), 3.76 (dd, 1H, J=3.0, 11.1 Hz), 3.72 (td, 1H, J=3.5, 11.1 Hz), 3.6-3.7 (m, 1H), 3.50 (dt, 2H, J=2.1, 11.9 Hz), 3.0-3.4 (m, 10H), 2.9-3.0 (m, 2H), 2.78 (dt, 1H, J=3.5, 12.2 Hz), 2.6-2.7 (m, 2H), 2.5-2.6 (m, 1H), 1.9-2.0 (m, 1H), 1.7-1.8 (m, 1H), 1.4-1.6 (m, 2H), 1.3-1.4 (m, 1H). m/z: 411 [M+H]+ | | | |
| 3-fluoro-5'-[(morpholin-2-yl)methyl]-2'-{[(3R)-oxan-3-yl]methoxy}-[1,1'-biphenyl]-4-carbonitrile | | | | |

| Example 66 | Procedure: A1+B2+D1 | SM1: BB-2 | SM2: 3-(propan-2- yl)pyrrolidin-3-ol | Yield: 62% |
|---|---|---|---|---|
| 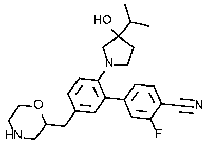 | 1H NMR (DMSO-d6, 500 MHz): δ (ppm) 7.93 (t, J = 7.6 Hz, 1H), 7.51 (ddd, J = 10.7, 5.0, 1.0 Hz, 1H), 7.38-7.44 (m, 1H), 7.12 (dd, J = 8.4, 1.8 Hz, 1H), 7.02 (s, 1H), 6.87 (dd, J = 8.4, 1.1 Hz, 1H), 6.54-9.92 (m, 3H), 3.99-4.50 (m, 1H), 3.92 (s, 2H), 3.81-3.88 (m, 1H), 3.63-3.73 (m, 1H), 3.50-3.59 (m, 1H), 3.20-3.28 (m, 1H), 3.08-3.49 (m, 2H), 2.94-3.05 (m, 2H), 2.79-2.92 (m, 3H), 2.58-2.72 (m, 3H), 2.56 (br d, J = 10.3 Hz, 1H), 1.52-1.76 (m, 3H), 0.82 (d, J = 6.6 Hz, 3H), 0.73 (d, J = 6.8 Hz, 3H). m/z: 424 [M+H]+ | | | |
| 3-fluoro-2'-[3-hydroxy-3-(propan-2-yl)pyrrolidin-1-yl]-5'-[(morpholin-2-yl)methyl]-[1,1'-biphenyl]-4-carbonitrile | | | | |

| Example 120 | Procedure: A4+G4+B2+D1 | SM1: BB-2 | SM2: 1-[(3R)-oxan-3-yl]methanamine hydrochloride | Yield: 83% |
|---|---|---|---|---|
| 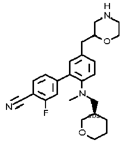 | 1H NMR (DMSO-d6, 600 MHz) δ 7.97 (t, 1H, J=7.6 Hz), 7.61 (dd, 1H, J=1.2, 10.9 Hz), 7.50 (dd, 1H, J=1.4, 8.0 Hz), 7.22 (dd, 1H, J=2.1, 8.2 Hz), 7.15 (d, 1H, J=8.2 Hz), 7.10 (d, 1H, J=2.1 Hz), 3.84 (s, 1H), 3.78 (dd, 1H, J=2.6, 11.7 Hz), 3.0-3.7 (m, 9H), 2.91 (br d, 1H, J=12.3 Hz), 2.8-2.9 (m, 2H), 2.7-2.8 (m, 1H), 2.6-2.7 (m, 2H), 2.5-2.6 (m, 3H), 2.44 (s, 3H), 1.7-1.8 (m, 1H), 1.5-1.6 (m, 1H), 1.3-1.5 (m, 2H), 0.9-1.1 (m, 1H). m/z: 424.3 [M+H]+ | | | |
| 3-fluoro-2'-[methyl({[(3R)-oxan-3-yl]methyl})amino]-5'-[(morpholin-2-yl)methyl]-[1,1'-biphenyl]-4-carbonitrile | | | | |

| Example 165 | Procedure: A1+B2+D1 | SM1: BB-2 | SM2: 6-methyl-2-azaspiro[3.3]heptan-6-ol hydrochloride | Yield: 69% |
|---|---|---|---|---|
| 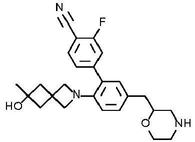 | 1H NMR (DMSO-d6, 500 MHz): δ (ppm) 7.94 (t, J = 7.6 Hz, 1H), 7.49 (dd, J = 10.5, 1.2 Hz, 1H), 7.39 (dd, J = 8.1, 1.5 Hz, 1H), 7.12 (dd, J = 8.3, 2.0 Hz, 1H), 6.97 (d, J = 2.0 Hz, 1H), 6.60-8.92 (m, 2H), 6.56 (d, J = 8.3 Hz, 1H), 4.30-5.17 (m, 1H), 3.88 (s, 2H), 3.81 (dd, J = 11.9, 2.6 Hz, 1H), 3.60-3.67 (m, 1H), 3.46-3.55 (m, 3H), 3.38 (s, 2H), 3.01-3.34 (m, 3H), 2.93 (br t, J = 13.7 Hz, 2H), 2.78 (td, J = 12.2, 3.5 Hz, 1H), 2.63 (dd, J = 11.6, 6.5 Hz, 2H), 2.56 (dd, J = 12.3, 10.9 Hz, 1H), 2.06 (s, 4H), 1.09 (s, 3H). m/z: 422 [M+H]+ | | | |
| 3-fluoro-2'-{6-hydroxy-6-methyl-2-azaspiro[3.3]heptan-2-yl}-5'-[(morpholin-2-yl)methyl]-[1,1'-biphenyl]-4-carbonitrile | | | | |

| Example 119 | Procedure: A6+E1+B1+D1 | SM1: BB-2J | SM2: 2-oxa-7-azaspiro[4.4]nonane | Yield: 56% |
|---|---|---|---|---|
| 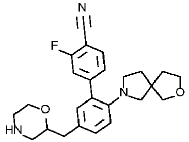 | 1H NMR (600 MHz, DMSO-d6) δ 7.97 - 7.89 (m, 1H), 7.59 - 7.53 (m, 1H), 7.47 - 7.42 (m, 1H), 7.14 (dd, J = 8.4, 2.1 Hz, 1H), 7.04 (d, J = 2.2 Hz, 1H), 6.95 - 6.89 (m, 1H), 3.82 - 3.77 (m, 1H), 3.75 - 3.67 (m, 2H), 3.65 - 3.60 (m, 1H), 3.51 - 3.46 (m, 3H), 2.87 (br t, J = 2.7 Hz, 6H), 2.79 - 2.72 (m, 1H), 2.70 - 2.59 (m, 2H), 2.57 - 2.51 (m, 1H), 1.85 - 1.69 (m, 4H). m/z: 422 [M+H]+ | | | |
| 3-fluoro-5'-[(morpholin-2-yl)methyl]-2'-{2-oxa-7-azaspiro[4.4]nonan-7-yl}-[1,1'-biphenyl]-4-carbonitrile | | | | |

| Example 60 | Procedure: A4+E1+B1+D2 | SM1: BB-2J | SM2: 4-cyclopropylpiperidin-4ol | Yield: 92% |
|---|---|---|---|---|
| 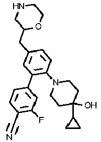 | 1H NMR (DMSO-d6, 500 MHz): δ (ppm) 7.97 (t, J = 7.6 Hz, 1H), 7.81 (dd, J = 11.2, 1.2 Hz, 1H), 7.71 (dd, J = 8.1, 1.5 Hz, 1H), 7.17-7.18 (m, 1H), 7.14 (d, J = 2.0 Hz, 1H), 7.08 (d, J = 8.3 Hz, 1H), 6.76-9.15 (m, 2H), 3.91 (s, 2H), 3.83 (brdd, J = 12.0, 2.7 Hz, 1H), 3.65-3.73 (m, 1H), 3.52 (td, J = 11.7, 2.0 Hz, 2H), 3.23-3.41 (m, 3H), 2.91-3.03 (m, 2H), 2.77-2.87 (m, 3H), 2.67-2.74 (m, 4H), 2.57-2.64 (m, 1H), 1.31-1.48 (m, 4H), 0.79 (tt, J = 8.4, 5.3 Hz, 1H), 0.26-0.32 (m, 2H), 0.15-0.22 (m, 2H). m/z: 436 [M+H]+ | | | |
| 2'-(4-cyclopropyl-4-hydroxypiperidin-1-yl)-3-fluoro-5'-[(morpholin-2-yl)methyl]-[1,1'-biphenyl]-4-carbonitrile | | | | |

| Example 70 | Procedure: A3+B2+D1 | SM1: BB-2 | SM2: 2-oxa-8-azaspiro[4.5]decane | Yield: 74% |
|---|---|---|---|---|
| 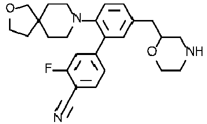 | 1H NMR (DMSO-d6, 600 MHz) +0.5 eq of tartrate: δ (ppm) 7.94-7.98 (m, 1H), 7.77-7.82 (m, 1H), 7.70 (dd, J = 8.1, 1.5 Hz, 1H), 7.21 (dd, J = 8.2, 2.1 Hz, 1H), 7.14 (d, J = 2.1 Hz, 1H), 7.08 (d, J = 8.2 Hz, 1H), 3.86 (s, 1H), 3.79 (dd, J = 11.9, 2.8 Hz, 1H), 3.63-3.71 (m, 3H), 3.49 (td, J = 11.8, 2.3 Hz, 1H), 3.41-3.42 (m, 2H), 2.85-2.96 (m, 2H), 2.51-2.79 (m, 8H), 1.66 (t, J = 7.1 Hz, 2H), 1.38-1.47 (m, 4H). m/z: 436.3 [M+H]+ | | | |
| 3-fluoro-5'-[(morpholin-2-yl)methyl]-2'-{2-oxa-8-azaspiro[4.5]decan-8-yl}-[1,1'-biphenyl]-4-carbonitrile | | | | |

| Example 118 | Procedure: A4+B2+D1 | SM1: BB-2 | SM2: 2-oxa-7-azaspiro[4.5]decane hydrochloride azaspiro[4.5]decane | Yield: 81% |
|---|---|---|---|---|
| 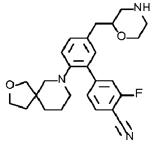 | 1H NMR (DMSO-d6, 600 MHz): δ (ppm) 7.99 (t, J = 7.6 Hz, 1H), 7.68 (d, J = 10.4 Hz, 1H), 7.56 (dd, J = 8.0, 1.2 Hz, 1H), 7.23 (dd, J = 8.4, 1.6 Hz, 1H), 7.08-7.15 (m, 2H), 3.89 (s, 1H), 3.72-3.85 (m, 1H), 3.65 (td, J = 8.2, 6.0 Hz, 2H), 3.46-3.57 (m, 3H), 3.39 (dd, J = 8.5, 5.4 Hz, 1H), 3.33 (br dd, J = 8.5, 1.5 Hz, 1H), 2.97 (br d, J = 12.2 Hz, 1H), 2.92 (br d, J = 12.2 Hz, 1H), 2.74-2.85 (m, 1H), 2.63-2.72 (m, 4H), 2.54-2.62 (m, 3H), 1.66 (dt, J = 12.1, 6.0 Hz, 1H), 1.52-1.59 (m, 1H), 1.43 (br d, J = 5.7 Hz, 1H), 1.24-1.40 (m, 3H). m/z: 436 [M+H]+ | | | |
| 3-fluoro-5'-[(morpholin-2-yl)methyl]-2'-{2-oxa-7-azaspiro[4.5]decan-7-yl}-[1,1'-biphenyl]-4-carbonitrile | | | | |

| Example 63 | Procedure: A4+E1+B1+D1 | SM1: BB-2J | SM2: 4-(ethoxymethyl)piperidin-4-ol n-4-ol | Yield: 87% |
|---|---|---|---|---|
| 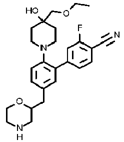 | 1H NMR (500 MHz, DMSO-d6) δ 7.95 (t, J = 7.6 Hz, 1H), 7.86 - 7.77 (m, 1H), 7.67 (dd, J = 8.2, 1.3 Hz, 1H), 7.24 - 7.18 (m, 1H), 7.16 - 7.12 (m, 1H), 7.11 - 7.06 (m, 1H), 4.45 - 4.04 (m, 1H), 3.88 - 3.80 (m, 1H), 3.75 - 3.67 (m, 1H), 3.58 - 3.50 (m, 1H), 3.44 (d, J = 7.1 Hz, 3H), 3.36 - 3.27 (m, 1H), 3.15 (s, 2H), 3.05 - 2.94 (m, 2H), 2.83 (br s, 3H), 2.76 - 2.53 (m, 5H), 1.58 - 1.43 (m, 2H), 1.36 - 1.28 (m, 2H), 1.11 (t, J = 7.0 Hz, 3H). m/z: 454.3 [M+H]+ | | | |
| 2'-[4-(ethoxymethyl)-4-hydroxypiperidin-1-yl]-3-fluoro-5'-[(morpholin-2-yl)methyl]-[1,1'-biphenyl]-4-carbonitrile | | | | |

| Example 152 | Procedure: A4+E1+B1+D1 | SM1: BB-2J | SM2: 4-(methoxymethyl)azep an-4-ol | Yield: 46% |
|---|---|---|---|---|
| 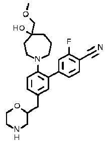 | 1H NMR (DMSO-d6, 500 MHz) δ 7.94 (t, 1H, J=7.6 Hz), 7.64 (d, 1H, J=10.8 Hz), 7.52 (d, 1H, J=8.1 Hz), 7.1-7.2 (m, 1H), 7.0-7.1 (m, 2H), 5.75 (s, 1H), 4.0-4.3 (m, 1H), 3.84 (s, 1H), 3.7-3.8 (m, 1H), 3.3-3.7 (m, 5H), 3.2-3.3 (m, 3H), 3.1-3.2 (m, 1H), 3.04 (s, 2H), 2.6-3.0 (m, 9H), 2.54 (d, 1H, J= 10.8 Hz), 1.7-1.8 (m, 1H), 1.4-1.6 (m, 3H), 1.3-1.4 (m, 2H). m/z: 454 [M+H]+ | | | |
| 3-fluoro-2'-[4-hydroxy-4-(methoxymethyl)azepan-1-yl]-5'-[(morpholin-2-yl)methyl]-[1,1'-biphenyl]-4-carbonitrile | | | | |

| Example 151 | Procedure: A6+E1+B1+D1 | SM1: BB-2J | SM2: 4-(trifluoromethyl)piperi din-4-ol | Yield: 50% |
|---|---|---|---|---|
| 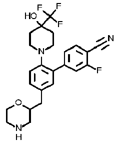 | 1H NMR (DMSO-d6, 600 MHz): δ (ppm) 7.97 (t, J = 7.4 Hz, 1H), 7.81 (dd, J = 11.1, 1.2 Hz, 1H), 7.68 (dd, J = 8.1, 1.5 Hz, 1H), 7.23 (dd, J = 8.2, 2.1 Hz, 1H), 7.16 (d, J = 2.1 Hz, 1H), 7.11 (d, J = 8.4 Hz, 1H), 5.85 (br s, 1H), 3.88 (s, 1H), 3.81 (dd, J = 12.0, 2.9 Hz, 1H), 3.64-3.70 (m, 1H), 3.49-3.52 (m, 1H), 2.96 (br d, J = 12.2 Hz, 1H), 2.91 (br d, J = 12.2 Hz, 1H), 2.76-2.85 (m, 5H), 2.64-2.72 (m, 2H), 2.55-2.62 (m, 1H), 1.56-1.60 (m, 4H). m/z: 464.3 [M+H]+ | | | |
| 3-fluoro-2'-[4-hydroxy-4-(trifluoromethyl)piperidin-1-yl]-5'-[(morpholin-2-yl)methyl]-[1,1'-biphenyl]-4-carbonitrile | | | | |

| Example 117 | Procedure: A1+B2+D1 | SM1: BB-2 | SM2: 1-{4H,5H,6H,7H-pyrazolo[1,5-a]pyridin-3-yl}methanamine | Yield: 90% |
|---|---|---|---|---|
| 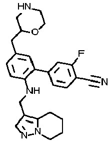 | 1H NMR (500 MHz, DMSO-d6) δ 7.98 (t, J = 7.6 Hz, 1H), 7.56 - 7.49 (m, 1H), 7.43 (dd, J = 7.9, 1.3 Hz, 1H), 7.29 (s, 1H), 7.06 (dd, J = 8.3, 2.0 Hz, 1H), 6.90 (d, J = 2.0 Hz, 1H), 6.69 (d, J = 8.3 Hz, 1H), 5.18 - 4.97 (m, 1H), 4.02 - 3.96 (m, 4H), 3.85 (br dd, J = 12.0, 2.7 Hz, 1H), 3.70 - 3.65 (m, 1H), 3.60 - 3.53 (m, 1H), 3.01 (br d, J = 13.4 Hz, 2H), 2.84 (br d, J = 2.9 Hz, 1H), 2.71 - 2.55 (m, 5H), 1.98 - 1.86 (m, 2H), 1.84 - 1.63 (m, 2H). m/z: 446.3 [M+H]+ | | | |
| 3-fluoro-5'-[(morpholin-2-yl)methyl]-2'-[({4H,5H,6H,7H-pyrazolo[1,5-a]pyridin-3-yl}methyl)amino]-[1,1'-biphenyl]-4-carbonitrile | | | | |

| Example 88 | Procedure: A1+B2+D1 | SM1: BB-2 | SM2: 1-(1 ,3-thiazol-2-yl)piperazine | Yield: 50% |
|---|---|---|---|---|
| 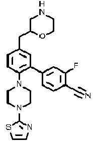 | 1H NMR (DMSO-d6, 600 MHz) δ 7.9-8.0 (m, 1H), 7.80 (dd, 1H, J=1.4, 11.1 Hz), 7.74 (dd, 1H, J=1.5, 8.1 Hz), 7.25 (dd, 1H, J=2.0, 8.3 Hz), 7.18 (d, 1H, J=2.1 Hz), 7.16 (d, 1H, J=3.5 Hz), 7.11 (d, 1H, J=8.2 Hz), 6.86 (d, 1H, J=3.5 Hz), 3.78 (br d, 1H, J=1.6 Hz), 3.74 (td, 1H, J=1.7, 10.2 Hz), 3.5-3.6 (m, 2H), 3.1-3.5 (m, 9H), 2.6-2.9 (m, 10H), 2.47 (brd, 1H, J=0.6 Hz). m/z: 464 [M+H]+ | | | |
| 3-fluoro-5'-[(morpholin-2-yl)methyl]-2'-[4-(1,3-thiazol-2-yl)piperazin-1-yl]-[1,1'-biphenyl]-4-carbonitrile | | | | |

| Example 59 | Procedure: A3+E1+B1+D1 | SM1 : BB-2J | SM2: 2-(piperidin-4-SM2: 2-(piperidin-4-yl)pyrimidine hydrochloride | Yield: 77% |
|---|---|---|---|---|
| 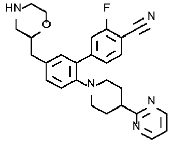 | 1H NMR (DMSO-d6, 400 MHz) δ 8.76 (dd, J = 4.9, 3.0 Hz, 2H), 8.02 - 7.96 (m, 1H), 7.83 (dd, J = 11.2, 1.4 Hz, 1H), 7.72 (dd, J = 8.1, 1.5 Hz, 1H), 7.37 - 7.33 (m, 1H), 7.24 (dd, J = 8.1, 2.1 Hz, 1H), 7.18 - 7.11 (m, 2H), 3.84 (s, 1H), 3.77 (t, J = 14.3 Hz, 2H), 3.63 (s, 2H), 3.46 (d, J = 11.1 Hz, 1H), 3.06 (d, J = 11.5 Hz, 2H), 2.96 - 2.53 (m, 11H).. m/z: 458 [M+H]+ | | | |
| 3-fluoro-5'-[(morpholin-2-yl)methyl]-2'-[4-(pyrimidin-2-yl)piperidin-1 -yl]-[1,1'-biphenyl]-4-carbonitrile | | | | |

| Example 97 | Procedure: A3+B2+D1 | SM1: BB-2 | SM2: 2-cyclopropyl-4H,5H,6H,7H-pyrazolo[1,5-a]pyrazine hydrochloride | Yield: 73% |
|---|---|---|---|---|
| 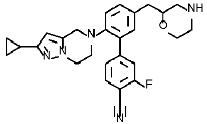 | 1H NMR (DMSO-d6, 500 MHz): δ (ppm) 7.95 (t, J = 7.6 Hz, 1H), 7.73 (dd, J = 10.9, 1.1 Hz, 1H), 7.65 (dd, J = 8.1, 1.5 Hz, 1H), 7.26 (dd, J = 8.3, 2.0 Hz, 1H), 7.18 (d, J = 2.0 Hz, 1H), 7.16 (d, J = 8.3 Hz, 1H), 6.13-8.41 (m, 2H), 5.72 (s, 1H), 4.05 (s, 2H), 3.84 (s, 2H), 3.77 (br dd, J = 11.5, 2.2 Hz, 1H), 3.73 (br t, J = 5.4 Hz, 2H), 3.59-3.67 (m, 1H), 3.46 (td, J = 11.6, 1.8 Hz, 1H), 3.28-3.40 (m, 3H), 3.14 (br t, J = 5.3 Hz, 2H), 2.91 (br d, J = 12.2 Hz, 1H), 2.81-2.86 (m, 1H), 2.73 (td, J = 12.1, 3.7 Hz, 1H), 2.65-2.70 (m, 2H), 2.51-2.56 (m, 1H), 1.74-1.86 (m, 1H), 0.77-0.82 (m, 2H), 0.52-0.62 (m, 2H). m/z: 458 [M+H]+ | | | |
| 2'-{2-cyclopropyl-4H,5H,6H,7H-pyrazolo[1,5-a]pyrazin-5-yl}-3-fluoro-5'-[(morpholin-2-yl)methyl]-[1,1'-biphenyl]-4-carbonitrile | | | | |

| Example 30 | Procedure: C2+B2+Chiral Separation+D1 | SM1: BB-1 | SM2: 1,3-thiazole | Yield: 58% |
|---|---|---|---|---|
| 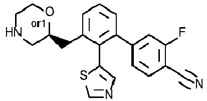 | 1H NMR (600 MHz, DMSO-d6) δ 9.07 (d, J = 0.7 Hz, 1H), 7.83 - 7.77 (m, 1H), 7.72 (d, J = 0.7 Hz, 1H), 7.56 - 7.47 (m, 2H), 7.38 - 7.31 (m, 2H), 7.15 (dd, J = 8.1, 1.5 Hz, 1H), 3.79 - 3.72 (m, 1H), 3.58 - 3.52 (m, 1H), 3.44 - 3.39 (m, 1H), 3.20 - 3.00 (m, 1H), 2.87 - 2.80 (m, 1H), 2.79 - 2.69 (m, 2H), 2.64 (d, J = 6.5 Hz, 2H), 2.45 (s, 1H). m/z: 380.1 [M+H]+ | | | |
| 3-fluoro-3'-{[(2S)-morpholin-2-yl]methyl)-2'-(1,3-thiazol-5-yl)-[1,1'-biphenyl]-4-carbonitrile | | | | |

| Example 23 | Procedure: C2+B2+Chiral Separation+D1 | SM1: BB-1 | SM2: 1,3-thiazole | Yield: 58% |
|---|---|---|---|---|
| 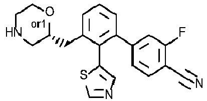 | 1H NMR (600 MHz, DMSO-d6) δ 9.07 (d, J = 0.7 Hz, 1H), 7.79 (dd, J = 7.8, 7.2 Hz, 1H), 7.72 (d, J = 0.6 Hz, 1H), 7.59 - 7.44 (m, 1H), 7.34 (dd, J = 2.9, 1.5 Hz, 1H), 7.34 - 7.32 (m, 1H), 7.19 - 7.13 (m, 1H), 3.77 - 3.69 (m, 1H), 3.55 - 3.50 (m, 1H), 3.41 - 3.39 (m, 1H), 3.17 - 3.08 (m, 1H), 2.82 - 2.76 (m, 1H), 2.75 - 2.68 (m, 2H), 2.63 (d, J = 6.9 Hz, 2H), 2.42 (s, 1H). m/z: 380.1 [M+H]+ | | | |
| 3-fluoro-3'-{[(2R)-morpholin-2-yl]methyl}-2'-(1,3-thiazol-5-yl)-[1,1'-biphenyl]-4-carbonitrile | | | | |

| Example 74 | Procedure: A4+E1+B1+D1 | SM1: BB-2K | SM2: 4-(methoxymethyl)piperi dine | Yield: 77% |
|---|---|---|---|---|
| 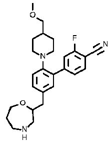 | 1H NMR (600 MHz, DMSO-d6) δ 9.20 - 8.06 (m, 1H), 8.00 - 7.93 (m, 1H), 7.78 (dd, J = 11.2, 1.3 Hz, 1H), 7.68 (dd, J = 8.1, 1.5 Hz, 1H), 7.61 - 7.34 (m, 1H), 7.22 (dd, J = 8.4, 2.1 Hz, 1H), 7.16 (d, J = 2.1 Hz, 1H), 7.06 (d, J = 8.2 Hz, 1H), 3.87 - 3.80 (m, 2H), 3.53 (ddd, J = 12.3, 7.4, 4.8 Hz, 1H), 3.24 - 3.21 (m, 3H), 3.19 - 3.08 (m, 4H), 2.99 - 2.90 (m, 3H), 2.77 - 2.61 (m, 3H), 2.54 - 2.50 (m, 2H), 1.90 - 1.75 (m, 2H), 1.55 (br s, 3H), 1.16 - 0.93 (m, 2H). m/z: 438 [M+H]+ | | | |
| 3-fluoro-2'-[4-(methoxymethyl)piperidin-1-yl]-5'-[(1,4-oxazepan-2-yl)methyl]-[1,1'-biphenyl]-4-carbonitrile | | | | |

| Example 68 | Procedure: B1+G1+B2+D1 | SM1: BB-2 | SM2: [3-fluoro-4-(4,4,5,5-tetramethyl-1,3,2-dioxaborolan-2-yl)phenyl]methanol | Yield: 51% |
|---|---|---|---|---|
| 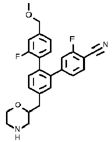 | 1H NMR (600 MHz, DMSO-d6) δ 7.83 - 7.76 (m, 1H), 7.44 - 7.40 (m, 1H), 7.38 (s, 2H), 7.29 - 7.25 (m, 2H), 7.17 - 7.13 (m, 1H), 7.08 (dd, J = 8.1, 1.6 Hz, 1H), 7.04 - 6.99 (m, 1H), 4.40 (s, 2H), 3.84 - 3.80 (m, 1H), 3.79 - 3.71 (m, 1H), 3.54 - 3.48 (m, 1H), 3.29 (s, 3H), 3.21 - 3.07 (m, 1H), 3.03 - 2.99 (m, 1H), 2.92 - 2.87 (m, 1H), 2.84 - 2.80 (m, 2H), 2.80 - 2.76 (m, 1H), 2.65 - 2.59 (m, 1H). m/z: 435 [M+H]+ | | | |
| 3-fluoro-2'-[2-fluoro-4-(methoxymethyl)phenyl]-5'-[(morpholin-2-yl)methyl]-[1,1'-biphenyl]-4-carbonitrile | | | | |

| Example 35 | Procedure: B4+B2+D1 | SM1: BB-1 | SM2: 4-boranyl-3-methyl-1,2-oxazole | Yield: 78% |
|---|---|---|---|---|
| 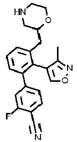 | 1H NMR (600 MHz, DMSO-d6) δ ppm 8.79 (d, J=4.69 Hz, 1 H) 8.06 - 8.65 (m, 2 H) 7.84 (t, J=7.48 Hz, 1 H) 7.46 - 7.56 (m, 2 H) 7.33 - 7.40 (m, 2 H) 7.14 - 7.21 (m, 1 H) 3.76 - 3.84 (m, 1 H) 3.57 - 3.63 (m, 1 H) 3.46 - 3.50 (m, 1 H) 2.92 - 3.00 (m, 2 H) 2.71 - 2.85 (m, 2 H) 2.54 - 2.70 (m, 2 H) 1.68 - 1.77 (m, 3 H). m/z: 378 [M+H]+ | | | |
| 3-fluoro-2'-(3-methyl-1,2-oxazol-4-yl)-3'-[(morpholin-2-yl)methyl]-[1,1'-biphenyl]-4-carbonitrile | | | | |

| Example 73 | Procedure: A3+B2+D1 | SM1: BB-2 | SM2: 4-(oxolan-2-yl)piperidine | Yield: 85% |
|---|---|---|---|---|
| 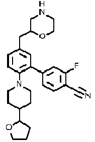 | 1H NMR (DMSO-d6, 600 MHz) δ 7.9-8.0 (m, 1H), 7.79 (dd, 1H, J=1.2, 11.2 Hz), 7.68 (dd, 1H, J=1.5, 8.1 Hz), 7.21 (dd, 1H, J=2.1, 8.2 Hz), 7.14 (d, 1H, J=2.1 Hz), 7.05 (d, 1H, J=8.2 Hz), 3.89 (s, 1H), 3.81 (dd, 1H, J=2.8, 11.9 Hz), 3.6-3.7 (m, 2H), 3.5-3.6 (m, 1H), 3.50 (dt, 1H, J=2.1 , 11.8 Hz), 3.4-3.5 (m, 1H), 3.1-3.4 (m, 3H), 2.9-3.0 (m, 4H), 2.79 (dt, 1H, J=3.5, 12.2 Hz), 2.6-2.7 (m, 2H), 2.5-2.6 (m, 1H), 2.4-2.5 (m, 4H), 1.6-1.9 (m, 4H), 1.4-1.5 (m, 2H), 1.2-1.3 (m, 2H), 1.0-1.2 (m, 2H). m/z: 450 [M+H]+ | | | |
| 3-fluoro-5'-[(morpholin-2-yl)methyl]-2'-[4-(oxolan-2-yl)piperidin-1-yl]-[1,1'-biphenyl]-4-carbonitrile | | | | |

| Example 100 | Procedure: A3+B2+D1 | SM1: BB-2 | SM2: 4-(oxolan-3-hydrochloride | yl)piperidinehidrochloride Yield: 62% |
|---|---|---|---|---|
| 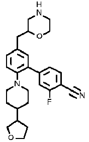 | 1H NMR (DMSO-d6, 500 MHz +-0.5 eq of tartrate): δ (ppm) 7.97 (t, J = 7.6 Hz, 1H), 7.75-7.85 (m, 1H), 7.66-7.72 (m, 1H), 7.18-7.23 (m, 1H), 7.14 (d, J = 2.0 Hz, 1H), 7.05 (d, J = 8.1 Hz, 1H), 3.88 (s, 1H), 3.45-3.85 (m, 7H), 3.25 (t, J = 8.1 Hz, 1H), 2.53-3.05 (m, 8H), 1.79-2.02 (m, 2H), 1.03-1.67 (m, 7H). m/z: 450 [M+H]+ | | | |
| 3-fluoro-5'-[(morpholin-2-yl)methyl]-2'-[4-(oxolan-3-yl)piperidin-1-yl]-[1,1'-biphenyl]-4-carbonitrile | | | | |

| Example 20 | Procedure: B9+B2+B2+D1 | SM1: BB-2 | SM2: 5-bromo-1 ,2-thiazole | Yield: 83% |
|---|---|---|---|---|
| 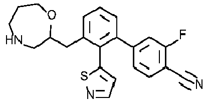 | 1H NMR (600 MHz, DMSO-d6) δ 8.52 (d, J = 1.6 Hz, 1H), 7.83 - 7.74 (m, 1H), 7.59 - 7.55 (m, 1H), 7.54 - 7.51 (m, 1H), 7.39 - 7.36 (m, 1H), 7.36 - 7.34 (m, 2H), 7.20 - 7.15 (m, 1H), 3.79 - 3.73 (m, 2H), 3.43 - 3.42 (m, 1H), 3.11 - 3.07 (m, 1H), 3.06 - 2.98 (m, 2H), 2.96 - 2.87 (m, 1H), 2.73 - 2.67 (m, 1H), 2.65 - 2.58 (m, 2H), 1.95 - 1.71 (m, 2H). m/z: 394.2 [M+H]+ | | | |
| 3-fluoro-3'-[(1,4-oxazepan-2-yl)methyl]-2'-(1,2-thiazol-5-yl)-[1,1'-biphenyl]-4-carbonitrile | | | | |

| Example 32 | Procedure: B2+B2+D2 | SM1: BB-1 | SM2: 3-(4,4,5,5-tetramethyl-1,3,2-dioxaborolan-2-yl)pyridine | Yield: 28% |
|---|---|---|---|---|
| 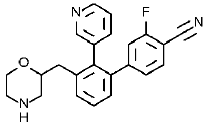 | 1H NMR (DMSO-d6, 500 MHz): δ (ppm) 8.45 (d, J = 4.2 Hz, 1H), 8.19-8.31 (m, 1H), 7.73 (t, J = 7.5 Hz, 1H), 7.52-7.64 (m, 1H), 7.49-7.52 (m, 2H), 7.30-7.38 (m, 2H), 7.21-7.28 (m, 1H), 7.02-7.07 (m, 1H), 3.88 (s, 1H), 3.72 (br d, J = 11.7 Hz, 1H), 3.64 (br s, 1H), 3.45-3.50 (m, 1H), 3.36-3.40 (m, 1H), 2.76-2.84 (m, 1H), 2.54-2.72 (m, 4H), 2.28-2.44 (m, 1H). m/z: 374 [M+H]+ | | | |
| 3-fluoro-3'-[(morpholin-2-yl)methyl]-2'-(pyridin-3-yl)-[1,1'-biphenyl]-4-carbonitrile | | | | |

| Example 150 | Procedure: L1+B2+D1 | SM1: BB-2 | SM2: 3-bromooxetane | Yield: 67% |
|---|---|---|---|---|
| 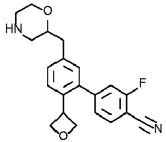 | 1H NMR (500 MHz, DMSO-d6) δ 7.98 (t, J = 7.5 Hz, 1H), 7.57 (d, J = 8.1 Hz, 1H), 7.51 - 7.45 (m, 1H), 7.41 - 7.35 (m, 1H), 7.31 (dd, J = 7.9, 1.1 Hz, 1H), 7.13 (s, 1H), 4.64 (dt, J = 8.1, 5.6 Hz, 2H), 4.49 (s, 2H), 4.46 - 4.38 (m, 1H), 3.86 - 3.80 (m, 1H), 3.76 - 3.68 (m, 1H), 3.54 (br d, J = 11.5 Hz, 1H), 3.20 - 3.09 (m, 1H), 3.07 - 2.98 (m, 1H), 2.97 - 2.90 (m, 1H), 2.86 - 2.78 (m, 1H), 2.77 - 2.72 (m, 2H), 2.63 (br s, 1H). m/z: 353 [M+H]+ | | | |
| 3-fluoro-5'-[(morpholin-2-yl)methyl]-2'-(oxetan-3-yl)-[1,1'-biphenyl]-4-carbonitrile | | | | |

| Example 57 | Procedure: E1+F4+E4+N1+D4+ I1+B1+B1+B2+D1 | SM1: methyl 2-amino-5-bromobenzoate | SM2: 4-[(4-methoxyphenyl)methyl]morpholin-3-one methoxyphenyl)methy | Yield: 37% |
|---|---|---|---|---|
| 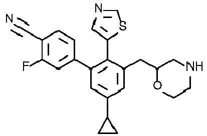 | 1H NMR (600 MHz, DMSO-d6) δ 9.05 (d, J = 0.7 Hz, 1H), 7.77 (br d, J = 0.7 Hz, 1H), 7.69 - 7.61 (m, 1H), 7.37 - 7.29 (m, 1H), 7.25 - 7.18 (m, 1H), 7.15 - 7.10 (m, 1H), 7.01 (d, J = 1.9 Hz, 1H), 3.87 - 3.80 (m, 1H), 3.64 - 3.59 (m, 1H), 3.50 (br dd, J = 6.0, 5.0 Hz, 1H), 3.47 - 3.45 (m, 1H), 3.04 - 2.98 (m, 1H), 2.94 - 2.89 (m, 1H), 2.87 - 2.80 (m, 1H), 2.67 - 2.62 (m, 2H), 2.60 - 2.55 (m, 1H), 2.03 - 1.97 (m, 1H), 1.06 - 0.99 (m, 2H), 0.82 - 0.78 (m, 2H). m/z: 420.2 [M+H]+ | | | |
| 5'-cyclopropyl-3-fluoro-3'-[(morpholin-2-yl)methyl]-2'-(1,3-thiazol-5-yl)-[1,1'-biphenyl]-4-carbonitrile | | | | |

| Example 149 | Procedure: A5+E1+B2+D2 | SM1: BB-2J | SM2: 1-tert-butylpiperazine | Yield: 96% |
|---|---|---|---|---|
| 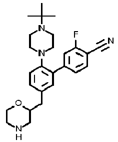 | 1H NMR (600 MHz, DMSO-d6) δ ppm 10.15 - 10.40 (m, 1 H), 9.04 - 9.41 (m, 2 H), 7.94 - 8.01 (m, 1 H), 7.82 (dd, J=10.9, 1.3 Hz, 1 H), 7.73 (dd, J=8.1, 1.5 Hz, 1 H), 7.29 (dd, J=8.2, 2.1 Hz, 1 H), 7.21 (d, J=2.1 Hz, 1 H), 7.13 (d, J=8.4 Hz, 1 H), 3.81 - 3.97 (m, 2 H), 3.63 - 3.71 (m, 1 H), 3.40 (br d, J=11.7 Hz, 2 H), 3.11 - 3.21 (m, 4 H), 3.03 (br d, J=12.6 Hz, 2 H), 2.88 - 2.98 (m, 1 H), 2.71 - 2.87 (m, 5 H), 1.34 (s, 9 H). m/z: 437 [M+H]+ | | | |
| 2'-(4-tert-butylpiperazin-1 -yl)-3-fluoro-5'-[(morpholin-2-yl)methyl]-[1,1'-biphenyl]-4-carbonitrile | | | | |

| Example 99 | Procedure: A1+B2+D1 | SM1: BB-2 | SM2: 6-methyl-2-azaspiro[3.3]heptan-6-ol hydrochloride | Yield: 91% |
|---|---|---|---|---|
| 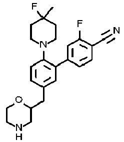 | 1H NMR (DMSO-d6, 600 MHz): δ (ppm) 7.93-7.98 (m, 1H), 7.74-7.81 (m, 1H), 7.69-7.73 (m, 1H), 7.19-7.25 (m, 1H), 7.13-7.16 (m, 1H), 7.06-7.12 (m, 1H), 3.83-3.89 (m, 1H), 3.73-3.82 (m, 1H), 3.59-3.69 (m, 1H), 3.46-3.51 (m, 1H), 2.85-2.98 (m, 2H), 2.72-2.82 (m, 5H), 2.63-2.72 (m, 2H), 2.53-2.59 (m, 1H), 1.46-1.75 (m, 4H), 1.27-1.35 (m, 3H). m/z: 412 [M+H]+ | | | |
| 3-fluoro-2'-(4-fluoro-4-methylpiperidin-1-yl)-5'-[(morpholin-2-yl)methyl]-[1,1'-biphenyl]-4-carbonitrile | | | | |

| Example 114 | Procedure: N2+A1+B2+D1 | SM1: tert-butyl (3aR,6aS)-5-oxo-octahydrocyclopenta[ c]pyrrole-2-carboxylate | SM2: BB-2 (step 2) | Yield: 56% |
|---|---|---|---|---|
| 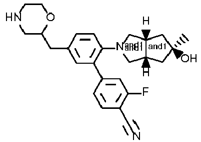 | 1H NMR (600 MHz, DMSO-d6) δ ppm 7.87 - 8.00 (m, 1 H) 7.69 - 7.85 (m, 2 H) 7.62 - 7.66 (m, 1 H) 7.57 - 7.62 (m, 1 H) 7.17 (dd, J=8.22, 2.05 Hz, 1 H) 7.07 (d, J=2.05 Hz, 1 H) 6.98 (d, J=8.36 Hz, 1 H) 4.30 - 4.72 (m, 1 H) 3.81 (br dd, J=11.96, 2.71 Hz, 1 H) 3.63 - 3.67 (m, 1 H) 3.51 (br dd, J=11.88, 2.05 Hz, 1 H) 2.88 - 2.99 (m, 2 H) 2.75 - 2.82 (m, 1 H) 2.62 - 2.73 (m, 6 H) 2.53 - 2.60 (m, 1 H) 2.40 - 2.47 (m, 2 H) 1.59 - 1.69 (m, 2 H) 1.30 - 1.39 (m, 2 H) 1.10 (s, 3 H). m/z: 436 [M+H]+ | | | |
| 2'-[(3aR,5S,6aS)-5-hydroxy-5-methyl-octahydrocyclopenta[c]pyrrol-2-yl]-3-fluoro-5'-[(morpholin-2-yl)methyl]-[1,1'-biphenyl]-4-carbonitrile | | | | |

| Example 28 | Procedure: B6+B2+D1 | SM1: BB-4-E1 | SM2: 5-bromo-1 ,2-thiazole | Yield: 79% |
|---|---|---|---|---|
| 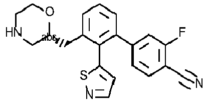 | 1H NMR (DMSO-d6, 500 MHz) δ 8.52 (d, 1H, J=1.7 Hz), 7.79 (dd, 1H, J=7.1, 7.8 Hz), 7.5-7.6 (m, 2H), 7.3-7.5 (m, 3H), 7.17 (dd, 1H, J=1.6, 7.9 Hz), 3.89 (s, 1H), 2.9-3.8 (m, 8H), 2.7-2.9 (m, 3H), 2.62 (d, 2H, J=6.4 Hz), 2.45 (dd, 1H, J=10.5, 12.2 Hz). m/z: 380 [M+H]+ | | | |
| 3-fluoro-3'-{[(2R)-morpholin-2-yl]methyl}-2'-(1,2-thiazol-5-yl)-[1,1'-biphenyl]-4-carbonitrile | | | | |

| Example 4 | Procedure: B6+B2+D1 | SM1: BB-4-E2 | SM2: 5-bromo-1,2-thiazole | Yield: 83% |
|---|---|---|---|---|
| 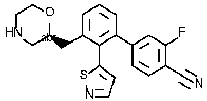 | 1H NMR (600 MHz, DMSO-d6) δ 8.55 - 8.49 (m, 1H), 8.38 - 7.90 (m, 2H), 7.79 (dd, J = 7.8, 7.2 Hz, 1H), 7.58 - 7.53 (m, 1H), 7.52 - 7.50 (m, 1H), 7.40 - 7.32 (m, 3H), 7.17 (dd, J = 8.0, 1.5 Hz, 1H), 3.84 - 3.74 (m, 1H), 3.61 - 3.55 (m, 1H), 3.47 - 3.44 (m, 1H), 2.91 - 2.85 (m, 1H), 2.85 - 2.79 (m, 1H), 2.79 - 2.73 (m, 1H), 2.66 - 2.59 (m, 2H). m/z: 380.1 [M+H]+ | | | |
| 3-fluoro-3'-{[(2S)-morpholin-2-yl]methyl}-2'-(1,2-thiazol-5-yl)-[1,1'-biphenyl]-4-carbonitrile | | | | |

| Example 1 | Procedure: B6+B2+D1 | SM1: BB-4 | SM2: 5-bromo-1 ,2-thiazole | Yield: 74% |
|---|---|---|---|---|
| 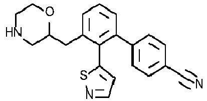 | 1H NMR (600 MHz, DMSO-d6) δ 8.49 (d, J = 1.8 Hz, 1H), 8.37 - 7.80 (m, 2H), 7.70 (d, J = 8.5 Hz, 2H), 7.57 - 7.52 (m, 1H), 7.51 - 7.47 (m, 1H), 7.37 - 7.29 (m, 4H), 3.84 - 3.76 (m, 1H), 3.62 - 3.57 (m, 2H), 3.47 - 3.44 (m, 1H), 2.77 (td, J = 12.2, 3.5 Hz, 3H), 2.67 - 2.61 (m, 2H). m/z: 362 [M+H]+ | | | |
| 3'-[(morph olin-2-yl)methyl]-2'-(1,2-thiazol-5-yl)-[1,1'-biphenyl]-4-carbonitrile | | | | |

| Example 12 | Procedure: B1+H2+N2+H1+R1. 1+R1.2+R1.3+I1+B2 +D2 | SM1: BB-1 | SM2: ethenylborane | Yield: 40% |
|---|---|---|---|---|
| 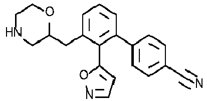 | 1H NMR (600 MHz, DMSO-d6) δ 9.16 (s, 1H), 8.55 (d,J= 1.8 Hz, 0H), 7.79 - 7.74 (m, 1H), 7.64 (t,J= 7.7 Hz, 0H), 7.52 (dd,J= 7.8, 1.1 Hz, 0H), 7.42 (dd,J= 7.7, 1.2 Hz, 0H), 7.33 - 7.28 (m, 1H), 6.43 (d,J= 1.8 Hz, 1H), 3.90 (dd,J= 12.6, 3.5 Hz, 1H), 3.84 - 3.72 (m, 2H), 3.61 (td,J= 12.5, 2.4 Hz, 1H), 3.12 (d,J= 12.4 Hz, 1H), 3.05 (d,J= 12.2 Hz, 0H), 2.97 - 2.88 (m, 1H), 2.70 (dd,J= 16.2, 5.3 Hz, 1H).. m/z: 346 [M+H]+ | | | |
| 3'-[(morph olin-2-yl)methyl]-2'-(1,2-oxazol-5-yl)-[1,1'-biphenyl]-4-carbonitrile | | | | |

| Example 10 | Procedure: B2+B2+D1 | SM1: BB-1 | SM2: (2,5-dihydrofuran-3-yl)borane | Yield: 93% |
|---|---|---|---|---|
| 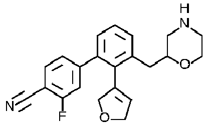 | 1H NMR (500 MHz, DMSO-d6) δ 7.97 - 7.91 (m, 1H), 7.82 - 7.59 (m, 1H), 7.52 (dd, J = 10.5, 1.5 Hz, 1H), 7.44 - 7.41 (m, 1H), 7.40 - 7.38 (m, 2H), 7.38 - 7.36 (m, 1H), 7.23 (dd, J = 6.0, 2.8 Hz, 1H), 5.93 (t, J = 1.8 Hz, 1H), 4.65 - 4.49 (m, 2H), 4.34 - 4.08 (m, 2H), 3.81 - 3.74 (m, 1H), 3.67 - 3.60 (m, 1H), 3.49 - 3.45 (m, 1H), 2.94 - 2.89 (m, 1H), 2.89 - 2.83 (m, 1H), 2.81 - 2.76 (m, 1H), 2.75 - 2.71 (m, 2H), 2.60 - 2.55 (m, 1H). m/z: 365 [M+H]+ | | | |
| 2'-(2,5-dihydrofu ran-3-yl)-3-fluoro-3'-[(morpholin-2-yl)methyl]-[1,1'-biphenyl]-4-carbonitrile | | | | |

| Example 17 | Procedure: Chiral Separation | SM1: Example 16 | | Yield: 13% |
|---|---|---|---|---|
| 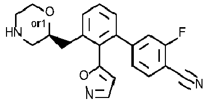 | 1H NMR (DMSO-d6, 600 MHz): δ (ppm) 9.05 (br s, 2H), 8.58 (d, J = 1.8 Hz, 1H), 7.85 (t, J = 7.4 Hz, 1H), 7.65 (t, J = 7.8 Hz, 1H), 7.55 (d, J = 6.7 Hz, 1H), 7.46 (dd, J = 7.7, 1.1 Hz, 1H), 7.32 (dd, J = 10.4, 1.5 Hz, 1H), 7.12 (dd, J = 8.1, 1.6 Hz, 1H), 6.50 (d, J = 1.9 Hz, 1H), 3.89 (dd, J = 12.5, 3.4 Hz, 1H), 3.78 (dtd, J = 10.9, 6.5, 2.1 Hz, 1H), 3.59 (td, J = 12.5, 2.3 Hz, 1H), 3.12 (br d, J = 12.8 Hz, 1H), 3.06 (br d, J = 12.5 Hz, 1H), 2.89-2.98 (m, 1H), 2.71-2.75 (m, 1H), 2.70 (d, J = 6.5 Hz, 2H). m/z: 364 [M+H]+ | | | |
| 3-fluoro-3'-{[(2S)- -morpholin-2-yl]methyl}-2'-(1,2-oxazol-5-yl)-[1,1'-biphenyl]-4-carbonitrile | | | | |

| Example 13 | Procedure: Chiral Separation | SM1: Example 16 | | Yield: 14% |
|---|---|---|---|---|
| 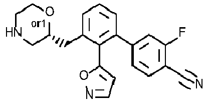 | 1H NMR (DMSO-d6, 600 MHz): δ (ppm) 8.70-9.04 (m, 2H), 8.59 (d, J = 1.9 Hz, 1H), 7.86 (dd, J = 7.9, 7.2 Hz, 1H), 7.62-7.68 (m, 1H), 7.55 (dd, J = 7.8, 1.0 Hz, 1H), 7.47 (dd, J = 7.6, 1.2 Hz, 1H), 7.32 (dd, J = 10.5, 1.5 Hz, 1H), 7.12 (dd, J = 8.1, 1.6 Hz, 1H), 6.50 (d, J = 1.8 Hz, 1H), 3.89 (dd, J = 12.8, 3.3 Hz, 1H), 3.75 (dtd, J = 10.9, 6.6, 2.1 Hz, 1H), 3.57 (td, J = 12.4, 2.3 Hz, 1H), 3.12 (br d, J = 12.6 Hz, 1H), 3.05 (br d, J = 12.5 Hz, 1H), 2.93 (td, J = 12.5, 3.7 Hz, 1H), 2.71-2.75 (m, 1H), 2.70 (d, J = 6.5 Hz, 2H). m/z: 364 [M+H]+ | | | |
| 3-fluoro-3'-{[(2R)- -morpholin-2-yl]methyl}-2'-(1,2-oxazol-5-yl)-[1,1'-biphenyl]-4-carbonitrile | | | | |

| Example 22 | Procedure: Chiral Separation | SM1: Example 10 | | Yield: 100% |
|---|---|---|---|---|
| 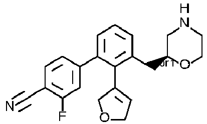 | 1H NMR (600 MHz, DMSO-d6) δ 7.98 - 7.88 (m, 1H), 7.52 (dd, J = 10.5, 1.4 Hz, 1H), 7.43 - 7.34 (m, 3H), 7.23 (dd, J = 6.4, 2.6 Hz, 1H), 7.08 - 6.10 (m, 1H), 5.93 (t, J = 1.8 Hz, 1H), 4.66 - 4.47 (m, 2H), 4.34 - 4.07 (m, 2H), 3.79 - 3.74 (m, 1H), 3.67 - 3.60 (m, 1H), 3.45 - 3.42 (m, 1H), 2.96 - 2.68 (m, 5H), 2.64 - 2.55 (m, 1H). m/z: 365 [M+H]+ | | | |
| 2'-(2,5-dihydrofura n-3-yl)-3-fluoro-3'-{[(2S)-morpholin-2-yl]methyl}-[1,1'-biphenyl]-4-carbonitrile | | | | |

| Example 8 | Procedure: Chiral Separation | SM1: Example 10 | | Yield: 22% |
|---|---|---|---|---|
| 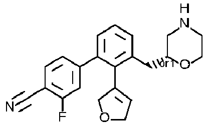 | 1H NMR (600 MHz, DMSO-d6) δ 7.93 (dd, J = 7.9, 7.2 Hz, 1H), 7.52 (dd, J = 10.6, 1.5 Hz, 1H), 7.44 - 7.34 (m, 3H), 7.22 (dd, J = 6.6, 2.3 Hz, 1H), 7.06 - 6.35 (m, 1H), 5.93 (t, J = 1.8 Hz, 1H), 4.68 - 4.47 (m, 2H), 4.33 - 4.10 (m, 2H), 3.79 - 3.73 (m, 1H), 3.65 - 3.60 (m, 1H), 3.46 - 3.42 (m, 1H), 2.93 - 2.86 (m, 1H), 2.86 - 2.79 (m, 1H), 2.78 - 2.74 (m, 1H), 2.74 - 2.68 (m, 2H), 2.58 - 2.52 (m, 1H). m/z: 365 [M+H]+ | | | |
| 2'-(2,5-dihydrofura n-3-yl)-3-fluoro-3'-{[(2R)-morpholin-2-yl]methyl}-[1,1'-biphenyl]-4-carbonitrile | | | | |

| Example 113 | Procedure: B2+B2+D1 | SM1: BB-4 | SM2: 4-bromo-5-methyl-1,2-thiazole | Yield: 69% |
|---|---|---|---|---|
| 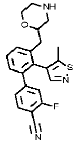 | 1H NMR (500 MHz, DMSO-d6) δ 8.45 - 8.24 (m, 1H), 7.80 - 7.74 (m, 1H), 7.56 -7.46 (m, 2H), 7.37 (dt, J = 6.7, 2.1 Hz, 1H), 7.24 (ddd, J = 10.6, 4.0, 1.5 Hz, 1H), 7.06 (ddd, J = 8.0, 4.7, 1.7 Hz, 1H), 3.76 - 3.71 (m, 1H), 3.44 (br d, J = 2.7 Hz, 2H), 2.87 - 2.77 (m, 1H), 2.75 - 2.66 (m, 2H), 2.63 - 2.53 (m, 1H), 2.48 - 2.31 (m, 2H), 2.05 - 1.93 (m, 3H). m/z: 394 [M+H]+ | | | |
| 3-fluoro-2'-(5-meth hyl-1,2-thiazol-4-yl)-3'-[(morpholin-2-yl)methyl]-[1,1'-biphenyl]-4-carbonitrile | | | | |

| Example 112 | Procedure: G2+B1+B1+D1 | SM1: 3-bromo-2-iodophenol | SM2: tert-butyl 6-hydroxy-2-azabicyclo[2.2.1]hept ane-2-carboxylate | Yield: 68% |
|---|---|---|---|---|
| 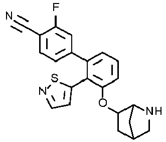 | 1H NMR (500 MHz, DMSO-d6) δ 8.37 (d, J = 1.7 Hz, 1H), 7.85 (dd, J = 7.8, 7.1 Hz, 1H), 7.58 (t, J = 7.9 Hz, 1H), 7.40 (dd, J = 10.3, 1.5 Hz, 1H), 7.34 - 7.27 (m, 1H), 7.26 - 7.21 (m, 1H), 7.16 (dd, J = 7.9, 1.6 Hz, 1H), 7.10 (d, J = 7.6 Hz, 1H), 7.07 - 7.01 (m, 1H), 6.91 (d, J = 1.7 Hz, 1H), 4.84 - 4.68 (m, 1H), 3.86 - 3.82 (m, 1H), 2.93 - 2.87 (m, 1H), 2.71 - 2.67 (m, 1H), 2.60 (br s, 1H), 2.12 - 2.02 (m, 1H), 1.64 - 1.45 (m, 3H). m/z: 392 [M+H]+ | | | |
| 3'-{2-azabicyclo[2.2. .1]heptan-6-yloxy}-3-fluoro-2'-(1,2-thiazol-5-yl)-[1,1'-biphenyl]-4-carbonitrile | | | | |

| Example 64 | Procedure: B1+B2+D1 | SM1: BB-1 | SM2: (cyclopent-1-en-1-yl)borane | Yield: 78% |
|---|---|---|---|---|
| 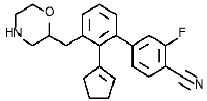 | 1H NMR (DMSO-d6, 500 MHz): δ (ppm) 7.91 (dd, J = 8.1, 7.1 Hz, 1H), 7.47 (dd, J = 10.6, 1.6 Hz, 1H), 7.32 (s, 1H), 7.29-7.38 (m, 3H), 7.18-7.24 (m, 1H), 7.32 (br s, 2H), 5.64 (quin, J = 2.1 Hz, 1H), 3.92 (s, 2H), 3.83 (dd, J = 12.0, 2.9 Hz, 1H), 3.63-3.73 (m, 1H), 3.51 (td, J = 12.0, 2.4 Hz, 1H), 3.09-4.26 (m, 3H), 2.96 (br d, J = 12.5 Hz, 2H), 2.84 (td, J = 12.2, 3.7 Hz, 1H), 2.73 (br d, J = 6.4 Hz, 2H), 2.63 (dd, J = 12.5, 10.8 Hz, 1H), 2.32 (ddd, J = 7.2, 4.5, 2.3 Hz, 2H), 2.07 (s, 1H), 1.95-2.17 (m, 1H), 1.71 (quin, J = 7.2 Hz, 2H). m/z: 363 [M+H]+ | | | |
| 2'-(cyclopent-1-en-1-yl)-3-fluoro-3'-[(morpholin-2-yl)methyl]-[1,1'-biphenyl]-4-carbonitrile | | | | |

| Example 9 | Procedure: B1+H2+N2+H1+R1. 1+R1.2+R1.3+I1+B2 +Chiral Separation | SM1: BB - 1 | SM2: 2-ethenyl-4,4,5,5-tetramethyl-1,3,2-dioxaborolane | Yield: 87% |
|---|---|---|---|---|
| 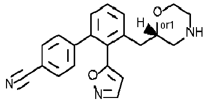 | 1H NMR (600 MHz, DMSO-d6) δ ppm 2.64 - 2.76 (m, 3 H) 2.93 (dt, J=2.90, 1.27 Hz, 1 H) 3.05 (brd, J=12.32 Hz, 1 H) 3.12 (brd, J=12.76 Hz, 1 H) 3.61 (td, J=12.51, 2.27 Hz, 1 H) 3.76 - 3.84 (m, 1 H) 3.89 (dd, J=12.62, 3.37 Hz, 1 H) 6.43 (d, J=1.91 Hz, 1 H) 7.27 - 7.36 (m, 2 H) 7.42 (d, J=7.63 Hz, 1 H) 7.52 (d, J=7.19 Hz, 1 H) 7.60 - 7.68 (m, 1 H) 7.72 - 7.81 (m, 2 H) 8.55 (d, J=1.76 Hz, 1 H) 9.12 (br s, 2 H). m/z: 346 [M+H]+ | | | |
| 3'-{[(2S)-mor pholin-2-yl]methyl}-2'-(1,2-oxazol-5-yl)-[1,1'-biphenyl]-4-carbonitrile | | | | |

| Example 7 | Procedure: B1+H2+N2+H1+R1. 1+R1.2+R1.3+I1+B2 +Chiral Separation | SM1: BB-1 | SM2: 2-ethenyl-4,4,5,5-tetramethyl-1,3,2-dioxaborolane | Yield: 88% |
|---|---|---|---|---|
| 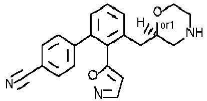 | 1H NMR (600 MHz, DMSO-d6) δ ppm 2.66 - 2.74 (m, 3 H) 2.89 - 2.97 (m, 1 H) 3.05 (brd, J=12.32 Hz, 1 H) 3.12 (brd, J=12.62 Hz, 1 H) 3.60 (td, J=12.43, 2.27 Hz, 1 H) 3.76 - 3.83 (m, 1 H) 3.90 (dd, J=12.62, 3.37 Hz, 1 H) 6.43 (d, J=1.91 Hz, 1 H) 7.28 - 7.32 (m, 2 H) 7.42 (dd, J=7.70, 0.95 Hz, 1 H) 7.52 (d, J=7.82 Hz, 1 H) 7.64 (t, J=7.78 Hz, 1 H) 7.77 (d, J=8.22 Hz, 2 H) 8.55 (d, J=1.91 Hz, 1 H) 9.10 (br s, 2 H). m/z: 346 [M+H]+ | | | |
| 3'-{[(2R)-morpholin-2-yl]methyl}-2'-(1,2-oxazol-5-yl)-[1,1'-biphenyl]-4-carbonitrile | | | | |

| Example 5 | Procedure: B6+B2+D2 | SM1: BB-4-E1 | SM2: 5-bromo-1 ,2-thiazole | Yield: 63% |
|---|---|---|---|---|
| 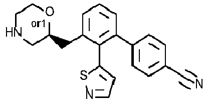 | 1H NMR (600 MHz, DMSO-d6) δ ppm 2.64 - 2.73 (m, 3 H) 2.91 (td, J=12.51, 3.89 Hz, 1 H) 3.03 (br d, J=12.32 Hz, 1 H) 3.11 (br d, J=12.62 Hz, 1 H) 3.59 (td, J=12.43, 2.27 Hz, 1 H) 3.74 - 3.81 (m, 1 H) 3.89 (dd, J=12.62, 3.52 Hz, 1 H) 7.32 - 7.36 (m, 4 H) 7.50 (d, J=7.19 Hz, 1 H) 7.56 (t, J=7.70 Hz, 1 H) 7.71 (d, J=8.22 Hz, 2 H) 8.49 (d, J=1.61 Hz, 1 H) 8.99 (br s, 2 H). m/z: 362 [M+H]+ | | | |
| 3'-{[(2S)-morpholin-2-yl]methyl}-2'-(1,2-thiazol-5-yl)-[1,1'-biphenyl]-4-carbonitrile | | | | |

| Example 11 | Procedure: B6+B2+D2 | SM1: BB-4-E2 | SM2: 5-bromo-1 ,2-thiazole | Yield: 79% |
|---|---|---|---|---|
| 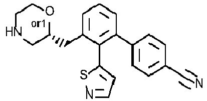 | 1H NMR (600 MHz, DMSO-d6) δ ppm 2.65 - 2.72 (m, 3 H) 2.92 (td, J=12.54, 3.81 Hz, 1 H) 3.04 (brd, J=12.47 Hz, 1 H) 3.11 (brd, J=12.47 Hz, 1 H) 3.60 (td, J=12.47, 2.35 Hz, 1 H) 3.76 - 3.82 (m, 1 H) 3.89 (dd, J=12.62, 3.52 Hz, 1 H) 7.32 - 7.36 (m, 4 H) 7.50 (d, J=7.19 Hz, 1 H) 7.56 (t, J=7.70 Hz, 1 H) 7.71 (d, J=8.07 Hz, 2 H) 8.49 (d, J=1.61 Hz, 1 H) 9.07 (br s, 2 H). m/z: 362 [M+H]+ | | | |
| 3'-{[(2R)-morpholin-2-yl]methyl}-2'-(1,2-thiazol-5-yl)-[1,1'-biphenyl]-4-carbonitrile | | | | |

| Example 15 | Procedure: C1+E3+B2+D2 | SM1: BB-1-E2 | SM2: 1,3-oxazole | Yield: 72% |
|---|---|---|---|---|
| 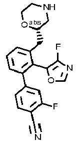 | 1H NMR (500 MHz, DMSO-d6) δ 9.23 (br s, 2H), 8.35 (d, J = 2.2 Hz, 1H), 7.89 (dd, J = 7.9, 7.0 Hz, 1H), 7.71 - 7.62 (m, 1H), 7.56 (dd, J = 7.8, 1.0 Hz, 1H), 7.46 (dd, J = 7.8, 1.2 Hz, 1H), 7.36 (dd, J = 10.4, 1.6 Hz, 1H), 7.10 (dd, J = 8.1, 1.7 Hz, 1H), 3.87 (dd, J = 12.6, 3.3 Hz, 1H), 3.83 - 3.77 (m, 1H), 3.61 (td, J = 12.4, 2.3 Hz, 1H), 3.11 (br d, J = 12.5 Hz, 2H), 2.98 - 2.86 (m, 1H), 2.84 - 2.67 (m, 3H). m/z: 382 [M+H]+ | | | |
| 3-fluoro-2'-(4-fluoro-1,3-oxazol-5-yl)-3'-{[(2S)-morpholin-2-yl]methyl}-[1,1'-biphenyl]-4-carbonitrile | | | | |

| Example 26 | Procedure: C1+E3+B2+D2 | SM1: BB-1-E1 | SM2: 1,3-oxazole | Yield: 74% |
|---|---|---|---|---|
| 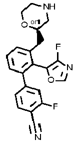 | 1H NMR (DMSO-d6, 500 MHz) δ 9.21 (br s, 2H), 8.35 (d, 1H, J=2.0 Hz), 7.89 (dd, 1H, J=7.1, 7.8 Hz), 7.6-7.7 (m, 1H), 7.56 (dd, 1H, J=1.2, 7.8 Hz), 7.46 (dd, 1H, J=1.1, 7.7 Hz), 7.37 (dd, 1H, J=1.5, 10.5 Hz), 7.10 (dd, 1H, J=1.6, 7.9 Hz), 3.87 (dd, 1H, J=3.4, 12.5 Hz), 3.7-3.8 (m, 1H), 3.5-3.7 (m, 1H), 3.11 (br d, 2H, J=12.5 Hz), 2.9-3.0 (m, 1H), 2.7-2.8 (m, 3H). m/z: 382 [M+H]+ | | | |
| 3-fluoro-2'-(4-fluoro-1,3-oxazol-5-yl)-3'-{[(2R)-morpholin-2-yl]methyl}-[1,1'-biphenyl]-4-carbonitrile | | | | |

| Example 148 | Procedure: B1+B2+D1 | SM1: BB-1 | SM2: 4-(4,4,5,5-tetramethyl-1,3,2-dioxaborolan-2-yl)pyridine | Yield: 83% |
|---|---|---|---|---|
| 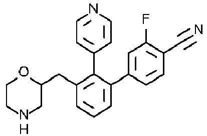 | 1H NMR (DMSO-d6, 500 MHz) δ 8.49 (d, 1H, J=5.1 Hz), 8.47 (d, 1H, J=4.9 Hz), 7.7-7.8 (m, 1H), 7.4-7.6 (m, 2H), 7.34 (dd, 1H, J=2.7, 6.4 Hz), 7.27 (dd, 1H, J=1.5, 10.5 Hz), 7.18 (dd, 1H, J=0.9, 5.0 Hz), 7.11 (dd, 1H, J=0.9, 5.0 Hz), 7.06 (dd, 1H, J=1.6, 7.9 Hz), 3.89 (s, 2H), 3.1-3.8 (m, 7H), 2.81 (brd, 1H, J=12.2 Hz), 2.70 (br d, 2H, J=12.0 Hz), 2.5-2.6 (m, 2H), 2.3-2.4 (m, 1H). m/z: 374 [M+H]+ | | | |
| 3-fluoro-3'-[(morpholin-2-yl)methyl]-2'-(pyridin-4-yl)-[1,1'-biphenyl]-4-carbonitrile | | | | |

| Example 111 | Procedure: C1+N2+F5+B2 | SM1: 2-bromo-3-chlorobenzaldehyde | SM2: 1,2-thiazole | Yield: 34% |
|---|---|---|---|---|
| 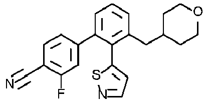 | 1H NMR (600 MHz, DMSO-d6) δ ppm 8.51 (d, J=1.3 Hz, 1 H), 7.78 (t, J=7.3 Hz, 1 H), 7.53 (m, 1 H), 7.46 (d, J=7.8 Hz, 1 H), 7.38 (d, J=10.4 Hz, 1 H), 7.35 (d, J=1.3 Hz, 1 H), 7.33 (d, J=7.6 Hz, 1 H), 7.18 (d, J=8.1 Hz, 1 H), 3.74 (dd, J=11.3, 3.5 Hz, 2 H), 3.14 (s, 2 H), 2.45 (d, J=7.0 Hz, 2 H), 1.58 (ddd, J=11.2, 7.2, 4.0 Hz, 1 H), 1.37 (m, 2 H), 1.10 (m, 2 H). m/z: 379.2 [M+H]+ | | | |
| 3-fluoro-3'-[(oxan-4-yl)methyl]-2'-(1,2-thiazol-5-yl)-[1,1'-biphenyl]-4-carbonitrile | | | | |

| Example 71 | Procedure: N1+D6+F3+I1+C1+ B2+D1 | SM1: 2-bromo-1-(bromomethyl)-3-chlorobenzene | SM2: 1-[(4-methoxyphenyl)methy l]piperidin-2-one | Yield: |
|---|---|---|---|---|
| 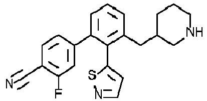 | 1H NMR (500 MHz, DMSO-d6) δ 9.10 - 8.63 (m, 1H), 8.55 - 8.51 (m, 1H), 8.38 - 7.88 (m, 1H), 7.80 (dd, J = 7.8, 7.1 Hz, 1H), 7.62 - 7.52 (m, 1H), 7.50 - 7.43 (m, 1H), 7.39 - 7.32 (m, 3H), 7.18 (dd, J = 8.1, 1.5 Hz, 1H), 3.11 (br d, J = 12.2 Hz, 1H), 2.94 (br d, J = 11.0 Hz, 1H), 2.72 - 2.63 (m, 1H), 2.49 - 2.40 (m, 3H), 1.84 - 1.72 (m, 1H), 1.71 - 1.61 (m, 1H), 1.58 - 1.50 (m, 1H), 1.49 - 1.36 (m, 1H), 1.12 - 0.98 (m, 1H). m/z: 378 [M+H]+ | | | |
| 3-fluoro-3'-[(piperidin-3-yl)methyl]-2'-(1,2-thiazol-5-yl)-[1,1'-biphenyl]-4-carbonitrile | | | | |

| Example 18 | Procedure: F3+E4+N1+D4+F3+ I1+C1+B2+D1 | SM1: methyl 2-bromo-3-chloro-5-fluorobenzoate | SM2: 4-[(4-methoxyphenyl)methy l]morpholin-3-one | Yield: 100% |
|---|---|---|---|---|
| 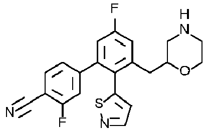 | 1H NMR (DMSO-d6, 500 MHz) δ 8.51 (d, 1H, J=1.7 Hz), 7.81 (dd, 1H, J=7.1, 7.8 Hz), 7.4-7.5 (m, 2H), 7.34 (d, 1H, J=1.7 Hz), 7.30 (dd, 1H, J=2.7, 9.0 Hz), 7.19 (dd, 1H, J=1.5, 8.1 Hz), 3.93 (s, 1H), 3.78 (dd, 1H, J=2.7, 11.7 Hz), 3.0-3.6 (m, 7H), 2.7-2.9 (m, 3H), 2.6-2.7 (m, 2H), 2.4-2.5 (m, 1H). m/z: 398.2 [M+H]+ | | | |
| 3,5'-difluoro-3'-[(morpholin-2-yl)methyl]-2'-(1,2-thiazol-5-yl)-[1,1'-biphenyl]-4-carbonitrile | | | | |

| Example 53 | Procedure: K3+F4+E4+N1+D4+ F3+I1+B1+B1+D1 | SM1: ethyl 2-amino-3-bromo-5-methylbenzoate | SM2: 4-[(4-methoxyphenyl)methy l]morpholin-3-one | Yield: 86% |
|---|---|---|---|---|
| 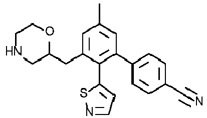 | 1H NMR (600 MHz, DMSO-d6) δ ppm 2.32 - 2.38 (m, 1 H) 2.38 (s, 3 H) 2.53 - 2.59 (m, 2 H) 2.62 - 2.77 (m, 3 H) 3.33 - 3.40 (m, 6 H) 3.44 - 3.49 (m, 1 H) 3.70 (dd, J=11.52, 2.27 Hz, 1 H) 3.80 (s, 2 H) 7.14 (d, J=1.03 Hz, 1 H) 7.26 (d, J=1.61 Hz, 1 H) 7.29 (d, J=1.03 Hz, 1 H) 7.32 (d, J=8.51 Hz, 2 H) 7.69 (d, J=8.51 Hz, 2 H) 8.47 (d, J=1.76 Hz, 1 H). m/z: 376.2 [M+H]+ | | | |
| 5'-methyl-3'-[(morpholin-2-yl)methyl]-2'-(1,2-thiazol-5-yl)-[1,1'-biphenyl]-4-carbonitrile | | | | |

| Example 43 | Procedure: C1+B2+D1 | SM1: BB-1 | SM2: 1,2-thiazole | Yield: 75% |
|---|---|---|---|---|
| 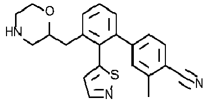 | 1H NMR (600 MHz, DMSO-d6) δ ppm 8.49 (d, J=1.6 Hz, 1 H), 7.59 (d, J=7.9 Hz, 1 H), 7.52 (m, 1 H), 7.46 (m, 1 H), 7.31 (td, J=3.7, 1.3 Hz, 2 H), 7.28 (d, J=1.0 Hz, 1 H), 7.09 (dd, J=8.0, 1.2 Hz, 1 H), 3.88 (s, 2 H), 3.75 (dd, J=11.8, 2.7 Hz, 1 H), 3.55 (m, 1 H), 3.41 (td, J=11.7, 2.5 Hz, 1 H), 2.77 (m, 3 H), 2.61 (d, J=6.5 Hz, 2 H), 2.45 (dd, J=12.3, 10.5 Hz, 1 H), 2.39 (t, J=0.8 Hz, 3 H). m/z: 376.2 [M+H]+ | | | |
| 3-methyl-3'-[(morpholin-2-yl)methyl]-2'-(1,2-thiazol-5-yl)-[1,1'-biphenyl]-4-carbonitrile | | | | |

| Example 45 | Procedure: B6+B2+D1 | SM1: BB-4 | SM2: 5-bromo-4-methyl-1,2-thiazole | Yield: 78% |
|---|---|---|---|---|
| 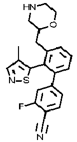 | 1H NMR (500 MHz, DMSO-d6) δ 9.10 - 8.63 (m, 1H), 8.55 - 8.51 (m, 1H), 8.38 - 7.88 (m, 1H), 7.80 (dd, J = 7.8, 7.1 Hz, 1H), 7.62 - 7.52 (m, 1H), 7.50 - 7.43 (m, 1H), 7.39 - 7.32 (m, 3H), 7.18 (dd, J = 8.1, 1.5 Hz, 1H), 3.11 (br d, J = 12.2 Hz, 1H), 2.94 (br d, J = 11.0 Hz, 1H), 2.72 - 2.63 (m, 1H), 2.49 - 2.40 (m, 3H), 1.84 - 1.72 (m, 1H), 1.71 - 1.61 (m, 1H), 1.58 - 1.50 (m, 1H), 1.49 - 1.36 (m, 1H), 1.12 - 0.98 (m, 1H). m/z: 394 [M+H]+ | | | |
| 3-fluoro-2'-(4-methyl-1,2-thiazol-5-yl)-3'-[(morpholin-2-yl)methyl]-[1,1'-biphenyl]-4-carbonitrile | | | | |

| Example 103 | Procedure: C1+B2+D1 | SM1: BB-2G | SM2: 1,3-thiazole | Yield: 76% |
|---|---|---|---|---|
| 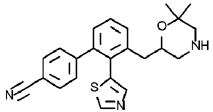 | 1H NMR (500 MHz, DMSO-d6) δ ppm 1.05 (s, 3 H) 1.15 (s, 3 H) 2.28 (t, J=11.62 Hz, 1 H) 2.47 (s, 1 H) 2.53 - 2.64 (m, 2 H) 2.69 - 2.76 (m, 2 H) 3.21 (br s, 5 H) 3.71 - 3.81 (m, 1 H) 3.97 (s, 1 H) 7.27 - 7.34 (m, 3 H) 7.45 - 7.49 (m, 1 H) 7.49 - 7.55 (m, 1 H) 7.67 - 7.73 (m, 3 H) 9.04 (d, J=0.73 Hz, 1 H). m/z: 390 [M+H]+ | | | |
| 3'-[(6,6-dimethylmorpholin-2-yl)methyl]-2'-(1,3-thiazol-5-yl)-[1,1'-biphenyl]-4-carbonitrile | | | | |

| Example 2 | Procedure: C1+E3+B2+D1 | SM1: BB-1-E2 | SM2: 1,3-oxazole | Yield: 86% |
|---|---|---|---|---|
| 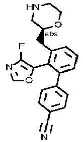 | 1H NMR (DMSO-d6, 500 MHz) δ 8.32 (d, 1H, J=2.0 Hz), 7.81 (d, 2H, J=8.6 Hz), 7.6-7.7 (m, 1H), 7.53 (dd, 1H, J=1.0, 7.8 Hz), 7.41 (dd, 1H, J=1.1, 7.7 Hz), 7.30 (d, 2H, J=8.6 Hz), 3.92 (s, 1H), 3.76 (dd, 1H, J=2.9, 11.7 Hz), 3.5-3.6 (m, 1H), 3.44 (dt, 1H, J=2.3, 11.8 Hz), 3.0-3.4 (m, 1H), 2.87 (br d, 2H, J=12.2 Hz), 2.7-2.8 (m, 1H), 2.6-2.7 (m, 2H), 2.5-2.6 (m, 1H). m/z: 364 [M+H]+ | | | |
| 2'-(4-fluoro-1,3-oxazol-5-yl)-3'-{[(2S)-morpholin-2-yl]methyl}-[1,1'-biphenyl]-4-carbonitrile | | | | |

| Example 3 | Procedure: C1 +E3+B2+D1 | SM1: BB-1-E1 | SM2: 1,3-oxazole | Yield: 78% |
|---|---|---|---|---|
| 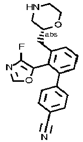 | 1H NMR (DMSO-d6, 500 MHz) δ 8.32 (d, 1H, J=2.2 Hz), 7.8-7.9 (m, 2H), 7.6-7.7 (m, 1H), 7.52 (dd, 1H, J=1.0, 7.8 Hz), 7.41 (dd, 1H, J=1.2, 7.6 Hz), 7.30 (d, 2H, J=8.6 Hz), 3.88 (s, 1H), 3.7-3.8 (m, 1H), 3.5-3.6 (m, 2H), 3.41 (dt, 2H, J=2.4, 11.7 Hz), 2.81 (br d, 2H, J=12.0 Hz), 2.6-2.8 (m, 3H), 2.46 (dd, 1H, J=10.6, 12.1 Hz), m/z: 364.2 [M+H]+ | | | |
| 2'-(4-fluoro-1,3-oxazol-5-yl)-3'-{[(2R)-morpholin-2-yl]methyl}-[1,1'-biphenyl]-4-carbonitrile | | | | |

| Example 37 | Procedure: C1 +B2+Chiral Separation+D2 | SM1: BB-1 | SM2: 4-chloro-1,3-thiazole | Yield: 49% |
|---|---|---|---|---|
| 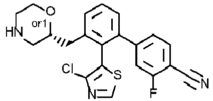 | 1H NMR (DMSO-d6, 600 MHz): δ (ppm) 9.15-9.18 (m, 1H), 8.90-9.11 (m, 2H), 7.82-7.88 (m, 1H), 7.58-7.64 (m, 1H), 7.51-7.57 (m, 1H), 7.37-7.43 (m, 1H), 7.28-7.34 (m, 1H), 7.08-7.13 (m, 1H), 3.82-3.92 (m, 1H), 3.75-3.78 (m, 1H), 3.49-3.61 (m, 1H), 3.01-3.14 (m, 2H), 2.85-2.94 (m, 1H), 2.75-2.80 (m, 1H), 2.67-2.74 (m, 1H), 2.59-2.64 (m, 1H). m/z: 414.2 [M+H]+ | | | |
| 2'-(4-chloro-1,3-thiazol-5-yl)-3-fluoro-3'-{[(2R)-morpholin-2-yl]methyl}-[1,1'-biphenyl]-4-carbonitrile | | | | |

| Example 31 | Procedure: C1+B2+Chiral Separation+D2 | SM1: BB-1 | SM2: 4-chloro-1,3-thiazole | Yield: 42% |
|---|---|---|---|---|
| 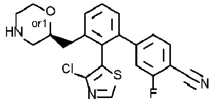 | 1H NMR (DMSO-d6, 600 MHz): δ (ppm) 9.15-9.18 (m, 1H), 8.90-9.11 (m, 2H), 7.82-7.88 (m, 1H), 7.58-7.64 (m, 1H), 7.51-7.57 (m, 1H), 7.37-7.43 (m, 1H), 7.28-7.34 (m, 1H), 7.08-7.13 (m, 1H), 3.82-3.92 (m, 1H), 3.75-3.78 (m, 1H), 3.49-3.61 (m, 1H), 3.01-3.14 (m, 2H), 2.85-2.94 (m, 1H), 2.75-2.80 (m, 1H), 2.67-2.74 (m, 1H), 2.59-2.64 (m, 1H). m/z: 414 [M+H]+ | | | |
| 2'-(4-chloro-1,3-thiazol-5-yl)-3-fluoro-3'-{[(2S)-morpholin-2-yl]methyl}-[1,1'-biphenyl]-4-carbonitrile | | | | |

| Example 14 | Procedure: Chiral Separation | SM1: Example 18 | | Yield: 24% |
|---|---|---|---|---|
| 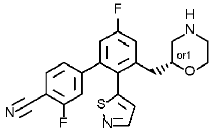 | 1H NMR (600 MHz, DMSO-d6) δ ppm 8.52 (d, J=1.61 Hz, 1 H) 7.74 - 7.86 (m, 1 H) 7.41 (ddd, J=14.45, 10.05, 2.05 Hz, 2 H) 7.34 (d, J=1.61 Hz, 1 H) 7.31 (dd, J=8.95, 2.64 Hz, 1 H) 7.19 (dd, J=8.07, 1.61 Hz, 1 H) 3.97 - 4.02 (m, 1 H) 3.78 - 3.84 (m, 1 H) 3.58 - 3.64 (m, 1 H) 3.44 - 3.49 (m, 3 H) 2.83 - 2.95 (m, 2 H) 2.74 - 2.81 (m, 1 H) 2.60 - 2.66 (m, 2 H) 2.51 - 2.54 (m, 1 H). m/z: 398.2 [M+H]+ | | | |
| 3,5'-difluoro-3'-{[(2R)-morpholin-2-yl]methyl}-2'-(1,2-thiazol-5-yl)-[1,1'-biphenyl]-4-carbonitrile | | | | |

| Example 6 | Procedure: Chiral Separation | SM1: Example 18 | | Yield: 29% |
|---|---|---|---|---|
| 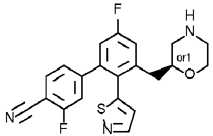 | 1H NMR (600 MHz, DMSO-d6) δ ppm 8.51 (d, J=1.61 Hz, 1 H) 7.80 (dd, J=7.92, 7.04 Hz, 1 H) 7.42 (dd, J=1 0.34, 1.39 Hz, 1 H) 7.37 - 7.40 (m, 1 H) 7.33 (d, J=1.61 Hz, 1 H) 7.30 (dd, J=8.95, 2.64 Hz, 1 H) 7.19 (dd, J=8.07, 1.47 Hz, 1 H) 3.78 (br dd, J=11.44, 2.05 Hz, 1 H) 3.57 - 3.62 (m, 1 H) 3.46 (br s, 1 H) 2.79 - 2.90 (m, 2 H) 2.71 - 2.77 (m, 1 H) 2.59 - 2.65 (m, 2 H). m/z: 398.1 [M+H]+ | | | |
| 3,5'-difluoro-3'-{[(2S)-morpholin-2-yl]methyl}-2'-(1,2-thiazol-5-yl)-[1,1'-biphenyl]-4-carbonitrile | | | | |

| Example 87 | Procedure: E5+P1+E4+A1.1 +C2 | SM1: 1-bromo-2-chloro-5-methyl-3-nitrobenzene | | Yield: 5% |
|---|---|---|---|---|
| 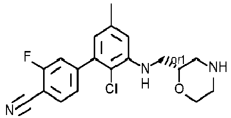 | 1H NMR (CDCl₃, 600 MHz): δ (ppm) 7.64 (dd, J = 7.8, 6.7 Hz, 1H), 7.26-7.32 (m, 2H), 6.52 (d, J = 1.3 Hz, 1H), 6.44 (d, J = 1.3 Hz, 1H), 4.79 (br s, 1H), 3.97 (br d, J = 11.4 Hz, 1H), 3.84 (br dd, J = 5.6, 4.5 Hz, 1H), 3.71 - 3.77 (m, 1H), 3.25-3.32 (m, 1H), 3.18-3.24 (m, 1H), 3.07 (br d, J = 12.0 Hz, 1H), 2.95 (br d, J = 5.6 Hz, 2H), 2.78 (t, J = 11.2 Hz, 1H), 2.31 (s, 3H). m/z: 360 [M+H]+ | | | |
| 2'-chloro-3-fluoro-5'-methyl-3'-({[(2S)-morpholin-2-yl]methyl}amino)-[1,1'-biphenyl]-4-carbonitrile | | | | |

| Example 89 | Procedure: E5+P1+E4+A1.1 +C2 | SM1: 1-bromo-2-chloro-5-methyl-3-nitrobenzene | | Yield: 92% |
|---|---|---|---|---|
| 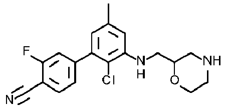 | 1H NMR (DMSO-d6, 500 MHz): δ (ppm) 8.72-9.42 (m, 2H), 7.95-8.02 (m, 1H), 7.56 (dd, J = 10.3, 1.5 Hz, 1H), 7.42 (dd, J = 7.9, 1.6 Hz, 1H), 6.71 (d, J = 1.5 Hz, 1H), 6.49 (d, J = 1.5 Hz, 1H), 5.57 (t, J = 6.1 Hz, 1H), 4.00 (dd, J = 12.6, 3.3 Hz, 1H), 3.87-3.95 (m, 1H), 3.73 (td, J = 12.4, 2.3 Hz, 1H), 3.31-3.40 (m, 2H), 3.27-3.30 (m, 1H), 3.18 (br d, J = 13.0 Hz, 1H), 3.00 (td, J = 12.5, 3.5 Hz, 1H), 2.84 (br t, J = 11.9 Hz, 1H), 2.27 (s, 3H). m/z: 360 [M+H]+ | | | |
| 2'-chloro-3-fluoro-5'-methyl-3'-{[(morpholin-2-yl)methyl]amino}-[1,1'-biphenyl]-4-carbonitrile | | | | |

| Example 90 | Procedure: G5+ B2+D1 | SM1: BB-1 | SM2: 1H-pyrazole | Yield: 42% |
|---|---|---|---|---|
| 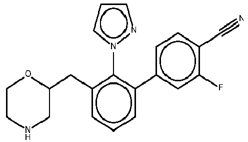 | 1H NMR (DMSO-d6, 500 MHz)+ around 0.5 mol of tartrate: δ 7.81 (t, J=7.58 Hz, 1H), 7.63-7.67 (m, 2H), 7.54-7.63 (m, 2H), 7.50 (d, J=7.34 Hz, 1H), 7.10-7.15 (m, 1H), 7.06 (d, J=8.07 Hz, 1H), 6.35 (t, J=2.08 Hz, 1H), 3.88 (s, 1H), 3.74 (br dd, J=2.69, 11.74 Hz, 1H), 3.49 (br s, 1H), 3.40 (br dd, J=9.66, 11.62 Hz, 1H), 2.76-2.85 (m, 1H), 2.63-2.75 (m, 2H), 2.51-2.54 (m, 1H), 2.33-2.47 (m, 2H) m/z: 363 [M+H]+ | | | |
| 2-fluoro-4-[3-(morpholin-2-ylmethyl)-2-pyrazol-1-yl-phenyl]benzonitrile | | | | |

| Example 109 | Procedure: N1 +04+F3+11 + C1+B2+D2 | SM1: 1-[(4-methoxyphenyl) methyl] pyrrolidin-2-one | SM2: 2-bromo-1-(bromomethyl)-3-chlorobenzene | Yield: 84% |
|---|---|---|---|---|
| 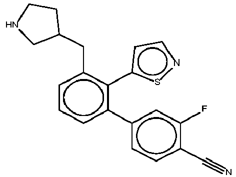 | 1H NMR (DMSO-d6, 500 MHz) δ 8.70 (m, 2 H), 8.53 (d, J=1.6 Hz, 1 H), 7.79 (m, 1 H), 7.58 (m, 2 H), 7.37 (m, 3 H), 7.18 (dd, J=8.0, 1.5 Hz, 1 H), 3.14 (m, 2 H), 3.04 (s, 1 H), 2.71 (m, 2 H), 2.61 (m, 1 H), 2.31 (m, 1 H), 1.86 (m, 1 H), 1.46 (dd, J=12.8, 8.7 Hz, 1 H). m/z: 364 [M+H]+ | | | |
| 2-fluoro-4-[2-isothiazol-5-yl-3-(pyrrolidin-3-ylmethyl)phenyl]benzonitrile;hydrochloride | | | | |

| Example 134 | Procedure: A1+ E1+B1+D1 | SM1: BB-2 | SM2: azetidine | Yield: 87% |
|---|---|---|---|---|
| 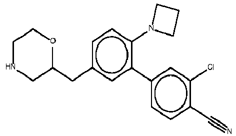 | 1H NMR (DMSO-d6, 500 MHz) δ 7.99 (d, J = 8.1 Hz, 1H), 7.70 (d, J = 1.5 Hz, 1H), 7.54 (dd, J = 1.6, 8.1 Hz, 1H), 7.14 (dd, J = 2.0, 8.3 Hz, 1H), 6.99 (d, J = 2.1 Hz, 1H), 6.58 (d, J = 8.2 Hz, 1H), 3.96 (br s, 2H), 3.86 (br dd, J = 3.2, 12.3 Hz, 1H), 3.75 - 3.64 (m, 1H), 3.59 - 3.49 (m, 5H), 3.47 - 3.42 (m, 2H), 3.01 (br t, J = 11.4 Hz, 3H), 2.84 (br d, J = 3.4 Hz, 2H), 2.71 - 2.56 (m, 4H), 2.16 - 2.02 (m, 2H). m/z: 368 [M+H]+ | | | |
| 4-[2-(azetidin-1-yl)-5-(morpholin-2-ylmethyl)phenyl]-2-chloro-benzonitrile | | | | |

**Table 3: Compounds of the disclosure and procedures used in their synthesis (Series 3).**

| **SERIES 3** | | | | |
|---|---|---|---|---|
| Example 299 | Procedure: A1.1+B1+C2 | SM1: 3-bromo-2-chloro-5-methylbenzaldehyde | SM2: (4-cyano-3-fluorophenyl)boronic acid | Yield: 77% |
| 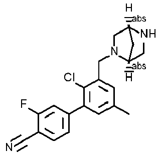 | 1H NMR (DMSO-d6,+TFA at 373K 500 MHz): δ (ppm) 7.96 (dd, J = 7.9, 7.0 Hz, 1H), 7.58-7.65 (m, 1H), 7.51-7.57 (m, 1H), 7.45 (dd, J = 8.1, 1.5 Hz, 1H), 7.34 (d, J = 1.7 Hz, 1H), 4.25-4.58 (m, 4H), 3.55-3.78 (m, 2H), 3.32-3.52 (m, 2H), 2.43-2.48 (m, 1H), 2.37-2.40 (m, 3H), 2.07-2.14 (m, 1H). m/z: 355.9 [M+H]+ | | | |
| 2'-chloro-3'-{[(1S,4S)-2,5-diazabicyclo[2.2.1]heptan-2-yl]methyl}-3-fluoro-5'-methyl-[1 ,1'-biphenyl]-4-carbonitrile | | | | |

| Example 226 | Procedure: A1.1+B1+C2 | SM1: 3-bromo-2-chloro-5-methylbenzaldehyde | SM2: (4-cyano-3-fluorophenyl)boronic acid | Yield: 48% |
|---|---|---|---|---|
| 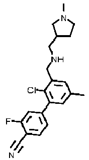 | 1H NMR (600 MHz, DMSO-d6) δ ppm 10.56 - 11.33 (m, 1 H) 8.96 - 9.92 (m, 2 H) 7.89 - 8.19 (m, 1 H) 7.71 (s, 1 H) 7.64 (brd, J=10.12 Hz, 1 H) 7.48 (dd, J=8.00, 1.25 Hz, 1 H) 7.37 (d, J=1.17 Hz, 1 H) 4.29 (br s, 2 H) 3.42 - 3.82 (m, 2 H) 2.99 - 3.27 (m, 4 H) 2.83 - 2.98 (m, 1 H) 2.74 - 2.82 (m, 3 H) 2.37 (s, 3 H) 1.65 - 2.33 (m, 2 H). m/z: 371 [M+H]+ | | | |
| 2'-chloro-3-fluoro-5'-methyl-3'-({[(1-methylpyrrolidin-3-yl)methyl]amino}methyl)-[1,1'-biphenyl]-4-carbonitrile | | | | |

| Example 348 | Procedure: A1.1+B1+C2 | SM1: 3-bromo-2-chloro-5-methylbenzaldehyde | SM2: (4-cyano-3-fluorophenyl)boronic acid | Yield: 74% |
|---|---|---|---|---|
| 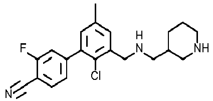 | 1H NMR (600 MHz, DMSO-d6) δ ppm 9.26 - 9.90 (m, 2 H) 8.72 - 9.18 (m, 2 H) 8.00 - 8.12 (m, 1 H) 7.71 (br d, J=1.00 Hz, 1 H) 7.63 (dd, J=10.27, 1.47 Hz, 1 H) 7.48 (dd, J=8.07, 1.47 Hz, 1 H) 7.38 (br d, J=1.00 Hz, 1 H) 4.30 (br s, 2 H) 3.44 (brd, J=11.74 Hz, 1 H) 3.19 (brd, J=12.18 Hz, 1 H) 2.90 - 3.09 (m, 2 H) 2.65 - 2.84 (m, 2 H) 2.38 (s, 3 H) 2.23 - 2.35 (m, 1 H) 1.86 - 1.97 (m, 1 H) 1.57 - 1.84 (m, 2 H) 1.18 - 1.36 (m, 1 H). m/z: 371 [M+H]+ | | | |
| 2'-chloro-3-fluoro-5'-methyl-3'-({[(piperidin-3-yl)methyl]amino}methyl)-[1,1'-biphenyl]-4-carbonitrile | | | | |

| Example 347 | Procedure: A1.1+B1+C2 | SM1: 3-bromo-2-chloro-5-methylbenzaldehyde | SM2: (4-cyano-3-fluorophenyl)boronic acid | Yield: 74% |
|---|---|---|---|---|
| 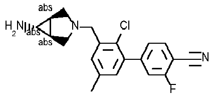 | 1H NMR (500 MHz, DMSO-d6) δ ppm 11.17 - 11.63 (m, 1 H) 8.48 (m, J=1.00 Hz, 3 H) 8.06 (br t, J=7.46 Hz, 1 H) 7.84 (s, 1 H) 7.57 - 7.70 (m, 1 H) 7.49 (m, J=7.60, 7.60 Hz, 1 H) 7.38 (br s, 1 H) 4.41 - 4.62 (m, 2 H) 3.52 - 3.73 (m, 5 H) 2.35 (s, 3 H) 2.17 - 2.32 (m, 2 H). m/z: 355.9 [M+H]+ | | | |
| 3'-{[(1R,5S,6R)-6-amino-3-azabicyclo[3.1.0]hexan-3-yl]methyl}-2'-chloro-3-fluoro-5'-methyl-[1 ,1'-biphenyl]-4-carbonitrile | | | | |

| Example 298 | Procedure: A1.1+B1+C2 | SM1: 3-bromo-2-chloro-5-methylbenzaldehyde | SM2: (4-cyano-3-fluorophenyl)boronic acid | Yield: 80% |
|---|---|---|---|---|
| 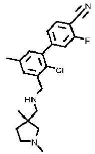 | 1H NMR (500 MHz, DMSO-d6) δ ppm 10.74 - 11.26 (m, 1 H) 9.14 - 9.67 (m, 2 H) 8.06 (m, J=7.50, 7.50, 3.40 Hz, 1 H) 7.79 (m, J=8.80 Hz, 1 H) 7.65 (dd, J=14.18, 10.76 Hz, 1 H) 7.50 (m, J=6.70, 6.70 Hz, 1 H) 7.38 (m, J=4.60 Hz, 1 H) 5.75 (s, 1 H) 4.35 (br s, 2 H) 3.75 (m, J=11.90, 5.00 Hz, 1 H) 3.49 - 3.65 (m, 1 H) 3.28 - 3.44 (m, 8 H) 3.08 - 3.26 (m, 3 H) 2.80 (m, J=6.70, 4.80 Hz, 4 H) 2.50 (dt, J=3.61, 1.74 Hz, 7 H) 2.38 (s, 3 H) 1.74 - 2.31 (m, 2 H) 1.31 (br d, J=6.85 Hz, 3 H) 1.09 (t, J=6.97 Hz, 1 H). m/z: 385 [M+H]+ | | | |
| 2'-chloro-3'-({[(1,3-dimethylpyrrolidin-3-yl)methyl]amino}methyl)-3-fluoro-5'-methyl-[1,1'-biphenyl]-4-carbonitrile | | | | |

| Example 346 | Procedure: A1.1+B1 | SM1: 3-bromo-2-chloro-5-methylbenzaldehyde | SM2: (4-cyano-3-fluorophenyl)boronic acid | Yield: 48% |
|---|---|---|---|---|
| 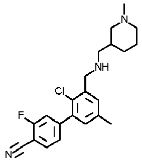 | 1H NMR (500 MHz, DMSO-d6) δ ppm 0.78 - 0.95 (m, 1 H) 1.36 - 1.51 (m, 1 H) 1.51 - 1.62 (m, 2 H) 1.64 - 1.74 (m, 2 H) 1.76 - 1.86 (m, 1 H) 1.96 - 2.28 (m, 4 H) 2.34 (s, 3 H) 2.37 - 2.46 (m, 2 H) 2.56 - 2.67 (m, 1 H) 2.72 - 2.86 (m, 1 H) 3.76 (s, 2 H) 7.15 (d, J=1.71 Hz, 1 H) 7.45 (m, J=2.70 Hz, 2 H) 7.56 - 7.67 (m, 1 H) 7.96 - 8.06 (m, 1 H). m/z: 386 [M+H]+ | | | |
| 2'-chloro-3-fluoro-5'-methyl-3'-({[(1-methylpiperidin-3-yl)methyl]amino}methyl)-[1,1'-biphenyl]-4-carbonitrile | | | | |

| Example 345 | Procedure: A1.1+B1+C2+B1 | SM1: 3-bromo-2-chloro-5-methylbenzaldehyde | SM2: (4-cyano-3-fluorophenyl)boronic acid | Yield: 100% |
|---|---|---|---|---|
| 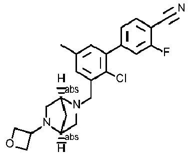 | 1H NMR (DMSO-d6, +TFA, 500 MHz): δ (ppm) 7.99-8.11 (m, 1H), 7.59-7.66 (m, 2H), 7.48 (dd, J = 8.1, 1.5 Hz, 1H), 7.40 (d, J = 1.7 Hz, 1H), 4.71-4.85 (m, 2H), 4.69 (dd, J = 8.3, 4.4 Hz, 1H), 4.56-4.66 (m, 4H), 4.54 (br s, 1H), 4.51 (br s, 1H), 3.76-3.96 (m, 2H), 3.62 (br d, J = 11.5 Hz, 1H), 3.41 (br d, J = 13.2 Hz, 1H), 2.58 (br d, J = 10.8 Hz, 1H), 2.38-2.44 (m, 1H), 2.37 (s, 3H). m/z: 411.9 [M+H]+ | | | |
| 2'-chloro-3-fluoro-5'-methyl-3'-{[(1S,4S)-5-(oxetan-3-yl)-2,5-diazabicyclo[2.2.1]heptan-2-yl]methyl}-[1,1'-biphenyl]-4-carbonitrile | | | | |

| Example 344 | Procedure: B1+H1.2+A1.1 + C1 | SM1: 3-bromo-4-iodobenzaldehyde | SM2: tert-butyl 5-oxa-2,8-diazaspiro[3.5]nonane -8-carboxylate | Yield: 98% |
|---|---|---|---|---|
| 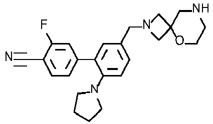 | 1H NMR (600 MHz, DMSO-d6) δ ppm 9.90 - 10.79 (m, 1 H) 8.73 - 9.41 (m, 2 H) 7.88 - 8.03 (m, 1 H) 7.50 (dd, J=1 0.56, 1.32 Hz, 1 H) 7.23 - 7.43 (m, 3 H) 6.96 (d, J=8.66 Hz, 1 H) 4.29 - 4.34 (m, 2 H) 4.14 - 4.20 (m, 2 H) 3.98 - 4.06 (m, 2 H) 3.83 - 3.87 (m, 2 H) 3.32 - 3.45 (m, 2 H) 3.05 - 3.18 (m, 2 H) 2.83 - 2.96 (m, 4 H) 1.63 - 1.85 (m, 4 H). m/z: 407 [M+H]+ | | | |
| 3-fluoro-5'-({5-oxa-2,8-diazaspiro[3.5]nonan-2-yl}methyl)-2'-(pyrrolidin-1-yl)-[1,1'-biphenyl]-4-carbonitrile | | | | |

| Example 297 | Procedure: A1.1+B1+C2+B1 | SM1: 3-bromo-2-chloro-5-methylbenzaldehyde | SM2: (4-cyano-3-fluorophenyl)boronic acid | Yield: 47% |
|---|---|---|---|---|
| 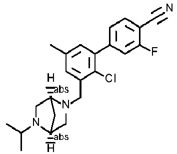 | 1H NMR (DMSO-d6, 600 MHz): δ (ppm) 8.00 (dd, J = 7.8, 7.1 Hz, 1H), 7.61 (dd, J = 10.3, 1.4 Hz, 1H), 7.41-7.48 (m, 2H), 7.14 (d, J = 1.8 Hz, 1H), 3.65-3.91 (m, 2H), 3.48 (br s, 1H), 3.27 (br s, 1H), 2.51-2.92 (m, 5H), 2.34 (s, 3H), 1.51-1.75 (m, 2H), 0.84-1.06 (m, 6H). m/z: 398 [M+H]+ | | | |
| 2'-chloro-3-fluoro-5'-methyl-3'-{[(1S,4S)-5-(propan-2-yl)-2,5-diazabicyclo[2.2.1]heptan-2-yl]methyl}-[1,1'-biphenyl]-4-carbonitrile | | | | |

| Example 240 | Procedure: A1.1+B1 | SM1: 3-bromo-2-chloro-5-methylbenzaldehyde | SM2: (4-cyano-3-fluorophenyl)boronic acid | Yield: 51% |
|---|---|---|---|---|
| 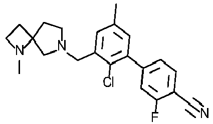 | 1H NMR (600 MHz, DMSO-d6) δ ppm 7.95 - 8.05 (m, 1 H) 7.62 (dd, J=10.42, 1.47 Hz, 1 H) 7.45 (dd, J=8.00, 1.54 Hz, 1 H) 7.39 (d, J=1.61 Hz, 1 H) 7.16 (d, J=1.76 Hz, 1 H) 3.53 - 3.74 (m, 2 H) 2.83 - 3.00 (m, 3 H) 2.61 (m, J=8.60, 8.60, 5.10 Hz, 1 H) 2.44 (q, J=8.12 Hz, 1 H) 2.36 - 2.40 (m, 1 H) 2.34 (s, 3 H) 2.15 (s, 3 H) 2.04 - 2.09 (m, 1 H) 1.98 - 2.04 (m, 2 H) 1.73 - 1.84 (m, 1 H). m/z: 384 [M+H]+ | | | |
| 2'-chloro-3-fluoro-5'-methyl-3'-({1-methyl-1,6-diazaspiro[3.4]octan-6-yl}methyl)-[1,1'-biphenyl]-4-carbonitrile | | | | |

| Example 296 | Procedure: B1+H5+A2.2 | SM1: 4-bromo-3-chlorobenzaldehyde | SM2: 1-methylpiperazine | Yield: 3% |
|---|---|---|---|---|
| 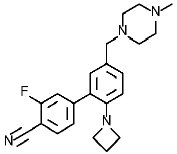 | 1H NMR (500 MHz, DMSO-d6) δ ppm 7.92 (t, J=7.58 Hz, 1 H) 7.48 - 7.56 (m, 1 H) 7.42 (dd, J=8.07, 1.47 Hz, 1 H) 7.20 (dd, J=8.31, 1.96 Hz, 1 H) 7.00 (d, J=1.96 Hz, 1 H) 6.59 (d, J=8.56 Hz, 1 H) 3.40 - 3.49 (m, 4 H) 3.36 (s, 2 H) 2.13 - 2.48 (m, 8 H) 2.13 (s, 3 H) 2.04 - 2.11 (m, 2 H). m/z: 365 [M+H]+ | | | |
| 2'-(azetidin-1-yl)-3-fluoro-5'-[(4-methylpiperazin-1-yl)methyl]-[1,1'-biphenyl]-4-carbonitrile | | | | |

| Example 295 | Procedure: B1+M1+B1+A1.2 | SM1: 4-bromo-3-chlorobenzaldehyde | SM2: 1-methylpiperazine | Yield: 23% |
|---|---|---|---|---|
| 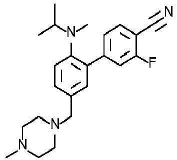 | 1H NMR (500 MHz, DMSO-d6, 300K) δ ppm 7.90 - 8.00 (m, 1 H), 7.70 (dd, J=11.0, 1.5 Hz, 1 H), 7.58 (dd, J=8.1, 1.5 Hz, 1 H), 7.26 (dd, J=8.2, 2.1 Hz, 1 H), 7.14 (d, J=2.2 Hz, 1 H), 7.11 (d, J=8.3 Hz, 1 H), 3.41 (s, 2 H), 2.98 (quin, J=6.6 Hz, 1 H), 2.53 (s, 3 H), 2.21 - 2.47 (m, 8 H), 2.14 (s, 3 H), 0.78 (d, J=6.6 Hz, 6 H). m/z: 381.1 [M+H]+ | | | |
| 3-fluoro-2'-[methyl(propan-2-yl)amino]-5'-[(4-methylpiperazin-1-yl)methyl]-[1,1'-biphenyl]-4-carbonitrile | | | | |

| Example 293 | Procedure: A1.1+I2+B1+C2 | SM1: (3-bromo-2,5-dimethylphenyl)metha nol | SM2: (4-cyano-3-fluorophenyl)boronic acid | Yield: 80% |
|---|---|---|---|---|
| 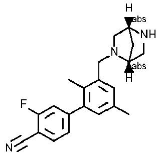 | 1H NMR (600 MHz, DMSO-d6) δ ppm 9.35 - 11.98 (m, 2 H) 8.02 (d, J=1.00 Hz, 1 H) 7.61 (s, 1 H) 7.53 (br d, J=2.49 Hz, 1 H) 7.38 (br dd, J=5.00 Hz, 1 H) 7.12 (s, 1 H) 4.21 - 4.93 (m, 4 H) 3.72 - 4.09 (m, 2 H) 3.16 - 3.46 (m, 2 H) 2.59 - 2.86 (m, 1 H) 2.33 (s, 3 H) 2.24 (s, 3 H) 1.98 - 2.15 (m, 1 H). m/z: 336 [M+H]+ | | | |
| 3'-{[(1R,4R)-2,5-diazabicyclo[2.2.1]heptan-2-yl]methyl}-3-fluoro-2' ,5'-dimethyl-[1 ,1'-biphenyl]-4-carbonitrile | | | | |

| Example 343 | Procedure: B1+H1.2+A1.1 + C1 | SM1: 3-bromo-4-iodobenzaldehyde | SM2: tert-butyl 5-oxa-2,8-diazaspiro[3.5]nonane -8-carboxylate | Yield: 99% |
|---|---|---|---|---|
| 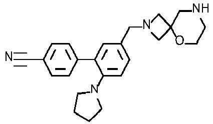 | 1H NMR (500 MHz, DMSO-d6) δ ppm 9.89 - 11.02 (m, 1 H) 8.75 - 9.65 (m, 2 H) 7.82 - 7.96 (m, 2 H) 7.52 - 7.60 (m, 2 H) 7.31 - 7.44 (m, 1 H) 7.19 - 7.31 (m, 1 H) 6.82 - 6.99 (m, 1 H) 4.30 - 4.35 (m, 2 H) 4.14 - 4.29 (m, 4 H) 3.79 - 3.87 (m, 2 H) 3.38 (brd, J=18.34 Hz, 2 H) 3.07 - 3.15 (m, 2 H) 2.79 - 2.91 (m, 4 H) 1.71 - 1.83 (m, 4 H). m/z: 389 [M+H]+ | | | |
| 5'-({5-oxa-2,8-diazaspiro[3.5]nonan-2-yl}methyl)-2'-(pyrrolidin-1-yl)-[1,1'-biphenyl]-4-carbonitrile | | | | |

| Example 292 | Procedure: A1.1+B1+C1 | SM1: 3-bromo-2-chlorobenzaldehyde | SM2: (4-cyano-3-fluorophenyl)boronic acid | Yield: 95% |
|---|---|---|---|---|
| 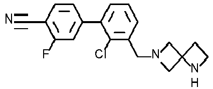 | 1H NMR (500 MHz, DMSO-d6) δ 0.00 (t, J = 7.6 Hz, 1H), 7.65 - 7.54 (m, 4H), 0.00 (dd, J = 8.1, 1.5 Hz, 1H), 4.77 - 4.65 (m, 4H), 4.63 - 4.52 (m, 2H), 0.00 (t, J = 8.3 Hz, 2H), 0.00 (t, J = 8.3 Hz, 2H). m/z: 342.2 [M+H]+ | | | |
| 2'-chloro-3'-({1,6-diazaspiro[3.3]heptan-6-yl}methyl)-3-fluoro-[1,1'-biphenyl]-4-carbonitrile | | | | |

| Example 240 | Procedure: A1.1+B1 | SM1: 3-bromo-2-chlorobenzaldehyde | SM2: (4-cyano-3-fluorophenyl)boronic acid | Yield: 86% |
|---|---|---|---|---|
| 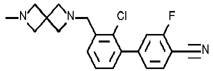 | 1H NMR (DMSO-d6, 600 MHz): δ (ppm) 8.02 (dd, J = 7.8, 7.1 Hz, 1H), 7.63 (dd, J = 10.3, 1.5 Hz, 1H), 7.50 (dd, J = 7.6, 1.6 Hz, 1H), 7.41-7.47 (m, 2H), 7.33 (dd, J = 7.6, 1.7 Hz, 1H), 3.89 (s, 1H), 3.67 (s, 2H), 3.65 (br s, 4H), 3.36 (s, 5H), 2.44 (s, 3H). m/z: 356 [M+H]+ | | | |
| 2'-chloro-3-fluoro-3'-({6-methyl-2,6-diazaspiro[3.3]heptan-2-yl}methyl)-[1,1'-biphenyl]-4-carbonitrile | | | | |

| Example 342 | Procedure: A1.1+B1+C1 | SM1: 3-bromo-2-chloro-6-fluorobenzaldehyde | SM2: (4-cyano-3-fluorophenyl)boronic acid | Yield: 79% |
|---|---|---|---|---|
| 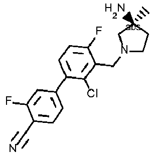 | 1H NMR (500 MHz, DMSO-d6) δ 8.04 (t, J = 7.6 Hz, 1H), 7.64 (dd, J = 10.4, 1.3 Hz, 1H), 7.52 - 7.45 (m, 2H), 7.42 - 7.33 (m, 1H), 7.01 - 4.75 (m, 2H), 3.84 (t, J = 2.6 Hz, 2H), 3.73 (s, 1H), 3.62 - 2.91 (m, 2H), 2.82 - 2.73 (m, 1H), 2.67 - 2.56 (m, 2H), 2.47 (d, J = 9.3 Hz, 1H), 1.72 (t, J = 7.1 Hz, 2H), 1.23 (s, 4H). m/z: 362.2 [M+H]+ | | | |
| 3'-{[(3S)-3-amino-3-methylpyrrolidin-1-yl]methyl}-2'-chloro-3,4'-difluoro-[1,1'-biphenyl]-4-carbonitrile | | | | |

| Example 291 | Procedure: A1.1+B1+C1 | SM1: 3-bromo-2-chloro-6-fluorobenzaldehyde | SM2: (4-cyano-3-fluorophenyl)boronic acid | Yield: 67% |
|---|---|---|---|---|
| 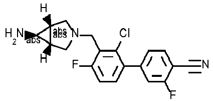 | 1H NMR (500 MHz, DMSO-d6) +0.3 mol of tartrate :δ 8.02-8.09 (m, 1H), 7.63-7.71 (m, 1H), 7.44-7.50 (m, 2H), 7.35-7.40 (m, 1H), 6.35-8.5 (m, 3H), 3.82-3.83 (s, 0.6H), 3.8 (br s, 2H), 2.90 (d, J=8.80 Hz, 2H), 2.51-2.55 (m, 2H), 2.45-2.48 (m, 1H), 1.58 (s, 2H). m/z: 360 [M+H]+ | | | |
| 3'-{[(1R,5S,6S)-6-amino-3-azabicyclo[3.1.0]hexan-3-yl]methyl}-2'-chloro-3,4'-difluoro-[1,1'-biphenyl]-4-carbonitrile | | | | |

| Example 341 | Procedure: A1.1+B1+C1 | SM1: 3-bromo-2-chlorobenzaldehyde | SM2: (4-cyano-3-fluorophenyl)boronic acid | Yield: 82% |
|---|---|---|---|---|
| 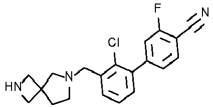 | 1H NMR (500 MHz, DMSO-d6) δ 10.56 - 9.82 (m, 1H), 9.07 - 8.41 (m, 2H), 0.00 (t, J = 7.6 Hz, 1H), 7.82 - 7.70 (m, 1H), 7.69 - 7.64 (m, 1H), 7.63 - 7.53 (m, 2H), 7.49 (dd, J = 8.1, 1.5 Hz, 1H), 4.85 - 4.41 (m, 2H), 4.19 - 3.96 (m, 4H), 3.95 - 3.79 (m, 4H), 2.49 - 2.12 (m, 2H). m/z: 356 [M+H]+ | | | |
| 2'-chloro-3'-({2,6-diazaspiro[3.4]octan-6-yl}methyl)-3-fluoro-[1,1'-biphenyl]-4-carbonitrile | | | | |

| Example 340 | Procedure: A1.1+B1+C1 | SM1: 3-bromo-2-chlorobenzaldehyde | SM2: (4-cyano-3-fluorophenyl)boronic acid | Yield: 73% |
|---|---|---|---|---|
| 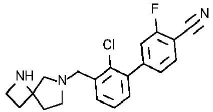 | 1H NMR (600 MHz, DMSO-d6) δ ppm 8.06 (t, J=7.41 Hz, 1 H) 7.76 (dd, J=7.19, 1.91 Hz, 1 H) 7.62 - 7.66 (m, 1 H) 7.60 (d, J=7.34 Hz, 1 H) 7.59 (d, J=2.20 Hz, 1 H) 7.44 - 7.51 (m, 1 H) 4.59 - 4.77 (m, 2 H) 3.70 - 4.34 (m, 4 H) 3.30 - 3.69 (m, 2 H) 2.61 (s, 4 H). m/z: 356 [M+H]+ | | | |
| 2'-chloro-3'-({1,6-diazaspiro[3.4]octan-6-yl}methyl)-3-fluoro-[1,1'-biphenyl]-4-carbonitrile | | | | |

| Example 208 | Procedure: A1.1+B1+C1 | SM1: 3-bromo-2-chloro-6-fluorobenzaldehyde | SM2: (4-cyano-3-fluorophenyl)boronic acid | Yield: 72% |
|---|---|---|---|---|
| 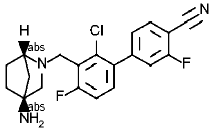 | 1H NMR (600 MHz, DMSO-d6) δ 8.5-5.0 (br s, 3H), 8.02 (t, J=7.47 Hz, 1H), 7.64 (dd, J=1.39, 10.34 Hz, 1H), 7.42-7.48 (m, 3H), 7.35 (t, J=8.88 Hz, 1H), 3.75-3.81 (m, 3H), 3.13 (s, 2H), 2.79 (br d, J=6.90 Hz, 1H), 2.44 (d, J=8.07 Hz, 1H), 1.90-1.95 (m, 1H), 1.60-1.66 (m, 4H), 1.48-1.54 (m, 1H), 1.44 (d, J=8.80 Hz, 1H). m/z: 374 [M+H]+ | | | |
| 3'-{[(1S,4S)-4-amino-2-azabicyclo[2.2.1]heptan-2-yl]methyl}-2'-chloro-3,4'-difluoro-[1 ,1'-biphenyl]-4-carbonitrile | | | | |

| Example 339 | Procedure: A1.1+B1+C1 | SM1: 3-bromo-2-chlorobenzaldehyde | SM2: (4-cyano-3-fluorophenyl)boronic acid | Yield: 90% |
|---|---|---|---|---|
| 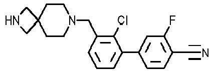 | 1H NMR (500 MHz, DMSO-d6) δ 9.77 - 9.49 (m, 1H), 8.73 (br s, 2H), 8.07 (t, J = 7.5 Hz, 1H), 7.76 (br s, 1H), 7.68 (d, J = 10.3 Hz, 1H), 7.59 (br s, 2H), 7.50 (dd, J = 8.1, 1.0 Hz, 1H), 4.50 (br s, 2H), 3.97 - 3.04 (m, 8H), 2.26 - 1.79 (m, 4H). m/z: 370.2 [M+H]+ | | | |
| 2'-chloro-3'-({2,7-diazaspiro[3.5]nonan-7-yl}methyl)-3-fluoro-[1,1'-biphenyl]-4-carbonitrile | | | | |

| Example 338 | Procedure: A1.1+B1+C1 | SM1: 3-bromo-2-chlorobenzaldehyde | SM2: (4-cyano-3-fluorophenyl)boronic acid | Yield: 76% |
|---|---|---|---|---|
| 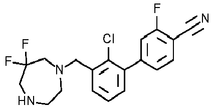 | 1H NMR (500 MHz, DMSO-d6) δ ppm 7.97 - 8.11 (m, 1 H) 7.61 - 7.70 (m, 2 H) 7.47 (s, 2 H) 7.30 - 7.39 (m, 1 H) 5.25 - 6.63 (m, 2 H) 3.89 (s, 2 H) 3.15 (br d, J=6.11 Hz, 4 H) 2.82 - 2.89 (m, 2 H) 2.69 - 2.75 (m, 2 H). m/z: 380.2 [M+H]+ | | | |
| 2'-chloro-3'-[(6,6-difluoro-1,4-diazepan-1-yl)methyl]-3-fluoro-[1,1'-biphenyl]-4-carbonitrile | | | | |

| Example 290 | Procedure: A1.1+B1 | SM1: 3-bromo-2,6-difluorobenzaldehyde | SM2: (4-cyano-3-fluorophenyl)boronic acid | Yield: 75% |
|---|---|---|---|---|
| 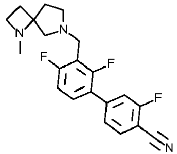 | 1H NMR (600 MHz, DMSO-d6) +0.5 eq of tartrate: δ 8.02-8.06 (m, 1H), 7.72-7.77 (m, 1H), 7.62-7.70 (m, 1H), 7.57-7.62 (m, 1H), 7.28 (t, J=8.66 Hz, 1H), 3.83-3.90 (m, 1H), 3.70-3.78 (m, 2H), 3.18-3.32 (br s, 2H), 3.08 (br s, 1H), 2.58-2.76 (m, 1H), 2.37-2.46 (m, 2H), 2.30 (br s, 3H), 2.10-2.25 (m, 3H), 1.81-1.93 (m, 1H). m/z: 372.1 [M+H]+ | | | |
| 2',3,4'-trifluoro-3'-({1-methyl-1,6-diazaspiro[3.4]octan-6-yl}methyl)-[1,1'-biphenyl]-4-carbonitrile | | | | |

| Example 289 | Procedure: A1.1+B1 | SM1: 3-bromo-2-chloro-6-fluorobenzaldehyde | SM2: (4-cyano-3-fluorophenyl)boronic acid | Yield: 71% |
|---|---|---|---|---|
| 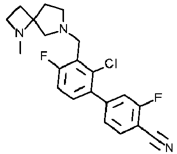 | 1H NMR (DMSO-d6, 600 MHz): δ (ppm) 7.98-8.08 (m, 1H), 7.64 (dd, J = 10.3, 1.5 Hz, 1H), 7.43-7.50 (m, 2H), 7.35-7.42 (m, 1H), 3.86 (s, 1H), 3.75-3.83 (m, 2H), 2.88-3.65 (m, 3H), 2.69 (td, J = 8.4, 4.8 Hz, 1H), 2.39-2.49 (m, 3H), 2.31 (s, 3H), 2.09-2.26 (m, 3H), 1.78-1.98 (m, 1H). m/z: 388 [M+H]+ | | | |
| 2'-chloro-3,4'-difluoro-3'-({1-methyl-1,6-diazaspiro[3.4]octan-6-yl}methyl)-[1,1'-biphenyl]-4-carbonitrile | | | | |

| Example 337 | Procedure: A1.1+B1+C1 | SM1: 3-bromo-2-chlorobenzaldehyde | SM2: (4-cyano-3-fluorophenyl)boronic acid | Yield: 62% |
|---|---|---|---|---|
| 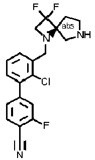 | 1H NMR (500 MHz, DMSO-d6) +-0.5 eq of tartrate: δ 8.03 (t, J=7.58 Hz, 1H), 7.61-7.67 (m, 1H), 7.54-7.58 (m, 1H), 7.44-7.48 (m, 2H), 7.38 (dd, J=1.47, 7.58 Hz, 1H), 3.97 (brd, J=14.18 Hz, 1H), 3.91 (s, 1H), 3.85 (brd, J=14.18 Hz, 1H), 3.55-3.66 (m, 2H), 3.40-3.45 (m, 1H), 3.10-3.22 (m, 2H), 2.99-3.09 (m, 1H), 2.18-2.29 (m, 2H). m/z: 392.2 [M+H]+ | | | |
| 2'-chloro-3'-{[(4S)-3,3-difluoro-1,6-diazaspiro[3.4]octan-1-yl]methyl}-3-fluoro-[1,1'-biphenyl]-4-carbonitrile | | | | |

| Example 239 | Procedure: P1+H1.1+E4+A2 .2 | SM1: 2-bromo-3-chloro-6-fluorobenzoic acid | SM2: 1-methyl-1 ,6-diazaspiro[3.4]octane | Yield: 78% |
|---|---|---|---|---|
| 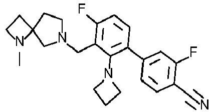 | 1H NMR (DMSO-d6, 500 MHz) δ 7.90 (t, 1H, J=7.6 Hz), 7.43 (dd, 1H, J=1.3, 10.6 Hz), 7.29 (dd, 1H, J=1.5, 8.1 Hz), 7.02 (dd, 1H, J=6.7, 8.4 Hz), 6.62 (t, 1H, J=9.0 Hz), 3.93 (s, 2H), 2.8-3.9 (m, 11H), 2.7-2.8 (m, 1H), 2.46 (br s, 3H), 2.2-2.4 (m, 5H), 1.9-2.0 (m, 3H). m/z: 409 [M+H]+ | | | |
| 2'-(azetidin-1-yl)-3,4'-difluoro-3'-({1-methyl-1,6-diazaspiro[3.4]octan-6-yl}methyl)-[1,1'-biphenyl]-4-carbonitrile | | | | |

| Example 288 | Procedure: A1.1+B1+C1 | SM1: 3-bromo-2-chlorobenzaldehyde | SM2: (4-cyano-3-fluorophenyl)boronic acid | Yield: 99% |
|---|---|---|---|---|
| 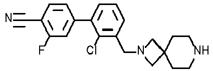 | 1H NMR (500 MHz, DMSO-d6) δ 10.50 (br s, 1H), 8.49 (br s, 2H), 8.08 (t, J = 7.5 Hz, 1H), 7.68 - 7.56 (m, 4H), 7.48 (dd, J = 8.1, 1.5 Hz, 1H), 4.68 (br s, 2H), 4.12 (br s, 4H), 3.19 - 2.90 (m, 4H), 1.99 (br s, 4H). m/z: 370 [M+H]+ | | | |
| 2'-chloro-3'-({2,7-diazaspiro[3.5]nonan-2-yl}methyl)-3-fluoro-[1,1'-biphenyl]-4-carbonitrile | | | | |

| Example 196 | Procedure: A1.1+B1+C1 | SM1: 3-bromo-2-chlorobenzaldehyde | SM2: (4-cyano-3-fluorophenyl)boronic acid | Yield: 82% |
|---|---|---|---|---|
| 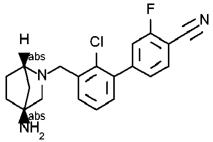 | 1H NMR (DMSO-d6, 600 MHz): δ (ppm) 8.02 (t, J = 7.3 Hz, 1H), 7.63 (dd, J = 10.3, 1.4 Hz, 1H), 7.58 (d, J = 6.2 Hz, 1H), 7.44-7.47 (m, 2H), 7.34 (dd, J = 7.6, 1.6 Hz, 1H), 3.80-3.81 (m, 2H), 3.75-3.83 (m, 2H), 3.36-3.43 (m, 6H), 3.23 (br s, 1H), 2.86 (br d, J = 5.9 Hz, 1H), 2.37 (d, J = 8.4 Hz, 1H), 1.94 (br d, J = 10.4 Hz, 1H), 1.80-1.85 (m, 1H), 1.69 (br d, J = 7.8 Hz, 2H), 1.54-1.60 (m, 2H). m/z: 356 [M+H]+ | | | |
| 3'-{[(1S,4S)-4-amino-2-azabicyclo[2.2.1]heptan-2-yl]methyl}-2'-chloro-3-fluoro-[1 ,1'-biphenyl]-4-carbonitrile | | | | |

| Example 125 | Procedure: H1.2+A1.1+A2.2 +C2 | SM1: 2-bromo-1-chloro-3-iodobenzene | SM2: tert-butyl N-methyl-N-(piperidin-4-yl)carbamate | Yield: 85% |
|---|---|---|---|---|
| 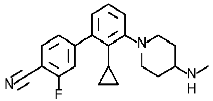 | 1H NMR (600 MHz, DMSO-d6) δ 9.04 - 8.63 (m, 2H), 7.96 (t, J = 7.6 Hz, 1H), 7.67 - 7.59 (m, 1H), 0.00 (dd, J = 8.2, 1.3 Hz, 1H), 0.00 (t, J = 7.8 Hz, 1H), 7.08 - 7.01 (m, 1H), 6.99 - 6.88 (m, 1H), 3.50 - 3.41 (m, 2H), 3.16 - 3.03 (m, 1H), 2.82 - 2.69 (m, 2H), 2.59 (t, J = 5.4 Hz, 3H), 2.21 - 2.05 (m, 2H), 2.01 - 1.92 (m, 1H), 1.84 - 1.72 (m, 2H), 0.79 - -0.12 (m, 4H). m/z: 350 [M+H]+ | | | |
| 2'-cyclopropyl-3-fluoro-3'-[4-(methylamino)piperidin-1-yl]-[1,1'-biphenyl]-4-carbonitrile | | | | |

| Example 336 | Procedure: B1+K1+A2.2+C1 | SM1: 2-bromo-3-chlorobenzaldehyde | SM2: tert-butyl N-methyl-N-(pyrrolidin-3-yl)carbamate | Yield: 79% |
|---|---|---|---|---|
| 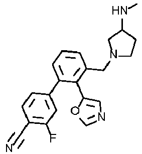 | 1H NMR (DMSO-d6, 600 MHz): δ (ppm) 8.29 (s, 1H), 7.85 (t, J = 7.6 Hz, 1H), 7.70 (d, J = 7.6 Hz, 1H), 7.62 (t, J = 7.7 Hz, 1H), 7.45 (d, J = 7.5 Hz, 1H), 7.32 (dd, J = 10.5, 1.2 Hz, 1H), 7.20 (s, 1H), 7.11 (dd, J = 8.0, 1.2 Hz, 1H), 6.19-9.16 (m, 2H), 3.78 (s, 2H), 3.52 (s, 2H), 3.44 (br s, 2H), 3.24-3.36 (m, 3H), 2.57-2.68 (m, 2H), 2.41 (s, 3H), 2.32-2.38 (m, 1H), 2.00-2.07 (m, 1H), 1.52-1.71 (m, 1H). m/z: 377 [M+H]+ | | | |
| 3-fluoro-3'-{[3-(methylamino)pyrrolidin-1-yl]methyl}-2'-(1,3-oxazol-5-yl)-[1,1'-biphenyl]-4-carbonitrile | | | | |

| Example 95 | Procedure: H1.1+K1+A2.2 | SM1: 2-bromo-1-chloro-3-iodobenzene | SM2: N,N-dimethylpiperidin-4-amine | Yield: 27% |
|---|---|---|---|---|
| 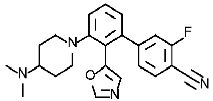 | 1H NMR (DMSO-d6, 500 MHz) δ 8.26 (s, 1H), 7.8-7.9 (m, 1H), 7.53 (t, 1H, J=7.9 Hz), 7.30 (dt, 2H, J=1.3, 5.3 Hz), 7.18 (s, 1H), 7.14 (dd, 1H, J=0.7, 7.6 Hz), 7.09 (dd, 1H, J=1.5, 8.1 Hz), 3.87 (s, 1H), 3.1-3.7 (m, 2H), 3.03 (br d, 2H, J=12.0 Hz), 2.61 (br t, 2H, J=11.5 Hz), 2.32 (br s, 7H), 1.79 (br d, 2H, J=12.0 Hz), 1.34 (br dd, 2H, J=2.8, 11.6 Hz). m/z: 391 [M+H]+ | | | |
| 3'-[4-(dimethylamino)piperidin-1-yl]-3-fluoro-2'-(1,3-oxazol-5-yl)-[1,1'-biphenyl]-4-carbonitrile | | | | |

| Example 335 | Procedure: K1+A2.2+B1+C1 | SM1: 2-bromo-3-chlorobenzaldehyde | SM2: 1,3-oxazole | Yield: 44% |
|---|---|---|---|---|
| 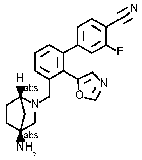 | 1H NMR (DMSO-d6, 600 MHz): δ (ppm) 8.29 (s, 1H), 7.84 (t, J = 7.5 Hz, 1H), 7.63-7.67 (m, 1H), 7.57-7.62 (m, 1H), 7.42 (d, J = 7.7 Hz, 1H), 7.32 (dd, J = 10.5, 1.4 Hz, 1H), 7.13 (s, 1H), 7.11 (dd, J = 8.1, 1.6 Hz, 1H), 5.04-8.13 (m, 3H), 3.78 (s, 2H), 3.50-3.63 (m, 2H), 3.09-3.70 (m, 3H), 3.00 (s, 1H), 2.66 (br d, J = 5.7 Hz, 1H), 2.20 (d, J = 8.2 Hz, 1H), 1.71-1.76 (m, 1H), 1.52-1.64 (m, 3H), 1.39-1.45 (m, 2H). m/z: 389.3 [M+H]+ | | | |
| 3'-{[(1S,4S)-4-amino-2-azabicyclo[2.2.1]heptan-2-yl]methyl}-3-fluoro-2'-(1,3-oxazol-5-yl)-[1,1'-biphenyl]-4-carbonitrile | | | | |

| Example 334 | Procedure: K2+A2.2+B1+C1 | SM1: 2-bromo-3-chloro-6-fluorobenzaldehyde | SM2: 1,3-oxazole | Yield: 64% |
|---|---|---|---|---|
| 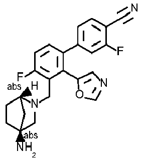 | 1H NMR (DMSO-d6, 500 MHz): +-0.6 eq of tartrate: δ (ppm) 8.26-8.32 (m, 1H), 7.80-7.92 (m, 1H), 7.45-7.55 (m, 2H), 7.31-7.37 (m, 1H), 7.23-7.29 (m, 1H), 7.08-7.14 (m, 1H), 3.75-3.82 (m, 1H), 3.54-3.63 (m, 2H), 2.87-2.93 (m, 1H), 2.65-2.72 (m, 1H), 2.23-2.30 (m, 1H), 1.68-1.79 (m, 1H), 1.48-1.66 (m, 3H), 1.37-1.48 (m, 2H). m/z: 407 [M+H]+ | | | |
| 3'-{[(1S,4S)-4-amino-2-azabicyclo[2.2.1]heptan-2-yl]methyl}-3,4'-difluoro-2'-(1,3-oxazol-5-yl)-[1,1'-biphenyl]-4-carbonitrile | | | | |

| Example 333 | Procedure: B1+A3+A1.2+C1 | SM1: 2-bromo-3-chlorobenzaldehyde | SM2: tert-butyl 2,6-diazaspiro[3.5]nonane -6-carboxylate | Yield: 24% |
|---|---|---|---|---|
| 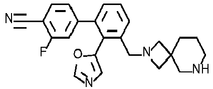 | 1H NMR (DMSO-d6, 500 MHz) +0.5 eq of tartrate: δ (ppm) 8.24-8.35 (m, 1H), 7.78-7.88 (m, 1H), 7.55-7.67 (m, 2H), 7.40-7.47 (m, 1H), 7.32 (dd, J = 10.5, 1.5 Hz, 1H), 7.16-7.22 (m, 1H), 7.05-7.14 (m, 1H), 3.70-3.77 (m, 1H), 3.51 (s, 2H), 2.97-3.03 (m, 3H), 2.87-2.92 (m, 2H), 2.77-2.83 (m, 2H), 2.65-2.74 (m, 2H), 1.56-1.68 (m, 2H), 1.36-1.48 (m, 2H). m/z: 403 [M+H]+ | | | |
| 3'-({2,6-diazaspiro[3.5]nonan-2-yl}methyl)-3-fluoro-2'-(1,3-oxazol-5-yl)-[1,1'-biphenyl]-4-carbonitrile | | | | |

| Example 332 | Procedure: A1.1+B1+C2 | SM1: 3-bromo-2-chlorobenzaldehyde | SM2: (4-cyano-3-fluorophenyl)boronic acid | Yield: 71% |
|---|---|---|---|---|
| 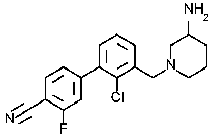 | 1H NMR (DMSO-d6, 500 MHz): +TFA: δ (ppm) 8.05 (t, J = 7.5 Hz, 1H), 7.90 (dd, J = 6.7, 2.6 Hz, 1H), 7.65 (dd, J = 10.0, 1.2 Hz, 1H), 7.57-7.63 (m, 2H), 7.50 (dd, J = 8.1, 1.5 Hz, 1H), 4.60-4.69 (m, 2H), 2.88-3.84 (m, 5H), 1.40-2.16 (m, 4H). m/z: 344 [M+H]+ | | | |
| 3'-[(3-aminopiperidin-1-yl)methyl]-2'-chloro-3-fluoro-[1,1'-biphenyl]-4-carbonitrile | | | | |

| Example 287 | Procedure: A1.1+B1+C2 | SM1: 3-bromo-2-chlorobenzaldehyde | SM2: (4-cyano-3-fluorophenyl)boronic acid | Yield: 66% |
|---|---|---|---|---|
| 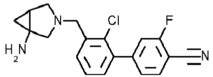 | 1H NMR (500 MHz, DMSO-d6) δ 12.32 - 11.06 (m, 1H), 9.42 - 8.38 (m, 3H), 8.06 (t, J = 7.5 Hz, 1H), 7.91 (d, J = 1.0 Hz, 1H), 7.66 (dd, J = 10.3, 1.5 Hz, 1H), 7.62 - 7.53 (m, 2H), 7.49 (dd, J = 8.1, 1.5 Hz, 1H), 4.74 - 4.53 (m, 2H), 4.01 - 3.38 (m, 4H), 2.27 - 2.07 (m, 1H), 1.68 - 1.25 (m, 2H). m/z: 341.9 [M+H]+ | | | |
| 3'-({1-amino-3-azabicyclo[3.1.0]hexan-3-yl}methyl)-2'-chloro-3-fluoro-[1,1'-biphenyl]-4-carbonitrile | | | | |

| Example 331 | Procedure: A1.1+B1+C1 | SM1: 3-bromo-2-chlorobenzaldehyde | SM2: (4-cyano-3-fluorophenyl)boronic acid | Yield: 77% |
|---|---|---|---|---|
| 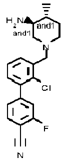 | 1H NMR (600 MHz, DMSO-d6) δ ppm 8.03 (t, J=7.48 Hz, 1 H) 7.61 - 7.65 (m, 1 H) 7.58 - 7.61 (m, 1 H) 7.41 - 7.50 (m, 2 H) 7.33 - 7.39 (m, 1 H) 5.25 - 7.17 (m, 3 H) 3.64 (d, J=17.02 Hz, 2 H) 2.91 - 3.01 (m, 1 H) 2.74 - 2.82 (m, 1 H) 2.58 - 2.68 (m, 1 H) 2.07 - 2.16 (m, 1 H) 1.88 - 1.96 (m, 1 H) 1.62 - 1.74 (m, 1 H) 1.30 - 1.40 (m, 1 H) 1.22 - 1.29 (m, 1 H) 0.95 - 1.02 (m, 3 H). m/z: 358.2 [M+H]+ | | | |
| 3'-{[(3S,4R)-3-amino-4-methylpiperidin-1-yl]methyl}-2'-chloro-3-fluoro-[1,1'-biphenyl]-4-carbonitrile | | | | |

| Example 330 | Procedure: A1.1+B1+C2 | SM1: 3-bromo-2-chlorobenzaldehyde | SM2: (4-cyano-3-fluorophenyl)boronic acid | Yield: 59% |
|---|---|---|---|---|
| 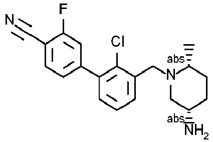 | 1H NMR (600 MHz, DMSO-d6) δ 11.59 - 10.50 (m, 1H), 8.77 - 8.23 (m, 3H), 8.12 - 8.06 (m, J = 6.6 Hz, 1H), 8.04 - 7.81 (m, 1H), 7.79 - 7.67 (m, J = 18.3, 10.1 Hz, 1H), 7.61 - 7.51 (m, 3H), 5.13 - 4.26 (m, 2H), 3.93 - 3.66 (m, 2H), 3.42 - 3.26 (m, 2H), 2.40 - 1.75 (m, 4H), 1.62 - 1.25 (m, 3H). m/z: 358 [M+H]+ | | | |
| 3'-{[(2R,5S)-5-amino-2-methylpiperidin-1-yl]methyl}-2'-chloro-3-fluoro-[1,1'-biphenyl]-4-carbonitrile | | | | |

| Example 329 | Procedure: A1.1+E1+F2+D3 +C2 | SM1: 3-bromo-2-chlorobenzaldehyde | SM2: (4-cyano-3-fluorophenyl)boronic acid | Yield: 78% |
|---|---|---|---|---|
| 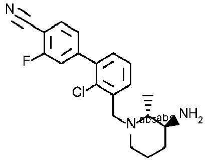 | 1H NMR (500 MHz, DMSO-d6) δ 7.97 (t, J = 7.5 Hz, 1H), 7.80 (s, 1H), 7.63 - 7.57 (m, 1H), 7.56 - 7.51 (m, 2H), 7.50 - 7.42 (m, 1H), 5.20 - 4.29 (m, 2H), 3.59 - 3.46 (m, 1H), 3.28 - 2.94 (m, 2H), 2.18 - 2.07 (m, 1H), 1.87 (s, 7H). m/z: 358 [M+H]+ | | | |
| 3'-{[(2R,3S)-3-amino-2-methylpiperidin-1-yl]methyl}-2'-chloro-3-fluoro-[1,1'-biphenyl]-4-carbonitrile | | | | |

| Example 286 | Procedure: A1.1+B1+C2 | SM1: 3-bromo-2-chlorobenzaldehyde | SM2: (4-cyano-3-fluorophenyl)boronic acid | Yield: 66% |
|---|---|---|---|---|
| 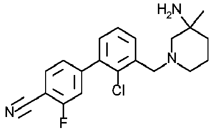 | 1H NMR (DMSO-d6, 600 MHz): δ (ppm) 10.99-11.45 (m, 1H), 8.47-9.01 (m, 2H), 8.02-8.10 (m, 1H), 7.89-8.02 (m, 1H), 7.30-7.86 (m, 5H), 4.40-4.76 (m, 2H), 2.53-3.76 (m, 4H), 1.65-2.45 (m, 4H), 1.27-1.61 (m, 3H). m/z: 358 [M+H]+ | | | |
| 3'-[(3-amino-3-methylpiperidin-1-yl)methyl]-2'-chloro-3-fluoro-[1,1'-biphenyl]-4-carbonitrile | | | | |

| Example 285 | Procedure: A1.1+B1+C2 | SM1: 3-bromo-2-chlorobenzaldehyde | SM2: (4-cyano-3-fluorophenyl)boronic acid | Yield: 97% |
|---|---|---|---|---|
| 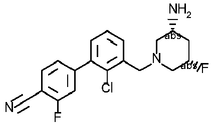 | 1H NMR (600 MHz, DMSO-d6) δ 8.57 - 8.25 (m, 1H), 8.04 (dd, J = 7.8, 7.2 Hz, 1H), 7.88 (br d, J = 7.2 Hz, 1H), 7.67 (d, J = 10.3 Hz, 1H), 7.54 (t, J = 7.6 Hz, 1H), 7.51 - 7.46 (m, 2H), 5.22 - 4.90 (m, 1H), 4.58 - 4.01 (m, 2H), 3.78 - 3.49 (m, 1H), 3.36 - 3.21 (m, 2H), 3.20 - 3.09 (m, 1H), 3.08 - 2.83 (m, 1H), 2.42 - 2.19 (m, 1H), 2.06 - 1.91 (m, 1H). m/z: 362 [M+H]+ | | | |
| 3'-{[(3R,5S)-3-amino-5-fluoropiperidin-1-yl]methyl}-2'-chloro-3-fluoro-[1,1'-biphenyl]-4-carbonitrile | | | | |

| Example 238 | Procedure: A1.1+B1+C2 | SM1: 3-bromo-2-chlorobenzaldehyde | SM2: (4-cyano-3-fluorophenyl)boronic acid | Yield: 62% |
|---|---|---|---|---|
| 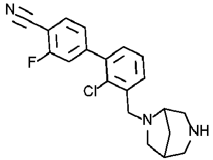 | 1H NMR (DMSO-d6, 600 MHz): δ (ppm) 9.97-11.54 (m, 1H), 8.29-9.01 (m, 1H), 7.89-8.20 (m, 3H), 7.69 (br d, J = 10.0 Hz, 1H), 7.36-7.61 (m, 3H), 3.76-5.32 (m, 4H), 2.64-3.52 (m, 6H), 1.74-2.44 (m, 2H). m/z: 356 [M+H]+ | | | |
| 2'-chloro-3'-({3,6-diazabicyclo[3.2.1]octan-6-yl}methyl)-3-fluoro-[1,1'-biphenyl]-4-carbonitrile | | | | |

| Example 284 | Procedure: A1.1+B1+C2 | SM1: 3-bromo-2-chlorobenzaldehyde | SM2: (4-cyano-3-fluorophenyl)boronic acid | Yield: 96% |
|---|---|---|---|---|
| 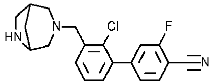 | 1H NMR (600 MHz, DMSO-d6) δ 9.86 - 9.41 (m, 1H), 9.08 - 8.72 (m, 1H), 8.03 (t, J = 7.5 Hz, 1H), 7.96 - 7.82 (m, 1H), 0.00 (d, J = 10.3 Hz, 1H), 7.54 - 7.50 (m, 1H), 7.50 - 7.48 (m, 1H), 7.48 - 7.44 (m, 1H), 4.55 - 4.14 (m, 2H), 4.11 - 3.98 (m, 1H), 3.63 - 3.49 (m, 1H), 3.48 - 3.23 (m, 2H), 3.22 - 3.17 (m, 1H), 3.17 - 2.79 (m, 2H), 2.77 - 2.64 (m, 1H), 2.05 - 1.91 (m, 1H), 1.86 - 1.77 (m, 1H). m/z: 356 [M+H]+ | | | |
| 2'-chloro-3'-({3,6-diazabicyclo[3.2.1]octan-3-yl}methyl)-3-fluoro-[1,1'-biphenyl]-4-carbonitrile | | | | |

| Example 283 | Procedure: A1.1+B1+C2 | SM1: 3-bromo-2-chlorobenzaldehyde | SM2: (4-cyano-3-fluorophenyl)boronic acid | Yield: 100% |
|---|---|---|---|---|
| 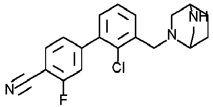 | 1H NMR (DMSO-d6, 600 MHz) +TFA: δ (ppm) 8.03 (t, J = 7.5 Hz, 1H), 7.96 (dd, J = 7.4, 1.7 Hz, 1H), 7.65 (d, J = 10.1 Hz, 1H), 7.55-7.60 (m, 2H), 7.49 (dd, J = 8.0, 1.4 Hz, 1H), 4.74 (q, J = 13.9 Hz, 2H), 3.78-3.92 (m, 3H), 3.60-3.74 (m, 2H), 3.40-3.50 (m, 1H), 1.70-2.42 (m, 4H). m/z: 355.9 [M+H]+ | | | |
| 2'-chloro-3'-({2,5-diazabicyclo[2.2.2]octan-2-yl}methyl)-3-fluoro-[1,1'-biphenyl]-4-carbonitrile | | | | |

| Example 282 | Procedure: A1.1+B1+C2 | SM1: 3-bromo-2-chlorobenzaldehyde | SM2: (4-cyano-3-fluorophenyl)boronic acid | Yield: 62% |
|---|---|---|---|---|
| 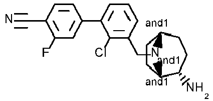 | 1H NMR (600 MHz, DMSO-d6, 300K) δ ppm 10.85 - 11.35 (m, 1 H), 7.00 - 8.78 (m, 9 H), 4.35 - 4.62 (m, 2 H), 3.96 - 4.21 (m, 3 H), 3.57 (s, 1 H), 3.12 - 3.31 (m, 1 H), 2.23 - 2.48 (m, 2 H), 1.09 - 2.15 (m, 6 H). m/z: 370 [M+H]+ | | | |
| 3'-{[(1S,2S,5S)-2-amino-8-azabicyclo[3.2.1]octan-8-yl]methyl}-2'-chloro-3-fluoro-[1,1'-biphenyl]-4-carbonitrile | | | | |

| Example 328 | Procedure: A1.1+B1+C1 | SM1: 3-bromo-2-chlorobenzaldehyde | SM2: (4-cyano-3-fluorophenyl)boronic acid | Yield: 66% |
|---|---|---|---|---|
| 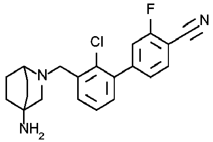 | 1H NMR (DMSO-d6, 500 MHz): δ (ppm) 7.98-8.06 (m, 1H), 7.58-7.67 (m, 2H), 7.42-7.49 (m, 2H), 7.33 (dd, J = 7.6, 1.5 Hz, 1H), 3.83-5.06 (m, 3H), 3.79 (s, 2H), 3.73 (s, 2H), 3.22-3.67 (m, 3H), 2.59 (br s, 1H), 2.52 (s, 2H), 1.89-2.02 (m, 2H), 1.43-1.60 (m, 6H). m/z: 370.1 [M+H]+ | | | |
| 3'-({4-amino-2-azabicyclo[2.2.2]octan-2-yl}methyl)-2'-chloro-3-fluoro-[1,1'-biphenyl]-4-carbonitrile | | | | |

| Example 176 | Procedure: A1.1+A2.2+E1+F 1+D3+C1 | SM1: 2-bromo-3-chlorobenzaldehyde | SM2: cyclopropylboronic acid | Yield: 89% |
|---|---|---|---|---|
| 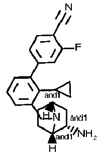 | 1H NMR (DMSO-d6, 500 MHz) +0.5 eq of tartrate: δ (ppm) 7.87-8.00 (m, 1H), 7.54-7.63 (m, 2H), 7.40-7.48 (m, 1H), 7.24-7.37 (m, 1H), 7.06-7.24 (m, 1H), 3.77-3.84 (m, 2H), 3.76 (s, 1H), 3.43-3.49 (m, 1H), 3.27-3.31 (m, 1H), 3.26 (t, J = 4.6 Hz, 1H), 2.01-2.17 (m, 2H), 1.70-1.96 (m, 3H), 1.41-1.52 (m, 1H), 0.91-1.03 (m, 1H), 0.61-0.76 (m, 2H), -0.09-0.06 (m, 2H). m/z: 362.2 [M+H]+ | | | |
| 3'-{[(1S,2S,4R)-2-amino-7-azabicyclo[2.2.1]heptan-7-yl]methyl}-2'-cyclopropyl-3-fluoro-[1,1'-biphenyl]-4-carbonitrile | | | | |

| Example 327 | Procedure: A1.1+B1+C2 | SM1: 3-bromo-2-chlorobenzaldehyde | SM2: (4-cyano-3-fluorophenyl)boronic acid | Yield: 65% |
|---|---|---|---|---|
| 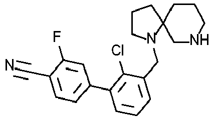 | 1H NMR (DMSO-d6, 600 MHz): δ (ppm) 10.63-11.49 (m, 1H), 9.97-10.56 (m, 1H), 8.31-9.64 (m, 1H), 8.11 (s, 1H), 7.77-7.91 (m, 1H), 7.62-7.77 (m, 1H), 7.29-7.62 (m, 3H), 4.80-5.09 (m, 1H), 4.33 (br d, J = 9.8 Hz, 1H), 3.64-4.00 (m, 1H), 2.64-3.49 (m, 5H), 1.47-2.43 (m, 8H). m/z: 384 [M+H]+ | | | |
| 2'-chloro-3'-({1,7-diazaspiro[4.5]decan-1-yl}methyl)-3-fluoro-[1,1'-biphenyl]-4-carbonitrile | | | | |

| Example 175 | Procedure: A1.1+A2.2+E1+F 1+D3+D1+C1 | SM1: 2-bromo-3-chlorobenzaldehyde | SM2: cyclopropylboronic acid | Yield: 81% |
|---|---|---|---|---|
| 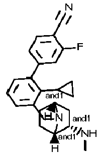 | 1H NMR (DMSO-d6, 600 MHz): δ (ppm) 7.92-8.01 (m, 1H), 7.59 (dd, J = 10.6, 1.4 Hz, 1H), 7.54-7.57 (m, 1H), 7.45 (dd, J = 8.0, 1.5 Hz, 1H), 7.33 (t, J = 7.6 Hz, 1H), 7.18 (dd, J = 7.6, 1.2 Hz, 1H), 6.68-9.31 (m, 2H), 3.98 (s, 2H), 3.77-3.87 (m, 2H), 3.47-3.51 (m, 1H), 3.43-3.47 (m, 1H), 3.34 (br t, J = 4.6 Hz, 2H), 3.05-3.28 (m, 2H), 2.53 (s, 3H), 2.10-2.20 (m, 1H), 2.03-2.10 (m, 1H), 1.81-2.03 (m, 2H), 1.68-1.77 (m, 1H), 1.43-1.57 (m, 1H), 1.12 (br dd, J = 12.6, 4.0 Hz, 1H), 0.72 (br dd, J = 8.4, 1.4 Hz, 2H), - 0.08-0.06 (m, 2H). m/z: 376.2 [M+H]+ | | | |
| 2'-cyclopropyl-3-fluoro-3'-{[(1S,2S,4R)-2-(methylamino)-7-azabicyclo[2.2.1]heptan-7-yl]methyl}-[1,1'-biphenyl]-4-carbonitrile | | | | |

| Example 326 | Procedure: A1.1+B1+C2 | SM1: 3-bromo-2-chlorobenzaldehyde | SM2: (4-cyano-3-fluorophenyl)boronic acid | Yield: 89% |
|---|---|---|---|---|
| 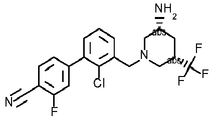 | 1H NMR (DMSO-d6, 500 MHz): δ (ppm) 8.23 (br s, 3H), 8.04 (t, J = 7.6 Hz, 1H), 7.65 (brdd, J = 10.3, 1.2 Hz, 2H), 7.46-7.53 (m, 2H), 7.42 (br d, J = 7.1 Hz, 1H), 3.70-3.77 (m, 2H), 3.26-3.38 (m, 1H), 3.17 (br d, J = 1.2 Hz, 2H), 2.81-2.99 (m, 1H), 1.99-2.44 (m, 3H), 1.36-1.50 (m, 1H). m/z: 412 [M+H]+ | | | |
| 3'-{[(3R,5S)-3-amino-5-(trifluoromethyl)piperidin-1-yl]methyl}-2'-chloro-3-fluoro-[1,1'-biphenyl]-4-carbonitrile | | | | |

| Example 281 | Procedure: A1.1+E1+F2+D3 +C2 | SM1: 3-bromo-2-chlorobenzaldehyde | SM2: (4-cyano-3-fluorophenyl)boronic acid | Yield: 80% |
|---|---|---|---|---|
| 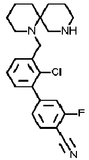 | 1H NMR (DMSO-d6, 500 MHz) at 373K: δ (ppm) 8.23-9.26 (m, 3H), 7.96 (dd, J = 7.9, 7.0 Hz, 1H), 7.85 (br d, J = 7.6 Hz, 1H), 7.55 (dd, J = 10.4, 1.3 Hz, 1H), 7.43-7.49 (m, 2H), 7.35 (br d, J = 7.8 Hz, 1H), 3.71-4.31 (m, 3H), 3.30-3.38 (m, 1H), 2.57-3.03 (m, 4H), 1.42-2.04 (m, 10H). m/z: 398 [M+H]+ | | | |
| 2'-chloro-3'-({1,8-diazaspiro[5.5]undecan-1-yl}methyl)-3-fluoro-[1,1'-biphenyl]-4-carbonitrile | | | | |

| Example 325 | Procedure: K1+A2.2+B1+C1 | SM1: 2-bromo-3-chlorobenzaldehyde | SM2: 1,3-oxazole | Yield: 61% |
|---|---|---|---|---|
| 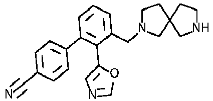 | 1H NMR (DMSO-d6, 600 MHz): δ (ppm) 8.26 (s, 1H), 7.77 (d, J = 8.5 Hz, 2H), 7.61-7.64 (m, 1H), 7.57-7.61 (m, 1H), 7.40 (dd, J = 7.4, 1.4 Hz, 1H), 7.28-7.34 (m, 2H), 7.19-9.20 (m, 2H), 7.08 (s, 1H), 3.77 (s, 2H), 3.47-3.55 (m, 2H), 3.17-3.72 (m, 3H), 2.98-3.12 (m, 2H), 2.85-2.97 (m, 2H), 2.51-2.57 (m, 1H), 2.46 (d, J = 8.9 Hz, 1H), 2.41-2.47 (m, 1H), 2.29 (d, J = 9.1 Hz, 1H), 1.69-1.87 (m, 4H). m/z: 385 [M+H]+ | | | |
| 3'-({2,7-diazaspiro[4.4]nonan-2-yl}methyl)-2'-(1,3-oxazol-5-yl)-[1,1'-biphenyl]-4-carbonitrile | | | | |

| Example 351 CPD0072945 | Procedure: K1+A2.2+B1+C1 | SM1: 2-bromo-3-chlorobenzaldehyde | SM2: 1,3-thiazole | Yield: 66% |
|---|---|---|---|---|
| 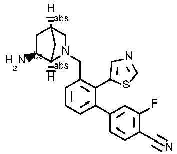 | 1H NMR (DMSO-d6, 600 MHz) δ 9.07 (d, 1H, J=0.7 Hz), 7.99 (d, 1H, J=7.8 Hz), 7.80 (t, 1H, J=7.6 Hz), 7.70 (d, 1H, J=0.6 Hz), 7.59 (t, 1H, J=7.8 Hz), 7.37 (d, 1H, J=7.6 Hz), 7.32 (d, 1H, J=10.4 Hz), 7.15 (dd, 1H, J=1.5, 8.0 Hz), 6.4-6.9 (m, 2H), 3.73 (s, 1H), 3.67 (s, 1H), 3.40 (br d, 1H, J=14.4 Hz), 3.31 (br d, 1H, J=9.7 Hz), 3.1-3.6 (m, 4H), 3.08 (br s, 1H), 2.73 (d, 1H, J=9.2 Hz), 2.32 (br s, 1H), 2.1-2.2 (m, 1H), 1.8-1.9 (m, 1H), 1.47 (brd, 1H, J=10.1 Hz), 1.31 (brd, 1H, J=10.1 Hz), 0.99 (brd, 1H, J=12.9 Hz), m/z: 405 [M+H]+ | | | |
| 3'-{[(1R,4R,6S)-6-amino-2-azabicyclo[2.2.1]heptan-2-yl]methyl}-3-fluoro-2'-(1 ,3-thiazol-5-yl)-[1 ,1'-biphenyl]-4-carbonitrile | | | | |

| Example 237 | Procedure: K1+A2.2+B1+C1 | SM1: 2-bromo-3-chlorobenzaldehyde | SM2: 1,3-thiazole | Yield: 66% |
|---|---|---|---|---|
| 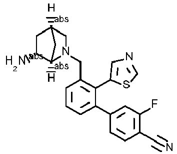 | 1H NMR (600 MHz, DMSO-d6) δ ppm 9.06 (d, J=0.59 Hz, 1 H) 7.75 - 7.84 (m, 1 H) 7.68 (d, J=0.73 Hz, 1 H) 7.65 (dd, J=7.70, 0.95 Hz, 1 H) 7.56 (t, J=7.70 Hz, 1 H) 7.37 (dd, J=7.78, 1.17 Hz, 1 H) 7.28 - 7.34 (m, 1 H) 7.14 (dd, J=8.00, 1.54 Hz, 1 H) 5.75 - 6.98 (m, 3 H) 3.50 (s, 2 H) 3.10 - 3.20 (m, 1 H) 2.94 (s, 1 H) 2.41 - 2.48 (m, 1 H) 2.28 - 2.36 (m, 1 H) 2.08 - 2.16 (m, 1 H) 1.64 - 1.75 (m, 1 H) 1.53 - 1.61 (m, 1 H) 1.36 - 1.44 (m, 1 H) 1.22 - 1.29 (m, 1 H). m/z: 405 [M+H]+ | | | |
| 3'-{[(1R,4R,6R)-6-amino-2-azabicyclo[2.2.1]heptan-2-yl]methyl}-3-fluoro-2'-(1,3-thiazol-5-yl)-[1,1'-biphenyl]-4-carbonitrile | | | | |

| Example 324 | Procedure: K1+A2.2+B1+C2 | SM1: 2-bromo-3-chlorobenzaldehyde | SM2: 1,3-thiazole | Yield: 96% |
|---|---|---|---|---|
| 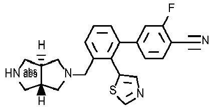 | 1H NMR (600 MHz, DMSO-d6, 300K) (2 conformers 50/50)δ ppm 12.26 (br s, 1 H), 9.79 (br d, J=2.6 Hz, 1 H), 9.60 (br s, 1 H), 9.14 (d, J=0.7 Hz, 1 H), 8.19 (d, J=7.9 Hz, 1 H), 7.93 (d, J=0.7 Hz, 1 H), 7.83 (dd, J=7.8, 7.1 Hz, 1 H), 7.71 (t, J=7.8 Hz, 1 H), 7.53 (dd, J=7.7, 1.0 Hz, 1 H), 7.35 (dd, J=10.3, 1.4 Hz, 1 H), 7.17 (dd, J=7.8, 1.4 Hz, 1 H), 4.43 - 4.54 (m, 1 H), 4.35 - 4.41 (m, 1 H), 3.65 (dt, J=10.3, 5.4 Hz, 1 H), 2.80 - 3.47 (m, 8 H), 2.54 - 2.68 (m, 1 H). m/z: 405.2 [M+H]+ | | | |
| 3'-{[(3aS,6aS)-octahydropyrrolo[3,4-c]pyrrol-2-yl]methyl}-3-fluoro-2'-(1,3-thiazol-5-yl)-[1,1'-biphenyl]-4-carbonitrile | | | | |

| Example 280 | Procedure: K1+A2.2+E1+F2 +D3+C1 | SM1: 2-bromo-3-chlorobenzaldehyde | SM2: 1,3-thiazole | Yield: 44% |
|---|---|---|---|---|
| 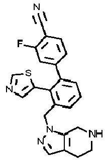 | 1H NMR (DMSO-d6, 500 MHz) δ 9.08 (s, 1H), 7.8-7.9 (m, 2H), 7.5-7.6 (m, 1H), 7.4-7.5 (m, 1H), 7.36 (dd, 1H, J=1.5, 10.5 Hz), 7.31 (s, 1H), 7.17 (dd, 1H, J=1.6, 7.9 Hz), 6.99 (d, 1H, J=7.8 Hz), 5.10 (s, 2H), 4.02 (s, 2H), 3.79 (br s, 2H), 3.2-3.6 (m, 5H), 3.0-3.1 (m, 2H), 2.5-2.6 (m, 2H). m/z: 416 [M+H]+ | | | |
| 3-fluoro-3'-({1H,4H,5H,6H,7H-pyrazolo[3,4-c]pyridin-1-yl}methyl)-2'-(1,3-thiazol-5-yl)-[1,1'-biphenyl]-4-carbonitrile | | | | |

| Example 279 | Procedure: K1+A2.2+B1+C1 | SM1: 2-bromo-3-chlorobenzaldehyde | SM2: 1,3-thiazole | Yield: 83% |
|---|---|---|---|---|
| 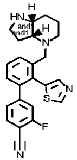 | 1H NMR (DMSO-d6, 600 MHz) δ 9.06 (d, 1H, J=0.6 Hz), 8.1-8.9 (m, 2H), 7.80 (dd, 1H, J=7.2, 7.9 Hz), 7.77 (d, 1H, J=0.6 Hz), 7.7-7.7 (m, 1H), 7.58 (t, 1H, J=7.7 Hz), 7.39 (dd, 1H, J=1.1, 7.7 Hz), 7.33 (dd, 1H, J=1.3, 10.4 Hz), 7.15 (dd, 1H, J=1.5, 8.1 Hz), 3.81 (s, 1H), 3.76 (d, 1H, J=14.7 Hz), 3.47 (brd, 2H, J=3.5 Hz), 3.20 (br d, 2H, J=7.3 Hz), 3.04 (d, 1H, J=14.5 Hz), 2.8-2.9 (m, 1H), 2.55 (td, 1H, J=3.1, 11.6 Hz), 1.9-2.0 (m, 2H), 1.6-1.9 (m, 4H), 1.4-1.5 (m, 1H). m/z: 419 [M+H]+ | | | |
| 3'-{[(3aR,7aR)-octahydro-1H-pyrrolo[3,2-b]pyridin-4-yl]methyl}-3-fluoro-2'-(1,3-thiazol-5-yl)-[1,1'-biphenyl]-4-carbonitrile | | | | |

| Example 236 | Procedure: K1+A2.2+E1+F2 +D3+C1 | SM1: 2-bromo-3-chlorobenzaldehyde | SM2: 1,3-thiazole | Yield: 41% |
|---|---|---|---|---|
| 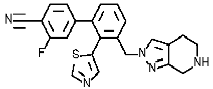 | 1H NMR (DMSO-d6, 500 MHz) δ 9.08 (s, 1H), 7.8-7.9 (m, 2H), 7.5-7.6 (m, 1H), 7.42 (dd, 1H, J=1.0, 7.6 Hz), 7.36 (dd, 1H, J=1.3, 10.4 Hz), 7.32 (s, 1H), 7.17 (dd, 2H, J=1.7, 8.1 Hz), 5.15 (s, 2H), 3.84 (s, 2H), 3.76 (s, 1H), 3.1-3.6 (m, 5H), 2.98 (br t, 2H, J=5.0 Hz), 2.53 (br t, 2H, J=5.7 Hz). m/z: 416 [M+H]+ | | | |
| 3-fluoro-3'-({2H,4H,5H,6H,7H-pyrazolo[3,4-c]pyridin-2-yl}methyl)-2'-(1,3-thiazol-5-yl)-[1,1'-biphenyl]-4-carbonitrile | | | | |

| Example 278 | Procedure: A1.1+A2.2+B1+ C2 | SM1: 2-bromo-3-chlorobenzaldehyde | SM2: cyclopropylboronic acid | Yield: 75% |
|---|---|---|---|---|
| 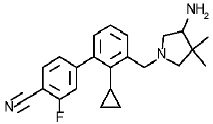 | 1H NMR (500 MHz, DMSO-d6) δ 11.88 - 10.87 (m, 1H), 8.96 - 8.38 (m, 3H), 8.01 (br t, J = 7.5 Hz, 1H), 7.87 - 7.76 (m, 1H), 7.73 - 7.62 (m, 1H), 7.57 - 7.49 (m, 1H), 7.47 - 7.42 (m, 1H), 7.39 (br s, 1H), 5.10 - 4.59 (m, 2H), 3.52 (br d, J = 12.5 Hz, 5H), 1.42 - 1.17 (m, 7H), 0.95 - 0.58 (m, 2H), -0.01 (br d, J = 3.7 Hz, 2H). m/z: 364 [M+H]+ | | | |
| 3'-[(4-amino-3,3-dimethylpyrrolidin-1-yl)methyl]-2'-cyclopropyl-3-fluoro-[1,1'-biphenyl]-4-carbonitrile | | | | |

| Example 277 | Procedure: A1.1+A2.2+B1 | SM1: 2-bromo-3-chlorobenzaldehyde | SM2: cyclopropylboronic acid | Yield: 21% |
|---|---|---|---|---|
| 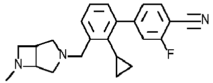 | 1H NMR (DMSO-d6, 400 MHz) δ 7.9-8.1 (m, 1H), 7.6-7.8 (m, 2H), 7.47 (dd, 1H, J=1.5, 8.0 Hz), 7.35 (t, 1H, J=7.6 Hz), 7.20 (dd, 1H, J=1.1, 7.6 Hz), 3.8-4.1 (m, 4H), 3.42 (t, 1H, J=7.5 Hz), 3.20 (d, 1H, J=10.8 Hz), 3.05 (dd, 1H, J=4.9, 7.4 Hz), 2.9-3.0 (m, 1H), 2.85 (d, 1H, J=9.5 Hz), 2.21 (s, 3H), 2.0-2.2 (m, 3H), 1.89 (s, 3H), 0.74 (dd, 2H, J=1.5, 8.5 Hz), 0.04 (br t, 2H, J=6.3 Hz), m/z: 362.3 [M+H]+ | | | |
| 2'-cyclopropyl-3-fluoro-3'-({6-methyl-3,6-diazabicyclo[3.2.0]heptan-3-yl}methyl)-[1,1'-biphenyl]-4-carbonitrile | | | | |

| Example 276 | Procedure: A1.1+A2.2+B1 | SM1: 2-bromo-3-chlorobenzaldehyde | SM2: cyclopropylboronic acid | Yield: 99% |
|---|---|---|---|---|
| 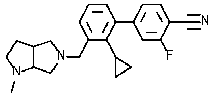 | 1H NMR (500 MHz, DMSO-d6) δ 12.20 - 11.29 (m, 1H), 11.03 - 10.25 (m, 1H), 8.01 (br t, J = 6.7 Hz, 1H), 7.80 - 7.59 (m, 2H), 7.57 - 7.49 (m, 1H), 7.48 - 7.34 (m, 2H), 4.84 (br s, 2H), 3.57 (s, 6H), 3.32 - 2.97 (m, 2H), 2.93 - 2.69 (m, 3H), 2.46 - 1.75 (m, 3H), 1.00 - 0.56 (m, 2H), 0.27 - -0.20 (m, 2H). m/z: 376 [M+H]+ | | | |
| 2'-cyclopropyl-3-fluoro-3'-({1-methyl-octahydropyrrolo[2,3-c]pyrrol-5-yl}methyl)-[1,1'-biphenyl]-4-carbonitrile | | | | |

| Example 116 | Procedure: H1.1+K1+A2.2+ C1 | SM1: 2-bromo-1-chloro-3-iodobenzene | SM2: tert-butyl N-methyl-N-(piperidin-4-yl)carbamate | Yield: 40% |
|---|---|---|---|---|
| 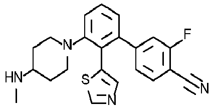 | 1H NMR (600 MHz, DMSO-d6) δ 9.08 (d, J = 0.6 Hz, 1H), 7.84 - 7.73 (m, 1H), 7.48 (d, J = 7.9 Hz, 1H), 7.36 (d, J = 0.6 Hz, 1H), 7.30 (dd, J = 8.1, 1.0 Hz, 1H), 7.29 - 7.24 (m, 1H), 7.16 - 7.11 (m, 1H), 7.10 - 7.02 (m, 1H), 3.02 - 2.88 (m, 1H), 2.64 - 2.52 (m, 2H), 2.34 (s, 2H), 1.88 - 1.13 (m, 3H). m/z: 393 [M+H]+ | | | |
| 3-fluoro-3'-[4-(methylamino)piperidin-1-yl]-2'-(1,3-thiazol-5-yl)-[1,1'-biphenyl]-4-carbonitrile | | | | |

| Example 216 | Procedure: H8+J1+A1.2+B1 +C2 | SM1: (4-bromophenyl)methan ol | SM2: 4-(methoxymethyl)piperi dine | Yield: 44% |
|---|---|---|---|---|
| 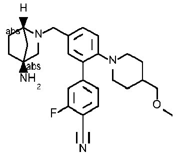 | 1H NMR (DMSO-d6, 600 MHz) δ 10.2-11.6 (m, 1H), 8.85 (br d, 3H, J=12.0 Hz), 8.0-8.1 (m, 1H), 7.86 (ddd, 1H, J=1.2, 11.0, 18.1 Hz), 7.7-7.8 (m, 1H), 7.5-7.7 (m, 2H), 7.17 (dd, 1H, J=4.5, 8.7 Hz), 4.1-4.5 (m, 2H), 3.8-4.1 (m, 1H), 3.22 (s, 5H), 3.17 (d, 2H, J=5.9 Hz), 2.8-3.1 (m, 2H), 2.50 (td, 4H, J=1.8, 3.7 Hz), 1.7-2.4 (m, 5H), 1.5-1.7 (m, 2H), 1.0-1.2 (m, 2H). m/z: 449 [M+H]+ | | | |
| 5'-{[(1S,4S)-4-amino-2-azabicyclo[2.2.1]heptan-2-yl]methyl}-3-fluoro-2'-[4-(methoxymethyl)piperidin-1-yl]-[1,1'-biphenyl]-4-carbonitrile | | | | |

| Example 209 | Procedure: D3+H6+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 63% |
|---|---|---|---|---|
| 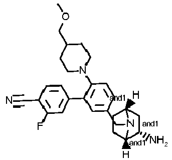 | 1H NMR (DMSO-d6, 600 MHz): δ (ppm) 7.96 (t, J = 7.6 Hz, 1H), 7.78 (dd, J = 11.1, 1.2 Hz, 1H), 7.67 (dd, J = 8.1, 1.5 Hz, 1H), 7.33 (dd, J = 8.4, 1.9 Hz, 1H), 7.23 (d, J = 1.9 Hz, 1H), 7.09 (d, J = 8.4 Hz, 1H), 6.56-8.41 (m, 3H), 3.75 (s, 2H), 3.50 (s, 2H), 3.37-3.43 (m, 1H), 3.28-3.36 (m, 3H), 3.25 (br s, 1H), 3.21 (s, 3H), 3.19-3.21 (m, 1H), 3.16 (d, J = 5.9 Hz, 2H), 2.95 (br d, J = 11.6 Hz, 2H), 2.51-2.56 (m, 2H), 1.60-2.12 (m, 4H), 1.50-1.61 (m, 3H), 1.39-1.48 (m, 1H), 1.02-1.14 (m, 2H), 0.95-1.02 (m, 1H). m/z: 449 [M+H]+ | | | |
| 5'-{[(1S,2S,4R)-2-amino-7-azabicyclo[2.2.1]heptan-7-yl]methyl}-3-fluoro-2'-[4-(methoxymethyl)piperidin-1-yl]-[1,1'-biphenyl]-4-carbonitrile | | | | |

| Example 275 | Procedure: D3+A1 . 1 +A2.2+ C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 79% |
|---|---|---|---|---|
| 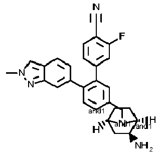 | 1H NMR (600 MHz, DMSO-d6) δ 8.28 (s, 1H), 7.79 - 7.68 (m, 1H), 7.55 - 7.40 (m, 5H), 7.33 (dd, J = 10.6, 1.3 Hz, 1H), 7.09 (dd, J = 8.1, 1.6 Hz, 1H), 6.91 - 6.83 (m, 1H), 4.18 - 4.08 (m, 3H), 3.75 - 3.73 (m, 1H), 3.64 (d, J = 2.8 Hz, 2H), 3.44 - 3.41 (m, 1H), 3.29 - 3.26 (m, 2H), 2.13 - 2.01 (m, 1H), 1.94 - 1.84 (m, 1H), 1.81 - 1.70 (m, 2H), 1.48 - 1.40 (m, 1H), 1.00 - 0.92 (m, 1H). m/z: 452 [M+H]+ | | | |
| 5'-{[(1S,2S,4R)-2-amino-7-azabicyclo[2.2.1]heptan-7-yl]methyl}-3-fluoro-2'-(2-methyl-2H-indazol-6-yl)-[1,1'-biphenyl]-4-carbonitrile | | | | |

| Example 115 | Procedure: H1.1+K1+A2.2+ C1 | SM1: 2-bromo-1-chloro-3-iodobenzene | SM2: tert-butyl N-(piperidin-4-yl)carbamate | Yield: 88% |
|---|---|---|---|---|
| 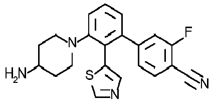 | 1H NMR (DMSO-d6, 600 MHz) δ 9.08 (d, 1H, J=0.6 Hz), 7.79 (dd, 1H, J=7.1, 7.8 Hz), 7.4-7.5 (m, 1H), 7.34 (d, 1H, J=0.6 Hz), 7.2-7.3 (m, 2H), 7.11 (dd, 1H, J=1.0, 7.6 Hz), 7.05 (dd, 1H, J=1.5, 8.1 Hz), 3.71 (s, 1H), 3.1-3.5 (m, 2H), 2.93 (br d, 2H, J=12.0 Hz), 2.6-2.7 (m, 1H), 2.58 (dt, 2H, J=1.9, 11.5 Hz), 1.63 (br d, 2H, J=1 0.9 Hz), 1.1-1.3 (m, 2H). m/z: 379.1 [M+H]+ | | | |
| 3'-(4-aminopiperidin-1-yl)-3-fluoro-2'-(1,3-thiazol-5-yl)-[1,1'-biphenyl]-4-carbonitrile | | | | |

| Example 217 | Procedure: L1+H2+J1+A1.1 +C3+I1+B1+C1 | SM1: (4-bromophenyl)methan ol | | Yield: 100% |
|---|---|---|---|---|
| 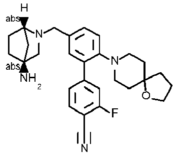 | 1H NMR (500 MHz, DMSO-d6) δ 8.03 - 7.90 (m, 1H), 7.80 (dd, J = 11.1, 1.3 Hz, 1H), 7.69 (dd, J = 8.1, 1.5 Hz, 1H), 7.29 (dd, J = 8.3, 2.0 Hz, 1H), 7.21 (d, J = 2.0 Hz, 1H), 7.09 (d, J = 8.1 Hz, 1H), 6.88 - 4.81 (m, 1H), 3.68 (t, J = 6.7 Hz, 2H), 3.64 - 3.58 (m, 2H), 3.14 (br s, 1H), 2.88 - 2.78 (m, 2H), 2.75 - 2.58 (m, 3H), 2.28 - 2.14 (m, 1H), 1.95 - 1.77 (m, 3H), 1.73 - 1.67 (m, 1H), 1.66 - 1.53 (m, 4H), 1.52 - 1.39 (m, 6H). m/z: 461 [M+H]+ | | | |
| 5'-{[(1S,4S)-4-amino-2-azabicyclo[2.2.1]heptan-2-yl]methyl}-3-fluoro-2'-{1-oxa-8-azaspiro[4.5]decan-8-yl}-[1,1'-biphenyl]-4-carbonitrile | | | | |

| Example 224 | Procedure: D3+H6+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 64% |
|---|---|---|---|---|
| 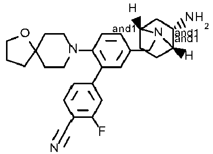 | 1H NMR (DMSO-d6, 600 MHz) δ 7.96 (t, 1H, J=7.6 Hz), 7.80 (dd, 1H, J=1.3, 11.0 Hz), 7.70 (dd, 1H, J=1.5, 8.2 Hz), 7.32 (dd, 1H, J=1.9, 8.2 Hz), 7.22 (d, 1H, J=1.9 Hz), 7.09 (d, 1H, J=8.2 Hz), 3.74 (s, 1H), 3.68 (t, 2H, J=6.7 Hz), 2.9-3.6 (m, 9H), 2.82 (dt, 2H, J=4.0, 7.8 Hz), 2.6-2.8 (m, 2H), 2.03 (br s, 1H), 1.6-1.9 (m, 6H), 1.3-1.5 (m, 5H), 0.89 (br dd, 1H, J=4.0, 12.2 Hz), m/z: 461 [M+H]+ | | | |
| 5'-{[(1S,2S,4R)-2-amino-7-azabicyclo[2.2.1]heptan-7-yl]methyl}-3-fluoro-2'-{1-oxa-8-azaspiro[4.5]decan-8-yl}-[1,1'-biphenyl]-4-carbonitrile | | | | |

| Example 218 | Procedure: D3+A4+A2.2+C2 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 68% |
|---|---|---|---|---|
| 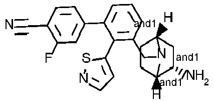 | 1H NMR (DMSO-d6, +TFA 600 MHz): δ (ppm) 8.48 (s, 1H), 7.92 (br d, J = 7.5 Hz, 1H), 7.69 (dt, J = 9.6, 7.6 Hz, 2H), 7.54 (dd, J = 7.7, 1.0 Hz, 1H), 7.41-7.49 (m, 1H), 7.23 (d, J = 10.3 Hz, 1H), 7.12 (dd, J = 8.0, 1.2 Hz, 1H), 4.18-4.33 (m, 2H), 4.09 (br s, 1H), 4.00 (br d, J = 1.6 Hz, 1H), 3.79-3.90 (m, 1H), 2.08-2.37 (m, 1H), 1.63-2.06 (m, 4H), 1.58 (br dd, J = 13.6, 3.2 Hz, 1H). m/z: 405.2 [M+H]+ | | | |
| 3'-{[(1S,2S,4R)-2-amino-7-azabicyclo[2.2.1]heptan-7-yl]methyl}-3-fluoro-2'-(1 ,2-thiazol-5-yl)-[1 ,1'-biphenyl]-4-carbonitrile | | | | |

| Example 274 | Procedure: A1.1+A2.2+B1+ C1 | SM1: 2-bromo-3-chlorobenzaldehyde | SM2: cyclopropylboronic acid | Yield: 100% |
|---|---|---|---|---|
| 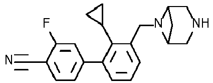 | 1H NMR (DMSO-d6, 600 MHz) δ 8.6-10.5 (m, 2H), 6.9-8.2 (m, 6H), 2.8-5.4 (m, 9H), 1.9-2.3 (m, 2H), 0.5-1.1 (m, 2H), 0.04 (br d, 2H, J=4.7 Hz). m/z: 348.2 [M+H]+ | | | |
| 2'-cyclopropyl-3'-({3,6-diazabicyclo[3.1.1]heptan-6-yl}methyl)-3-fluoro-[1 ,1'-biphenyl]-4-carbonitrile | | | | |

| Example 323 | Procedure: A1.1+A2.2+B1+ C1 | SM1: 2-bromo-3-chlorobenzaldehyde | SM2: cyclopropylboronic acid | Yield: 65% |
|---|---|---|---|---|
| 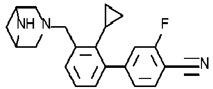 | 1H NMR (600 MHz, DMSO-d6) δ 8.00 - 7.93 (m, 1H), 7.62 (dd, J = 10.6, 1.4 Hz, 1H), 7.52 - 7.44 (m, 2H), 7.33 (s, 1H), 7.21 (dd, J = 7.7, 1.2 Hz, 1H), 4.01 (s, 2H), 3.95 (br d, J = 5.0 Hz, 2H), 3.75 (s, 1H), 3.25 - 3.20 (m, 1H), 3.13 (br d, J = 11.2 Hz, 2H), 2.92 (d, J = 11.3 Hz, 2H), 2.51 (br s, 1H), 2.20 - 2.03 (m, 2H), 0.84 - 0.63 (m, 2H), 0.01 (dd, J = 5.8, 1.4 Hz, 2H). m/z: 348.2 [M+H]+ | | | |
| 2'-cyclopropyl-3'-({3,6-diazabicyclo[3.1.1]heptan-3-yl}methyl)-3-fluoro-[1 ,1'-biphenyl]-4-carbonitrile | | | | |

| Example 349 | Procedure: A1.1+A2.2+B1+ C2 | SM1: 2-bromo-3-chlorobenzaldehyde | SM2: cyclopropylboronic acid | Yield: 74% |
|---|---|---|---|---|
| 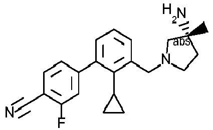 | 1H NMR (DMSO-d6, 500 MHz) δ 10.5-11.7 (m, 1H), 8.4-9.0 (m, 3H), 8.02 (br t, 1H, J=7.3 Hz), 7.1-7.8 (m, 5H), 4.5-5.1 (m, 2H), 3.4-4.0 (m, 4H), 2.50 (td, 3H, J=1.7, 3.6 Hz), 1.53 (br s, 3H), 0.6-1.0 (m, 2H), -0.2-0.2 (m, 2H). m/z: 350.1 [M+H]+ | | | |
| 3'-{[(3R)-3-amino-3-methylpyrrolidin-1-yl]methyl}-2'-cyclopropyl-3-fluoro-[1,1'-biphenyl]-4-carbonitrile | | | | |

| Example 202 | Procedure: A1.1+A2.2+B1+ C1 | SM1: 2-bromo-3-chlorobenzaldehyde | SM2: cyclopropylboronic acid | Yield: 35% |
|---|---|---|---|---|
| 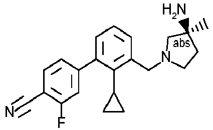 | 1H NMR (500 MHz, DMSO-d6) δ 7.96 (t, J = 7.5 Hz, 1H), 7.60 (dd, J = 10.6, 1.3 Hz, 1H), 7.56 - 7.51 (m, 1H), 7.47 (dd, J = 8.1, 1.5 Hz, 1H), 7.32 (t, J = 7.6 Hz, 1H), 7.22 - 7.15 (m, 1H), 3.94 - 3.79 (m, 2H), 3.32 - 2.93 (m, 2H), 2.92 - 2.82 (m, 1H), 2.71 - 2.63 (m, 1H), 2.58 - 2.52 (m, 1H), 2.43 - 2.33 (m, 1H), 2.18 - 2.05 (m, 1H), 1.89 - 1.74 (m, 2H), 1.31 (s, 3H), 0.79 - 0.67 (m, 2H), 0.11 - -0.09 (m, 2H). m/z: 350.1 [M+H]+ | | | |
| 3'-{[(3S)-3-amino-3-methylpyrrolidin-1-yl]methyl}-2'-cyclopropyl-3-fluoro-[1,1'-biphenyl]-4-carbonitrile | | | | |

| Example 211 | Procedure: A1.1+A2.2+B1+ C2 | SM1: 2-bromo-3-chlorobenzaldehyde | SM2: cyclopropylboronic acid | Yield: 97% |
|---|---|---|---|---|
| 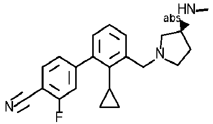 | 1H NMR (DMSO-d6, 500 MHz): δ (ppm) 10.73-11.60 (m, 1H), 9.16-10.06 (m, 2H), 7.93-8.11 (m, 1H), 7.63-7.80 (m, 2H), 7.49-7.57 (m, 1H), 7.42-7.49 (m, 1H), 7.34-7.42 (m, 1H), 4.46-5.00 (m, 2H), 3.50-4.14 (m, 5H), 2.57-2.65 (m, 3H), 2.02-2.49 (m, 3H), 0.65-0.96 (m, 2H), -0.08-0.13 (m, 2H). m/z: 350 [M+H]+ | | | |
| 2'-cyclopropyl-3-fluoro-3'-{[(3S)-3-(methylamino)pyrrolidin-1-yl]methyl}-[1,1'-biphenyl]-4-carbonitrile | | | | |

| Example 228 | Procedure: A1.1+A2.2+B1+ C1 | SM1: 2-bromo-3-chlorobenzaldehyde | SM2: cyclopropylboronic acid | Yield: 85% |
|---|---|---|---|---|
| 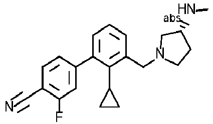 | 1H NMR (500 MHz, DMSO-d6) δ 8.01 - 7.93 (m, 1H), 7.64 - 7.56 (m, 1H), 7.55 - 7.50 (m, 1H), 7.49 - 7.43 (m, 1H), 7.33 (t, J = 7.6 Hz, 1H), 7.26 - 7.14 (m, 1H), 3.88 (d, J = 2.2 Hz, 2H), 3.59 - 3.54 (m, 1H), 3.18 - 2.94 (m, 1H), 2.84 - 2.75 (m, 1H), 2.70 (d, J = 5.4 Hz, 2H), 2.52 - 2.50 (m, 3H), 2.48 - 2.42 (m, 1H), 2.20 - 2.01 (m, 2H), 1.83 - 1.63 (m, 1H), 0.71 (br s, 2H), -0.01 (br dd, J = 3.7, 2.0 Hz, 2H). m/z: 350 [M+H]+ | | | |
| 2'-cyclopropyl-3-fluoro-3'-{[(3R)-3-(methylamino)pyrrolidin-1-yl]methyl}-[1,1'-biphenyl]-4-carbonitrile | | | | |

| Example 171 | Procedure: D3+A1 . 1 +A2.2+ C2 | SM1: 2-bromo-1-(bromomethyl)-3-chlorobenzene | SM2: tert-butyl N-[(1S,4S)-2-azabicyclo[2.2.1]hept an-4-yl]carbamate | Yield: 96% |
|---|---|---|---|---|
| 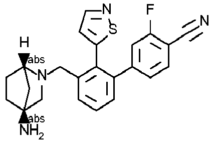 | 1H NMR (DMSO-d6, T=350K ,500 MHz) δ 8.54 (d, 1H, J=1.7 Hz), 8.02 (d, 1H, J=8.1 Hz), 7.78 (dd, 1H, J=7.1, 7.8 Hz), 7.73 (t, 1H, J=7.8 Hz), 7.58 (dd, 1H, J=1.1, 7.7 Hz), 7.48 (d, 1H, J=1.7 Hz), 7.32 (dd, 1H, J=1.5, 10.3 Hz), 7.20 (dd, 1H, J=1.6, 7.9 Hz), 4.1-4.4 (m, 2H), 3.91 (s, 1H), 3.2-3.6 (m, 2H), 1.7-2.3 (m, 6H). m/z: 405 [M+H]+ | | | |
| 3'-{[(1S,4S)-4-amino-2-azabicyclo[2.2.1]heptan-2-yl]methyl}-3-fluoro-2'-(1,2-thiazol-5-yl)-[1,1'-biphenyl]-4-carbonitrile | | | | |

| Example 193 | Procedure: A1.1+A2.2+B1+ C1 | SM1: 2-bromo-3-chlorobenzaldehyde | SM2: cyclopropylboronic acid | Yield: 100% |
|---|---|---|---|---|
| 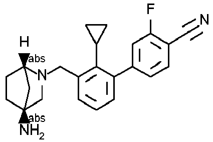 | 1H NMR (DMSO-d6, 500 MHz) + 0.5 eq of tartrate: δ (ppm) 7.96 (dd, J = 7.8, 7.1 Hz, 1H), 7.57-7.60 (m, 1H), 7.48-7.51 (m, 1H), 7.45 (dd, J = 8.1, 1.5 Hz, 1H), 7.32 (t, J = 7.6 Hz, 1H), 7.17 (dd, J = 7.6, 1.2 Hz, 1H), 3.83-3.97 (m, 2H), 3.78 (s, 1H), 3.21 (s, 1H), 2.83 (dd, J = 8.4, 2.6 Hz, 1H), 2.33 (d, J = 8.3 Hz, 1H), 2.01-2.06 (m, 1H), 1.94-2.01 (m, 1H), 1.82 (dd, J = 8.8, 1.7 Hz, 1H), 1.63-1.72 (m, 2H), 1.50-1.57 (m, 2H), 0.72 (dd, J = 8.3, 1.7 Hz, 2H), -0.08-0.02 (m, 2H). m/z: 362 [M+H]+ | | | |
| 3'-{[(1S,4S)-4-amino-2-azabicyclo[2.2.1]heptan-2-yl]methyl}-2'-cyclopropyl-3-fluoro-[1 ,1'-biphenyl]-4-carbonitrile | | | | |

| Example 205 | Procedure: A1.1+A2.2+B1+ C1 | SM1: 2-bromo-3-chlorobenzaldehyde | SM2: cyclopropylboronic acid | Yield: 80% |
|---|---|---|---|---|
| 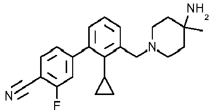 | 1H NMR (DMSO-d6, 500 MHz) δ 7.95 (t, 1H, J=7.6 Hz), 7.59 (dd, 1H, J=1.3, 10.6 Hz), 7.4-7.5 (m, 2H), 7.32 (t, 1H, J=7.6 Hz), 7.18 (dd, 1H, J=1.0, 7.6 Hz), 3.76 (s, 2H), 3.75 (s, 2H), 2.7-3.7 (m, 3H), 2.6-2.7 (m, 2H), 2.36 (br t, 2H, J=8.4 Hz), 2.0-2.1 (m, 1H), 1.5-1.8 (m, 4H), 1.21 (s, 3H), 0.6-0.8 (m, 2H), -0.1-0.0 (m, 2H). m/z: 364 [M+H]+ | | | |
| 3'-[(4-amino-4-methylpiperidin-1-yl)methyl]-2'-cyclopropyl-3-fluoro-[1,1'-biphenyl]-4-carbonitrile | | | | |

| Example 215 | Procedure: A1.1+A2.2+B1+ D1+C1 | SM1: 2-bromo-3-chlorobenzaldehyde | SM2: cyclopropylboronic acid | Yield: 74% |
|---|---|---|---|---|
| 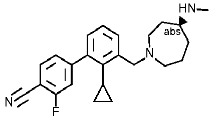 | 1H NMR (DMSO-d6, 500 MHz) δ 11.7-13.2 (m, 2H), 8.3-9.1 (m, 2H), 6.8-8.2 (m, 7H), 4.8-5.7 (m, 2H), 4.4-4.5 (m, 2H), 4.31 (s, 3H), 3.32 (br s, 3H), 2.58 (s, 3H), 1.5-2.3 (m, 7H), 0.6-1.0 (m, 2H), -0.3-0.3 (m, 2H). m/z: 378 [M+H]+ | | | |
| 2'-cyclopropyl-3-fluoro-3'-{[(4S)-4-(methylamino)azepan-1-yl]methyl}-[1,1'-biphenyl]-4-carbonitrile | | | | |

| Example 273 | Procedure: A1.1+A2.2+B1+ C1 | SM1: 2-bromo-3-chlorobenzaldehyde | SM2: cyclopropylboronic acid | Yield: 87% |
|---|---|---|---|---|
| 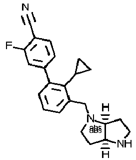 | 1H NMR (600 MHz, DMSO-d6) δ 8.90 - 8.14 (m, 1H), 7.99 - 7.93 (m, 1H), 7.89 - 7.65 (m, 1H), 7.58 (dd, J = 10.6, 1.3 Hz, 1H), 7.47 (dd, J = 7.6, 1.0 Hz, 1H), 7.45 (dd, J = 8.0, 1.5 Hz, 1H), 7.32 (t, J = 7.6 Hz, 1H), 7.19 (dd, J = 7.6, 1.3 Hz, 1H), 4.14 - 4.06 (m, 1H), 4.05 - 3.96 (m, 1H), 3.73 - 3.67 (m, 1H), 3.20 - 3.16 (m, 1H), 3.15 - 3.10 (m, 1H), 3.09 - 3.03 (m, 1H), 2.85 (s, 1H), 2.22 - 2.03 (m, 3H), 1.88 - 1.80 (m, 1H), 1.75 - 1.54 (m, 2H), 0.80 - 0.65 (m, 2H), 0.03 - -0.10 (m, 2H). m/z: 362 [M+H]+ | | | |
| 3'-{[(3aS,6aS)-octahydropyrrolo[3,2-b]pyrrol-1-yl]methyl}-2'-cyclopropyl-3-fluoro-[1,1'-biphenyl]-4-carbonitrile | | | | |

| Example 272 | Procedure: A1.1+A2.2+B1+ D1+C1 | SM1: 2-bromo-3-chlorobenzaldehyde | SM2: cyclopropylboronic acid | Yield: 86% |
|---|---|---|---|---|
| 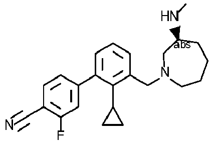 | 1H NMR (DMSO-d6, 500 MHz) δ 7.9-8.0 (m, 1H), 7.5-7.6 (m, 2H), 7.46 (dd, 1H, J=1.6, 7.9 Hz), 7.34 (t, 1H, J=7.6 Hz), 7.20 (dd, 1H, J=1.3, 7.7 Hz), 3.8-4.1 (m, 2H), 3.75 (s, 1H), 3.1-3.6 (m, 4H), 2.5-3.1 (m, 6H), 2.32 (s, 3H), 2.0-2.2 (m, 1H), 1.8-2.0 (m, 1H), 1.5-1.8 (m, 5H), 0.7-0.8 (m, 2H), -0.2-0.1 (m, 2H). m/z: 378 [M+H]+ | | | |
| 2'-cyclopropyl-3-fluoro-3'-{[(3S)-3-(methylamino)azepan-1-yl]methyl}-[1,1'-biphenyl]-4-carbonitrile | | | | |

| Example 322 | Procedure: A1.1+A2.2+B1+ C1 | SM1: 2-bromo-3-chlorobenzaldehyde | SM2: cyclopropylboronic acid | Yield: 68% |
|---|---|---|---|---|
| 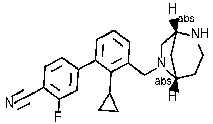 | 1H NMR (DMSO-d6, 500 MHz) δ 8.1-9.1 (m, 2H), 7.96 (t, 1H, J=7.5 Hz), 7.5-7.7 (m, 2H), 7.46 (dd, 1H, J=1.5, 7.8 Hz), 7.34 (t, 1H, J=7.7 Hz), 7.19 (dd, 1H, J=1.0, 7.6 Hz), 4.0-4.2 (m, 3H), 3.87 (s, 1H), 2.8-3.7 (m, 9H), 2.0-2.2 (m, 1H), 1.9-2.0 (m, 1H), 1.84 (d, 1H, J=12.0 Hz), 1.76 (brd, 1H, J=13.9 Hz), 1.60 (dt, 1H, J=6.6, 13.0 Hz), 0.7-0.8 (m, 2H), -0.03 (br t, 2H, J=6.1 Hz), m/z: 362 [M+H]+ | | | |
| 2'-cyclopropyl-3'-{[(1R,5S)-2,6-diazabicyclo[3.2.1]octan-6-yl]methyl}-3-fluoro-[1,1'-biphenyl]-4-carbonitrile | | | | |

| Example 321 | Procedure: A1.1+A2.2+B1+ C2 | SM1: 2-bromo-3-chlorobenzaldehyde | SM2: cyclopropylboronic acid | Yield: 92% |
|---|---|---|---|---|
| 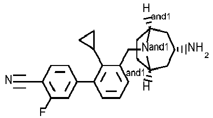 | 1H NMR (600 MHz, DMSO-d6) δ 10.82 - 10.15 (m, 1H), 8.74 - 8.18 (m, 3H), 8.01 (s, 1H), 7.89 (d, J = 7.6 Hz, 1H), 7.71 (br d, J = 11.7 Hz, 1H), 7.59 - 7.53 (m, 1H), 7.46 (q, J = 7.3 Hz, 1H), 7.39 (d, J = 7.6 Hz, 1H), 4.98 - 4.36 (m, 2H), 4.22 - 3.93 (m, 2H), 3.73 - 3.44 (m, 1H), 2.47 - 2.23 (m, 5H), 2.13 - 1.91 (m, 4H), 0.94 - -0.17 (m, 4H). m/z: 376 [M+H]+ | | | |
| 3'-{[(1R,3S,5S)-3-amino-8-azabicyclo[3.2.1]octan-8-yl]methyl}-2'-cyclopropyl-3-fluoro-[1,1'-biphenyl]-4-carbonitrile | | | | |

| Example 300 | Procedure: A1.1+A2.2+B1+ C1 | SM1: 2-bromo-3-chlorobenzaldehyde | SM2: cyclopropylboronic acid | Yield: 93% |
|---|---|---|---|---|
| 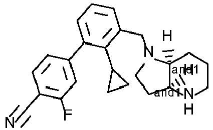 | 1H NMR (DMSO-d6, 600 MHz) δ 8.5-9.3 (m, 1H), 7.8-8.1 (m, 2H), 7.70 (d, 1H, J=7.0 Hz), 7.57 (dd, 1H, J=1.3, 10.6 Hz), 7.46 (dd, 1H, J=1.5, 8.1 Hz), 7.33 (t, 1H, J=7.6 Hz), 7.20 (dd, 1H, J=1.0, 7.6 Hz), 4.23 (d, 1H, J=14.4 Hz), 4.10 (br s, 1H), 3.68 (brdd, 1H, J=3.5, 5.7 Hz), 3.0-3.6 (m, 6H), 2.8-3.0 (m, 1H), 2.71 (br d, 1H, J=2.8 Hz), 2.0-2.3 (m, 4H), 1.5-1.9 (m, 4H), 0.6-0.9 (m, 2H), -0.2-0.2 (m, 2H). m/z: 376 [M+H]+ | | | |
| 3'-{[(3aS,7aS)-octahydro-1H-pyrrolo[3,2-b]pyridin-1-yl]methyl}-2'-cyclopropyl-3-fluoro-[1,1'-biphenyl]-4-carbonitrile | | | | |

| Example 235 | Procedure: D3+H7+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 60% |
|---|---|---|---|---|
| 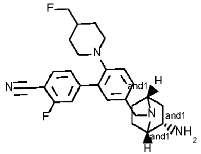 | 1H NMR (DMSO-d6,+ TFA, 600 MHz) δ 7.99 (t, 1H, J=7.6 Hz), 7.7-7.9 (m, 2H), 7.52 (dd, 1H, J=2.0, 8.4 Hz), 7.46 (d, 1H, J=1.9 Hz), 7.20 (d, 1H, J=8.2 Hz), 4.0-4.4 (m, 6H), 3.6-3.9 (m, 1H), 3.0-3.2 (m, 2H), 2.5-2.7 (m, 2H), 1.4-2.4 (m, 9H), 1.0-1.2 (m, 2H). m/z: 437 [M+H]+ | | | |
| 5'-{[(1S,2S,4R)-2-amino-7-azabicyclo[2.2.1]heptan-7-yl]methyl}-3-fluoro-2'-[4-(fluoromethyl)piperidin-1-yl]-[1,1'-biphenyl]-4-carbonitrile | | | | |

| Example 320 | Procedure: D3+N1 +A2.2+C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 36% |
|---|---|---|---|---|
| 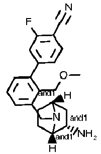 | 1H NMR (DMSO-d6, 600 MHz): δ (ppm) 7.99-8.03 (m, 1H), 7.52-7.57 (m, 2H), 7.41-7.45 (m, 2H), 7.25 (dd, J = 7.7, 1.1 Hz, 1H), 4.29 (q, J = 10.4 Hz, 2H), 4.06 (br s, 3H), 3.65-3.74 (m, 2H), 3.46 (br d, J = 5.7 Hz, 1H), 3.36 (br s, 1H), 3.27-3.27 (m, 1H), 3.21-3.22 (m, 2H), 2.09-2.12 (m, 1H), 1.90 (br d, J = 8.9 Hz, 1H), 1.80-1.88 (m, 1H), 1.69 (ddd, J = 13.2, 9.1, 4.0 Hz, 1H), 1.46-1.52 (m, 1H), 1.06-1.13 (m, 1H). m/z: 366 [M+H]+ | | | |
| 3'-{[(1S,2S,4R)-2-amino-7-azabicyclo[2.2.1]heptan-7-yl]methyl}-3-fluoro-2'-(methoxymethyl)-[1,1'-biphenyl]-4-carbonitrile | | | | |

| Example 270 | Procedure: H3+E1+F2+D3+ A2.2+C1 | SM1: 3-bromo-2-chlorobenzaldehyde | SM2: pyrrolidine | Yield: 38% |
|---|---|---|---|---|
| 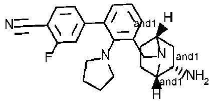 | 1H NMR (DMSO-d6, 500 MHz) δ 7.9-8.0 (m, 1H), 7.56 (dd, 1H, J=2.0, 7.3 Hz), 7.48 (dd, 1H, J=1.2, 10.8 Hz), 7.36 (dd, 1H, J=1.5, 8.1 Hz), 7.2-7.2 (m, 1H), 7.1-7.2 (m, 1H), 3.74 (s, 1H), 3.1-3.6 (m, 9H), 2.91 (br t, 4H, J=6.2 Hz), 1.9-2.1 (m, 1H), 1.7-1.9 (m, 2H), 1.5-1.7 (m, 5H), 1.3-1.5 (m, 1H), 0.91 (br dd, 1H, J=3.5, 12.1 Hz). m/z: 391 [M+H]+ | | | |
| 3'-{[(1S,2S,4R)-2-amino-7-azabicyclo[2.2.1]heptan-7-yl]methyl}-3-fluoro-2'-(pyrrolidin-1-yl)-[1,1'-biphenyl]-4-carbonitrile | | | | |

| Example 213 | Procedure: D2+E1+F1+D3+ A1.1+C1 | SM1: 3-bromo-2-hydroxybenzaldehyde | SM2: 2-iodopropane | Yield: 70% |
|---|---|---|---|---|
| 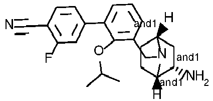 | 1H NMR (DMSO-d6, 600 MHz) δ 7.9-8.0 (m, 1H), 7.68 (dd, 1H, J=1.3, 10.7 Hz), 7.5-7.6 (m, 2H), 7.32 (dd, 1H, J=1.7, 7.6 Hz), 7.22 (t, 1H, J=7.6 Hz), 3.7-3.8 (m, 2H), 3.6-3.7 (m, 2H), 3.1-3.5 (m, 4H), 2.07 (br s, 1H), 1.7-2.0 (m, 3H), 1.4-1.5 (m, 1H), 0.93 (dd, 7H, J=3.1, 6.2 Hz), m/z: 380.2 [M+H]+ | | | |
| 3'-{[(1S,2S,4R)-2-amino-7-azabicyclo[2.2.1]heptan-7-yl]methyl}-3-fluoro-2'-(propan-2-yloxy)-[1,1'-biphenyl]-4-carbonitrile | | | | |

| Example 203 | Procedure: H3+A2.2+B2+C1 | SM1: 3-bromo-2-chlorobenzaldehyde | SM2: azetidine | Yield: 77% |
|---|---|---|---|---|
| 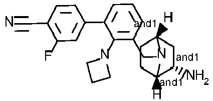 | 1H NMR (DMSO-d6, 500 MHz) δ 7.8-7.9 (m, 1H), 7.44 (dd, 1H, J=1.5, 10.5 Hz), 7.30 (dt, 2H, J=1.7, 8.2 Hz), 6.97 (dd, 1H, J=1.6, 7.5 Hz), 6.78 (t, 1H, J=7.5 Hz), 3.80 (s, 2H), 2.7-3.7 (m, 15H), 1.6-2.2 (m, 6H), 1.46 (br dd, 1H, J=3.8, 7.5 Hz), 1.05 (br dd, 1H, J=4.3, 12.6 Hz). m/z: 377 [M+H]+ | | | |
| 3'-{[(1S,2S,4R)-2-amino-7-azabicyclo[2.2.1]heptan-7-yl]methyl}-2'-(azetidin-1-yl)-3-fluoro-[1,1'-biphenyl]-4-carbonitrile | | | | |

| Example 269 | Procedure: B1+B1+F2+D3+ A2.1+C2 | SM1: (2-amino-3-bromophenyl)methan ol | SM2: cyclopropanecarbalde hyde | Yield: 92% |
|---|---|---|---|---|
| 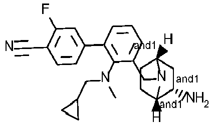 | 1H NMR (DMSO-d6, 600 MHz) δ 11.42 (br d, 1H, J=4.3 Hz), 8.70 (br s, 3H), 7.9-8.1 (m, 2H), 7.49 (dd, 1H, J=1.2, 10.1 Hz), 7.3-7.4 (m, 2H), 7.2-7.3 (m, 1H), 3.2-4.8 (m, 7H), 2.52 (s, 3H), 0.6-2.3 (m, 7H), 0.2-0.6 (m, 2H), 0.00 (br s, 2H). m/z: 405 [M+H]+ | | | |
| 3'-{[(1S,2S,4R)-2-amino-7-azabicyclo[2.2.1]heptan-7-yl]methyl}-2'-[(cyclopropylmethyl)(methyl)amino]-3-fluoro-[1,1'-biphenyl]-4-carbonitrile | | | | |

| Example 268 | Procedure: D3+A1.3+A1.1 + C2 | SM1: 1-bromo-3-(bromomethyl)-5-chloro-2-iodobenzene | SM2: tert-butyl N-[(1S,2S,4R)-7-azabicyclo[2.2.1]hept an-2-yl]carbamate | Yield: 84% |
|---|---|---|---|---|
| 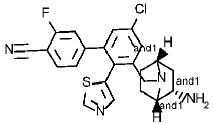 | 1H NMR (500 MHz, DMSO-d6, 300K) δ ppm 10.88 - 11.90 (m, 1 H), 9.11 - 9.19 (m, 1 H), 8.73 (br s, 3 H), 8.33 (br s, 1 H), 7.97 (s, 1 H), 7.81 - 7.88 (m, 1 H), 7.68 (d, J=2.0 Hz, 1 H), 7.42 (dd, J=10.3, 1.2 Hz, 1 H), 7.20 (dd, J=8.1 , 1.5 Hz, 1 H), 3.62 - 4.42 (m, 5 H), 2.50 (dt, J=3.6, 1.7 Hz, 1 H), 1.40 - 2.31 (m, 5 H). m/z: 439 [M+H]+ | | | |
| 3'-{[(1S,2S,4R)-2-amino-7-azabicyclo[2.2.1]heptan-7-yl]methyl}-5'-chloro-3-fluoro-2'-(1,3-thiazol-5-yl)-[1,1'-biphenyl]-4-carbonitrile | | | | |

| Example 267 | Procedure: A1.1+A2.2+B1+ C1 | SM1: 2-bromo-3-chlorobenzaldehyde | SM2: cyclopropylboronic acid | Yield: 100% |
|---|---|---|---|---|
| 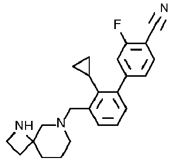 | 1H NMR (DMSO-d6, 500 MHz): δ (ppm) 7.94-7.99 (m, 1H), 7.59 (dd, J = 10.6, 1.3 Hz, 1H), 7.55 (dd, J = 7.8, 1.0 Hz, 1H), 7.46 (dd, J = 8.1, 1.5 Hz, 1H), 7.34 (t, J = 7.7 Hz, 1H), 7.20 (dd, J = 7.6, 1.2 Hz, 1H), 5.88-9.15 (m, 3H), 3.75 (s, 2H), 3.73-3.85 (m, 2H), 3.70 (dt, J = 14.2, 8.4 Hz, 2H), 3.45-3.58 (m, 2H), 2.55-2.86 (m, 2H), 2.19-2.45 (m, 2H), 2.11-2.18 (m, 2H), 2.03-2.10 (m, 1H), 1.44-1.99 (m, 4H), 0.67-0.80 (m, 2H), -0.13-0.03 (m, 2H). m/z: 376.1 [M+H]+ | | | |
| 2'-cyclopropyl-3'-({1,6-diazaspiro[3.5]nonan-6-yl}methyl)-3-fluoro-[1,1'-biphenyl]-4-carbonitrile | | | | |

| Example 266 | Procedure: A1.1+A2.2+B1+ C1 | SM1: 2-bromo-3-chlorobenzaldehyde | SM2: cyclopropylboronic acid | Yield: 80% |
|---|---|---|---|---|
| 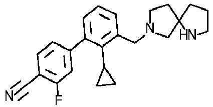 | 1H NMR (500 MHz, DMSO-d6) δ 13.48 - 11.97 (m, 1H), 11.01 - 8.40 (m, 1H), 8.00 (s, 1H), 7.62 (br d, J = 10.3 Hz, 1H), 7.57 (br d, J = 7.6 Hz, 1H), 7.52 - 7.45 (m, 1H), 7.44 - 7.13 (m, 2H), 5.54 - 4.43 (m, 2H), 4.31 (s, 2H), 4.27 - 3.79 (m, 4H), 3.34 - 3.23 (m, 2H), 2.07 (s, 7H), 0.88 - 0.55 (m, 2H), 0.10 - -0.06 (m, 2H). m/z: 376 [M+H]+ | | | |
| 2'-cyclopropyl-3'-({1,7-diazaspiro[4.4]nonan-7-yl}methyl)-3-fluoro-[1,1'-biphenyl]-4-carbonitrile | | | | |

| Example 199 | Procedure: A1.1+A2.2+B1+ C1 | SM1: 2-bromo-3-chlorobenzaldehyde | SM2: cyclopropylboronic acid | Yield: 65% |
|---|---|---|---|---|
| 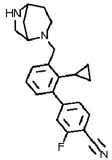 | 1H NMR (600 MHz, DMSO-d6) δ 8.99 - 8.49 (m, 2H), 8.03 - 7.90 (m, 1H), 7.63 - 7.56 (m, 1H), 7.46 (td, J = 8.3, 1.3 Hz, 2H), 7.33 (t, J = 7.6 Hz, 1H), 4.03 (br s, 1H), 3.87 - 3.63 (m, 2H), 3.58 - 3.49 (m, 1H), 3.49 - 3.44 (m, 1H), 3.10 - 3.00 (m, 1H), 2.84 (dd, J = 12.4, 6.1 Hz, 1H), 2.46 - 2.34 (m, 1H), 2.07 (s, 1H), 1.95 - 1.66 (m, 4H), 0.79 - -0.19 (m, 4H). m/z: 362 [M+H]+ | | | |
| 2'-cyclopropyl-3'-({2,6-diazabicyclo[3.2.1]octan-2-yl}methyl)-3-fluoro-[1,1'-biphenyl]-4-carbonitrile | | | | |

| Example 227 | Procedure: A1.1+A2.2+B1+ C1 | SM1: 2-bromo-3-chlorobenzaldehyde | SM2: cyclopropylboronic acid | Yield: 72% |
|---|---|---|---|---|
| 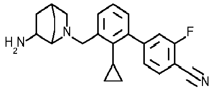 | 1H NMR (500 MHz, DMSO-d6) δ 7.96 (dd, J = 7.8, 7.1 Hz, 1H), 7.86 - 7.66 (m, 1H), 7.63 - 7.56 (m, 1H), 7.51 (dd, J = 7.6, 1.2 Hz, 1H), 7.45 (dd, J = 8.1, 1.5 Hz, 1H), 7.35 - 7.29 (m, 1H), 7.18 (dd, J = 7.7, 1.3 Hz, 1H), 7.03 (br s, 1H), 6.89 - 6.32 (m, 1H), 4.01 - 3.92 (m, 2H), 3.54 - 3.49 (m, 1H), 2.75 - 2.65 (m, 2H), 2.65 - 2.60 (m, 1H), 2.14 - 2.04 (m, 2H), 1.93 - 1.82 (m, 1H), 1.80 - 1.67 (m, 2H), 1.63 - 1.51 (m, 2H), 1.37 - 1.28 (m, 1H), 0.79 - 0.65 (m, 2H), 0.07 - -0.11 (m, 2H). m/z: 376 [M+H]+ | | | |
| 3'-({6-amino-2-azabicyclo[2.2.2]octan-2-yl}methyl)-2'-cyclopropyl-3-fluoro-[1,1'-biphenyl]-4-carbonitrile | | | | |

| Example 210 | Procedure: A1.1+A2.2+B1+ C2 | SM1: 2-bromo-3-chlorobenzaldehyde | SM2: cyclopropylboronic acid | Yield: 29% |
|---|---|---|---|---|
| 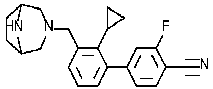 | 1H NMR (600 MHz, DMSO-d6) δ 9.28 - 8.32 (m, 2H), 8.01 - 7.89 (m, 1H), 7.59 (dd, J = 10.6, 1.3 Hz, 1H), 7.49 - 7.41 (m, 2H), 7.37 - 7.30 (m, 1H), 7.26 - 7.15 (m, 1H), 4.01 - 3.93 (m, 2H), 3.80 - 3.74 (m, 2H), 2.73 (dd, J = 12.3, 2.4 Hz, 2H), 2.52 (br d, J = 12.0 Hz, 2H), 2.15 - 2.05 (m, 1H), 2.02 - 1.78 (m, 4H), 0.77 - -0.15 (m, 4H). m/z: 362 [M+H]+ | | | |
| 2'-cyclopropyl-3'-({3,8-diazabicyclo[3.2.1]octan-3-yl}methyl)-3-fluoro-[1,1'-biphenyl]-4-carbonitrile | | | | |

| Example 265 | Procedure: A1.1+A2.2+B1+ C1 | SM1: 2-bromo-3-chlorobenzaldehyde | SM2: cyclopropylboronic acid | Yield: 100% |
|---|---|---|---|---|
| 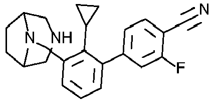 | 1H NMR (500 MHz, DMSO-d6) δ 9.35 - 8.06 (m, 1H), 8.02 - 7.91 (m, 1H), 7.59 (br d, J = 1.2 Hz, 2H), 7.47 (dd, J = 8.1, 1.5 Hz, 1H), 7.34 (t, J = 7.7 Hz, 1H), 7.24 - 7.15 (m, 1H), 3.87 - 3.66 (m, 2H), 3.29 - 3.24 (m, 2H), 3.13 - 2.94 (m, 4H), 2.23 - 2.05 (m, 3H), 1.95 - 1.75 (m, 2H), 0.80 - -0.12 (m, 4H). m/z: 362.2 [M+H]+ | | | |
| 2'-cyclopropyl-3'-({3,8-diazabicyclo[3.2.1]octan-8-yl}methyl)-3-fluoro-[1,1'-biphenyl]-4-carbonitrile | | | | |

| Example 225 | Procedure: A1.1+A2.2+B1+ C1 | SM1: 2-bromo-3-chlorobenzaldehyde | SM2: cyclopropylboronic acid | Yield: 84% |
|---|---|---|---|---|
| 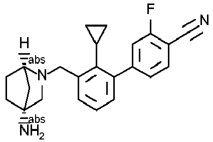 | 1H NMR (DMSO-d6, 600 MHz) δ 7.9-8.0 (m, 1H), 7.58 (dd, 1H, J=1.4, 10.6 Hz), 7.49 (d, 1H, J=6.9 Hz), 7.45 (dd, 1H, J=1.5, 8.1 Hz), 7.32 (t, 1H, J=7.6 Hz), 7.16 (dd, 1H, J=1.2, 7.6 Hz), 3.8-4.1 (m, 2H), 3.77 (s, 1H), 3.21 (s, 4H), 2.82 (dd, 1H, J=2.4, 8.4 Hz), 2.32 (d, 1H, J=8.4 Hz), 1.9-2.1 (m, 2H), 1.80 (dd, 1H, J=1.8, 8.9 Hz), 1.6-1.7 (m, 2H), 1.4-1.6 (m, 2H), 0.72 (dd, 2H, J=1.5, 8.4 Hz), -0.2-0.1 (m, 2H). m/z: 362 [M+H]+ | | | |
| 3'-{[(1R,4R)-4-amino-2-azabicyclo[2.2.1]heptan-2-yl]methyl}-2'-cyclopropyl-3-fluoro-[1 ,1'-biphenyl]-4-carbonitrile | | | | |

| Example 174 | Procedure: K1+E1+L1+J2+A 2.2+C3+I1+B1+C 1 | SM1: 2-bromo-3-chlorobenzaldehyde | SM2: 1,3-oxazole | Yield: 74% |
|---|---|---|---|---|
| 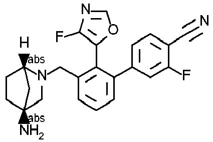 | 1H NMR (600 MHz, DMSO-d6) δ 8.32 (d, J = 2.1 Hz, 1H), 8.20 - 7.97 (m, 1H), 7.89 (dd, J = 7.9, 7.2 Hz, 1H), 7.66 (d, J = 5.0 Hz, 2H), 7.46 (t, J = 4.5 Hz, 1H), 7.38 (dd, J = 10.4, 1.5 Hz, 1H), 7.12 (dd, J = 8.1, 1.6 Hz, 1H), 3.64 - 3.58 (m, 2H), 3.02 - 2.95 (m, 1H), 2.70 - 2.64 (m, 1H), 2.27 - 2.19 (m, 1H), 1.77 - 1.70 (m, 1H), 1.69 - 1.54 (m, 3H), 1.52 - 1.43 (m, 2H). m/z: 407 [M+H]+ | | | |
| 3'-{[(1S,4S)-4-amino-2-azabicyclo[2.2.1]heptan-2-yl]methyl}-3-fluoro-2'-(4-fluoro-1,3-oxazol-5-yl)-[1,1'-biphenyl]-4-carbonitrile | | | | |

| Example 264 | Procedure: A1.1+A2.2+B1+ C1 | SM1: 4-bromo-3-chlorobenzaldehyde | SM2: (2-methyl-2H-indazol-5-yl)boronic acid | Yield: 72% |
|---|---|---|---|---|
| 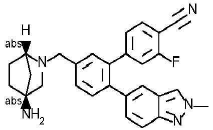 | 1H NMR (DMSO-d6, 600 MHz): δ (ppm) 8.28 (s, 1H), 7.75 (dd, J = 7.8, 7.3 Hz, 1H), 7.51 (dd, J = 1.6, 0.9 Hz, 1H), 7.47 (s, 2H), 7.43-7.45 (m, 1H), 7.41 (s, 1H), 7.32 (dd, J = 10.6, 1.3 Hz, 1H), 7.08 (dd, J = 8.0, 1.5 Hz, 1H), 6.87 (dd, J = 8.9, 1.7 Hz, 1H), 6.46-8.10 (m, 3H), 4.14 (s, 3H), 3.81 (s, 2H), 3.77 (br s, 2H), 3.41-3.48 (m, 3H), 3.25-3.27 (m, 1H), 2.84 (dd, J = 8.4, 2.6 Hz, 1H), 2.34 (br d, J = 7.9 Hz, 1H), 1.92-2.02 (m, 1H), 1.81-1.88 (m, 1H), 1.62-1.75 (m, 2H), 1.53-1.61 (m, 2H). m/z: 452 [M+H]+ | | | |
| 5'-{[(1S,4S)-4-amino-2-azabicyclo[2.2.1]heptan-2-yl]methyl}-3-fluoro-2'-(2-methyl-2H-indazol-5-yl)-[1,1'-biphenyl]-4-carbonitrile | | | | |

| Example 263 | Procedure: A1.1+A2.2+J3+B 1+C1 | SM1: 4-bromo-3-chlorobenzaldehyde | SM2: (2-methyl-2H-indazol-5-yl)boronic acid | Yield: 80% |
|---|---|---|---|---|
| 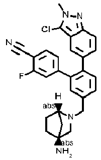 | 1H NMR (600 MHz, DMSO-d6) δ 7.76 (dd, J = 7.9, 7.2 Hz, 1H), 7.51 - 7.40 (m, 5H), 7.36 (dd, J = 10.5, 1.4 Hz, 1H), 7.10 (dd, J = 8.1, 1.6 Hz, 1H), 6.89 (dd, J = 8.9, 1.8 Hz, 1H), 6.78 - 4.96 (m, 2H), 4.12 (s, 2H), 3.75 (s, 2H), 3.22 - 3.20 (m, 1H), 2.83 - 2.74 (m, 1H), 2.33 - 2.21 (m, 1H), 2.00 - 1.89 (m, 1H), 1.80 - 1.72 (m, 1H), 1.68 - 1.58 (m, 2H), 1.53 - 1.44 (m, 2H). m/z: 486 [M+H]+ | | | |
| 5'-{[(1S,4S)-4-amino-2-azabicyclo[2.2.1]heptan-2-yl]methyl}-2'-(3-chloro-2-methyl-2H-indazol-5-yl)-3-fluoro-[1 , 1 '-biphenyl]-4-carbonitrile | | | | |

| Example 234 | Procedure: L1+H2+J1+A1.1 +C3+B3+C1 | SM1: (4-bromophenyl)methanol | | Yield: 91% |
|---|---|---|---|---|
| 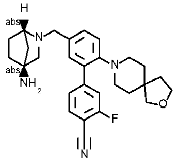 | 1H NMR (500 MHz, DMSO-d6) δ ppm 7.94 - 8.00 (m, 1 H), 7.80 (dd, J=11.0, 1.5 Hz, 1 H), 7.69 (dd, J=8.1, 1.5 Hz, 1 H), 7.31 (dd, J=8.3, 2.0 Hz, 1 H), 7.22 (d, J=2.0 Hz, 1 H), 7.11 (d, J=8.3 Hz, 1 H), 6.64 (br d, J=3.4 Hz, 3 H), 3.78 (s, 1 H), 3.70 (t, J=7.1 Hz, 2 H), 3.59 - 3.67 (m, 2 H), 3.42 (s, 2 H), 3.17 (br s, 1 H), 2.65 - 2.79 (m, 5 H), 2.26 (br d, J=8.3 Hz, 1 H), 1.85 - 1.95 (m, 1 H), 1.36 - 1.80 (m, 11 H). m/z: 461 [M+H]+ | | | |
| 5'-{[(1S,4S)-4-amino-2-azabicyclo[2.2.1]heptan-2-yl]methyl}-3-fluoro-2'-{2-oxa-8-azaspiro[4.5]decan-8-yl}-[1,1'-biphenyl]-4-carbonitrile | | | | |

| Example 262 | Procedure: L1+H2+J1+A1.1 +C3+I1+B1+C1 | SM1: (4-bromophenyl)methanol | | Yield: 80% |
|---|---|---|---|---|
| 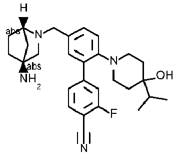 | 1H NMR (500 MHz, DMSO-d6) δ 8.90 - 8.05 (m, 3H), 8.00 - 7.94 (m, 1H), 7.82 (dd, J = 11.2, 1.5 Hz, 1H), 7.68 (dd, J = 8.1, 1.5 Hz, 1H), 7.31 (dd, J = 8.3, 2.0 Hz, 1H), 7.23 (d, J = 1.7 Hz, 1H), 7.11 (d, J = 8.3 Hz, 1H), 3.85 - 3.74 (m, 1H), 3.70 - 3.59 (m, 2H), 3.28 - 3.24 (m, 1H), 2.89 - 2.81 (m, 2H), 2.79 (s, 1H), 2.76 - 2.66 (m, 2H), 2.39 - 2.28 (m, 1H), 1.97 - 1.88 (m, 1H), 1.83 (br d, J = 7.8 Hz, 1H), 1.74 - 1.61 (m, 2H), 1.60 - 1.53 (m, 2H), 1.51 - 1.42 (m, 1H), 1.42 - 1.28 (m, 4H), 0.82 (d, J = 6.8 Hz, 6H). m/z: 463 [M+H]+ | | | |
| 5'-{[(1S,4S)-4-amino-2-azabicyclo[2.2.1]heptan-2-yl]methyl}-3-fluoro-2'-[4-hydroxy-4-(propan-2-yl)piperidin-1-yl]-[1,1'-biphenyl]-4-carbonitrile | | | | |

| Example 172 | Procedure: H3+A2.2+B1+C1 | SM1: 3-bromo-2-chlorobenzaldehyde | SM2: azetidine | Yield: 46% |
|---|---|---|---|---|
| 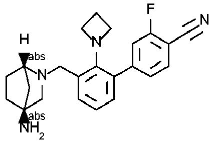 | 1H NMR (600 MHz, DMSO-d6) δ 7.94 - 7.78 (m, 1H), 7.43 (dd, J = 10.6, 1.5 Hz, 1H), 7.35 - 7.21 (m, 2H), 6.95 (dd, J = 7.6, 1.7 Hz, 1H), 6.78 (t, J = 7.5 Hz, 1H), 3.61 (br d, J = 7.0 Hz, 6H), 3.16 - 3.10 (m, 1H), 2.72 - 2.66 (m, 1H), 2.13 (d, J = 8.4 Hz, 1H), 2.02 - 1.88 (m, 3H), 1.78 - 1.71 (m, 1H), 1.66 - 1.41 (m, 4H). m/z: 377 [M+H]+ | | | |
| 3'-{[(1S,4S)-4-amino-2-azabicyclo[2.2.1]heptan-2-yl]methyl}-2'-(azetidin-1-yl)-3-fluoro-[1 ,1'-biphenyl]-4-carbonitrile | | | | |

| Example 191 | Procedure: A1.1+A2.2+E1+F 1+D3+C2 | SM1: 2-bromo-3-chlorobenzaldehyde | SM2: cyclopropylboronic acid | Yield: 88% |
|---|---|---|---|---|
| 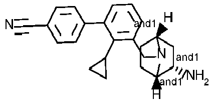 | 1H NMR (600 MHz, DMSO-d6) δ 11.08 (br s, 1H), 9.00 - 8.41 (m, 3H), 7.91 (br d, J = 7.8 Hz, 2H), 7.70 (br d, J = 7.0 Hz, 3H), 7.48 - 7.41 (m, 1H), 7.40 - 7.30 (m, 1H), 4.77 - 4.50 (m, 2H), 4.42 - 4.29 (m, 1H), 4.27 - 4.11 (m, 2H), 2.82 - 2.54 (m, 1H), 2.49 - 2.45 (m, 1H), 2.44 - 2.26 (m, 2H), 2.24 - 2.11 (m, 1H), 2.08 - 1.92 (m, 1H), 1.88 - 1.61 (m, 1H), 0.91 - 0.61 (m, 2H), -0.01 (br s, 2H). m/z: 344.1 [M+H]+ | | | |
| 3'-{[(1S,2S,4R)-2-amino-7-azabicyclo[2.2.1]heptan-7-yl]methyl}-2'-cyclopropyl-[1,1'-biphenyl]-4-carbonitrile | | | | |

| Example 319 | Procedure: A1.1+A2.2+E1+F 1+D3+C1 | SM1: 2-bromo-3-chlorobenzaldehyde | SM2: cyclopropylboronic acid | Yield: 89% |
|---|---|---|---|---|
| 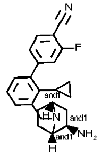 | 1H NMR (DMSO-d6, 500 MHz): δ (ppm) 7.90-8.02 (m, 1H), 7.69 (dd, J = 7.7, 0.9 Hz, 1H), 7.62 (dd, J = 10.6, 1.3 Hz, 1H), 7.48 (dd, J = 8.1, 1.5 Hz, 1H), 7.32 (t, J = 7.7 Hz, 1H), 7.18 (dd, J = 7.8, 1.2 Hz, 1H), 4.12-7.56 (m, 3H), 3.74-3.80 (m, 2H), 3.73 (s, 2H), 3.34-3.68 (m, 3H), 3.30 (t, J = 4.2 Hz, 1H), 3.14 (d, J = 4.2 Hz, 1H), 2.96 (dd, J = 8.1, 2.7 Hz, 1H), 2.09-2.20 (m, 1H), 1.81-1.94 (m, 2H), 1.66 (dd, J = 12.6, 7.9 Hz, 1H), 1.25-1.42 (m, 3H), 0.67-0.79 (m, 2H), -0.11-0.05 (m, 2H). m/z: 362.1 [M+H]+ | | | |
| 3'-{[(1S,2R,4R)-2-amino-7-azabicyclo[2.2.1]heptan-7-yl]methyl}-2'-cyclopropyl-3-fluoro-[1,1'-biphenyl]-4-carbonitrile | | | | |

| Example 184 | Procedure: A1.1+A2.2+B1+ C1 | SM1: 2-bromo-3-chlorobenzaldehyde | SM2: cyclopropylboronic acid | Yield: 90% |
|---|---|---|---|---|
| 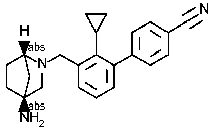 | 1H NMR (500 MHz, DMSO-d6) δ 9.36 - 8.00 (m, 2H), 7.90 - 7.84 (m, 2H), 7.62 - 7.54 (m, 2H), 7.46 (dd, J = 7.7, 1.1 Hz, 1H), 7.32 (t, J = 7.7 Hz, 1H), 7.16 (dd, J = 7.6, 1.2 Hz, 1H), 4.01 - 3.88 (m, 2H), 3.30 - 3.29 (m, 1H), 2.96 - 2.83 (m, 1H), 2.46 - 2.38 (m, 1H), 2.06 - 1.90 (m, 3H), 1.81 - 1.59 (m, 4H), 0.75 - 0.61 (m, 2H), 0.04 - -0.14 (m, 2H). m/z: 344 [M+H]+ | | | |
| 3'-{[(1S,4S)-4-amino-2-azabicyclo[2.2.1]heptan-2-yl]methyl}-2'-cyclopropyl-[1,1'-biphenyl]-4-carbonitrile | | | | |

| Example 318 | Procedure: A1.1+A2.2+B1 + C1 | SM1: 2-bromo-3-chlorobenzaldehyde | SM2: cyclopropylboronic acid | Yield: 74% |
|---|---|---|---|---|
| 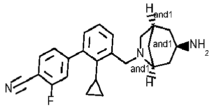 | 1H NMR (600 MHz, DMSO-d6) δ 7.97 (t, J = 7.5 Hz, 1H), 7.64 - 7.59 (m, 1H), 7.59 - 7.52 (m, 1H), 7.51 - 7.45 (m, 1H), 7.37 - 7.30 (m, 1H), 7.25 - 7.16 (m, 1H), 7.02 (br d, J = 8.9 Hz, 1H), 4.16 - 4.06 (m, 1H), 3.96 - 3.85 (m, 1H), 3.37 - 3.36 (m, 1H), 3.19 - 3.13 (m, 2H), 2.67 - 2.58 (m, 1H), 2.48 - 2.41 (m, 1H), 2.07 - 2.00 (m, 1H), 1.99 - 1.89 (m, 1H), 1.88 - 1.77 (m, 2H), 1.74 - 1.64 (m, 1H), 1.63 - 1.53 (m, 1H), 1.42 (d, J = 11.0 Hz, 1H), 0.80 - 0.67 (m, 2H), -0.03 (s, 2H). m/z: 376 [M+H]+ | | | |
| 3'-{[(1S,3R,5S)-3-amino-6-azabicyclo[3.2.1]octan-6-yl]methyl}-2'-cyclopropyl-3-fluoro-[1 ,1'-biphenyl]-4-carbonitrile | | | | |

| Example 261 | Procedure: A1.1+A2.2+E1+F 1+D3+C2 | SM1: 2-bromo-3-chlorobenzaldehyde | SM2: cyclopropylboronic acid | Yield: 99% |
|---|---|---|---|---|
| 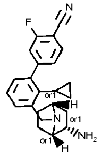 | 1H NMR (DMSO-d6, 600 MHz): δ (ppm) 10.44-11.19 (m, 1H), 8.27-9.01 (m, 3H), 7.93-8.07 (m, 1H), 7.68-7.75 (m, 2H), 7.52-7.60 (m, 1H), 7.45 (br t, J = 7.1 Hz, 1H), 7.30-7.42 (m, 1H), 4.48-4.73 (m, 2H), 3.72-4.42 (m, 3H), 2.51-2.60 (m, 2H), 2.15-2.25 (m, 1H), 2.06-2.47 (m, 2H), 1.97-2.06 (m, 1H), 1.63-1.74 (m, 1H), 0.60-0.95 (m, 2H), -0.08-0.10 (m, 2H). m/z: 362 [M+H]+ | | | |
| 3'-{[(1S,2S,4R)-2-amino-7-azabicyclo[2.2.1]heptan-7-yl]methyl}-2'-cyclopropyl-3-fluoro-[1,1'-biphenyl]-4-carbonitrile | | | | |

| Example 186 | Procedure: A1.1+A2.2+E1 +F 1+D3+C2 | SM1: 2-bromo-3-chlorobenzaldehyde | SM2: cyclopropylboronic acid | Yield: 80% |
|---|---|---|---|---|
| 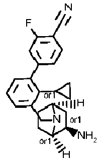 | 1H NMR (DMSO-d6, 600 MHz): δ (ppm) 10.40-11.19 (m, 1H), 8.10-8.98 (m, 3H), 7.92-8.07 (m, 1H), 7.64-7.80 (m, 2H), 7.52-7.62 (m, 1H), 7.45 (br t, J = 7.4 Hz, 1H), 7.30-7.41 (m, 1H), 4.43-4.74 (m, 2H), 4.24 (m, 2H), 4.08-4.22 (br s 1H), 3.76-3.86 (m, 1H), 2.51 (br d, J = 1.9 Hz, 2H), 1.78-2.44 (m, 4H), 1.61-1.75 (m, 1H), 0.61-0.94 (m, 2H), -0.15-0.18 (m, 2H). m/z: 362 [M+H]+ | | | |
| 3'-{[(1R,2R,4S)-2-amino-7-azabicyclo[2.2.1]heptan-7-yl]methyl}-2'-cyclopropyl-3-fluoro-[1,1'-biphenyl]-4-carbonitrile | | | | |

| Example 233 | Procedure: A1.1+A2.2+J3+B 1+C1 | SM1: 4-bromo-3-chlorobenzaldehyde | SM2: (2-methyl-2H-indazol-5-yl)boronic acid | Yield: 88% |
|---|---|---|---|---|
| 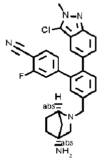 | 1H NMR (600 MHz, DMSO-d6) δ ppm 10.78 - 11.18 (m, 1 H), 8.73 - 9.24 (m, 3 H), 7.82 - 8.01 (m, 2 H), 7.79 (br t, J=7.5 Hz, 1 H), 7.61 - 7.71 (m, 1 H), 7.40 - 7.57 (m, 3 H), 7.10 - 7.20 (m, 1 H), 6.89 (brd, J=8.9 Hz, 1 H), 4.17 - 4.79 (m, 2 H), 4.13 (s, 3 H), 3.81 - 4.11 (m, 1 H), 3.38 (d, J=6.9 Hz, 1 H), 2.97 - 3.22 (m, 1 H), 2.52 - 2.58 (m, 1 H), 2.29 - 2.45 (m, 1 H), 1.59 - 2.14 (m, 4 H). m/z: 486 [M+H]+ | | | |
| 5'-{[(1R,4R)-4-amino-2-azabicyclo[2.2.1]heptan-2-yl]methyl}-2'-(3-chloro-2-methyl-2H-indazol-5-yl)-3-fluoro-[1 , 1 '-biphenyl]-4-carbonitrile | | | | |

| Example 232 | Procedure: D3+H3+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 83% |
|---|---|---|---|---|
| 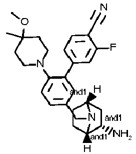 | 1H NMR (600 MHz, DMSO-d6) δ 8.05 - 7.88 (m, 1H), 7.84 - 7.77 (m, 1H), 7.70 (dd, J = 8.1, 1.6 Hz, 1H), 7.32 (dd, J = 8.4, 2.1 Hz, 1H), 7.22 (d, J = 2.1 Hz, 1H), 7.10 (d, J = 8.4 Hz, 1H), 6.97 - 5.49 (m, 3H), 3.52 - 3.49 (m, 2H), 3.36 (br s, 1H), 3.18 (q, J = 5.0 Hz, 2H), 3.06 (s, 3H), 2.80 - 2.71 (m, 2H), 2.70 - 2.63 (m, 2H), 2.06 - 1.96 (m, 1H), 1.88 - 1.65 (m, 3H), 1.60 (br d, J = 13.4 Hz, 2H), 1.45 - 1.31 (m, 3H), 1.08 (s, 3H), 0.95 - 0.87 (m, 1H). m/z: 449 [M+H]+ | | | |
| 5'-{[(1S,2S,4R)-2-amino-7-azabicyclo[2.2.1]heptan-7-yl]methyl}-3-fluoro-2'-(4-methoxy-4-methylpiperidin-1-yl)-[1,1'-biphenyl]-4-carbonitrile | | | | |

| Example 222 | Procedure: D3+A1.1+A2.2+ C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 72% |
|---|---|---|---|---|
| 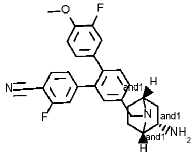 | 1H NMR (DMSO-d6, 600 MHz) + ~ 0.7 mol of tartrate: δ (ppm) 7.81 (dd, J = 7.8, 7.1 Hz, 1H), 7.50 (dd, J = 8.1, 1.6 Hz, 1H), 7.40-7.42 (m, 2H), 7.34 (dd, J = 10.5, 1.4 Hz, 1H), 7.09 (dd, J = 8.1, 1.6 Hz, 1H), 7.07 (t, J = 8.8 Hz, 1H), 6.99 (dd, J = 12.3, 2.2 Hz, 1H), 6.83 (dt, J = 8.4, 1.1 Hz, 1H), 3.81 (s, 3H), 3.74 (s, 1H), 3.59-3.65 (m, 2H), 3.38-3.41 (m, 1H), 3.22-3.25 (m, 2H), 1.98-2.09 (m, 1H), 1.70-1.90 (m, 3H), 1.34-1.46 (m, 1H), 0.94 (brdd, J = 12.3, 4.1 Hz, 1H). m/z: 446 [M+H]+ | | | |
| 4-(4-{[(1S,2S,4R)-2-amino-7-azabicyclo[2.2.1]heptan-7-yl]methyl}-3'-fluoro-4'-methoxy-[1,1'-biphenyl]-2-yl)-2-fluorobenzonitrile | | | | |

| Example 231 | Procedure: B2+F3+A1.1+C1 | SM1: 3-bromo-4-hydroxybenzaldehyde | SM2: tert-butyl N-[(1S,2S,4R)-7-azabicyclo[2.2.1]hept an-2-yl]carbamate | Yield: 74% |
|---|---|---|---|---|
| 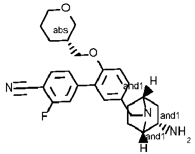 | 1H NMR (DMSO-d6, 600 MHz) δ 7.97 (t, 1H, J=7.6 Hz), 7.65 (dd, 1H, J=1.3, 11.0 Hz), 7.54 (dd, 1H, J=1.5, 8.1 Hz), 7.37 (dd, 1H, J=2.1, 8.4 Hz), 7.35 (d, 1H, J=2.1 Hz), 7.11 (d, 1H, J=8.4 Hz), 3.8-3.9 (m, 2H), 3.7-3.8 (m, 3H), 3.0-3.6 (m, 11H), 2.0-2.1 (m, 1H), 1.9-2.0 (m, 1H), 1.6-1.9 (m, 4H), 1.5-1.6 (m, 1H), 1.4-1.5 (m, 1H), 1.3-1.4 (m, 2H), 0.86 (br dd, 1H, J=3.3, 12.3 Hz), m/z: 436 [M+H]+ | | | |
| 5'-{[(1S,2S,4R)-2-amino-7-azabicyclo[2.2.1]heptan-7-yl]methyl}-3-fluoro-2'-{[(3R)-oxan-3-yl]methoxy}-[1 ,1'-biphenyl]-4-carbonitrile | | | | |

| Example 260 | Procedure: D3+H3+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 59% |
|---|---|---|---|---|
| 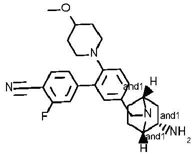 | 1H NMR (600 MHz, DMSO-d6) δ 8.01 - 7.93 (m, 1H), 7.80 (dd, J = 11.1, 1.4 Hz, 1H), 7.69 (dd, J = 8.1, 1.5 Hz, 1H), 7.32 (dd, J = 8.2, 2.1 Hz, 1H), 7.23 (d, J = 2.1 Hz, 1H), 7.09 (d, J = 8.2 Hz, 1H), 6.55 - 5.09 (m, 1H), 3.49 (s, 2H), 3.34 - 3.34 (m, 1H), 3.23 - 3.20 (m, 5H), 3.18 - 3.15 (m, 2H), 2.94 - 2.82 (m, 2H), 2.12 - 1.94 (m, 1H), 1.76 (br dd, J = 9.0, 4.2 Hz, 5H), 1.44 - 1.25 (m, 3H), 0.86 (br dd, J = 12.3, 4.3 Hz, 1H). m/z: 435 [M+H]+ | | | |
| 5'-{[(1S,2S,4R)-2-amino-7-azabicyclo[2.2.1]hepta n-7-yl]methyl}-3-fluoro-2'-(4-methoxypiperid in-1-yl)-[1,1'-biphenyl]-4-carbonitrile | | | | |

| Example 317 | Procedure: A1.1+B2+A2.2+ C1 | SM1: (2-chloro-4-formylphenyl)boronic acid | SM2: 2-bromo-5-methoxypyrazine | Yield: 43% |
|---|---|---|---|---|
| 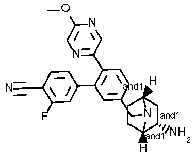 | 1H NMR (600 MHz, DMSO-d6) δ 8.20 (d, J = 1.5 Hz, 1H), 8.10 (d, J = 1.5 Hz, 1H), 7.80 (dd, J = 7.8, 7.1 Hz, 1H), 7.64 - 7.60 (m, 1H), 7.59 - 7.55 (m, 1H), 7.45 (d, J = 1.3 Hz, 1H), 7.35 (dd, J = 10.5, 1.4 Hz, 1H), 7.05 (dd, J = 8.1, 1.5 Hz, 1H), 6.58 - 5.38 (m, 1H), 3.90 (s, 3H), 3.65 (d, J = 3.2 Hz, 2H), 3.42 - 3.41 (m, 1H), 3.25 - 3.23 (m, 2H), 2.13 - 2.02 (m, 1H), 1.91 - 1.83 (m, 1H), 1.82 - 1.67 (m, 2H), 1.43 (ddd, J = 11.8, 8.8, 4.4 Hz, 1H), 0.92 (dd, J = 12.3, 4.2 Hz, 1H). m/z: 430 [M+H]+ | | | |
| 5'-{[(1S,2S,4R)-2-amino-7-azabicyclo[2.2.1]heptan-7-yl]methyl}-3-fluoro-2'-(5-methoxypyrazin-2-yl)-[1,1'-biphenyl]-4-carbonitrile | | | | |

| Example 230 | Procedure: D3+A1.1+A2.2+ C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 57% |
|---|---|---|---|---|
| 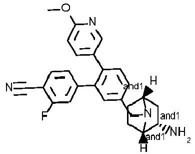 | 1H NMR (600 MHz, DMSO-d6) δ ppm 7.97 (dd, J=2.5, 0.7 Hz, 1 H), 7.82 (dd, J=7.9, 7.0 Hz, 1 H), 7.53 (dd, J=7.8, 1.8 Hz, 1 H), 7.40 - 7.47 (m, 2 H), 7.32 - 7.40 (m, 2 H), 7.10 (dd, J=8.1, 1.5 Hz, 1 H), 6.72 (dd, J=8.6, 0.7 Hz, 1 H), 5.16 - 7.27 (m, 3 H), 3.83 (s, 3 H), 3.75 (s, 1 H), 3.59 - 3.68 (m, 2 H), 3.36 - 3.44 (m, 1 H), 3.23 (q, J=4.7 Hz, 2 H), 1.99 - 2.12 (m, 1 H), 1.63 - 1.93 (m, 3 H), 1.42 (ddd, J=11.9, 8.9, 4.5 Hz, 1 H), 0.92 (dd, J=12.2, 4.3 Hz, 1 H). m/z: 429 [M+H]+ | | | |
| 5'-{[(1S,2S,4R)-2-amino-7-azabicyclo[2.2.1]heptan-7-yl]methyl}-3-fluoro-2'-(6-methoxypyridin-3-yl)-[1,1'-biphenyl]-4-carbonitrile | | | | |

| Example 316 | Procedure: D3+D2-2+A1.1+C1 | SM1: 2-bromo-4-(bromomethyl)phenol | SM2: tert-butyl N-[(1S,2S,4R)-7-azabicyclo[2.2.1]hept an-2-yl]carbamate | Yield: 83% |
|---|---|---|---|---|
| 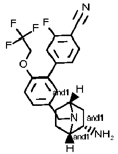 | 1H NMR (600 MHz, DMSO-d6) δ ppm 8.00 (m, 1 H), 8.20 (m, 2 H), 7.65 (dd, J=11.0, 1.5 Hz, 1 H), 7.55 (dd, J=8.1, 1.5 Hz, 1 H), 7.43 (m, 2 H), 7.24 (d, J=8.4 Hz, 1 H), 4.81 (m, 1 H), 4.01 (m, 3 H), 3.55 (brd, J=2.1 Hz, 2 H), 3.44 (m, 3 H), 3.31 (m, 3 H), 3.23 (m, 2 H), 2.07 (m, 1 H), 1.84 (m, 2 H), 1.67 (m, 1 H), 1.46 (m, 1 H), 1.07 (dd, J=12.6, 4.3 Hz, 1 H). m/z: 420 [M+H]+ | | | |
| 5'-{[(1S,2S,4R)-2-amino-7-azabicyclo[2.2.1]heptan-7-yl]methyl}-3-fluoro-2'-(2,2,2-trifluoroethoxy)-[1,1'-biphenyl]-4-carbonitrile | | | | |

| Example 259 | Procedure: L1+H2+J1+A1.1 +C3+I1 +B2+C1 | SM1: (4-bromophenyl)methan ol | | Yield: 74% |
|---|---|---|---|---|
| 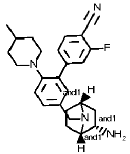 | 1H NMR (600 MHz, DMSO-d6) δ ppm 12.46 (m, 1 H), 7.97 (m, 1 H), 7.75 (m, 5 H), 7.33 (dd, J=8.2, 2.1 Hz, 1 H), 7.23 (d, J=2.1 Hz, 1 H), 7.09 (d, J=8.4 Hz, 1 H), 5.36 (m, 1 H), 4.20 (br s, 2 H), 3.51 (s, 2 H), 3.42 (br s, 1 H), 3.24 (m, 2 H), 2.92 (br d, J=11.9 Hz, 2 H), 2.53 (m, 2 H), 2.05 (m, 1 H), 1.83 (m, 2 H), 1.64 (ddd, J=13.1, 9.2, 4.0 Hz, 1 H), 1.52 (br d, J=1 0.9 Hz, 2 H), 1.44 (m, 1 H), 1.35 (m, 1 H), 1.04 (m, 3 H), 0.88 (d, J=6.5 Hz, 3 H). m/z: 419 [M+H]+ | | | |
| 5'-{[(1S,2S,4R)-2-amino-7-azabicyclo[2.2.1]heptan-7-yl]methyl}-3-fluoro-2'-(4-methylpiperidin-1-yl)-[1,1'-biphenyl]-4-carbonitrile | | | | |

| Example 258 | Procedure: A1.1+A2.2+B2+ C1 | SM1: (2-chloro-4-formylphenyl)boronic acid | SM2: 2-bromo-5-fluoropyridine | Yield: 27% |
|---|---|---|---|---|
| 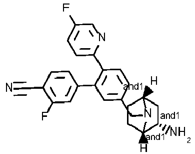 | 1H NMR (DMSO-d6, 600 MHz) δ 8.48 (d, 1H, J=2.9 Hz), 7.8-7.8 (m, 1H), 7.66 (dt, 1H, J=2.9, 8.7 Hz), 7.6-7.6 (m, 1H), 7.5-7.6 (m, 1H), 7.44 (d, 1H, J=1.3 Hz), 7.3-7.3 (m, 2H), 7.01 (dd, 1H, J=1.5, 8.0 Hz), 3.72 (s, 1H), 3.6-3.7 (m, 2H), 3.0-3.5 (m, 7H), 2.0-2.1 (m, 1H), 1.8-1.9 (m, 2H), 1.6-1.7 (m, 1H), 1.3-1.5 (m, 1H), 0.84 (brdd, 1H, J=4.2, 12.1 Hz). m/z: 417 [M+H]+ | | | |
| 5'-{[(1S,2S,4R)-2-amino-7-azabicyclo[2.2.1]heptan-7-yl]methyl}-3-fluoro-2'-(5-fluoropyridin-2-yl)-[1,1'-biphenyl]-4-carbonitrile | | | | |

| Example 315 | Procedure: B2+F3+A1.1+C1 | SM1: 3-bromo-4-hydroxybenzaldehyde | SM2: tert-butyl N-[(1S,2S,4R)-7-azabicyclo[2.2.1]hept an-2-yl]carbamate | Yield: 25% |
|---|---|---|---|---|
| 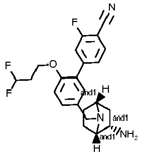 | 1H NMR (600 MHz, DMSO-d6) + 3eq of tartrate δ ppm 7.95 (m, 1 H), 7.68 (dd, J=11.0, 1.5 Hz, 1 H), 7.56 (dd, J=8.1, 1.5 Hz, 1 H), 7.39 (m, 2 H), 7.15 (d, J=8.5 Hz, 1 H), 6.14 (tt, J=56.3, 4.5 Hz, 1 H), 4.23 (m, 3 H), 3.54 (m, J=2.2 Hz, 2 H), 3.30 (m, 3 H), 2.28 (m, 2 H), 2.07 (br d, J=2.3 Hz, 1 H), 1.83 (m, 2 H), 1.65 (ddd, J=13.4, 9.2, 4.0 Hz, 1 H), 1.47 (m, 1 H), 1.07 (m, 1 H). m/z: 416 [M+H]+ | | | |
| 5'-{[(1S,2S,4R)-2-amino-7-azabicyclo[2.2.1]heptan-7-yl]methyl}-2'-(3,3-difluoropropoxy)-3-fluoro-[1,1'-biphenyl]-4-carbonitrile | | | | |

| Example 257 | Procedure: A1.1+B2+A2.2+ C1 | SM1: (2-chloro-4-formylphenyl)boronic acid | SM2: 2-bromo-3-methylpyridine | Yield: 78% |
|---|---|---|---|---|
| 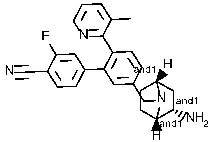 | 1H NMR (DMSO-d6, 600 MHz): δ (ppm) 8.37-8.39 (m, 1H), 7.75 (dd, J = 7.9, 7.2 Hz, 2H), 7.49-7.60 (m, 3H), 7.37 (d, J = 7.8 Hz, 1H), 7.25 (dd, J = 7.7, 4.8 Hz, 1H), 7.15 (dd, J = 10.6, 1.5 Hz, 1H), 3.83 (br s, 1H), 3.66-3.72 (m, 2H), 3.48-3.50 (m, 1H), 3.36 (br d, J = 4.5 Hz, 1H), 3.31 (br d, J = 4.7 Hz, 1H), 2.08-2.15 (m, 1H), 1.78-1.93 (m, 5H), 1.70 (ddd, J = 13.3, 9.0, 4.0 Hz, 1H), 1.45-1.51 (m, 1H), 1.08 (br s, 1H). m/z: 413 [M+H]+ | | | |
| 5'-{[(1S,2S,4R)-2-amino-7-azabicyclo[2.2.1]heptan-7-yl]methyl}-3-fluoro-2'-(3-methylpyridin-2-yl)-[1,1'-biphenyl]-4-carbonitrile | | | | |

| Example 256 | Procedure: A1.1+B2+A2.2+ C1 | SM1: (2-chloro-4-formylphenyl)boronic acid | SM2: 2-bromo-6-methylpyridine | Yield: 71% |
|---|---|---|---|---|
| 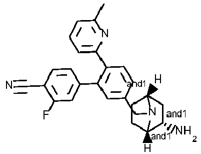 | 1H NMR (DMSO-d6, 600 MHz) δ 7.78 (dd, 1H, J=7.2, 7.9 Hz), 7.61 (d, 1H, J=7.8 Hz), 7.57 (t, 1H, J=7.7 Hz), 7.54 (dd, 1H, J=1.6, 7.9 Hz), 7.43 (d, 1H, J=1.5 Hz), 7.28 (dd, 1H, J=1.5, 10.6 Hz), 7.12 (d, 1H, J=7.6 Hz), 7.03 (dd, 1H, J=1.6, 8.1 Hz), 6.98 (d, 1H, J=7.8 Hz), 3.75 (s, 2H), 3.6-3.7 (m, 3H), 3.4-3.5 (m, 4H), 3.1-3.3 (m, 5H), 2.33 (s, 3H), 2.0-2.1 (m, 1H), 1.7-1.9 (m, 3H), 1.4-1.5 (m, 1H), 0.95 (dd, 1H, J=4.3, 12.3 Hz), m/z: 413 [M+H]+ | | | |
| 5'-{[(1S,2S,4R)-2-amino-7-azabicyclo[2.2.1]heptan-7-yl]methyl}-3-fluoro-2'-(6-methylpyridin-2-yl)-[1,1'-biphenyl]-4-carbonitrile | | | | |

| Example 255 | Procedure: L1+H2+J1+A1.1 +C3+I1 +B2+C1 | SM1: (4-bromophenyl)methan ol | | Yield: 79% |
|---|---|---|---|---|
| 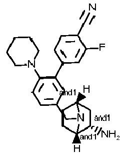 | 1H NMR (500 MHz, DMSO-d6) δ 8.02 - 7.92 (m, 1H), 7.81 (dd, J = 11.2, 1.5 Hz, 1H), 7.69 (dd, J = 8.1, 1.5 Hz, 1H), 7.33 (dd, J = 8.3, 2.0 Hz, 1H), 7.23 (d, J = 2.2 Hz, 1H), 7.08 (d, J = 8.3 Hz, 1H), 6.92 - 5.32 (m, 1H), 3.50 (s, 2H), 3.37 - 3.35 (m, 1H), 3.19 - 3.16 (m, 2H), 2.70 (br s, 4H), 2.06 - 1.95 (m, 1H), 1.89 - 1.63 (m, 3H), 1.42 (br s, 7H), 0.97 - 0.85 (m, 1H). m/z: 405 [M+H]+ | | | |
| 5'-{[(1S,2S,4R)-2-amino-7-azabicyclo[2.2.1]heptan-7-yl]methyl}-3-fluoro-2'-(piperidin-1-yl)-[1,1'-biphenyl]-4-carbonitrile | | | | |

| Example 206 | Procedure: D3+H3+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 74% |
|---|---|---|---|---|
| 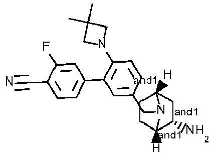 | 1H NMR (600 MHz, DMSO-d6) δ 7.99 - 7.86 (m, 1H), 7.51 (dd, J = 10.6, 1.4 Hz, 1H), 7.42 (dd, J = 8.1, 1.5 Hz, 1H), 7.24 (dd, J = 8.4, 1.9 Hz, 1H), 7.06 (d, J = 1.9 Hz, 1H), 6.98 - 6.71 (m, 1H), 6.58 (d, J = 8.4 Hz, 1H), 3.47 - 3.46 (m, 2H), 3.40 - 3.39 (m, 1H), 3.26 - 3.25 (m, 1H), 3.22 - 3.20 (m, 1H), 3.17 (s, 4H), 2.10 - 1.99 (m, 1H), 1.88 - 1.79 (m, 1H), 1.78 - 1.63 (m, 2H), 1.46 - 1.38 (m, 1H), 1.14 (s, 6H), 0.99 (dd, J = 12.5, 4.2 Hz, 1H). m/z: 405.2 [M+H]+ | | | |
| 5'-{[(1S,2S,4R)-2-amino-7-azabicyclo[2.2.1]heptan-7-yl]methyl}-2'-(3,3-dimethylazetidin-1-yl)-3-fluoro-[1 ,1'-biphenyl]-4-carbonitrile | | | | |

| Example 254 | Procedure: D3+H1.1+B1+A2 .2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 52% |
|---|---|---|---|---|
| 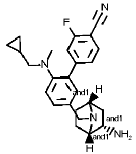 | 1H NMR (DMSO-d6, 600 MHz) δ 7.9-8.0 (m, 1H), 7.72 (dd, 1H, J=1.3, 11.0 Hz), 7.60 (dd, 1H, J=1.6, 8.1 Hz), 7.32 (dd, 1H, J=2.1, 8.4 Hz), 7.20 (d, 1H, J=2.1 Hz), 7.14 (d, 1H, J=8.2 Hz), 3.73 (s, 2H), 3.49 (s, 10H), 2.63 (s, 3H), 2.52 (d, 2H, J=6.6 Hz), 1.9-2.1 (m, 1H), 1.6-1.9 (m, 3H), 1.39 (br d, 1H, J=1.0 Hz), 0.8-0.9 (m, 1H), 0.6-0.8 (m, 1H), 0.34 (dd, 2H, J=1.6, 8.1 Hz), -0.12 (dd, 2H, J=1.4, 4.8 Hz), m/z: 405 [M+H]+ | | | |
| 5'-{[(1S,2S,4R)-2-amino-7-azabicyclo[2.2.1]heptan-7-yl]methyl}-2'-[(cyclopropylmethyl)(methyl)amino]-3-fluoro-[1,1'-biphenyl]-4-carbonitrile | | | | |

| Example 253 | Procedure: D3+N1 +A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 79% |
|---|---|---|---|---|
| 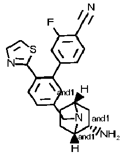 | 1H NMR (DMSO-d6, 600 MHz): δ (ppm) 10.22-12.79 (m, 1H), 7.90 (dd, J = 8.0, 7.0 Hz, 1H), 7.83 (d, J = 7.9 Hz, 1H), 7.79 (d, J = 3.2 Hz, 1H), 7.73 (d, J = 3.2 Hz, 1H), 7.58 (dd, J = 7.9, 1.8 Hz, 1H), 7.45 (dd, J = 10.4, 1.5 Hz, 1H), 7.41 (d, J = 1.3 Hz, 1H), 7.22 (dd, J = 8.1, 1.6 Hz, 1H), 3.85-6.24 (m, 3H), 3.73 (s, 1H), 3.61-3.69 (m, 2H), 3.43-3.44 (m, 3H), 3.29-3.30 (m, 1H), 3.15-3.25 (m, 2H), 2.01-2.11 (m, 1H), 1.79-1.89 (m, 2H), 1.66-1.74 (m, 1H), 1.37-1.45 (m, 1H), 0.82-0.91 (m, 1H). m/z: 405 [M+H]+ | | | |
| 5'-{[(1S,2S,4R)-2-amino-7-azabicyclo[2.2.1]heptan-7-yl]methyl}-3-fluoro-2'-(1 ,3-thiazol-2-yl)-[1 ,1'-biphenyl]-4-carbonitrile | | | | |

| Example 314 | Procedure: A1.1+A2.2+B2+ C1 | SM1: (2-chloro-4-formylphenyl)boronic acid | SM2: 5-bromo-1 ,2-thiazole | Yield: 36% |
|---|---|---|---|---|
| 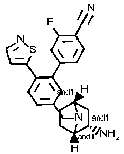 | 1H NMR (DMSO-d6, 600 MHz): δ (ppm) 8.45 (d, J = 1.8 Hz, 1H), 7.91 (dd, J = 8.0, 7.0 Hz, 1H), 7.64 (d, J = 7.9 Hz, 1H), 7.57 (dd, J = 7.9, 1.8 Hz, 1H), 7.50 (dd, J = 10.2, 1.5 Hz, 1H), 7.42 (d, J = 1.5 Hz, 1H), 7.25 (dd, J = 8.0, 1.5 Hz, 1H), 7.21 (d, J = 1.8 Hz, 1H), 5.00-7.17 (m, 3H), 3.74 (s, 2H), 3.60-3.68 (m, 3H), 3.36-3.40 (m, 2H), 3.19-3.24 (m, 3H), 1.99-2.13 (m, 1H), 1.77-1.89 (m, 2H), 1.67-1.75 (m, 1H), 1.38-1.46 (m, 1H), 0.90 (dd, J = 12.3, 4.3 Hz, 1H). m/z: 405 [M+H]+ | | | |
| 5'-{[(1S,2S,4R)-2-amino-7-azabicyclo[2.2.1]heptan-7-yl]methyl}-3-fluoro-2'-(1 ,2-thiazol-5-yl)-[1 ,1'-biphenyl]-4-carbonitrile | | | | |

| Example 252 | Procedure: A1.1+A2.2+B2+ C1 | SM1: (2-chloro-4-formylphenyl)boronic acid | SM2: 3-bromo-1 ,2-thiazole | Yield: 74% |
|---|---|---|---|---|
| 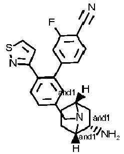 | 1H NMR (600 MHz, DMSO-d6) + 0.4 eq of tartrate δ ppm 8.99 (d, J=4.5 Hz, 1 H), 7.83 (dd, J=7.8, 7.1 Hz, 1 H), 7.68 (d, J=7.9 Hz, 1 H), 7.56 (dd, J=7.8, 1.7 Hz, 1 H), 7.44 (d, J=1.3 Hz, 1 H), 7.30 (dd, J=10.4, 1.5 Hz, 1 H), 7.08 (m, 2 H), 3.72 (s, 1 H), 3.64 (m, 2 H), 3.32 (br d, J=5.3 Hz, 1 H), 3.17 (br t, J=4.5 Hz, 1 H), 3.12 (t, J=4.4 Hz, 1 H), 2.04 (m, 1 H), 1.86 (m, 2 H), 1.66 (br t, J=4.5 Hz, 1 H), 1.39 (m, 1 H), 0.78 (m, 1 H). m/z: 405 [M+H]+ | | | |
| 5'-{[(1S,2S,4R)-2-amino-7-azabicyclo[2.2.1]heptan-7-yl]methyl}-3-fluoro-2'-(1 ,2-thiazol-3-yl)-[1 ,1'-biphenyl]-4-carbonitrile | | | | |

| Example 251 | Procedure: D3+H3+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 70% |
|---|---|---|---|---|
| 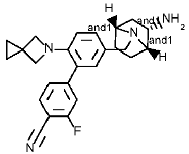 | 1H NMR (DMSO-d6, 500 MHz): δ (ppm) 7.90-7.96 (m, 1H), 7.49-7.54 (m, 1H), 7.42-7.46 (m, 2H), 7.31-7.32 (m, 1H), 6.71 (d, J = 8.6 Hz, 1H), 3.72-3.85 (m, 1H), 3.71-4.30 (m, 5H), 3.57-3.64 (m, 4H), 1.53-2.39 (m, 7H), 1.09-1.24 (m, 1H), 0.52 (s, 4H). m/z: 403.2 [M+H]+ | | | |
| 5'-{[(1S,2S,4R)-2-amino-7-azabicyclo[2.2.1]heptan-7-yl]methyl}-2'-{5-azaspiro[2.3]hexan-5-yl}-3-fluoro-[1 ,1'-biphenyl]-4-carbonitrile | | | | |

| Example 313 | Procedure: D2+A1.1+I3+B2+ A2.2+C1 | SM1: (4-bromo-2-chlorophenyl)methano l | | Yield: 79% |
|---|---|---|---|---|
| 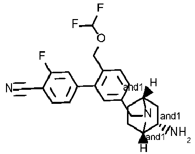 | 1H NMR (500 MHz, DMSO-d6) δ 8.03 (br t, J = 7.3 Hz, 1H), 7.58 (br d, J = 10.3 Hz, 1H), 7.55 - 7.46 (m, 2H), 7.42 (brd, J = 7.8 Hz, 1H), 7.31 (s, 1H), 7.21 - 7.04 (m, 3H), 6.67 (br t, J = 75.6 Hz, 1H), 4.83 (s, 2H), 3.60 (br s, 2H), 3.39 - 3.36 (m, 1H), 3.21 - 3.17 (m, 2H), 2.13 - 1.94 (m, 1H), 1.89 - 1.62 (m, 3H), 1.52 - 1.34 (m, 1H), 1.00 - 0.72 (m, 1H). m/z: 402 [M+H]+ | | | |
| 5'-{[(1S,2S,4R)-2-amino-7-azabicyclo[2.2.1]heptan-7-yl]methyl}-2'-[(difluoromethoxy)methyl]-3-fluoro-[1,1'-biphenyl]-4-carbonitrile | | | | |

| Example 312 | Procedure: D3+D2+A1.1+C1 | SM1: 2-bromo-4-(bromomethyl)phenol | SM2: tert-butyl N-[(1S,2S,4R)-7-azabicyclo[2.2.1]hept an-2-yl]carbamate | Yield: 81% |
|---|---|---|---|---|
| 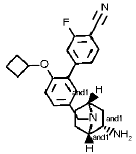 | 1H NMR (DMSO-d6, 600 MHz) δ 7.9-8.0 (m, 1H), 7.68 (dd, 1H, J=1.5, 11.2 Hz), 7.59 (dd, 1H, J=1.5, 8.1 Hz), 7.3-7.4 (m, 2H), 6.93 (d, 1H, J=8.9 Hz), 4.72 (quin, 1H, J=7.2 Hz), 3.74 (s, 1H), 3.50 (s, 2H), 3.36 (br s, 6H), 3.1-3.2 (m, 3H), 2.4-2.4 (m, 2H), 2.0-2.1 (m, 3H), 1.5-1.9 (m, 4H), 1.4-1.4 (m, 1H), 0.90 (br dd, 1H, J=4.1, 12.3 Hz). m/z: 392 [M+H]+ | | | |
| 5'-{[(1S,2S,4R)-2-amino-7-azabicyclo[2.2.1]heptan-7-yl]methyl}-2'-cyclobutoxy-3-fluoro-[1,1'-biphenyl]-4-carbonitrile | | | | |

| Example 311 | Procedure: B2+F3+A1.1+C1 | SM1: 3-bromo-4-hydroxybenzaldehyde | SM2: tert-butyl N-[(1S,2S,4R)-7-azabicyclo[2.2.1]hept an-2-yl]carbamate | Yield: 77% |
|---|---|---|---|---|
| 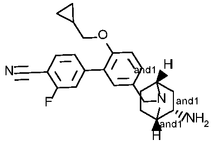 | 1H NMR (600 MHz, DMSO-d6) δ 8.02 - 7.92 (m, 1H), 7.72 (dd, J = 11.2, 1.5 Hz, 1H), 7.60 (dd, J = 8.1, 1.5 Hz, 1H), 7.40 - 7.31 (m, 2H), 7.08 (d, J = 9.1 Hz, 1H), 6.92 - 5.69 (m, 3H), 3.89 (d, J = 6.9 Hz, 2H), 3.51 (d, J = 1.8 Hz, 2H), 3.38 - 3.32 (m, 1H), 3.20 - 3.15 (m, 2H), 2.11 - 1.95 (m, 1H), 1.87 - 1.74 (m, 2H), 1.74 - 1.63 (m, 1H), 1.46 - 1.34 (m, 1H), 1.20 - 1.08 (m, 1H), 0.90 (br dd, J = 12.2, 3.7 Hz, 1H), 0.61 - 0.20 (m, 4H). m/z: 392 [M+H]+ | | | |
| 5'-{[(1S,2S,4R)-2-amino-7-azabicyclo[2.2.1]heptan-7-yl]methyl}-2'-(cyclopropylmethoxy)-3-fluoro-[1,1'-biphenyl]-4-carbonitrile | | | | |

| Example 310 | Procedure: D3+D2+A1.1+C2 | SM1: 2-bromo-4-(bromomethyl)phenol | SM2: tert-butyl N-[(1S,2S,4R)-7-azabicyclo[2.2.1]hept an-2-yl]carbamate | Yield: 83% |
|---|---|---|---|---|
| 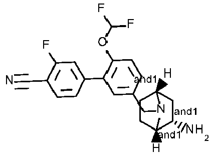 | 1H NMR (600 MHz, DMSO-d6) δ 12.03 - 10.57 (m, 1H), 8.55 (br s, 3H), 8.07 (s, 1H), 8.02 - 7.88 (m, 1H), 7.87 - 7.82 (m, 1H), 7.82 - 7.74 (m, 1H), 7.70 - 7.58 (m, 1H), 7.49 - 7.38 (m, 1H), 7.30 (s, 1H), 4.59 - 4.21 (m, 2H), 4.19 - 3.90 (m, 3H), 2.49 - 2.41 (m, 1H), 2.34 - 1.57 (m, 5H). m/z: 388.2 [M+H]+ | | | |
| 5'-{[(1S,2S,4R)-2-amino-7-azabicyclo[2.2.1]heptan-7-yl]methyl}-2'-(difluoromethoxy)-3-fluoro-[1,1'-biphenyl]-4-carbonitrile | | | | |

| Example 170 | Procedure: D3+A1 . 1 +A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 74% |
|---|---|---|---|---|
| 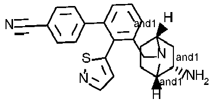 | 1H NMR (DMSO-d6, 600 MHz): δ (ppm) 8.41 (d, J = 1.8 Hz, 1H), 7.71 (d, J = 7.9 Hz, 2H), 7.62 (dd, J = 7.8, 1.2 Hz, 1H), 7.55 (t, J = 7.7 Hz, 1H), 7.38 (dd, J = 7.6, 1.3 Hz, 1H), 7.33 (d, J = 8.0 Hz, 2H), 7.18 (d, J = 1.8 Hz, 1H), 3.75 (s, 1H), 3.63 (br d, J = 8.2 Hz, 1H), 3.33-3.39 (m, 3H), 3.07-3.14 (m, 2H), 1.96-2.03 (m, 1H), 1.66-1.77 (m, 2H), 1.49-1.64 (m, 1H), 1.35-1.40 (m, 1H), 1.24 (br d, J = 2.8 Hz, 0H), 0.83-0.97 (m, 1H). m/z: 387 [M+H]+ | | | |
| 3'-{[(1S,2S,4R)-2-amino-7-azabicyclo[2.2.1]heptan-7-yl]methyl}-2'-(1 ,2-thiazol-5-yl)-[1 ,1'-biphenyl]-4-carbonitrile | | | | |

| Example 250 | Procedure: D3+D2+A 1.1 +C 1 | SM1: 2-bromo-4-(bromomethyl)phenol | SM2: tert-butyl N-[(1S,2S,4R)-7-azabicyclo[2.2.1]hept an-2-yl]carbamate | Yield: 81% |
|---|---|---|---|---|
| 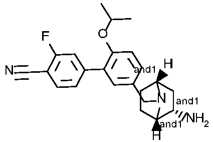 | 1H NMR (DMSO-d6, 500 MHz): δ (ppm) 7.94 (dd, J = 8.1, 7.3 Hz, 1H), 7.65 (dd, J = 11.2, 1.5 Hz, 1H), 7.57 (dd, J = 8.1, 1.5 Hz, 1H), 7.33-7.40 (m, 2H), 7.11 (d, J = 8.6 Hz, 1H), 5.01-6.94 (m, 2H), 4.62 (quin, J = 6.1 Hz, 1H), 3.73 (s, 2H), 3.48-3.54 (m, 2H), 3.35-3.42 (m, 5H), 3.18 (br t, J = 4.9 Hz, 2H), 1.63-2.13 (m, 4H), 1.36-1.44 (m, 1H), 1.23 (d, J = 6.1 Hz, 6H), 0.84-0.94 (m, 1H). m/z: 380 [M+H]+ | | | |
| 5'-{[(1S,2S,4R)-2-amino-7-azabicyclo[2.2.1]heptan-7-yl]methyl}-3-fluoro-2'-(propan-2-yloxy)-[1,1'-biphenyl]-4-carbonitrile | | | | |

| Example 309 | Procedure: D3+A1 . 1 +A2.2+ C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 26% |
|---|---|---|---|---|
| 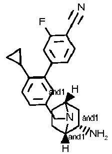 | 1H NMR (600 MHz, DMSO-d6) δ 13.06 - 8.33 (m, 3H), 8.02 - 7.96 (m, 1H), 7.61 (dd, J = 10.5, 1.4 Hz, 1H), 7.48 (dd, J = 8.0, 1.5 Hz, 1H), 7.32 (dd, J = 8.1, 1.6 Hz, 1H), 7.19 (d, J = 1.8 Hz, 1H), 7.02 (d, J = 8.1 Hz, 1H), 3.52 (d, J = 2.8 Hz, 2H), 3.34 - 3.32 (m, 1H), 3.15 - 3.12 (m, 2H), 2.07 - 1.95 (m, 1H), 1.86 - 1.75 (m, 3H), 1.72 - 1.57 (m, 1H), 1.42 - 1.31 (m, 1H), 0.89 - 0.57 (m, 5H). m/z: 377 [M+H]+ | | | |
| 5'-{[(1S,2S,4R)-2-amino-7-azabicyclo[2.2.1]heptan-7-yl]methyl}-2'-cyclopropyl-3-fluoro-[1,1'-biphenyl]-4-carbonitrile | | | | |

| Example 178 | Procedure: A1.1+A2.2+B1 + D1+C2 | SM1: 2-bromo-3-chlorobenzaldehyde | SM2: cyclopropylboronic acid | Yield: 78% |
|---|---|---|---|---|
| 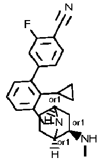 | 1H NMR (DMSO-d6, 600 MHz): δ (ppm) 10.73-11.25 (m, 1H), 9.77-10.01 (m, 1H), 9.50-9.73 (m, 1H), 7.95-8.08 (m, 1H), 7.65-7.78 (m, 2H), 7.56 (br d, J = 7.2 Hz, 1H), 7.42-7.48 (m, 1H), 7.36-7.42 (m, 1H), 4.47-4.69 (m, 2H), 4.18-4.46 (m, 2H), 3.79-4.12 (m, 1H), 2.51-2.83 (m, 5H), 1.73-2.48 (m, 5H), 0.70-0.99 (m, 2H), -0.07-0.09 (m, 2H). m/z: 376 [M+H]+ | | | |
| 2'-cyclopropyl-3-fluoro-3'-{[(1R,2R,4S)-2-(methylamino)-7-azabicyclo[2.2.1]heptan-7-yl]methyl}-[1,1'-biphenyl]-4-carbonitrile | | | | |

| Example 249 | Procedure: D3+A1 . 1 +A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 76% |
|---|---|---|---|---|
| 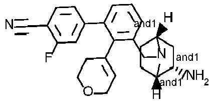 | 1H NMR (DMSO-d6, 600 MHz): δ (ppm) 7.94 (dd, J = 8.0, 7.1 Hz, 1H), 7.54-7.57 (m, 1H), 7.51-7.54 (m, 1H), 7.39-7.42 (m, 1H), 7.37 (t, J = 7.6 Hz, 1H), 7.23 (dd, J = 7.6, 1.3 Hz, 1H), 5.58-5.77 (m, 1H), 4.83-7.15 (m, 3H), 4.09-4.17 (m, 1H), 3.96-4.04 (m, 1H), 3.75 (s, 2H), 3.58-3.71 (m, 4H), 3.42-3.48 (m, 2H), 3.27-3.39 (m, 2H), 3.11-3.25 (m, 2H), 1.93-2.15 (m, 2H), 1.37-1.89 (m, 5H), 0.86-0.97 (m, 1H). m/z: 404.2 [M+H]+ | | | |
| 3'-{[(1S,2S,4R)-2-amino-7-azabicyclo[2.2.1]heptan-7-yl]methyl}-2'-(3,6-dihydro-2H-pyran-4-yl)-3-fluoro-[1 , 1 '-biphenyl]-4-carbonitrile | | | | |

| Example 308 | Procedure: A1.1 +A2.2+B1 + C1 | SM1: 4-bromo-3-chlorobenzaldehyde | SM2: cyclopropylboronic acid | Yield: 85% |
|---|---|---|---|---|
| 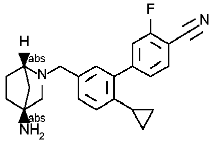 | 1H NMR (DMSO-d6, 500 MHz): δ (ppm) 10.27-11.70 (m, 2H), 8.00 (dd, J = 8.1, 7.1 Hz, 1H), 7.60 (dd, J = 10.5, 1.7 Hz, 1H), 7.47 (dd, J = 7.9, 1.6 Hz, 1H), 7.29 (dd, J = 8.1, 2.0 Hz, 1H), 7.17 (d, J = 1.7 Hz, 1H), 7.02 (d, J = 8.1 Hz, 1H), 4.51-6.87 (m, 2H), 3.75 (s, 2H), 3.60-3.68 (m, 2H), 3.34-3.41 (m, 2H), 3.13 (s, 1H), 2.69 (dd, J = 8.4, 2.8 Hz, 1H), 2.20 (d, J = 8.6 Hz, 1H), 1.84-1.97 (m, 1H), 1.78 (tt, J = 8.4, 5.4 Hz, 1H), 1.69 (dd, J = 8.8, 2.0 Hz, 1H), 1.51-1.63 (m, 2H), 1.37-1.49 (m, 2H), 0.76-0.88 (m, 2H), 0.58-0.69 (m, 2H). m/z: 362 [M+H]+ | | | |
| 5'-{[(1S,4S)-4-amino-2-azabicyclo[2.2.1]heptan-2-yl]methyl}-2'-cyclopropyl-3-fluoro-[1 ,1'-biphenyl]-4-carbonitrile | | | | |

| Example 181 | Procedure: A1. 1 +A2.2+B2+ C1 | SM1: 2-bromo-3-chloro-6-fluorobenzaldehyde | SM2: cyclopropylboronic acid | Yield: 73% |
|---|---|---|---|---|
| 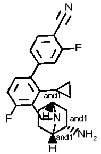 | 1H NMR (600 MHz, DMSO-d6) δ 7.99 - 7.89 (m, 1H), 7.60 (dd, J = 10.6, 1.5 Hz, 1H), 7.45 (dd, J = 8.1, 1.5 Hz, 1H), 7.25 (dd, J = 8.6, 5.9 Hz, 1H), 7.22 - 7.16 (m, 1H), 7.12 - 5.49 (m, 3H), 3.87 - 3.79 (m, 2H), 3.37 - 3.33 (m, 1H), 3.31 - 3.29 (m, 1H), 3.28 - 3.26 (m, 1H), 2.22 - 2.14 (m, 1H), 2.05 - 1.99 (m, 1H), 1.96 - 1.87 (m, 1H), 1.86 - 1.74 (m, 2H), 1.53 - 1.40 (m, 1H), 0.93 (br dd, J = 12.3, 4.0 Hz, 1H), 0.82 - 0.58 (m, 2H), 0.22 - 0.01 (m, 2H). m/z: 380 [M+H]+ | | | |
| 3'-{[(1S,2S,4R)-2-amino-7-azabicyclo[2.2.1]heptan-7-yl]methyl}-2'-cyclopropyl-3,4'-difluoro-[1,1'-biphenyl]-4-carbonitrile | | | | |

| Example 189 | Procedure: A1.1+A2.2+B1+ C1 | SM1: 2-bromo-3-chloro-6-fluorobenzaldehyde | SM2: cyclopropylboronic acid | Yield: 69% |
|---|---|---|---|---|
| 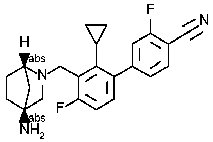 | 1H NMR (600 MHz, DMSO-d6) δ 8.00 - 7.88 (m, 1H), 7.57 (dd, J = 10.6, 1.5 Hz, 1H), 7.43 (dd, J = 8.0, 1.5 Hz, 1H), 7.25 (dd, J = 8.6, 5.9 Hz, 1H), 7.21 - 7.13 (m, 1H), 7.05 - 5.86 (m, 3H), 3.95 - 3.85 (m, 2H), 3.14 (s, 1H), 2.81 (dd, J = 8.2, 2.8 Hz, 1H), 2.44 (d, J = 8.2 Hz, 1H), 2.22 - 2.08 (m, 1H), 1.98 - 1.92 (m, 1H), 1.77 - 1.39 (m, 5H), 0.84 - -0.12 (m, 4H). m/z: 380 [M+H]+ | | | |
| 3'-{[(1S,4S)-4-amino-2-azabicyclo[2.2.1]heptan-2-yl]methyl}-2'-cyclopropyl-3,4'-difluoro-[1,1'-biphenyl]-4-carbonitrile | | | | |

| Example 229 | Procedure: A1. 1 +A2.2+B2+ C1 | SM1: 2-bromo-3-chloro-5-fluorobenzaldehyde | SM2: cyclopropylboronic acid | Yield: 76% |
|---|---|---|---|---|
| 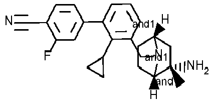 | 1H NMR (500 MHz, DMSO-d6) δ ppm -0.02 (dd, J=5.87, 1.47 Hz, 2 H) 0.67 - 0.78 (m, 2 H) 1.18 (d, J=11.98 Hz, 1 H) 1.34 (s, 3 H) 1.41 - 1.54 (m, 1 H) 1.66 (ddd, J=12.04, 5.07, 2.45 Hz, 1 H) 1.77 - 1.99 (m, 3 H) 2.01 - 2.15 (m, 1 H) 2.94 (d, J=4.16 Hz, 1 H) 3.24 (t, J=4.52 Hz, 1 H) 3.33 - 3.62 (m, 3 H) 3.69 - 3.74 (m, 1 H) 3.74 (s, 2 H) 3.78 - 3.85 (m, 1 H) 4.99 - 8.29 (m, 3 H) 7.17 (dd, J=7.70, 1.34 Hz, 1 H) 7.32 (t, J=7.70 Hz, 1 H) 7.45 (dd, J=7.95, 1.59 Hz, 1 H) 7.55 - 7.62 (m, 1 H) 7.96 (dd, J=7.83, 7.09 Hz, 1 H). m/z: 376.3 [M+H]+ | | | |
| 3'-{[(1S,2S,4R)-2-amino-2-methyl-7-azabicyclo[2.2.1]heptan-7-yl]methyl}-2'-cyclopropyl-3-fluoro-[1,1'-biphenyl]-4-carbonitrile | | | | |

| Example 248 | Procedure: A1.1 +A2.2+E1 +F 1+D3+B1+C2 | SM1: 2-bromo-3-chlorobenzaldehyde | SM2: cyclopropylboronic acid | Yield: 86% |
|---|---|---|---|---|
| 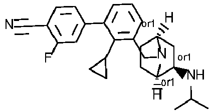 | 1H NMR (600 MHz, DMSO-d6) δ 11.12 - 10.40 (m, 1H), 9.88 - 9.30 (m, 2H), 8.02 (t, J = 7.5 Hz, 1H), 7.80 - 7.60 (m, 2H), 7.56 (br d, J = 7.8 Hz, 1H), 7.49 - 7.42 (m, 1H), 7.42 - 7.37 (m, 1H), 4.78 - 4.56 (m, 2H), 4.55 - 4.45 (m, 1H), 4.36 - 4.18 (m, 2H), 3.40 - 3.28 (m, 1H), 2.87 - 2.78 (m, 1H), 2.52 (br s, 1H), 2.43 - 2.09 (m, 4H), 2.08 - 1.87 (m, 1H), 1.43 - 1.17 (m, 6H), 0.97 - -0.18 (m, 4H). m/z: 404 [M+H]+ | | | |
| 2'-cyclopropyl-3-fluoro-3'-{[(1R,2R,4S)-2-[(propan-2-yl)amino]-7-azabicyclo[2.2.1]heptan-7-yl]methyl}-[1,1'-biphenyl]-4-carbonitrile | | | | |

| Example 307 | Procedure: D3+A5+A2.2+C2 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 74% |
|---|---|---|---|---|
| 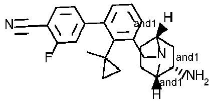 | 1H NMR (600 MHz, DMSO-d6) δ ppm 10.76 (m, 1 H), 7.91 (m, 10 H), 4.31 (m, 5 H), 2.03 (m, 4 H), 1.41 (s, 3 H), 0.49 (m, 3 H). m/z: 376.3 [M+H]+ | | | |
| 3'-{[(1S,2S,4R)-2-amino-7-azabicyclo[2.2.1]heptan-7-yl]methyl}-3-fluoro-2'-(1-methylcyclopropyl)-[1,1'-biphenyl]-4-carbonitrile | | | | |

| Example 220 | Procedure: D3+A1.1+E2+A2 .2+C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 58% |
|---|---|---|---|---|
| 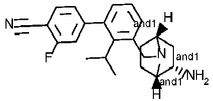 | 1H NMR (500 MHz, DMSO-d6) δ 8.03 - 7.89 (m, 1H), 7.54 (br d, J = 7.1 Hz, 1H), 7.49 - 7.45 (m, 1H), 7.29 (dd, J = 7.8, 1.5 Hz, 1H), 7.22 (t, J = 7.6 Hz, 1H), 6.95 (dd, J = 7.6, 1.2 Hz, 1H), 6.83 - 5.47 (m, 3H), 3.67 (br d, J = 5.1 Hz, 2H), 3.39 (br s, 1H), 3.28 (br d, J = 12.0 Hz, 3H), 2.13-2.01 (m, 1H), 1.94 - 1.84 (m, 1H), 1.84 - 1.65 (m, 2H), 1.53 - 1.36 (m, 1H), 1.10 (br d, J = 7.1 Hz, 6H), 0.96 (br d, J = 8.8 Hz, 1H). m/z: 364 [M+H]+ | | | |
| 3'-{[(1S,2S,4R)-2-amino-7-azabicyclo[2.2.1]heptan-7-yl]methyl}-3-fluoro-2'-(propan-2-yl)-[1,1'-biphenyl]-4-carbonitrile | | | | |

| Example 247 | Procedure: Q1+A1.1+A2.2+ C1 | SM1: (2E)-but-2-ene | SM2: tert-butyl N-[(1S,2S,4R)-7-[(2-bromo-3-chlorophenyl)methyl]-7-azabicyclo[2.2.1]hept an-2-yl]carbamate | Yield: 99% |
|---|---|---|---|---|
| 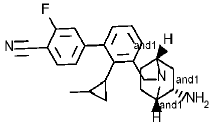 | 1H NMR (500 MHz, DMSO-d6) δ ppm 0.14 (dq, J=9.84, 4.38 Hz, 1 H) 0.29 - 0.52 (m, 2 H) 0.89 (d, J=5.87 Hz, 3 H) 0.96 (dd, J=12.23, 4.16 Hz, 1 H) 1.41 - 1.54 (m, 1 H) 1.71 - 1.95 (m, 4 H) 2.07 - 2.15 (m, 1 H) 3.25 (q, J=4.40 Hz, 1 H) 3.28 (q, J=4.16 Hz, 1 H) 3.45 (br dd, J=1 0.03, 4.65 Hz, 1 H) 3.42 - 3.66 (m, 3 H) 3.74 (s, 2 H) 3.76 - 3.82 (m, 2 H) 5.45 - 8.10 (m, 3 H) 7.14 (dd, J=7.58, 1.22 Hz, 1 H) 7.30 (t, J=7.70 Hz, 1 H) 7.44 (dd, J=7.95, 1.59 Hz, 1 H) 7.54 - 7.62 (m, 1 H) 7.95 - 8.01 (m, 1 H). m/z: 376.3 [M+H]+ | | | |
| 3'-{[(1S,2S,4R)-2-amino-7-azabicyclo[2.2.1]heptan-7-yl]methyl}-3-fluoro-2'-(2-methylcyclopropyl)-[1,1'-biphenyl]-4-carbonitrile | | | | |

| Example 306 | Procedure: B2+A1.1+C1 | SM1: 3-bromo-2-methylbenzaldehyde | SM2: tert-butyl N-[(1S,2S,4R)-7-azabicyclo[2.2.1]hept an-2-yl]carbamate | Yield: 73% |
|---|---|---|---|---|
| 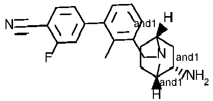 | 1H NMR (500 MHz, DMSO-d6) δ ppm 7.99 (dd, J=8.1, 7.1 Hz, 1 H), 7.53 (dd, J=10.3, 1.5 Hz, 1 H), 7.42 (d, J=6.8 Hz, 1 H), 7.36 (dd, J=8.1, 1.5 Hz, 1 H), 7.26 (t, J=7.6 Hz, 1 H), 7.14 (dd, J=7.7, 1.1 Hz, 1 H), 3.78 (s, 1 H), 3.58 (br d, J=3.2 Hz, 4 H), 3.42 (m, 3 H), 3.29 (br t, J=3.9 Hz, 2 H), 3.23 (br t, J=4.6 Hz, 2 H), 2.21 (s, 3 H), 2.07 (m, 1 H), 1.90 (m, 1 H), 1.76 (m, 2 H), 1.48 (m, 1 H), 1.02 (dd, J=12.5, 3.9 Hz, 1 H). m/z: 336 [M+H]+ | | | |
| 3'-{[(1S,2S,4R)-2-amino-7-azabicyclo[2.2.1]heptan-7-yl]methyl}-3-fluoro-2'-methyl-[1,1'-biphenyl]-4-carbonitrile | | | | |

| Example 218 | Procedure: D3+A5+A2.2+C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 72% |
|---|---|---|---|---|
| 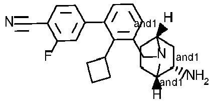 | 1H NMR (500 MHz, DMSO-d6) δ ppm 9.57 - 13.74 (m, 3 H) 7.84 - 8.00 (m, 1 H) 7.41 - 7.59 (m, 2 H) 7.33 (dd, J=8.07, 1.47 Hz, 1 H) 7.22 (t, J=7.58 Hz, 1 H) 7.01 (dd, J=7.58, 1.47 Hz, 1 H) 3.92 - 4.06 (m, 1 H) 3.58 (s, 2 H) 3.44 - 3.47 (m, 1 H) 3.18 - 3.24 (m, 2 H) 2.03 - 2.20 (m, 1 H) 1.56 - 2.01 (m, 8 H) 1.38 - 1.52 (m, 2 H) 0.94 - 1.03 (m, 1 H). m/z: 376 [M+H]+ | | | |
| 3'-{[(1S,2S,4R)-2-amino-7-azabicyclo[2.2.1]heptan-7-yl]methyl}-2'-cyclobutyl-3-fluoro-[1 ,1'-biphenyl]-4-carbonitrile | | | | |

| Example 305 | Procedure: B2+A1.1+C1 | SM1: 3-bromo-4-chlorobenzaldehyde | SM2: tert-butyl N-[(1S,2S,4R)-7-azabicyclo[2.2.1]hept an-2-yl]carbamate | Yield: 68% |
|---|---|---|---|---|
| 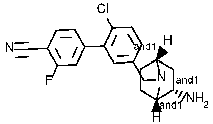 | 1H NMR (DMSO-d6, 500 MHz) δ 8.03 (dd, 1H, J=7.1, 7.8 Hz), 7.67 (dd, 1H, J=1.5, 10.3 Hz), 7.55 (d, 1H, J=8.3 Hz), 7.50 (dd, 1H, J=1.6, 7.9 Hz), 7.4-7.5 (m, 1H), 7.41 (d, 1H, J=2.0 Hz), 3.73 (s, 1H), 3.5-3.6 (m, 2H), 3.2-3.5 (m, 7H), 3.0-3.1 (m, 2H), 1.9-2.1 (m, 1H), 1.7-1.9 (m, 2H), 1.5-1.7 (m, 1H), 1.3-1.4 (m, 1H), 0.75 (dd, 1H, J=4.4, 12.0 Hz). m/z: 356 [M+H]+ | | | |
| 5'-{[(1S,2S,4R)-2-amino-7-azabicyclo[2.2.1]heptan-7-yl]methyl}-2'-chloro-3-fluoro-[1 ,1'-biphenyl]-4-carbonitrile | | | | |

| Example 246 | Procedure: D3+O1+A2.2+C1 | SM1: 1-bromo-4-(bromomethyl)-2-chlorobenzene | SM2: tert-butyl N-[(1S,2S,4R)-7-azabicyclo[2.2.1]hept an-2-yl]carbamate | Yield: 95% |
|---|---|---|---|---|
| 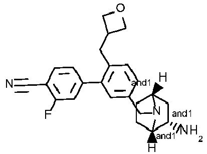 | 1H NMR (DMSO-d6, 600 MHz): δ (ppm) 7.97-8.04 (m, 1H), 7.56 (dd, J = 10.3, 1.2 Hz, 1H), 7.38 (dd, J = 7.8, 1.4 Hz, 1H), 7.34 (dd, J = 8.0, 1.7 Hz, 1H), 7.19 (d, J = 8.1 Hz, 1H), 7.16 (d, J = 1.6 Hz, 1H), 6.79-8.41 (m, 2H), 4.52 (dd, J = 7.8, 5.9 Hz, 2H), 4.11-4.18 (m, 2H), 3.83 (br s, 2H), 3.51-3.57 (m, 2H), 3.46-3.48 (m, 1H), 3.30 (br d, J = 4.7 Hz, 2H), 3.20-3.23 (m, 3H), 3.02-3.11 (m, 1H), 2.90-2.96 (m, 2H), 1.39-2.14 (m, 6H), 0.98-1.13 (m, 1H). m/z: 392.3 [M+H]+ | | | |
| 5'-{[(1S,2S,4R)-2-amino-7-azabicyclo[2.2.1]heptan-7-yl]methyl}-3-fluoro-2'-[(oxetan-3-yl)methyl]-[1,1'-biphenyl]-4-carbonitrile | | | | |

| Example 304 | Procedure: B2+A1.1+A2.2+ C1 | SM1: 2-bromo-3-chloro-5-methylbenzaldehyde | SM2: tert-butyl N-[(1S,2S,4R)-7-azabicyclo[2.2.1]hept an-2-yl]carbamate | Yield: 84% |
|---|---|---|---|---|
| 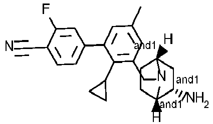 | 1H NMR (500 MHz, DMSO-d6) δ 8.00 - 7.84 (m, 1H), 7.57 (br d, J = 10.5 Hz, 1H), 7.44 (br d, J = 7.8 Hz, 1H), 7.36 (s, 1H), 6.98 (s, 1H), 6.86 - 6.30 (m, 3H), 3.73 (br s, 2H), 3.47 - 3.45 (m, 1H), 3.29 (br s, 2H), 2.35 - 2.28 (m, 3H), 2.15 - 2.06 (m, 1H), 2.03 - 1.95 (m, 1H), 1.93 - 1.84 (m, 1H), 1.83 - 1.70 (m, 2H), 1.52 - 1.42 (m, 1H), 1.01 - 0.91 (m, 1H), -0.06 (br s, 4H). m/z: 376 [M+H]+ | | | |
| 3'-{[(1S,2S,4R)-2-amino-7-azabicyclo[2.2.1]heptan-7-yl]methyl}-2'-cyclopropyl-3-fluoro-5'-methyl-[1,1'-biphenyl]-4-carbonitrile | | | | |

| Example 180 | Procedure: B2+A1.1+C1 | SM1: 3-bromo-2-methoxybenzaldehyd e | SM2: tert-butyl N-[(1S,2S,4R)-7-azabicyclo[2.2.1]hept an-2-yl]carbamate | Yield: 82% |
|---|---|---|---|---|
| 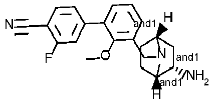 | 1H NMR (600 MHz, DMSO-d6) δ ppm 8.00 (dd, J=7.8, 7.3 Hz, 1 H), 7.70 (dd, J=10.8, 1.4 Hz, 1 H), 7.61 (dd, J=8.1, 1.5 Hz, 1 H), 7.53 (dd, J=7.6, 1.6 Hz, 1 H), 7.35 (dd, J=7.6, 1.8 Hz, 1 H), 7.24 (t, J=7.6 Hz, 1 H), 3.74 (s, 2 H), 3.61 (m, 2 H), 3.31 (m, 6 H), 1.91 (m, 5 H), 1.44 (br d, J=4.1 Hz, 1 H). m/z: 352 [M+H]+ | | | |
| 3'-{[(1S,2S,4R)-2-amino-7-azabicyclo[2.2.1]heptan-7-yl]methyl}-3-fluoro-2'-methoxy-[1 ,1'-biphenyl]-4-carbonitrile | | | | |

| Example 245 | Procedure: B2+F3+A1.1+C1 | SM1: 3-bromo-2-hydroxybenzaldehyde | SM2: tert-butyl N-[(1S,2S,4R)-7-azabicyclo[2.2.1]hept an-2-yl]carbamate | Yield: 88% |
|---|---|---|---|---|
| 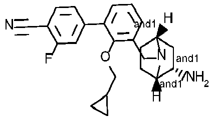 | 1H NMR (500 MHz, DMSO-d6) δ 8.03 - 7.97 (m, 1H), 7.72 (dd, J = 11.0, 1.5 Hz, 1H), 7.62 (dd, J = 8.1, 1.5 Hz, 1H), 7.51 (dd, J = 7.6, 1.7 Hz, 1H), 7.36 - 7.32 (m, 1H), 7.27 - 7.17 (m, 1H), 7.04 (br s, 3H), 3.69 - 3.57 (m, 2H), 3.41 - 3.35 (m, 1H), 3.31 (dd, J = 7.0, 3.1 Hz, 2H), 3.28 (br t, J = 4.2 Hz, 1H), 3.24 (t, J = 4.8 Hz, 1H), 2.13 - 1.65 (m, 4H), 1.53 - 1.34 (m, 1H), 1.03 - 0.83 (m, 2H), 0.53 - -0.11 (m, 4H). m/z: 392 [M+H]+ | | | |
| 3'-{[(1S,2S,4R)-2-amino-7-azabicyclo[2.2.1]heptan-7-yl]methyl}-2'-(cyclopropylmethoxy)-3-fluoro-[1,1'-biphenyl]-4-carbonitrile | | | | |

| Example 303 | Procedure: D3+A2.1+A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 66% |
|---|---|---|---|---|
| 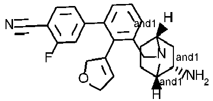 | 1H NMR (DMSO-d6, 600 MHz) δ 7.9-8.0 (m, 1H), 7.5-7.6 (m, 2H), 7.3-7.5 (m, 2H), 7.26 (dd, 1H, J=1.0, 7.6 Hz), 5.95 (t, 1H, J=1.8 Hz), 4.5-4.6 (m, 2H), 4.22 (br t, 2H, J=3.7 Hz), 3.74 (br d, 1H, J=2.6 Hz), 3.54 (q, 3H, J=13.7 Hz), 2.8-3.5 (m, 9H), 1.9-2.1 (m, 1H), 1.80 (br d, 2H, J=6.3 Hz), 1.6-1.7 (m, 1H), 1.3-1.4 (m, 1H), 0.85 (brdd, 1H, J=4.1, 12.5 Hz). m/z: 390 [M+H]+ | | | |
| 3'-{[(1S,2S,4R)-2-amino-7-azabicyclo[2.2.1]heptan-7-yl]methyl}-2'-(2,5-dihydrofuran-3-yl)-3-fluoro-[1,1'-biphenyl]-4-carbonitrile | | | | |

| Example 244 | Procedure: D3+A2.1+A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 83% |
|---|---|---|---|---|
| 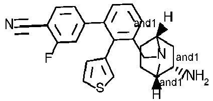 | 1H NMR (DMSO-d6, 500 MHz) +0.6 mol of tartrate: δ (ppm) 7.74-7.77 (m, 1H), 7.63-7.66 (m, 1H), 7.45-7.50 (m, 2H), 7.35 (dd, J = 7.6, 1.2 Hz, 1H), 7.26 (dd, J = 2.9, 1.2 Hz, 1H), 7.20 (dd, J = 10.8, 1.5 Hz, 1H), 7.09 (dd, J = 8.1, 1.5 Hz, 1H), 6.86 (dd, J = 4.9, 1.2 Hz, 1H), 3.84 (s, 1H), 3.57 (s, 1H), 3.35-3.42 (m, 3H), 3.24 (br t, J = 4.3 Hz, 1H), 3.13 (br t, J = 4.5 Hz, 1H), 1.91-2.00 (m, 1H), 1.54-1.64 (m, 2H), 1.50 (br d, J = 12.0 Hz, 1H), 1.33-1.40 (m, 1H), 1.02 (dd, J = 12.7, 4.2 Hz, 1H). m/z: 404 [M+H]+ | | | |
| 3'-{[(1S,2S,4R)-2-amino-7-azabicyclo[2.2.1]heptan-7-yl]methyl}-3-fluoro-2'-(thiophen-3-yl)-[1,1'-biphenyl]-4-carbonitrile | | | | |

| Example 243 | Procedure: D3+A2.1+A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 60% |
|---|---|---|---|---|
| 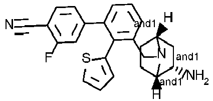 | 1H NMR (DMSO-d6, 600 MHz) δ 7.7-7.8 (m, 1H), 7.69 (dd, 1H, J=0.7, 7.8 Hz), 7.5-7.6 (m, 2H), 7.35 (dd, 1H, J=1.1, 7.7 Hz), 7.29 (dd, 1H, J=1.4, 10.6 Hz), 7.16 (dd, 1H, J=1.5, 8.1 Hz), 7.00 (dd, 1H, J=3.4, 5.1 Hz), 6.96 (dd, 1H, J=1.0, 3.4 Hz), 3.74 (s, 1H), 3.1-3.6 (m, 8H), 3.1-3.1 (m, 3H), 1.9-2.0 (m, 1H), 1.73 (br t, 1H, J=4.1 Hz), 1.5-1.6 (m, 1H), 1.4-1.5 (m, 1H), 1.3-1.4 (m, 1H), 0.82 (dd, 1H, J=4.3, 12.0 Hz), m/z: 404 [M+H]+ | | | |
| 3'-{[(1S,2S,4R)-2-amino-7-azabicyclo[2.2.1]heptan-7-yl]methyl}-3-fluoro-2'-(thiophen-2-yl)-[1,1'-biphenyl]-4-carbonitrile | | | | |

| Example 207 | Procedure: B1+A1.1+A2.2+ C1 | SM1: 2-bromo-3-chloro-6-fluorobenzaldehyde | SM2: tert-butyl N-[(1S,2S,4R)-7-azabicyclo[2.2.1]hept an-2-yl]carbamate | Yield: 71% |
|---|---|---|---|---|
| 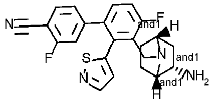 | 1H NMR (DMSO-d6, 600 MHz) δ 8.43 (d, 1H, J=1.8 Hz), 7.79 (dd, 1H, J=7.1, 7.8 Hz), 7.4-7.5 (m, 2H), 7.35 (dd, 1H, J=1.5, 10.4 Hz), 7.21 (d, 1H, J=1.8 Hz), 7.12 (dd, 1H, J=1.6, 8.1 Hz), 3.75 (s, 1H), 2.8-3.6 (m, 10H), 1.98 (tt, 1H, J=2.5, 10.3 Hz), 1.7-1.9 (m, 2H), 1.5-1.7 (m, 1H), 1.3-1.5 (m, 1H), 0.93 (br dd, 1H, J=3.8, 12.3 Hz), m/z: 423 [M+H]+ | | | |
| 3'-{[(1S,2S,4R)-2-amino-7-azabicyclo[2.2.1]heptan-7-yl]methyl}-3,4'-difluoro-2'-(1 ,2-thiazol-5-yl)-[1 ,1'-biphenyl]-4-carbonitrile | | | | |

| Example 195 | Procedure: A1.1 +A2.2+B1 + D1+C1 | SM1: 2-bromo-3-chlorobenzaldehyde | SM2: cyclopropylboronic acid | Yield: 94% |
|---|---|---|---|---|
| 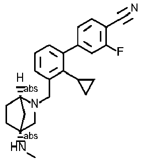 | 1H NMR (DMSO-d6, 600 MHz) δ 7.95 (t, 1H, J=7.5 Hz), 7.58 (dd, 1H, J=1.2, 10.6 Hz), 7.50 (d, 1H, J=7.3 Hz), 7.45 (dd, 1H, J=1.3, 8.1 Hz), 7.32 (t, 1H, J=7.6 Hz), 7.1-7.2 (m, 1H), 3.9-4.0 (m, 2H), 3.80 (s, 1H), 3.0-3.7 (m, 6H), 2.90 (br dd, 1H, J=3.2, 8.5 Hz), 2.41 (s, 3H), 2.29 (br d, 1H, J=8.2 Hz), 1.9-2.1 (m, 2H), 1.77 (br d, 1H, J=7.6 Hz), 1.6-1.7 (m, 2H), 1.4-1.6 (m, 2H), 0.72 (br d, 2H, J=8.5 Hz), -0.1-0.1 (m, 2H). m/z: 376 [M+H]+ | | | |
| 2'-cyclopropyl-3-fluoro-3'-{[(1R,4R)-4-(methylamino)-2-azabicyclo[2.2.1]heptan-2-yl]methyl}-[1,1'-biphenyl]-4-carbonitrile | | | | |

| Example 198 | Procedure: D3+A1 . 1 +A2.2+ C1 | SM1: 2-bromo-1-(bromomethyl)-3-chlorobenzene | SM2: tert-butyl N-(4,4-dimethylpyrrolidin-3-yl)carbamate | Yield: 82% |
|---|---|---|---|---|
| 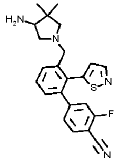 | 1H NMR (DMSO-d6, 600 MHz) δ 8.45 (d, 1H, J=1.2 Hz), 7.80 (t, 1H, J=7.5 Hz), 7.6-7.7 (m, 1H), 7.5-7.6 (m, 1H), 7.42 (d, 1H, J=7.5 Hz), 7.35 (dd, 1H, J=1.5, 10.4 Hz), 7.22 (s, 1H), 7.13 (dd, 1H, J=1.5, 8.1 Hz), 3.79 (br s, 3H), 2.8-3.7 (m, 9H), 2.2-2.4 (m, 3H), 1.0-1.1 (m, 6H). m/z: 407 [M+H]+ | | | |
| 3'-[(4-amino-3,3-dimethylpyrrolidin-1-yl)methyl]-3-fluoro-2'-(1,2-thiazol-5-yl)-[1,1'-biphenyl]-4-carbonitrile | | | | |

| Example 188 | Procedure: K1+E1+L1+A2.2 +C3+I1 +B1+C1 | SM1: 2-bromo-3-chlorobenzaldehyde | SM2: 4-chloro-1 ,3-thiazole | Yield: 70% |
|---|---|---|---|---|
| 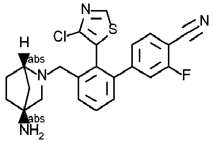 | 1H NMR (DMSO-d6, 500 MHz) δ 9.13 (d, 1H, J=1.5 Hz), 7.8-8.0 (m, 1H), 7.6-7.7 (m, 2H), 7.4-7.5 (m, 1H), 7.32 (ddd, 1H, J=1.5, 4.8, 10.4 Hz), 7.13 (ddd, 1H, J=1.5, 4.2, 8.0 Hz), 3.80 (s, 1H), 3.0-3.7 (m, 8H), 2.91 (br d, 1H, J=7.1 Hz), 2.6-2.8 (m, 1H), 2.1-2.3 (m, 1H), 1.3-1.9 (m, 6H). m/z: 439 [M+H]+ | | | |
| 3'-{[(1S,4S)-4-amino-2-azabicyclo[2.2.1]heptan-2-yl]methyl}-2'-(4-chloro-1,3-thiazol-5-yl)-3-fluoro-[1,1'-biphenyl]-4-carbonitrile | | | | |

| Example 182 | Procedure: D3+A1.1+D1+A2 .2+C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 95% |
|---|---|---|---|---|
| 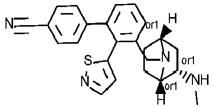 | 1H NMR (DMSO-d6, 600 MHz) δ 8.40 (d, 1H, J=1.6 Hz), 7.71 (d, 2H, J=8.2 Hz), 7.62 (dd, 1H, J=1.1, 7.7 Hz), 7.56 (t, 1H, J=7.6 Hz), 7.37 (dd, 1H, J=1.2, 7.6 Hz), 7.3-7.3 (m, 2H), 7.18 (d, 1H, J=1.8 Hz), 3.79 (s, 1H), 3.0-3.7 (m, 10H), 2.33 (s, 3H), 1.9-2.0 (m, 1H), 1.70 (s, 3H), 1.3-1.4 (m, 1H), 0.8-0.9 (m, 1H). m/z: 401 [M+H]+ | | | |
| 3'-{[(1S,2S,4R)-2-(methylamino)-7-azabicyclo[2.2.1]heptan-7 -yl]methyl}-2'-(1 ,2-thiazol-5-yl)-[1 ,1'-biphenyl]-4-carbonitrile | | | | |

| Example 173 | Procedure: D3+A1.1+D1+A2 .2+C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 84% |
|---|---|---|---|---|
| 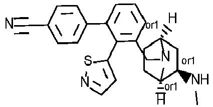 | 1H NMR (600 MHz, DMSO-d6) δ ppm 0.92 (br dd, J=12.10, 2.86 Hz, 1 H) 1.30 - 1.42 (m, 1 H) 1.49 - 1.61 (m, 1 H) 1.69 (br dd, J=10.34, 3.59 Hz, 2 H) 1.90 - 2.08 (m, 1 H) 2.36 (s, 3 H) 3.10 (t, J=4.55 Hz, 1 H) 3.15 - 3.20 (m, 1 H) 3.23 (br t, J=4.25 Hz, 1 H) 3.34 - 3.41 (m, 4 H) 3.48 - 3.65 (m, 2 H) 3.80 (s, 1 H) 6.69 - 8.14 (m, 2 H) 7.18 (d, J=1.61 Hz, 1 H) 7.32 (d, J=8.36 Hz, 2 H) 7.37 (dd, J=7.63, 1.17 Hz, 1 H) 7.56 (t, J=7.70 Hz, 1 H) 7.62 (dd, J=7.78, 1.03 Hz, 1 H) 7.71 (d, J=8.22 Hz, 1 H) 8.41 (d, J=1.61 Hz, 1 H) 11.13 -12.58 (m, 1 H). m/z: 401 [M+H]+ | | | |
| 3'-{[(1R,2R,4S)-2-(methylamino)-7-azabicyclo[2.2.1]heptan-7-yl]methyl}-2'-(1,2-thiazol-5-yl)-[1,1'-biphenyl]-4-carbonitrile | | | | |

| Example 190 | Procedure: D3+A1.1+D1+A2 .2+C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 90% |
|---|---|---|---|---|
| 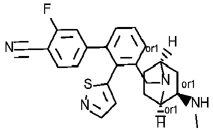 | 1H NMR (DMSO-d6, 600 MHz) δ 8.43 (d, 1H, J=1.8 Hz), 7.79 (dd, 1H, J=7.2, 7.8 Hz), 7.64 (dd, 1H, J=1.1, 7.7 Hz), 7.57 (t, 1H, J=7.7 Hz), 7.41 (dd, 1H, J=1.2, 7.7 Hz), 7.35 (dd, 1H, J=1.5, 10.4 Hz), 7.21 (d, 1H, J=1.6 Hz), 7.14 (dd, 1H, J=1.5, 8.0 Hz), 3.79 (s, 2H), 2.7-3.7 (m, 9H), 2.34 (s, 3H), 1.9-2.0 (m, 1H), 1.6-1.8 (m, 2H), 1.55 (br s, 1H), 1.3-1.4 (m, 1H), 0.8-0.9 (m, 1H). m/z: 419 [M+H]+ | | | |
| 3-fluoro-3'-{[(1 R,2R,4S)-2-(methylamino)-7-azabicyclo[2.2.1]heptan-7 -yl]methyl}-2'-(1 ,2-thiazol-5-yl)-[1,1'-biphenyl]-4-carbonitrile | | | | |

| Example 302 | Procedure: D3+A1.1 +A2.2+ C1 | SM1: 2-bromo-1-(bromomethyl)-3-chlorobenzene | SM2: tert-butyl N-[(3S)-3-methylpyrrolidin-3-yl]carbamate | Yield: 100% |
|---|---|---|---|---|
| 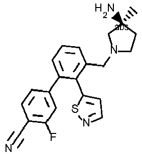 | 1H NMR (600 MHz, DMSO-d6) δ ppm 8.48 (d, J=1.8 Hz, 1 H), 7.92 (m, 4 H), 7.72 (d, J=7.2 Hz, 1 H), 7.60 (t, J=7.7 Hz, 1 H), 7.42 (m, 1 H), 7.35 (dd, J=10.3, 1.4 Hz, 1 H), 7.29 (d, J=1.6 Hz, 1 H), 7.15 (dd, J=8.1, 1.3 Hz, 1 H), 3.43 (d, J=2.6 Hz, 2 H), 2.73 (br d, J=5.6 Hz, 1 H), 2.63 (d, J=9.8 Hz, 1 H), 2.43 (br d, J=6.0 Hz, 1 H), 2.33 (d, J=9.8 Hz, 1 H), 1.90 (s, 1 H), 1.82 (s, 1 H), 1.33 (s, 3 H). m/z: 393 [M+H]+ | | | |
| 3'-{[(3S)-3-amino-3-methylpyrrolidin-1-yl]methyl}-3-fluoro-2'-(1,2-thiazol-5-yl)-[1,1'-biphenyl]-4-carbonitrile | | | | |

| Example 242 | Procedure: D3+A1.1+A2.2+ C1 | SM1: 2-bromo-1-(bromomethyl)-3-chlorobenzene | SM2: tert-butyl N-[(3R)-3-methylpyrrolidin-3-yl]carbamate | Yield: 83% |
|---|---|---|---|---|
| 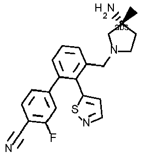 | 1H NMR (DMSO-d6, 600 MHz) δ 8.48 (d, 1H, J=1.6 Hz), 7.8-8.1 (m, 4H), 7.72 (dd, 1H, J=1.0, 7.8 Hz), 7.60 (t, 1H, J=7.7 Hz), 7.43 (dd, 1H, J=1.1, 7.7 Hz), 7.35 (dd, 1H, J=1.4, 10.3 Hz), 7.29 (d, 1H, J=1.8 Hz), 7.15 (dd, 1H, J=1.5, 8.1 Hz), 3.92 (br s, 1H), 2.8-3.7 (m, 5H), 2.72 (dt, 1H, J=5.7, 8.7 Hz), 2.64 (d, 1H, J=9.8 Hz), 2.4-2.5 (m, 1H), 2.34 (d, 1H, J=9.8 Hz), 1.8-2.0 (m, 2H), 1.33 (s, 3H). m/z: 393 [M+H]+ | | | |
| 3'-{[(3R)-3-amino-3-methylpyrrolidin-1-yl]methyl}-3-fluoro-2'-(1,2-thiazol-5-yl)-[1,1'-biphenyl]-4-carbonitrile | | | | |

| Example 204 | Procedure: D3+A1 . 1 +A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 60% |
|---|---|---|---|---|
| 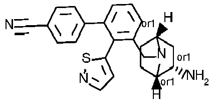 | 1H NMR (600 MHz, DMSO-d6) δ ppm 8.40 (d, J=1.6 Hz, 1 H), 7.71 (m, 2 H), 7.62 (m, 1 H), 7.55 (t, J=7.6 Hz, 1 H), 7.38 (dd, J=7.6, 1.0 Hz, 1 H), 7.32 (m, 2 H), 7.17 (d, J=1.6 Hz, 1 H), 3.40 (m, 3 H), 3.09 (m, 2 H), 2.00 (m, 1 H), 1.53 (m, 4 H), 0.90 (m, 1 H). m/z: 387 [M+H]+ | | | |
| 3'-{[(1S,2S,4R)-2-amino-7-azabicyclo[2.2.1]heptan-7-yl]methyl}-2'-(1 ,2-thiazol-5-yl)-[1 ,1'-biphenyl]-4-carbonitrile | | | | |

| Example 187 | Procedure: D3+A1.1 +A2.2+ C1 | SM1: 2-bromo-1-(bromomethyl)-3-chlorobenzene | SM2: tert-butyl N-[(1S,2S,4R)-7-azabicyclo[2.2.1]hept an-2-yl]carbamate | Yield: 11% |
|---|---|---|---|---|
| 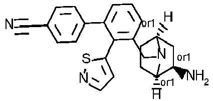 | 1H NMR (600 MHz, DMSO-d6) δ ppm 1.05 (dd, J=12.47, 4.25 Hz, 1 H) 1.38 - 1.46 (m, 1 H) 1.57 - 1.77 (m, 3 H) 1.97 - 2.07 (m, 1 H) 3.12 - 3.16 (m, 1 H) 3.21 - 3.24 (m, 1 H) 3.39 - 3.41 (m, 6 H) 4.11 (br dd, J=2.93, 0.88 Hz, 2 H) 7.18 (d, J=1.61 Hz, 1 H) 7.32 (d, J=8.36 Hz, 2 H) 7.39 (dd, J=7.63, 1.17 Hz, 1 H) 7.56 (t, J=7.63 Hz, 1 H) 7.58 - 8.04 (m, 3 H) 7.59 - 7.64 (m, 1 H) 7.71 (d, J=8.36 Hz, 2 H) 8.41 (d, J=1.61 Hz, 1 H). m/z: 387 [M+H]+ | | | |
| 3'-{[(1R,2R,4S)-2-amino-7-azabicyclo[2.2.1]heptan-7-yl]methyl}-2'-(1 ,2-thiazol-5-yl)-[1 ,1'-biphenyl]-4-carbonitrile | | | | |

| Example 241 | Procedure: H3+B2+C3+F4+ A2.2+C2 | SM1: 2-bromo-3-chlorobenzaldehyde | SM2: 4-[(tertbutyldimethylsilyl)oxy] piperidine | Yield: 100% |
|---|---|---|---|---|
| 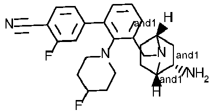 | 1H NMR (600 MHz, DMSO-d6) δ ppm 11.38 (s, 1 H) 8.45 - 9.05 (m, 3 H) 7.86 - 8.09 (m, 2 H) 7.42 - 7.73 (m, 1 H) 7.28 - 7.41 (m, 2 H) 7.04 - 7.26 (m, 1 H) 4.47 - 5.04 (m, 1 H) 4.28 - 4.45 (m, 2 H) 3.70 - 4.27 (m, 3 H) 2.62 - 3.03 (m, 4 H) 2.08 - 2.44 (m, 2 H) 1.95 - 2.05 (m, 2 H) 1.78 - 1.92 (m, 2 H) 1.35 - 1.75 (m, 4 H). m/z: 423 [M+H]+ | | | |
| 3'-{[(1S,2S,4R)-2-amino-7-azabicyclo[2.2.1]heptan-7-yl]methyl}-3-fluoro-2'-(4-fluoropiperidin-1-yl)-[1,1'-biphenyl]-4-carbonitrile | | | | |

| Example 301 | Procedure: S1+B2+A2.2+C1 | SM1: 1-bromo-3-chloro-2-(difluoromethyl)benze ne | SM2: tert-butyl N-[(1S,2S,4R)-7-azabicyclo[2.2.1]hept an-2-yl]carbamate | Yield: 73% |
|---|---|---|---|---|
| 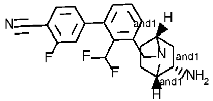 | 1H NMR (500 MHz, DMSO-d6) δ ppm 0.95 (dd, J=12.23, 4.16 Hz, 1 H) 1.37 - 1.53 (m, 1 H) 1.66 - 1.92 (m, 3 H) 1.98 - 2.11 (m, 1 H) 3.21 (t, J=4.65 Hz, 1 H) 3.24 (t, J=4.28 Hz, 1 H) 3.33 - 3.43 (m, 1 H) 3.41 - 3.69 (m, 3 H) 3.76 (s, 2 H) 3.79 (d, J=1.71 Hz, 2 H) 5.13 - 7.39 (m, 3 H) 7.08 - 7.24 (m, 1 H) 7.27 (d, J=7.34 Hz, 1 H) 7.35 (dd, J=8.07, 1.47 Hz, 1 H) 7.50 (dd, J=10.27, 1.47 Hz, 1 H) 7.58 (t, J=7.70 Hz, 1 H) 7.67 (d, J=7.83 Hz, 1 H) 7.94 - 8.03 (m, 1 H). m/z: 372 [M+H]+ | | | |
| 3'-{[(1S,2S,4R)-2-amino-7-azabicyclo[2.2.1]heptan-7-yl]methyl}-2'-(difluoromethyl)-3-fluoro-[1,1'-biphenyl]-4-carbonitrile | | | | |

| Example 179 | Procedure: B2+D2+A1.1+C1 | SM1: 3-bromo-2-hydroxybenzaldehyde | SM2: tert-butyl N-[(1S,2S,4R)-7-azabicyclo[2.2.1]hept an-2-yl]carbamate | Yield: 86% |
|---|---|---|---|---|
| 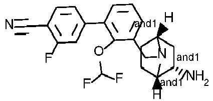 | 1H NMR (DMSO-d6, 600 MHz) δ 8.01 (dd, 1H, J=7.3, 7.8 Hz), 7.65 (dd, 1H, J=1.5, 10.6 Hz), 7.61 (dd, 1H, J=3.3, 6.1 Hz), 7.52 (dd, 1H, J=1.5, 8.1 Hz), 7.3-7.5 (m, 2H), 6.85 (t, 1H, J=74.3 Hz), 3.73 (s, 1H), 2.7-3.7 (m, 10H), 2.0-2.1 (m, 1H), 1.8-1.9 (m, 2H), 1.72 (br d, 1H, J=4.5 Hz), 1.4-1.5 (m, 1H), 0.7-0.9 (m, 1H). m/z: 388 [M+H]+ | | | |
| 3'-{[(1S,2S,4R)-2-amino-7-azabicyclo[2.2.1]heptan-7-yl]methyl}-2'-(difluoromethoxy)-3-fluoro-[1,1'-biphenyl]-4-carbonitrile | | | | |

| Example 223 | Procedure: A1.1+A2.2+B2+ C1 | SM1: 2-bromo-3-chloro-5-fluorobenzaldehyde | SM2: cyclopropylboronic acid | Yield: 81% |
|---|---|---|---|---|
| 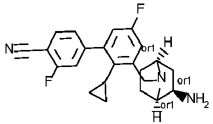 | 1H NMR (600 MHz, DMSO-d6) δ ppm -0.06 (q, J=5.14 Hz, 2 H) 0.68 - 0.72 (m, 2 H) 1.07 (dd, J=12.47, 4.11 Hz, 1 H) 1.47 - 1.53 (m, 1 H) 1.73 - 1.84 (m, 2 H) 1.85 - 1.93 (m, 1 H) 1.93 - 2.00 (m, 1 H) 2.12 - 2.19 (m, 1 H) 3.31 (br t, J=4.70 Hz, 1 H) 3.38 (br t, J=4.25 Hz, 1 H) 3.46 - 3.62 (m, 1 H) 3.75 - 3.85 (m, 2 H) 7.05 (dd, J=9.24, 2.93 Hz, 1 H) 7.42 (dd, J=9.98, 2.79 Hz, 1 H) 7.48 (dd, J=8.00, 1.39 Hz, 1 H) 7.64 (d, J=10.61 Hz, 1 H) 7.98 (t, J=7.48 Hz, 1 H). m/z: 380.3 [M+H]+ | | | |
| 3'-{[(1R,2R,4S)-2-amino-7-azabicyclo[2.2.1]heptan-7-yl]methyl}-2'-cyclopropyl-3,5'-difluoro-[1,1'-biphenyl]-4-carbonitrile | | | | |

| Example 183 | Procedure: B2+D2+A1.1+C1 | SM1: 3-bromo-2-hydroxybenzaldehyde | SM2: tert-butyl N-[(1S,2S,4R)-7-azabicyclo[2.2.1]hept an-2-yl]carbamate | Yield: 87% |
|---|---|---|---|---|
| 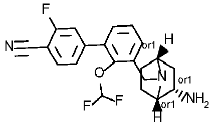 | 1H NMR (DMSO-d6, 600 MHz) δ 8.01 (t, 1H, J=7.5 Hz), 7.65 (dd, 1H, J=1.2, 10.6 Hz), 7.62 (dd, 1H, J=3.2, 6.2 Hz), 7.52 (dd, 1H, J=1.3, 8.1 Hz), 7.4-7.5 (m, 2H), 6.84 (t, 1H, J=74.2 Hz), 3.75 (s, 1H), 3.6-3.7 (m, 4H), 2.7-3.5 (m, 8H), 2.0-2.2 (m, 1H), 1.7-1.9 (m, 3H), 1.4-1.5 (m, 1H), 0.92 (br d, 1H, J=12.0 Hz). m/z: 387 [M+H]+ | | | |
| 3'-{[(1S,2S,4R)-2-amino-7-azabicyclo[2.2.1]heptan-7-yl]methyl}-2'-(difluoromethoxy)-3-fluoro-[1,1'-biphenyl]-4-carbonitrile | | | | |

| Example 197 | Procedure: D3+A1.1+A2.2+ C1 | SM1: 2-bromo-1-(bromomethyl)-3-chlorobenzene | SM2: tert-butyl N-(4,4-dimethylpyrrolidin-3-yl)carbamate | Yield: 84% |
|---|---|---|---|---|
| 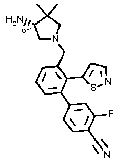 | 1H NMR (600 MHz, DMSO-d6) δ ppm 0.96 (s, 3 H) 1.01 (s, 3 H) 2.24 - 2.35 (m, 3 H) 2.85 (dd, J=9.68, 7.63 Hz, 1 H) 3.03 - 3.07 (m, 1 H) 3.34 - 3.45 (m, 5 H) 3.74 (s, 2 H) 4.40 - 6.70 (m, 3 H) 7.13 (dd, J=8.00, 1.25 Hz, 1 H) 7.20 (d, J=1.61 Hz, 1 H) 7.35 (d, J=10.42 Hz, 1 H) 7.41 (d, J=7.48 Hz, 1 H) 7.55 - 7.59 (m, 1 H) 7.60 - 7.64 (m, 1 H) 7.79 (t, J=7.48 Hz, 1 H) 8.44 (d, J=1.47 Hz, 1 H). m/z: 407 [M+H]+ | | | |
| 3'-{[(4R)-4-amino-3,3-dimethylpyrrolidin-1-yl]methyl}-3-fluoro-2'-(1,2-thiazol-5-yl)-[1,1'-biphenyl]-4-carbonitrile | | | | |

| Example 194 | Procedure: D3+A1.1+A2.2+ C1 | SM1: 2-bromo-1-(bromomethyl)-3-chlorobenzene | SM2: tert-butyl N-(4,4-dimethylpyrrolidin-3-yl)carbamate | Yield: 80% |
|---|---|---|---|---|
| 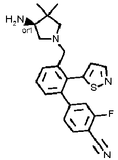 | 1H NMR (600 MHz, DMSO-d6) δ 8.44 (d, J = 1.5 Hz, 1H), 7.79 (t, J = 7.5 Hz, 1H), 7.64 - 7.60 (m, 1H), 7.60 - 7.53 (m, 1H), 7.41 (d, J = 7.5 Hz, 1H), 7.39 - 7.29 (m, 1H), 7.20 (d, J = 1.6 Hz, 1H), 7.13 (dd, J = 8.0, 1.1 Hz, 1H), 3.42 - 3.41 (m, 2H), 3.07 - 3.05 (m, 1H), 2.88 - 2.83 (m, 1H), 2.29 (s, 3H), 1.06 - 0.93 (m, 6H). m/z: 407 [M+H]+ | | | |
| 3'-{[(4S)-4-amino-3,3-dimethylpyrrolidin-1-yl]methyl}-3-fluoro-2'-(1,2-thiazol-5-yl)-[1,1'-biphenyl]-4-carbonitrile | | | | |

| Example 221 | Procedure: A6+Q1+A1.1+F1 +D3+C1 | SM1: (3-bromo-2-iodophenyl)methanol | SM2: 4,4,5,5-tetramethyl-2-(prop-2-en-1-yl)-1,3,2-dioxaborolane | Yield: 71% |
|---|---|---|---|---|
| 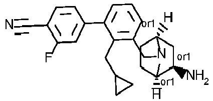 | 1H NMR (DMSO-d6, 500 MHz) δ 7.9-8.0 (m, 1H), 7.54 (dd, 1H, J=1.5, 10.3 Hz), 7.48 (dd, 1H, J=1.1, 7.7 Hz), 7.37 (dd, 1H, J=1.5, 8.1 Hz), 7.27 (t, 1H, J=7.7 Hz), 7.09 (dd, 1H, J=1.3, 7.7 Hz), 3.75 (s, 1H), 3.6-3.7 (m, 2H), 3.3-3.4 (m, 2H), 3.23 (td, 5H, J=4.5, 15.2 Hz), 2.66 (d, 2H, J=6.1 Hz), 2.0-2.1 (m, 1H), 1.7-2.0 (m, 3H), 1.4-1.5 (m, 1H), 0.94 (dd, 1H, J=4.0, 12.3 Hz), 0.5-0.7 (m, 1H), 0.2-0.3 (m, 2H), -0.2--0.2 (m, 2H). m/z: 376 [M+H]+ | | | |
| 3'-{[(1R,2R,4S)-2-amino-7-azabicyclo[2.2.1]heptan-7-yl]methyl}-2'-(cyclopropylmethyl)-3-fluoro-[1,1'-biphenyl]-4-carbonitrile | | | | |

| Example 81 | Procedure: H1.2+K3+A2.2+ C1 | SM1: 2-bromo-1-chloro-3-iodobenzene | SM2: tert-butyl N-methyl-N-(piperidin-4-yl)carbamate | Yield: 79% |
|---|---|---|---|---|
| 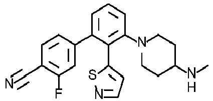 | 1H NMR (600 MHz, DMSO-d6) δ ppm 8.33 (d, J=1.8 Hz, 1 H), 7.83 (dd, J=7.9, 7.0 Hz, 1 H), 7.51 (m, 1 H), 7.39 (dd, J=8.2, 1.2 Hz, 1 H), 7.35 (dd, J=10.4, 1.5 Hz, 1 H), 7.15 (dd, J=7.6, 1.2 Hz, 1 H), 7.10 (dd, J=8.0, 1.5 Hz, 1 H), 6.81 (d, J=1.8 Hz, 1 H), 3.74 (s, 1 H), 2.97 (br d, J=12.2 Hz, 3 H), 2.68 (m, 3 H), 2.40 (s, 3 H), 1.84 (br d, J=10.9 Hz, 2 H), 1.38 (m, 2 H). m/z: 393.2 [M+H]+ | | | |
| 3-fluoro-3'-[4-(methylamino)piperidin-1-yl]-2'-(1,2-thiazol-5-yl)-[1,1'-biphenyl]-4-carbonitrile | | | | |

| Example 110 | Procedure: H1.2+K3+A2.2+ C1 | SM1: 2-bromo-1-chloro-3-iodobenzene | SM2: tert-butyl N-(piperidin-4-yl)carbamate | Yield: 77% |
|---|---|---|---|---|
| 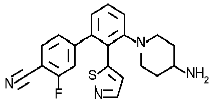 | 1H NMR (600 MHz, DMSO-d6) δ ppm 8.34 (d, J=1.8 Hz, 1 H), 7.87 (m, 4 H), 7.53 (m, 1 H), 7.40 (dd, J=8.1, 1.1 Hz, 1 H), 7.35 (dd, J=10.3, 1.5 Hz, 1 H), 7.17 (dd, J=7.6, 1.2 Hz, 1 H), 7.11 (dd, J=8.0, 1.5 Hz, 1 H), 6.80 (d, J=1.8 Hz, 1 H), 4.11 (br s, 2 H), 3.09 (m, 1 H), 2.96 (br d, J=12.0 Hz, 2 H), 2.72 (m, 2 H), 1.83 (br d, J=9.8 Hz, 2 H), 1.56 (m, 2 H). m/z: 379.2 [M+H]+ | | | |
| 3'-(4-aminopiperidin-1-yl)-3-fluoro-2'-(1,2-thiazol-5-yl)-[1,1'-biphenyl]-4-carbonitrile | | | | |

| Example 185 | Procedure: K1+A2.2+B1+C1 | SM1: 2-bromo-3-chloro-6-fluorobenzaldehyde | SM2: 1,2-thiazole | Yield: 55% |
|---|---|---|---|---|
| 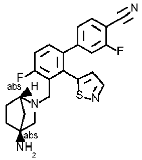 | 1H NMR (DMSO-d6, 600 MHz) +0.6 mol of tartrate: δ (ppm) 8.40 (d, J = 1.6 Hz, 1H), 7.78 (d, J = 0.9 Hz, 1H), 7.44-7.50 (m, 2H), 7.34 (dd, J = 10.5, 1.4 Hz, 1H), 7.13 (d, J = 1.8 Hz, 1H), 7.08-7.11 (m, 1H), 3.76 (s, 1H), 3.43-3.48 (m, 3H), 2.86 (s, 1H), 2.72 (dd, J = 8.2, 3.2 Hz, 1H), 2.23 (d, J = 8.2 Hz, 1H), 1.77-1.88 (m, 1H), 1.50-1.65 (m, 3H), 1.38-1.50 (m, 2H). m/z: 423.3 [M+H]+ | | | |
| 3'-{[(1S,4S)-4-amino-2-azabicyclo[2.2.1]heptan-2-yl]methyl}-3,4'-difluoro-2'-(1 ,2-thiazol-5-yl)-[1 ,1'-biphenyl]-4-carbonitrile | | | | |

| Example 108 | Procedure: H1.2+K3+A2.2+ C1 | SM1: 2-bromo-1-chloro-3-iodobenzene | SM2: tert-butyl octahydro-1 H-pyrrolo[3,2-c]pyridine-1-carboxylate | Yield: 44% |
|---|---|---|---|---|
| 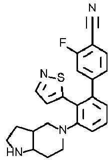 | 1H NMR (600 MHz, DMSO-d6) δ ppm 8.37 (d, J=1.8 Hz, 1 H), 7.81 (dd, J=7.9, 7.0 Hz, 1 H), 7.54 (m, 1 H), 7.34 (m, 2 H), 7.17 (dd, J=7.6, 1.0 Hz, 3 H), 7.10 (dd, J=8.0, 1.5 Hz, 1 H), 6.96 (d, J=1.8 Hz, 1 H), 3.76 (s, 2 H), 3.43 (m, 1 H), 3.17 (td, J=10.5, 5.0 Hz, 1 H), 3.05 (m, 1 H), 2.85 (m, 3 H), 2.62 (m, 1 H), 2.22 (m, 1 H), 1.72 (br s, 2 H), 1.61 (m, 1 H), 1.50 (m, 1 H). m/z: 405.3 [M+H]+ | | | |
| 3-fluoro-3'-{octahydro-1H-pyrrolo[3,2-c]pyridin-5-yl}-2'-(1,2-thiazol-5-yl)-[1,1'-biphenyl]-4-carbonitrile | | | | |

| Example 201 | Procedure: A1.1+A2.2+B2+ D1+C1 | SM1: 2-bromo-3-chloro-5-fluorobenzaldehyde | SM2: cyclopropylboronic acid | Yield: 79% |
|---|---|---|---|---|
| 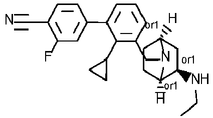 | 1H NMR (DMSO-d6, 500 MHz) δ 7.9-8.0 (m, 1H), 7.5-7.6 (m, 2H), 7.46 (dd, 1H, J=1.5, 8.1 Hz), 7.32 (t, 1H, J=7.7 Hz), 7.17 (dd, 1H, J=1.2, 7.6 Hz), 3.8-3.9 (m, 3H), 2.9-3.7 (m, 8H), 2.6-2.8 (m, 2H), 2.0-2.1 (m, 2H), 1.6-2.0 (m, 3H), 1.4-1.5 (m, 1H), 1.09 (t, 3H, J=7.2 Hz), 0.9-1.0 (m, 1H), 0.6-0.8 (m, 2H), -0.1-0.1 (m, 2H). m/z: 390 [M+H]+ | | | |
| 2'-cyclopropyl-3'-{[(1R,2R,4S)-2-(ethylamino)-7-azabicyclo[2.2.1]heptan-7-yl]methyl}-3-fluoro-[1,1'-biphenyl]-4-carbonitrile | | | | |

| Example 212 | Procedure: A6+Q1+A1.1+F1 +D3+C1 | SM1: tert-butyl N-[(1S,2S,4R)-7-({4'-cyano-3'-fluoro-2-propyl-[1,1'-biphenyl]-3-yl}methyl)-7-azabicyclo[2.2.1]hept an-2-yl]carbamate | | Yield: 71% |
|---|---|---|---|---|
| 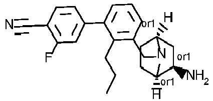 | 1H NMR (600 MHz, DMSO-d6) δ ppm 10.80 (m, 1 H), 8.79 (m, 3 H), 8.02 (m, 1 H), 7.32 (m, 5 H), 4.29 (m, 5 H), 2.70 (m, 2 H), 1.98 (m, 5 H), 1.24 (m, 3 H), 0.69 (m, 3 H). m/z: 364.3 [M+H]+ | | | |
| 3'-{[(1R,2R,4S)-2-amino-7-azabicyclo[2.2.1]heptan-7-yl]methyl}-3-fluoro-2'-propyl-[1,1'-biphenyl]-4-carbonitrile | | | | |

| Example 200 | Procedure: D3+A1.1+C1+C1 | SM1: 1-bromo-3-(bromomethyl)-2-(trifluoromethyl)benze ne | SM2: tert-butyl N-[(1 S,2S,4R)-7-azabicyclo[2.2.1]hept an-2-yl]carbamate hydrochloride | Yield: 83% |
|---|---|---|---|---|
| 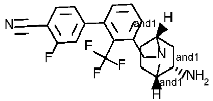 | 1H NMR (DMSO-d6, 500 MHz) δ 7.9-8.0 (m, 2H), 7.6-7.7 (m, 1H), 7.5-7.6 (m, 1H), 7.3-7.4 (m, 1H), 7.2-7.3 (m, 1H), 3.7-3.8 (m, 4H), 2.9-3.7 (m, 9H), 1.9-2.1 (m, 2H), 1.7-1.9 (m, 1H), 1.5-1.7 (m, 1H), 1.38 (ddd, 1H, J=4.3, 9.3, 12.1 Hz), 0.70 (dd, 1H, J=4.3, 11.9 Hz), m/z: 390 [M+H]+ | | | |
| 3'-{[(1S,2S,4R)-2-amino-7-azabicyclo[2.2.1]heptan-7-yl]methyl}-3-fluoro-2'-(trifluoromethyl)-[1,1'-biphenyl]-4-carbonitrile | | | | |

| Example 58 | Procedure: B1+I3+B2+A2.2+ C1 | SM1: 1-bromo-3-chloro-2-(trifluoromethoxy)ben zene | SM2: 2-ethenyl-4,4,5,5-tetramethyl-1,3,2-dioxaborolane | Yield: 56% |
|---|---|---|---|---|
| 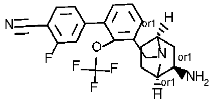 | 1H NMR (600 MHz, DMSO-d6) δ ppm 0.92 (dd, J=12.47, 4.11 Hz, 1 H) 1.29 - 1.40 (m, 1 H) 1.45 - 1.66 (m, 3 H) 1.80 - 1.95 (m, 1 H) 2.99 (br t, J=4.40 Hz, 1 H) 3.06 (t, J=4.40 Hz, 1 H) 3.16 - 3.68 (m, 3 H) 3.19 - 3.25 (m, 1 H) 3.31 (t, J=14.09 Hz, 2 H) 3.78 (s, 2 H) 5.71 - 9.06 (m, 3 H) 7.36 (br t, J=6.02 Hz, 1 H) 7.42 (ddd, J=5.69, 3.70, 1.61 Hz, 1 H) 7.54 - 7.63 (m, 3 H) 8.01 (t, J=7.41 Hz, 1 H). m/z: 406.2 [M+H]+ | | | |
| 3'-{[(1R,2R,4S)-2-amino-7-azabicyclo[2.2.1]heptan-7-yl]methyl}-3-fluoro-2'-(trifluoromethoxy)-[1,1'-biphenyl]-4-carbonitrile | | | | |

| Example 177 | Procedure: B1+D2.1+A1.1+ C1 | SM1: 3-bromo-2-hydroxybenzaldehyde | SM2: tert-butyl N-[(1 S,4S)-2-azabicyclo[2.2.1]hept an-4-yl]carbamate | Yield: 90% |
|---|---|---|---|---|
| 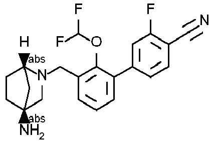 | 1H NMR (600 MHz, DMSO-d6) δ ppm 1.38 (d, J=8.66 Hz, 1 H) 1.40 - 1.47 (m, 1 H) 1.52 - 1.66 (m, 3 H) 1.84 - 1.92 (m, 1 H) 2.25 (d, J=8.07 Hz, 1 H) 2.67 (dd, J=8.29, 3.01 Hz, 1 H) 3.09 (s, 1 H) 3.36 - 3.43 (m, 6 H) 3.65 - 3.72 (m, 2 H) 3.73 (s, 2 H) 6.79 (t, J=74.31 Hz, 1 H) 7.39 - 7.45 (m, 2 H) 7.51 (dd, J=8.00, 1.54 Hz, 1 H) 7.55 - 7.61 (m, 1 H) 7.64 (dd, J=1 0.56, 1.47 Hz, 1 H) 7.98 - 8.04 (m, 1 H). m/z: 388.2 [M+H]+ | | | |
| 3'-{[(1S,4S)-4-amino-2-azabicyclo[2.2.1]heptan-2-yl]methyl}-2'-(difluoromethoxy)-3-fluoro-[1,1'-biphenyl]-4-carbonitrile | | | | |

| Example 169 | Procedure: B2+D2.1+A1.1+ C2 | SM1: 3-bromo-6-fluoro-2-hydroxybenzaldehyde | SM2: tert-butyl N-[(1 R,2R,4S)-7-azabicyclo[2.2.1]hept an-2-yl]carbamate | Yield: 73% |
|---|---|---|---|---|
| 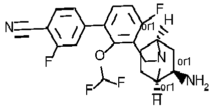 | 1H NMR (DMSO-d6, 600 MHz) δ 10.6-11.7 (m, 1H), 8.1-9.2 (m, 3H), 8.06 (br t, 1H, J=7.3 Hz), 7.1-7.9 (m, 4H), 6.4-7.0 (m, 1H), 3.8-4.9 (m, 5H), 1.4-2.5 (m, 6H. m/z: 406.2 [M+H]+ | | | |
| 3'-{[(1R,2R,4S)-2-amino-7-azabicyclo[2.2.1]heptan-7-yl]methyl}-2'-(difluoromethoxy)-3,4'-difluoro-[1,1'-biphenyl]-4-carbonitrile | | | | |

| Example 219 | Procedure: H2+A1.1+C3+I1+ B2+C1 | SM1: (4-bromophenyl)methoxy -tert-butyl-dimethylsilane | SM2: 4-ethoxypiperidine | Yield: 62% |
|---|---|---|---|---|
| 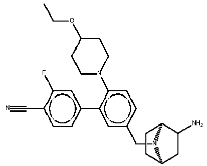 | 1H NMR (DMSO-d6, 500 MHz) δ 8.01 - 7.92 (m, 1H), 7.80 (dd, J = 11.1, 1.3 Hz, 1H), 7.69 (dd, J = 8.3, 1.5 Hz, 1H), 7.32 (dd, J = 8.2, 2.1 Hz, 1H), 7.22 (d, J = 2.0 Hz, 1H), 7.08 (d, J = 8.3 Hz, 1H), 3.50 - 3.45 (m, 2H), 3.44 (s, 2H), 3.23 - 3.18 (m, 1H), 3.06 (t, J = 4.8 Hz, 1H), 2.97 - 2.87 (m, 3H), 2.61 - 2.53 (m, 2H), 2.36 (s, 2H), 2.02 - 1.90 (m, 2H), 1.83 - 1.72 (m, 3H), 1.60 - 1.49 (m, 1H), 1.44 - 1.25 (m, 3H), 1.08 (t, J = 7.1 Hz, 3H), 0.70 - 0.53 (m, 1H). m/z: 449 [M+H]+ | | | |
| 4-[2-(4-ethoxy-1-piperidyl)-4-[[rac-(1S,2S,4R)-2-amino-7-azabicyclo[2.2.1]heptan-7-yl]methyl]phenyl]-2-fluoro-benzonitrile | | | | |

| Example 294 | Procedure: A1.1+I3+A2.2+ B1+C2 | SM1: 1-(3-bromo-5-chlorophenyl)pyrrolidine | SM2: 4,4,5,5-tetramethyl-2-vinyl-1,3,2-dioxaborolane | Yield: 66% |
|---|---|---|---|---|
| 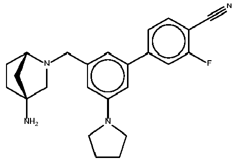 | 1H NMR (DMSO-d6, 500 MHz) δ 10.86-11.95 (m, 1H), 8.87-9.07 (m, 3H), 8.00-8.05 (m, 1H), 7.90-7.99 (m, 1H), 7.77-7.86 (m, 1H), 7.28-7.45 (m, 1H), 7.00-7.08 (m, 1H), 6.85-6.92 (m, 1H), 4.15-4.56 (m, 2H), 3.84-4.07 (m, 1H), 3.30-3.40 (m, 5H), 2.53-3.08 (m, 1H), 2.26-2.45 (m, 2H), 1.73-2.05 (m, 8H). m/z: 391 [M+H]+ | | | |
| 4-[3-[[(1S,4S)-4-amino-2-azabicyclo[2.2.1]heptan-2-yl]methyl]-5-pyrrolidin-1-yl-phenyl]-2-fluoro-benzonitrile | | | | |

| Example 300 | Procedure: A1.1+I3+A2.2+ B1 | SM1: 1-(3-bromo-5-chlorophenyl)pyrrolidine | SM2: 4,4,5,5-tetramethyl-2-vinyl-1,3,2-dioxaborolane | Yield: 34% |
|---|---|---|---|---|
| 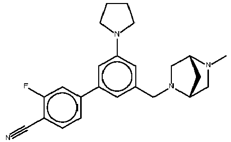 | 1H NMR (DMSO-d6, 500 MHz) δ 7.95 (t, *J* = 7.7 Hz, 1H), 7.82 (dd, *J* = 11.2, 1.2 Hz, 1H), 7.70 (dd, *J* = 8.2, 1.6 Hz, 1H), 6.91 (s, 1H), 6.69 (s, 1H), 6.62 (s, 1H), 3.54-3.78 (m, 2H), 3.27-3.30 (m, 4H), 3.24 (s, 1H), 3.13 (br s, 1H), 2.51-2.79 (m, 4H), 2.27 (s, 3H), 1.82-2.04 (m, 4H), 1.52-1.67 (m, 2H). m/z: 391 [M+H]+ | | | |
| 2-fluoro-4-[3-[[(1S,4S)-5-methyl-2,5-diazabicyclo[2.2.1]heptan-2-yl]methyl]-5-pyrrolidin-1-yl-phenyl]benzonitrile | | | | |

### Materials and Methods

### Abbreviations

- ACN: Acetonitrile
- AcOH: Acetic acid
- AIBN: 2-[({E})-(1-cyano-1-methyl-ethyl)azo]-2-methyl-propanenitrile
- BINAP: [1,1'-Binaphthalene]-2,2'-diylbis[diphenylphosphine]
- Boc: *tert*-butyloxycarbonyl
- cataCXium^{®}: Di(1-adamantyl)-n-butylphosphine
- CAN: Ceric ammonium nitrate
- CMBP: Cyanomethylene tributylphosphorane or Tributylphosphoranylidene acetonitrile
- DCM: Dichloromethane
- DIPEA: Diisopropylethylamine
- DMA: *N,N*-Dimethylacetamide
- DME: 1,2-Dimethoxyethane
- DMSO: Dimethylsulfoxide
- Et: Ethyl
- EtOAc: Ethyl acetate
- EtsN: Triethylamine
- EtOH: Ethanol
- h: hour
- H₂O: water
- JonPhos: ditert-butyl-(2-phenylphenyl)phosphane
- LiHMDS: [bis(trimethylsilyl)amino]lithium
- m-CPBA: 3-chlorobenzenecarboperoxoic acid
- min: minutes
- MeOH: Methanol
- MS: Mass spectrometry
- NaHCOs: Sodium bicarbonate
- NFSI: N-(benzenesulfonyl)-N-fluoro-benzenesulfonamide
- NH₄Cl: Ammonium chloride
- Pd₂(dba)₃: Tris(dibenzylideneacetone)dipalladium(0)
- Pd(OAc)₂: Palladium(II) acetate
- Pd-113: Dibromobis(tri-tert-butylphosphino)dipalladium(I)
- QPhos: 1 ,2,3,4,5-Pentaphenyl-1'-(di-tert-butylphosphino)ferrocene
- rt: Room temperature (18 to 22 °C)
- TBAF: Tetrabutylammonium fluoride
- TCDI: di(imidazol-1-yl)methanethione
- TFA: Trifluoroacetic acid
- THF: Tetrahydrofuran
- TTMSS: 1,1,1,3,3,3-hexamethyl-2-(trimethylsilyl)trisilane
- Xantphos: 4,5-Bis(diphenylphosphino)-9,9-dimethylxanthene

**Sample preparation:** Powders were solubilized in DMSO-d₆, vortexed vigorously until the solution was clear and transferred to a NMR tube for data acquisition.

### NMR spectroscopy:

Liquid-state NMR experiments were recorded on a 600 MHz (14.1 Tesla) Bruker Avance III NMR spectrometer (600 MHz for ¹H, 151 MHz for ¹³C) using a triple-resonance ¹H,¹⁵N,¹³C CP-TCI 5 mm cryoprobe (Bruker Biospin, Germany).

Liquid-state NMR experiments were recorded on a 500 MHz (11.75 Tesla) Bruker Avance I NMR spectrometer (500 MHz for ¹H, 125 MHz for ¹³C) using a Dual Resonance BBI 5 mm probe (Bruker Biospin, Germany).

Liquid-state NMR experiments were recorded on a 400 MHz (9.4 Tesla) Bruker Avance NEO NMR spectrometer (400 MHz for ¹H, 100 MHz for ¹³C) using a SEI 5 mm probe (Bruker Biospin, Germany).

All the experiments used for the resonance assignment procedure and the elucidation of the products structure (1D ¹H, 2D ¹H-¹H-COSY, 2D ¹H-¹H-ROESY, 2D ¹H-¹³C-HSQC, 2D ¹H-¹³C-HMBC) were recorded at 300 K. ¹H chemical shifts are reported in δ (ppm) as s (singlet), d (doublet), t (triplet), q (quartet), dd (double doublet), m (multiplet) or br s (broad singlet)

### LCMS chromatography:

LCMS chromatography were recorded the following apparatus using:
- Waters HPLC : Alliance 2695, UV : PDA 996, MS : ZQ (simple Quad) ZQ2
- Waters UPLC : Acquity, UV : Acquity PDA, MS : Qda
- Waters UPLC : Acquity, UV : Acquity TUV, MS : Qda
- Waters UPLC : Acquity, UV : Acquity PDA, MS : QDa, ELSD

The apparatus was tested using a column Gemini NX-C18 Phenomenex (30 x 2 mm) 3µm for the Waters HPLC or a CSH C18 Waters (50 x 2.1 mm), 1,7 µm for the UPLC Waters. All of them used a combination of the following eluents: H₂O + 0.05% TFA (v/v) and ACN + 0.035% TFA (v/v) and a positive electrospray ES+ as ionization mode. The UV detection was set up at 220 and 254 nm.

Temperatures are given in degrees Celsius (°C). The reactants used in the examples may be obtained from commercial sources or they may be prepared from commercially available starting materials as described herein or by methods known in the art. All of the compounds of the invention are synthesized according to the Examples described herein. The progress of the reactions described herein were followed as appropriate by e.g. LC, GC or TLC, and as the skilled person will readily realize, reaction times and temperatures may be adjusted accordingly.

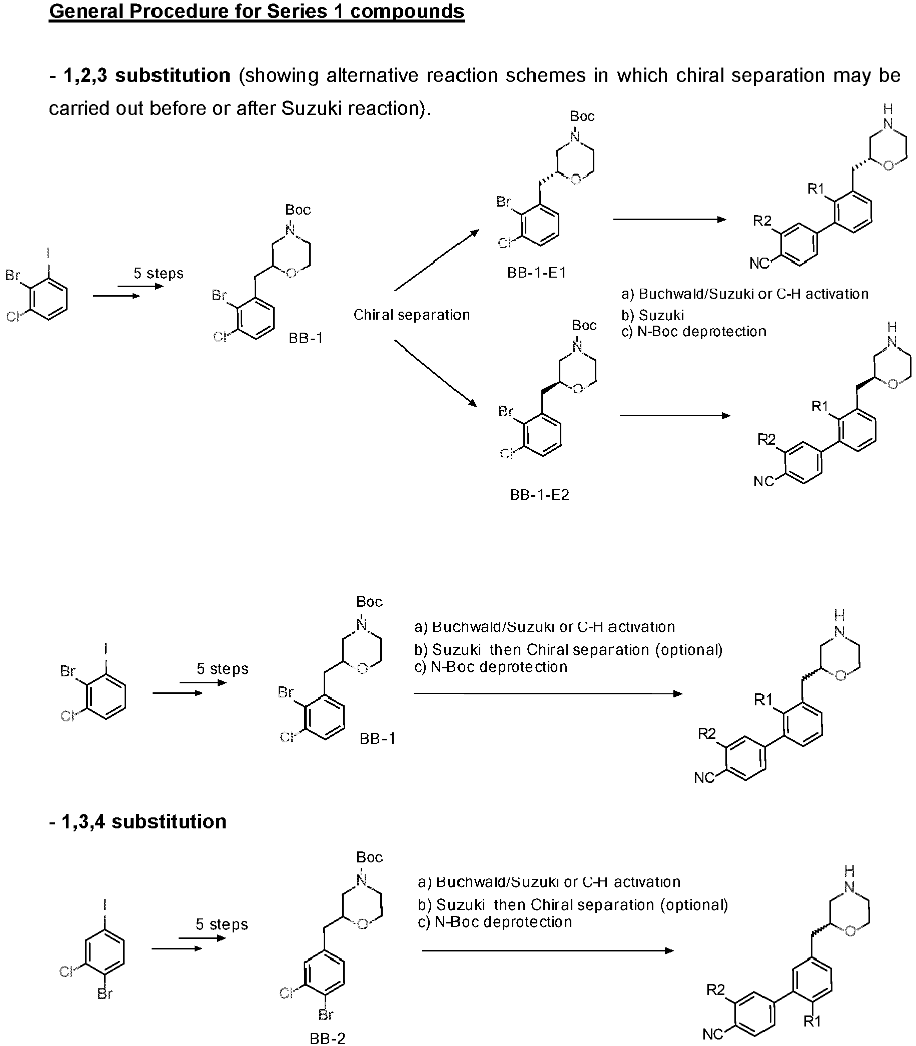

### Building Block synthesis:

### Synthesis of tert-butyl 2-[(2-bromo-3- chlorophenyl)methyl]morpholine-4-carboxylate (BB-1)

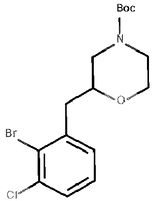

### Small-scale synthesis protocol

### General procedure for Allyl addition: Method B0

To a solution of **2-bromo-1-chloro-3-iodobenzene** (10 g, 30 mmol) in anhydrous THF (395 mL) were added 4,4,5,5-tetramethyl-2-(prop-2-en-1-yl)-1,3,2-dioxaborolane (6.9 g, 39 mmol, 1.30 eq.) and cesium fluoride (11.4 g, 74.8 mmol, 2.5 eq.). The reaction mixture was degassed with argon for 5 min, palladium triphenylphosphane (0.10 eq.) was added, and the reaction mixture was stirred at 80°C for 6h then overnight at 50°C. The reaction mixture was cooled to room temperature, diluted with water and the aqueous layer was extracted three times with EtOAc. The combined organic layers were dried over a phase separator and concentrated under reduced pressure. The crude was purified by flash chromatography column on silica gel (EtOAc in heptane from 0 to 100%) to afford **1-allyl-2-bromo-3-chloro-benzene** (6.5 g, 64%) as a colourless oil.

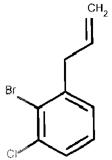

¹H NMR (DMSO-*d*₆,400 MHz,) δ (ppm): 7.50 (dd, J = 7.9, 1.7 Hz, 1H), 7.36 (t, J = 7.8 Hz, 1H), 7.29 (dd, J = 7.7, 1.7 Hz, 1H), 5.95 (ddt, J = 16.6, 10.1, 6.4 Hz, 1H), 5.16 - 4.99 (m, 2H), 3.54 (dt, *J* = 6.4, 1.6 Hz, 2H).

### General procedure for Epoxidation: Method H0

To a solution of **1-allyl-2-bromo-3-chloro-benzene** (6.5 g, 19.1 mmol) in anhydrous DCM (147 mL) was added 3-chlorobenzenecarboperoxoic acid (14.5 g, 65 mmol, 3.4 eq.). The reaction mixture was stirred at room temperature overnight. A saturated aqueous solution of Na₂S₂O₃ and DCM were added and the aqueous layer was extracted three times with DCM. The combined organic layers were washed with a saturated aqueous solution of NaHCOs and brine then dried over a phase separator and concentrated under reduced pressure. The crude was purified by flash chromatography column on silica gel (EtOAc in heptane from 0 to 30%) to afford **2-[(2-bromo-3-chloro-phenyl)methyl]oxirane** (4.8 g, 90%) as a pale yellow oil.

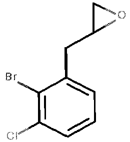

¹H NMR (DMSO-*d*₆, 400 MHz,) δ (ppm): 7.53 (dd, *J* = 5.4, 4.2 Hz, 1H), 7.43 - 7.29 (m, 2H), 3.21 (tdd, J = 5.2, 4.0, 2.5 Hz, 1H), 3.09 - 2.96 (m, 2H), 2.76 (dd, J = 5.1, 3.9 Hz, 1H), 2.56 (dd, *J* = 5.1, 2.6 Hz, 1H).

### General procedure for Epoxide ring opening: Method G0

To a solution of **2-[(2-bromo-3-chloro-phenyl)methyl]oxirane** (4.7 g; 16.8 mmol) in anhydrous DCM (7.7 mL) was added 2-aminoethanol (7.41 mL, 117.7mmol, 7 eq.). The reaction mixture was stirred at room temperature overnight. Water was added, the phases separated and the aqueous layer extracted with DCM three times. The combined organic layers were washed with brine, dried over a phase separator and concentrated under reduced pressure to afford **1-(2-bromo-3-chloro-phenyl)-3-(2-hydroxyethylamino)propan-2-ol** (5.5 g, 93%) as a yellow solid. ¹H NMR (DMSO-*d*₆,400 MHz,) δ (ppm): 7.51 - 7.41 (m, 1H), 7.36 - 7.25 (m, 2H), 4.71 (d, J = 5.5 Hz, 1H), 4.44 (t, J = 5.3 Hz, 1H), 3.89 - 3.70 (m, 1H), 3.44 (qd, J = 5.4, 3.1 Hz, 2H), 2.96 (dd, J = 13.6, 4.8 Hz, 1H), 2.78 (dd, J = 13.6, 8.1 Hz, 1H), 2.60 - 2.52 (m, 4H); m/z = 310 [M+H]+.

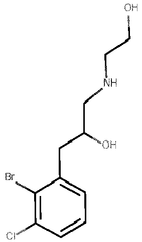

### General procedure for N-Boc protection: Method I1

To a solution of **1-(2-bromo-3-chloro-phenyl)-3-(2-hydroxyethylamino)propan-2-ol** (5.5 g, 15.7 mmol) in DCM (143 mL) were added tert-butoxycarbonyl tert-butyl carbonate (3.7 g, 17.25 mmol, 1.1 eq) and trimethylamine (3.3 mL, 1.5 eq). The reaction mixture was stirred at room temperature for 2h. Water was added, the two phases were separated and the aqueous layer was extracted with DCM. The combined organic layers were dried over a phase separator and concentrated under reduced pressure. The crude material was purified by flash chromatography column on silica gel (MeOH in DCM from 0 to 10%) to afford **tert-butyl N-[3-(2-bromo-3-chlorophenyl)-2-hydroxy-propyl]-N-(2-hydroxyethyl)carbamate** (7.3 g, 93%) as a pale yellow oil.

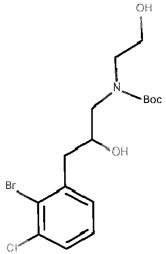

¹H NMR (DMSO-*d*₆,400 MHz,) δ (ppm): 7.48 (dd, J = 7.1, 2.4 Hz, 1H), 7.37 - 7.26 (m, 2H), 5.00 (dd, *J* = 64.5, 5.9 Hz, 1H), 4.72 (t, *J* = 5.5 Hz, 1H), 4.06 - 3.86 (m, 1H), 3.56 - 3.42 (m, 2H), 3.42 -3.33 (m, 2H), 3.26 (dt, *J* = 13.5, 6.2 Hz, 1H), 3.21 - 2.83 (m, 2H), 2.82 - 2.59 (m, 1H), 1.34 (d, J = 43.0 Hz, 9H); m/z = 310 [M+H-Boc]⁺.

### General procedure for Mitsunobu reaction: Method G3

To a solution of **tert-butyl N-[3-(2-bromo-3-chloro-phenyl)-2-hydroxy-propyl]-N-(2-hydroxyethyl)carbamate** (7.3 g, 14.6 mmol) in anhydrous toluene (112 mL) were added triphenylphosphane (4.6 g, 17.5 mmol, 1.2 eq) and isopropyl (N{E})-N-isopropoxycarbonyliminocarbamate (3.5 mL, 17.5 mmol, 1.2 eq). The reaction mixture was stirred at room temperature overnight. The reaction mixture was concentrated under reduced pressure and the crude material was purified by flash chromatography column on silica gel (EtOAc in heptane from 0 to 50%) to afford the title compound **BB-1** (4.97 g, 87%) as a white solid.

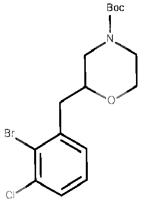

¹H NMR (DMSO-*d*₆,400 MHz,) δ (ppm): 7.52 (dd, J = 6.2, 3.3 Hz, 1H), 7.42 - 7.27 (m, 2H), 3.86 - 3.46 (m, 4H), 3.35 (dd, J = 11.6, 2.9 Hz, 2H), 2.95 (d, J = 6.5 Hz, 3H), 1.38 (s, 9H); m/z = 336 [M*-t*Bu]⁺.

### Synthesis of tert-butyl 2-[(3-bromo-4-chlorophenyl)methyl]morpholine-4-carboxylate (BB-2)

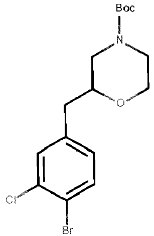

Same procedure as for the synthesis of tert-butyl 2-[(2-bromo-3-chlorophenyl)methyl]morpholine-4-carboxylate (BB-1). Starting from **1-bromo-2-chloro-4-iodobenzene,** tert-butyl 2-[(3-bromo-4-chlorophenyl)methyl]morpholine-4-carboxylate **(BB-2)** was obtained as a white solid.

¹H NMR (DMSO-*d*₆, 400 MHz) *δ* (ppm) : 7.67 (d, J = 8.2 Hz, 1H), 7.53 (d, J = 2.0 Hz, 1H), 7.17 (dd, *J* = 8.2, 2.0 Hz, 1H), 3.75 (d, J= 10.1 Hz, 2H), 3.67 (d, J= 12.3 Hz, 1H), 3.50 (d, J= 5.0 Hz, 1H), 3.33 (m, 2H), 2.85 (s, 1H), 2.73 (m, 2H), 1.38 (s, 9H); m/z = 292 [M+2H - Boc]+.

### Synthesis of tert-butyl 6-[(4-bromo-3-chlorophenyl)methyl]-7-oxa-4-azaspiro[2.5]octane-4-carboxylate (BB-2A)

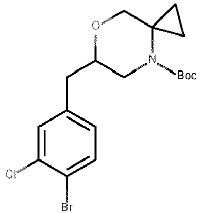

Same procedure as for the synthesis of tert-butyl 2-[(2-bromo-3-chlorophenyl)methyl]morpholine-4-carboxylate (BB-1). Starting from **1-bromo-2-chloro-4-iodobenzene,** using (1-aminocyclopropyl)methanol for epoxide ring opening. **tert-butyl 6-[(4-bromo-3-chlorophenyl)methyl]-7-oxa-4-azaspiro[2.5]octane-4-carboxylate (BB-2A)** was obtained as a colourless oil.

¹H NMR (DMSO-*d*₆, 400 MHz) *δ* (ppm): 7.52 (d, J = 8.2 Hz, 1H), 7.35 (d, J = 2.1 Hz, 1H), 7.00 (dd, J = 8.2, 2.1 Hz, 1H), 3.99 - 3.84 (m, 2H), 3.67 - 3.58 (m, 1H), 3.02 (d, J = 11.4 Hz, 1H), 2.91 (dd, J= 13.2, 10.3 Hz, 1H), 2.75 (dd, J= 14.2, 7.8 Hz, 1H), 2.66 (dd, J= 14.2, 5.0 Hz, 1H), 1.43 (s, 9H), 0.93 - 0.85 (m, 1H), 0.75 - 0.68 (m, 1H), 0.67 - 0.59 (m, 1H); m/z = 318 [M+H]⁺ - Boc.

### Synthesis of tert-butyl 2-[(4-bromo-3-chloro-2-fluorophenyl)methyl]morpholine-4-carboxylate (BB-2B)

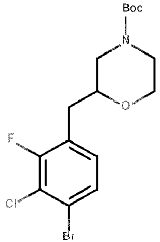

Same procedure as for the synthesis of tert-butyl 2-[(2-bromo-3-chlorophenyl)methyl]morpholine-4-carboxylate (BB-1). Starting from **1-bromo-2-chloro-3-fluoro-4-iodobenzene,** tert-butyl 2-[(4-bromo-3-chloro-2-fluorophenyl)methyl]morpholine-4-carboxylate **(BB-2B)** was obtained as a colourless oil.

¹H NMR (DMSO-*d*₆, 400 MHz) *δ* (ppm): 7.57 (dd, J = 8.3, 1.5 Hz, 1H), 7.31 (t, J = 7.9 Hz, 1H), 3.84 - 3.61 (m, 3H), 3.52 (dddd, J = 10.3, 7.7, 5.2, 2.6 Hz, 1H), 3.32 (td, J = 11.6, 2.8 Hz, 2H), 3.02 - 2.70 (m, 3H), 1.39 (d, J = 4.4 Hz, 9H); m/z = 410 [M+2H - Boc]+.

### Synthesis of tert-butyl 2-[(3-bromo-4-chloro-5-ethylphenyl)methyl]morpholine-4-carboxylate (BB-2C)

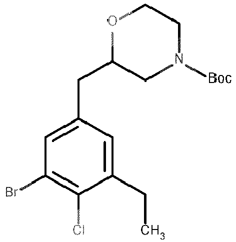

Same procedure as for the synthesis of tert-butyl 2-[(2-bromo-3-chlorophenyl)methyl]morpholine-4-carboxylate (BB-1). Starting from **1,5-dibromo-2-chloro-3-ethylbenzene, tert-butyl 2-[(3-bromo-4-chloro-5-ethylphenyl)methyl]morpholine-4-**carboxylate **(BB-2C)** was obtained as a colourless oil (70% yield on last step).

¹H NMR (DMSO-*d*₆, 400 MHz) *δ* (ppm) : 7.51 (d, J = 2.0 Hz, 1H), 7.24 (d, J = 2.0 Hz, 1H), 3.80 - 3.63 (m, 3H), 3.54 - 3.45 (m, 1H), 3.34 (dd, J = 11.6, 2.8 Hz, 1H), 2.85 (s, 1H), 2.77 - 2.66 (m, 4H), 1.38 (s, 9H), 1.16 (t, J = 7.5 Hz, 3H).; m/z = 362 [M+H -(t-Bu)]+.

### Synthesis of 2-[(4-bromo-3-chloro-phenyl)methyl]-4-methyl-morpholine (BB-2D)

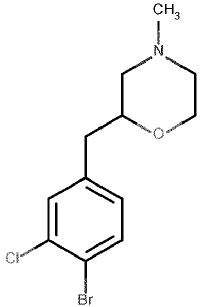

Same procedure as for the synthesis of tert-butyl 2-[(2-bromo-3-chlorophenyl)methyl]morpholine-4-carboxylate (BB-1). Starting from **2-bromo-1-chloro-3-iodobenzene and using 2-(methylamino)ethan-1-ol for the epoxide ring opening step,** 2-[(4-bromo-3-chloro-phenyl)methyl]-4-methyl-morpholine **(BB-2D)** was obtained as a white solid (84% yield on the last step).
m/z = 304 [M+H]+.

### Synthesis of tert-butyl 2-[2-(4-bromo-3-chlorophenyl)propan-2-yl]morpholine-4-carboxylate (BB-2E)

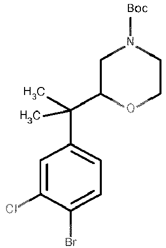

### Allyl addition:

To a solution of **1-bromo-2-chloro-4-iodobenzene** (150 mg, 0.473 mmol) in anhydrous THF (4.5 mL) were added 5 M potassium hydroxide (0.38 mL, 1.89 mmol, 4 eq.) and 4,4,5,5-tetramethyl-2-(3-methylbut-2-en-1-yl)-1,3,2-dioxaborolane (95%, 0.12 mL, 0.520 mmol, 1.1 eq.). The reaction mixture was degassed with argon for 5 min, palladium triphenylphosphane (27 mg, 0.0236 mmol, 0.05 eq.) was added, and the reaction mixture was stirred at 50°C for 12h then at 80°C for an hour. The reaction mixture was cooled to room temperature, water then NH₄Cl saturated aqueous solution and DCM were added. The aqueous layer was extracted three times with DCM, the combined organic layers washed with water and brine then dried over a phase separator and concentrated under reduced pressure. The crude was purified by flash chromatography column on silica gel (EtOAc in cyclohexane from 0 to 100%) to afford a (50/50) mixture of 1-bromo-2-chloro-4-(1,1-dimethylallyl)benzene and 1-bromo-2-chloro-4-(3-methylbut-2-enyl)benzene (79.7 mg, 65%) as a colourless oil.

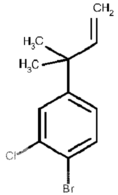

¹H NMR (MeOD, 400 MHz) *δ* : 7.56 (dd, J = 15.0, 8.3 Hz, 2H), 7.46 (d, J = 2.3 Hz, 1H), 7.32 - 7.27 (m, 1H), 7.20 (dd, J = 8.5, 2.3 Hz, 1H), 7.03 - 6.95 (m, 1H), 6.07 - 5.94 (m, 1H), 5.28 (tdt, J = 7.4, 2.9, 1.5 Hz, 1H), 5.07 (dd, J = 7.6, 1.1 Hz, 2H), 1.80 - 1.66 (m, 6H), 1.38 (s, 8H).

The next steps followed the same procedure as for BB-1: epoxidation on the mixture, epoxide ring opening with 2-aminoethanol allowing separation of the unreacted by-product, N-Boc protection and Mitsunobu to give **tert-butyl 2-[1-(4-bromo-3-chloro-phenyl)-1-methylethyl]morpholine-4-carboxylate (BB-2E)** as a colourless oil.

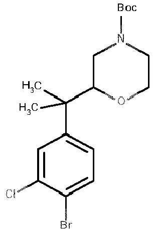

¹H NMR (CDCl₃, 400 MHz) *δ* : 7.52 (d, *J* = 8.5 Hz, 1H), 7.46 (d, *J* = 2.3 Hz, 1H), 7.15 (dd, *J* = 8.5, 2.3 Hz, 1H), 3.98 - 3.56 (m, 3H), 3.43 (t, J = 11.6 Hz, 1H), 3.29 (d, *J* = 10.5 Hz, 1H), 2.79 (t, *J* = 12.5 Hz, 1H), 2.43 (t, *J* = 12.3 Hz, 1H), 1.54 (d, *J* = 29.9 Hz, 2H), 1.43 (s, 10H), 1.33 (d, J= 7.9 Hz, 6H); m/z = 362 [M+H -(t-Bu)]+.

### Synthesis of tert-butyl 6-[(4-bromo-3-chlorophenyl)methyl]-2,2-dimethylmorpholine-4-carboxylate (BB-2F)

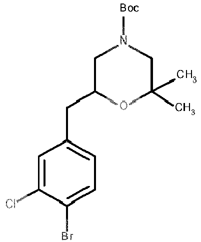

Same procedure as for the synthesis of tert-butyl 2-[(2-bromo-3-chlorophenyl)methyl]morpholine-4-carboxylate (BB-1). Starting from **1-bromo-2-chloro-4-iodobenzene, using** 2-amino-2-methylpropan-1-ol forthe epoxide ring opening step and method G2 for the last step, **tert-butyl 6-[(4-bromo-3-chlorophenyl)methyl]-2,2-dimethylmorpholine-4-carboxylate (BB-2F)** obtained as a yellow solid (32 % last step).

¹H NMR (DMSO-*d*₆, 400 MHz) *δ* (ppm): 7.77 - 7.60 (m, 2H), 7.35 (dd, J = 8.4, 2.1 Hz, 1H), 6.38 (s, 2H), 4.45 (s, 1H), 4.09 (d, J = 12.5 Hz, 2H), 3.15 (s, 2H), 1.39 (s, J = 5.9 Hz, 9H), 1.09 (s, 6H) ; m/z = 320 [M+H]⁺ - Boc.

### Synthesis of tert-butyl 6-[(2-bromo-3-chlorophenyl)methyl]-2,2-dimethylmorpholine-4-carboxylate (BB-2G)

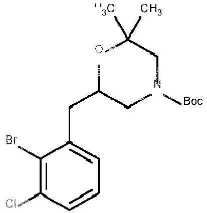

Same procedure as for the synthesis of tert-butyl 2-[(2-bromo-3-chlorophenyl)methyl]morpholine-4-carboxylate (BB-1) using **1-amino-2-methyl-propan-2-ol** dissolved in DMF for the epoxide ring opening step. **Tert-butyl 6-[(2-bromo-3-chloro-phenyl)methyl]-2,2-dimethyl-morpholine-4-carboxylate (BB-2G)** obtained as a colourless oil (34 % yield on last step).

¹H NMR (DMSO-*d*₆, 400 MHz) *δ* (ppm): 7.51 (dd, J = 7.5, 2.1 Hz, 1H), 7.43 - 7.26 (m, 2H), 3.95 - 3.51 (m, 3H), 2.87 (q, J = 6.9 Hz, 2H), 1.35 (s, 9H), 1.10 (d, J = 9.2 Hz, 6H); m/z = 364 [M+H - (tBu)]*.

### Synthesis of tert-butyl 2-[(4-bromo-3-chlorophenyl)methyl]-1,4-oxazepane-4-carboxylate (BB-2H)

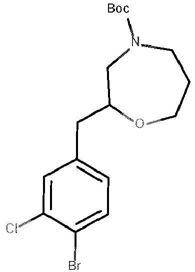

Same procedure as for the synthesis **of tert-butyl 2-[(3-bromo-4-chlorophenyl)methyl]morpholine-4-carboxylate (BB-2)** using **3-aminopropan-1-ol** for the epoxide ring opening step. **Tert-butyl 2-[(4-bromo-3-chloro-phenyl)methyl]-1,4-oxazepane-4-carboxylate (BB-2H)** obtained as a colourless oil (8 % yield on last step).

¹H NMR (DMSO-*d*₆, 400 MHz) *δ* (ppm): 7.73 - 7.61 (d, 1H), 7.52 (s, J = 2.6 Hz, 1H), 7.16 (dd, J = 8.2, 2.1 Hz, 1H), 3.90 (dt, J = 12.6, 4.4 Hz, 1H), 3.76 - 3.51 (m, 3H), 3.28 (m, J = 7.8 Hz, 1H), 3.17 (m, J = 25.6, 13.3, 6.5 Hz, 1H), 2.96 (m, J = 9.5, 5.0 Hz, 1H), 2.69 (m, J = 20.8, 13.5, 9.2 Hz, 2H), 1.75 (m, J = 12.0, 6.7 Hz, 2H), 1.35 (d, J = 35.1 Hz, 9H); m/z = 306 [M+H]⁺ - Boc.

### Synthesis of tert-butyl 2-[(2-bromo-3-chlorophenyl)methyl]-1,4-oxazepane-4-carboxylate (BB-2I)

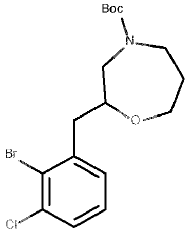

Same procedure as for the synthesis **of tert-butyl 2-[(4-bromo-3-chlorophenyl)methyl]-1,4-oxazepane-4-carboxylate** (BB-2H) starting from 2-bromo-1-chloro-3-iodobenzene. **Tert-butyl 2-[(2-bromo-3-chloro-phenyl)methyl]-1,4-oxazepane-4-carboxylate (BB-2I**) obtained as a pale yellow oil (9 % yield on last step).

¹H NMR (DMSO-*d*₆, 400 MHz) *δ* (ppm) : 7.57 - 7.43 (m, 1H), 7.41 - 7.24 (m, 2H), 3.91 (dt, J = 12.6, 4.5 Hz, 1H), 3.71 (dd, *J* = 27.1, 13.5 Hz, 3H), 3.13 - 2.80 (m, 4H), 1.92 - 1.65 (m, 2H), 1.27 (d, *J* = 14.7 Hz, 10H); m/z = 306 [M+H]⁺ - Boc.

### Synthesis of tert-butyl 2-[(4-bromophenyl)methyl]morpholine-4-carboxylate (BB-2J)

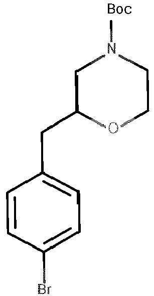

Same procedure as for the synthesis of tert-butyl 2-[(2-bromo-3-chlorophenyl)methyl]morpholine-4-carboxylate (BB-1). Starting from **1-bromo-4-iodobenzene, tert-butyl 2-[(4-bromophenyl)methyl]morpholine-4-carboxylate (BB-2J)** was obtained as an yellow oil.

¹H NMR (DMSO-*d*₆, 400 MHz) *δ* (ppm): 7.52 - 7.43 (m, 2H), 7.26 - 7.17 (m, 2H), 3.82 - 3.63 (m, 3H), 3.54 - 3.43 (m, 1H), 3.34 (dd, J = 11.6, 2.8 Hz, 1H), 2.93 - 2.53 (m, 4H), 1.38 (s, 9H); m/z = 258 [M+2H - Boc]+.

### Synthesis of tert-butyl 2-[(4-bromophenyl)methyl]morpholine-4-carboxylate (BB-2K)

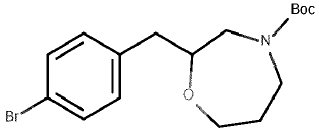

Same procedure as for the synthesis of tert-butyl 2-[(4-bromophenyl)methyl]morpholine-4-carboxylate (BB-2J) using 3-aminopropan-1-ol for the epoxide ring opening step. G2 method was used for Mitsunobu reaction affording **tert-butyl 2-[(4-bromophenyl)methyl]morpholine-4-carboxylate (BB-2K)** as an orange oil (40% yield on this step).

¹H NMR (DMSO-*d*₆, 400 MHz) *δ* (ppm) : 7.46 (d, J = 8.0 Hz, 2H), 7.27 - 7.11 (m, 2H), 3.89 (d, J = 12.8 Hz, 1H), 3.72 - 3.51 (m, 3H), 3.29 - 3.03 (m, 2H), 3.01 - 2.85 (m, 1H), 2.80 - 2.55 (m, 2H), 1.73 (dt, J = 13.9, 6.1 Hz, 2H), 1.34 (d, J = 35.4 Hz, 9H); m/z = 270 [M - Boc]+.

### Building Block's boronic ester formation

### Synthesis of tert-butyl 2-{[3-chloro-4-(4,4,5,5-tetramethyl-1,3,2-dioxaborolan-2-yl)phenyl]methyl}morpholine-4-carboxylate (BB-3)

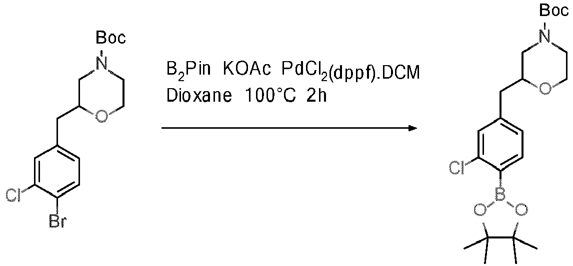

To a solution of tert-butyl 2-[(4-bromo-3-chloro-phenyl)methyl]morpholine-4-carboxylate (250 mg, 0.64 mmol) in 1,4-dioxane (6.4 mL, 0.1M) were added sequentially 4,4,5,5-tetramethyl-2-(4,4,5,5-tetramethyl-1,3,2-dioxaborolan-2-yl)-1,3,2-dioxaborolane (243 mg, 0.95 mmom, 1.5 eq.), KOAc (2 eq.) and PdCl₂(dppf)•CH₂Cl₂ (52 mg, 0.0640 mmol, 0.1 eq.) . The reaction mixture was degassed with nitrogen for 5 min and stirred at 100°C for 2h. The reaction mixture was cooled to rt, filtered over celite, the solid washed with MeOH and the combined filtrates concentrated under reduced pressure. The crude was purified by flash chromatography column (EtOAc in heptane, from 0 to 30%) to afford the title compound **BB-3** as a white solid (63 % Yield).

¹H NMR (DMSO-*d*₆, 400 MHz) *δ* (ppm): 7.56 (d, J = 7.6 Hz, 1H), 7.32 (d, J = 1.5 Hz, 1H), 7.21 (dd, J = 7.7, 1.5 Hz, 1H), 3.85 - 3.59 (m, 3H), 3.55 - 3.45 (m, 1H), 2.90 - 2.56 (m, 4H), 1.39 (s, 9H), 1.30 (s, 12H); m/z = 300 [M (boronic acid)+H - Boc]+.

### Synthesis of tert-butyl 2-{[3-chloro-2-(4,4,5,5-tetramethyl-1,3,2-dioxaborolan-2-yl)phenyl] methyl}morpholine-4-carboxylate (BB-4)

A vial was charged with tert-butyl 2-[(2-bromo-3-chloro-phenyl)methyl]morpholine-4-carboxylate (1.00 g, 2.53 mmol), 4,4,5,5-tetramethyl-2-(4,4,5,5-tetramethyl-1,3,2-dioxaborolan-2-yl)-1,3,2-dioxaborolane **(BB-1)** (99%, 1.30 g, 5.07 mmol), tricyclohexylphosphane (95%, 56 mg, 0.190 mmol) and KOAc (251 mg, 2.53 mmol) in 1 ,4-Dioxane-Anhydrous (10.136 mL). The reaction was degassed and (1{E},4{E})-1,5-diphenylpenta-1,4-dien-3-one;palladium (122 mg, 0.127 mmol) was added. The mixture was stirred overnight at 95°C under N₂ Water was added and the mixture was extracted with AcOEt three times. The combined organic layers were washed with water, brine, dried over phase separator and concentrated to afford a brown oil. The crude product was purified by flash chromatography column (EtOAc in heptane, from 0 to 20%) to afford **tert-butyl 2-[[3-chloro-2-(4,4,5,5-tetramethyl-1,3,2-dioxaborolan-2-yl)phenyl]methyl] morpholine-4-carboxylate (BB-4)** (571mg, 48.4% yield) as a dark green oil.

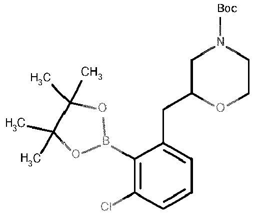

1H NMR (400 MHz, DMSO) δ (ppm): 7.36 - 7.29 (m, 1H), 7.26 (dd, J = 8.1, 1.1 Hz, 1H), 7.18 (dd, J = 7.5, 1.2 Hz, 1H), 3.78 (dd, J = 11.6, 3.2 Hz, 1H), 3.70 (d, J = 12.3 Hz, 2H), 3.49 - 3.39 (m, 1H), 3.27 (td, J = 11.6, 2.8 Hz, 1H), 2.79 (d, J = 14.2 Hz, 2H), 2.67 (dd, J = 13.6, 5.9 Hz, 2H), 1.37 (s, 9H), 1.35 (d, J = 2.0 Hz, 12H); m/z = 338 [M+H - Boc]⁺.

### Method A: Buchwald-Hartwig

### General procedure A1:

To a solution of Ar-Br (1-1.2 eq.) in 1,4-dioxane (0.1 M) were added NaOtBu (3.00 eq.) and Xantphos (0.10 eq.). The reaction mixture was degassed with argon for 5-10 min and Pd₂dba₃ (0.05 - 0.1 eq.) and amine (1-1.5 eq.) were added. The reaction mixture was heated at 90 - 100°C for 5-24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/heptane or MeOH/DCM) to afford the desired product.

### Synthesis of tert-butyl 2-{[3-chloro-4-(morpholin-4-yl)phenyl]methyl}morpholine-4-carboxylate (Intermediate 1)

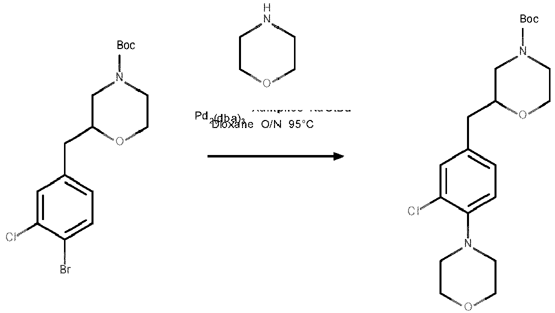

From tert-butyl 2-[(4-bromo-3-chlorophenyl)methyl]morpholine-4-carboxylate (BB-2) (500 mg, 1.27 mmol, 1.15 eq.) and morpholine (97 mg, 1.11 mmol, 1 eq.), tert-butyl 2-{[3-chloro-4-(morpholin-4-yl)phenyl]methyl}morpholine-4-carboxylate was obtained as an yellow oil (73% yield).

¹H NMR(DMSO-*d*₆, 400 MHz) : δ (ppm) 7.31 (d, J = 1.9 Hz, 1H), 7.18 (dd, J = 8.2, 2.0 Hz, 1H), 7.09 (d, J = 8.2 Hz, 1H), 3.83 - 3.63 (m, 7H), 3.47 (dtd, J = 9.3, 6.5, 2.6 Hz, 1H), 3.35 (dd, J = 11.7, 2.9 Hz, 1H), 3.01 - 2.91 (m, 4H), 2.85 (s, 1H), 2.67 (d, J = 6.2 Hz, 2H), 2.59 (s, 1H), 1.38 (s, 9H); m/z = 397 [M+H]⁺.

### General procedure A2:

In a sealed vial, Ar-Br (1eq.) and 3-(methoxymethyl)azetidine hydrochloride were dissolved in 1,4-dioxane (0.1 M). A 2M solution of NaOtBu was added (3 eq.). The reaction mixture was degassed with nitrogen for 5-10 min and tBuXPhos Pd G3 (0.1 eq.) and amine (1-1.5 eq.) was added. The reaction mixture was stirred at 95°C for 2h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column on silica gel (EtOAc in heptane from 0 to 50%) to afford the desired product.

### Synthesis of tert-butyl 2-({3-chloro-4-[3-(methoxymethyl)azetidin-1-yl]phenyl}methyl) morpholine-4-carboxylate (Intermediate 2)

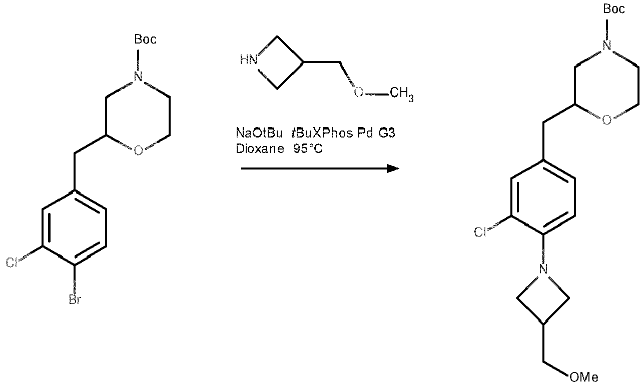

From tert-butyl 2-[(4-bromo-3-chlorophenyl)methyl]morpholine-4-carboxylate (BB-2) (200 mg, 0.51 mmol, 1 eq.) and 3-(methoxymethyl)azetidine hydrochloride (114 mg, 0.76 mmol, 1.5 eq.), tert-butyl 2-({3-chloro-4-[3-(methoxymethyl)azetidin-1-yl]phenyl}methyl)morpholine-4-carboxylate was obtained as an yellow oil (99% yield).

¹H NMR (DMSO-*d*₆, 500 MHz) δ (ppm) 7.10 (d, *J* = 2.0 Hz, 1H), 7.02 (dd, J = 8.3, 2.0 Hz, 1H), 6.51 (d, J = 8.3 Hz, 1H), 4.05 - 3.96 (m, 3H), 3.77 (dd, J = 11.4, 3.2 Hz, 1H), 3.74 - 3.60 (m, 4H), 3.51 (d, J = 6.6 Hz, 2H), 3.44 - 3.37 (m, 1H), 3.35 - 3.29 (m, 1H), 3.27 (s, 3H), 2.91 - 2.77 (m, 1H), 2.59 (d, J = 5.6 Hz, 3H), 1.37 (s, 9H); m/z = 411 [M+H]⁺.

### General procedure A3:

To a solution of Ar-Br (1.00 eq.) in 1,4-dioxane (0.1M) in a sealed tube were added amine (1.0 eq.), NaOtBu (3.00 eq.), JonPhos or BINAP (0.05 eq.) and diacetoxypalladium (0.5 eq) while degassing with nitrogen. The reaction mixture was stirred at 90°C for 2h. The reaction mixture was cooled to rt, quenched with water and extracted with EtOAc three times. The combined organic layers were dried over a phase separator and concentrated under reduced pressure. The crude was purified by flash chromatography column on silica gel (EtOAc/heptane 0 to 100%) to afford the desired product.

### Synthesis of tert-butyl 2-[[4-(4-pyrimidin-2-yl-1-piperidyl)phenyl]methyl]morpholine-4-carboxylate (Intermediate 3)

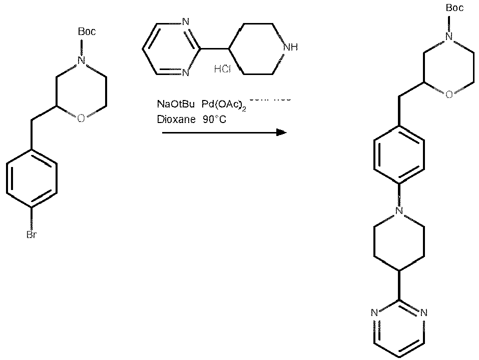

From tert-butyl 2-[(4-bromophenyl)methyl]morpholine-4-carboxylate (BB-2J) (300 mg, 0.84 mmol) and 2-(piperidin-4-yl)pyrimidine hydrochloride (212 mg, 1.01 mmol, 1.2 eq.), tert-butyl 2-[[4-(4-pyrimidin-2-yl-1-piperidyl)phenyl]methyl]morpholine-4-carboxylate was obtained as an yellow solid (63 % yield).

¹H NMR(DMSO-*d*₆, 400 MHz) : δ (ppm) 8.75 (d, J= 4.9 Hz, 2H), 7.35 (t, *J* = 4.9 Hz, 1H), 7.07 (d, J = 8.6 Hz, 2H), 6.89 (d, J = 8.7 Hz, 2H), 3.83 - 3.62 (m, 5H), 3.47 - 3.31 (m, 2H), 2.97 (tt, J = 11.5, 3.8 Hz, 1H), 2.80 (td, *J* = 12.2, 2.5 Hz, 3H), 2.61 (ddd, *J* = 35.7, 13.8, 6.4 Hz, 3H), 1.99 (s, 3H), 1.88 (qd, J = 12.4, 3.9 Hz, 2H), 1.36 (s, 9H).; m/z = 439 [M+H]⁺.

### General procedure A4:

To a solution of Ar-Br (1.00 eq.) in toluene (0.1M) in a sealed tube were added sequentially amine (1.1 eq.) and NaOtBu or KOtBu (2.2 eq.). The mixture was degassed with nitrogen for 5 min and Pd-113 (0.07 eq.) was added. The reaction mixture was stirred at 100°C for 2h. The reaction mixture was cooled to rt, quenched with water and extracted with EtOAc three times. The combined organic layers were dried over a phase separator and concentrated under reduced pressure. The crude was purified by flash chromatography column on silica gel (EtOAc in heptane 0 to 100%) to afford the desired product.

### Synthesis of tert-butyl 2-[[4-[4-(ethoxymethyl)-4-hydroxy-1-piperidyl]phenyl]methyl] morpholine-4-carboxylate (Intermediate 4)

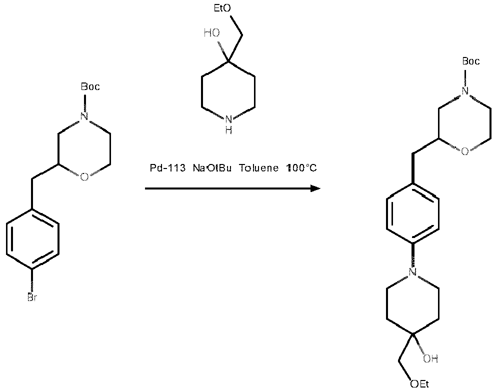

From tert-butyl 2-[(4-bromophenyl)methyl]morpholine-4-carboxylate (BB-2J) (100 mg, 0.28 mmol) and 4-(ethoxymethyl)piperidin-4-ol (49 mg, 0.31 mmol, 1.1 eq.), tert-butyl 2-[[4-[4-(ethoxymethyl)-4-hydroxy-1-piperidyl]phenyl]methyl]morpholine-4-carboxylate was obtained as an yellow oil.

¹H NMR(DMSO-*d*₆, 400 MHz) : δ (ppm) 7.09 - 6.97 (m, 2H), 6.91 - 6.80 (m, 2H), 4.29 (s, 1H), 3.68 (m, 1H), 3.46 (m, *J* = 7.0 Hz, 2H), 3.31 (s, 8H), 3.20 (s, 2H), 2.99 (td, *J* = 12.0, 2.8 Hz, 2H), 2.85 (s, 1H), 2.60 (ddd, J= 37.1, 13.9, 6.8 Hz, 2H), 1.68 (td, J= 12.6, 4.5 Hz, 2H), 1.48 (s, 1H), 1.37 (s, 9H), 1.11 (t, J = 7.0 Hz, 3H) ; m/z = 435 [M+H]⁺.

### General procedure A5:

To a stirred solution of Ar-Br (1 eq.) in 1,4-Dioxane (0.1 M) were added sequentially the amine (1.1 eq.), BINAP (0.4 eq.) and cesium carbonate (3 eq.). The reaction mixture was degassed for 5 min and Pd₂(dba)₃ (0.2 eq.) was added. The mixture was stirred at 100°C for 12 h. DCM and water were added. The aqueous layer was extracted with DCM three times. The combined organic layers were dried over a phase separator and concentrated under reduced pressure. The crude was purified by flash chromatography column on silica gel (EtOAc in heptane 0 to 100%) to afford the desired product.

### Synthesis of tert-butyl 2-({3-chloro-4-[4-(hydroxymethyl)piperidin-1-yl]phenyl}methyl) morpholine-4-carboxylate (Intermediate 5)

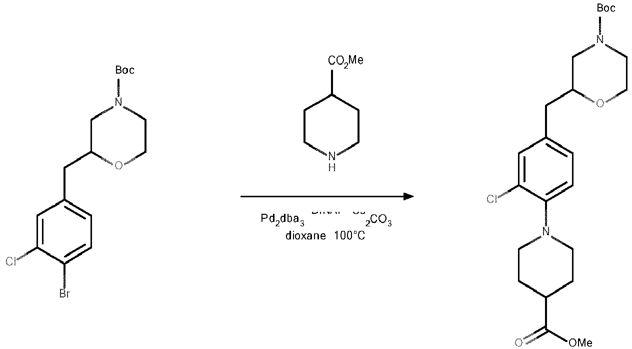

From tert-butyl 2-[(4-bromo-3-chloro-phenyl)methyl]morpholine-4-carboxylate (300 mg, 0.768 mmol) and methyl piperidine-4-carboxylate (125 mg, 0.845 mmol, 1.1 eq) tert-butyl 2-[[3-chloro-4-(4-methoxycarbonyl-1-piperidyl)phenyl]methyl]morpholine-4-carboxylate was obtained as an orange oil (353 mg, 93 % yield).

¹H NMR(DMSO-*d*₆, 400 MHz) : δ (ppm) 7.29 (d, J = 2.0 Hz, 1H), 7.15 (dd, J = 8.2, 2.1 Hz, 1H), 7.06 (d, J = 8.2 Hz, 1H), 3.84 - 3.65 (m, 3H), 3.64 (s, 3H), 3.51 - 3.42 (m, 1H), 3.31 (m, 1H), 3.20 (d, J = 11.8 Hz, 2H), 2.85 (s, 1H), 2.74 - 2.54 (m, 5H), 2.51 (m, J = 1.8 Hz, 1H), 1.93 (dd, J = 13.3, 3.6 Hz, 2H), 1.74 (qd, J = 11.2, 3.7 Hz, 2H), 1.38 (s, 9H); m/z = 453 [M+H]⁺.

### General procedure A6:

To a stirred solution of Ar-Br (1 eq.), amine (2 eq.), 2-(2-dicyclohexylphosphanylphenyl)-N,N-dimethyl-aniline (0.2 eq.) and tripotassium phosphate (4 eq.) in DME (0.1 M) was added (1{E},4{E})-1 ,5-diphenylpenta-1,4-dien-3-one palladium (0.2 eq.). The mixture was stirred at 85°C for 12 h. The mixture was diluted with EtOAc and filtered through a pad of celite. Filtrate was washed with brine. The organic layer was dried over a phase separator and evaporated under reduced pressure. The crude material was purified twice by flash column chromatography (eluting 0 to 50% of EtOAc in cyclohexane) to give expected product.

### Synthesis of tert-butyl 2-[[4-(2-oxa-7-azaspiro[4.4]nonan-7-yl)phenyl]methyl]morpholine-4-carboxylate (Intermediate 6)

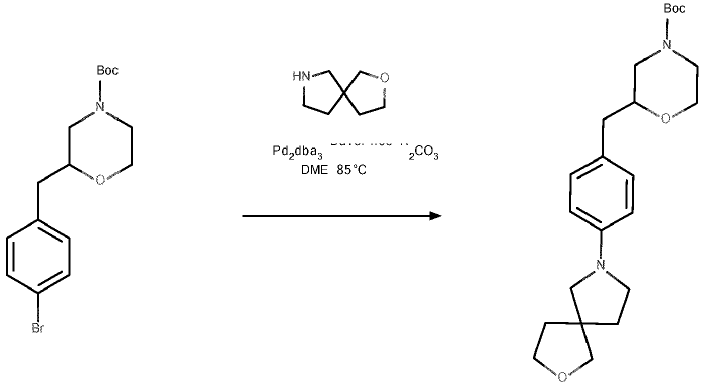

From tert-butyl 2-[(4-bromophenyl)methyl]morpholine-4-carboxylate (100 mg, 0.28 mmol) and 2-oxa-7-azaspiro[4.4]nonane (71 mg, 0.56 mmol, 2 eq) tert-butyl 2-[[4-(2-oxa-7-azaspiro[4.4]nonan-7-yl)phenyl]methyl]morpholine-4-carboxylate was obtained as an yellow oil (63 mg, purity 70%, 39 % yield). m/z = 403.1 [M+H]⁺.

### Method B: Suzuki-Miyaura coupling

### General procedure B1:

To a solution of Ar-Br (Ar-I or Ar-CI) (1.00 eq.) in 1,4-dioxane/H₂O (4:1, 0.1M) were added R-B(OH)₂ (or R-Bpin, or R-BF₃K) (1.20 eq.) and Na₂CO₃ (3.00 eq.). The reaction mixture was degassed with argon for 5 min and Pd(dppf)Cl₂·DCM (0.10 eq.) was added and the reaction mixture was heated at 100°C for 1-24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc in heptane, from 0 to 100%) to afford the desired product

### Synthesis of tert-butyl 2-[[3-chloro-4-[2-fluoro-4-(hydroxymethyl)phenyl]phenyl]methyl] morpholine-4-carboxylate (Intermediate 7)

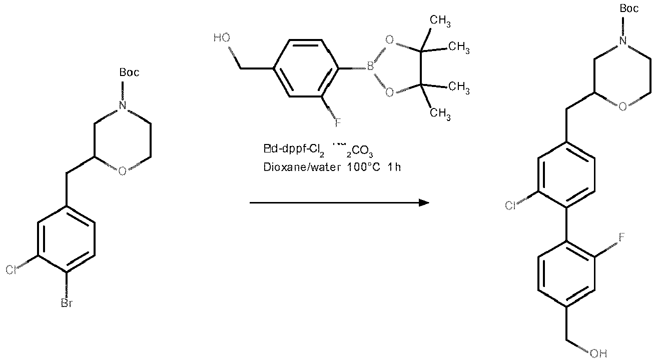

From tert-butyl 2-[(4-bromo-3-chloro-phenyl)methyl]morpholine-4-carboxylate (200 mg, 0.51 mmol) and [3-fluoro-4-(tetramethyl-1,3,2-dioxaborolan-2-yl)phenyl]methanol (162 mg, 0.62 mmol) tert-butyl 2-[[3-chloro-4-[2-fluoro-4-(hydroxymethyl)phenyl]phenyl]methyl]morpholine-4-carboxylate was obtained as an yellow sticky oil (220 mg, 98.6 % yield).

¹H NMR(DMSO-*d*₆, 400 MHz) : δ (ppm) 7.48 (s, J = 1.1 Hz, 1H), 7.34 - 7.27 (m, 3H), 7.24 (s, 1H), 7.23 - 7.20 (m, 1H), 5.36 (t, J = 5.8 Hz, 1H), 4.57 (d, *J* = 5.8 Hz, 2H), 3.81 (d, *J* = 12.0 Hz, 2H), 3.70 (d, J = 13.1 Hz, 1H), 3.64 - 3.52 (m, 1H), 3.31 (m, 1H), 3.01 - 2.55 (m, 4H), 1.40 (s, 9H); m/z = 336 [M-Boc]⁺.

### General procedure B2:

To a solution of Ar-CI or Ar-Br (1.00 eq.) in 1 ,4-dioxane/H₂O (4:1, 0.1M) were added R-B(OH)₂ (or R-Bpin) (1.40 - 2.5 eq.) and K₃PO₄ or K₂CO₃ (2 - 5 eq.). The reaction mixture was degassed with argon for 5 min and Pd₂(dba)₃ (0.1 - 0.5 eq.) and PCys (0.15 - 1 eq.) were added and the reaction mixture was heated at 100°C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column on silica gel (EtOAc in heptane, from 0 to 100%) to afford the desired product.

### Synthesis of tert-butyl rel-(2R)-2-[[3-(4-cyano-3-fluoro-phenyl)-2-isothiazol-5-yl-phenyl] methyl]morpholine-4-carboxylate (Intermediate 8)

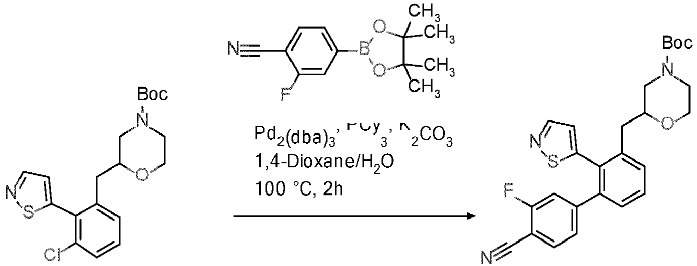

From tert-butyl rel-(2R)-2-[(3-chloro-2-isothiazol-5-yl-phenyl)methyl]morpholine-4-carboxylate (712 mg, 1.78 mmol) and 2-fluoro-4-(tetramethyl-1,3,2-dioxaborolan-2-yl)benzonitrile (1.12 g, 4.46 mmol) tert-butyl rel-(2R)-2-[[3-(4-cyano-3-fluoro-phenyl)-2-isothiazol-5-yl-phenyl]methyl]morpholine-4-carboxylate was obtained as a sticky red oil (601 mg, 67 % yield). ¹H NMR(DMSO-*d*₆, 400 MHz) : δ (ppm) 8.52 (d, *J* = 1.7 Hz, 1H), 7.80 (t, *J* = 8.0, 7.0 Hz, 1H), 7.58 - 7.53 (m, 2H), 7.42 - 7.34 (m, 3H), 7.19 (dd, *J* = 8.0, 1.5 Hz, 1H), 3.81 - 3.50 (m, 1H), 3.40 (m, 1H), 3.25 (dt, J = 11.6, 2.8 Hz, 1H), 2.82 (s, 1H), 2.66 (m, J = 4.9, 4.4 Hz, 2H), 2.51 (m, J = 1.9 Hz, 1H), 1.38 (s, 9H); m/z = 424 [MH-tBu]+.

### General procedure B3:

To a solution of Ar-Br (1.00 eq.) in DMSO (0.14M) were added sequentially R-B(OH)₂ (or R-Bpin) (1- 2 eq.) and Cs₂CO₃ (3 eq.) and KOAc (1 eq.). The reaction mixture was degassed with argon for 5 min and PdCl₂(PPh₃)₂ (0.1 - 0.5 eq.) was added and the mixture was stirred at 120°C for 3 - 12 h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column on silica gel (EtOAc in heptane) to afford the desired product.

### Synthesis of tert-butyl 2-[[3-chloro-2-(4-methylthiazol-5-yl)phenyl]methyl]morpholine-4-carboxylate (Intermediate 9)

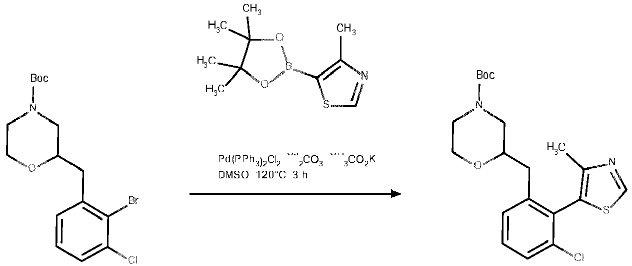

From tert-butyl 2-[(2-bromo-3-chloro-phenyl)methyl]morpholine-4-carboxylate (300 mg, 0.77 mmol) and 4-methyl-5-(4,4,5,5-tetramethyl-1,3,2-dioxaborolan-2-yl)-1,3-thiazole (180 mg, 0.77 mmol), tert-butyl 2-[[3-chloro-2-(4-methylthiazol-5-yl)phenyl]methyl]morpholine-4-carboxylate was obtained as an yellow oil (120 mg, 37 % yield).

¹H NMR(DMSO-*d*₆, 400 MHz) : δ (ppm) 9.17 (s, 1H), 7.51 (m, J = 7.2, 2.3, 1.1 Hz, 1H), 7.48 - 7.39 (m, 2H), 3.68 (dd, J = 31.0, 12.4 Hz, 2H), 3.55 (s, 1H), 3.31 (s, 1H), 3.24 (t, J = 10.6, 9.5, 2.2 Hz, 1H), 2.80 (s, 1H), 2.67 (m, J = 6.0 Hz, 2H), 2.49 (s, 1H), 2.11 (s, J = 0.9 Hz, 3H), 1.36 (s, J = 1.9 Hz, 9H); m/z = 409 [M+H]⁺.

### General procedure B4:

To a stirred solution of Ar-Br (1 eq.) in anhydrous dioxane (0.8 M) at RT under nitrogen were added sequentially R-B(OH)₂ (2 eq.), K₃PO₄ (2 eq.) and Pd(PPh3)4 (0.05 - 0.1 eq). The reaction mixture was stirred at 110°C for 12 h. The suspension was filtered, washed with methanol, then the filtrate was concentrated under reduced pressure. The crude material was purified by flash chromatography on silica gel using a gradient of EtOAc in Heptane. The desired fractions were combined and concentrated to afford the expected compound.

### Synthesis of tert-butyl 2-[(3-chloro-2-cyclopropyl-phenyl)methyl]morpholine-4-carboxylate (Intermediate 10)

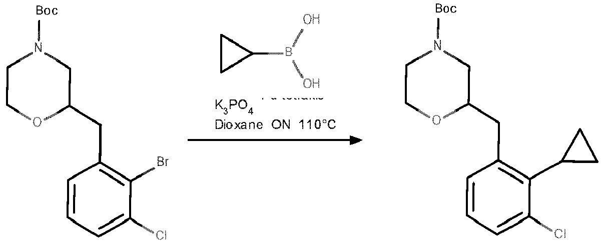

From tert-butyl 2-[(2-bromo-3-chloro-phenyl)methyl]morpholine-4-carboxylate (70 mg, 0.12 mmol) and cyclopropylboronic acid (30 mg, 0.34 mmol), tert-butyl 2-[(3-chloro-2-cyclopropyl-phenyl)methyl]morpholine-4-carboxylate was obtained as a colourless oil.

¹H NMR(DMSO-*d₆*, 400 MHz) : δ (ppm) 7.26 (dd, J= 7.3, 1.9 Hz, 1H), 7.24 -7.14 (m, 2H), 3.83 - 3.54 (m, 4H), 3.38 - 3.33 (m, 1H), 3.12 - 2.94 (m, 2H), 2.87 (br s, 1H), 2.78 - 2.53 (m, 1H), 1.83 - 1.69 (m, 1H), 1.37 (s, 9H), 1.09 (dd, J = 8.7, 4.5 Hz, 2H), 0.60 (dt, J = 35.3, 5.7 Hz, 2H); m/z = 252 [M-Boc]+.

### General procedure B5:

To a solution of Ar-Br (1.00 eq.) in toluene/H₂O (5:1, 0.1M) were added K₃PO₄ (4 eq.), R-B(OH)₂ (1.5 eq.) and PCys (0.1eq.). The reaction mixture was degassed with argon for 5 min and Pd(OAc)₂ (0.1 eq.) was added. The reaction mixture was heated at 100°C for 12 h. The reaction mixture was cooled to rt, diluted with EtOAc and quenched with water. The two layers were separated, and the aqueous layer was extracted three times with EtOAc. The combined organic layers were dried over a phase separator and concentrated under reduced pressure. When necessary, the crude material was purified by flash chromatography column on silica gel to afford the desired product.

### Synthesis of 5-(azetidin-3-yl)-2-cyclopropyl-pyridine (Intermediate 11)

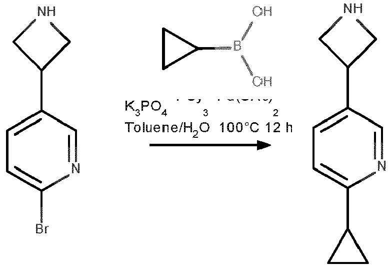

From 5-(azetidin-3-yl)-2-bromopyridine, trifluoroacetic acid (200 mg, 0.61 mmol) and cyclopropylboronic acid (82 mg, 0.92 mmol). 5-(azetidin-3-yl)-2-cyclopropyl-pyridine obtained as a sticky yellow oil (122 mg, 78 %). The crude was engaged in the next step. m/z = 175 [M+H]+.

### General procedure B6:

To a solution of Ar-Br (1.00 eq.) in dioxane/H₂O (4:1, 0.12 M) were added sequentially R-B(OH)₂ (or R-Bpin) (1- 2 eq.) and K3PO4 (2 eq.). The reaction mixture was degassed with argon for 5 min and PdCl₂(PPh₃)₂ (0.05 - 0.1 eq.) was added and the mixture was stirred at 100°C for 3 - 12 h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column on silica gel (using a gradient of EtOAc in heptane) to afford the desired product.

### Synthesis of tert-butyl 2-[(3-chloro-2-isothiazol-5-yl-phenyl)methyl]morpholine-4-carboxylate (Intermediate 12)

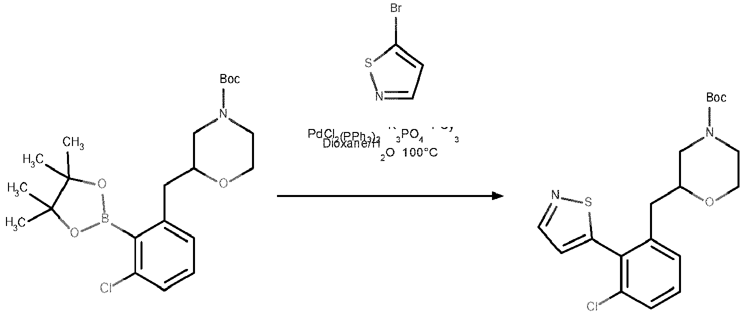

From tert-butyl 2-[[3-chloro-2-(4,4,5,5-tetramethyl-1,3,2-dioxaborolan-2-yl)phenyl]methyl] morpholine-4-carboxylate (1.1 g, 2.42 mmol) and 5-bromo-1,2-thiazole (459 mg, 2.65 mmol tert-butyl 2-[(3-chloro-2-isothiazol-5-yl-phenyl)methyl]morpholine-4-carboxylate obtained as a sticky colourless oil (334 mg, 34 %).

¹H NMR(DMSO-*d₆*, 400 MHz) : δ (ppm) 8.70 (d, J = 1.7 Hz, 1H), 7.59 - 7.37 (m, 4H), 3.73 (d, J = 11.5 Hz, 1H), 3.64 (d, J = 13.4 Hz, 1H), 3.58 (m, 1H), 3.38 (m, 1H), 3.29 - 3.20 (td, 1H), 2.80 (m, 1H), 2.64 - 2.53 (m, 3H), 1.38 (s, 9H); m/z = 395 [M+H]+.

### General procedure B7:

To a solution of Ar-Br (1.00 eq.) in dioxane (0.09 M) under Argon were added Xantphos (0.28 eq.), sodium *tert*-butoxide (3 eq.) and Pd₂(dba)₃ (0.1 eq.). The reaction mixture was degassed with argon for 5 min and potassium cyclopropyl(trifluoro)boranuide (1 eq.) was added. The mixture was stirred at 90°C for 12 h. The reaction mixture was cooled to rt, water was added and the mixture was extracted with EtOAc twice. The combined organic layers were dried using a phase separator and concentrated under reduced pressure. The crude was purified by flash chromatography column on silica gel (using a gradient of EtOAc in heptane) to afford the desired product.

### Synthesis of tert-butyl 2-[(3-chloro-4-cyclopropyl-phenyl)methyl]morpholine-4-carboxylate (Intermediate 13)

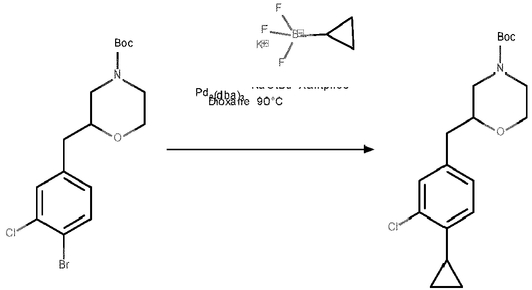

From tert-butyl 2-[(4-bromo-3-chloro-phenyl)methyl]morpholine-4-carboxylate (200 mg, 0.51 mmol) and potassium cyclopropyl(trifluoro)boranuide (78 mg, 0.51 mmol) tert-butyl 2-[(3-chloro-4-cyclopropyl-phenyl)methyl]morpholine-4-carboxylate obtained as a yellow oil (100 mg, purity 50%, 30 % yield). m/z = 296 [M+H]⁺ - (t-Bu).

### General procedure B8_{:}

To a solution of Ar-Br (1.00 eq.) in dioxane/water (0.12M) under Argon were added 4-bromo-1 ,3-oxazole hydrochloride (1.05 eq.) and disodium;carbonate (3 eq.) and Pd₂(dba)₃ (0.1 eq.). The reaction mixture was degassed with argon for 5 min and palladium triphenylphosphane (0.05 eq.) was added. The mixture was stirred at 110°C for 6 h. The reaction mixture was cooled to rt, water and DCM were added and the aqueous layer was extracted with DCM (three times). The combined organic layers were dried using a phase separator and concentrated under reduced pressure. The crude was purified by flash chromatography column on silica gel (using a gradient of EtOAc in heptane) to afford the desired product.

### Synthesis of tert-butyl 2-[(3-chloro-2-oxazol-4-yl-phenyl)methyl]morpholine-4-carboxylate (Intermediate 14)

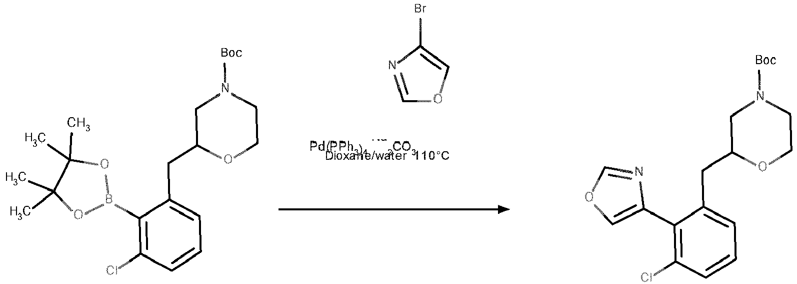

From tert-butyl 2-[[3-chloro-2-(4,4,5,5-tetramethyl-1,3,2-dioxaborolan-2-yl)phenyl]methyl] morpholine-4-carboxylate (100 mg, 0.22 mmol) and 4-bromo-1,3-oxazole hydrochloride (45 mg, 0.23 mmol, 1.05 eq.) tert-butyl 2-[(3-chloro-2-oxazol-4-yl-phenyl)methyl]morpholine-4-carboxylate obtained as a colourless oil (24 mg, 26 % yield).

¹H NMR(DMSO-*d₆*, 400 MHz) : δ (ppm) 8.55 (d, *J* = 0.9 Hz, 1H), 8.26 (d, *J* = 0.9 Hz, 1H), 7.45 (dd, *J* = 7.8, 1.6 Hz, 1H), 7.39 (t, *J* = 7.7 Hz, 1H), 7.35 (dd, *J* = 7.6, 1.4 Hz, 1H), 3.72 (dd, *J* = 11.6, 2.6 Hz, 1H), 3.67-3.54 (m, 2H), 3.41-3.34 (m, 1H), 3.24 (td, *J* = 11.6, 2.8 Hz, 1H), 2.87-2.59 (m, 4H), 1.37 (s, 9H); m/z = 279 [M+H]⁺ - *(Boc).*

### General procedure B9:

To a solution of Ar-BPin (1.00 eq.) in dioxane (0.2M) under Argon were added 4,4,5,5-tetramethyl-2-(4,4,5,5-tetramethyl-1,3,2-dioxaborolan-2-yl)-1,3,2-dioxaborolane (1.2 eq.), tricyclohexylphosphane (0.75 eq.) and potassium;acetate (1 eq.). The reaction mixture was degassed with argon for 5 min and Pd₂(dba)₃ (0.05 eq.) was added. The mixture was stirred at 100°C for 7 h. The reaction mixture was cooled to rt, water was added and the mixture was extracted with EtOAc (three times). The combined organic layers were washed with brine, dried over a phase separator and concentrated under reduced pressure. The crude was purified by flash chromatography column on silica gel (using a gradient of EtOAc in heptane from 0 to 100%) to afford the desired product.

### Synthesis of tert-butyl 2-[[2-(1-methylpyrazol-4-yl)-3-(4,4,5,5-tetramethyl-1,3,2-dioxaborolan-2-yl)phenyl]methyl]morpholine-4-carboxylate (Intermediate 15)

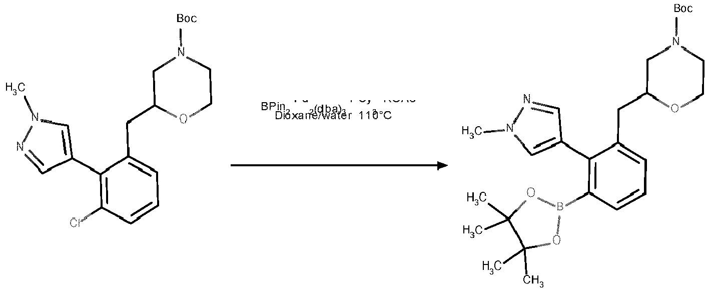

From tert-butyl 2-[[3-chloro-2-(1-methylpyrazol-4-yl)phenyl]methyl]morpholine-4-carboxylate (65 mg, 0.16 mmol) and 4,4,5,5-tetramethyl-2-(4,4,5,5-tetramethyl-1,3,2-dioxaborolan-2-yl)-1,3,2-dioxaborolane (51 mg, 0.19 mmol, 1.2 eq.) tert-butyl 2-[[2-(1-methylpyrazol-4-yl)-3-(4,4,5,5-tetramethyl-1,3,2-dioxaborolan-2-yl)phenyl]methyl]morpholine-4-carboxylate obtained as a colourless oil (15 mg, 18.6 % yield). m/z = 484.2 [M+H]⁺.

### Method C: C-H activation

### General procedure C1

To a solution of Ar-Br (1 eq.) in DMA (0.2 M) were added K₂CO₃ (3 eq.), cataCXium^{®} (0.1 eq.) and pivalic acid (0.4 eq.). The reaction mixture was degassed with argon for 5 min and Pd(OAc)2 (0.05 eq.) followed by Ar-H (1 eq.) were added. The reaction mixture was stirred at 110°C for 4 h. The reaction mixture was cooled to rt, filtered and concentrated under high vacuum. The crude material was purified by flash chromatography column on silica gel (EtOAc in heptane, from 0 to 100%) to afford the desired product.

### Synthesis of tert-butyl 2-[[3-chloro-2-(2-methyloxazol-5-yl)phenyl]methyl]morpholine-4-carboxylate (Intermediate 16)

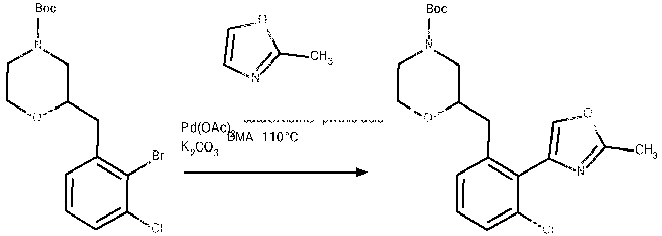

From tert-butyl 2-[(2-bromo-3-chloro-phenyl)methyl]morpholine-4-carboxylate (100 mg, 0.256 mmol) by 2-methyl-1,3-oxazole (22 mg, 0.256 mmol tert-butyl 2-[[3-chloro-2-(2-methyloxazol-5-yl)phenyl]methyl]morpholine-4-carboxylate was obtained as an yellow oil (69 mg, 68.6 % yield). m/z = 393 [M-Boc]+.

### General procedure C2

A sealed tube was charged with Pd(OAc)₂ (0.1 eq.), K₂CO₃ (2 eq.), 2,2-dimethylpropanoic acid (0.4 eq.) and tricyclohexylphosphonium tetrafluoroborate (0.15). The tube was purged with argon 3 times.

DMA (0.2 M) was added followed by Ar-Br (1 eq.) and Ar-H ( (1.3 eq.).The resulting mixture was stirred at 110°C for 5h. Water was added and the mixture extracted with AcOEt three times. The combined organic layers were washed with water, brine, dried over phase separator and concentrated under reduced pressure. The crude material was purified by flash chromatography column (EtOAc in heptane) to afford the desired product.

### Synthesis of tert-butyl 2-[(3-chloro-2-thiazol-5-yl-phenyl)methyl]morpholine-4-carboxylate (Intermediate 17)

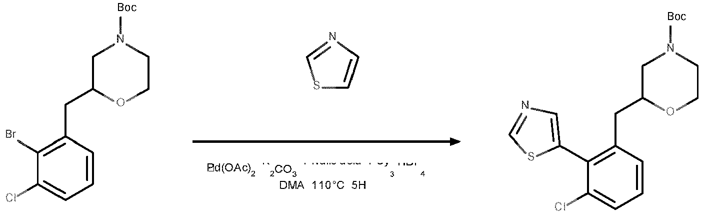

From tert-butyl 2-[(2-bromo-3-chloro-phenyl)methyl]morpholine-4-carboxylate (600 mg, 1.53 mmol) and 1,3-thiazole (1.3 eq), tert-butyl 2-[(3-chloro-2-thiazol-5-yl-phenyl)methyl]morpholine-4-carboxylate was obtained as an orange oil (232 mg, 38.2% yield).

¹H NMR(DMSO-*d₆*, 400 MHz) : δ (ppm) 9.29 (d, *J* = 0.5 Hz, 1H), 7.84 (d, *J* = 0.6 Hz, 1H), 7.53 - 7.37 (m, 3H), 3.80 - 3.49 (m, 3H), 3.43 - 3.33 (m, 1H), 3.31 - 3.18 (m, 1H), 2.81 (s, 1H), 2.63 - 2.55 (m, 2H), 1.37 (s, 9H); m/z = 395.1 [M]⁺

### General procedure C3:

A vial was charged with Ar-H (1eq.), Ar-Br (1.04 eq.), Pd(OAc)₂ (0.05 eq.), QPhos (0.075 eq.) and tetrabutylammonium;acetate (3 eq.) in anhydrous Dioxane (0.4 M) under Argon. The mixture was stirred at 105°C for 12 h. Water was added and the mixture extracted with AcOEt three times. The combined organic layers were washed with water, brine, dried over phase separator and concentrated under reduced pressure. The crude material was purified by flash chromatography column (using a gradient of EtOAc in heptane from 0 to 100 %) to afford the desired product.

### Synthesis of tert-butyl 2-[[3-chloro-2-(1-methylpyrazol-4-yl)phenyl]methyl]morpholine-4-carboxylate (Intermediate 18)

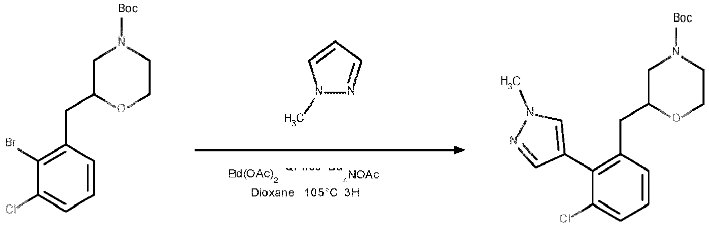

From tert-butyl 2-[(2-bromo-3-chloro-phenyl)methyl]morpholine-4-carboxylate (1 g, 2.53 mmol, 1.04 eq) and 1-methyl-1H-pyrazole (1 eq.), tert-butyl 2-[[3-chloro-2-(1-methylpyrazol-4-yl)phenyl]methyl]morpholine-4-carboxylate was obtained as a brown oil (125 mg, 13 % yield). ¹H NMR(DMSO-*d₆*, 400 MHz) : δ (ppm) 7.75 (s, 1H), 7.42 - 7.38 (m, 2H), 7.34 - 7.25 (m, 2H), 3.91 (s, 3H), 3.73 (dd, *J* = 11.6, 2.5 Hz, 1H), 3.64 (d, *J* = 13.4 Hz, 1H), 3.56 (d, *J* = 9.4 Hz, 1H), 3.36 (ddt, *J* = 9.2, 6.6, 3.1 Hz, 1H), 3.30 - 3.21 (m, 1H), 2.82 (s, 1H), 2.65 (d, *J* = 6.3 Hz, 2H), 2.50 (s, 1H), 1.38 (s, 10H); m/z = 392 [M+H]⁺

### Method D: deprotection

### - N-Boc deprotection

### General procedure D1:

To a solution of Boc-*N* product (1.00 eq.) in DCM (0.2M) was added 2,2,2-trifluoroacetic acid (15-20 eq.). The reaction mixture was stirred at rt for 1-24h. The reaction mixture was diluted with DCM and an aqueous saturated solution of NaHCOs was added. The two layers were separated and the aqueous layer was extracted three times with DCM. The combined organic layers were dried over a phase separator and concentrated under reduced pressure. When necessary, the crude was purified by flash chromatography column (using a gradient of MeOH in DCM) to afford the desired product. The product was isolates either as a free amine or as a tartrate salt. Tartrate salt formation: (2{R},3{R})-2,3-dihydroxybutanedioic acid (0.50 eq.) was solubilized in H₂O (3 mL) and the product in ACN (0.3 mL) was added. The residue was freeze-dried overnight to afford the desired product as a tartrate salt.

### Example 113: (2R,3R)-2,3-dihydroxybutanedioic acid;2-fluoro-4-[2-(5-methylisothiazol-4-yl)-3-(morpholin-2-ylmethyl)phenyl] benzonitrile

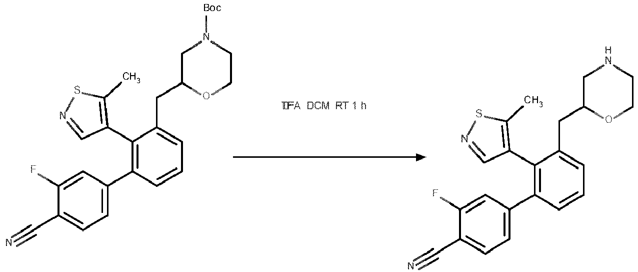

From tert-butyl 2-[[3-(4-cyano-3-fluoro-phenyl)-2-(5-methylisothiazol-4-yl)phenyl]methyl] morpholine-4-carboxylate (64 mg, 0.12 mmol), (2R,3R)-2,3-dihydroxybutanedioic acid 2-fluoro-4-[2-(5-methylisothiazol-4-yl)-3-(morpholin-2-ylmethyl)phenyl]benzonitrile obtained as a white powder (41 mg, 40 % yield).

¹H NMR (500 MHz, DMSO-d₆): δ (ppm) 8.45 - 8.24 (m, 1H), 7.80 - 7.74 (m, 1H), 7.56 - 7.46 (m, 2H), 7.37 (dt, *J* = 6.7, 2.1 Hz, 1H), 7.24 (ddd, *J* = 10.6, 4.0, 1.5 Hz, 1H), 7.06 (ddd, *J* = 8.0, 4.7, 1.7 Hz, 1H), 3.76 - 3.71 (m, 1H), 3.44 (br d, *J* = 2.7 Hz, 2H), 2.87 - 2.77 (m, 1H), 2.75 - 2.66 (m, 2H), 2.63 - 2.53 (m, 1H), 2.48 - 2.31 (m, 2H), 2.05 - 1.93 (m, 3H) m/z = 394 [M+H]+

### General procedure D2:

To a solution of Boc-*N* product (1.00 eq.) in DCM (0.1M) was added HCl in 1,4-dioxane or IPA (2M, 30.0 eq.). The reaction mixture was stirred at rt for 1-24h. The reaction mixture was concentrated under reduced pressure to afford the desired product as a HCl salt. When necessary, the crude was purified by flash chromatography column (using a gradient of MeOH in DCM 0 to 10%). Same procedure as D1 if the tartaric salt is prepared from the free amine after work-up with NaHCOs/DCM.

### Example 5: 4-[2-isothiazol-5-yl-3-[[(2R)-morpholin-2-yl]methyl]phenyl]benzonitrile hydrochloride

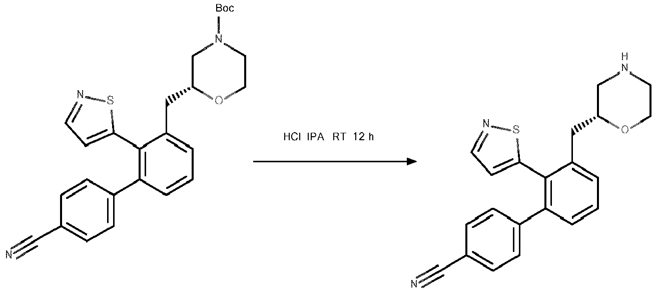

From tert-butyl (2R)-2-[[3-(4-cyanophenyl)-2-isothiazol-5-yl-phenyl]methyl]morpholine-4-carboxylate (203 mg, 0.44 mmol), 4-[2-isothiazol-5-yl-3-[[(2R)-morpholin-2-yl]methyl]phenyl]benzonitrile hydrochloride obtained as a beige solid (116 mg, 63 % yield). ¹H NMR (600 MHz, DMSO-d6): δ (ppm) 2.64 - 2.73 (m, 3 H) 2.91 (td, J=12.51, 3.89 Hz, 1 H) 3.03 (br d, J=12.32 Hz, 1 H) 3.11 (br d, J=12.62 Hz, 1 H) 3.59 (td, J=12.43, 2.27 Hz, 1 H) 3.74 - 3.81 (m, 1 H) 3.89 (dd, J=12.62, 3.52 Hz, 1 H) 7.32 - 7.36 (m, 4 H) 7.50 (d, J=7.19 Hz, 1 H) 7.56 (t, J=7.70 Hz, 1 H) 7.71 (d, J=8.22 Hz, 2 H) 8.49 (d, J=1.61 Hz, 1 H) 8.99 (br s, 2 H); m/z = 362 [M+H]⁺

### - O-TBS deprotection

### General procedure D3:

To a solution of R-OTBS (1 eq.) in anhydrous THF (0.1M) was added tetrabutylammonium fluoride (1.3 eq.) at 0°C under argon. The reaction mixture was stirred at rt for 12h then at reflux further 12h. Water and EtOAc were added. The aqueous layer was extracted with EtOAc (x3). The combined organic layers were dried using a phase separator and concentrated under reduced pressure. The crude was purified by flash chromatography column (using a gradient of MeOH in DCM 0 to 10%) to afford the desired product.

### Synthesis of tert-butyl 2-[[3-(4-cyano-3-fluoro-phenyl)-4-(4-hydroxy-4-methyl-1-piperidyl) phenyl]methyl] morpholine-4-carboxylate (Intermediate 19)

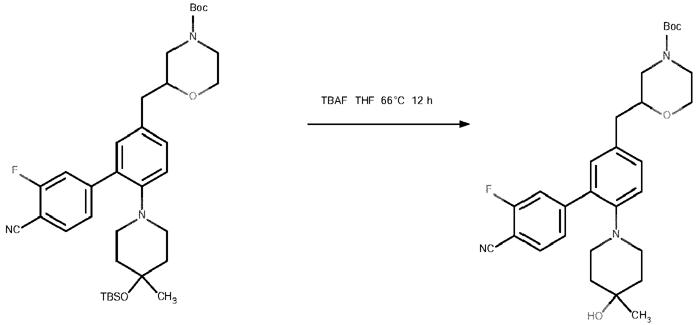

From tert-butyl 2-[[4-[4-[tert-butyl(dimethyl)silyl]oxy-4-methyl-1-piperidyl]-3-(4-cyano-3-fluorophenyl)phenyl]methyl]morpholine-4-carboxylate (64 mg, 0.10 mmol), tert-butyl 2-[[3-(4-cyano-3-fluoro-phenyl)-4-(4-hydroxy-4-methyl-1-piperidyl)phenyl]methyl]morpholine-4-carboxylate obtained as a white solid (30 mg, 58 % yield).

¹H NMR (400 MHz, DMSO-d6): δ (ppm) 7.95 (m, 1H), 7.83 - 7.78 (m, 1H), 7.73 - 7.68 (m, 1H), 7.22 (dd, *J* = 8.3, 2.1 Hz, 1H), 7.17 (d, *J* = 2.1 Hz, 1H), 7.08 (d, *J* = 8.2 Hz, 1H), 4.18 (s, 1H), 3.81 - 3.70 (m, 2H), 3.70 - 3.64 (m, 1H), 3.55 - 3.46 (m, 1H), 3.37 - 3.32 (m, 1H), 2.88 - 2.78 (m, 3H), 2.72 - 2.52 (m, 5H), 1.42 - 1.38 (m, 4H), 1.36 (s, 9H), 1.10 (s, 3H); m/z = 510 [M+H]+

### - N-PMB deprotection

### General procedure D4:

To a stirred solution of CAN (6 eq.) in ACN/water (0.05 M) was added at 0°C (R₂)N-PMB in ACN. The reaction mixture was stirred at rt for 1h20. CAN was evaporated and the mixture extracted with EtOAc. The combined organic layers were dried using a phase separator and concentrated under reduced pressure. The crude was purified by flash chromatography column (using a gradient of MeOH in DCM 0 to 10%) to afford the desired product.

### Synthesis of 2-[(3-bromo-4-iodo-5-methyl-phenyl)methyl]morpholin-3-one (Intermediate 20)

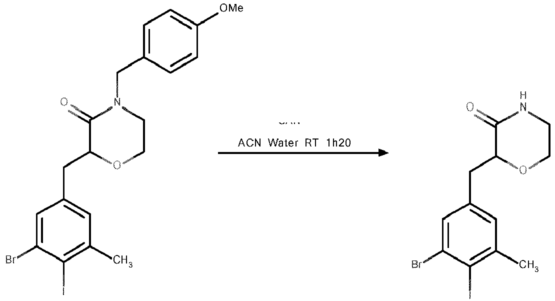

From 2-[(3-bromo-4-iodo-5-methyl-phenyl)methyl]-4-[(4-methoxyphenyl)methyl]morpholin-3-one (796 mg, 1.08 mmol), 2-[(3-bromo-4-iodo-5-methyl-phenyl)methyl]morpholin-3-one obtained as a white solid (342 mg, 75 % yield).

¹H NMR (400 MHz, DMSO-d6): δ (ppm) 7.99 (s, 1H), 7.42 (d, *J* = 1.9 Hz, 1H), 7.19 (d, *J* = 2.0 Hz, 1H), 4.24 (dd, *J* = 8.6, 3.5 Hz, 1H), 3.94 - 3.83 (m, 1H), 3.60 (ddd, *J* = 11.8, 9.9, 3.6 Hz, 1H), 3.23 (td, *J* = 11.2, 4.3 Hz, 1H), 3.18 - 3.03 (m, 2H), 2.83 (dd, *J*= 14.4, 8.6 Hz, 1H), 2.47 (s, 3H); m/z = 411 [M+H]+

### - N-CBz deprotection

### General procedure D5:

A sealed vial was charged with R-N-CBz (1 eq.) and ammonium formate (10 eq.) in IPA (0.12 M) and Water (1%v). The reaction mixture was degassed with nitrogen for 10 min, palladium (10%, 0.25 eq.) was added under argon and the reaction mixture was stirred at 60°C for 5h.The reaction mixture was allowed to cool to RT, filtered on a pad of celite and washed with IPA. The filtrate was concentrated under reduced pressure to afford the expected compound

### Synthesis of 5-(azetidin-3-yl)-2-methoxy-pyridine (Intermediate 21)

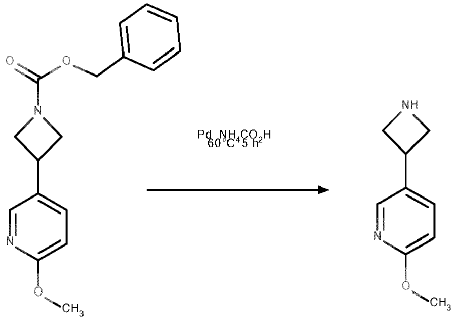

From benzyl 3-(6-methoxy-3-pyridyl)azetidine-1-carboxylate (370 mg, 1.24 mmol), 5-(azetidin-3-yl)-2-methoxy-pyridine obtained as a beige solid (148 mg, purity 90, 65 % yield).

¹H NMR (400 MHz, DMSO-d6): δ (ppm) 8.38 (s, 1H), 8.12 (d, *J* = 2.5 Hz, 1H), 7.85 (dd, *J* = 8.5, 2.5 Hz, 1H), 6.84 (d, *J* = 8.6 Hz, 1H), 4.05 - 3.76 (m, 7H).

### General procedure D6:

To a stirred solution of (R₂)N-PMB in Toluene (0.10 M) at rt under N2 was added methanesulfonic acid (5 eq.). The reaction mixture was stirred at 120°C for 2 h. The reaction mixture was basified with a saturated aqueous solution of NaHCOs and DCM was added. The phases were separated and the aqueous phase was extracted with DCM (3x). The reaction mixture was concentrated under reduced pressure. The crude material was purified by flash chromatography on silica gel (using a gradient of AcOEt in Heptane from 0% to 100%) to afford the expected compound.

### Synthesis of 3-[(2-bromo-3-chloro-phenyl)methyl]piperidin-2-one (Intermediate 22)

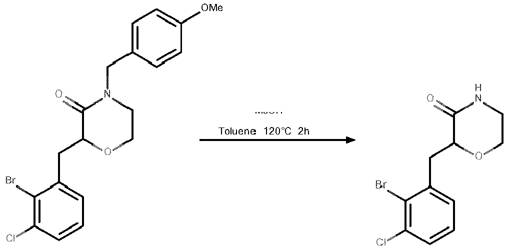

From 3-[(2-bromo-3-chloro-phenyl)methyl]-1-[(4-methoxyphenyl)methyl]piperidin-2-one (250 mg, 0.59 mmol), 3-[(2-bromo-3-chloro-phenyl)methyl]piperidin-2-one obtained as a white solid (131 mg, 73 % yield).

¹H NMR (400 MHz, DMSO-d6): δ (ppm) 7.57 - 7.43 (m, 2H), 7.41 - 7.23 (m, 2H), 3.49 (dd, *J* = 13.6, 4.4 Hz, 1H), 3.12 (t, *J* = 6.1 Hz, 2H), 2.72 (dd, *J* = 13.6, 10.5 Hz, 1H), 2.54 (d, *J* = 4.7 Hz, 1H), 1.82 - 1.67 (m, 1H), 1.67 - 1.47 (m, 2H), 1.47 - 1.29 (m, 1H).; m/z = 304 [M+H]+

### Method E: Halogenation

### General procedure E1:

### Aromatic bromination NH2 directed

To a solution of aminoaryl product (1.00 eq.) in DCM (0.2 M) was added NBS (1-2 eq.) at 0°C or RT under N2 atmosphere. The reaction mixture was stirred at the same temperature for 2 h, quenched with a NaHCOs saturated aqueous solution and extracted with DCM three times. The combined organic layers were dried over a phase separator and concentrated under reduced pressure. The crude material was purified by flash chromatography column (using a gradient of EtOAc in heptane) to afford the desired product.

### Synthesis of tert-butyl 2-[[3-bromo-4-(4-pyrimidin-2-yl-1-piperidyl)phenyl]methyl] morpholine-4-carboxylate (Intermediate 23)

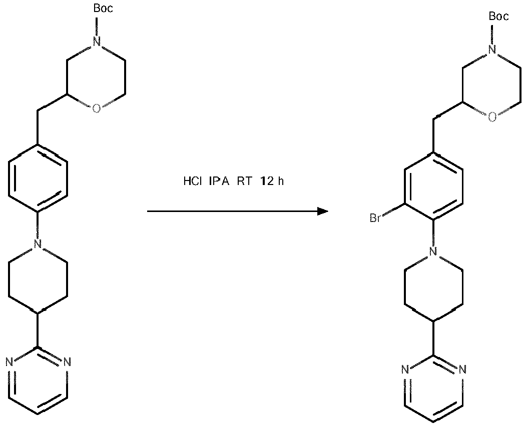

From tert-butyl 2-[[4-(4-pyrimidin-2-yl-1-piperidyl)phenyl]methyl]morpholine-4-carboxylate (246 mg, 0.56 mmol), tert-butyl 2-[[3-bromo-4-(4-pyrimidin-2-yl-1-piperidyl)phenyl]methyl]morpholine-4-carboxylate obtained as a yellow solid (254 mg, 80 % yield).

¹H NMR (400 MHz, DMSO-d₆): δ (ppm)8.77 (d, *J* = 4.9 Hz, 2H), 7.48 (d, *J* = 2.0 Hz, 1H), 7.36 (t, *J* = 4.9 Hz, 1H), 7.21 (dd, *J* = 8.2, 2.0 Hz, 1H), 7.12 (d, *J* = 8.2 Hz, 1H), 3.84 - 3.60 (m, 5H), 3.52 - 3.42 (m, 2H), 2.96 (dt, *J* = 10.3, 5.2 Hz, 1H), 2.93 - 2.74 (m, 4H), 2.67 (d, *J* = 6.0 Hz, 3H), 2.00 (td, *J* = 11.1, 10.2, 4.8 Hz, 4H), 1.38 (s, 9H).; m/z = 519 [M+H]+

Alternatively Bromination (NBS) or Chlorination (NCS) could be carried out in DMF at 80 to 100°C for 30min to 3h.

### Synthesis of tert-butyl 2-[[4-(3-chloro-2-methyl-indazol-5-yl)-3-(4-cyano-3-fluoro-phenyl) phenyl]methyl]morpholine-4-carboxylate (Intermediate 24)

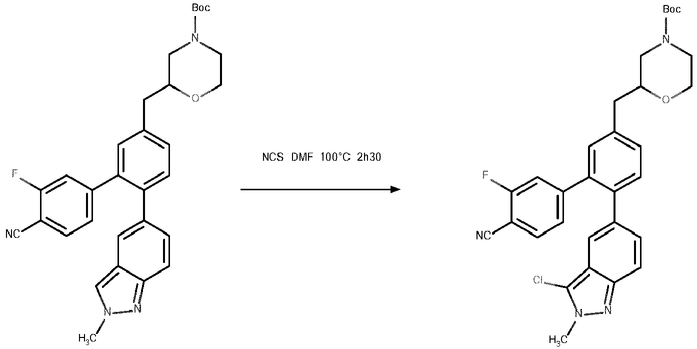

Tert-butyl 2-[[3-(4-cyano-3-fluoro-phenyl)-4-(2-methylindazol-5-yl)phenyl]methyl]morpholine-4-carboxylate (11 mg, 0.0209 mmol) was dissolved in DMF-Anhydrous (1 mL). The RM was degassed under N2, then 1-chloropyrrolidine-2,5-dione (3.1 mg, 0.0230 mmol) was added. The RM was stirred at 100°C for 2h30 then concentrated under reduced pressure. The crude was purified by flash column chromatography on silica gel (using a gradient of MeOH in from 0% to 0.5 %) to afford the desired product as a white solid(4 mg, 34 % yield).

¹H NMR (400 MHz, DMSO-d₆): δ (ppm) 7.80 - 7.71 (m, 1H), 7.51 - 7.34 (m, 6H), 7.10 (dd, J = 8.1, 1.5 Hz, 1H), 6.89 (dd, J = 8.9, 1.7 Hz, 1H), 4.12 (s, 3H), 3.80 (d, J = 8.7 Hz, 2H), 3.65 (dd, J = 29.0, 10.1 Hz, 2H), 3.37 (td, J = 11.5, 2.7 Hz, 1H), 2.85 (q, J = 6.7, 5.2 Hz, 4H), 1.38 (s, 9H); m/z = 562[M+H]+

### - With DAST

### General procedure E2:

To a solution of aldehyde (1.00 eq.) in DCM (0.1 M) was added DAST (3 eq.) at 0°C under N2 atmosphere. The reaction mixture was allowed to warm-up to rt and stirred at rt for 2-5 h. The reaction mixture was concentrated under reduced pressure and the crude material purified by flash chromatography on silica gel (using a gradient of AcOEt in Heptane) to afford the desired product.

### Synthesis of tert-butyl 2-[[3-chloro-4-[4-(difluoromethyl)-1-piperidyl]phenyl]methyl] morpholine-4-carboxylate (Intermediate 25)

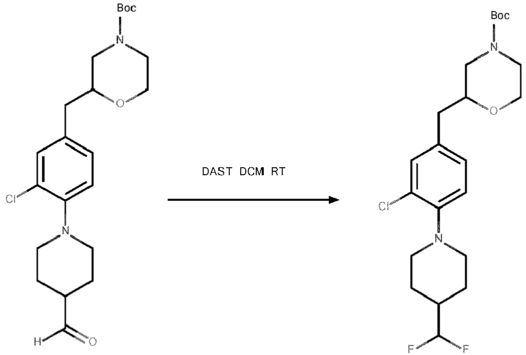

From tert-butyl 2-[[3-chloro-4-(4-formyl-1-piperidyl)phenyl]methyl]morpholine-4-carboxylate (300 mg, 0.71 mmol), tert-butyl 2-[[3-chloro-4-[4-(difluoromethyl)-1-piperidyl]phenyl]methyl] morpholine-4-carboxylate obtained as a yellow oil (139 mg, 41 % yield).

¹H NMR (400 MHz, DMSO-d₆): δ (ppm) 7.30 (d, *J* = 2.0 Hz, 1H), 7.16 (dd, *J* = 8.2, 2.0 Hz, 1H), 7.07 (d, *J* = 8.2 Hz, 1H), 5.99 (td, *J* = 56.8, 4.6 Hz, 1H), 3.92 - 3.58 (m, 3H), 3.55 - 3.40 (m, 1H), 3.31 (m, 4H), 2.85 (s, 1H), 2.74 - 2.55 (m, 4H), 1.98 - 1.85 (m, 1H), 1.78 (d, *J* = 12.2 Hz, 2H), 1.55 (qd, *J* = 12.4, 4.0 Hz, 2H), 1.38 (s, 9H).; m/z = 445 [M]+

### - With NFSI

### General procedure E3:

A solution of Ar-H (1.00 eq.) in THF (0.06 M) was degased with N2 (bubbling 5 min) then cooled to -78°C. A solution of 1.3 M LiHMDS (2 eq.) was added dropwise and the mixture was stirred at -78°C for 1 h. NFSI (2 eq.) was added and the mixture stirred at -78°C for an hour before warming up to rt and stirring for 12 h. The MR was quenched with NH₄Cl saturated aqueous solution and was extracted with MeTHF (twice).The combined organic layers were dried over phase separator and evaporated under reduced pressure. The crude material purified by flash chromatography on silica gel (using a gradient of AcOEt in cyclohexane) to afford the desired product.

### Synthesis of tert-butyl rel-(2R)-2-[[3-chloro-2-(4-fluorooxazol-5-yl)phenyl]methyl] morpholine-4-carboxylate (Intermediate 26)

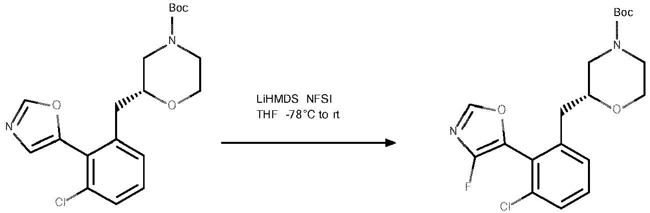

From tert-butyl rel-(2R)-2-[(3-chloro-2-oxazol-5-yl-phenyl)methyl]morpholine-4-carboxylate (596 mg, 1.55 mmol), tert-butyl rel-(2R)-2-[[3-chloro-2-(4-fluorooxazol-5-yl)phenyl]methyl]morpholine-4-carboxylate obtained as a yellow oil (283 mg, 41 % yield).

¹H NMR (400 MHz, DMSO-*d*₆) δ (ppm) 8.53 (d, *J* = 2.1 Hz, 1H), 7.59 - 7.50 (m, 2H), 7.47 (dd, *J* = 5.4, 3.7 Hz, 1H), 3.77 - 3.58 (m, 4H), 3.26 (td, *J* = 11.6, 2.8 Hz, 1H), 2.80 (s, 1H), 2.72 - 2.61 (m, 2H), 1.37 (s, 9H); m/z = 340 [M-tBu]+

### - alcohol to alkylbromide (Appel)

### General procedure E4:

To a solution of R-OH (1.00 eq.) in DCM (0.07 M) was added triphenylphosphane (1.1 eq.) and carbon tetrabromide (1.1). The mixture was stirred at room temperature for 1h30. DCM was added and the mixture was washed with brine. The organic layer was concentrated under reduced pressure. The crude material was purified by flash chromatography on silica gel (using a gradient of AcOEt in cyclohexane from 0 to 100%) afford the desired product.

### Synthesis of 1-bromo-5-(bromomethyl)-2-iodo-3-methyl-benzene (Intermediate 27)

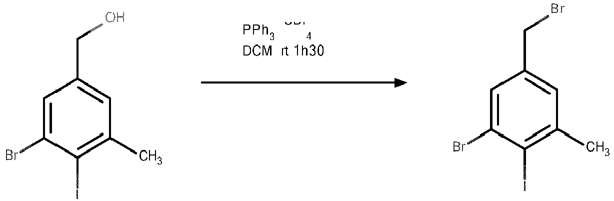

From (3-bromo-4-iodo-5-methyl-phenyl)methanol (2.47 g, 7.5 mmol), 1-bromo-5-(bromomethyl)-2-iodo-3-methyl-benzene obtained as a white solid (2.19 g, 75 % yield).

¹H NMR (400 MHz, DMSO-*d*₆) δ (ppm) 7.65 (d, *J* = 2.1 Hz, 1H), 7.39 (d, *J* = 2.3 Hz, 1H), 4.62 (s, 2H), 2.50 (s, 3H); m/z = 310 [M-Br]+

### Method F: Reduction

### General procedure F1:

To a solution of ester (1.00 eq.) in anhydrous MeOH (0.1M) under nitrogen atmosphere at 0 °C was added NaBH₄ (2 - 6 eq.). The reaction mixture was stirred at rt for 1 h. Brine and DCM were added. The two layers were separated and the aqueous layer was extracted three times with DCM. The combined organic layers were dried over a phase separator and concentrated under reduced pressure. The crude material was purified by flash chromatography column (EtOAc in heptane) to afford the desired product.

### Synthesis of tert-butyl 2-[[3-chloro-4-[3-(hydroxymethyl)azetidin-1-yl]phenyl]methyl] morpholine-4-carboxylate (Intermediate 28)

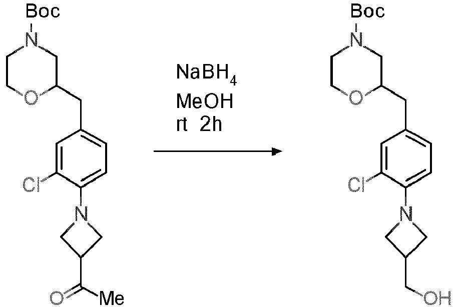

From tert-butyl 2-[[3-chloro-4-(3-methoxycarbonylazetidin-1-yl)phenyl]methyl]morpholine-4-carboxylate (100 mg, 0.21 mmol), tert-butyl 2-[[3-chloro-4-[3-(hydroxymethyl)azetidin-1-yl]phenyl]methyl]morpholine-4-carboxylate was obtained as colourless oil (70 mg, 81 %).

¹H NMR (DMSO-*d*₆, 400 MHz) *δ* (ppm) 7.10 (d, *J* = 1.9 Hz, 1H), 7.02 (dd, *J* = 8.3, 2.0 Hz, 1H), 6.51 (d, *J* = 8.3 Hz, 1H), 4.71 (t, *J* = 5.3 Hz, 1H), 4.18 - 3.85 (m, 3H), 3.84 - 3.61 (m, 5H), 3.57 (dd, *J* = 6.4, 5.3 Hz, 2H), 3.45 - 3.32 (m, 2H), 2.85 (s, 1H), 2.69 (tt, *J* = 7.8, 5.9 Hz, 1H), 2.60 (d, *J* = 6.2 Hz, 2H), 1.38 (s, 9H).; m/z = 397 [M+H]+.

### General procedure F2:

To a solution of ester (1.00 eq.) in anhydrous THF (0.1M) under nitrogen atmosphere at 0 °C, was added dropwise LiBH₄ 4M solution (1 - 2 eq.). The reaction mixture was stirred at 0°C for 1 h then at rt for 12h. The reaction mixture was quenched with a saturated aqueous solution of NH₄Cl and DCM was added. The phases were separated and the aqueous phase was extracted with DCM three times. The combined organic layers were dried using a phase separator and concentrated under vacuum. The crude material was purified by flash chromatography on silica gel (using a gradient of AcOEt in Cyclohexane) to afford the desired product.

### Synthesis of tert-butyl 2-[[3-chloro-4-[4-(hydroxymethyl)-1-piperidyl]phenyl]methyl] morpholine-4-carboxylate (Intermediate 29)

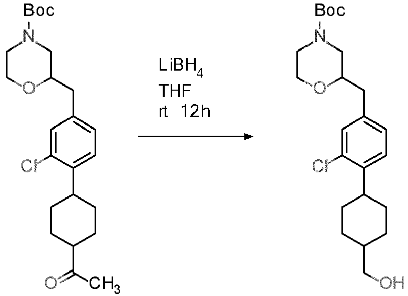

From tert-butyl 2-[[3-chloro-4-(4-methoxycarbonyl-1-piperidyl)phenyl]methyl]morpholine-4-carboxylate (280 mg, 0.57 mmol), tert-butyl 2-[[3-chloro-4-[4-(hydroxymethyl)-1-piperidyl]phenyl]methyl]morpholine-4-carboxylate obtained as colourless oil (270 mg, quant.).

¹H NMR (DMSO-*d*₆, 400 MHz): *δ* (ppm) 7.28 (d, *J* = 2.0 Hz, 1H), 7.14 (dd, *J* = 8.2, 2.0 Hz, 1H), 7.06 (d, *J* = 8.2 Hz, 1H), 4.47 (t, *J* = 5.3 Hz, 1H), 3.83 - 3.62 (m, 3H), 3.46 (dtd, *J* = 9.1, 6.5, 2.5 Hz, 1H), 3.31 (m, 3H), 3.23 (d, *J* = 11.5 Hz, 2H), 2.85 (s, 1H), 2.66 (d, *J* = 6.5 Hz, 2H), 2.58 (t, *J* = 12.6, 10.2 Hz, 3H), 1.80 - 1.69 (dd, 2H), 1.55 - 1.42 (m, 1H), 1.38 (s, 9H), 1.29 (m, *J* = 12.1, 3.8 Hz, 2H); m/z = 425 [M]+.

### General procedure F3:

To a solution of amide / lactame (1 eq.) in anhydrous THF (0.1M) under nitrogen atmosphere at 0 °C, was added dropwise 1 M borane tetrahydrofuran (2.9 eq.) or LiAlH₄. The reaction mixture was stirred at rt for 1.5 h. Upon formation of a borane complex observed, the mixture was quenched with MeOH and concentrated. The residue was diluted in MeOH, NaOH 1M as added (2 eq) and the mixture was stirred at 80°C for 2 hours. The mixture was concentrated and water and DCM were added.The mixture was extracted with DCM, phases were separated and the combined organic layers concentrated. The crude material was purified by flash chromatography on silica gel (using a gradient of AcOEt in heptane) to afford the desired product.

### Synthesis of rac-(8aS)-3-[(4-bromo-3-chloro-phenyl)methyl]-3,4,6,7,8,8a-hexahydro-1H-pyrrolo[2,1-c][1,4]oxazine (Intermediate 30)

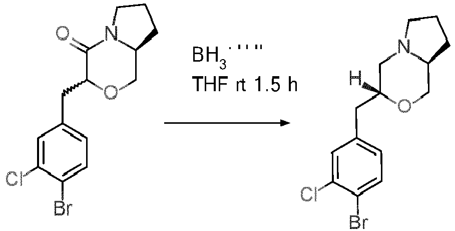

From rac-(8aS)-3-[(4-bromo-3-chloro-phenyl)methyl]-6,7,8,8a-tetrahydro-1H-pyrrolo[2,1-c] [1,4]oxazin-4-one (200 mg, 0.58 mmol), rac-(8aS)-3-[(4-bromo-3-chloro-phenyl)methyl]-3,4,6,7,8,8a-hexahydro-1H-pyrrolo[2,1-c][1,4]oxazine obtained as colourless oil (120 mg, 61 %).

¹H NMR (DMSO-*d*₆, 400 MHz): *δ* (ppm) 7.28 (d, *J* = 2.0 Hz, 1H), 7.14 (dd, *J* = 8.2, 2.0 Hz, 1H), 7.06 (d, *J* = 8.2 Hz, 1H), 3.82 - 3.63 (m, 3H), 3.46 (dtd, *J* = 9.1, 6.5, 2.5 Hz, 1H), 3.38 - 3.32 (m, 1H), 3.23 (d, *J* = 11.3 Hz, 2H), 2.58 (dd, *J* = 12.6, 10.2 Hz, 2H), 1.79 - 1.69 (m, 2H), 1.58 - 1.45 (m, 1H), 1.32 (td, *J* = 12.1, 3.7 Hz, 2H); m/z = 425 [M]+.

### General procedure F4:

To a solution of ester (1 eq.) in anhydrous THF (0.1M) under nitrogen atmosphere at 0 °C, was added dropwise 1 M diisobutylalumane (3 eq.). The reaction mixture was stirred at rt for 30 min. At 0°C, methanol was added and the reaction mixture was concentrated under reduced pressure. EtOAc was added and the solution washed with an aqueous saturated solution of Rochelle's salt. The organic layer was concentrated and the crude material was purified by flash chromatography column on silica gel (using a gradient of AcOEt in heptane) to afford the desired product.

### Synthesis of (3-bromo-4-iodo-5-methyl-phenyl)methanol (Intermediate 31)

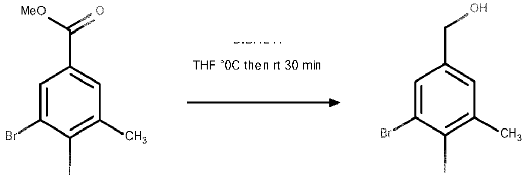

From methyl 3-bromo-4-iodo-5-methylbenzoate (100 mg, 0.28 mmol), (3-bromo-4-iodo-5-methylphenyl)methanol was obtained as a white solid (83 mg, 90 % yield).

¹H NMR (DMSO-*d*₆, 400 MHz): *δ* (ppm) 7.48 (dd, *J* = 2.0, 0.8 Hz, 1H), 7.24 (dq, *J* = 1.5, 0.7 Hz, 1H), 4.42 (dt, *J* = 5.8, 0.8 Hz, 2H), 2.49 (s, 3H); m/z = 327.9 [M+H]⁺.

### General procedure F5:

A vial under Argon was successively charged with alcohol (1 eq.) and di(imidazol-1-yl)methanethione (2 eq) in anhydrous THF (0.05 M). The reaction mixture was stirred at 75°C for 12 h then concentrated under reduced pressure. The crude product was diluted in anhydrous Toluene (0.05 M) and were added 1,1,1,3,3,3-hexamethyl-2-(trimethylsilyl)trisilane (3 eq.) and AIBN (0.1 eq.). The reaction mixture stirred at 110°C for 2h, cooled at rt and bubbled with Argon for 5min. Additional TTMSS (3 eq.) and AIBN (0.1 eq.) were added, and the mixture stirred at 110°C for 1h. The reaction mixture was cooled at rt, quenched with water (15 mL) and DCM (15 mL) was added. The phases were separated and the aqueous phase was extracted with DCM (three times). The combined organic layers were dried using a phase separator and concentrated under vacuum. The crude material was purified by Flash chromatography column on silica gel (using a gradient of AcOEt in Cyclohexane from 0 to 100%) to afford the expected compound.

### Synthesis of 5-[2-chloro-6-(tetrahydropyran-4-ylmethyl)phenyl]isothiazole (Intermediate 32)

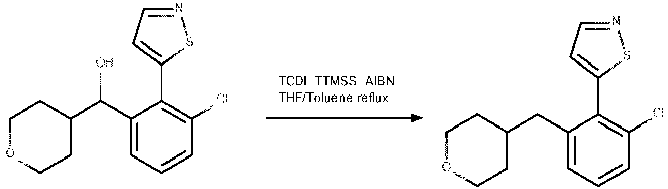

From (3-chloro-2-isothiazol-5-yl-phenyl)-tetrahydropyran-4-yl-methanol (150 mg, 0.45 mmol), 5-[2-chloro-6-(tetrahydropyran-4-ylmethyl)phenyl]isothiazole was obtained as a pale yellow oil (40 mg, 25 % yield).

¹H NMR (DMSO-*d*₆, 400 MHz): *δ* (ppm) 8.71 (d, J = 1.7 Hz, 1H), 7.56 - 7.06 (m, 4H), 3.75 (dd, *J* = 11.9, 4.0 Hz, 2H), 3.15 (td, *J* = 11.7, 2.1 Hz, 2H), 2.37 (d, *J* = 7.1 Hz, 2H), 1.59 (ddt, *J* = 11.3, 7.7, 3.9 Hz, 1H), 1.36 - 1.24 (m, 2H), 1.08 (qd, J= 12.0, 4.5 Hz, 2H); m/z = 420 [M+H]⁺.

### Method G: O/N-alkyl formation

### General procedure G0: N-alkylation

A sealed vial was charged with R-Br or epoxide (1 eq.) and amine was added (2-10 eq). The reaction mixture was stirred at room temperature for 12h. Water was added, the aqueous layer extracted with DCM three times. The combined organic layers were washed with brine, dried over a phase separator and concentrated under reduced pressure to afford the expected product.

### Synthesis of 1-[3-chloro-4-(3-pyridyloxymethyl)phenyl]-3-(2-hydroxyethylamino)propan-2-ol (Intermediate 33)

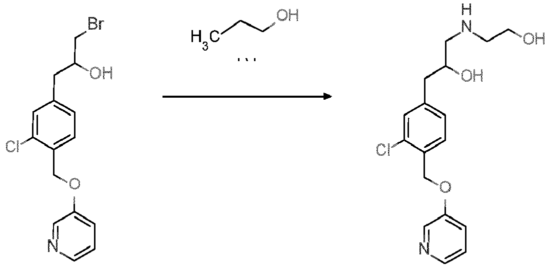

From 1-bromo-3-[3-chloro-4-(3-pyridyloxymethyl)phenyl]propan-2-ol (275 mg, 0.77 mmol) and 2-aminoethanol (462 µL, 7.7 mmol, 10 eq.), 1-[3-chloro-4-(3-pyridyloxymethyl)phenyl]-3-(2-hydroxyethylamino)propan-2-ol was obtained as bege solid (239 mg, 87% Yield). m/z = 337 [M +H]⁺.

### General procedure G1: O-alkylation

To a solution of alcohol (1 eq.) in anhydrous THF (0.1M) under nitrogen atmosphere at 0 °C was added NaH (1-2 eq.). The reaction mixture was stirred at rt for 20 min prior to addition of the halide (1 eq.). The reaction mixture was stirred at rt for 1-2 h. Water and EtOAc were added. The two layers were separated and the aqueous layer was extracted three times with EtOAc. The combined organic layers were dried over a phase separator and concentrated under reduced pressure. If necessary, the crude material was purified by flash chromatography column (EtOAc in heptane) to afford the desired product.

### Synthesis of tert-butyl 2-[[3-chloro-4-[3-(methoxymethyl)isoxazol-5-yl]phenyl]methyl] morpholine-4-carboxylate (Intermediate 34)

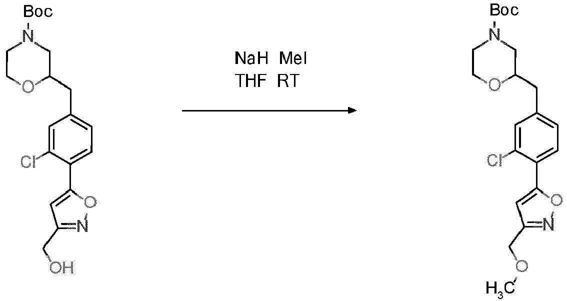

From tert-butyl 2-[[3-chloro-4-[3-(hydroxymethyl)isoxazol-5-yl]phenyl]methyl]morpholine-4-carboxylate (115 mg, 0.27 mmol) and iodomethane (38 mg, 0.27 mmol), tert-butyl 2-[[3-chloro-4-[3-(methoxymethyl)isoxazol-5-yl]phenyl]methyl]morpholine-4-carboxylate was obtained without purification as colourless oil (118 mg, quant.).

¹H NMR (DMSO-*d*₆, 400 MHz): *δ* (ppm) 7.83 (d, *J* = 8.0 Hz, 1H), 7.58 (d, *J* = 1.6 Hz, 1H), 7.42 (dd, *J* = 8.1, 1.7 Hz, 1H), 7.03 (s, 1H), 4.56 (s, 2H), 3.86 - 3.74 (m, 2H), 3.69 (d, *J* = 13.2 Hz, 1H), 3.63 - 3.51 (m, 1H), 3.36 (s, 3H), 3.36 (t, 1H),2.98 - 2.72 (m, 2H), 2.73 - 2.55 (m, 2H), 1.40 (s, 9H).; m/z = 367 [M +H- tBu]+.

### General procedure G2: Mitsunobu/Tsunoda

To a solution of Ar-OH (1.00 eq.) and R-OH (1.10 eq.) in Toluene (0.07M) under nitrogen atmosphere was added CMBP (Tsunoda reagent) (2.00 eq.). The reaction mixture was stirred at 100 °C for 2 h. The reaction mixture was quenched with a saturated aqueous solution of NaHCOs and EtOAc was added. The two layers were separated, the aqueous layer extracted with EtOAc (x3) and the combined organic layers were dried using a phase separator and concentrated under reduced pressureThe crude was purified by flash chromatography column (EtOAc in heptane) to afford the desired product.

### Synthesis of tert-butyl 2-[(3-chloro-4-{[(3R)-oxan-3-yl]methoxy}phenyl)methyl] morpholine-4-carboxylate (Intermediate 35)

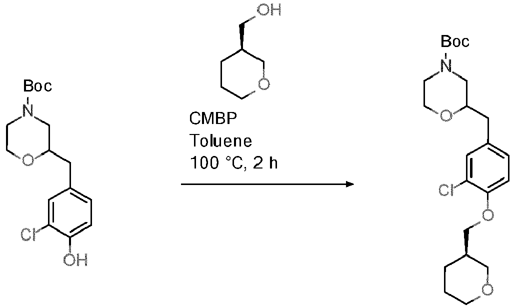

From tert-butyl 2-[(3-chloro-4-hydroxy-phenyl)methyl]morpholine-4-carboxylate (71 mg, 0.22 mmol) and (3S)-oxan-3-ylmethanol (28 mg, 0.24 mmol), tert-butyl 2-[[3-chloro-4-[[rac-(3R)-tetrahydropyran-3-yl]methoxy]phenyl]methyl]morpholine-4-carboxylate was obtained as colourless oil (82 mg, 89 % yield).

¹H NMR (DMSO-*d*₆, 400 MHz) *δ* 7.30 (d, *J* = 2.1 Hz, 1H), 7.14 (dd, *J* = 8.4, 2.1 Hz, 1H), 7.05 (d, *J* = 8.5 Hz, 1H), 3.96 - 3.85 (m, 3H), 3.82 - 3.58 (m, 4H), 3.50 - 3.41 (m, 1H), 3.39 - 3.32 (m, 2H), 3.30 - 3.24 (m, 2H), 2.84 (s, 1H), 2.65 (d, *J* = 6.6 Hz, 2H), 2.08 - 1.95 (m, 1H), 1.90 - 1.80 (m, 1H), 1.65 - 1.39 (m, 3H), 1.37 (s, 9H); m/z = 326 [M-Boc]+.

### General procedure G3: Mitsunobu using DIAD/PPH3

To a solution of diol (or alcohol 1 and alcohol 2) (1eq.) in anhydrous toluene (0.1M) were added triphenylphosphane (1.2 eq.) and isopropyl (N{E})-N-isopropoxycarbonyliminocarbamate (1.2 eq). The reaction mixture was stirred at room temperature for 1 - 12 h then concentrated under reduced pressure. The crude material was purified by flash chromatography column on silica gel (EtOAc in heptane from 0 to 100%) to afford the expected compound. *See as example BB-1 synthesis.*

### General procedure G4: N-alkylation

In a microwave vial, to a solution of amine (1 eq.) in DMF-Anhydrous (0.1 M) were added sequentially K₂CO₃ (3 eq.) and R-X (10 eq.). The mixture was stirred at 110°C for 1 h under microwave irradiation. The mixture was diluted in AcOEt and water was added. The aqueous layer was extracted with EtOAc, the combined organic layers washed with water then brine, filtered over phase separator and concentrated under reduced pressure. The crude material was purified by flash chromatography on silica gel (using a gradient EtOAc in cyclohexane) to afford the desired product.

### Synthesis of tert-butyl 2-[[3-chloro-4-[methyl-[[(3R)-tetrahydropyran-3-yl]methyl]amino] phenyl] methyl]morpholine-4-carboxylate (Intermediate 36)

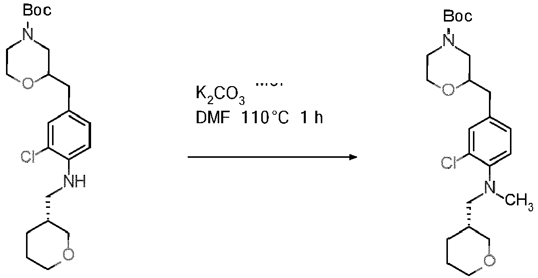

From tert-butyl 2-[[3-chloro-4-[[(3R)-tetrahydropyran-3-yl]methylamino]phenyl]methyl] morpholine-4-carboxylate (196 mg, 0.461 mmol) and iodomethane (287 uL, 4.61 mmol), tert-butyl 2-[[3-chloro-4-[methyl-[[(3R)-tetrahydropyran-3-yl]methyl]amino]phenyl]methyl] morpholine -4-carboxylate obtained as colourless oil (170 mg, 84 % yield).

¹H NMR (DMSO-*d*₆, 400 MHz) *δ* 7.30 - 7.27 (m, 1H), 7.15 (d, *J* = 1.1 Hz, 2H), 3.86 - 3.59 (m, 5H), 3.52 - 3.43 (m, 1H), 3.39 - 3.23 (m, 2H), 3.07 (dd, *J* = 11.2, 9.0 Hz, 1H), 2.93 - 2.73 (m, 3H), 2.66 (d, *J* = 6.6 Hz, 2H), 2.63 (s, 3H), 1.81 - 1.62 (m, 3H), 1.57 - 1.41 (m, 1H), 1.37 (s, 9H), 1.26 - 1.11 (m, 2H); m/z = 439 [M]+.

### General procedure G5: O-Arylation / N-arylation

To a solution of alcohol or amine (1 eq.) in DMF-Anhydrous or DMA (0.05 M) were added Ar-Br (2 eq.) and Cs₂CO₃ (5 eq.). The reaction mixture was degazed under N2, then copper iodide (0.2 eq.) was added, with additional 1,10-phenantroline (2 eq.) for amine coupling. The reaction mixture was stirred at 100°C for 12 h, or up to 180°C (with DMA in a sealed vial). If necessary, more reagents were added and the mixture stirred for further 12 h at 100°C. The mixture was filtered over decalite, washed with EtOAc and the filtrate was concentrated under reduced pressure. The crude was purified by flash column chromatography on silica gel (using a gradient of EtOAc in Heptane from 0% to 100%) to afford the desired product.

### Synthesis of tert-butyl 2-[[3-chloro-4-[(2-methyl-4-pyridyl)oxy]phenyl]methyl]morpholine-4-carboxylate (Intermediate 37)

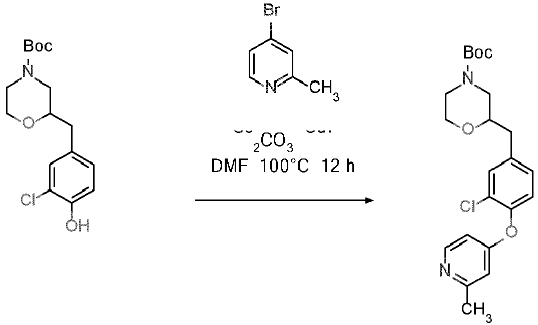

From tert-butyl 2-[(3-chloro-4-hydroxy-phenyl)methyl]morpholine-4-carboxylate (50 mg, 0.15mmol) and 4-bromo-2-methylpyridine (53 mg, 0.305 mmol), tert-butyl 2-[[3-chloro-4-[(2-methyl-4-pyridyl)oxy]phenyl]methyl]morpholine-4-carboxylate was obtained as a colourless oil (23 mg, 36 % yield).

¹H NMR (DMSO-*d*₆, 400 MHz) *δ* 8.32 (d, J = 5.6 Hz, 1H), 7.56 (d, *J* = 1.9 Hz, 1H), 7.34 (dd, *J* = 8.3, 2.0 Hz, 1H), 7.26 (d, *J* = 8.3 Hz, 1H), 6.73 (d, J = 2.4 Hz, 1H), 6.64 (d, *J* = 3.4 Hz, 1H), 3.75 (dd, *J* = 46.7, 12.8 Hz, 3H), 3.60 - 3.52 (m, 1H), 3.43 - 3.32 (m, 1H), 2.95 - 2.60 (m, 4H), 2.41 (s, 3H), 1.39 (s, 9H); m/z = 419 [M+H]⁺.

### General procedure G6: reductive amination

To a solution of amine (1 eq.) in MeOH/AcOH 10/1 (0.1 M) were added the aldehyde or ketone (1-20 eq.). The mixture was stirred for 5 min and sodium cyanoborohydride supported (117 mg, 0.235 mmol) was added. The mixture was stirred at RT for 2-12 h. The resin was filtered, washed with EtOAc and the filtrate concentrated. The residue was taken up with EtOAc and washed with an aqueous saturated solution of bicarbonate. The aqueous phase was extracte (x3), the combined organic layers were filtered through a separatory phase filter and concentrated under reduced pressure. If necessary the crude was purified by flash column chromatography on silica gel (using a gradient of EtOAc in Heptane from 0% to 100%) to afford the desired product.

### Synthesis 4-[2-(azetidin-1-yl)-5-[(4-isopropylmorpholin-2-yl)methyl]phenyl]-2-fluoro-benzonitrile (Intermediate 38)

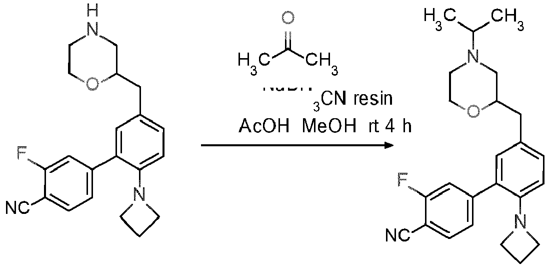

From 4-[2-(azetidin-1-yl)-5-(morpholin-2-ylmethyl)phenyl]-2-fluoro-benzonitrile (28 mg, 0.078 mmol) and propanone (0.116 mL, 1.56 mmol), 4-[2-(azetidin-1-yl)-5-[(4-isopropylmorpholin-2-yl)methyl]phenyl]-2-fluoro-benzonitrilewas obtained as a colourless oil (10.6 mg, 32 % yield).

¹H NMR (DMSO-*d*₆, 400 MHz) *δ* 7.93 (dd, *J* = 8.0, 7.1 Hz, 1H), 7.51 (dd, *J* = 10.7, 1.5 Hz, 1H), 7.43 (dd, *J* = 8.0, 1.5 Hz, 1H), 7.14 (dd, *J* = 8.3, 2.1 Hz, 1H), 6.98 (d, *J* = 2.1 Hz, 1H), 6.56 (d, *J* = 8.3 Hz, 1H), 3.85 - 3.61 (m, 1H), 3.61 - 3.36 (m, 6H), 2.75 - 2.53 (m, 5H), 2.27 - 2.03 (m, 3H), 2.03 - 1.81 (m, 1H), 0.94 (dd, *J* = 6.5, 3.8 Hz, 6H); m/z = 394 [M+H]⁺.

### General procedure G7: O-arylation

A microwavevial was charged with Ar-Br (1 eq.), bromocopper (0.5 eq.), sodium methanolate (25 eq.) and Ethyl acetate (1.72 M). The reaction mixture was stirred at 120°C under microwae irradiation for 30 min. The reaction mixture was quenched with water, the resulting suspension filtered and washed with methanol. The filtrate was concentrated under reduce pressure and EtOAc was added. The aqueous layer was extracted with EtOAc (x3). The combined organic layers were dried using a phase separator and concentrated under reduced pressure. The crude was purified by flash column chromatography on silica gel (using a gradient of EtOAc in Heptane from 0% to 100%) to afford the desired product.

### Synthesis of tert-butyl 2-[(3-chloro-4-methoxy-phenyl)methyl]morpholine-4-carboxylate (Intermediate 39)

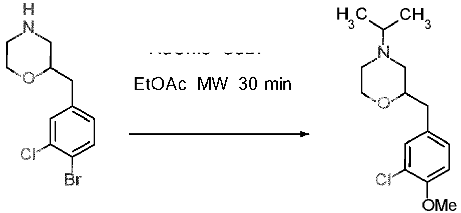

From tert-butyl 2-[(4-bromo-3-chloro-phenyl)methyl]morpholine-4-carboxylate (250 mg, 0.64 mmol) and sodium methanolate (3.7 mL, 16 mmol), tert-butyl 2-[(3-chloro-4-methoxyphenyl)methyl]morpholine-4-carboxylate was obtained as a colourless oil (67 mg, 27.6 % yield). ¹H NMR (DMSO-*d*₆, 400 MHz) *δ* 7.31 (d, *J* = 2.1 Hz, 1H), 7.17 (dd, *J* = 8.4, 2.2 Hz, 1H), 7.06 (d, *J* = 8.4 Hz, 1H), 3.83 (s, 3H), 3.81 - 3.60 (m, 3H), 3.49 - 3.43 (m, 1H), 3.34 (dd, *J* = 11.6, 2.8 Hz, 3H), 2.85 (s, 1H), 2.66 (d, J = 6.5 Hz, 2H), 1.38 (s, 6H); m/z = 242 [M+H]⁺ - Boc.

### General procedure G8: epoxide ring opening by hydride

To a solution of epoxide (1 eq.) in anhydrous THF at 0°C under argon was added dropwise 1 M lithium triethylorohydride (5 eq.). The mixture was stirred for 5 min and sodium cyanoborohydride supported (117 mg, 0.235 mmol) was added. The reaction mixture was stirred at 50°C for 4-12 h. More LiEtsBH was added if necessary. The reaction mixture was quenched with water and EtOAcwas added. The aqueous layer was extracted with EtOAc (x3). The combined organic layers were dried using a phase separator and concentrated under reduced pressure. The crude material was purified by flash chromatography on silica gel (using a gradient of DCM:NH₃ (9:1) in DCM from 5% to 50%) to afford the desired product.

### Synthesis of 4-[(3-chloro-2-cyclopropyl-phenyl)methyl]-1-methyl-piperidin-4-ol (Intermediate 40)

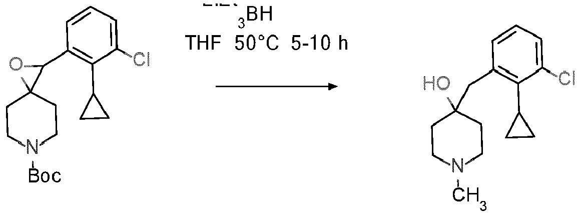

From tert-butyl 2-(3-chloro-2-cyclopropyl-phenyl)-1-oxa-6-azaspiro[2.5]octane-6-carboxylate (50 mg, 0.13 mmol), 4-[(3-chloro-2-cyclopropyl-phenyl)methyl]-1-methyl-piperidin-4-ol was obtained as a colourless oil (15 mg, 36.7 % yield).

¹H NMR (DMSO-*d*₆, 400 MHz) *δ* 7.22 (ddd, *J* = 8.7, 7.7, 1.5 Hz, 2H), 7.19 - 7.07 (m, 1H), 4.08 (s, 1H), 3.00 (s, 2H), 2.38 (d, *J* = 11.1 Hz, 2H), 2.17 (t, *J* = 10.6 Hz, 2H), 2.10 (s, 3H), 1.85 - 1.64 (m, 1H), 1.50 (td, *J* = 12.4, 4.4 Hz, 2H), 1.36 (d, *J* = 12.9 Hz, 2H), 1.19 -1.05 (m, 2H), 0.53 (td, *J* = 6.2, 4.4 Hz, 2H); m/z = 280 [M]⁺.

### Method H: Oxidation

### - Epoxidation

### General procedure H0:

In a round bottom flask, to a stirred solution of alkene (1 eq.) in DCM (0.1 M) was added m-CPBA (1.5 eq.). The mixture was stirred at rt for 12 h. Na₂S₂O₃ saturated aqueous solution and DCM were added. The aqueous layer was extracted with DCM (x3). The combined organic layer dried over sodium sulfate, filtered and concentrated under vacuum. The crude material was purified by Flash chromatography on silica gel (using a gradient of EtOAc in Cyclohexane) to afford the desired product.

*See BB-1 synthesis for an example.*

### - oxidation of alcohol to aldehyde or ketone

### General procedure H1: DMP

To a stirred solution of aldehyde (1 eq.) in DCM-Anhydrous (0.1 M) at rt under argon was added Dess-Martin periodinane (95%, 1.3 eq.) in DCM-Anhydrous (0.1 M) under argon with a dropping funnel over 10 min. The reaction mixture was stirred at rt for 4 h. The reaction mixture was quenched with a saturated aqueous solution of Na₂S₂O₃, basified with a saturated aqueous solution of NaHCOs and DCM was added. The phases were separated and the aqueous layer was extracted with DCM (4 times). The combined organic layers were washed with brine, dried over sodium sulfate, filtered and concentrated under vacuum. The crude material was purified by Flash chromatography on silica gel (using a gradient of EtOAc in Cyclohexane) to afford the desired product.

### Synthesis of tert-butyl 2-[[3-chloro-4-(4-formyl-1-piperidyl)phenyl]methyl]morpholine-4-carboxylate (Intermediate 41)

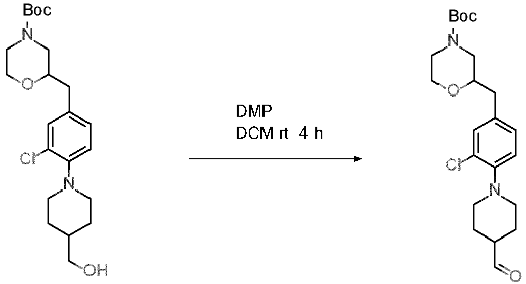

From tert-butyl 2-[[3-chloro-4-[4-(hydroxymethyl)-1 -piperidyl]phenyl]methyl]morpholine-4-carboxylate (876 mg, 2.06 mmol), tert-butyl 2-[[3-chloro-4-(4-formyl-1-piperidyl)phenyl]methyl]morpholine-4-carboxylate obtained as yellow solid (645 mg, 74 % yield). ¹H NMR (DMSO-*d*₆, 400 MHz) *δ* 7.93 (dd, J= 8.0, 7.1 Hz, 1H), 7.51 (dd, J = 10.7, 1.5 Hz, 1H), 7.42 (dd, *J* = 8.0, 1.5 Hz, 1H), 7.13 (dd, *J* = 8.3, 2.1 Hz, 1H), 6.97 (d, *J* = 2.1 Hz, 1H), 6.56 (d, J = 8.3 Hz, 1H), 3.77 - 3.70 (m, 1H), 3.55 - 3.48 (m, 1H), 3.43 (t, J = 7.3 Hz, 4H), 3.41 - 3.34 (m, 1H), 2.68 - 2.52 (m, 5H), 2.20 - 2.03 (m, 3H), 1.97 - 1.89 (m, 1H), 0.94 (dd, J = 6.5, 3.8 Hz, 6H); m/z = 423 [M]+.

### General procedure H2: oxidative cleavage

A 500mL round bottom flask was successively charged with alkene (1 eq.), 2,6-dimethylpyridine (2 eq.) and tetraoxoosmium (0.02 eq.) in 1,4-Dioxane/Water 10/1 (0.07 M). The reaction mixture was stirred at RT for 10 min and sodium periodate (4 eq.) was added. The mixture was stirred at RT for 12-48 hours (Strong stirring because a precipitate appeared). Water was added and dioxane was evaporated. The aqueous layer was extracted (x3) with DCM. The combined organic layers were dried over a phase separator and concentrated under reduced pressure. The crude material was purified by flash chromatography on silica gel (using a gradient of AcOEt in Cyclohexane from 0% to 100%) to afford the desired product.

### Synthesis of tert-butyl 2-[(3-chloro-2-formyl-phenyl)methyl]morpholine-4-carboxylate (Intermediate 42)

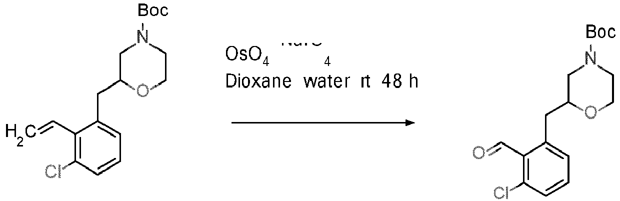

From tert-butyl 2-[(3-chloro-2-vinyl-phenyl)methyl]morpholine-4-carboxylate (4.28 g, 12 mmol), tert-butyl 2-[(3-chloro-2-formyl-phenyl)methyl]morpholine-4-carboxylate was obtained as a white solid (2.14 g, 49 % yield).

¹H NMR (DMSO-*d*₆, 400 MHz) *δ* 10.47 (s, 1H), 7.66 - 7.48 (m, 2H), 7.36 (dd, *J =* 7.4, 1.4 Hz, 1H), 3.86 - 3.58 (m, 3H), 3.49 - 3.38 (m, 1H), 3.26 (td, *J=* 11.6, 2.8 Hz, 1H), 3.10 (dd, *J=* 13.5, 4.3 Hz, 1H), 2.90 (d, *J* = 43.5 Hz, 2H), 2.64 (s, 1H), 1.39 (s, 9H); m/z = 240 [M - (*t*-Bu)]⁺.

### Method I: Protection

### - N-Boc protection

### General procedure I1:

To a stirred solution of amine (1 eq.) in DCM-Anhydrous (0.1 M) under argon was added EtsN (1 eq.) followed by Boc2O (1 eq.).The reaction mixture was stirred at rt for 12 h. The solvents were removed under reduced pressure. Water was added, the aqueous layer was extracted with DCM three times, the combined organic layers were dried over phase separator and concentrated under reduced pressure. The crude material was purified by Flash chromatography on silica gel (using a gradient of MeOH in DCM) to afford the desired product.

### Synthesis of tert-butyl 4-hydroxy-4-methyl-piperidine-1-carboxylate (Intermediate 43)

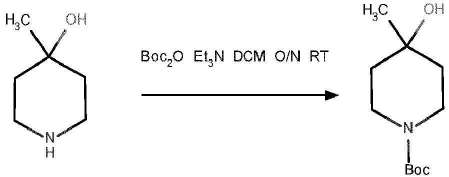

From 4-methylpiperidin-4-ol (1 g, 8.42 mmol), tert-butyl 4-hydroxy-4-methyl-piperidine-1-carboxylate obtained as yellow oil (1.75 g, 96.5 % yield).

¹H NMR (DMSO-*d*₆, 400 MHz) 4.29 (s, 1H), 3.52 (dt, *J* = 13.2, 4.3 Hz, 2H), 3.12 (s, 2H), 1.44 - 1.39 (m, 2H), 1.38 (s, 9H), 1.37 - 1.29 (m, 2H), 1.11 (s, 3H).

### - O-TBS protection

### General procedure I2:

To a stirred solution of alcohol (1 eq.) in DCM-Anhydrous (0.15 M) under argon was added EtsN (4 eq.) and the mixture was cooled to 0°C. TBDMSOTf (2 eq.) was added dropwise and the reaction was stirred at rt for 2 h. The solvents were removed under reduced pressure. The crude material was purified by Flash chromatography on silica gel (using a gradient of EtOAc in Cyclohexane) to afford the desired product.

### Synthesis of 4-[(tert-butyldimethylsilyl)oxy]-4-methylpiperidine (Intermediate 44)

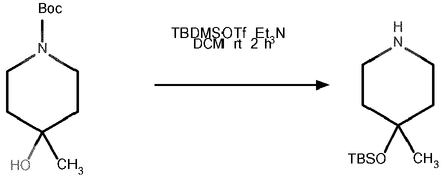

From tert-butyl 4-hydroxy-4-methyl-piperidine-1-carboxylate (1 g, 4.65 mmol 4-[(tertbutyldimethylsilyl)oxy]-4-methylpiperidine obtained as yellow solid (500 mg, 44.6 % yield) with a spontaneous deprotection of the N-Boc during the reaction.

¹H NMR (DMSO-*d*₆, 400 MHz) *δ* 2.87 - 2.68 (m, 4H), 1.54 - 1.38 (m, 4H), 1.21 (s, 3H), 0.87 (s, 9H), 0.08 (s, 6H).

### Method J: amide formation

To a stirred solution of alcohol (1 eq.) in DCM-Anhydrous (0.1 M) at 0°C under argon was added the acylchloride (1.4 eq.) dissolved in DCM dropwise. The mixture was stirred at rt for 12 h. The solvents were removed under reduced pressure. The residue was suspended in diethyl ether, filtered through a frit (pore size 4) and washed with further portions of diethyl ether. The combined ethereal phases were evaporated. The crude material was purified by Flash chromatography on silica gel (using a gradient of MeOH in DCM) to afford the desired product.

### Synthesis of 2-chloro-1-[(2S)-2-(hydroxymethyl)pyrrolidin-1-yl]ethenone (Intermediate 45)

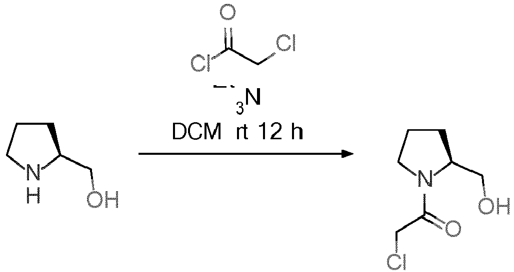

From [(2{S})-pyrrolidin-2-yl]methanol (200 mg, 1.94 mmol), 2-chloro-1-[(2S)-2-(hydroxymethyl)pyrrolidin-1-yl]ethanone obtained as colorless oil (238 mg, 62 % yield).

¹H NMR (DMSO-*d*₆, 400 MHz) *δ* 4.34 - 4.27 (m, 4H), 4.02 - 3.89 (m, 3H), 3.49 (ddd, J = 10.5, 5.6, 3.6 Hz, 2H), 2.02 - 1.86 (m, 4H); m/z = 178 [M+H]⁺.

### Method K: Other Ar-X transformations

### - Ar-Br to Ar-OH

### General procedure K1:

In a vial, to a stirred suspension Ar-Br (1 eq.) in 1 ,4-Dioxane / Water 1/1 (0.032 M) under argon were added sequentially potassium hydroxide (3 eq.) and 4-{5-[bis(adamantan-1-yl)phosphanyl]-1H-pyrazol-1-yl}-1,3,5-triphenyl-1H-pyrazole (0.15 eq.).The reaction mixture was degassed with argon for 5 min, then Pd₂dba₃ (0.1 eq) was added. The mixture was stirred at 100°C for 12 h. 1M aqueous solution of HCl and DCM were added. The aqueous layer was extracted with DCM (x3). The combined organic layers were dried using a phase separator and concentrated under reduced pressure. The crude material was purified by flash chromatography on silica gel (using a gradient of EtOAc in Heptane from 0% to 100%) to affordthe expected compound.

### Synthesis of tert-butyl 2-[(3-chloro-4-hydroxy-phenyl)methyl]morpholine-4-carboxylate (Intermediate 46)

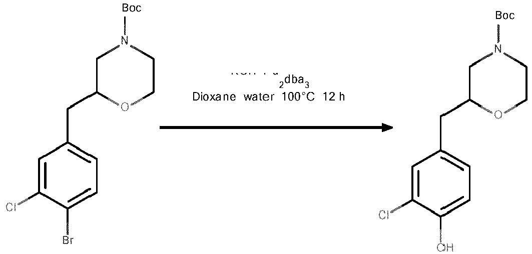

From tert-butyl 2-[(4-bromo-3-chloro-phenyl)methyl]morpholine-4-carboxylate (250 mg, 0.63 mmol) and KOH (119 mg, 1.91 mmol, 3 eq.) , tert-butyl 2-[(3-chloro-4-hydroxyphenyl)methyl]morpholine-4-carboxylate was obtained as colorless oil (150 mg, 71 % yield).

¹H NMR (DMSO-*d*₆, 400 MHz) δ 9.93 (s, 1H), 7.19 (d, *J* = 2.1 Hz, 1H), 6.98 (dd, *J* = 8.3, 2.1 Hz, 1H), 6.86 (d, *J* = 8.3 Hz, 1H), 3.77 (d, *J* = 11.1 Hz, 1H), 3.68 (t, *J* = 14.1 Hz, 2H), 3.41 (ddt, *J* = 9.4, 6.5, 3.3 Hz, 1H), 3.36 - 3.31 (m, 2H), 2.84 (s, 1H), 2.69 - 2.54 (m, 2H), 1.37 (s, 9H); m/z = 228 [M+H]⁺- Boc.

### - Cyanation

### General procedure K2

In a microwave vial were introduced sequentially Ar-Br (1 eq.), cyclopentyl(diphenyl)phosphane iron (0.3 eq.), zinc (0.8 eq.) and Pd₂dba₃ (0.15 eq.) under nitrogen bubbling. The mixture was heated at 50°C under N2 bubbling and after 10 min, zinc dicyanide (0.5 eq.) was added. The mixture was stirred at 120°C for 12 h. The mixture was filtered, washed with EtOAC. NaHCOs saturated aqueous solution was added to the filtrate, the mixture extracted with EtOAc (3 times). The combined organics were filtered using a phase separator and evaporated under reduced pressure. The crude material was purified by flash column chromatography on silica gel (using a gradient of AcOEt in heptane from 0% to 100%) to afford the desired compound.

### Synthesis of tert-butyl 2-[(3-chloro-2-cyano-phenyl)methyl]morpholine-4-carboxylate (Intermediate 47)

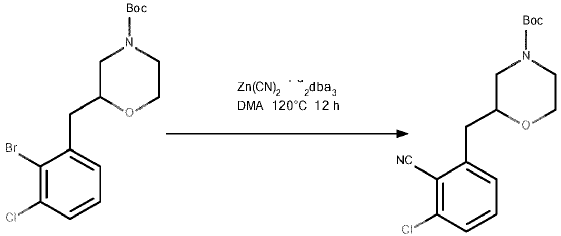

From tert-butyl 2-[(2-bromo-3-chloro-phenyl)methyl]morpholine-4-carboxylate (50 mg, 0.12 mmol) and KOH, tert-butyl 2-[(3-chloro-2-cyano-phenyl)methyl]morpholine-4-carboxylate was obtained as colorless oil (7 mg, 16 % yield).

¹H NMR (DMSO-*d*₆, 400 MHz) δ 7.71 - 7.58 (m, 2H), 7.53 (dd, *J* = 7.5, 1.1 Hz, 1H), 3.80 (dd, *J* = 25.8, 13.5 Hz, 2H), 3.67 (d, *J* = 13.8 Hz, 2H), 3.34 (d, J = 2.8 Hz, 1H), 3.04 (dd, *J* = 14.2, 4.5 Hz, 1H), 2.92 (dd, *J* = 14.1, 8.5 Hz, 3H), 1.39 (s, 9H); m/z = 237 [M+H]⁺ - Boc.

### - Sandmeyer

### General procedure K3:

In a round bottom flask, to a stirred solution of amine (1 eq.) in sulphuric acid (0.97 M) at 0°C was slowly added sodium nitrite (2.2 eq.). The mixture was stirred at 0°C during 5h then poured into iced-water under stirring. The resulting solution was slowly added to a stirred solution of potassium iodide (6 eq) in 200 mL of iced-water. The aqueous layer was extracted twice with EtOAc. The combined organic layers were washed with an aqueous saturated solution of Na₂S₂O₃, dried using a phase separator and concentrated under reduced pressure. The crude material was purified by flash chromatography on silica gel (using a gradient of EtOAc in heptane from 0% to 100%) to afford the desired product.

### Synthesis of ethyl 3-bromo-2-iodo-5-methyl-benzoate (Intermediate 48)

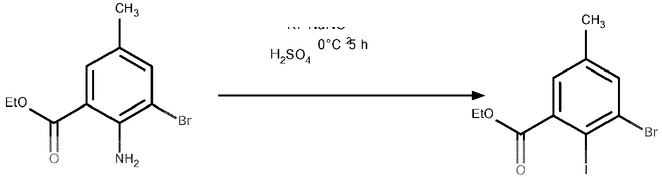

From ethyl 2-amino-3-bromo-5-methylbenzoate (2 g, 7 mmol), ethyl 3-bromo-2-iodo-5-methylbenzoate was obtained as a yellow oil (2.35 g, purity 75%, 68 % yield).

¹H NMR (DMSO-*d*₆, 400 MHz) δ 7 7.73 (dt, *J* = 2.8, 1.4 Hz, 1H), 7.31 (dd, *J* = 2.0, 0.8 Hz, 1H), 4.32 (q, *J* = 7.1 Hz, 2H), 2.31 - 2.25 (m, 3H), 1.33 (t, *J* = 7.1 Hz, 3H).

### Method L: Photoredox

### General procedure L1

In 5mL vial, the arylhalide (1eq), 3-Bromooxetane (97%, 17 uL, 0.195 mmol), lr[dF(CF3)ppy]2(dtbbpy)PF6 (99%, 0.03 eq), TTMSS (97%, 1eq) and sodium carbonate (2 eq) were solubilized in DME (0.1 M) under Ar atmosphere. Dichloronickel;1,2-dimethoxyethane (97%, 0.015 eq) (x5) and 4-tert-butyl-2-(4-tert-butyl-2-pyridyl)pyridine (98%, 0.018 eq) (x5) were solubilized in DME (0.1 M) under Ar. The mixture was stirred at rt for 15min. 0.1 mL of the precatalyst solution was added to the solution.

The mixture was degassed by sparging with Ar for 10min and stirred at 40°C for 7h under irradiation with 34W blue LED lamp (pale green coloration). The mixture was quenched with air in open flask and concentrated. The crude was purified by flash chromatography (EtOAc in cyclohexane 0-50%) to afford the expected product.

### Synthesis of tert-butyl 2-[[3-chloro-4-(oxetan-3-yl)phenyl]methyl]morpholine-4-carboxylate (Intermediate 49)

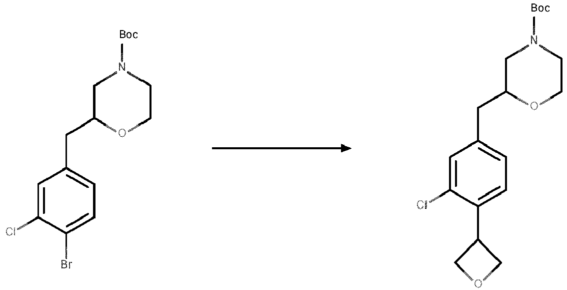

From tert-butyl 2-[(4-bromo-3-chloro-phenyl)methyl]morpholine-4-carboxylate (51 mg, 1 eq) and 3-Bromooxetane (27.6 mg, 1.5 eq), yellow oil (48% yield).

¹H NMR(DMSO-*d₆*, 400 MHz) : δ (ppm) 7.35 (d, *J* = 7.9 Hz, 1H), 7.23 (d, *J* = 1.7 Hz, 2H), 5.05 (dd, *J* = 8.5, 6.0 Hz, 2H), 4.83 (dd, *J* = 8.5, 6.0 Hz, 2H), 3.94 - 3.76 (m, 3H), 3.64 - 3.53 (m, 2H), 3.51 - 3.44 (m, 2H), 2.79 (dd, *J* = 13.9, 7.5 Hz, 1H), 2.69 (dd, *J* = 14.1, 5.3 Hz, 2H), 1.45 (s, 9H); m/z = 312 [M+H]+ -(t-Bu).

### General procedure L2

(Angew. Chem. Int. Ed. 2019, 58, 1823 -1827)
In a 10 mL dried pyrex screw-top reaction tube with suitable size stir bar, dipotassium hydrogen phosphate (2 eq.), Ar-Br (1 eq.), benzaldehyde (0.5 eq.) was dissolved in THF-Anhydrous (0.1 M). In another flask, 1,2-dimethoxyethane - dibromonickel (1:1) (97%, 0.1 eq.), 4-tert-butyl-2-(4-tert-butyl-2-pyridyl)pyridine (98%, 0.1 eq.) were dissolved in THF-Anhydrous (0.1 M) under N₂, then put in the ultrasonic sink for 2-3 minutes until the solution turned to homogeneous. The nickel catalyst solution was added to the reaction tube with syringe. The reaction mixture was then cooled to -78 °C and degassed via vacuum evacuation (5 min), backfilled with nitrogen, and warmed to room temperature. This process was repeated three times. After the reaction was thoroughly degassed, it was stirred at rt under UVA lights (380 nm) irradiation for 24h. The reaction mixture was diluted with DCM. Organic layers were washed with water, and aqueous layers were extracted with DCM. The combined organic phase were dried over a phase separator and concentrated under reduced pressure. The crude material was purified by flash chromatography on silica gel (using a gradient of EtOAc in cyclohexane from 0 to 100%) to afford the desired product.

### Synthesis of tert-butyl 2-[(3-chloro-4-tetrahydrofuran-2-yl-phenyl)methyl]morpholine-4-carboxylate (Intermediate 50)

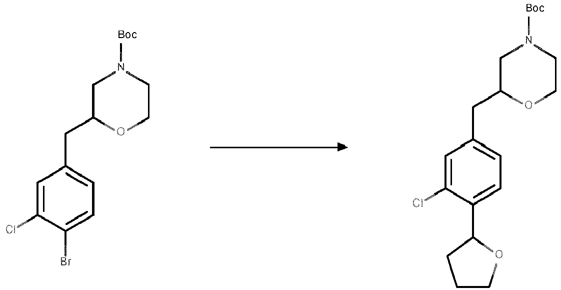

From tert-butyl 2-[(4-bromo-3-chloro-phenyl)methyl]morpholine-4-carboxylate (300 mg, 1 eq) and THF (solvent), tert-butyl 2-[(3-chloro-4-tetrahydrofuran-2-yl-phenyl)methyl]morpholine-4-carboxylate was obtaine as colourless oil (79 mg, 25 % yield).

¹H NMR(CDCl₃, 400 MHz) : δ (ppm) 7.42 (d, *J* = 7.9 Hz, 1H), 7.19 (s, 1H), 7.10 (d, *J* = 8.0 Hz, 1H), 5.17 (t, *J* = 7.0 Hz, 1H), 4.13 (q, *J* = 7.1 Hz, 1H), 3.94 (q, *J* = 7.4 Hz, 2H), 3.89 - 3.77 (m, 3H), 3.64 - 3.53 (m, 1H), 3.53 - 3.42 (m, 1H), 3.01 - 2.87 (m, 1H), 2.78 (dd, *J* = 14.1, 7.2 Hz, 1H), 2.75 - 2.57 (m, 2H), 2.48 (dq, *J* = 13.8, 6.9 Hz, 1H), 1.96 (tq, *J* = 14.4, 6.4 Hz, 3H), 1.76 - 1.61 (m, 1H), 1.44 (s, 9H).); m/z = 312 [M+H]+ -(t-Bu).

### Method M:

### General procedure M1: hydrogenation

To a stirred solution of alkene (1.00 eq.) in MeOH (0.1 M) was added dioxoplatinum (0.11 eq) under N2 atmosphere. The reaction mixture was stirred under H2 atmosphere at room temperature for 24 h. The mixture was filtered, washed with MeOH and solvent evaporated under high vacuum. The crude material was purified by flash chromatography column (EtOAc in DCM) to afford the desired product.

### Synthesis of tert-butyl 2-[(3-chloro-4-tetrahydropyran-4-yl-phenyl)methyl]morpholine-4-carboxylate (Intermediate 51)

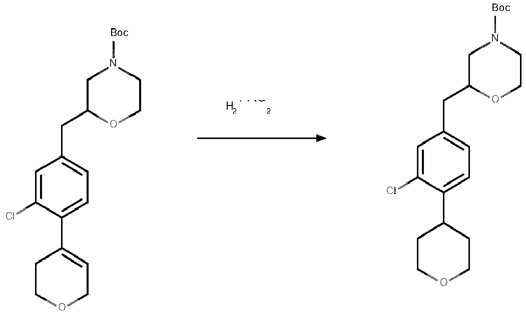

From tert-butyl 2-{[3-chloro-4-(3,6-dihydro-2H-pyran-4-yl)phenyl]methyl}morpholine-4-carboxylate (79 mg), tert-butyl 2-[(3-chloro-4-tetrahydropyran-4-yl-phenyl)methyl]morpholine-4-carboxylate obtained as a colorless oil (55 mg, 71% yield). m/z = 296 [M+H]⁺ - Boc.

### General procedure M2: hydrobromination

A sealed vial was charged with alkene (1 eq.) and ammonium acetate (0.15 eq.) in acetone (0.28 M). NBS (1.1 eq.) and Water (0.28 M) were added and the reaction mixture was stirred at RT for 1h then quenched with a saturated solution of Na₂S₂O₃. A saturated solution of NaHCO3 was also added. The aqueous layer was extracted with DCM (x3). The combined organic layers were washed with brine, dried using a phase separator and concentrated under reduced pressure. The crude material was purified by flash chromatography on silica gel (using a gradient of DCM/MeOH (9:1) in DCM from 0% to 100%) to afford the desired product.

### Synthesis of 1-bromo-3-[3-chloro-4-(3-pyridyloxymethyl)phenyl]propan-2-ol (Intermediate 52)

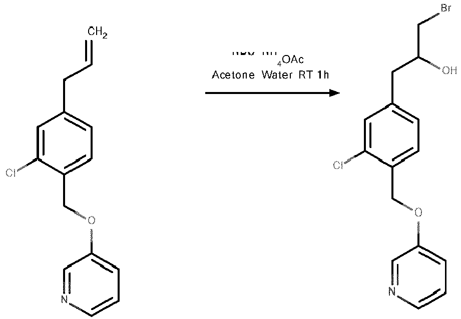

From 3-[(4-allyl-2-chloro-phenyl)methoxy]pyridine (227 mg, 0.87 mmol, 1 eq), 1-bromo-3-[3-chloro-4-(3-pyridyloxymethyl)phenyl]propan-2-ol was obtained as a beige solid (54 mg, 16 % yield).

¹H NMR(DMSO-*d₆*, 400 MHz) : δ (ppm) 8.38 (d, *J* = 2.9 Hz, 1H), 8.21 (dd, *J* = 4.6, 1.3 Hz, 1H), 7.56 (d, *J* = 7.8 Hz, 1H), 7.50 (ddd, *J* = 8.5, 3.0, 1.3 Hz, 1H), 7.46 (d, *J* = 1.6 Hz, 1H), 7.38 (s, 1H), 7.30 (dd, *J* = 7.8, 1.6 Hz, 1H), 5.33 (t, J = 5.7 Hz, 1H), 5.21 (s, 2H), 4.37 (dq, *J* = 10.2, 5.5 Hz, 1H), 3.75 - 3.61 (m, 2H), 3.42 - 3.34 (m, 1H), 3.00 (dd, *J* = 14.6, 9.2 Hz, 1H); m/z = 356 [M+H]⁺.

### Method N: C-C bond formation

### General procedure N1: Condensation with morpholinone

In a flame-dried 25mL two-neck round-bottom flask lactam (1 eq.) was solubilized in THF-Anhydrous (0.1 ML) under Ar and cooled at -78°C. 1 M (diisopropylamino)lithium (1.5 eq.) was added dropwise to the solution. The mixture was stirred at - 78°C for 30 min and Ar-CH₂-Br (1.3 eq.) in THF-Anhydrous (0.1 M) was added. The mixture was stirred at -78°C for 10 min and warmed up to rt for 12 h. NH₄Cl saturated aqueous solution was added and the mixture was extracted with EtOAc (three times). The combined organic layers were concentrated under vacuum. The crude material was purified by flash chromatography column (using a gradient of EtOAc in heptane) to afford the desired product.

### Synthesis of (8aS)-3-[(4-bromo-3-chloro-phenyl)methyl]-6,7,8,8a-tetrahydro-1H-pyrrolo [2,1-c][1,4]oxazin-4-one (Intermediate 53)

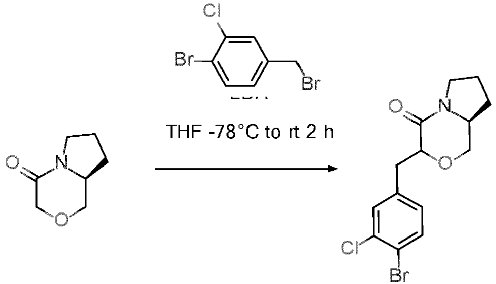

From (8aS)-6,7,8,8a-tetrahydro-1H-pyrrolo[2,1-c][1,4]oxazin-4-one (60 mg, 0.425 mmol), (8aS)-3-[(4-bromo-3-chloro-phenyl)methyl]-6,7,8,8a-tetrahydro-1H-pyrrolo[2,1-c][1,4]oxazin-4-one obtained as a white solid (90 mg, 57% yield).

¹H NMR (DMSO-d₆, 600 MHz): δ (ppm) 7.65 (d, *J=* 8.2 Hz, 1H), 7.46 (d, *J* = 2.1 Hz, 1H), 7.14 (dd, *J* = 8.2, 2.1 Hz, 1H), 4.18 (dd, *J* = 7.9, 3.5 Hz, 1H), 4.09 (dd, *J* = 11.3, 4.0 Hz, 1H), 3.43-3.51 (m, 2H), 3.25 (dd, *J* = 11.2, 10.3 Hz, 2H), 3.14 (dd, *J* = 14.4, 3.5 Hz, 1H), 2.88 (dd, *J* = 14.4, 8.1 Hz, 1H), 1.84-1.94 (m, 2H), 1.70 (ttd, *J* = 12.3, 9.6, 7.0 Hz, 1H), 1.30 (qd, *J* = 11.6, 7.4 Hz, 1H); m/z = 346 [M+H]+.

### General procedure N2: Grignard addition

A flame-dried 3-neck round bottom flask (250 mL) with a magnetic stirrer was charged with carbonyl (1 eq.) dissolved in THF-Anhydrous (0.17 M). The solution was cooled to -20°C (homogeneous yellow solution). 3 M bromo(methyl)magnesium in Et₂O (1.5 eq.) was added. This reaction was stirred at -20°C for 2 h. After 10 min, the RM became beige heterogeneous. The mixture was warmed up to 0°C, a saturated aqueous solution of NH₄Cl and EtOAc were added and the mixture extracted with EtOAc (twice). The combined organic layers were dried over a phase separator and concentrated. The crude material was purified by flash chromatography column on silica gel (using a gradient of EtOAc in heptane) to afford the desired product.

### Synthesis of tert-butyl (3aR,6aS)-5-hydroxy-5-methyl-1,3,3a,4,6,6a-hexahydrocyclopenta [c]pyrrole-2-carboxylate (Intermediate 54)

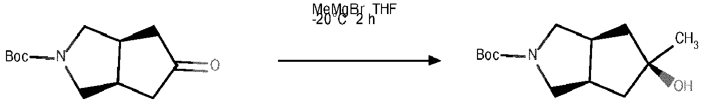

From tert-butyl (3aR,6aS)-5-oxo-octahydrocyclopenta[c]pyrrole-2-carboxylate (2 g, 8.43 mmol), tert-butyl (3aR,6aS)-5-hydroxy-5-methyl-1,3,3a,4,6,6a-hexahydrocyclopenta[c]pyrrole-2-carboxylate (1.26 g, 55.715% yield) obtained as a colorless oil.

¹H NMR (CDCl₃, 400 MHz) : δ (ppm) 3.54 - 3.43 (m, 2H), 3.34 (dd, *J* =11.2, 3.6 Hz, 2H), 2.76 - 2.61 (m, 2H), 1.98 - 1.87 (m, 2H), 1.73 (s, 1H), 1.66 (dd, *J* =13.3, 4.8 Hz, 2H), 1.44 (s, 9H), 1.31 (s, 3H).

### General procedure N3: Wittig

In a vial, to a stirred solution of dimethyl phosphonate (2 eq.) in THF-Anhydrous (0.325 M) at 0°C was added sodium hydride (2 eq) portionwise. The mixture was stirred at 0°C for 10 min, then R-Br (1 eq.) in THF-Anhydrous (0.325 M) was added. The mixture was stirred at RT for 1h then was cooled to 0°C. A solution of ketone (2 eq.) in THF-Anhydrous (2xv/0.325 M) was added followed by sodium hydride (2 eq.) portionwise. The mixture was stirred at RT for 5h then quenched with a saturated aqueous solution of NH₄Cl and EtOAc was added. The aqueous layer was extracted with EtOAc (x3). The combined organic layers were dried using a phase separator and concentrated under reduced pressure. The crude material was purified by flash chromatography on silica gel (using a gradient of EtOAc in Heptane from 0% to 100%) to afford the expected compound.

### Synthesis of tert-butyl 4-[(3-chloro-2-cyclopropyl-phenyl)methylene]piperidine-1-carboxylate (Intermediate 55)

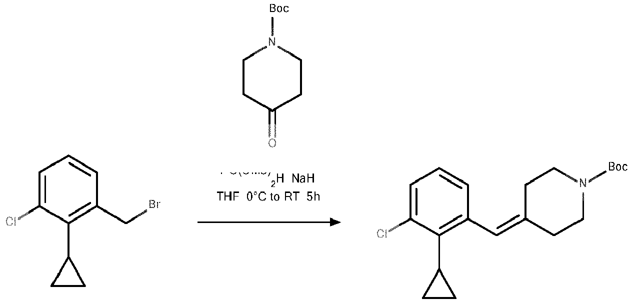

From 1-(bromomethyl)-3-chloro-2-cyclopropyl-benzene (228 mg, 0.91 mmol, 1 eq.) and tert-butyl 4-oxopiperidine-1-carboxylate (374 mg, 1.82 mmol, 2 eq.), tert-butyl 4-[(3-chloro-2-cyclopropyl-phenyl)methylene]piperidine-1-carboxylate (249 mg, 78 % yield) obtained as a colorless oil.

¹H NMR(DMSO-*d₆*, 400 MHz): δ (ppm) 7.30 (dd, *J* = 7.9, 1.3 Hz, 1H), 7.20 (t, *J* = 7.8 Hz, 1H), 7.04 (dd, *J* = 7.4, 1.2 Hz, 1H), 6.49 (s, 1H), 3.44 (t, *J* = 5.9 Hz, 2H), 3.30 (d, *J* = 5.6 Hz, 2H), 2.42 - 2.25 (m, 2H), 2.16 (t, *J* = 5.7 Hz, 2H), 1.74 (ddd, *J* = 14.3, 8.8, 5.9 Hz, 1H), 1.41 (s, 9H), 1.11 - 0.91 (m, 2H), 0.53 (td, *J* = 6.2, 4.5 Hz, 2H).m/z = 248 [M+H]⁺ - Boc.

### Method O: Stille coupling

### General procedure O1:

A sealed vial under argon was charged with Ar-Br (1eq.), dichloropalladium triphenylphosphane (0.15 eq.) and iodocopper (0.15 eq.) in 1,4-Dioxane-Anhydrous (0.128 ML). The mixture was purged with Argon during 5 min and stannane (2 eq.) was added under Ar. The mixture was stirred at 150°C for 1h. The mixture was filtered and concentrated under reduced pressure, purification by flash chromatography on silica gel (using a gradient of EtOAc in Heptane from 0% to 100%) afforded the desired product.

### Synthesis of tert-butyl 2-[(3-chloro-2-oxazol-2-yl-phenyl)methyl]morpholine-4-carboxylate (Intermediate 56)

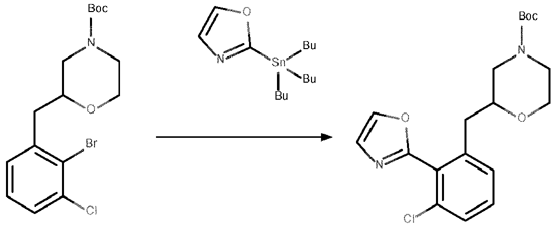

From tert-butyl 2-[(2-bromo-3-chlorophenyl)methyl]morpholine-4-carboxylate (250 mg, 0.64 mmol, 1 eq.) and 2-(tributylstannanyl)-1,3-oxazole (458 mg, 1.28 mmol, 2 eq.), tert-butyl 2-[(3-chloro-2-oxazol-2-yl-phenyl)methyl]morpholine-4-carboxylate (70 mg, 28 % yield) obtained as a colorless oil.

¹H NMR(DMSO-*d₆*, 400 MHz): δ (ppm) 8.34 (d, *J* =0.9 Hz, 1H), 7.54 (d, *J* =1.7 Hz, 1H), 7.53 (s, 1H), 7.47 (d, *J* =0.9 Hz, 1H), 7.43 (dd, *J* =5.2, 3.7 Hz, 1H), 3.77 - 3.60 (m, 3H), 3.56 (d, *J* =14.0 Hz, 1H), 3.23 (td, *J* =11.6, 2.8 Hz, 1H), 2.79 (s, 1H), 2.73 - 2.54 (m, 2H), 2.46 (s, 1H), 1.37 (s, 10H)); m/z = 279 [M+H]⁺- Boc.

### General procedure O2:

A sealed vial under argon was charged with Ar-Br (1eq.) and stannane (1.2 eq.) in DMF (0.1 M). The reaction was purged 3 times with argon and palladium triphenylphosphane (0.1 eq.) was added. The resulting mixture was stirred 2h at 100°C. Water was added and the mixture was extracted with AcOEt. The combined organic layers were washed with water, brine, dried over phase separator and concentrated under vacuum. The crude material was purified on silica gel column (using a gradient of EtOAc in heptane from 0°C to 100°C) to afford the expected product.

### Synthesis of tert-butyl 2-[[3-chloro-2-(2-methylpyrazol-3-yl)phenyl]methyl]morpholine-4-carboxylate (Intermediate 57)

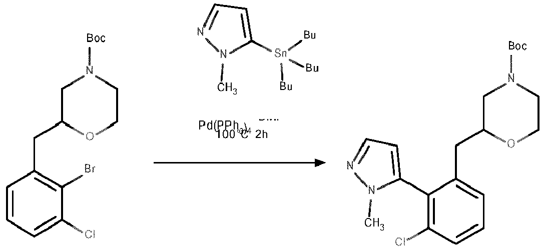

From tert-butyl 2-[(2-bromo-3-chloro-phenyl)methyl]morpholine-4-carboxylate (100 mg, 0.256 mmol, 1 eq.) and 1-methyl-5-(tributylstannyl)-1H-pyrazole (114 mg, 0.307 mmol, 1.2 eq.), tert-butyl 2-[[3-chloro-2-(2-methylpyrazol-3-yl)phenyl]methyl]morpholine-4-carboxylate (90 mg, 90 % yield) obtained as a yellow oil.

¹H NMR(DMSO-*d₆*, 400 MHz): δ (ppm) 7.56 - 7.36 (m, 4H), 6.28 (dd, *J* = 10.6, 1.8 Hz, 1H), 3.76 - 3.67 (m, 1H), 3.63 (d, *J* = 13.6 Hz, 2H), 3.52 (d, *J* = 3.5 Hz, 3H), 3.24 (tt, *J* = 11.5, 3.0 Hz, 2H), 2.80 (s, 1H), 2.65 (ddd, *J* = 36.7, 14.1, 6.9 Hz, 1H), 2.48 - 2.34 (m, 2H), 1.37 (s, 9H); m/z = 392 [M+H]⁺.

### Method P:

### General procedure P1: Negishi

A suspension of zinc (3 eq.) in DMA (0.15 M) in a dry flask was heated under N₂ to 65-70°C for 5 min. A mixture of chloro(trimethyl)silane (0.3 eq.) and 1,2-dibromoethane (0.27 eq.) was added dropwise, stirring for 10 min, followed by slow addition of alkyl-I (2 eq.) in DMA (3v, 0.15 M). The reaction was cooled slowly to room temperature then added to a mixture of copper iodide (0.31 eq.), bis(cyclopentyldiphenylphosphane) dichloromethane dichloropalladium iron (0.10 eq.) and Ar-I (1 eq.) in DMA (3 mL) and stirred for 12 h at 70°C. The mixture was allowed to cool to RT and water was added. The aqueous layer was extracted with DCM (x3). The combined organic layers were washed with brine (NaCl), dried using a phase separator and concentrated under reduced pressure. The crude material was purified by flash chromatography on silica gel (using a gradient of DCM/7N NH3 (9:1) in DCM from 0% to 50%) to afford the desired compound.

### Synthesis of benzyl 3-(6-methoxy-3-pyridyl)azetidine-1-carboxylate (Intermediate 58)

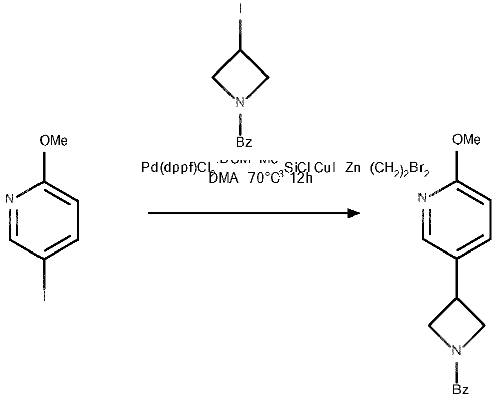

From 5-iodo-2-methoxypyridine (250 mg, 1.04 mmol, 1 eq.) and benzyl 3-iodoazetidine-1-carboxylate (701 mg, 2.10 mmol, 2 eq.), benzyl 3-(6-methoxy-3-pyridyl)azetidine-1-carboxylate (370 mg, 99.9 % yield) obtained as a orange oil.

¹H NMR(DMSO-*d₆*, 400 MHz): δ (ppm) 8.10 (d, *J* = 2.5 Hz, 1H), 7.79 (dd, *J* = 8.6, 2.6 Hz, 1H), 7.38 (t, *J* = 1.6 Hz, 2H), 7.36 - 7.26 (m, 3H), 6.82 (dd, *J* = 8.5, 0.7 Hz, 1H), 5.06 (s, 2H), 4.37 - 4.26 (m, 2H), 3.96 - 3.90 (m, 2H), 3.88 - 3.84 (m, 1H), 3.83 (s, 3H); m/z = 299 [M+H]⁺.

### General procedure Q1: Chan Lam

To a solution of Ar-BPin or boronic acid (1 eq.), amine (2 eq.) and KF (2 eq.) in Acetonitrile (0.0914 M) was added diacetoxycopper (2 eq.). The mixture was stirred at 50°C for 12 h. The mixture was filtered on celite and solvent was evaporated under reduced pressure. The crude material was purified by flash chromatography on silica gel (using a gradient of EtOAc in heptane 0% to 100%) to afford the desired compound.

### Synthesis of tert-butyl 2-[[3-chloro-4-(4-methoxy-3-methyl-pyrazol-1-yl)phenyl]methyl] morpholine-4-carboxylate (Intermediate 59)

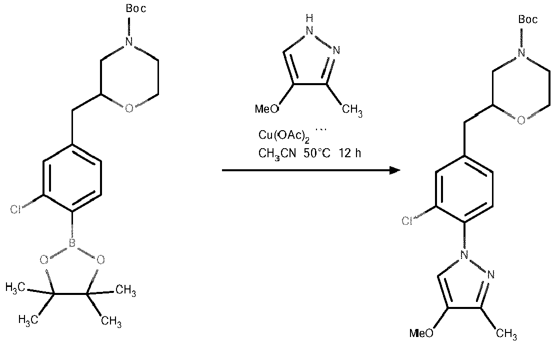

From tert-butyl 2-[[3-chloro-4-(4,4,5,5-tetramethyl-1,3,2-dioxaborolan-2-yl)phenyl]methyl] morpholine-4-carboxylate (100 mg, 0.228 mmol, 1 eq.) and 4-methoxy-3-methyl-1H-pyrazole (51 mg, 0.456 mmol, 2 eq.), tert-butyl 2-[[3-chloro-4-(4-methoxy-3-methyl-pyrazol-1-yl)phenyl]methyl]morpholine-4-carboxylate (52 mg, 54 % yield) obtained as a colourless oil.

¹H NMR(DMSO-*d₆*, 400 MHz): δ (ppm) 7.78 (s, 1H), 7.52 (d, *J*=1.8 Hz, 1H), 7.43 (d, *J*=8.2 Hz, 1H), 7.32 (dd, *J*=8.3, 1.9 Hz, 1H), 3.80 (dd, *J*=11.9, 2.8 Hz, 2H), 3.73 (s, 3H), 3.72 - 3.65 (m, 1H), 3.55 (dddd, *J*=10.3, 7.7, 5.2, 2.5 Hz, 1H), 3.41 - 3.32 (m, 1H), 3.00 - 2.55 (m, 1H), 2.14 (s, 3H), 1.40 (s, 9H); m/z = 366 [M+H]⁺- t-Bu.

### Method R: Isoxazole formation

### General procedure R1: 1.1 DMF-DMA

### Litt: Org. Lett. 2019, 21, 835-839

In a sealed vial Under nitrogen, ketone (1 eq.) was diluted in 1,1-dimethoxy-N,N-dimethyl-methanamine (54 eq.). The mixture was stirred at 100°C for 12 h. The mixture was cooled at RT and concentrated under reduced pressure to directly afford the crude product.

### Synthesis of tert-butyl 2-[[3-chloro-2-[(E)-3-(dimethylamino)prop-2-enoyl]phenyl]methyl] morpholine-4-carboxylate (Intermediate 60)

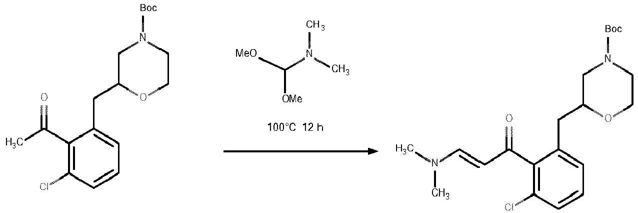

From tert-butyl 2-[(2-acetyl-3-chloro-phenyl)methyl]morpholine-4-carboxylate (250 mg, 0.657 mmol, 1 eq.), tert-butyl 2-[[3-chloro-2-[(E)-3-(dimethylamino)prop-2-enoyl]phenyl]methyl]morpholine-4-carboxylate (329 mg, quant. crude) obtained as an orange oil. m/z = 409 [M+H]⁺

### 1,2-dihydroisoxazole

In a vial, to a stirred solution of dimethylaminoketene (1 eq.) in Ethanol (0.26 M) was added hydroxylamine hydrochloride (1.2 eq.). The mixture was stirred at reflux for 1 h and concentrated under reduced pressure. The crude was directly used in next step without any purification.

### Synthesis of tert-butyl 2-[[3-chloro-2-(5-hydroxy-4H-isoxazol-5-yl)phenyl]methyl] morpholine-4-carboxylate (Intermediate 61)

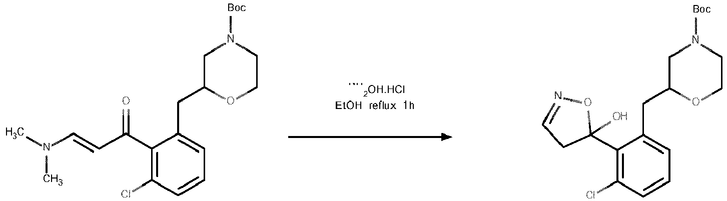

From tert-butyl 2-[[3-chloro-2-[(E)-3-(dimethylamino)prop-2-enoyl]phenyl]methyl]morpholine-4-carboxylate (327 mg, 0.80 mmol, 1 eq.), tert-butyl 2-[[3-chloro-2-(5-hydroxy-4H-isoxazol-5-yl)phenyl]methyl]morpholine-4-carboxylate (422 mg, quant. crude) obtained as an orange oil. m/z = 297 [M+H]⁺- Boc.

### isoxazole

In a vial, to a stirred solution of dihydroisoxazole (1 eq.) in DMF (0.099 M) was added sulfuric acid (20 eq.) dropwise. The reaction mixture was stirred at RT for 12 h (Additional sulfuric acid was added if necessary to get to completion of the reaction). The mixture was diluted slowly with water at 0°C and DCM was added. Then, the reaction mixture was quenched, still at 0°C, with a 50% w/w aqueous solution of NaOH dropwise (pH = 8). The aqueous layer was extracted with DCM (x3). The combined organic layers were dried using a phase separator and concentrated under reduced pressure to afford the expected compound.

### Synthesis of 2-[(3-chloro-2-isoxazol-5-yl-phenyl)methyl]morpholine (Intermediate 62)

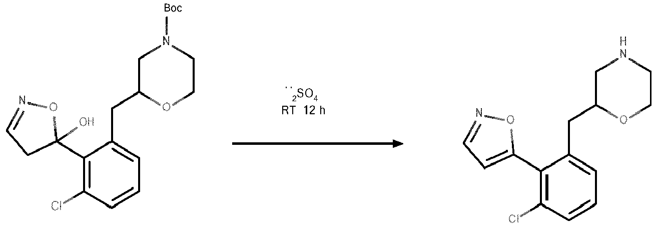

From tert-butyl 2-[[3-chloro-2-(5-hydroxy-4H-isoxazol-5-yl)phenyl]methyl]morpholine-4-carboxylate (422 mg, 0.97 mmol, 1 eq.), 2-[(3-chloro-2-isoxazol-5-yl-phenyl)methyl]morpholine (162 mg, 55 % yield) obtained as an orange oil.

¹H NMR(DMSO-*d₆*, 400 MHz): δ (ppm) 8.75 (d, J = 1.9 Hz, 1H), 7.53 - 7.49 (m, 2H), 7.40 (dd, J = 5.4, 3.6 Hz, 1H), 6.74 (d, J = 1.8 Hz, 1H), 3.64 (d, J = 11.4 Hz, 2H), 2.82 - 2.69 (m, 1H), 2.69 - 2.61 (m, 1H), 2.63 - 2.52 (m, 3H), 2.37 - 2.19 (m, 2H). m/z = 279 [M+H]⁺

### General Procedure for Series 3 compounds

### Method A: Suzuki-Miyaura coupling

### General procedure A1:

### Procedure A1.1: Ar-Br

### Procedure A1.2: Ar-Cl

To a solution of Ar-Br (or Ar-CI) (1.00 eq.) in 1,4-dioxane/H₂O (4:1, 0.1M) were added R-B(OH)₂ (or R-Bpin, or R-BFsK) (1.20 eq.) and Na₂CO₃ (3.00 eq.). The reaction mixture was degassed with argon for 5 min and Pd(dppf)Cl₂ DCM (0.10 eq.) was added and the reaction mixture was heated at 100°C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 100%) to afford the desired product.

### Synthesis of 3-chloro-2-cyclopropyl-benzaldehyde (Intermediate 63)

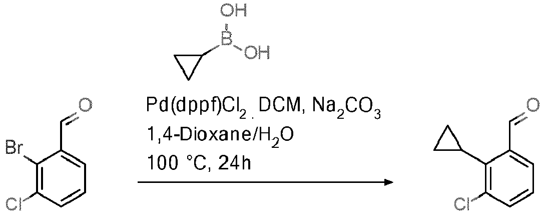

3-chloro-2-cyclopropyl-benzaldehyde (860 mg, 68%), orange oil. General procedure A1.1 from 2-bromo-3-chlorobenzaldehyde (1.5 g, 6.70 mmol, 1.00 eq.) and cyclopropylboronic acid (700 mg, 8.15 mmol, 1.20 eq.).

¹H NMR (DMSO-*d*₆, 400 MHz) *δ* 10.68 (d, *J* = 0.8 Hz, 1H), 7.73-7.68 (m, 2H), 7.43 (t, *J* = 7.9 Hz, 1H), 2.13-2.06 (m, 1H), 1.23-1.18 (m, 2H), 0.65-0.61 (m, 2H); m/z = 181.2 [M+H]+.

### General procedure A2_{:}

### Procedure A2.1: Ar-Br

### Procedure A2.2: Ar-Cl

To a solution of Ar-Br (or Ar-CI) (1.00 eq.) in 1 ,4-dioxane/H₂O (4:1, 0.1 M) were added R-B(OH)₂ (or R-Bpin) (1.40 eq.) and K₃PO₄ (2.00 eq.). The reaction mixture was degassed with argon for 5 min and Pd₂(dba)₃ (0.10 eq.) and PCys (0.15 eq.) were added and the reaction mixture was heated at 100°C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 100%) to afford the desired product.

### Synthesis of 4-(2-cyclopropyl-3-formyl-phenyl)-2-fluoro-benzonitrile (Intermediate 64)

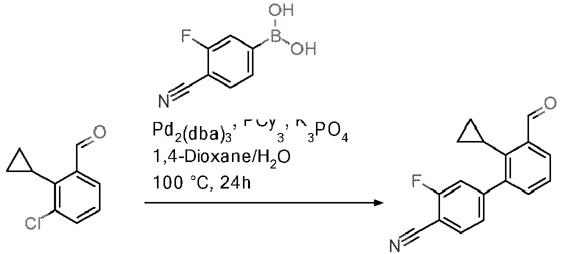

4-(2-cyclopropyl-3-formyl-phenyl)-2-fluoro-benzonitrile (1.32 g, 78%), pale yellow solid. General procedure A2.2 from 3-chloro-2-cyclopropyl-benzaldehyde (1.28 g, 6.38 mmol, 1.00 eq.) and (4-cyano-3-fluorophenyl)boronic acid (1.50 g, 8.93 mmol, 1.40 eq.).

¹H NMR (DMSO-*d*₆, 400 MHz) *δ* 10.82 (d, *J* = 0.7 Hz, 1H), 8.02 (dd, *J* = 8.0, 7.0 Hz, 1H), 7.84 (dd, *J* = 7.5, 1.6 Hz, 1H), 7.72 (dd, *J* = 10.6, 1.5 Hz, 1H), 7.61 (dd, *J* = 7.6, 1.6 Hz, 1H), 7.54-7.52 (m, 2H), 2.48-2.42 (m, 1H), 0.87-0.82 (m, 2H), 0.13 (td, *J* = 6.0, 4.4 Hz, 2H); m/z = 266.2 [M+H]+.

### General procedure A3:

To a solution of Ar-Br (1.00 eq.) in 1,4-dioxane/H₂O (4:1, 0.1M) were added R-B(OH)₂ (or R-Bpin) (2.10 eq.) and Na₂CO₃ (or K₃PO₄) (2.40 eq.). The reaction mixture was degassed with argon for 5 min and Pd(PPh₃)₄ (0.05 eq.) was added and the reaction mixture was heated at 100°C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 100%) to afford the desired product.

### Synthesis of tert-butyl 2-[(3-chloro-2-oxazol-5-yl-phenyl)methyl]-2,8-diazaspiro [3.5]nonane-8-carboxylate (Intermediate 65)

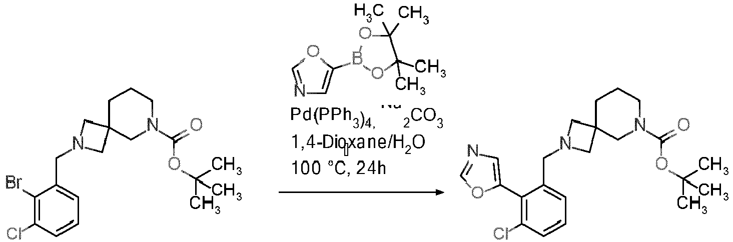

*tert*-Butyl 2-[(3-chloro-2-oxazol-5-yl-phenyl)methyl]-2,8-diazaspiro[3.5]nonane-8-carboxylate (65 mg, 25%), yellow oil. General procedure A3 from *tert*-butyl 2-[(2-bromo-3-chloro-phenyl)methyl]-2,8-diazaspiro[3.5]nonane-8-carboxylate (160 mg, 0.37 mmol, 1.00 eq.) and 5-(tetramethyl-1,3,2-dioxaborolan-2-yl)-1,3-oxazole (155 mg, 0.79 mmol, 2.10 eq.).

¹H NMR (DMSO-*d*₆, 400 MHz) *δ* 8.53 (s, 1H), 7.65-7.47 (m, 3H), 7.39 (s, 1H), 3.43 (br, s, 4H), 3.21 (t, *J* = 5.5 Hz, 2H), 2.94-2.92 (m, 2H), 2.68 (d, *J* = 6.7 Hz, 2H), 1.59 (t, *J* = 6.0 Hz, 2H), 1.38 (s, 9H), 1.33-1.31 (m, 2H); m/z = 418.1 [M+H]+.

### General procedure A4:

To a solution of Ar-Br (1.00 eq.) in toluene (0.1M) were added R-Bpin (1.30 eq.), KF (7.00 eq.) and NaBr (1.80 eq.). The reaction mixture was degassed with argon for 5 min and Pd(PPh₃)₄ (0.10 eq.) was added and the reaction mixture was heated at 125°C for 24h. The reaction mixture was cooled to rt, diluted with AcOEt and quenched with water. The two layers were separated and the aqueous layer was extracted three times with AcOEt. The combined organic layers were dried over a phase separator and concentrated under reduced pressure. The crude was purified by flash chromatography column (EtOAc/cyclohexane 0 to 20%) to afford the desired product.

### Synthesis of tert-butyl N-[rac-(1S,2S,4R)-7-[(3-chloro-2-isothiazol-5-yl-phenyl)methyl]-7-azabicyclo[2.2.1]heptan-2-yl]carbamate (Intermediate 66)

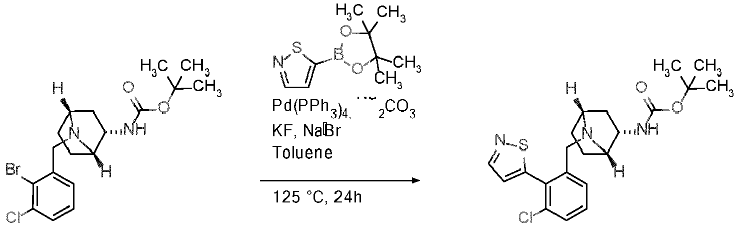

*tert*-Butyl *N*-[rac-(1S,2S,4R)-7-[(3-chloro-2-isothiazol-5-yl-phenyl)methyl]-7-azabicyclo[2.2.1] heptan-2-yl]carbamate (46 mg, 60%), white solid. General procedure A4 from *tert*-butyl N-[rac-(1S,2S,4R)-7-[(2-bromo-3-chloro-phenyl)methyl]-7-azabicyclo[2.2.1]heptan-2-yl]carbamate (73 mg, 0.18 mmol, 1.00 eq.) and 5-(4,4,5,5-tetramethyl-1,3,2-dioxaborolan-2-yl)-1,2-thiazole (51 mg, 0.23 mmol, 1.30 eq.).

¹H NMR (DMSO-*d*₆, 400 MHz) *δ* 8.65 (d, *J* = 1.7 Hz, 1H), 7.56-7.42 (m, 4H), 6.96 (d, *J* = 6.6 Hz, 1H), 3.64 (br, s, 1H), 3.27-3.24 (m, 2H), 3.09 (t, *J* = 4.2 Hz, 1H), 2.96 (t, *J* = 4.4 Hz, 1H), 1.91-1.86 (m, 1H), 1.60-1.58 (m, 2H), 1.46-1.29 (m, 11H), 0.96 (dd, *J* = 11.9, 4.5 Hz, 1H); m/z = 420.0 [M+H]+.

### General procedure A5:

To a solution of Ar-Br (1.00 eq.) in toluene/H₂O (9:1, 0.2M) were added R-BF₃K (1.10 eq.), Cs₂CO₃ (3.00 eq.). The reaction mixture was degassed with argon for 5 min and Pd(OAc)₂ (0.10 eq.) and cataCXium A (0.10 eq.) were added and the reaction mixture was heated at 100°C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 100%) to afford the desired product.

### Synthesis of tert-butyl N-[rac-(1S,2S,4R)-7-[(3-chloro-2-cyclobutyl-phenyl)methyl]-7-azabicyclo[2.2.1]heptan-2-yl]carbamate (Intermediate 67)

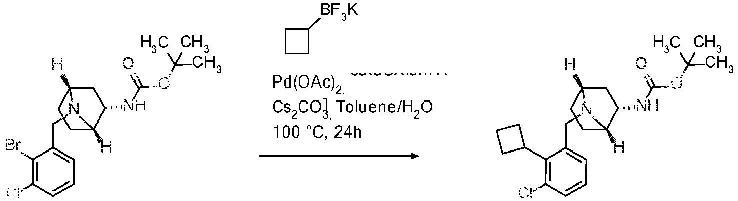

*tert*-Butyl *N*-[rac-(1S,2S,4R)-7-[(3-chloro-2-cyclobutyl-phenyl)methyl]-7-azabicyclo[2.2.1]heptan-2-yl]carbamate (118 mg, 42%), yellow sticky oil. General procedure A5 from *tert*-butyl *N*-[rac-(1S,2S,4R)-7-[(2-bromo-3-chloro-phenyl)methyl]-7-azabicyclo[2.2.1]heptan-2-yl]carbamate (300 mg, 0.71 mmol, 1.00 eq.) and potassium cyclobutyltrifluoroboranuide (131 mg, 0.79 mmol, 1.10 eq.).

¹H NMR (DMSO-*d*₆, 400 MHz) *δ* 7.27 (dd, *J* = 7.9, 1.5 Hz, 1H), 7.22 (d, *J* = 7.4 Hz, 1H), 7.12 (t, *J* = 7.7 Hz, 1H), 6.96 (d, *J* = 6.6 Hz, 1H), 4.22 (p, *J* = 9.6 Hz, 1H), 3.64 (br, s, 1H), 3.59-3.51 (m, 2H), 3.30 - 3.28 (m, 1H), 3.11 (t, *J* = 4.3 Hz, 1H), 3.04 (t, *J* = 4.7 Hz, 1H), 2.78-2.66 (m, 2H), 2.28-2.22 (m, 2H), 1.96-1.88 (m, 3H), 1.76 (br, s, 1H), 1.65-1.58 (m, 2H), 1.36 (s, 10H), 0.97 (dd, *J* = 12.0, 4.7 Hz, 1H); m/z = 391.3 [M+H]+.

### General procedure A6:

To a solution of Ar-I (1.00 eq.) in THF (0.15M) were added R-BPin (1.10 eq.), CsF (2.50 eq.). The reaction mixture was degassed with argon for 5 min, heated at 60 °C and Pd(PPh₃)₄ (0.10 eq.) was added and the reaction mixture was heated at 80 °C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/heptane 0 to 20%) to afford the desired product.

### Synthesis of (2-allyl-3-bromo-phenyl)methanol (Intermediate 68)

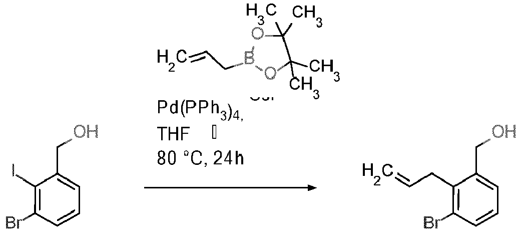

(2-allyl-3-bromo-phenyl)methanol (411 mg, 54%), pale yellow. General procedure A6 from 3-Bromo-2-iodobenzyl alcohol (1.00 g mg, 3.10 mmol, 1.00 eq.) and 2-allyl-4,4,5,5-tetramethyl-1,3,2-dioxaborolane (573 mg, 3.41 mmol, 1.10 eq.).

¹H NMR (DMSO-*d*₆, 400 MHz) *δ* 7.52 (dd, *J* = 7.9, 1.3 Hz, 1H), 7.44 (dd, *J* = 7.6, 1.2 Hz, 1H), 7.18 (t, *J* = 7.8 Hz, 1H), 5.97 - 5.81 (m, 1H), 5.27 (t, *J* = 5.5 Hz, 1H), 5.03 (dq, *J* = 10.1, 1.7 Hz, 1H), 4.89 (dq, *J* = 17.1, 1.8 Hz, 1H), 4.54 (d, *J* = 5.5 Hz, 2H), 3.54 (dt, *J* = 5.9, 1.8 Hz, 2H).

### Method B: Reductive amination

### General procedure B1:

To a solution of the aldehyde (1.00 eq.) in MeOH/AcOH (9:1, 0.1M) was added the amine (1.50 eq.). The reaction mixture was stirred for 10 min at rt then supported NaBH₃CN (1.50 eq.) was added. The resulting mixture was stirred at rt for 1-24h. The reaction mixture was filtered and concentrated under reduced pressure. The crude was purified on by flash chromatography column (EtOAc/cyclohexane 0 to 100%) to afford the desired product.

### Synthesis of tert-butyl 3-[[3-(4-cyano-3-fluoro-phenyl)-2-cyclopropyl-phenyl]methyl]-3,6-diazabicyclo[3.1.1]heptane-6-carboxylate (Intermediate 69)

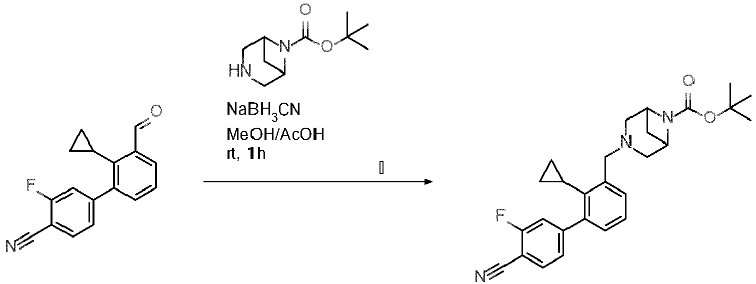

*tert*-Butyl-3-[[3-(4-cyano-3-fluoro-phenyl)-2-cyclopropyl-phenyl]methyl]-3,6-diazabicyclo[3.1.1] heptane-6-carboxylate (82 mg, 69%), white solid. General procedure B1 from 4-(2-cyclopropyl-3-formyl-phenyl)-2-fluoro-benzonitrile (70 mg, 0.26 mmol, 1.00 eq.) and *tert*-butyl 3,6-diazabicyclo[3.1.1]heptane-6-carboxylate (80 mg, 0.40 mmol, 1.50 eq.).

¹H NMR (DMSO-*d*₆, 400 MHz) *δ* 7.98-7.94 (m, 1H), 7.60 (dd, *J* = 10.7, 1.3 Hz, 1H), 7.49-7.45 (m, 2H), 7.31 (t, *J* = 7.6 Hz, 1H), 7.18 (dd, *J* = 7.6, 1.2 Hz, 1H), 4.00-3.95 (m, 4H), 3.11 (br, s, 2H), 2.83-2.73 (m, 2H), 2.33 (q, *J* = 6.3 Hz, 1H), 2.11-2.03 (m, 1H), 1.65 (d, *J* = 7.9 Hz, 1H), 1.41 (s, 9H), 0.74-0.69 (m, 2H), -0.01 (q, *J* = 5.6 Hz, 2H); m/z = 448.2 [M+H]+.

### General procedure B2:

To a solution of the amine (1.30 eq.) in DCM (0.2M) was added NEts (1.00 eq.). The reaction mixture was stirred at rt for 10 min then the aldehyde (1.00 eq.) was added. The reaction mixture was stirred at rt for 1h then STAB (1.80 eq.) was added. The resulting mixture was stirred at rt for 1-24h. The reaction mixture was diluted with DCM and quenched with water. The two layers were separated and the aqueous layer was extracted three times with DCM. The combined organic layers were dried over a phase separator and concentrated under reduced pressure. The crude was purified by flash chromatography column (MeOH/DCM 0 to 10%) to afford the desired product.

### Synthesis of tert-butyl N-[rac-(1S,2S,4R)-7-[(3-bromo-4-hydroxy-phenyl)methyl]-7-azabicyclo[2.2.1]heptan-2-yl]carbamate (Intermediate 70)

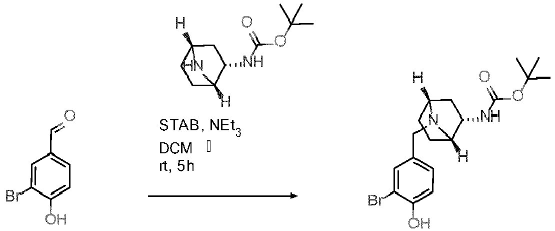

tert-Butyl *N*-[rac-(1S,2S,4R)-7-[(3-bromo-4-hydroxy-phenyl)methyl]-7-azabicyclo[2.2.1]heptan-2-yl]carbamate (376 mg, 98%), white foam. General procedure B2 starting from tert-butyl rac-(1S,2S,4R)-7-azabicyclo[2.2.1]hept-2-ylcarbamate hydrochloride (312 mg, 1.25 mmol, 1.30 eq.) and 3-bromo-4-hydroxybenzaldehyde (97%, 200 mg, 0.97 mmol, 1.00 eq.).

¹H NMR (DMSO-*d*₆, 400 MHz) *δ* 11.92 (s, 1H), 10.06 (s, 1H), 7.41 (s, 1H), 7.13 (d, *J* = 10.3 Hz, 1H), 6.97 (s, 1H), 6.89 (d, *J* = 8.3 Hz, 1H), 4.08 (br, s, 1H), 3.68 (br, s, 1H), 3.41 (br, s, 2H), 3.17-3.09 (m, 3H), 1.99-1.90 (m, 1H), 1.77-1.52 (m, 2H), 1.36 (s, 9H), 1.00 (d, *J* = 8.5 Hz, 1H); m/z = 397.3 [M+H]+.

### General procedure B3:

To a solution of the aldehyde (1.00 eq.) in toluene (0.05M) was added the amine (0.90 eq.). The reaction mixture was degassed for 5 min then NaHCOs (0.04 eq.) and dichloroiridium;1,2,3,4,5-pentamethylcyclopentane (0.04 eq) were added. The reaction mixture was stirred at 110 °C for 24h. The reaction mixture was diluted with AcOEt and quenched with a saturated aqueous NaHCOs solution. The two layers were separated and the aqueous layer was extracted three times with AcOEt. The combined organic layers were dried over a phase separator and concentrated under reduced pressure. The crude was purified by flash chromatography column (MeOH/DCM 0 to 10%) to afford the desired product.

### Synthesis of tert-butyl N-[(1S,4S)-2-[[3-(4-cyano-3-fluoro-phenyl)-4-(2-oxa-8-azaspiro[4.5]decan-8-yl)phenyl]methyl]-2-azabicyclo[2.2.1]heptan-4-yl]carbamate (Intermediate 71)

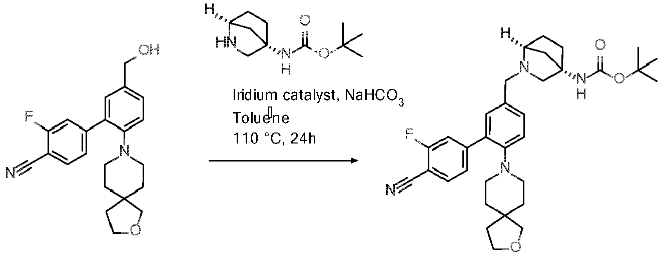

*tert*-Butyl *N*-[(1S,4S)-2-[[3-(4-cyano-3-fluoro-phenyl)-4-(2-oxa-8-azaspiro[4.5]decan-8-yl)phenyl]methyl]-2-azabicyclo[2.2.1]heptan-4-yl]carbamate (32 mg, 67%), yellow gum. General procedure B3 from 2-fluoro-4-[5-(hydroxymethyl)-2-(2-oxa-8-azaspiro[4.5]decan-8-yl)phenyl]benzonitrile (38 mg, 0.10 mmol,1.00 eq.) and *tert*-butyl *N*-[(1S,4S)-2-azabicyclo[2.2.1]heptan-4-yl]carbamate (20 mg, 0.095 mmol, 0.90 eq.).

¹H NMR (DMSO-*d*₆, 400 MHz) *δ* 7.97 (s, 1H), 7.82-7.79 (m, 1H), 7.70 (d, *J* = 6.9 Hz, 1H), 7.30-7.11 (m, 3H), 3.70 (t, *J* = 7.1 Hz, 2H), 3.57 (br, s, 1H), 3.42 (s, 2H), 3.05 (br, s, 1H), 2.73, (br, s, 5H), 2.30 (br, s, 1H), 1.89 (br, s, 2H), 1.66 (t, *J* = 7.2 Hz, 3H), 1.44-1.35 (m, 17H); m/z = 561.5 [M+H]+.

### Method C: Deprotection

### General procedure C1:

To a solution of Boc-*N* product (1.00 eq.) in DCM (0.2M) was added 2,2,2-trifluoroacetic acid (15.0 eq.). The reaction mixture was stirred at rt for 1-24h. The reaction mixture was diluted with DCM and quenched with a saturated aqueous NaHCOs solution. The two layers were separated and the aqueous layer was extracted three times with DCM. The combined organic layers were dried over a phase separator and concentrated under reduced pressure. When necessary, the crude was purified by flash chromatography column (MeOH/DCM 0 to 10%) to afford the desired product.

(2{R},3{R})-2,3-dihydroxybutanedioic acid (0.50 eq.) was solubilized in H₂O (3 mL) and the product in ACN (0.3 mL) was added. The residue was lyophilized overnight to afford the desired product as a tartrate salt.

### Example 323:

### Synthesis of 4-[2-cyclopropyl-3-(3,6-diazabicyclo[3.1.1]heptan-3-ylmethyl)phenyl]-2-fluoro-benzonitrile;(2R,3R)-2,3-dihydroxybutanedioic acid

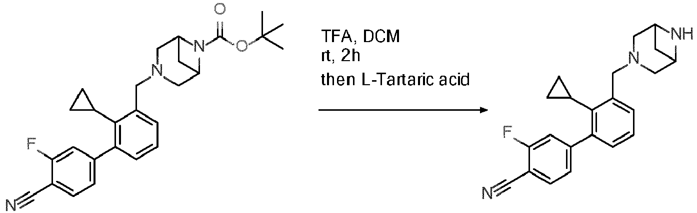

4-[2-cyclopropyl-3-(3,6-diazabicyclo[3.1.1]heptan-3-ylmethyl)phenyl]-2-fluoro-benzonitrile;(2R,3R)-2,3-dihydroxybutanedioic acid (62 mg, 65%), beige solid. General procedure C1 from *tert*-butyl 3-[[3-(4-cyano-3-fluoro-phenyl)-2-cyclopropyl-phenyl]methyl]-3,6-diazabicyclo[3.1.1]heptane-6-carboxylate (82 mg, 0.18 mmol, 1.00 eq.).

¹H NMR (DMSO-*d₆*,600 MHz,) *δ* 8.00-7.93 (m, 1H), 7.62 (dd, *J* = 10.6, 1.4 Hz, 1H), 7.52-7.44 (m, 2H), 7.33 (s, 1H), 7.21 (dd, *J* = 7.7, 1.2 Hz, 1H), 4.01 (s, 2H), 3.95 (br, d, *J* = 5.0 Hz, 2H), 3.75 (s, 1H), 3.25-3.20 (m, 1H), 3.13 (br d, *J* = 11.2 Hz, 2H), 2.92 (d, *J* = 11.3 Hz, 2H), 2.51 (br s, 1H), 2.20-2.03 (m, 2H), 0.84-0.63 (m, 2H), 0.01 (dd, *J* = 5.8, 1.4 Hz, 2H); m/z = 348.2 [M+H]+.

### General procedure C2:

To a solution of Boc-*N* product (1.00 eq.) in DCM (0.1 M) was added HCl in 1 ,4-dioxane (2M, 30.0 eq.). The reaction mixture was stirred at rt for 1-24h. The reaction mixture was concentrated under reduced pressure. When necessary, the crude was purified by flash chromatography column (MeOH/DCM 0 to 10%) to afford the desired product. The crude was solubilized in H₂O (1 mL) and lyophilized overnight to afford the desired product as a HCl salt.

### Example 171:

### Synthesis of 4-[3-[[(1S,4S)-4-amino-2-azabicyclo[2.2.1]heptan-2-yl]methyl]-2-isothiazol-5-yl-phenyl]-2-fluoro-benzonitrile hydrochloride

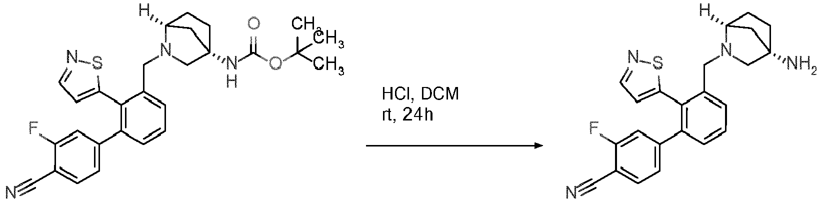

4-[3-[[(1S,4S)-4-amino-2-azabicyclo[2.2.1]heptan-2-yl]methyl]-2-isothiazol-5-yl-phenyl]-2-fluoro-benzonitrile;hydrochloride (11.2 mg, 96%), pale yellow solid. General procedure C2 from *tert-*butyl N-[(1S,4S)-2-[[3-(4-cyano-3-fluoro-phenyl)-2-isothiazol-5-yl-phenyl]methyl]-2-azabicyclo [2.2.1]heptan-4-yl]carbamate (13 mg, 0.026 mmol, 1.00 eq.).

¹H NMR (DMSO-*d*₆, T=350K, 500 MHz) *δ* 8.54 (d, 1H, *J* = 1.7 Hz), 8.02 (d, 1H, *J* = 8.1 Hz), 7.78 (dd, 1H, *J* = 7.1, 7.8 Hz), 7.73 (t, 1H, *J* = 7.8 Hz), 7.58 (dd, 1H, *J* = 1.1, 7.7 Hz), 7.48 (d, 1H, *J* = 1.7 Hz), 7.32 (dd, 1H, *J* = 1.5, 10.3 Hz), 7.20 (dd, 1H, *J* = 1.6, 7.9 Hz), 4.1-4.4 (m, 2H), 3.91 (s, 1H), 3.2-3.6 (m, 2H), 1.7-2.3 (m, 6H)

m/z = 405 [M+H]+.

### General procedure C3:

To a solution of protected alcohol product (1.00 eq.) in THF (0.1M) under nitrogen atmosphere was added tetrabutylammonium fluoride (1M, 1.3 eq.). The reaction mixture was stirred at rt for 1-24h. The reaction mixture was diluted with DCM and quenched with a saturated aqueous NH₄Cl solution. The two layers were separated and the aqueous layer was extracted three times with DCM. The combined organic layers were dried over a phase separator and concentrated under reduced pressure. The crude was purified by flash chromatography column (EtOAc/cyclohexane 0 to 100%) to afford the desired product.

### Synthesis of 4-[2-(4-chlorothiazol-5-yl)-3-(hydroxymethyl)phenyl]-2-fluoro-benzonitrile (Intermediate 72)

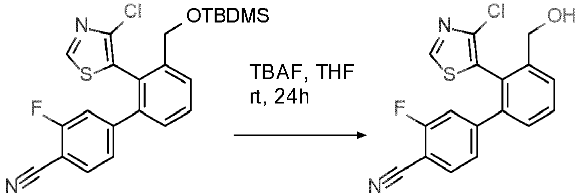

4-[2-(4-Chlorothiazol-5-yl)-3-(hydroxymethyl)phenyl]-2-fluoro-benzonitrile (105 mg, 65%), white solid. General procedure C3 from 4-[3-[[tert-butyl(dimethyl)silyl]oxymethyl]-2-(4-chlorothiazol-5-yl)phenyl]-2-fluoro-benzonitrile (214 mg, 0.47 mmol, 1.00 eq.).

¹H NMR (DMSO-*d*₆, 400 MHz) *δ* 9.13 (s, 1H), 7.86 (dd, *J* = 8.0, 7.0 Hz, 1H), 7.74 (dd, *J* = 7.8, 1.3 Hz, 1H), 7.66 (t, *J* = 7.7 Hz, 1H), 7.41 (dd, *J* = 7.6, 1.4 Hz, 1H), 7.32 (dd, J = 10.4, 1.5 Hz, 1H), 7.12 (dd, *J* = 8.0, 1.6 Hz, 1H), 5.35 (t, *J* = 5.4 Hz, 1H), 4.36 (dd, *J* = 14.2, 5.2 Hz, 1H); m/z = 345.3 [M+H]+.

### General procedure C4:

To a solution of Pd(OH)₂ (20%, 0.10 eq.) in EtOH (0.04M) under nitrogen atmosphere was added protected alcohol derivative (1.00 eq.). The reaction mixture was stirred at rt for 24h under H₂ atmosphere. The reaction mixture was filtered through a pad of celite and washed with EtOH. The filtrate was concentrated under reduced pressure. The crude was engaged in the following reaction without further purification.

### Synthesis of tert-butyl N-[(1S,4S)-2-azabicyclo[2.2.1]heptan-4-yl]carbamate (Intermediate 73)

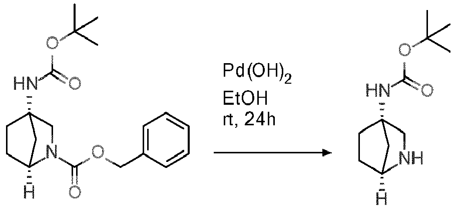

*tert*-Butyl N-[(1S,4S)-2-azabicyclo[2.2.1]heptan-4-yl]carbamate (1.38 g, 99%), pale yellow solid. General procedure C4 from benzyl (1S,4S)-4-(tert-butoxycarbonylamino)-2-azabicyclo[2.2.1]heptane-2-carboxylate (1.59 g, 4.60 mmol, 1.00 eq.).

¹H NMR (DMSO-*d*₆, 400 MHz) *δ* 7.05 (s, 1H), 4.33 (s, 1H), 3.14 (s, 1H), 2.75 (s, 1H), 1.69-1.46 (m, 6H), 1.37 (s, 9H).

### Method D: Alkvlation/Substitution

### General procedure D1:

To a solution of *N*-Boc product (1.00 eq.) in DMF (0.1M) under nitrogen atmosphere at 0 °C was added NaH (60%, 1.50 eq.). The reaction mixture was stirred at 0 °C for 30 min then Alkyl-I (1.20 eq.) was added. The reaction mixture was stirred at 0 °C for 5-24h. The reaction mixture was diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 50%) to afford the desired product.

### Synthesis of tert-butyl N-methyl-N-[rel-(1R,2R,4S)-7-[[3-(4-cyano-3-fluoro-phenyl)-2-cyclopropyl-phenyl]methyl]-7-azabicyclo[2.2.1]heptan-2-yl]carbamate (Intermediate 74)

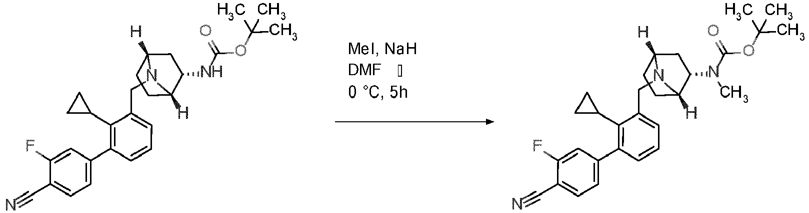

tert-Butyl *N*-methyl-*N*-[rel-(1R,2R,4S)-7-[[3-(4-cyano-3-fluoro-phenyl)-2-cyclopropyl-phenyl] methyl]-7-azabicyclo[2.2.1]heptan-2-yl]carbamate (25 mg, 62%), white solid. General procedure D1 from tert-butyl *N*-[rel-(1R,2R,4S)-7-[[3-(4-cyano-3-fluoro-phenyl)-2-cyclopropyl-phenyl]methyl]-7-azabicyclo[2.2.1]heptan-2-yl]carbamate (39 mg, 0.084 mmol, 1.00 eq.).

¹H NMR (DMSO-*d*₆, 400 MHz) *δ* 7.98- 7.94 (m, 1H), 7.62-7.59 (m, 2H), 7.47 (dd, J = 8.0, 1.5 Hz, 1H), 7.32 (t, J = 7.6 Hz, 1H), 7.17 (dd, J = 7.7, 1.4 Hz, 1H), 4.11-4.07 (m, 1H), 3.87-3.77 (m, 2H), 3.45 (t, J = 4.4 Hz, 1H), 3.29 (s, 1H), 2.82 (s, 3H), 2.08-1.91 (m, 4H), 1.53-1.40 (m, 3H), 1.38 (s, 9H), 0.72-0.70 (m, 2H), 0.02-0.00 (m, 2H); m/z = 476.4 [M+H]+.

### General procedure D2:

### Procedure D2.1: K₂CO₃

### Procedure D2.2: Cs₂CO₃

To a solution of phenol derivative (1.00 eq.) were added R-I (or R-Br) (1.50 eq.) and K₂CO₃ (3.00 eq.). The reaction mixture was stirred at 60 °C for 30 min then Alkyl-I (1.20 eq.) was added. The reaction mixture was stirred at 0 °C for 2h. The reaction mixture was diluted with AcOEt and quenched with water. The two layers were separated and the aqueous layer was extracted three times with AcOEt. The combined organic layers were dried over a phase separator and concentrated under reduced pressure. The crude was purified by flash chromatography column (EtOAc/cyclohexane 0 to 30%) to afford the desired product.

### Synthesis of 3-bromo-2-isopropoxy-benzaldehyde (Intermediate 75)

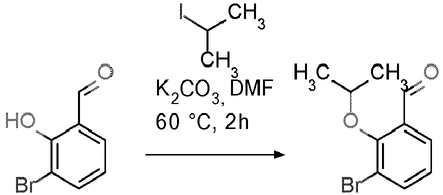

3-Bromo-2-isopropoxy-benzaldehyde (260 mg, quant.), pale yellow solid. General procedure D2.1 from 3-bromo-2-hydroxybenzaldehyde (175 mg, 0.84 mmol, 1.00 eq.) and 2-iodopropane (218 mg, 1.27 mmol, 1.50 eq.).

¹H NMR (DMSO-*d*₆, 400 MHz) *δ* 10.26 (d, *J* = 0.9 Hz, 1H), 7.99 (dd, *J* = 7.9, 1.7 Hz, 1H), 7.75 (dd, *J* = 7.7, 1.7 Hz, 1H), 7.24 (td, *J* = 7.8, 0.8 Hz, 1H), 4.51 (hept, *J* = 6.1 Hz, 1H), 1.32 (d, *J* = 6.1 Hz, 7H).

### General procedure D3

To a solution of benzyl bromide derivative (or OMs) (1.00 eq.) were added amine (1.50 eq.) and K₂CO₃ (3.00 eq.). The reaction mixture was stirred at 60 °C for 30 min then Alkyl-I (1.20 eq.) was added. The reaction mixture was stirred at 0 °C for 2h. The reaction mixture was diluted with AcOEt and quenched with water. The two layers were separated and the aqueous layer was extracted three times with AcOEt. The combined organic layers were dried over a phase separator and concentrated under reduced pressure. The crude was purified by flash chromatography column (EtOAc/cyclohexane 0 to 30%) to afford the desired product.

### Synthesis of tert-butyl N-[rac-(1S,2S,4R)-7-[(3-bromo-2-isopropoxy-phenyl)methyl]-7-azabicyclo[2.2.1]heptan-2-yl]carbamate (Intermediate 76)

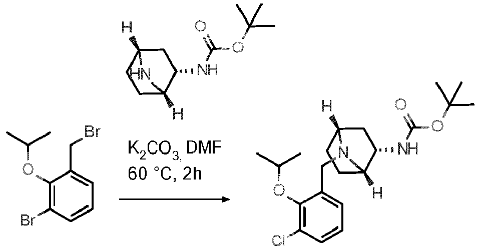

*tert*-Butyl *N*-[rac-(1S,2S,4R)-7-[(3-bromo-2-isopropoxy-phenyl)methyl]-7-azabicyclo[2.2.1] heptan-2-yl]carbamate (265 mg, 91%), colorless oil. General procedure D3 from 1-bromo-3-(bromomethyl)-2-isopropoxy-benzene (210 mg, 0.64 mmol, 1.00 eq.) and *tert*-butyl rac-(1S,2S,4R)-7-azabicyclo[2.2.1]hept-2-ylcarbamate hydrochloride (252 mg, 0.96 mmol, 1.50 eq.). ¹H NMR (DMSO-*d*₆, 400 MHz) *δ* 7.51-7.47 (m, 2H), 7.05-6.98 (m, 2H), 4.52 (p, *J* = 6.2 Hz, 1H), 3.69 (br, s, 1H), 3.60-3.51 (m, 2H), 3.20 (br, s, 1H), 3.12 (t, *J* = 4.7 Hz, 1H), 1.98-1.91 (m, 1H), 1.83-1.52 (m, 3H), 1.37 (s, 9H), 1.26 (dd, *J* = 6.1, 2.6 Hz, 6H), 1.00 (dd, *J* = 11.9, 4.6 Hz, 1H); m/z = 441.3 [M+H]+.

### Method E: Reduction

### General procedure E1:

To a solution of aldehyde (1.00 eq.) in MeOH (0.1M) under nitrogen atmosphere at 0 °C was added NaBH₄ (1.10 eq.). The reaction mixture was stirred at 0 °C for 2h. The reaction mixture was diluted with DCM and quenched with a saturated aqueous NaHCO₃ solution. The two layers were separated and the aqueous layer was extracted three times with DCM. The combined organic layers were dried over a phase separator and concentrated under reduced pressure. The crude was purified by flash chromatography column (EtOAc/cyclohexane 0 to 50%) to afford the desired product.

### Synthesis of [3-chloro-2-(4-chlorothiazol-5-yl)phenyl]methanol (Intermediate 77)

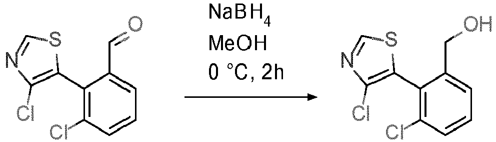

[3-Chloro-2-(4-chlorothiazol-5-yl)phenyl]methanol (720 mg, quant.), yellow solid. General procedure E1 from 3-chloro-2-(4-chlorothiazol-5-yl)benzaldehyde (433 mg, 1.68 mmol, 1.00 eq.). ¹H NMR (DMSO-*d*₆, 400 MHz) *δ* 9.32 (s, 1H), 7.61-7.57 (m, 1H), 7.55-7.53 (m, 2H), 5.38 (t, *J* = 5.3 Hz, 1H), 4.29-4.20 (m, 2H); m/z = 260.0 [M+H]+.

### General procedure E2:

To a solution of Alkene (1.00 eq.) in EtOAc (0.02M) under nitrogen atmosphere was added PtO₂ (0.20 eq.). The reaction mixture was purged with dihydrogen and stirred under hydrogen atmosphere at RT for 24h. The mixture was filtered over celite and washed with AcOEt. The solvent was evaporated under reduced pressure. The crude was purified by flash chromatography column (EtOAc/cyclohexane 5 to 30%) to afford the desired product as a mixture with another product (60:40).

### Synthesis of tert-butyl N-[rac-(1S,2S,4R)-7-[(3-chloro-2-isopropyl-phenyl)methyl]-7-azabicyclo[2.2.1]heptan-2-yl]carbamate (Intermediate 78)

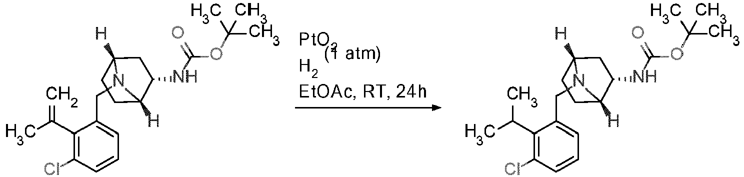

tert-butyl N-[rac-(1S,2S,4R)-7-[(3-chloro-2-isopropyl-phenyl)methyl]-7-azabicyclo[2.2.1]heptan-2-yl]carbamate (80 mg, 60%), colorless wax. General procedure E2 from tert-butyl N-[rac-(1S,2S,4R)-7-[(3-chloro-2-isopropenyl-phenyl)methyl]-7-azabicyclo[2.2.1]heptan-2-yl]carbamate (80 mg, 0.21 mmol, 1.00 eq.).

¹H NMR (DMSO-*d*₆, 400 MHz) *δ* 7.34 - 7.08 (m, 3H), 6.96 (d, *J* = 6.6 Hz, 1H), 3.90 - 3.73 (m, 1H), 3.71 - 3.49 (m, 2H), 3.18 - 3.02 (m, 2H), 1.92 - 1.49 (m, 4H), 1.36 (s, 9H), 1.31 - 1.24 (m, 8H), 1.05 - 0.95 (m, 1H); m/z = 379.3 [M+H]+.

### General procedure E3:

To a solution of alkene (1.00 eq.) in acetic acid (0.05M) under nitrogen atmosphere at 0 °C was added NaBH₃CN (5.00 eq.). The reaction mixture was stirred at RT for 24h. The reaction mixture was filtered, washed with methanol and concentrated under reduced pressure. The crude was taken up in DCM and a saturated aqueous NaHCOs solution was added. The two layers were separated and the aqueous layer was extracted three times with DCM. The combined organic layers were dried over a phase separator and concentrated under reduced pressure. The crude was purified by flash chromatography column (EtOAc/heptane 5 to 25%) to afford the desired product.

### Synthesis of tert-butyl 2-[(4-bromoindolin-1-yl)methyl]morpholine-4-carboxylate (Intermediate 79)

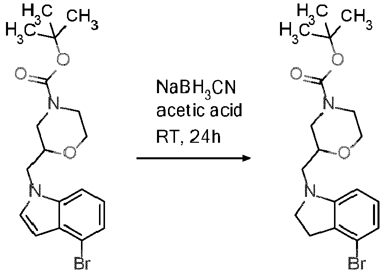

tert-butyl 2-[(4-bromoindolin-1-yl)methyl]morpholine-4-carboxylate (133 mg, 87%), colorless oil. General procedure E3 from tert-butyl 2-[(4-bromoindol-1-yl)methyl]morpholine-4-carboxylate (150 mg, 0.39 mmol, 1.00 eq.).

¹H NMR (DMSO-*d*₆, 400 MHz) *δ* 6.92 (t, *J* = 7.9 Hz, 1H), 6.69 (d, *J* = 7.6 Hz, 1H), 6.48 (d, *J* = 7.8 Hz, 1H), 3.99 - 3.78 (m, 2H), 3.70 (d, *J* = 13.4 Hz, 1H), 3.60 - 3.54 (m, 1H), 3.50 (dd, *J* = 15.7, 8.4 Hz, 2H), 3.40 (td, *J* = 11.6, 2.8 Hz, 1H), 3.15 (qd, *J* = 14.4, 5.7 Hz, 2H), 2.91 (t, *J* = 8.6 Hz, 3H), 2.67 (s, 1H), 1.41 (s, 9H); m/z = 397.3 [M+H]+.

### General procedure E4:

To a solution of amide derivative (1.00 eq.) in THF (0.1M) under nitrogen atmosphere was added borane tetrahydrofuran or DIBAL-H (1M, 2.50 eq.). The reaction mixture was stirred at rt for 1-24h. The reaction mixture was slowly quenched with a aqueous 2M HCl in MeOH solution. The reaction mixture was stirred 4h at rt. The reaction mixture was basified to pH 9 with a saturated aqueous NaHCOs solution. The two layers were separated and the aqueous layer was extracted three times with Et₂O. The combined organic layers were dried over a phase separator and concentrated under reduced pressure. The crude was purified by flash chromatography column (EtOAc/cyclohexane 5 to 20%) to afford the desired product.

### Synthesis of tert-butyl 2-[(3-bromo-2-chloro-5-methyl-anilino)methyl]morpholine-4-carboxylate (Intermediate 80)

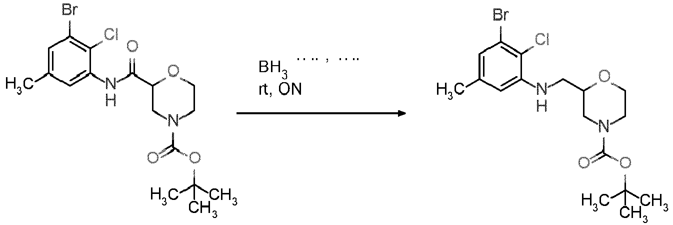

*tert*-butyl 2-[(3-bromo-2-chloro-5-methyl-anilino)methyl]morpholine-4-carboxylate (77 mg, 74%), pale yellow oil. General procedure E4 from *tert*-butyl 2-[(3-bromo-2-chloro-5-methylphenyl)carbamoyl]morpholine-4-carboxylate (95 mg, 0.22 mmol, 1.00 eq.). m/z = 421.2 [M+H]+.

### General procedure E5:

To a solution of nitrobenzene derivative (1.00 eq.) and zinc (10.0 eq.) in MeOH (0.1M) was added NH₄Cl (10.0 eq.). The reaction mixture was stirred at rt for 24h. The mixture was filtered over celite and washed with AcOEt. The solvent was evaporated under reduced pressure. The crude was purified by flash chromatography column (EtOAc/cyclohexane 5 to 30%) to afford the desired product.

### Synthesis of 3-bromo-2-chloro-5-methyl-aniline (Intermediate 81)

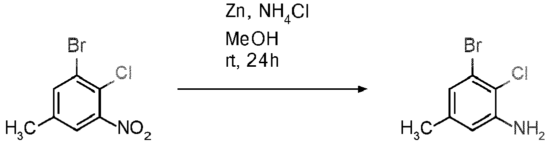

3-bromo-2-chloro-5-methyl-aniline (350 mg, 60%), brown solid. General procedure E5 from 1-bromo-2-chloro-5-methyl-3-nitrobenzene (650 mg, 2.60 mmol, 1.00 eq.).

¹H NMR (DMSO-*d*₆, 400 MHz) *δ* 6.71 (dd, J = 2.0, 0.7 Hz, 1H), 6.58 (dd, J = 1.9, 0.8 Hz, 1H), 5.56 (s, 2H), 2.14 (s, 3H); m/z = 222.1 [M+H]⁺.

### Method F: Alcohol transformation

### General procedure F1:

To a solution of benzyl alcohol (1.00 eq.) in DCM (0.1M) under nitrogen atmosphere were added CBr₄ (1.10 eq.) and PPh₃ (1.10 eq.). The reaction mixture was stirred at rt for 2h. The reaction mixture was diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 10%) to afford the desired product.

### Synthesis of 1-bromo-3-(bromomethyl)-2-isopropoxy-benzene (Intermediate 82)

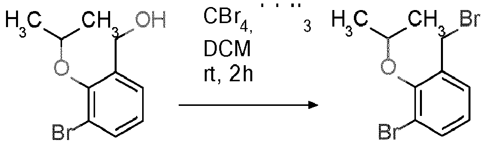

1-Bromo-3-(bromomethyl)-2-isopropoxy-benzene (230 mg, 92%), colorless liquid. General procedure F1 from (3-bromo-2-isopropoxy-phenyl)methanol (210 mg, 0.76 mmol, 1.00 eq.). ¹H NMR (DMSO-*d*₆, 400 MHz) *δ* 7.60 (dd, *J* = 8.0, 1.6 Hz, 1H), 7.49 (dd, *J* = 7.7, 1.6 Hz, 1H), 7.07 (t, *J* = 7.8 Hz, 1H), 4.58 (dt, *J* = 12.3, 6.2 Hz, 1H), 1.30 (d, *J* = 6.1 Hz, 6H).

### General procedure F2:

To a solution of benzyl alcohol (1.00 eq.) and NEts (2.00 eq.) in DCM (0.03M) under nitrogen atmosphere at 0 °C was added methanesulfonyl chloride (1.50 eq.). The reaction mixture was stirred at 0 °C for 1h. The reaction mixture was diluted with DCM and quenched with a saturated aqueous NaHCOs solution. The two layers were separated and the aqueous layer was extracted three times with DCM. The combined organic layers were dried over a phase separator and concentrated under reduced pressure. The crude was engaged in the following reaction without further purification.

### Synthesis of [3-(4-cyano-3-fluoro-phenyl)-2-thiazol-5-yl-phenyl]methyl methanesulfonate (Intermediate 83)

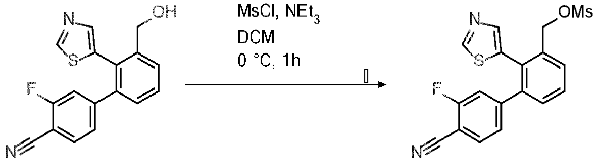

[3-(4-cyano-3-fluoro-phenyl)-2-thiazol-5-yl-phenyl]methyl methanesulfonate (120 mg, 79%), dark orange oil. General procedure F2 from 2-fluoro-4-[3-(hydroxymethyl)-2-thiazol-5-yl-phenyl]benzonitrile (90 mg, 0.29 mmol, 1.00 eq.).

¹H NMR (DMSO-*d*₆, 400 MHz) *δ* 9.10 (d, *J* = 0.8 Hz, 1H), 7.84-7.81 (m, 2H), 7.76 (dd, *J* = 7.7, 1.4 Hz, 1H), 7.68 (t, *J* = 7.7 Hz, 1H), 7.55 (dd, *J* = 7.7, 1.4 Hz, 1H), 7.39 (dd, *J* = 10.5, 1.5 Hz, 1H), 7.18 (dd, J = 8.0, 1.6 Hz, 1H), 3.35 (s, 2H), 3.11 (s, 3H); m/z = 389.2 [M+H]+.

### General procedure F3:

To a solution of benzyl alcohol (1.00 eq.) and R-OH (1.10 eq.) in Toluene (0.07M) under nitrogen atmosphere was added Tsunoda reagent (2.00 eq.). The reaction mixture was stirred at 100 °C for 1h. The reaction mixture was diluted with DCM and quenched with a saturated aqueous NaHCOs solution. The two layers were separated and the aqueous layer was extracted three times with DCM. The combined organic layers were dried over a phase separator and concentrated under reduced pressure. The crude was purified by flash chromatography column (MeOH/DCM 0 to 10%) to afford the desired product.

### Synthesis of tert-butyl N-[rac-(1S,2S,4R)-7-[[3-bromo-4-[[rac-(3R)-tetrahydropyran-3-yl]methoxy]phenyl]methyl]-7-azabicyclo[2.2.1]heptan-2-yl]carbamate (Intermediate 84)

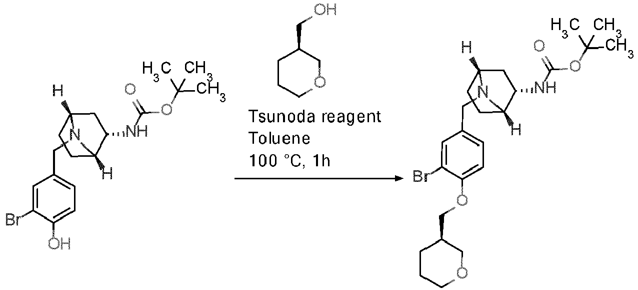

*tert*-Butyl N-[rac-(1S,2S,4R)-7-[[3-bromo-4-[[rac-(3R)-tetrahydropyran-3-yl]methoxy]phenyl] methyl]-7-azabicyclo[2.2.1]heptan-2-yl]carbamate (118 mg, 94%), colorless liquid. General procedure F3 from *tert*-butyl *N*-[rac-(1S,2S,4R)-7-[(3-bromo-4-hydroxy-phenyl)methyl]-7-azabicyclo[2.2.1]heptan-2-yl]carbamate (100 mg, 0.24 mmol, 1.00 eq.).

¹H NMR (DMSO-*d*₆, 400 MHz) *δ* 7.50 (d, *J* =2.1 Hz, 1H), 7.28-7.26 (m, 1H), 7.03 (d, *J* = 8.4 Hz, 1H), 6.96 (d, *J* = 6.6 Hz, 1H), 3.96-3.86 (m, 3H), 3.75 (dt, *J* = 11.3, 3.9 Hz, 1H), 3.66 (br, s, 1H), 3.42 (s, 2H), 3.37-3.32 (m, 2H), 3.14 (br, s, 1H), 3.06 (br, s, 1H), 2.09-2.02 (m, 2H), 1.84-1.40 (m, 8H), 1.36 (s, 9H), 0.98 (dd, *J* = 12.0, 4.6 Hz, 1H); m/z = 495.4 [M+H]+.

### General procedure F4:

To a solution of R-OH (1.0 eq.) in DCM (0.1M) under nitrogen atmosphere was added N-ethyl-N-(trifluoro-lambda∼4∼-sulfanyl)ethanamine (1.7 eq.). The reaction mixture was stirred at RT for 24h. The reaction mixture was diluted with DCM and quenched with a saturated aqueous NaHCOs solution. The two layers were separated and the aqueous layer was extracted three times with DCM. The combined organic layers were dried over a phase separator and concentrated under reduced pressure. The crude was purified by flash chromatography column (AcOEt/n-heptane 0 to 70%) to afford the desired product as a mixture with another specie.

### Synthesis of tert-butyl N-[rac-(1S,2S,4R)-7-[[3-chloro-2-(4-fluoro-1-piperidyl)phenyl]methyl]-7-azabicyclo[2.2.1]heptan-2-yl]carbamate (Intermediate 85)

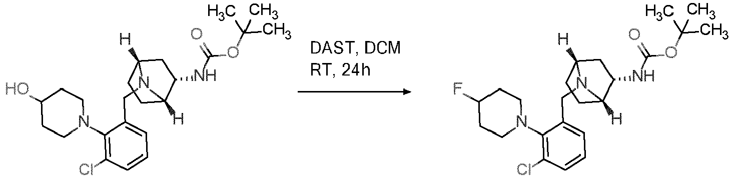

tert-butyl-N-[rac-(1S,2S,4R)-7-[[3-chloro-2-(4-fluoro-1-piperidyl)phenyl]methyl]-7-azabicyclo[2.2.1]heptan-2-yl]carbamate (55 mg, 20%), white solid. General procedure F4 from tert-butyl N-[rac-(1S,2S,4R)-7-[[3-chloro-2-(4-hydroxy-1-piperidyl)phenyl]methyl]-7-azabicyclo[2.2.1]heptan-2-yl]carbamate (130 mg, 0.30 mmol, 1.00 eq.).

¹H NMR (DMSO-*d*₆, 400 MHz) *δ* 7.42 - 7.27 (m, 2H), 7.21 - 7.10 (m, 1H), 6.97 (s, 1H), 3.81 (s, 1H), 3.70 - 3.57 (m, 3H), 3.44 (d, *J* = 9.6 Hz, 1H), 3.33 - 3.16 (m, 5H), 3.02 (d, *J* = 50.4 Hz, 2H), 2.82 (s, 1H), 2.30 (d, *J* = 24.5 Hz, 1H), 2.04 (t, *J* = 15.9 Hz, 1H), 1.98 - 1.52 (m, 4H), 1.37 (s, 9H), 1.00 (t, *J* = 8.9 Hz, 1H); m/z = 418.3 [M+H]+.

### Method G: Curtius rearrangement

### General procedure G1:

To a solution of benzyl alcohol (1.00 eq.) in DCM (0.1M) under nitrogen atmosphere were added CBr₄ (1.10 eq.) and PPh₃ (1.10 eq.). The reaction mixture was stirred at rt for 2h. The reaction mixture was diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 0 to 10%) to afford the desired product.

### Synthesis of benzyl 4-(tert-butoxycarbonylamino)-2-azabicyclo[2.2.1]heptane-2-carboxylate (Intermediate 86)

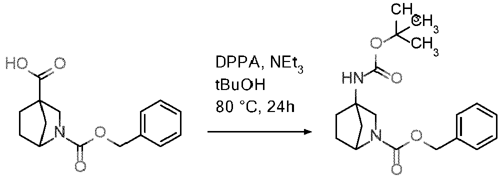

Benzyl 4-(tert-butoxycarbonylamino)-2-azabicyclo[2.2.1]heptane-2-carboxylate (10.21 g, 87%), pale yellow oil. General procedure G1 from 2-benzyloxycarbonyl-2-azabicyclo[2.2.1]heptane-4-carboxylic acid (6.29 g, 30.1 mmol, 1.00 eq.).

¹H NMR (DMSO-*d*₆, 400 MHz) *δ* 7.39-7.27 (m, 5H), 5.07-5.00 (m, 2H), 4.04 (d, J = 14.5 Hz, 0H), 3.25 (br, s, 2H), 1.77-1.68 (m, 5H), 1.58 (br, s, 1H), 1.38 (d, J = 4.9 Hz, 9H); m/z = 347.4 [M+H]+.

### Method H: Buchwald-Hartwig coupling

### General procedure H1:

### H1.1: Ar-Br

### H1.2: Ar-I

To a solution of Ar-Br (or Ar-I) (1.00 eq.) in 1,4-dioxane (0.1M) were added NaOtBu (3.00 eq.) and Xantphos (0.10 eq.). The reaction mixture was degassed with argon for 5 min and Pd₂dba₃ (0.05 eq.) and amine (1.40 eq.) were added. The reaction mixture was heated at 100°C for 5-24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (MeOH/DCM 0 to 10%) to afford the desired product.

### Synthesis of [2-(azetidin-1-yl)-3-chloro-6-fluoro-phenyl]-(1-methyl-1,7-diazaspiro[3.4] octan-7-yl)methanone (Intermediate 87)

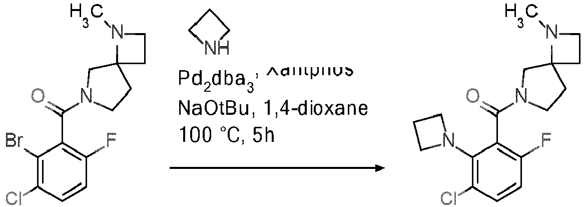

[2-(Azetidin-1-yl)-3-chloro-6-fluoro-phenyl]-(1-methyl-1,7-diazaspiro[3.4]octan-7-yl)methanone (92 mg, 19%), orange sticky oil. General procedure H1.1 from 2(2-bromo-3-chloro-6-fluorophenyl)-(1-methyl-1,7-diazaspiro[3.4]octan-7-yl)methanone (412 mg, 1.12 mmol, 1.00 eq.) and azetidine (0.11 mL, 1.55 mmol, 1.40 eq.).

¹H NMR (DMSO-*d*₆, 400 MHz) *δ* 7.28-7.23 (m, 1H), 6.58 (td, J = 8.6, 5.3 Hz, 1H), 4.24-4.18 (m, 2H), 4.10-4.00 (m, 2H), 3.59-3.35 (m, 3H), 3.29-2.95 (m, 4H), 2.19-1.91 (m, 9H); m/z = 338.2 [M+H]+.

### General procedure H2:

To a solution of Ar-Br (1.00 eq.) in toluene (0.1M) were added amine (1.00 eq.) and NaOtBu (3.00 eq.). The reaction mixture was degassed with argon for 5 min and Pd-113 (0.06 eq.) was added. The reaction mixture was heated at 100°C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (AcOEt/cyclohexane 0 to 25%) to afford the desired product.

### Synthesis of tert-butyl-dimethyl-[[4-(4-methyl-1-piperidyl)phenyl]methoxy]silane (Intermediate 88)

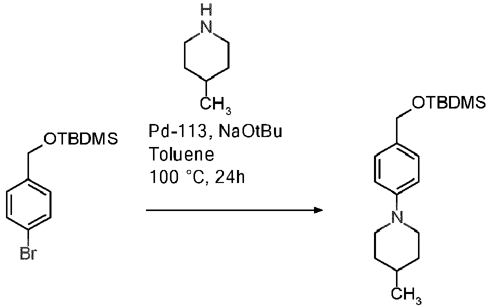

*tert*-Butyl-dimethyl-[[4-(4-methyl-1-piperidyl)phenyl]methoxy]silane (214 mg, quant.), colorless oil. General procedure H2 from (4-bromophenyl)methoxy-tert-butyl-dimethyl-silane (200 mg, 0.65 mmol, 1.00 eq.) and 4-methylpiperidine (81 µL, 0.65 mmol, 1.00 eq.).

¹H NMR (DMSO-*d*₆, 400 MHz) *δ* 7.13-7.10 (m, 2H), 6.90-6.86 (m, 2H), 4.57 (s, 2H), 3.62 (d, J = 12.3 Hz, 2H), 2.61 (td, J = 12.3, 2.6 Hz, 2H), 1.67 (d, J = 12.9 Hz, 2H), 1.48 (ddq, J = 14.7, 7.1, 3.7 Hz, 1H), 1.21 (qd, J = 12.1, 4.0 Hz, 2H), 0.93 (d, J = 6.5 Hz, 3H), 0.88 (s, 9H), 0.05 (s, 6H); m/z = 320.4 [M+H]⁺.

### General procedure H3:

To a solution of Ar-Br (1.00 eq.) in toluene (0.1M) were added amine (1.10 eq.), Cs₂CO₃ (3.00 eq.) and (rac)-BINAP (0.15 eq.). The reaction mixture was degassed with argon for 5 min and Pd₂dba₃ (0.10 eq.) was added. The reaction mixture was heated at 95 °C for 24h. The reaction mixture was cooled to rt, diluted with EtOAc and quenched with water. The two layers were separated and the aqueous layer was extracted three times with EtOAc. The combined organic layers were dried over a phase separator and concentrated under reduced pressure. The crude was purified by flash chromatography column (AcOEt/cyclohexane 0 to 30%) to afford the desired product.

### Synthesis of 3-chloro-2-pyrrolidin-1-yl-benzaldehyde (Intermediate 89)

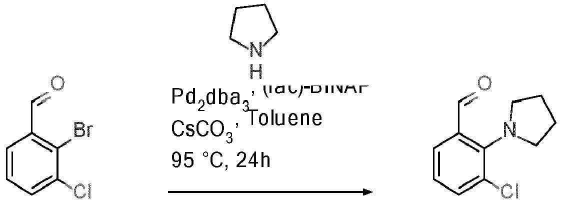

3-chloro-2-pyrrolidin-1-yl-benzaldehyde (545 mg, 53%.), yellow oil. General procedure H3 from 2-bromo-3-chlorobenzaldehyde (600 mg, 2.68 mmol, 1.00 eq.) and pyrrolidine (246 µL, 2.95 mmol, 1.10 eq.).

¹H NMR (DMSO-*d*₆, 400 MHz) *δ* 10.26 (d, *J* = 0.8 Hz, 1H), 7.79 - 7.75 (m, 1H), 7.66 (dd, J = 7.7, 1.6 Hz, 1H), 7.33 (td, *J* = 7.8, 0.8 Hz, 1H), 3.34 - 3.31 (m, 4H), 2.04 - 1.97 (m, 4H); m/z = 210.2 [M+H]+.

### General procedure H4:

To a solution of Ar-Br (1.00 eq.) in toluene (0.1M) were added [Pd(C₄H₉)₃PBr]₂ (0.06 eq.) and NaOtBu (2.00 eq.). The reaction mixture was degassed with argon for 5 min and amine (1.20 eq.) was added. The reaction mixture was heated at 100 °C for 2h. The reaction mixture was cooled to rt, diluted with EtOAc and quenched with water. The two layers were separated and the aqueous layer was extracted three times with EtOAc. The combined organic layers were dried over a phase separator and concentrated under reduced pressure. The crude was purified by flash chromatography column (AcOEt/cyclohexane 0 to 20%) to afford the desired product.

### Synthesis of tert-butyl 2-[[4-(azetidin-1-yl)-3-chloro-N-methyl-anilino]methyl]morpholine-4-carboxylate (Intermediate 90)

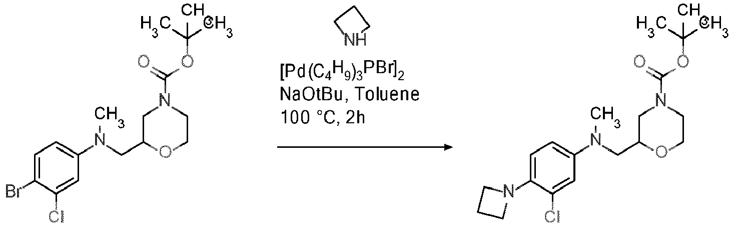

*tert*-butyl 2-[[4-(azetidin-1-yl)-3-chloro-N-methyl-anilino]methyl]morpholine-4-carboxylate (100 mg, 68%.), colorless oil. General procedure H4 from *tert*-butyl 2-[(4-bromo-3-chloro-N-methyl-anilino)methyl]morpholine-4-carboxylate (149 mg, 0.36 mmol, 1.00 eq.) and azetidine (30 µL, 0.42 mmol, 1.20 eq.). m/z = 396.4 [M+H]⁺.
(No NMR)

### General procedure H5:

To a solution of Ar-Br (1.00 eq.) in dioxane (0.2M) under argon atmosphere were added amine (3.00 eq.), NaOtBu (4.00 eq.), Xantphos (0.10 eq.) and Pd₂dba₃ (0.05 eq.). The reaction mixture was heated at 100 °C for 1h. The reaction mixture was cooled to rt and concentrated under reduced. The crude was directly purified by flash chromatography column (MeOH/heptane 0 to 30%) to afford the desired product.

### Synthesis of 1-[[4-(azetidin-1-yl)-3-chloro-phenyl]methyl]-4-methyl-piperazine (Intermediate 91)

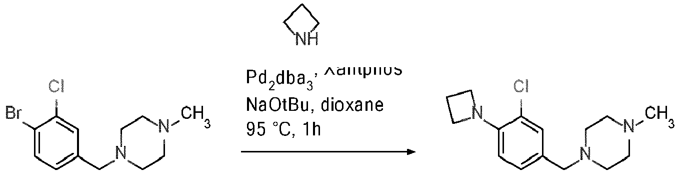

1-[[4-(azetidin-1-yl)-3-chloro-phenyl]methyl]-4-methyl-piperazine (416 mg, 97%.), brown oil. General procedure H5 from 1-[(4-bromo-3-chloro-phenyl)methyl]-4-methyl-piperazine (467 mg, 1.54 mmol, 1.00 eq.) and azetidine (327 µL, 4.61 mmol, 3.00 eq.). m/z = 278.3 [M+H]⁺.
(No NMR)

### General procedure H6:

To a solution of Ar-Br (1.00 eq.) in dioxane (0.1M) were added amine (1.25 eq.) and NaOtBu (3.50 eq.). The reaction mixture was degassed with argon for 5 min and Pd(OAc)₂ (0.50 eq.) and JohnPhos (0.05 eq.) were added. The reaction mixture was heated at 90 °C for 24h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (AcOEt/cyclohexane 0 to 50%) to afford the desired product.

### Synthesis of tert-butyl N-[rac-(1S,2S,4R)-7-[[3-chloro-4-(1-oxa-8-azaspiro[4.5]decan-8-yl)phenyl]methyl]-7-azabicyclo[2.2.1]heptan-2-yl]carbamate (Intermediate 92)

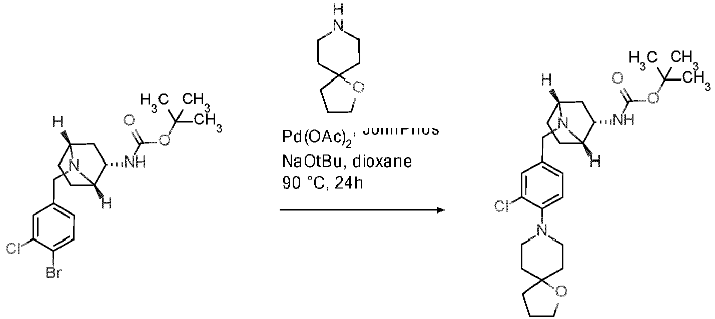

tert-butyl N-[rac-(1S,2S,4R)-7-[[3-chloro-4-(1-oxa-8-azaspiro[4.5]decan-8-yl)phenyl]methyl]-7-azabicyclo[2.2.1]heptan-2-yl]carbamate (66 mg, 33%.), yellow oil. General procedure H6 from tert-butyl N-[rac-(1S,2S,4R)-7-[(4-bromo-3-chloro-phenyl)methyl]-7-azabicyclo[2.2.1]heptan-2-yl]carbamate (150 mg, 0.36 mmol, 1.00 eq.) and 1-oxa-8-azaspiro[4.5]decane hydrochloride (84 mg, 0.45 mmol, 1.25 eq.).

¹H NMR (DMSO-*d*₆, 400 MHz) *δ* 7.34 (d, *J* = 2.0 Hz, 1H), 7.27 - 7.20 (m, 1H), 7.10 (d, *J* = 8.1 Hz, 1H), 3.75 (t, *J* = 6.7 Hz, 2H), 3.71 - 3.59 (m, 1H), 3.43 (br s, 2H), 3.20 - 3.04 (m, 2H), 3.02 - 2.88 (m, 4H), 1.98 - 1.85 (m, 3H), 1.80 - 1.51 (m, 6H), 1.36 (s, 9H), 0.99 (dd, *J* = 12.0, 4.6 Hz, 1H); m/z = 476.1 [M+H]⁺.

### General procedure H7:

To a solution of Ar-Br (1.00 eq.) in dioxane (0.05M) were added amine (1.50 eq.) and Pd₂dba₃ (0.10 eq.). The reaction mixture was degassed with argon for 5 min and NaOtBu (4.1 eq.) was added. The reaction mixture was heated at 95 °C for 24h. The reaction mixture was cooled to rt, diluted with EtOAc and quenched with water. The two layers were separated and the aqueous layer was extracted three times with EtOAc. The combined organic layers were dried over a phase separator and concentrated under reduced pressure. The crude was purified by flash chromatography column (AcOEt/cyclohexane 0 to 100%) to afford the desired product.

### Synthesis of tert-butyl N-[rac-(1S,2S,4R)-7-[[3-chloro-4-[4-(fluoromethyl)-1-piperidyl]phenyl]methyl]-7-azabicyclo[2.2.1]heptan-2-yl]carbamate (Intermediate 93)

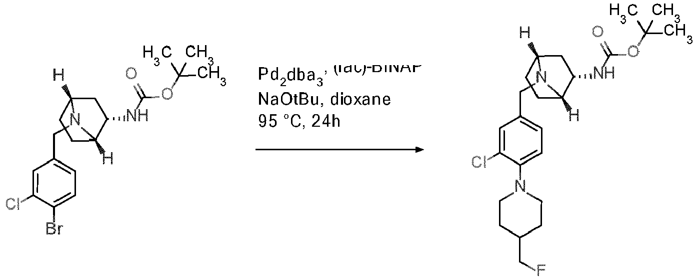

tert-butyl N-[rac-(1S,2S,4R)-7-[[3-chloro-4-[4-(fluoromethyl)-1-pipe ridyl]phenyl]methyl]-7-azabicyclo[2.2.1]heptan-2-yl]carbamate (45 mg, 31%.), yellow foam. General procedure H7 from tert-butyl N-[rac-(1S,2S,4R)-7-[(4-bromo-3-chloro-phenyl)methyl]-7-azabicyclo[2.2.1]heptan-2-yl]carbamate (100 mg, 0.24 mmol, 1.00 eq.) and 4-(fluoromethyl)piperidine hydrochloride (58 mg, 0.36 mmol, 1.50 eq.).

¹H NMR (DMSO-*d*₆, 400 MHz) *δ* 7.24 (d, *J* = 8.3 Hz, 1H), 7.10 (d, *J* = 8.2 Hz, 1H), 6.97 (d, *J* = 6.6 Hz, 1H), 4.42 (d, *J* = 5.9 Hz, 1H), 4.30 (d, *J* = 5.6 Hz, 1H), 3.67 (br s, 1H), 3.52 - 3.41 (m, 2H), 3.28 (d, *J* = 10.8 Hz, 2H), 3.12 (d, *J* = 28.6 Hz, 2H), 2.64 (t, *J =* 11.9 Hz, 2H), 1.93 (d, *J* = 15.8 Hz, 1H), 1.85 - 1.51 (m, 6H), 1.49 - 1.39 (m, 2H), 1.36 (s, 9H), 1.24 (s, 2H), 0.99 (dd, *J* = 11.7, 4.4 Hz, 1H); m/z = 452.4 [M+H]⁺.

### Method I: Oxidation

### General procedure I1:

To a solution of benzyl alcohol (1.0 eq.) in DCM (0.05M) under nitrogen atmosphere was added MnO₂ (10 eq.). The reaction mixture was stirred at rt for 24h. The reaction mixture was filtered on dicalite and washed with DCM, the volatiles were concentrated under reduced pressure to afford the desired product. The crude was engaged in the following step without further purification.

### Synthesis of 4-[2-(4-chlorothiazol-5-yl)-3-formyl-phenyl]-2-fluoro-benzonitrile (Intermediate 94)

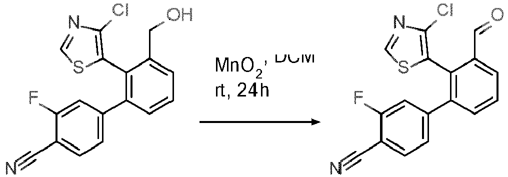

4-[2-(4-Chlorothiazol-5-yl)-3-formyl-phenyl]-2-fluoro-benzonitrile (106 mg, 94%), white solid. General procedure I1 from 4-[2-(4-chlorothiazol-5-yl)-3-(hydroxymethyl)phenyl]-2-fluoro-benzonitrile (105 mg, 0.305 mmol, 1.00 eq.).

¹H NMR (DMSO-*d*₆, 400 MHz) *δ* 9.85 (s, 1H), 9.19 (s, 1H), 8.11 (dd, *J* = 7.0, 2.2 Hz, 1H), 7.93-7.84 (m, 3H), 7.42 (dd, *J* = 10.3, 1.6 Hz, 1H), 7.15 (dd, *J* = 8.0, 1.6 Hz, 1H); m/z = 343.2 [M+H]+.

### General procedure I2:

To a solution of benzyl alcohol (1.0 eq.) in DCM (0.1M) under nitrogen atmosphere was added Dess-Martin periodinane (1.1 eq.). The reaction mixture was stirred at rt for 24h. The reaction mixture was diluted with DCM and quenched with a saturated aqueous NaHCOs solution and a saturated aqueous Na₂S₂O₃ solution. The two layers were separated and the aqueous layer was extracted three times with DCM. The combined organic layers were dried over a phase separator and concentrated under reduced pressure. The crude was engaged in the following step without further purification.

### Synthesis of 2-fluoro-4-(3-formyl-2,5-dimethyl-phenyl)benzonitrile (Intermediate 95)

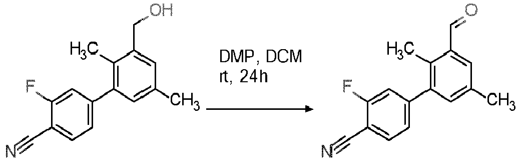

2-fluoro-4-(3-formyl-2,5-dimethyl-phenyl)benzonitrile (280 mg, 86%), white solid. General procedure I2 from 2-fluoro-4-[3-(hydroxymethyl)-2,5-dimethyl-phenyl]benzonitrile (297 mg, 1.163 mmol, 1.00 eq.).

¹H NMR (DMSO-*d*₆, 400 MHz) *δ* 10.31 (s, 1H), 8.02 (dd, *J* = 8.0, 7.0 Hz, 1H), 7.74 (d, *J* = 2.0 Hz, 1H), 7.65 - 7.54 (m, 1H), 7.43 - 7.36 (m, 2H), 2.44 (s, 3H), 2.39 (s, 3H).

### General procedure I3:

To a solution of alkene (1.0 eq.) in 1,4-dioxane (0.07M) under nitrogen atmosphere was added 2,6-dimethylpyridine (2 eq.) and tetraoxoosmium (0.02 eq.). The reaction mixture was stirred at rt for 10 min and sodium;periodate (4 eq.) was added. The reaction mixture was stirred at rt for 3h. The reaction mixture was diluted with DCM and quenched with water. The two layers were separated and the aqueous layer was extracted three times with DCM. The combined organic layers were dried over a phase separator and concentrated under reduced pressure. The crude was purified by flash chromatography column (AcOEt/cyclohexane 0 to 20%) to afford the desired product.

### Synthesis of 3-chloro-4-(difluoromethoxymethyl)benzaldehyde (Intermediate 96)

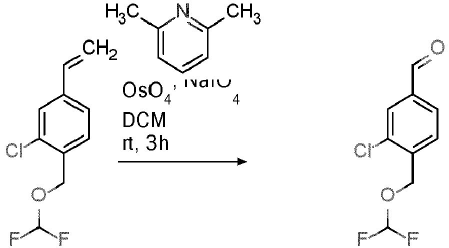

3-chloro-4-(difluoromethoxymethyl)benzaldehyde (76 mg, 72%), colorless oil. General procedure I3 from 2-chloro-1-(difluoromethoxymethyl)-4-vinyl-benzene (102 mg, 0.46 mmol, 1.00 eq.).

¹H NMR (CDCl₃, 400 MHz) *δ* 9.99 (s, 1H), 7.90 (d, *J* = 1.6 Hz, 1H), 7.82 (dd, *J* = 7.9, 1.6 Hz, 1H), 7.69 (d, *J* = 7.9 Hz, 1H), 6.41 (t, *J* = 73.5 Hz, 1H), 5.07 (s, 2H).

### Method J: Addition group

### General procedure J1:

To a solution of Ar-H (1.0 eq.) in DCM (0.1M) under nitrogen atmosphere at 0 °C was added 1-bromopyrrolidine-2,5-dione (1.2 eq.). The reaction mixture was stirred at 0 °C for 1h. The reaction mixture was diluted with DCM and quenched with water. The two layers were separated and the aqueous layer was extracted three times with DCM. The combined organic layers were dried over a phase separator and concentrated under reduced pressure. The crude was purified by flash chromatography column (AcOEt/cyclohexane 0 to 20%) to afford the desired product.

### Synthesis of 1-(2-bromo-4-{[(tert-butyldimethylsilyl)oxy]methyl}phenyl)-4-methylpiperidine (Intermediate 97)

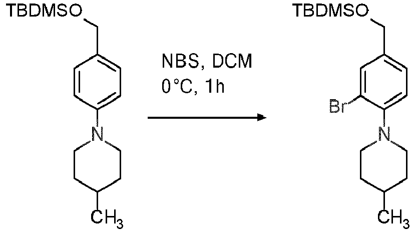

1-(2-bromo-4-{[(tert-butyldimethylsilyl)oxy]methyl}phenyl)-4-methylpiperidine (108 mg, 63%), yellow oil. General procedure J1 from 1-(4-{[(tert-butyldimethylsilyl)oxy]methyl}phenyl)-4-methylpiperidine (111 mg, 0.340 mmol, 1.00 eq.).

¹H NMR (DMSO-*d*₆, 400 MHz) *δ* 7.50 (dd, *J* = 1.9, 0.8 Hz, 1H), 7.27 - 7.22 (m, 1H), 7.12 (d, *J* = 8.2 Hz, 1H), 4.64 (d, *J* = 0.9 Hz, 2H), 3.19 (d, *J* = 11.7 Hz, 2H), 2.64 - 2.54 (m, 2H), 1.69 (t, *J* = 12.2 Hz, 3H), 1.48 (ddd, *J* = 11.0, 7.1, 3.9 Hz, 1H), 1.36 - 1.24 (m, 2H), 0.97 (d, *J* = 6.5 Hz, 3H), 0.90 (s, 9H), 0.08 (s, 6H); m/z = 398.4 [M+H]+.

### General procedure J2:

To a solution of Ar-H (1.0 eq.) in THF (0.05M) under nitrogen atmosphere at -78 °C was added a solution of 1 M [bis(trimethylsilyl)amino]lithium (1M, 2 eq.). The reaction mixture was stirred at -78 °C for 1h then *N*-(benzenesulfonyl)-*N*-fluoro-benzenesulfonamide (2 eq.) was added and the reaction mixture was allowed to warm up to room temperature and stirred for 24h. The reaction mixture was diluted with DCM and quenched with a saturated aqueous NH₄Cl solution. The two layers were separated and the aqueous layer was extracted three times with DCM. The combined organic layers were dried over a phase separator and concentrated under reduced pressure. The crude was purified by flash chromatography column (AcOEt/n-heptane 0 to 30%) to afford the desired product.

### Synthesis of 5-(2-{[(tert-butyldimethylsilyl)oxy]methyl}-6-chlorophenyl)-4-fluoro-1,3-oxazole (Intermediate 98)

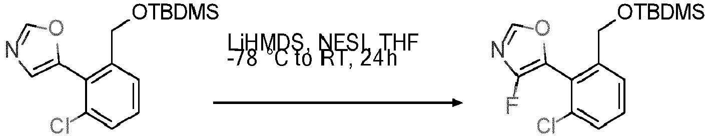

5-(2-{[(tert-butyldimethylsilyl)oxy]methyl}-6-chlorophenyl)-4-fluoro-1,3-oxazole (97 mg, 51%), yellow oil. General procedure J2.1 from 5-(2-{[(tert-butyldimethylsilyl)oxy]methyl}-6-chlorophenyl)-1,3-oxazole (180 mg, 0.550 mmol, 1.00 eq.).

¹H NMR (DMSO-*d*₆, 400 MHz) *δ* 8.53 (d, *J* = 2.1 Hz, 1H), 7.63 - 7.55 (m, 3H), 4.59 (s, 2H), 0.83 (s, 9H), -0.01 (s, 6H); m/z = 342.3 [M+H]⁺.

### General procedure J3:

To a solution of Ar-H (1.0 eq.) in DMF (0.05M) under argon atmosphere at room temperature was added 1-chloropyrrolidine-2,5-dione (1 eq.). The reaction mixture was stirred at 100 °C for 1h. The reaction mixture was concentrated under reduced pressure. The crude was purified by flash chromatography column (AcOEt/cyclohexane 0 to 50%) to afford the desired product.

### Synthesis of 4-[2-(3-chloro-2-methyl-indazol-5-yl)-5-formyl-phenyl]-2-fluoro-benzonitrile (Intermediate 99)

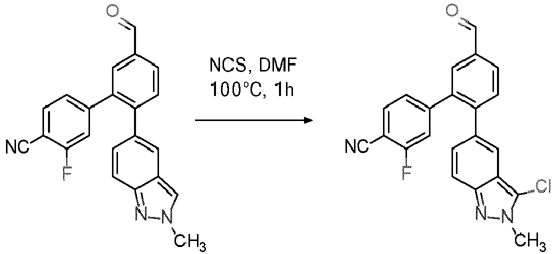

4-[2-(3-chloro-2-methyl-indazol-5-yl)-5-formyl-phenyl]-2-fluoro-benzonitrile (62 mg, 73%), off-white solid. General procedure J3 from 2-fluoro-4-[5-formyl-2-(2-methylindazol-5-yl)phenyl]benzonitrile (67 mg, 0.189 mmol, 1.00 eq.).

¹H NMR (CDCl₃, 400 MHz) *δ* 10.12 (s, 1H), 8.01 (dd, *J* = 7.9, 1.7 Hz, 1H), 7.92 (d, *J* = 1.6 Hz, 1H), 7.72 (d, *J* = 7.9 Hz, 1H), 7.50 - 7.43 (m, 4H), 7.11 - 7.05 (m, 2H), 6.90 (dd, *J* = 9.0, 1.7 Hz, 1H), 4.18 (s, 3H).

; m/z = 390.3 [M+H]+.

### Method K: CH activation

### General procedure K1:

To a solution of Ar-Br (1.00 eq.) in DMA (0.2M) were added Ar-H (1.30 eq.), K₂CO₃ (2.00 eq.), Pivalic acid (0.40 eq.) and PCy₃.HBF₄ (0.15 eq.). The reaction mixture was degassed for 5 min then Pd(OAc)₂ (0.10 eq.) was added. The reaction mixture was stirred at 110 °C for 1-24h. The reaction mixture was diluted with AcOEt and quenched with water. The two layers were separated and the aqueous layer was extracted three times with AcOEt. The combined organic layers were dried over a phase separator and concentrated under reduced pressure. When necessary, the crude was purified by flash chromatography column (AcOEt/cyclohexane 0 to 100%) to afford the desired product.

### Synthesis of 3-chloro-2-(4-chlorothiazol-5-yl)benzaldehyde (Intermediate 100)

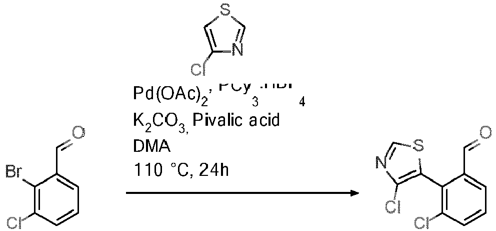

3-chloro-2-(4-chlorothiazol-5-yl)benzaldehyde (570 mg, 38%), yellow solid. General procedure K1 from 2-bromo-3-chlorobenzaldehyde (1.0 g, 4.47 mmol, 1.00 eq.) and 4-chloro-1,3-thiazole (509 µL, 5.81 mmol, 1.30 eq.).

¹H NMR (DMSO-*d₆*,400 MHz,) *δ* 9.77 (d, *J* = 0.6 Hz, 1H), 9.38 (s, 1H), 9.32 (s, 1H), 7.99 (ddd, *J* = 7.8, 5.6, 1.3 Hz, 2H), 7.79 (td, *J* = 7.9, 0.6 Hz, 1H); m/z = 258.0 [M+H]+.

### General procedure K2:

To a solution of Ar-Br (1.00 eq.) in DMA (0.2M) were added Ar-H (1.02 eq.), K₂CO₃ (2.64 eq.), Pivalic acid (0.38 eq.) and Me₄*t*ButylXphos (0.12 eq.). The reaction mixture was degassed for 5 min then Pd(OAc)₂ (0.10 eq.) was added. The reaction mixture was stirred at 110 °C for 1-24h. The reaction mixture was filtered and concentrated under reduced pressure. The crude was purified by flash chromatography column (EtOAc/cyclohexane 20 to 80%) to afford the desired product.

### Synthesis of 3-chloro-6-fluoro-2-oxazol-5-yl-benzaldehyde (Intermediate 101)

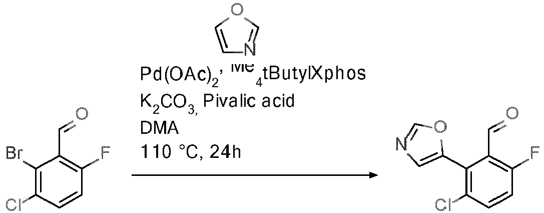

3-chloro-6-fluoro-2-oxazol-5-yl-benzaldehyde (66 mg, 37%), orange oil. General procedure K1 from 2-bromo-3-chloro-6-fluorobenzaldehyde (130 mg, 0.52 mmol, 1.00 eq.) and 1,3-oxazole (35 µL, 0.53 mmol, 1.02 eq.).

¹H NMR (DMSO-*d₆*,400 MHz,) *δ* 9.91 (s, 1H), 8.63 (s, 1H), 7.99 (dd, *J* = 9.0, 4.9 Hz, 1H), 7.64 (dd, *J* = 10.1, 9.0 Hz, 1H), 7.53 (s, 1H); m/z = 226.0 [M+H]+.

### General procedure K3:

To a solution of Ar-Br (1.00 eq.) in toluene (0.2M) were added Ar-H (2.00 eq.), K₂CO₃ (3.00 eq.), Pivalic acid (0.40 eq.) and cataCXium^{®} (0.10 eq.). The reaction mixture was degassed for 5 min then Pd(OAc)₂ (0.05 eq.) was added. The reaction mixture was stirred at 110 °C for 1-24h. The reaction mixture was diluted with DCM and quenched with water. The two layers were separated and the aqueous layer was extracted three times with DCM. The combined organic layers were dried over a phase separator and concentrated under reduced pressure. When necessary, the crude was purified by flash chromatography column (AcOEt/cyclohexane 0 to 30%) to afford the desired product

### Synthesis of tert-butyl N-[1-(3-chloro-2-isothiazol-5-yl-phenyl)-4-piperidyl]carbamate (Intermediate 102)

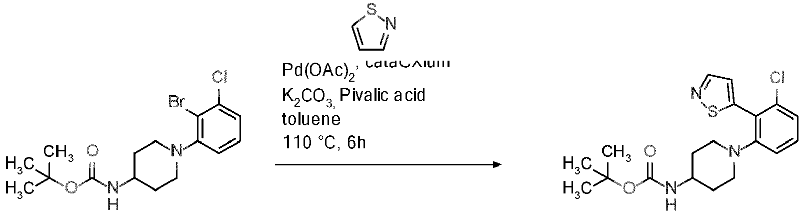

tert-butyl N-[1-(3-chloro-2-isothiazol-5-yl-phenyl)-4-piperidyl]carbamate (100 mg, 55%), yellow solid. General procedure K3 from tert-butyl N-[1-(2-bromo-3-chloro-phenyl)-4-piperidyl]carbamate (200 mg, 0.48 mmol, 1.00 eq.) and 1,2-thiazole (84 mg, 0.96 mmol, 2.00 eq.).

¹H NMR (DMSO-*d*₆, 400 MHz,) *δ* 8.63 (d, *J* = 1.8 Hz, 1H), 7.72 (d, *J* = 1.8 Hz, 1H), 7.40 (t, *J* = 8.0 Hz, 1H), 7.32 (dd, *J* = 8.1, 1.2 Hz, 1H), 7.24 (dd, *J* = 8.0, 1.3 Hz, 1H), 6.84 (d, *J* = 7.6 Hz, 1H), 2.94 (d, *J* = 12.1 Hz, 2H), 2.63 (t, *J* = 11.2 Hz, 2H), 1.65 (d, *J* = 12.5 Hz, 2H), 1.37 (s, 10H); m/z = 394.2 [M+H]⁺.

### Method L: Protection

### General procedure L1:

To a solution of benzyl alcohol (1.00 eq.) in DMA (0.1M) under nitrogen atmosphere were added TBDMSCI (1.20 eq.) and imidazole (2.00 eq.). The reaction mixture was stirred at rt for 24h. The reaction mixture was diluted with AcOEt. The organic layer was washed with a saturated aqueous Na₂CO₃ solution then brine. The two layers were separated the organic layer was dried over a phase separator and concentrated under reduced pressure. The crude was purified by flash chromatography column (EtOAc/cyclohexane 0 to 50%) to afford the desired product.

### Synthesis of tert-butyl-[[3-chloro-2-(4-chlorothiazol-5-yl)phenyl]methoxy]-dimethyl-silane (Intermediate 103)

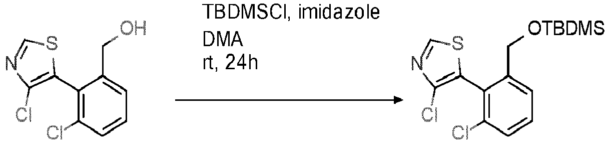

*tert*-butyl-[[3-chloro-2-(4-chlorothiazol-5-yl)phenyl]methoxy]-dimethyl-silane (389 mg, 35%), yellow oil. General procedure L1 from [3-chloro-2-(4-chlorothiazol-5-yl)phenyl]methanol (720 mg, 2.77 mmol, 1.00 eq.).

¹H NMR (DMSO-*d*₆, 400 MHz) *δ* 9.33 (s, 1H), 7.57-7.55 (m, 3H), 4.44 (d, *J* = 1.7 Hz, 3H), 0.84 (s, 9H), -0.02 (d, *J* = 1.7 Hz, 6H); m/z = 374.2 [M+H]+.

### General procedure L2:

To a solution of amine (1.00 eq.) in H₂O (0.1M) was added CbzCl (1.50 eq.). NaHCOs (3.00 eq.) was added by portion and the reaction mixture was stirred at rt for 24h. The reaction mixture was diluted with Et₂O and quenched with a saturated aqueous NaHCO₃ solution. The aqueous layer (pH=8-9) was washed twice with Et2O. Then, the aqueous layer was acidified to pH=3-4 by dropwise addition of HCl 37%, and was extracted three times with Et₂O. The combined organic layers were dried over a phase separator and concentrated under reduced pressure. The crude was engaged in the following step without further purification.

### Synthesis of 2-benzyloxycarbonyl-2-azabicyclo[2.2.1]heptane-4-carboxylic acid (Intermediate 104)

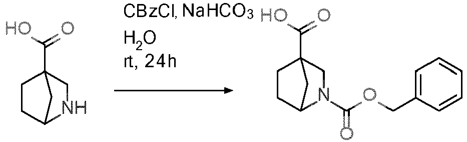

2-Benzyloxycarbonyl-2-azabicyclo[2.2.1]heptane-4-carboxylic acid (1.27 g, 80%), white solid. General procedure L2 from 2-azabicyclo[2.2.1]heptane-4-carboxylic acid;hydrochloride (1.00 g, 5.63 mmol, 1.00 eq.).

¹H NMR (DMSO-*d*₆, 400 MHz) *δ* 12.62 (s, 1H), 7.39-7.29 (m, 5H), 5.08-5.00 (m, 2H), 4.21 (d, *J* = 12.8 Hz, 1H), 3.45 (ddd, *J* = 24.2, 9.4, 3.0 Hz, 1H), 3.19 (dd, *J* = 23.6, 8.3 Hz, 1H), 1.97 (tt, *J* = 11.1, 3.4 Hz, 1H), 1.86-1.76 (m, 2H), 1.69-1.61 (m, 3H); m/z = 276.2 [M+H]+.

### Method M: Ullmann coupling

### General procedure M1:

To a solution of methananamine in water (2M, 5 eq.) was added Ar-Br (1.00 eq.) and copper (0.10 eq.). The reaction mixture was heated at 95°C for 24h. The reaction mixture was cooled to rt and diluted with. The two layers were separated and the aqueous layer was extracted three times with EtOAc. The combined organic layers were dried over a phase separator and concentrated under reduced pressure. The crude was engaged in the following step without further purification.

### Synthesis of 2-chloro-N-methyl-4-[(4-methylpiperazin-1-yl)methyl]aniline (Intermediate 105)

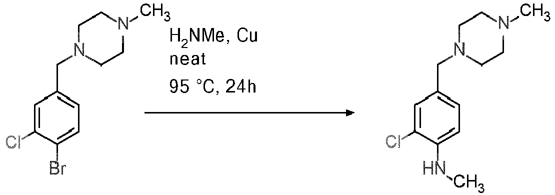

2-chloro-N-methyl-4-[(4-methylpiperazin-1-yl)methyl]aniline (35 mg, 81%), pale yellow oil. General procedure M1 from 1-[(4-bromo-3-chloro-phenyl)methyl]-4-methyl-piperazine (50 mg, 0.16 mmol, 1.00 eq.).

¹H NMR (CDCl₃, 400 MHz) *δ* 7.22 (d, *J* = 2.1 Hz, 1H), 7.09 (dd, *J* = 8.2, 2.0 Hz, 1H), 6.58 (d, *J* = 8.2 Hz, 1H), 4.27 (d, *J* = 7.1 Hz, 1H), 3.38 (s, 2H), 2.89 (d, *J* = 5.1 Hz, 3H), 2.44 (br s, 7H), 2.28 (s, 3H), 1.74 (br s, 1H); m/z = 254.3 [M+H]+.

### Method N: Stille coupling

### General procedure N1:

A solution of Ar-Br (1.00 eq.) in toluene (0.1M) was degassed with argon for 5 min and Ar-SnBus (2.00 eq.) and Pd(PPh₃)₄ (0.10 eq.) was added. The reaction mixture was heated at 120°C for 2h. The reaction mixture was cooled to rt, diluted with DCM and quenched with water. The two layers were separated and the aqueous layer was extracted three times with DCM. The combined organic layers were dried over a phase separator and concentrated under reduced pressure. The crude was purified by flash chromatography column (EtOAc/cyclohexane 1 to 50%) to afford the desired product.

### Synthesis of tert-butyl N-[rac-(1S,2S,4R)-7-[(3-chloro-4-thiazol-2-yl-phenyl)methyl]-7-azabicyclo[2.2.1]heptan-2-yl]carbamate (Intermediate 106)

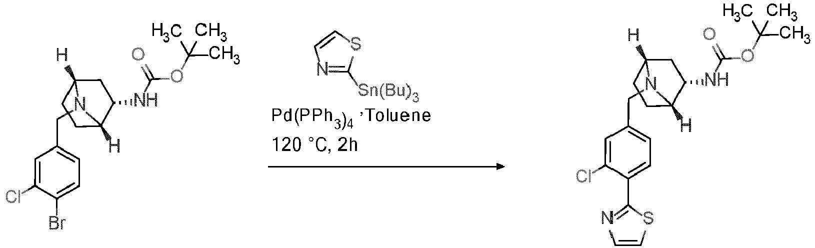

tert-butyl N-[rac-(1S,2S,4R)-7-[(3-chloro-4-thiazol-2-yl-phenyl)methyl]-7-azabicyclo[2.2.1 ]heptan-2-yl]carbamate (32 mg, 29%), pale yellow oil. General procedure N1 from tert-butyl N-[rac-(1S,2S,4R)-7-[(4-bromo-3-chloro-phenyl)methyl]-7-azabicyclo[2.2.1]heptan-2-yl]carbamate (100 mg, 0.24 mmol, 1.00 eq.) and 2-(tributylstannanyl)-1,3-thiazole (151 µL, 0.48 mmol, 2.00 eq.).

¹H NMR (DMSO-*d*₆, 400 MHz) *δ* 8.14 (d, *J* = 8.1 Hz, 1H), 8.03 (d, *J* = 3.2 Hz, 1H), 7.95 (d, *J* = 3.2 Hz, 1H), 7.60 (d, *J* = 1.6 Hz, 1H), 7.47 (dd, *J* = 8.0, 1.7 Hz, 1H), 3.79 - 3.70 (m, 1H), 3.23 (m, 1H), 3.14 (t, *J* = 4.7 Hz, 1H), 2.07 - 1.93 (m, 1H), 1.84 - 1.54 (m, 3H), 1.37 (s, 9H), 1.34 - 1.28 (m, 1H), 1.21 - 1.11 (m, 1H), 1.03 (dd, *J* = 12.1, 4.7 Hz, 1H), 0.89 (t, *J* = 7.3 Hz, 3H); m/z = 420.4 [M+H]+.

### Method O: Photoredox Cross-Electrophile Coupling

### General procedure O1:

To a solution of Ar-Br (1.00 eq.) in DME (0.01M) under nitrogen atmosphere were added R-Br (2.00 eq.), Ir[dF(CF₃)ppy]₂(dtbbpy)PF₆ (0.03 eq.), TTMSS (1.00 eq) and Na₂CO₃ (2.00 eq.). To a separate vial was added NiCl₂•glyme (0.075 eq.) and 4,4'-di-tert-butyl-2,2'-bipyridine (0.075 eq.). in DME (0.01M) under nitrogen atmosphere. The precatalyst mixture was stirred at room temperature for 15 min then 0.2 mL (0.01 eq.) of the precatalyst solution was added to the reaction mixture. The reaction mixture was stirred at 40°C for 6h under irradiation with 34 W blue LED lamp. The reaction mixture was quenched by exposure to air and concentrated under reduced pressure. The crude was purified by flash chromatography column (MeOH/DCM 0 to 10%) to afford the desired product.

### Synthesis of tert-butyl N-[rac-(1S,2S,4R)-7-[(3-chloro-4-thiazol-2-yl-phenyl)methyl]-7-azabicyclo[2.2.1]heptan-2-yl]carbamate (Intermediate 107)

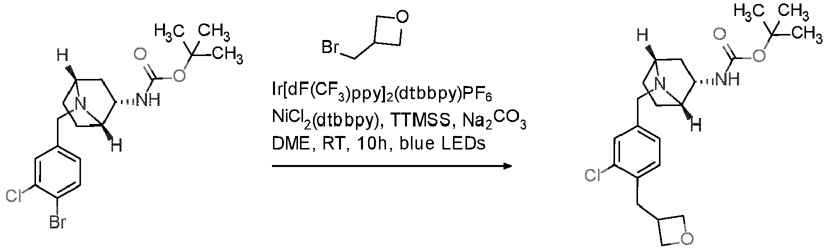

tert-butyl N-[rac-(1S,2S,4R)-7-[(3-chloro-4-thiazol-2-yl-phenyl)methyl]-7-azabicyclo[2.2.1] heptan-2-yl]carbamate (32 mg, 29%), yellow oil. General procedure O1 from tert-butyl N-[rac-(1S,2S,4R)-7-[(4-bromo-3-chloro-phenyl)methyl]-7-azabicyclo[2.2.1]heptan-2-yl]carbamate (83 mg, 0.20 mmol, 1.00 eq.) and 3-(bromomethyl)oxetane (63 mg, 0.40 mmol, 2.00 eq.).

¹H NMR (CDCl₃, 400 MHz) *δ* 7.39 (s, 1H), 7.07 (m, 2H), 4.81 (dd, *J* = 7.7, 6.0 Hz, 2H), 4.56 (br s, 1H), 4.49 (t, *J* = 6.1 Hz, 2H), 4.04 (br s, 1H), 3.66 - 3.50 (m, 3H), 3.44 - 3.31 (m, 1H), 3.11 (d, *J* = 7.8 Hz, 2H), 2.36 (br s, 1H), 1.91 (br s, 1H), 1.59 (m, 5H), 1.42 (s, 9H); m/z = 407.5 [M+H]+.

### Method P: Peptidic coupling

### General procedure P1:

To a solution of carboxylic acid (1.00 eq.) in DMF (0.1M) were added amine (1.10 eq.), NEts (5.00 eq.) and HATU (1.20 eq.). The reaction mixture was stirred at rt for 24h. The reaction mixture was diluted with AcOEt and quenched with a saturated aqueous NaHCOs solution. The two layers were separated and the aqueous layer was extracted three times with AcOEt. The combined organic layers were dried over a phase separator and concentrated under reduced pressure. When necessary, the crude was purified by flash chromatography column (MeOH/DCM 0 to 10%) to afford the desired product.

### Synthesis of (2-bromo-3-chloro-6-fluoro-phenyl)-(1-methyl-1,7-diazaspiro[3.4]octan-7-yl)methanone (Intermediate 108)

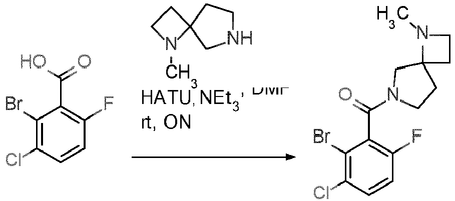

(2-bromo-3-chloro-6-fluoro-phenyl)-(1-methyl-1,7-diazaspiro[3.4]octan-7-yl)methanone (412 mg, 94%), beige foam. General procedure P1 from 2-bromo-3-chloro-6-fluorobenzoic acid (300 mg, 1.18 mmol, 1.00 eq.) and 1-methyl-1,7-diazaspiro[3.4]octane;dihydrochloride (259 mg, 1.30 mmol, 1.10 eq.). ¹H NMR (DMSO-*d*₆, 400 MHz) *δ* 7.79 (dt, J = 9.0, 5.8 Hz, 1H), 7.52-7.45 (m, 1H), 3.66-3.46 (m, 2H), 3.31 (s, 3H) NCH₃, 3.21-3.17 (m, 2H), 2.36-2.11 (m, 6H); m/z = 363.1 [M+H]⁺.

### Method Q: Cyclopropanation

### General procedure Q1:

To a solution of diethylzinc (1M, 2.00 eq.) in DCM (0.1M) under nitrogen atmosphere was added at 0 °C 2,2,2-trifluoroacetic acid (2.00 eq.). The reaction mixture was stirred for 20 min at 0 °C then diiodomethane (2.00 eq.) was added. The reaction mixture was stirred for 20 min at 0 °C then a solution of alkene (1eq.) in DCM (0.1 M) was added. The reaction mixture was stirred at rt for 24h. The reaction mixture was diluted with DCM and quenched with a 1M HCl solution. The two layers were separated and the aqueous layer was extracted three times with DCM. The combined organic layers were dried over a phase separator and concentrated under reduced pressure. The crude was purified by flash chromatography column (EtOAc/cyclohexane 0 to 20%) to afford the desired product.

### Synthesis of [3-bromo-2-(cyclopropylmethyl)phenyl]methanol (Intermediate 109)

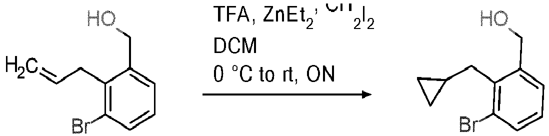

[3-bromo-2-(cyclopropylmethyl)phenyl]methanol (84 mg, 30%), colorless oil. General procedure Q1 from (2-allyl-3-bromo-phenyl)methanol (333 mg, 1.17 mmol, 1.00 eq.). ¹H NMR (DMSO-*d*₆, 400 MHz) *δ* 7.50 (dd, *J* = 8.0, 1.3 Hz, 1H), 7.44 (dd, *J* = 7.6, 1.2 Hz, 1H), 7.19 - 7.12 (m, 1H), 5.26 (t, *J* = 5.5 Hz, 1H), 4.59 (d, *J* = 5.4 Hz, 2H), 2.78 (d, *J* = 6.4 Hz, 2H), 1.07 - 0.95 (m, 1H), 0.49 - 0.38 (m, 2H), 0.30 - 0.22 (m, 2H).

### Method S: Bouveault-type reaction

### General procedure S1:

To a solution of Ar-Br (1.00 eq.) in THF (0.1M) under nitrogen atmosphere was added at -78 °C was added butyllithium (1.6M, 1.30 eq.) and N,N-dimethylformamide (1.50 eq.). The reaction mixture was stirred at room temperature for 2h. The reaction mixture was diluted with DCM and quenched with a saturated aqueous NH₄Cl solution. The two layers were separated and the aqueous layer was extracted three times with DCM. The combined organic layers were dried over a phase separator and concentrated under reduced pressure. The crude was purified by flash chromatography column (EtOAc/heptane 0 to 20%) to afford the desired product.

### Synthesis of 3-chloro-2-(difluoromethyl)benzaldehyde (Intermediate 110)

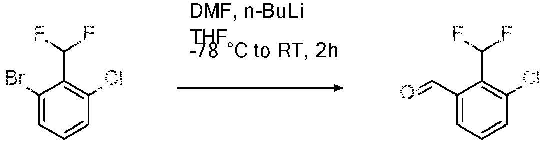

3-chloro-2-(difluoromethyl)benzaldehyde (63 mg, 37%), pale yellow oil. General procedure S1 from 1-bromo-3-chloro-2-(difluoromethyl)benzene (200 mg, 0.82 mmol, 1.00 eq.).

¹H NMR (CDCl₃, 400 MHz) *δ* 10.35 (t, *J* = 0.6 Hz, 1H), 7.97 - 7.91 (m, 2H), 7.82 - 7.77 (m, 1H), CHF₂ signal not clear.

### Sequence example used in Series 3 general procedure

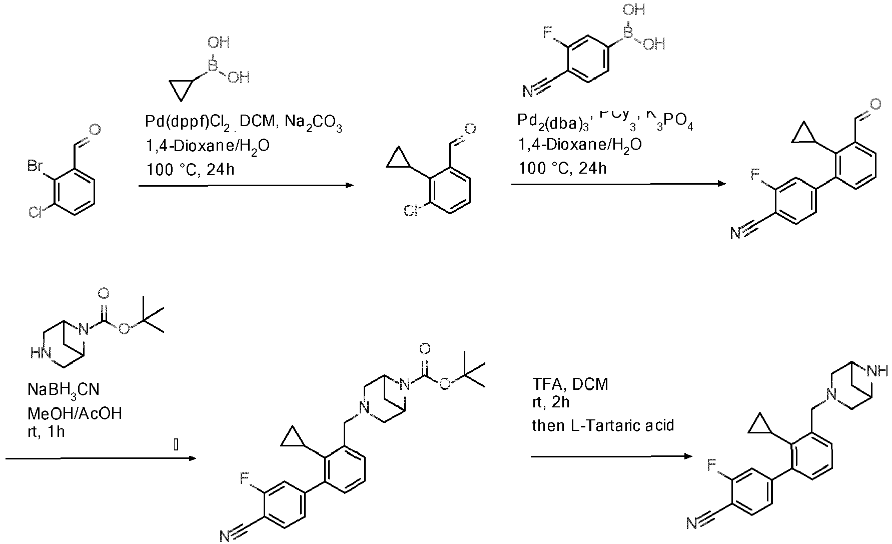

### A1.1+A2.2+B1+C1 (Synthesis of Example 323)

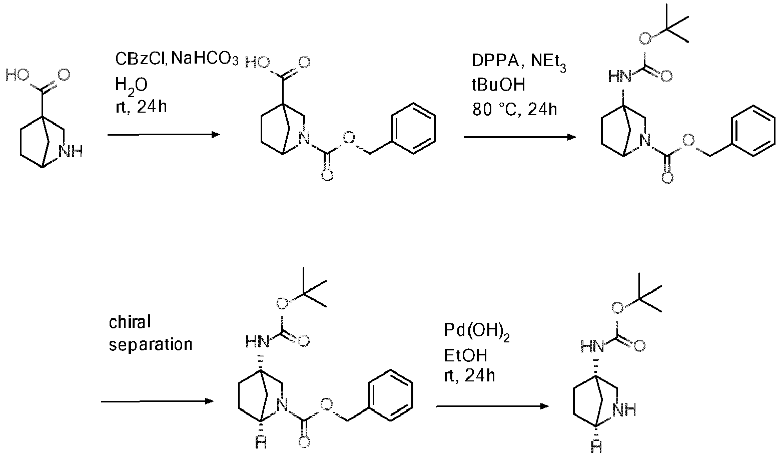

### L2+G1+C4

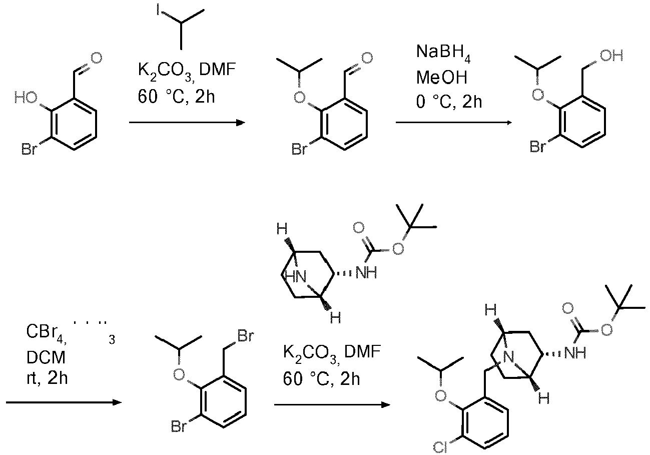

### D2.1+E1+F1+D3

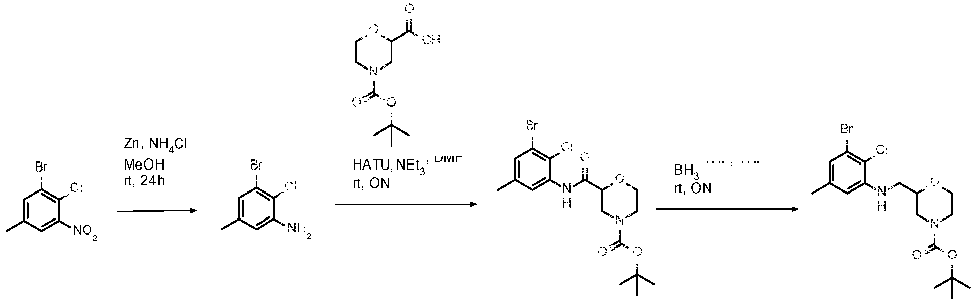

### E5+P1+E4

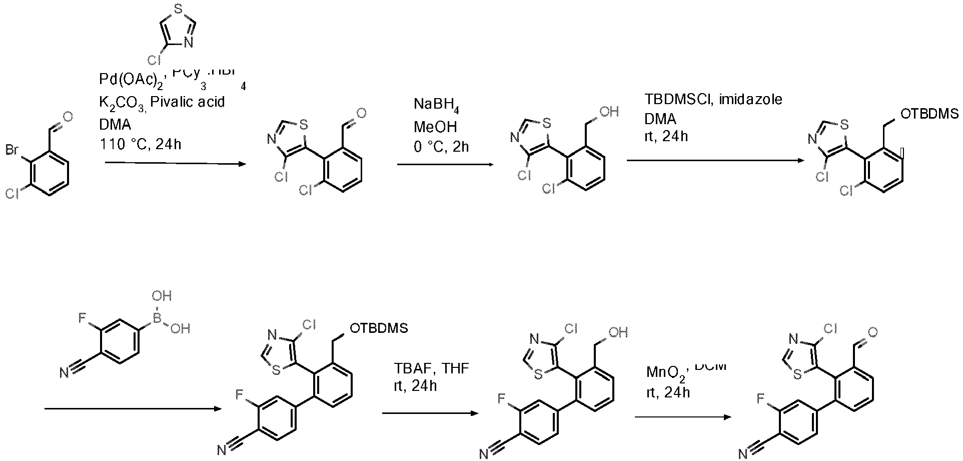

### K1+E1+L1+A2.2+C3+I1

### Biochemical Assays

### LSD1 inhibition assay

LSD1 biochemical activity was measured using the HTRF^{®} LSD1 Histone H3K4 mono-demethylation assay (me1->me0). Briefly, the enzymatic buffer was prepared by addition of 50mM Tris-HCl (Sigma cat# T2663-IL), 50 mM NaCl (ThermoFisher cat# AM9759) and 0.01 % Tween 20 (ThermoFisher cat# J20605.AP) in distilled water (final pH 8.5). This buffer was supplemented with 10 µM FAD (Fisher Scientific cat#11411838) and 1 mM DTT (Sigma cat# D0632). For the assay, H3K4(me1) biotinylated peptide substrate (Anaspec, cat# 64355) was added to provide a final assay concentration of 95 nM. The streptavidin-XL665 (Cisbio cat # 610SAXLA) and H3K4 me0-Eu(K) antibody-cryptate (Cisbio, cat# 61KA0KAD) detection reagents were mixed according to the manufacturer's instructions. Test compounds were diluted in DMSO in a series of 10 semi-log step doses; 10 nL of each compound dose were dispensed in 384 well plates. Recombinant human LSD1 was diluted into enzymatic buffer to provide a final assay concentration of 20nM and added to the tested compounds for 15 minutes. The reaction was started by addition of the substrate and incubated at 25°C for 60 minutes. The detection reagents were added, and the plate was incubated in the dark for 90 minutes. Fluorescence was measured on an Envision 2103 plate reader with optical setup for excitation at 665 nM and emission at 620 nm in the HTRF mode. The ratio of acceptor and donor emission signals was calculated for each well. Percent inhibition values were calculated from the HTRF ratios at different doses and fitted to a 4-parameter logistic curve to determine IC50 values. Results for compounds of the disclosure in this assay are shown in Table 4 below.

Data were reported as % inhibition for each concentration tested, and IC50 values were estimated using QPatch software. At least two cells were tested, and even more if results diverged. Results for compounds of the disclosure in this assay are shown in Table 4 below.

**Table 4: Biochemical assay data (nM range: A ≤ 10 nM; 10 nM < B ≤ 25 nM; 25 nM < C ≤ 100 nM; 100 nM < D ≤ 1,000 nM; E > 1,000 nM)**

| **Compound Reference (Example No.)** | **LSD1 inhibition IC50 (nM range)** | **Compound Reference (Example No.)** | **LSD1 inhibition IC50 (nM range)** |
|---|---|---|---|
| 1 | B | 176 | C |
| 2 | B | 177 | A |
| 3 | B | 178 | B |
| 4 | A | 179 | B |
| 5 | B | 180 | C |
| 6 | B | 181 | B |
| 7 | B | 182 | A |
| 8 | A | 183 | A |
| 9 | B | 184 | C |
| 10 | B | 185 | A |
| 11 | B | 186 | B |
| 12 | B | 187 | B |
| 13 | B | 188 | B |
| 14 | B | 189 | B |
| 15 | A | 190 | A |
| 16 | B | 191 | C |
| 17 | B | 192 | A |
| 18 | B | 193 | B |
| 19 | B | 194 | A |
| 20 | A | 195 | B |
| 21 | D | 196 | C |
| 22 | B | 197 | A |
| 23 | B | 198 | B |
| 24 | B | 199 | B |
| 25 | B | 200 | C |
| 26 | A | 201 | B |
| 27 | C | 202 | C |
| 28 | A | 203 | C |
| 29 | B | 204 | B |
| 30 | B | 205 | C |
| 31 | C | 206 | B |
| 32 | C | 207 | A |
| 33 | C | 208 | C |
| 34 | B | 209 | B |
| 35 | C | 210 | B |
| 36 | B | 211 | C |
| 37 | B | 212 | C |
| 38 | B | 213 | C |
| 39 | B | 214 | E |
| 40 | D | 215 | C |
| 41 | D | 216 | A |
| 42 | D | 217 | B |
| 43 | C | 218 | B |
| 44 | D | 219 | B |
| 45 | D | 220 | C |
| 46 | C | 221 | C |
| 47 | C | 222 | B |
| 48 | B | 223 | B |
| 49 | C | 224 | B |
| 50 | D | 225 | D |
| 51 | C | 226 | D |
| 52 | D | 227 | C |
| 53 | B | 228 | D |
| 54 | D | 229 | C |
| 55 | D | 230 | B |
| 56 | C | 231 | B |
| 57 | B | 232 | B |
| 58 | D | 233 | B |
| 59 | D | 234 | A |
| 60 | C | 235 | B |
| 61 | D | 236 | C |
| 62 | D | 237 | B |
| 63 | D | 238 | D |
| 64 | C | 239 | C |
| 65 | E | 240 | C |
| 66 | D | 241 | D |
| 67 | C | 242 | B |
| 68 | C | 243 | C |
| 69 | D | 244 | C |
| 70 | C | 245 | C |
| 71 | A | 246 | D |
| 72 | D | 247 | C |
| 73 | C | 248 | D |
| 74 | B | 249 | C |
| 75 | D | 250 | B |
| 76 | D | 251 | C |
| 77 | D | 252 | C |
| 78 | D | 253 | C |
| 79 | B | 254 | B |
| 80 | D | 255 | B |
| 81 | B | 256 | C |
| 82 | C | 257 | B |
| 83 | C | 258 | C |
| 84 | E | 259 | B |
| 85 | D | 260 | B |
| 86 | D | 261 | D |
| 87 | E | 262 | B |
| 88 | D | 263 | A |
| 89 | E | 264 | B |
| 90 | E | 265 | C |
| 91 | C | 266 | D |
| 92 | D | 267 | D |
| 93 | C | 268 | B |
| 94 | C | 269 | D |
| 95 | C | 270 | D |
| 96 | D | 271 | D |
| 97 | E | 272 | D |
| 98 | D | 273 | C |
| 99 | D | 274 | D |
| 100 | C | 275 | B |
| 101 | D | 276 | D |
| 102 | C | 277 | C |
| 103 | B | 278 | D |
| 104 | C | 279 | B |
| 105 | D | 280 | D |
| 106 | D | 281 | E |
| 107 | D | 282 | D |
| 108 | A | 283 | D |
| 109 | A | 284 | D |
| 110 | B | 285 | D |
| 111 | C | 286 | E |
| 112 | B | 287 | E |
| 113 | D | 288 | D |
| 114 | D | 289 | D |
| 115 | B | 290 | E |
| 116 | B | 291 | D |
| 117 | E | 292 | E |
| 118 | D | 293 | D |
| 119 | D | 294 | D |
| 120 | D | 295 | E |
| 121 | D | 296 | E |
| 122 | D | 297 | D |
| 123 | E | 298 | D |
| 124 | D | 299 | C |
| 125 | D | 300 | D |
| 126 | D | 301 | C |
| 127 | D | 302 | A |
| 128 | C | 303 | A |
| 129 | D | 304 | B |
| 130 | E | 305 | C |
| 131 | E | 306 | D |
| 132 | E | 307 | D |
| 133 | D | 308 | C |
| 134 | E | 309 | B |
| 135 | E | 310 | B |
| 136 | E | 311 | B |
| 137 | D | 312 | B |
| 138 | C | 313 | B |
| 139 | D | 314 | B |
| 140 | E | 315 | A |
| 141 | D | 316 | B |
| 142 | E | 317 | B |
| 143 | D | 318 | C |
| 144 | E | 319 | C |
| 145 | E | 320 | D |
| 146 | E | 321 | B |
| 147 | D | 322 | C |
| 148 | D | 323 | B |
| 149 | D | 324 | B |
| 150 | E | 325 | B |
| 151 | D | 326 | E |
| 152 | D | 327 | E |
| 153 | C | 328 | D |
| 154 | B | 329 | E |
| 155 | D | 330 | E |
| 156 | C | 331 | E |
| 157 | E | 332 | D |
| 158 | E | 333 | B |
| 159 | E | 334 | B |
| 160 | E | 335 | A |
| 161 | E | 336 | B |
| 162 | E | 337 | E |
| 163 | D | 338 | E |
| 164 | D | 339 | C |
| 165 | D | 340 | D |
| 166 | D | 341 | D |
| 167 | E | 342 | D |
| 168 | D | 343 | E |
| 169 | B | 344 | E |
| 170 | B | 345 | E |
| 171 | B | 346 | D |
| 172 | B | 347 | D |
| 173 | A | 348 | D |
| 174 | B | 349 | D |
| 175 | B | | |

Some particular expressions of the inventive concept are set out in the following numbered clauses.

### CLAUSES

1. A compound of formula (I), or a pharmaceutically acceptable salt, solvate, prodrug, or pharmaceutically active metabolite thereof: 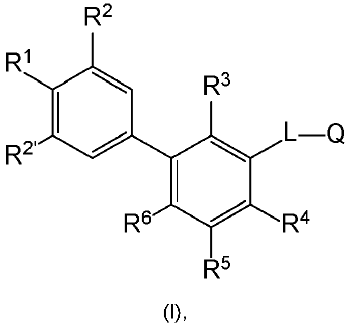 wherein
   R¹ is nitro or cyano;
   R² and R^{2'} are each independently selected from C₁₋₃ alkyl, hydrogen or halogen
   R³ is selected from hydrogen, C₁₋₃ alkyl, C₁₋₃ haloalkyl, C₃-C₆ cycloalkyl, C₃-C₆ cycloalkenyl, C₃-C₆ heterocycloalkyl, C₄-C₅ heterocycloalkenyl, C₃-C₅ heterocycloalkyl C₁-C₃ alkyl, C₅-C₆ aryl, C₃-C₆ heteroaryl, C₁₋C₄ alkoxyl, C₃-C₆ cycloalkyl-C₁-C₃ alkoxyl, C₁₋C₄ haloalkoxyl, C₁-C₃ haloalkyl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, cyano, wherein each alkyl, cycloalkyl, cycloalkenyl, heterocycloalkyl, heterocycloalkenyl, heterocycloalkyl alkyl, aryl, heteroaryl, alkoxyl, cycloalkyl alkoxyl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, amino;
   R⁴ is selected from hydrogen or halogen;
   R⁵ is selected from the group consisting of hydrogen, C₁₋₃ alkyl, C₃-C₆ cycloalkyl, C₃-C₆ cycloalkenyl, C₃-C₆ aryl, C₃-C₆ heteroaryl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, wherein each alkyl, cycloalkyl, cycloalkenyl, aryl, heteroaryl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, or amino;
   R⁶ is selected from the group consisting of hydrogen, C₁₋C₄ alkoxyl, C₁₋C₄ alkoxyl, amino, alkylamino, aminoalkyl, dialkylamino, C₈₋C₁₁ spirocycloalkyl, C₅-C₁₀ heterospirocycloalkyl, C₅-C₁₁ monocyclic or bicyclic aryl, monocyclic or bicyclic heteroaryl, C₂-C₆ cyclic amine, C₂-C₅ heterocyclic amine, C₃-C₆ unsaturated or aromatic cyclic amine, C₃-C₅ unsaturated or aromatic heterocyclic amine, C₇₋C₁₀ spirocyclic amine, or C₄₋C₉ heterospirocyclic amine, wherein each is optionally substituted with 1 to 3 substituents selected from the group consisting of hydroxyl, amino, C₁-C₆ alkyl, C₁-C₆ alkoxyl, halogen, C₁-C₃ haloalkyl, optionally substituted C₃-C₆ cycloalkyl, optionally substituted C₂-C₅ heterocycloalkyl, or optionally substituted C₃-C₆ heteroaryl, wherein said optionally substituted cycloalkyl, heterocycloalkyl and heteroaryl may be substituted with 1 or 2 substituents selected from the group consisting of C₁-C₃ alkyl, C₁-C₃ alkoxyl, hydroxyl, halogen or amine;
   L is a linker selected from the group consisting of a bond, -CH₂-, -CH₂-CH₂-, -CH(CH₃)-, -C(CH₃)₂-, -C=C-, -CH₂-O-, -C(O)-, -O-, -O-CH₂-, -NH-CH₂-, -N(CH₃)-CH₂-, --NH-C(O)-, -N(CH₃)-C(O)-, -CH₂-NH-CH₂-
   Q is a monocyclic or bicyclic ring system selected from the group consisting of C₅-C₉ cycloalkyl, C₄-C₈ heterocycloalkyl, C₈₋C₁₁ spirocycloalkyl, C₅-C₁₀ heterospirocycloalkyl, C₃-C₈ cyclic amine, C₃-C₈ heterocyclic amine, C₇₋C₁₀ spirocyclic amine, or C₄₋C₉ heterospirocyclic amine, wherein each cycloalkyl or heterocycloalkyl group may be saturated or unsaturated, said bicyclic ring may be a fused or bridged bicycle, and wherein Q is optionally substituted with 1 to 3 R⁷, wherein each R⁷ is independently selected from hydrogen, C₁-C₆ alkyl, C₁-C₆ haloalkyl, C₁-C₆ alkylcarbonyl, hydroxyl, oxo, halogen, C₁-C₆ alkylamine or amino.
2. The compound of formula (I) or a pharmaceutically acceptable salt, solvate, prodrug, or pharmaceutically active metabolite thereof according to Clause 1, wherein each heterocyclic or heteroaryl substituent may include from 1 to 4 heteroatoms independently selected from O, S and N.
3. The compound of formula (I) or a pharmaceutically acceptable salt, solvate, prodrug, or pharmaceutically active metabolite thereof according to Clause 1 or Clause 2, wherein each heterocyclic or heteroaryl substituent is connected to the remainder of the compound through a carbon atom.
4. The compound of formula (I) or a pharmaceutically acceptable salt, solvate, prodrug, or pharmaceutically active metabolite thereof according to Clause 1 or Clause 2, wherein the heterocyclic or heteroaryl substituent is connected to the remainder of the compound through a heteroatom.
5. The compound of formula (I) or a pharmaceutically acceptable salt, solvate, prodrug, or pharmaceutically active metabolite thereof according to Clause 1 or Clause 2, wherein the heterocyclic or heteroaryl substituent comprises at least one N atom, and said heterocyclic or heteroaryl substituent is connected to the remainder of the compound through an N atom.
6. The compound of formula (I) or a pharmaceutically acceptable salt, solvate, prodrug, or pharmaceutically active metabolite thereof according to any of Clauses 1 to 5, wherein:
   (i) one of R² and R^{2'} is hydrogen and the other is F;
   (ii) one of R² and R^{2'} is hydrogen and the other is CI; or
   (iii) both R² and R^{2'} are hydrogen.
7. The compound of formula (I) or a pharmaceutically acceptable salt, solvate, prodrug, or pharmaceutically active metabolite thereof according to any of Clauses 1 to 6, wherein R³ is selected from hydrogen, halogen, C₃-C₆ cycloalkyl, C₃-C₆ heterocycloalkyl, C₅-C₆ aryl, C₃-C₆ heteroaryl, or C₂-C₅ heterocyclic amine.
8. The compound of formula (I) or a pharmaceutically acceptable salt, solvate, prodrug, or pharmaceutically active metabolite thereof according to Clause 7, wherein:
   (i) R³ is hydrogen or CI;
   (ii) R³ is an optionally substituted 5 or 6 membered heteroaryl;
   (iii) R³ is an optionally substituted 4 to 6 membered heterocycloalkyl; or
   (iv) R³ is an optionally substituted 3 to 5 membered cycloalkyl;
      wherein each hetero moiety may include 1 or 2 heteroatoms independently selected from O, S and N; and
      wherein said optional substituents are independently selected from 1 or 2 substituents of the group consisting of methyl, F and Cl.
9. The compound of formula (I) or a pharmaceutically acceptable salt, solvate, prodrug, or pharmaceutically active metabolite thereof according to any of Clauses 1 to 8, wherein:
   (i) R⁴ is hydrogen;
   (ii) R⁴ is F and R⁵ and R⁶ are each hydrogen;
   (iii) R⁴ is F and R⁵ and R⁶ are each hydrogen; or
   (iii) R⁴, R⁵ and R⁶ are all hydrogen.
10. The compound of formula (I) or a pharmaceutically acceptable salt, solvate, prodrug, or pharmaceutically active metabolite thereof according to any of Clauses 1 to 9, wherein:
   (i) R⁵ is hydrogen;
   (ii) R⁵ is F;
   (iii) R⁵ is a 4 or 5 membered heterocycloalkyl;
   (iv) R⁵ is C₁-C₃ alkyl;
   (v) R⁵ is 3, 4 or 5 membered cycloalkyl
   (vi) R⁵ is optionally substituted C₃-C₆ cycloalkyl, C₃-C₆ cycloalkenyl, C₃-C₆ heterocycloalkyl, C₃-C₆ aryl or C₃-C₆ heteroaryl.
11. The compound of formula (I) or a pharmaceutically acceptable salt, solvate, prodrug, or pharmaceutically active metabolite thereof according to any of Clauses 1 to 10, wherein R⁵ is selected from hydrogen, F, methyl or pyrrolidine.
12. The compound of formula (I) or a pharmaceutically acceptable salt, solvate, prodrug, or pharmaceutically active metabolite thereof according to any of Clauses 1 to 11, wherein:
   (i) R⁶ is hydrogen;
   (ii) R⁶ is F or CI;
   (iii) R⁶ is an optionally substituted 4 or 5 membered heterocycloalkyl;
   (iv) R⁶ is an optionally substituted 8, 9 or 10 membered bicyclic or spirocyclic heterocycloalkyl;
   (v) R⁶ is an optionally substituted 5 or 6 membered heteroaryl;
   (vi) R⁶ is an optionally substituted 4, 5 or 6 membered cycloalkyl or spirocycloalkyl;
   (vii) R⁶ is a C₁-C₃ haloalkoxy; or
   (viii) R⁶ is a C₂-C₈ alkylamine or dialkylamine
      wherein each hetero moiety may include 1 or 2 heteroatoms independently selected from O, S and N; and
      wherein said optional substituents are independently selected from 1 or 2 substituents from the group consisting of C₁-C₃ alkyl, C₁-C₃ haloalkyl, C₁-C₃ alkoxyl, F, Cl and hydroxyl.
13. The compound of formula (I) or a pharmaceutically acceptable salt, solvate, prodrug, or pharmaceutically active metabolite thereof according to any of Clauses 1 to 12, wherein R⁶ is selected from hydrogen, F, methyl or pyrollidine.
14. The compound of formula (I) or a pharmaceutically acceptable salt, solvate, prodrug, or pharmaceutically active metabolite thereof according to any of Clauses 1 to 13, wherein L is selected from -CH₂- or -CH₂-CH₂-.
15. The compound of formula (I) or a pharmaceutically acceptable salt, solvate, prodrug, or pharmaceutically active metabolite thereof according to any of Clauses 1 to 14, wherein Q is selected from the group consisting of optionally substituted 5 to 7 membered heterocycloalkyl and optionally substituted 4 to 7 membered mono or fused or bridged bicycloalkylamine;
   wherein each heterocyclic moiety may include 1 or 2 heteroatoms independently selected from O and N; and
   wherein said optional substituents are independently selected from 1 to 3 substituents from the group consisting of C₁-C₃ alkyl, C₁-C₃ haloalkyl, C₁-C₃ alkoxyl, C₁-C₃ alkylamine, C₁-C₃ dialkylamine, C₁-C₃ carbonyl, amine, amino-C₁-C₃ alkyl, oxo, F, Cl and hydroxyl.
16. The compound of formula (I) or a pharmaceutically acceptable salt, solvate, prodrug, or pharmaceutically active metabolite thereof according to any of Clauses 1 to 15, wherein:
   (i) R⁷ is independently selected from C₁-C₃ alkyl, C₁-C₃ haloalkyl, hydroxyl, oxo, F, C₁-C₃ alkylamino, C₁-C₃ dialkylamino or amino;
   (ii) R⁷ is independently selected from methyl, ethyl, amine and F;
   wherein there are 1, 2 or 3 R⁷ groups.
17. The compound of formula (I) or a pharmaceutically acceptable salt, solvate, prodrug, or pharmaceutically active metabolite thereof according to any of Clauses 1 to 4, wherein:
   (i) R¹ is cyano; and/or
   (ii) R² and R^{2'} are each independently selected from hydrogen or F; and/or
   (iii) R³ is selected from hydrogen, propyl, i-propyl, CHF₂, cyclopropyl, -CH₂-cyclopropyl, -CH₂-oxetane, isothiazole, oxazole, pyridyl, methoxyl, -O-CH₂-cyclopropyl, -O-CH₂-CF₃, -O-CF₃, CF₃, Cl, amino, - N(CH₃)(CH₂-isopropyl), pyrrolidine, cyano; and/or
   (iv) R⁴ is hydrogen or F; and/or
   (v) R⁵ is selected from hydrogen, methyl, cyclopropyl, cyclopentenyl, F, amino, NH-CH₂-CH₂-N(CH₃)₂; and/or
   (vi) R⁶ is selected from hydrogen, -O-cyclobutyl, -O-CH₂-oxane, -O-CH₂-CH₂-CHF₂, -O-CH₂-CH₂-CF₃, -O-CH₂-CF₃, -CH₂₋O-CHF₂, amino, -N(CH₃)(CH-(CH₃)₂, benzimdazole, pyridine; and/or
   (vii) L is a linker selected from a bond, -CH₂-, -C≡C-, -CH₂-NH-CH₂-, and/or
   (viii) Q is C₄-C₈ heterocycloalkyl which may comprise a further heteroatom selected from N or O; or Q is morpholinyl; and wherein
   each heteroaryl, heterocycloalkyl and/or cycloalkyl group is optionally substituted.
18. The compound of formula (I) or a pharmaceutically acceptable salt, solvate, prodrug, or pharmaceutically active metabolite thereof according to Clause 17, wherein each heteroaryl, heterocycloalkyl and/or cycloalkyl group is optionally substituted with one or more groups selected from F, Cl, methyl, hydroxyl and amino.
19. The compound of formula (I) or a pharmaceutically acceptable salt, solvate, prodrug, or pharmaceutically active metabolite thereof according to any of Clauses 1 to 18, wherein R³ is selected from the group consisting of the following structures: 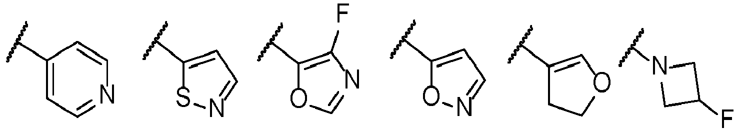 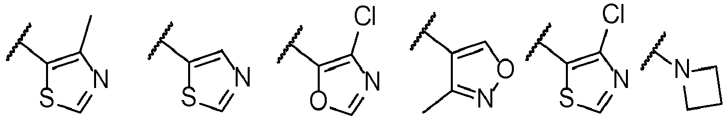 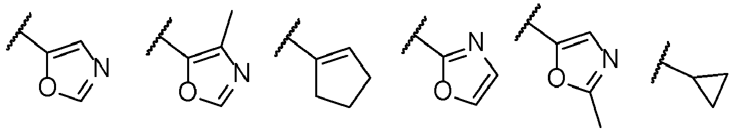 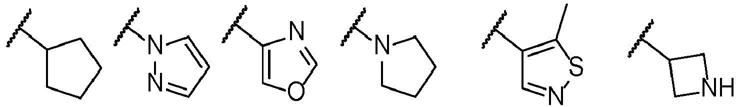 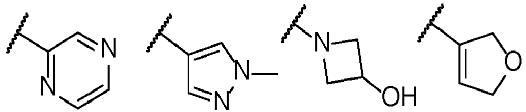 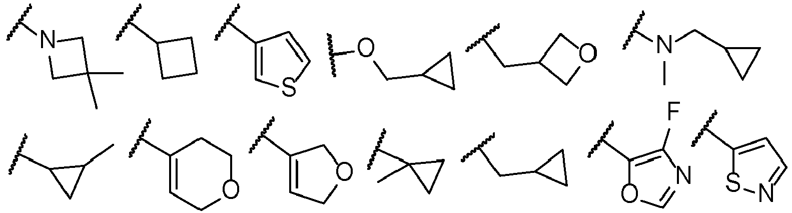
20. The compound of formula (I) or a pharmaceutically acceptable salt, solvate, prodrug, or pharmaceutically active metabolite thereof according to any of Clauses 1 to 19, wherein R³ is selected from the group consisting of the following structures: 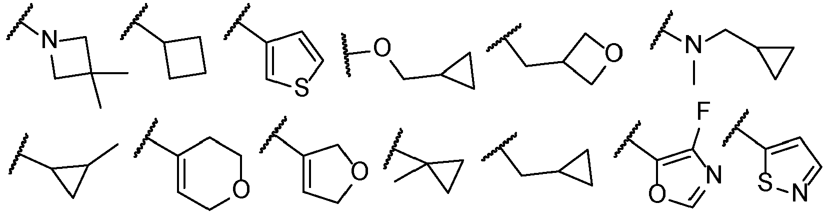
21. The compound of formula (I) or a pharmaceutically acceptable salt, solvate, prodrug, or pharmaceutically active metabolite thereof according to any of Clauses 1 to 20, wherein R⁵ is selected from the group consisting of hydrogen and the following structures: 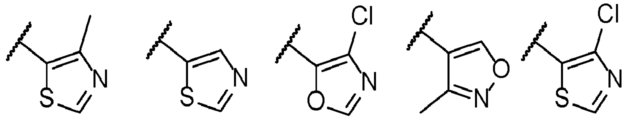 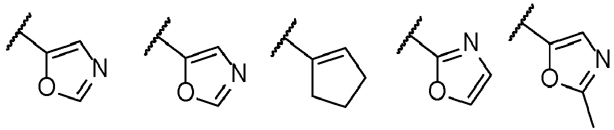 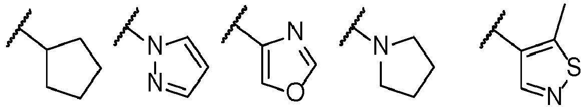 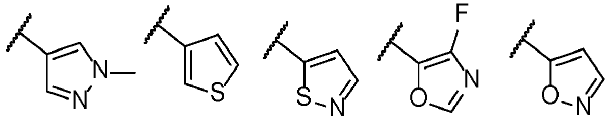
22. The compound of formula (I) or a pharmaceutically acceptable salt, solvate, prodrug, or pharmaceutically active metabolite thereof according to any of Clauses 1 to 21, wherein R⁶ is selected from the group consisting of hydrogen and the following structures: 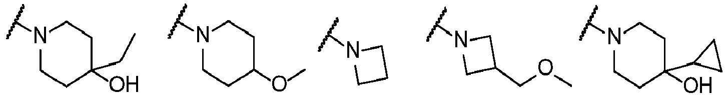 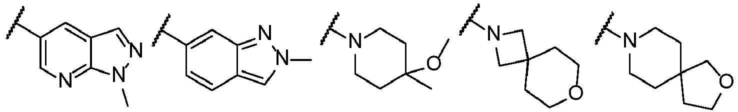 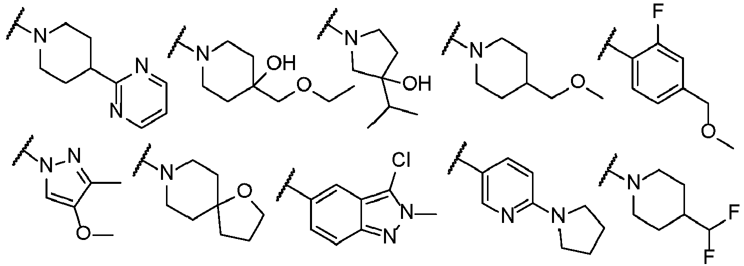 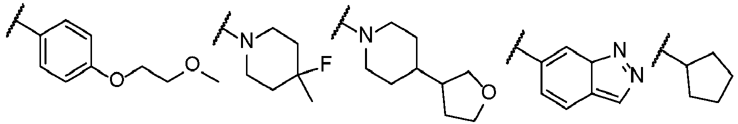 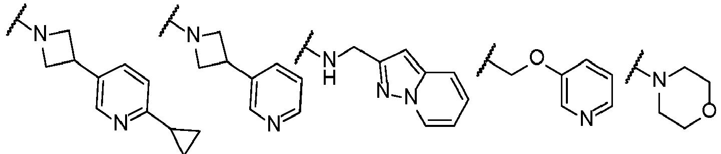 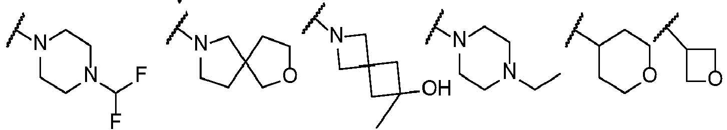 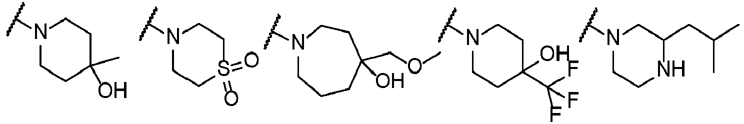 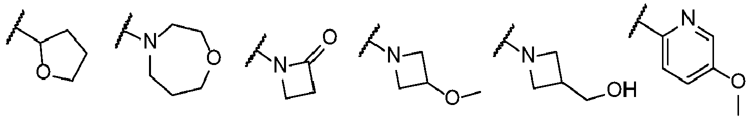 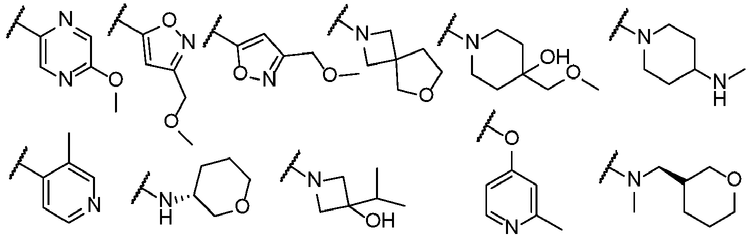 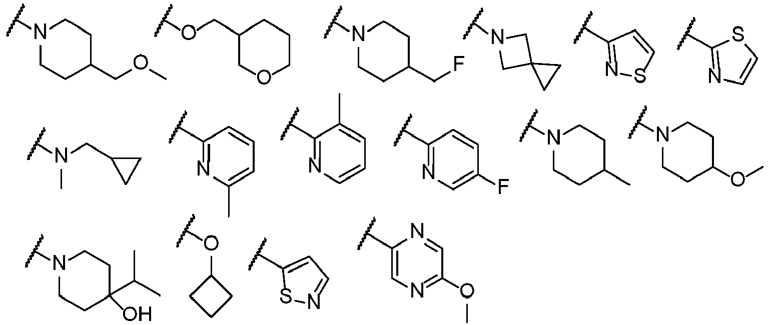
23. The compound of formula (I) or a pharmaceutically acceptable salt, solvate, prodrug, or pharmaceutically active metabolite thereof according to any of Clauses 1 to 22, wherein R⁶ is selected from the group consisting of hydrogen and the following structures: 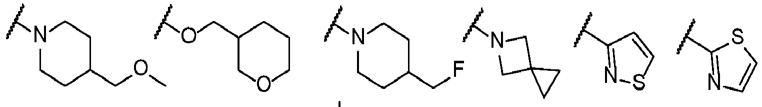 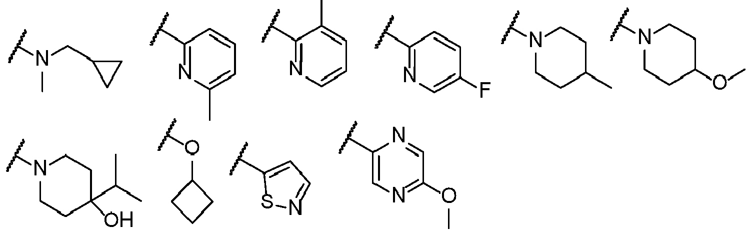
24. The compound of formula (I) or a pharmaceutically acceptable salt, solvate, prodrug, or pharmaceutically active metabolite thereof according to any of Clauses 1 to 23, wherein Q is selected from the group consisting of the following structures: 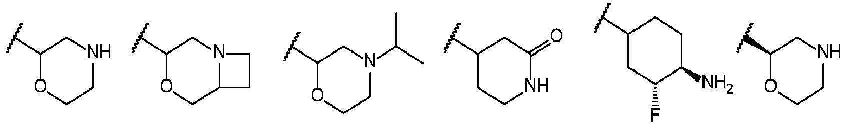 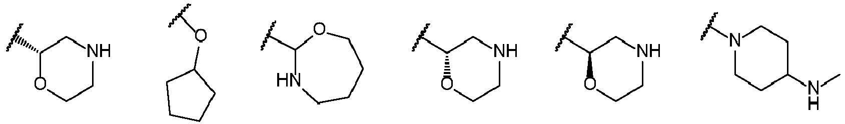 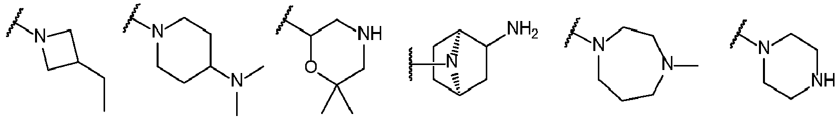 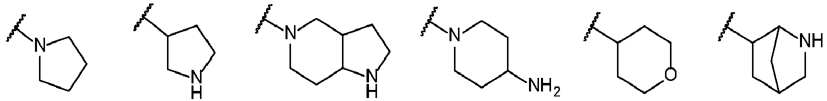 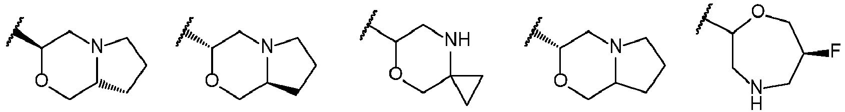 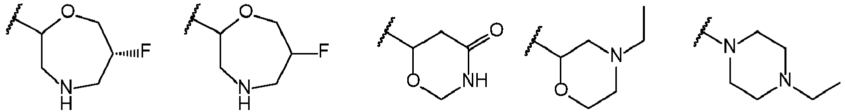 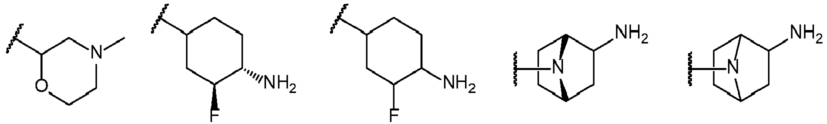 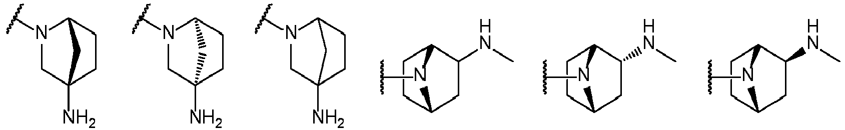 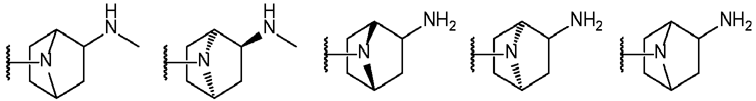 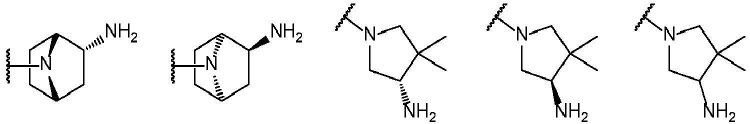 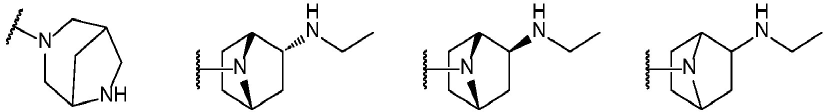 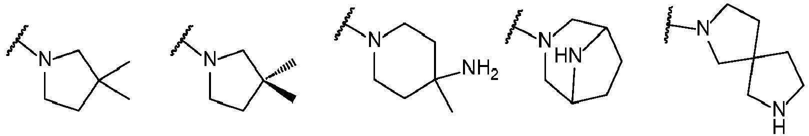 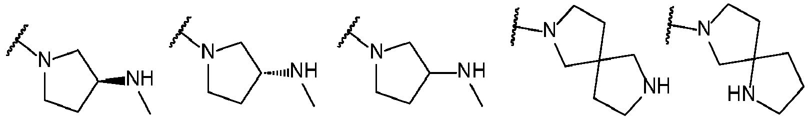 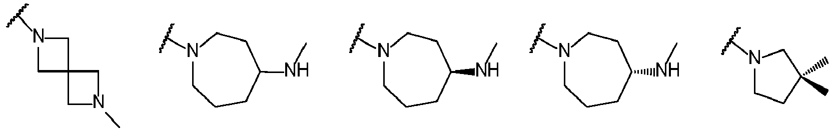 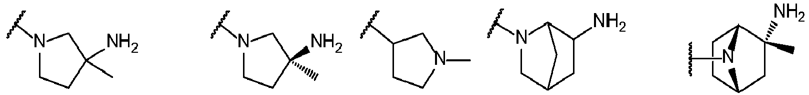 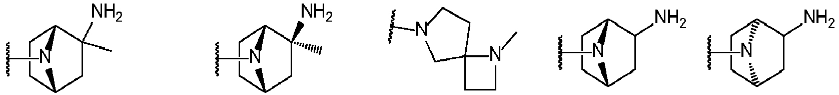 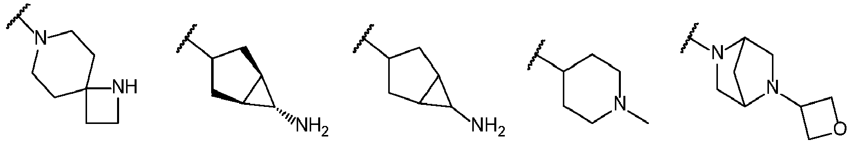 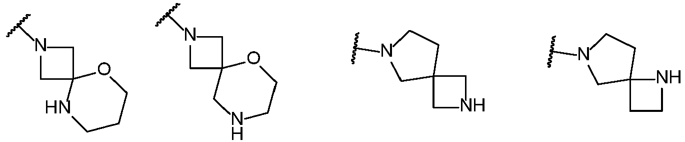 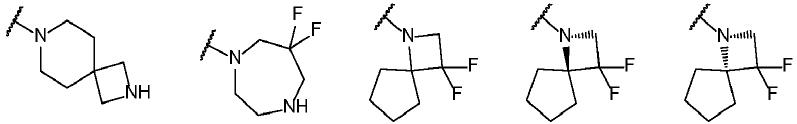 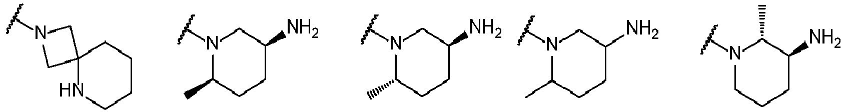 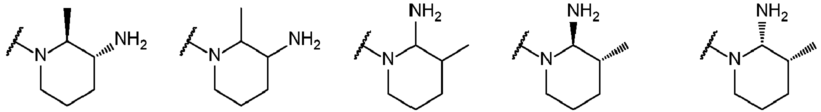 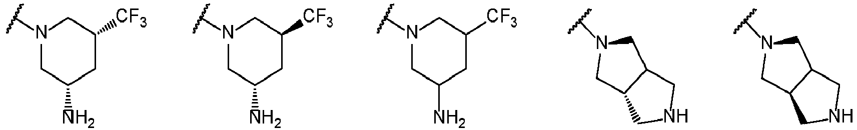 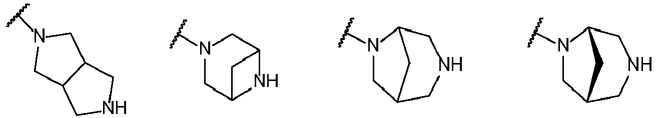 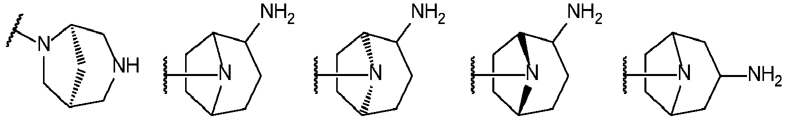 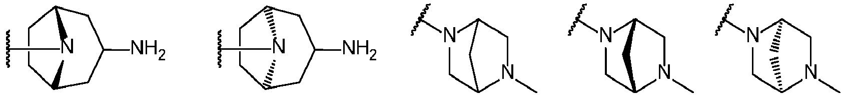 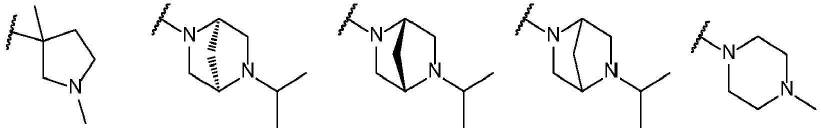 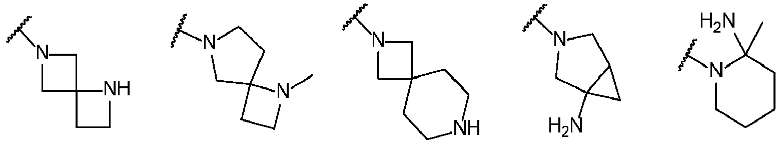 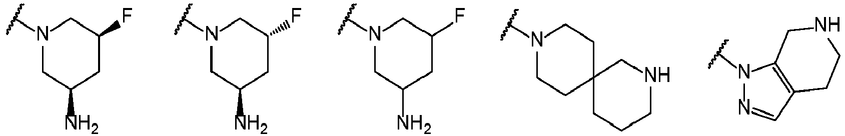 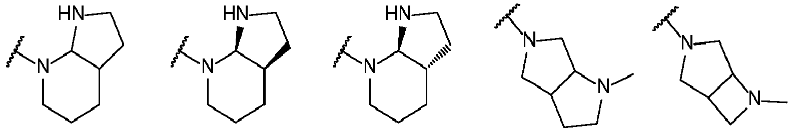 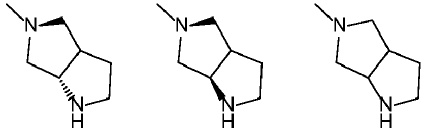
25. The compound of formula (I) or a pharmaceutically acceptable salt, solvate, prodrug, or pharmaceutically active metabolite thereof according to any of Clauses 1 to 24, wherein Q is selected from the group consisting of the following structures: 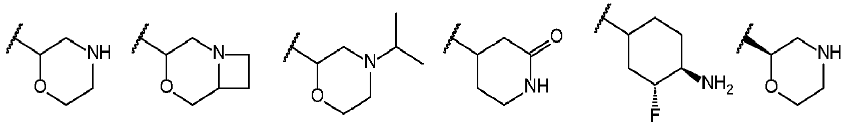 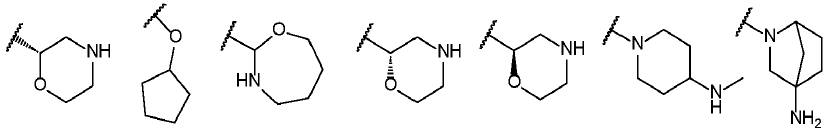 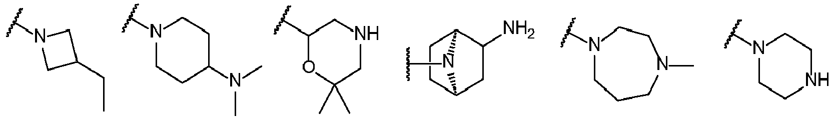 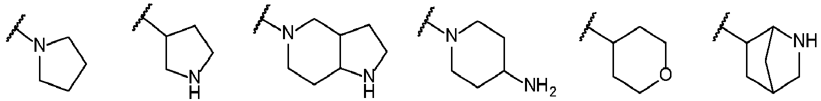 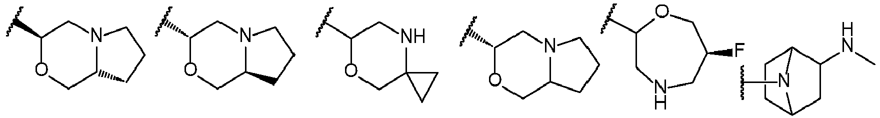 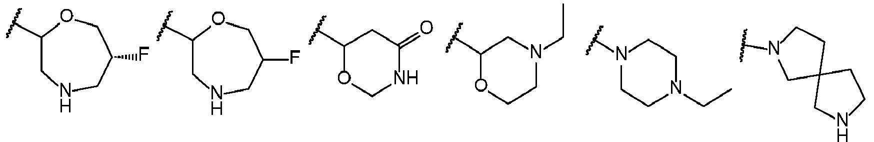 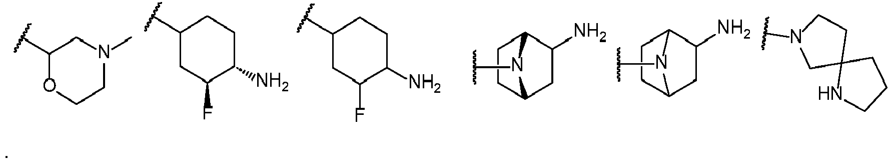
26. The compound of formula (I) or a pharmaceutically acceptable salt, solvate, prodrug, or pharmaceutically active metabolite thereof according to any of Clauses 1 to 25, wherein Q is selected from the group consisting of the following structures: 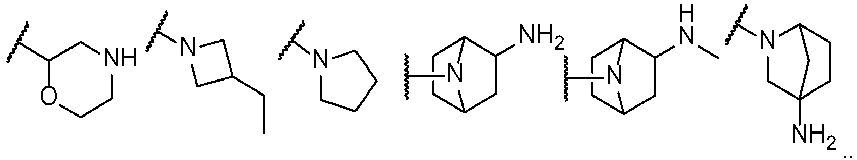
27. A compound selected from any one of:
   (i) the group of compounds shown in Table 1;
   (ii) the group of compounds of Table 1 having an IC50 against LSD1 ≤ 1,000 nM;
   (iii) the group of compounds of Table 1 having an IC50 against LSD1 ≤ 250 nM;
   (iv) the group of compounds of Table 1 having an IC50 against LSD1 ≤ 100 nM;
   (v) the group of compounds of Table 1 having an IC50 against LSD1 ≤ 50 nM;
   (vi) the group of compounds of Table 1 having an IC50 against LSD1 ≤ 25 nM; or
   (vii) the group of compounds of Table 1 having an IC50 against LSD1 ≤ 10 nM;
   or a pharmaceutically acceptable salt, solvate, prodrug, or pharmaceutically active metabolite thereof.
28. A compound selected from any one of Examples 1 to 351.
29. A compound selected from any one of Examples 4, 8, 15, 20, 26, 28, 71, 108, 109, 173, 177, 182, 183, 185, 190, 192, 194, 197, 207, 216, 234, 263, 302, 303, 315 and 335.
30. A compound selected from any one of Examples 1, 2, 3, 5, 6, 7, 9, 10, 11, 12, 13, 14, 16, 17, 18, 19, 22, 23, 24, 25, 29, 30, 34, 36, 37, 38, 39, 48, 53, 57, 74, 79, 81, 103, 110, 112, 115, 116, 154, 169, 170, 171, 172, 174, 175, 178, 179, 181, 186, 187, 188, 189, 193, 195, 198, 199, 201, 204, 206, 209, 210, 217, 218, 219, 222, 223, 224, 230, 231, 232, 233, 235, 237, 242, 250, 254, 255, 257, 259, 260, 262, 264, 268, 275, 279, 304, 309, 310, 311, 312, 313, 314, 316, 317, 321, 323, 324, 325, 333, 334 and 336.
31. A compound selected from any one of Examples 27, 31, 32, 33, 35, 43, 46, 47, 49, 51, 56, 60, 64, 67, 68, 70, 73, 82, 83, 91, 93, 94, 95, 100, 102, 104, 111, 128, 138, 153, 156, 176, 180, 184, 191, 196, 200, 202, 203, 205, 208, 211, 212, 213, 215, 220, 221, 227, 229, 236, 239, 240, 243, 244, 245, 247, 249, 251, 252, 253, 256, 258, 265, 273, 277, 299, 301, 305, 308, 318, 319, 322 and 339.
32. A compound selected from any one of Examples 21, 40, 41, 42, 44, 45, 50, 52, 54, 55, 58, 59, 61, 62, 63, 66, 69, 72, 75, 76, 77, 78, 80, 85, 86, 88, 92, 96, 98, 99, 101, 105, 106, 107, 113, 114, 118, 119, 120, 121, 122, 124, 125, 126, 127, 129, 133, 137, 139, 141, 143, 147, 148, 149, 151, 152, 155, 163, 164, 165, 166, 168, 225, 226, 228, 238, 241, 246, 248, 261, 266, 267, 269, 270, 271, 272, 274, 276, 278, 280, 282, 283, 284, 285, 288, 289, 291, 293, 294, 297, 298, 300, 306, 307, 320, 328, 332, 340, 341, 342, 346, 347, 348 and 349.
33. A compound selected from any one of Examples 65, 84, 87, 89, 90, 97, 117, 123, 130, 131, 132, 134, 135, 136, 140, 142, 144, 145, 146, 150, 175, 157, 158, 159, 160, 161, 162, 167, 214, 281, 286, 287, 290, 292, 295, 296, 326, 327, 329, 330, 331, 337, 338, 343, 344 and 345.
34. A pharmaceutical composition comprising a compound according to any of Clauses 1 to 33 or a pharmaceutically acceptable salt, solvate, prodrug, or pharmaceutically active metabolite thereof, or combinations thereof, and one or more pharmaceutically acceptable carrier.
35. A pharmaceutical composition comprising a compound of formula (I): 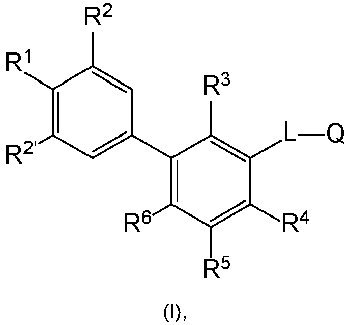 or a pharmaceutically acceptable salt, solvate, prodrug, or pharmaceutically active metabolite thereof, or combinations thereof,
   wherein
   R¹ is nitro or cyano;
   R² and R^{2'} are each independently selected from C₁₋₃ alkyl, hydrogen or halogen;
   R³ is selected from hydrogen, C₁₋₃ alkyl, C₁₋₃ haloalkyl, C₃-C₆ cycloalkyl, C₃-C₆ cycloalkenyl, C₃-C₆ heterocycloalkyl, C₄-C₅ heterocycloalkenyl, C₃-C₅ heterocycloalkyl C₁-C₃ alkyl, C₅-C₆ aryl, C₃-C₆ heteroaryl, C₁₋C₄ alkoxyl, C₃-C₆ cycloalkyl-C₁-C₃ alkoxyl, C₁₋C₄ haloalkoxyl, C₁-C₃ haloalkyl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, cyano, wherein each alkyl, cycloalkyl, cycloalkenyl, heterocycloalkyl, heterocycloalkenyl, heterocycloalkyl alkyl, aryl, heteroaryl, alkoxyl, cycloalkyl alkoxyl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, amino;
   R⁴ is selected from hydrogen or halogen;
   R⁵ is selected from the group consisting of hydrogen, C₁₋₃ alkyl, C₃-C₆ cycloalkyl, C₃-C₆ cycloalkenyl, C₃-C₆ aryl, C₃-C₆ heteroaryl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, wherein each alkyl, cycloalkyl, cycloalkenyl, aryl, heteroaryl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, or amino;
   R⁶ is selected from the group consisting of hydrogen, C₁₋C₄ alkoxyl, C₁₋C₄ alkoxyl, amino, alkylamino, aminoalkyl, dialkylamino, C₈₋C₁₁ spirocycloalkyl, C₅-C₁₀ heterospirocycloalkyl, C₅-C₁₁ monocyclic or bicyclic aryl, monocyclic or bicyclic heteroaryl, C₂-C₆ cyclic amine, C₂-C₅ heterocyclic amine, C₃-C₆ unsaturated or aromatic cyclic amine, C₃-C₅ unsaturated or aromatic heterocyclic amine, C₇₋C₁₀ spirocyclic amine, or C₄₋C₉ heterospirocyclic amine, wherein each is optionally substituted with 1 to 3 substituents selected from the group consisting of hydroxyl, amino, C₁-C₆ alkyl, C₁-C₆ alkoxyl, halogen, C₁-C₃ haloalkyl, optionally substituted C₃-C₆ cycloalkyl, optionally substituted C₂-C₅ heterocycloalkyl, or optionally substituted C₃-C₆ heteroaryl, wherein said optionally substituted cycloalkyl, heterocycloalkyl and heteroaryl may be substituted with 1 or 2 substituents selected from the group consisting of C₁-C₃ alkyl, C₁-C₃ alkoxyl, hydroxyl, halogen or amine;
   L is a linker selected from the group consisting of a bond, -CH₂-, -CH₂-CH₂-, -CH(CH₃)-,
   -C(CH₃)₂-, -C=C-, -CH₂-O-, -C(O)-, -O-, -O-CH₂-, -NH-CH₂-, -N(CH₃)-CH₂-, --NH-C(O)-, -N(CH₃)-C(O)-, -CH₂-NH-CH₂-
   Q is a monocyclic or bicyclic ring system selected from the group consisting of C₅-C₉ cycloalkyl, C₄-C₈ heterocycloalkyl, C₈₋C₁₁ spirocycloalkyl, C₅-C₁₀ heterospirocycloalkyl, C₃-C₈ cyclic amine, C₃-C₈ heterocyclic amine, C₇₋C₁₀ spirocyclic amine, or C₄₋C₉ heterospirocyclic amine, wherein each cycloalkyl or heterocycloalkyl group may be saturated or unsaturated, said bicyclic ring may be a fused or bridged bicycle, and wherein Q is optionally substituted with 1 to 3 R⁷, wherein each R⁷ is independently selected from hydrogen, C₁-C₆ alkyl, C₁-C₆ haloalkyl, C₁-C₆ alkylcarbonyl, hydroxyl, oxo, halogen, C₁-C₆ alkylamine or amino;
   and one or more pharmaceutically acceptable carrier.
36. The pharmaceutical composition according to Clause 35, wherein the compound is defined according to any of Clauses 2 to 33.
37. A pharmaceutical composition comprising a compound selected from:
   (i) the group of compounds shown in Table 1;
   (ii) the group of compounds of Table 1 having an IC50 against LSD1 ≤ 1,000 nM;
   (iii) the group of compounds of Table 1 having an IC50 against LSD1 ≤ 250 nM;
   (iv) the group of compounds of Table 1 having an IC50 against LSD1 ≤ 100 nM;
   (v) the group of compounds of Table 1 having an IC50 against LSD1 ≤ 50 nM;
   (vi) the group of compounds of Table 1 having an IC50 against LSD1 ≤ 25 nM; or
   (vii) the group of compounds of Table 1 having an IC50 against LSD1 ≤ 10 nM;
   or a pharmaceutically acceptable salt, solvate, prodrug, or pharmaceutically active metabolite thereof, or combinations thereof, and one or more pharmaceutically acceptable carrier.
38. A pharmaceutical composition comprising a compound selected from any one of the compounds of Examples 1 to 351, or a pharmaceutically acceptable salt, solvate, prodrug, or pharmaceutically active metabolite thereof, or combinations thereof, and one or more pharmaceutically acceptable carrier.
39. The compound according to any of Clauses 1 to 33, or the pharmaceutical composition according to any of Clauses 34 to 38 for use in medicine.
40. The compound or the pharmaceutical composition for use according to Clause 39, wherein the use is in the treatment of diseases, conditions or disorders associated with LSD1.
41. The compound or the pharmaceutical composition for use according to Clause 39 or Clause 40, wherein the use is in the treatment of cancers, oncologic diseases, autoimmune disorders and/or inflammatory diseases.
42. The compound of pharmaceutical composition for use according to any of Clauses 39 to 41, wherein the use is in the treatment of diseases, conditions or disorders selected from the group consisting of: breast, prostate, head and neck, brain, laryngeal, oral and thyroid cancers (e.g. papillary thyroid carcinoma), hematological cancers (e.g. non-Hodgkin lymphoma, B cell lymphoma; chronic myelogenous leukemia), sarcomas, lung cancers, gastrointestinal cancers, genitourinary tract cancers, liver cancers, bone cancers, nervous system cancers, gynecological cancers and skin cancers.
43. The compound of pharmaceutical composition for use according to any of Clauses 39 to 42, wherein the use is in the treatment of diseases, conditions or disorders selected from the group consisting of:
   lymphoma, including diffuse large B-cell lymphoma (DLBCL), mantle cell lymphoma, non-Hodgkin lymphoma, relapsed or refractory NHL and recurrent follicular lymphoma, Hodgkin lymphoma;
   leukemia, including acute lymphoblastic leukemia (ALL), acute myelogenous leukemia (AML), acute promyelocytic leukemia (APL), chronic lymphocytic leukemia (CLL), chronic myelogenous leukemia (CML);
   myeloproliferative disease, including primary myelofibrosis (PMF), polycythemia vera (PV), essential thrombocytosis (ET);
   myelodysplasia syndrome (MDS), and multiple myeloma;
   sarcoma including chondrosarcoma, Ewing's sarcoma, osteosarcoma, rhabdomyosarcoma, angiosarcoma, fibrosarcoma, liposarcoma, myxoma, rhabdomyoma, fibroma, lipoma, harmatoma, and teratoma;
   lung cancer, including non-small cell lung cancer (NSCLC), small cell lung cancer, bronchogenic carcinoma, squamous cell, undifferentiated small cell, undifferentiated large cell, adenocarcinoma, alveolar (bronchiolar) carcinoma, bronchial adenoma, chondromatous hamartoma, and mesothelioma;
   gastrointestinal cancer, including esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma), and colorectal cancer;
   genitourinary tract cancer, including cancers of the kidney (adenocarcinoma, Wilm's tumor, nephroblastomal), bladder and urethra (squamous cell carcinoma, transitional cell carcinoma, adenocarcinoma), prostate (adenocarcinoma, sarcoma), and testis (seminoma, teratoma, embryonal carcinoma, teratocarcinoma, choriocarcinoma, sarcoma, interstitial cell carcinoma, fibroma, fibroadenoma, adenomatoid tumors, lipoma);
   liver cancer, including hepatoma (hepatocellular carcinoma), cholangiocarcinoma, hepatoblastoma, angiosarcoma, hepatocellular adenoma, and hemangioma;
   bone cancer, including osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma, and giant cell tumors;
   nervous system cancer, including cancers of the skull (osteoma, hemangioma, granuloma, xanthoma, osteitis deformans), meninges (meningioma, meningiosarcoma, gliomatosis), brain (astrocytoma, meduoblastoma, glioma, ependymoma, germinoma (pinealoma), glioblastoma multiform, oligodendroglioma, schwannoma, retinoblastoma, congenital tumors), and spinal cord (neurofibroma, meningioma, glioma, sarcoma), as well as neuroblastoma and Lhermitte-Duclos disease;
   gynecological cancer, including cancers of the uterus (endometrial carcinoma), cervix (cervical carcinoma, pre -tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), and fallopian tubes (carcinoma);
   skin cancer, including melanoma, basal cell carcinoma, squamous cell carcinoma, Kaposi's sarcoma, moles dysplastic nevi, lipoma, angioma, dermatofibroma, and keloids.
44. The compound of pharmaceutical composition for use according to any of Clauses 39 to 43, wherein the use is in the treatment of diseases, conditions or disorders selected from the group consisting of: glioblastoma and acute myeloid leukemia (AML).
45. The compound according to any of Clauses 1 to 33, the pharmaceutical composition according to any of Clauses 34 to 38, or the compound or pharmaceutical composition for use according to any of Clauses 39 to 44, wherein the compound is an inhibitor of LSD1.
46. The compound or pharmaceutical composition for use according to any of Clauses 39 to 45, wherein the use is in a method comprising administering the compound orally, topically, by inhalation, by intranasal administration, or systemically by intravenous, intraperitoneal, subcutaneous, or intramuscular injection.
47. The compound or pharmaceutical composition for use according to any of Clauses 39 to 46, wherein the use is in a method comprising administering the compound according to formula (I) in combination with one or more additional therapeutic agent.
48. The compound or pharmaceutical composition for use according to Clause 47, wherein the administering comprises administering the compound according to formula (I) simultaneously, sequentially or separately from the one or more additional therapeutic agent.
49. The compound or pharmaceutical composition for use according to any of Clauses 39 to 48, which comprises administering to a subject an effective amount of the compound of formula (I), wherein the effective amount is between about 500 nM and about 10 µM in the blood of the subject or in the plasma of the subject.
50. A method of treating a disease, disorder or condition in a subject in need thereof, the method comprising administering to the subject a therapeutically effective amount of a compound according to any of Clauses 1 to 33, or a pharmaceutical composition according to any of Clauses 34 to 38.
51. The method of Clause 50, wherein the disease, disorder or condition is associated with LSD1.
52. The method of Clause 50 or Clause 51, wherein the disease, disorder or condition is associated with overexpression of or elevated levels of LSD1 in the subject.
53. A method of treating a disease, disorder or condition associated with LSD1 in a subject in need thereof, the method comprising administering to the subject a therapeutically effective amount of a compound according to any of Clauses 1 to 33, or a pharmaceutical composition according to any of Clauses 34 to 38.
54. The method of any of Clauses 50 to 53, wherein the compound is an inhibitor of LSD1.
55. The method of any of Clauses 50 to 54, wherein the method comprises administering the compound or pharmaceutical composition orally, topically, by inhalation, by intranasal administration, or systemically by intravenous, intraperitoneal, subcutaneous, or intramuscular injection.
56. The method according to any of Clauses 50 to 55, wherein the method comprises administering the compound according to formula (I) in combination with one or more additional therapeutic agent.
57. The method according to any of Clauses 50 to 56, wherein the method comprises administering the compound according to formula (I) simultaneously, sequentially or separately from the one or more additional therapeutic agent.
58. The method according to any of Clauses 50 to 57, wherein the method comprises administering to a subject an effective amount of the compound of formula (I), wherein the effective amount is between about 500 nM and about 10 µM in the blood of the subject or in the plasma of the subject.
59. The method according to any of Clauses 50 to 58, wherein the method is for the treatment of cancers, oncologic diseases, autoimmune disorders and/or inflammatory diseases.
60. The method according to any of Clauses 50 to 59, wherein the method is for the treatment of diseases, conditions or disorders selected from the group consisting of: breast, prostate, head and neck, brain, laryngeal, oral and thyroid cancers (e.g. papillary thyroid carcinoma), hematological cancers (e.g. non-Hodgkin lymphoma, B cell lymphoma; chronic myelogenous leukemia), sarcomas, lung cancers, gastrointestinal cancers, genitourinary tract cancers, liver cancers, bone cancers, nervous system cancers, gynecological cancers and skin cancers.
61. The method according to any of Clauses 50 to 60, wherein the method is for the treatment of cancer, and the cancer is selected from the group consisting of:
   lymphoma, including diffuse large B-cell lymphoma (DLBCL), mantle cell lymphoma, non-Hodgkin lymphoma, relapsed or refractory NHL and recurrent follicular lymphoma, Hodgkin lymphoma;
   leukemia, including acute lymphoblastic leukemia (ALL), acute myelogenous leukemia (AML), acute promyelocytic leukemia (APL), chronic lymphocytic leukemia (CLL), chronic myelogenous leukemia (CML);
   myeloproliferative disease, including primary myelofibrosis (PMF), polycythemia vera (PV), essential thrombocytosis (ET);
   myelodysplasia syndrome (MDS), and multiple myeloma;
   sarcoma including chondrosarcoma, Ewing's sarcoma, osteosarcoma, rhabdomyosarcoma, angiosarcoma, fibrosarcoma, liposarcoma, myxoma, rhabdomyoma, fibroma, lipoma, harmatoma, and teratoma;
   lung cancer, including non-small cell lung cancer (NSCLC), small cell lung cancer, bronchogenic carcinoma, squamous cell, undifferentiated small cell, undifferentiated large cell, adenocarcinoma, alveolar (bronchiolar) carcinoma, bronchial adenoma, chondromatous hamartoma, and mesothelioma;
   gastrointestinal cancer, including esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma), and colorectal cancer;
   genitourinary tract cancer, including cancers of the kidney (adenocarcinoma, Wilm's tumor, nephroblastomal), bladder and urethra (squamous cell carcinoma, transitional cell carcinoma, adenocarcinoma), prostate (adenocarcinoma, sarcoma), and testis (seminoma, teratoma, embryonal carcinoma, teratocarcinoma, choriocarcinoma, sarcoma, interstitial cell carcinoma, fibroma, fibroadenoma, adenomatoid tumors, lipoma);
   liver cancer, including hepatoma (hepatocellular carcinoma), cholangiocarcinoma, hepatoblastoma, angiosarcoma, hepatocellular adenoma, and hemangioma;
   bone cancer, including osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma, and giant cell tumors;
   nervous system cancer, including cancers of the skull (osteoma, hemangioma, granuloma, xanthoma, osteitis deformans), meninges (meningioma, meningiosarcoma, gliomatosis), brain (astrocytoma, meduoblastoma, glioma, ependymoma, germinoma (pinealoma), glioblastoma multiform, oligodendroglioma, schwannoma, retinoblastoma, congenital tumors), and spinal cord (neurofibroma, meningioma, glioma, sarcoma), as well as neuroblastoma and Lhermitte-Duclos disease;
   gynecological cancer, including cancers of the uterus (endometrial carcinoma), cervix (cervical carcinoma, pre -tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), and fallopian tubes (carcinoma);
   skin cancer, including melanoma, basal cell carcinoma, squamous cell carcinoma, Kaposi's sarcoma, moles dysplastic nevi, lipoma, angioma, dermatofibroma, and keloids.
62. The method according to any of Clauses 50 to 61, wherein the method is for the treatment of diseases, conditions or disorders selected from the group consisting of: glioblastoma and acute myeloid leukemia (AML).

Additional expressions of the inventive concept are set out in each of the following clauses:
Clause A1. A compound of formula (I), or a pharmaceutically acceptable salt, solvate, prodrug, or pharmaceutically active metabolite thereof: 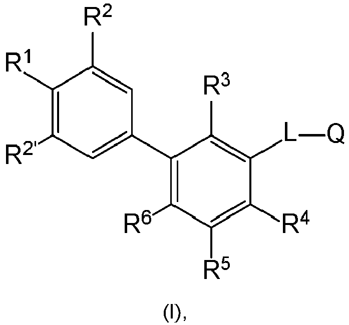 wherein
   R¹ is nitro or cyano;
   R² and R^{2'} are each independently selected from C₁₋₃ alkyl, hydrogen or halogen
   R³ is selected from hydrogen, C₁₋₃ alkyl, C₁₋₃ haloalkyl, C₃-C₆ cycloalkyl, C₃-C₆ cycloalkenyl, C₃-C₆ heterocycloalkyl, C₄-C₅ heterocycloalkenyl, C₃-C₅ heterocycloalkyl C₁-C₃ alkyl, C₅-C₆ aryl, C₃-C₆ heteroaryl, C₁₋C₄ alkoxyl, C₃-C₆ cycloalkyl-C₁-C₃ alkoxyl, C₁₋C₄ haloalkoxyl, C₁-C₃ haloalkyl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, cyano, wherein each alkyl, cycloalkyl, cycloalkenyl, heterocycloalkyl, heterocycloalkenyl, heterocycloalkyl alkyl, aryl, heteroaryl, alkoxyl, cycloalkyl alkoxyl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, amino;
   R⁴ is selected from hydrogen or halogen;
   R⁵ is selected from the group consisting of hydrogen, C₁₋₃ alkyl, C₃-C₆ cycloalkyl, C₃-C₆ cycloalkenyl, C₃-C₆ aryl, C₃-C₆ heteroaryl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, wherein each alkyl, cycloalkyl, cycloalkenyl, aryl, heteroaryl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, or amino;
   R⁶ is selected from the group consisting of hydrogen, C₁₋C₄ alkoxyl, C₁₋C₄ alkoxyl, amino, alkylamino, aminoalkyl, dialkylamino, C₈-C₁₁ spirocycloalkyl, C₅-C₁₀ heterospirocycloalkyl, C₅-C₁₁ monocyclic or bicyclic aryl, monocyclic or bicyclic heteroaryl, C₂-C₆ cyclic amine, C₂-C₅ heterocyclic amine, C₃-C₆ unsaturated or aromatic cyclic amine, C₃-C₅ unsaturated or aromatic heterocyclic amine, C₇₋C₁₀ spirocyclic amine, or C₄-C₉ heterospirocyclic amine, wherein each is optionally substituted with 1 to 3 substituents selected from the group consisting of hydroxyl, amino, C₁-C₆ alkyl, C₁-C₆ alkoxyl, halogen, C₁-C₃ haloalkyl, optionally substituted C₃-C₆ cycloalkyl, optionally substituted C₂-C₅ heterocycloalkyl, or optionally substituted C₃-C₆ heteroaryl, wherein said optionally substituted cycloalkyl, heterocycloalkyl and heteroaryl may be substituted with 1 or 2 substituents selected from the group consisting of C₁-C₃ alkyl, C₁-C₃ alkoxyl, hydroxyl, halogen or amine;
   L is a linker selected from the group consisting of a bond, -CH₂-, -CH₂-CH₂-, -CH(CH₃)-, -C(CH₃)₂-, -C=C-, -CH₂-O-, -C(O)-, -O-, -O-CH₂-, -NH-CH₂-, -N(CH₃)-CH₂-, --NH-C(O)-, -N(CH₃)-C(O)-, -CH₂-NH-CH₂-
   Q is a monocyclic or bicyclic ring system selected from the group consisting of C₅-C₉ cycloalkyl, C₄-C₈ heterocycloalkyl, C₈-C₁₁ spirocycloalkyl, C₅-C₁₀ heterospirocycloalkyl, C₃-C₅ cyclic amine, C₃-C₅ heterocyclic amine, C₇₋C₁₀ spirocyclic amine, or C₄-C₉ heterospirocyclic amine, wherein each cycloalkyl or heterocycloalkyl group may be saturated or unsaturated, said bicyclic ring may be a fused or bridged bicycle, and wherein Q is optionally substituted with 1 to 3 R⁷, wherein each R⁷ is independently selected from hydrogen, C₁-C₆ alkyl, C₁-C₆ haloalkyl, C₁-C₆ alkylcarbonyl, hydroxyl, oxo, halogen, C₁-C₆ alkylamine or amino.
A2. The compound of formula (I) or a pharmaceutically acceptable salt, solvate, prodrug, or pharmaceutically active metabolite thereof according to Clause A1, wherein each heterocyclic or heteroaryl substituent may include from 1 to 4 heteroatoms independently selected from O, S and N .
A3. The compound of formula (I) or a pharmaceutically acceptable salt, solvate, prodrug, or pharmaceutically active metabolite thereof according to Clause A1 or Clause A2, wherein:
   (i) one of R² and R^{2'} is hydrogen and the other is F;
   (ii) one of R² and R^{2'} is hydrogen and the other is CI; or
   (iii) both R² and R^{2'} are hydrogen.
A4. The compound of formula (I) or a pharmaceutically acceptable salt, solvate, prodrug, or pharmaceutically active metabolite thereof according to any of Clauses A1 to A3, wherein R³ is selected from hydrogen, halogen, C₃-C₆ cycloalkyl, C₃-C₆ heterocycloalkyl, C₅-C₆ aryl, C₃-C₆ heteroaryl, or C₂-C₅ heterocyclic amine.
A5. The compound of formula (I) or a pharmaceutically acceptable salt, solvate, prodrug, or pharmaceutically active metabolite thereof according to Clause A4, wherein:
   (i) R³ is hydrogen or Cl;
   (ii) R³ is an optionally substituted 5 or 6 membered heteroaryl;
   (iii) R³ is an optionally substituted 4 to 6 membered heterocycloalkyl; or
   (iv) R³ is an optionally substituted 3 to 5 membered cycloalkyl;
      wherein each hetero moiety may include 1 or 2 heteroatoms independently selected from O, S and N; and
      wherein said optional substituents are independently selected from 1 or 2 substituents of the group consisting of methyl, F and CI.
A6. The compound of formula (I) or a pharmaceutically acceptable salt, solvate, prodrug, or pharmaceutically active metabolite thereof according to any of Clauses A1 to A5, wherein:
   (i) R⁴ is hydrogen;
   (ii) R⁴ is F and R⁵ and R⁶ are each hydrogen;
   (iii) R⁴ is F and R⁵ and R⁶ are each hydrogen; or
   (iii) R⁴, R⁵ and R⁶ are all hydrogen.
A7. The compound of formula (I) or a pharmaceutically acceptable salt, solvate, prodrug, or pharmaceutically active metabolite thereof according to any of Clauses A1 to A6, wherein:
   (i) R⁵ is hydrogen;
   (ii) R⁵ is F;
   (iii) R⁵ is a 4 or 5 membered heterocycloalkyl;
   (iv) R⁵ is C₁-C₃ alkyl;
   (v) R⁵ is 3, 4 or 5 membered cycloalkyl
   (vi) R⁵ is optionally substituted C₃-C₆ cycloalkyl, C₃-C₆ cycloalkenyl, C₃-C₆ heterocycloalkyl, C₃-C₆ aryl or C₃-C₆ heteroaryl.
A8. The compound of formula (I) or a pharmaceutically acceptable salt, solvate, prodrug, or pharmaceutically active metabolite thereof according to any of Clauses A1 to A7, wherein R⁵ is selected from hydrogen, F, methyl or pyrrolidine.
A9. The compound of formula (I) or a pharmaceutically acceptable salt, solvate, prodrug, or pharmaceutically active metabolite thereof according to any of Clauses A1 to A8, wherein:
   (i) R⁶ is hydrogen;
   (ii) R⁶ is F or CI;
   (iii) R⁶ is an optionally substituted 4 or 5 membered heterocycloalkyl;
   (iv) R⁶ is an optionally substituted 8, 9 or 10 membered bicyclic or spirocyclic heterocycloalkyl;
   (v) R⁶ is an optionally substituted 5 or 6 membered heteroaryl;
   (vi) R⁶ is an optionally substituted 4, 5 or 6 membered cycloalkyl or spirocycloalkyl;
   (vii) R⁶ is a C₁-C₃ haloalkoxy; or
   (viii) R⁶ is a C₂-C₈ alkylamine or dialkylamine
      wherein each hetero moiety may include 1 or 2 heteroatoms independently selected from O, S and N; and
      wherein said optional substituents are independently selected from 1 or 2 substituents from the group consisting of C₁-C₃ alkyl, C₁-C₃ haloalkyl, C₁-C₃ alkoxyl, F, Cl and hydroxyl.
A10. The compound of formula (I) or a pharmaceutically acceptable salt, solvate, prodrug, or pharmaceutically active metabolite thereof according to any of Clauses A1 to A9, wherein R⁶ is selected from hydrogen, F, methyl or pyrollidine.
A11. The compound of formula (I) or a pharmaceutically acceptable salt, solvate, prodrug, or pharmaceutically active metabolite thereof according to any of Clauses A1 to A10, wherein L is selected from -CH₂- or -CH₂-CH₂-.
A12. The compound of formula (I) or a pharmaceutically acceptable salt, solvate, prodrug, or pharmaceutically active metabolite thereof according to any of Clauses A1 to A11, wherein Q is selected from the group consisting of optionally substituted 5 to 7 membered heterocycloalkyl and optionally substituted 4 to 7 membered mono or fused or bridged bicycloalkylamine;
   wherein each heterocyclic moiety may include 1 or 2 heteroatoms independently selected from O and N; and
   wherein said optional substituents are independently selected from 1 to 3 substituents from the group consisting of C₁-C₃ alkyl, C₁-C₃ haloalkyl, C₁-C₃ alkoxyl, C₁-C₃ alkylamine, C₁-C₃ dialkylamine, C₁-C₃ carbonyl, amine, amino-C₁-C₃ alkyl, oxo, F, Cl and hydroxyl.
A13. The compound of formula (I) or a pharmaceutically acceptable salt, solvate, prodrug, or pharmaceutically active metabolite thereof according to any of Clauses A1 to A12, wherein:
   (i) R⁷ is independently selected from C₁-C₃ alkyl, C₁-C₃ haloalkyl, hydroxyl, oxo, F, C₁-C₃ alkylamino, C₁-C₃ dialkylamino or amino;
   (ii) R⁷ is independently selected from methyl, ethyl, amine and F;
   wherein there are 1, 2 or 3 R⁷ groups.
A14. The compound of formula (I) or a pharmaceutically acceptable salt, solvate, prodrug, or pharmaceutically active metabolite thereof according to Clause A1, wherein:
   (i) R¹ is cyano; and/or
   (ii) R² and R^{2'} are each independently selected from hydrogen or F; and/or
   (iii) R³ is selected from hydrogen, propyl, i-propyl, CHF₂, cyclopropyl, -CH₂-cyclopropyl, -CH₂-oxetane, isothiazole, oxazole, pyridyl, methoxyl, -O-CH₂-cyclopropyl, -O-CH₂-CF₃, -O-CF₃, CF₃, Cl, amino, - N(CH₃)(CH₂-isopropyl), pyrrolidine, cyano; and/or
   (iv) R⁴ is hydrogen or F; and/or
   (v) R⁵ is selected from hydrogen, methyl, cyclopropyl, cyclopentenyl, F, amino, NH-CH₂-CH₂-N(CH₃)₂; and/or
   (vi) R⁶ is selected from hydrogen, -O-cyclobutyl, -O-CH₂-oxane, -O-CH₂-CH₂-CHF₂, -O-CH₂-CH₂-CF₃, -O-CH₂-CF₃, -CH₂₋O-CHF₂, amino, -N(CH₃)(CH-(CH₃)₂, benzimdazole, pyridine; and/or
   (vii) L is a linker selected from a bond, -CH₂-, -C≡C-, -CH₂-NH-CH₂-, and/or
   (viii) Q is C₄-C₈ heterocycloalkyl which may comprise a further heteroatom selected from N or O; or Q is morpholinyl; and wherein
   each heteroaryl, heterocycloalkyl and/or cycloalkyl group is optionally substituted.
A15. The compound of formula (I) or a pharmaceutically acceptable salt, solvate, prodrug, or pharmaceutically active metabolite thereof according to any of Clauses A1 to A14, wherein R³ is selected from the group consisting of the following structures: 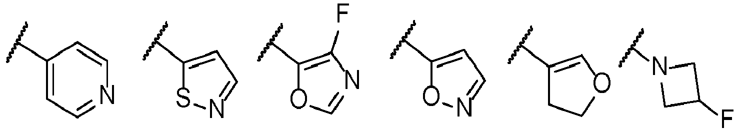 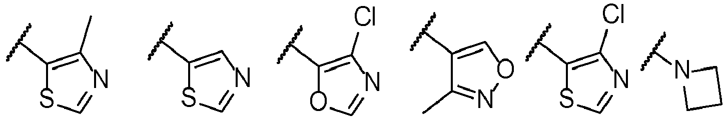 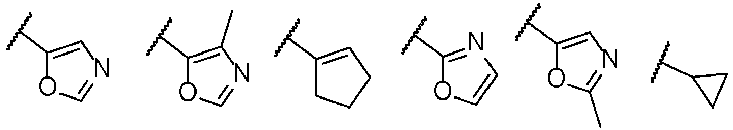 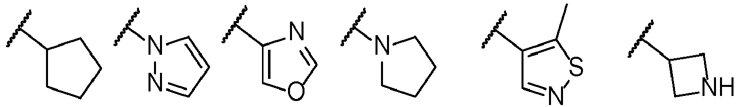 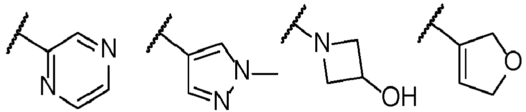 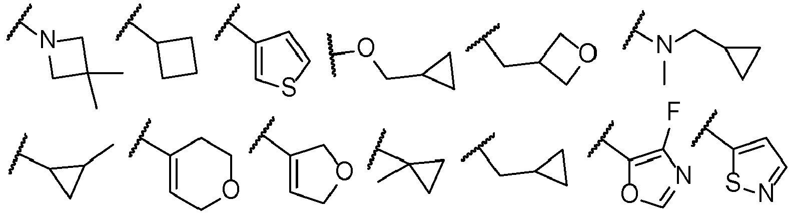
A16. The compound of formula (I) or a pharmaceutically acceptable salt, solvate, prodrug, or pharmaceutically active metabolite thereof according to any of Clauses A1 to A15, wherein R⁵ is selected from the group consisting of hydrogen and the following structures: 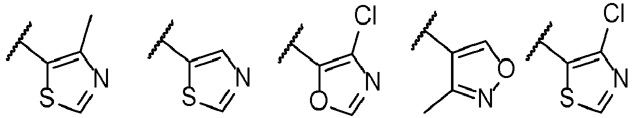 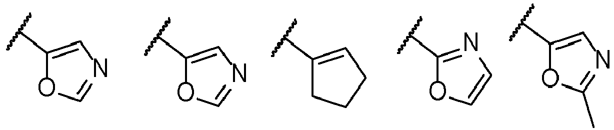 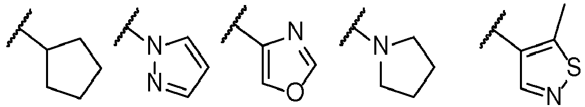 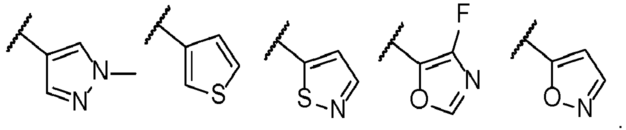
A17. The compound of formula (I) or a pharmaceutically acceptable salt, solvate, prodrug, or pharmaceutically active metabolite thereof according to any of Clauses A1 to A16, wherein R⁶ is selected from the group consisting of hydrogen and the following structures: 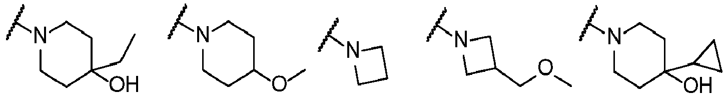 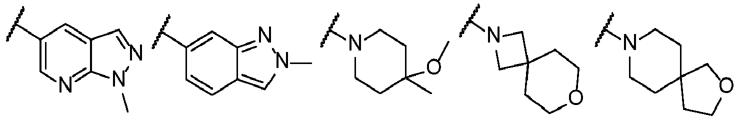 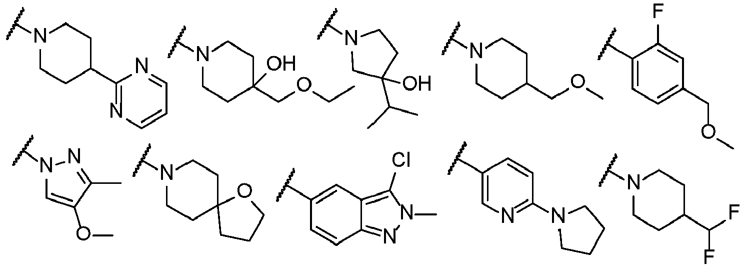 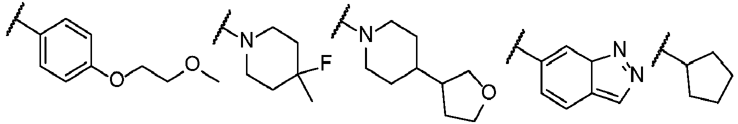 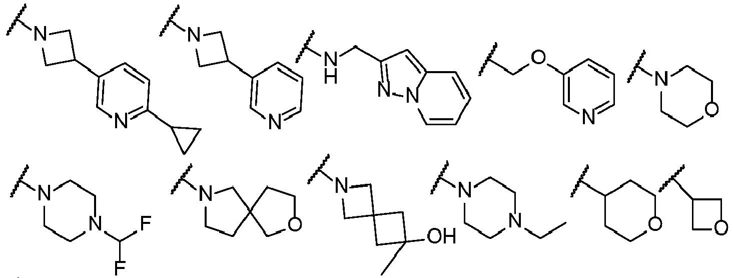 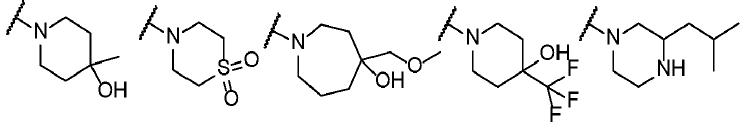 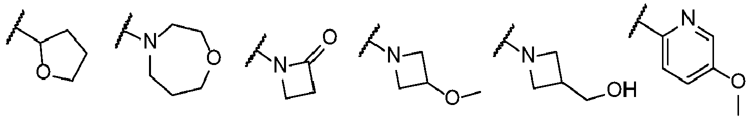 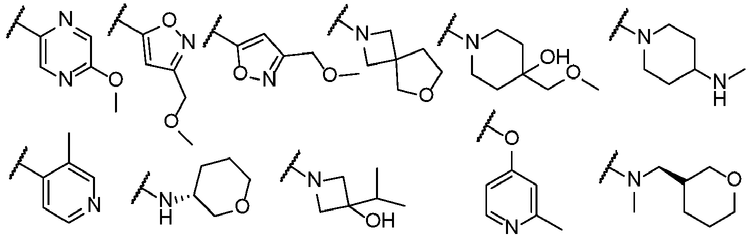 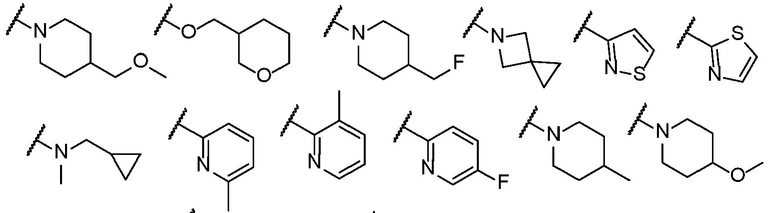 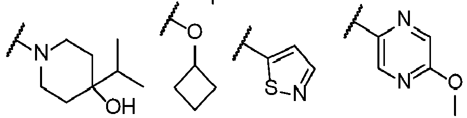
A18. The compound of formula (I) or a pharmaceutically acceptable salt, solvate, prodrug, or pharmaceutically active metabolite thereof according to any of Clauses A1 to A17, wherein Q is selected from the group consisting of the following structures: 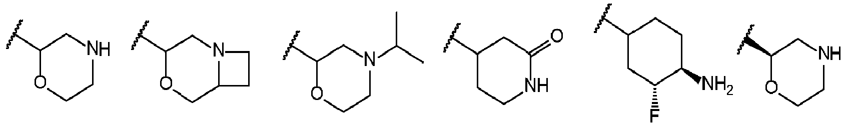 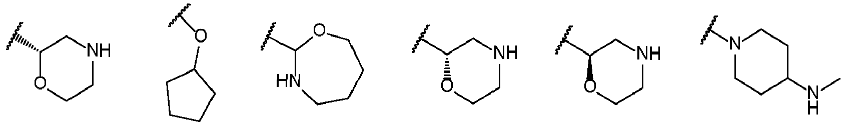 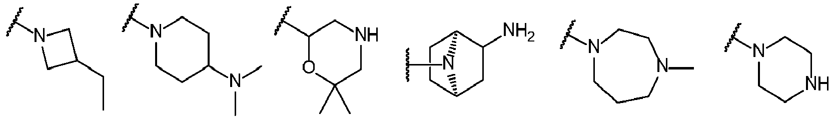 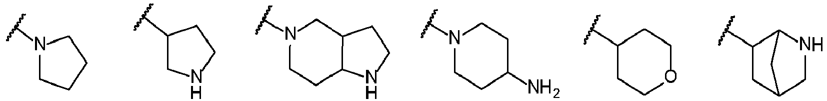 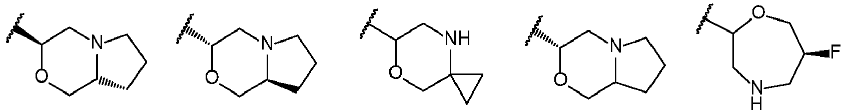 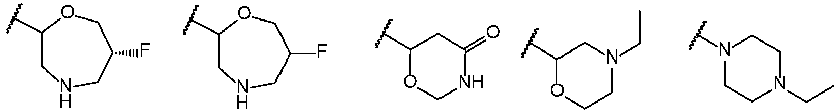 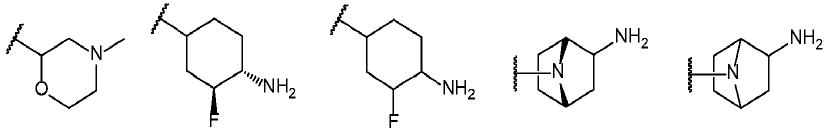 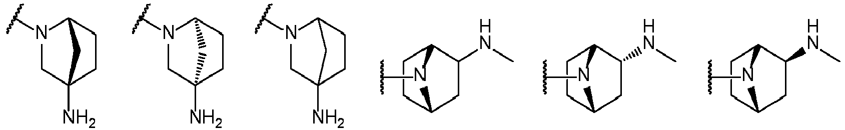 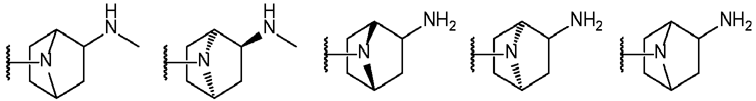 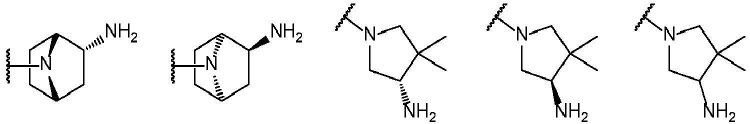 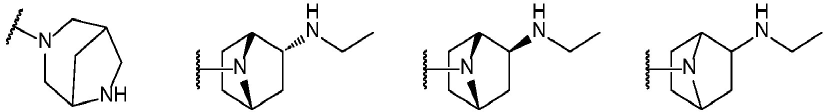 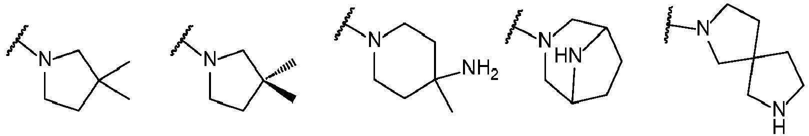 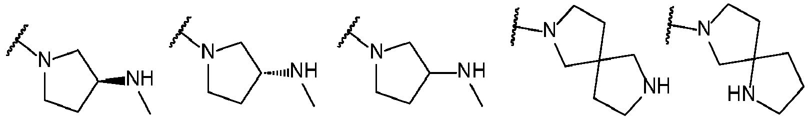 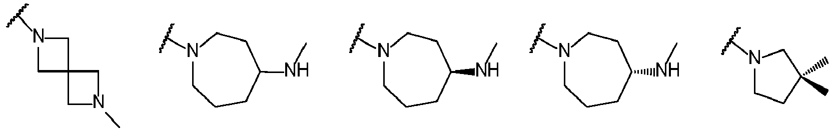 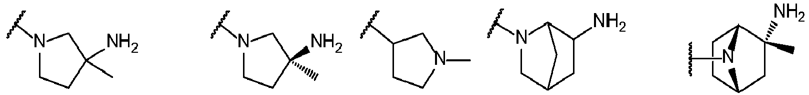 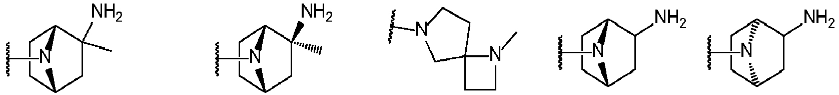 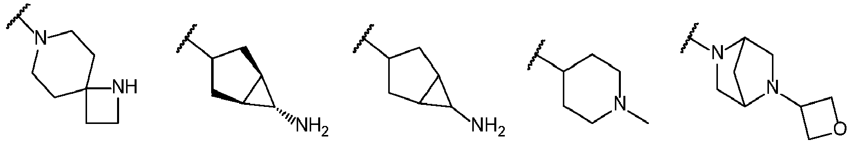 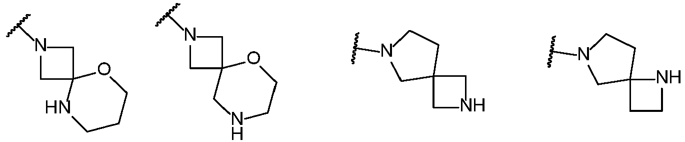 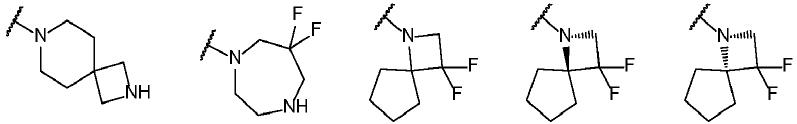 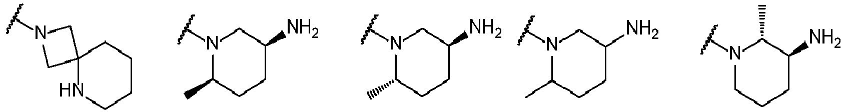 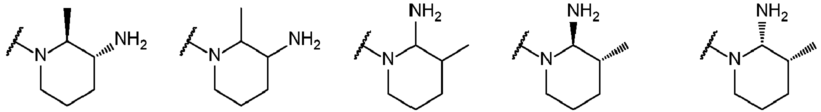 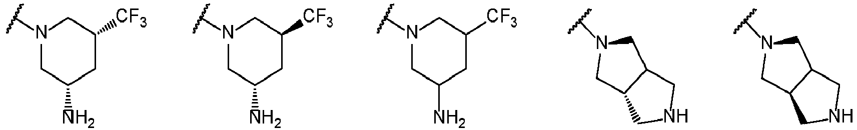 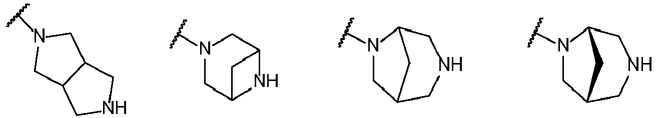 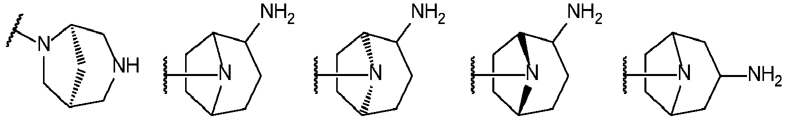 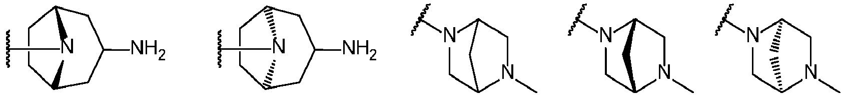 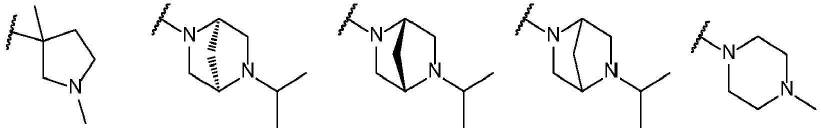 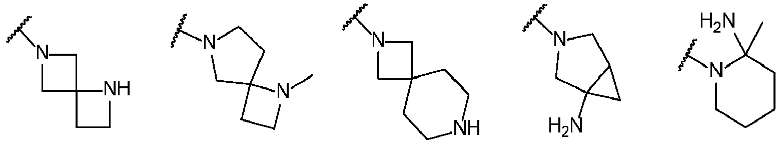 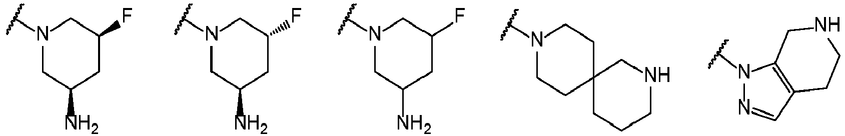 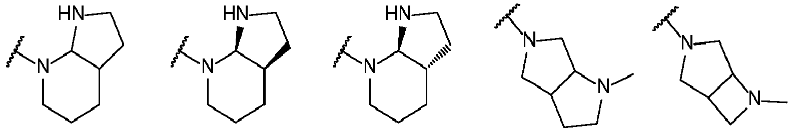 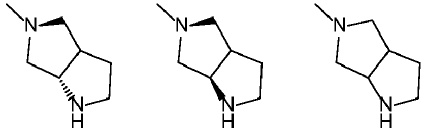
A19. The compound of formula (I) or a pharmaceutically acceptable salt, solvate, prodrug, or pharmaceutically active metabolite thereof according to any of Clauses A1 to A18, wherein Q is selected from the group consisting of the following structures: 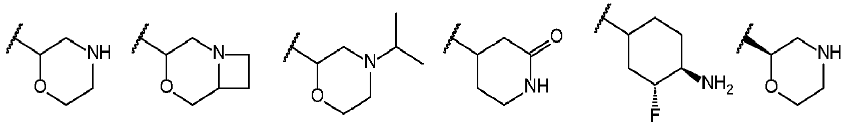 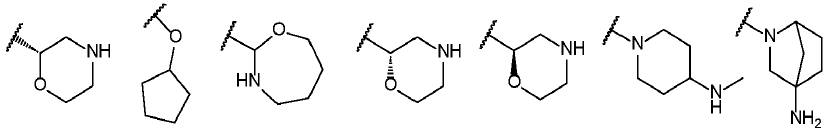 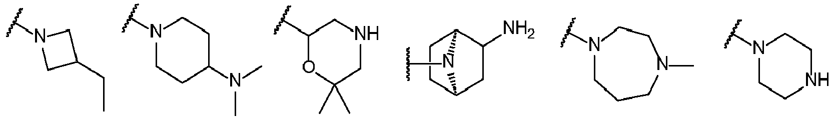 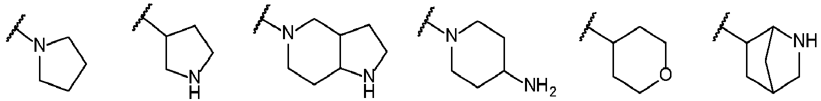 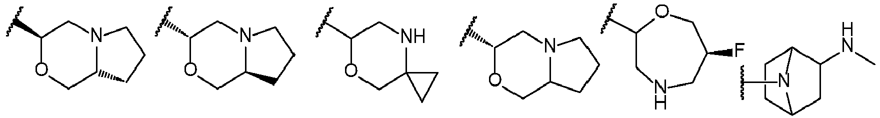 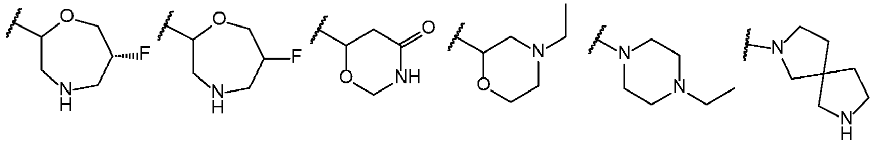 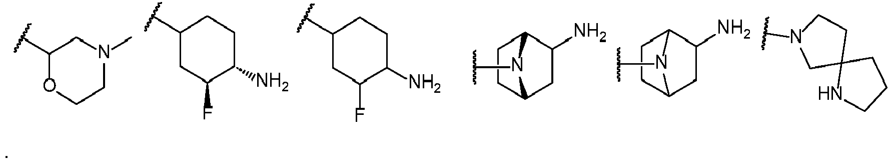
A20. A compound selected from any one of Examples 1 to 351.
A21. A compound selected from any one of:
   (i) Examples 4, 8, 15, 20, 26, 28, 71, 108, 109, 173, 177, 182, 183, 185, 190, 192, 194, 197, 207, 216, 234, 263, 302, 303, 315 and 335; or
   (ii) Examples 1, 2, 3, 5, 6, 7, 9, 10, 11, 12, 13, 14, 16, 17, 18, 19, 22, 23, 24, 25, 29, 30, 34, 36, 37, 38, 39, 48, 53, 57, 74, 79, 81, 103, 110, 112, 115, 116, 154, 169, 170, 171, 172, 174, 175, 178, 179, 181, 186, 187, 188, 189, 193, 195, 198, 199, 201, 204, 206, 209, 210, 217, 218, 219, 222, 223, 224, 230, 231, 232, 233, 235, 237, 242, 250, 254, 255, 257, 259, 260, 262, 264, 268, 275, 279, 304, 309, 310, 311, 312, 313, 314, 316, 317, 321, 323, 324, 325, 333, 334 and 336; or
   (iii) Examples 27, 31, 32, 33, 35, 43, 46, 47, 49, 51, 56, 60, 64, 67, 68, 70, 73, 82, 83, 91, 93, 94, 95, 100, 102, 104, 111, 128, 138, 153, 156, 176, 180, 184, 191, 196, 200, 202, 203, 205, 208, 211, 212, 213, 215, 220, 221, 227, 229, 236, 239, 240, 243, 244, 245, 247, 249, 251, 252, 253, 256, 258, 265, 273, 277, 299, 301, 305, 308, 318, 319, 322 and 339; or
   (iv) Examples 21, 40, 41, 42, 44, 45, 50, 52, 54, 55, 58, 59, 61, 62, 63, 66, 69, 72, 75, 76, 77, 78, 80, 85, 86, 88, 92, 96, 98, 99, 101, 105, 106, 107, 113, 114, 118, 119, 120, 121, 122, 124, 125, 126, 127, 129, 133, 137, 139, 141, 143, 147, 148, 149, 151, 152, 155, 163, 164, 165, 166, 168, 225, 226, 228, 238, 241, 246, 248, 261, 266, 267, 269, 270, 271, 272, 274, 276, 278, 280, 282, 283, 284, 285, 288, 289, 291, 293, 294, 297, 298, 300, 306, 307, 320, 328, 332, 340, 341, 342, 346, 347, 348 and 349; or
   (v) Examples 65, 84, 87, 89, 90, 97, 117, 123, 130, 131, 132, 134, 135, 136, 140, 142, 144, 145, 146, 150, 175, 157, 158, 159, 160, 161, 162, 167, 214, 281, 286, 287, 290, 292, 295, 296, 326, 327, 329, 330, 331, 337, 338, 343, 344 and 345.
A22. A pharmaceutical composition comprising a compound according to any of Clauses A1 to A21 or a pharmaceutically acceptable salt, solvate, prodrug, or pharmaceutically active metabolite thereof, or combinations thereof, and one or more pharmaceutically acceptable carrier.
A23. The compound according to any of Clauses A1 to A21, or the pharmaceutical composition according to Clause A22 for use in the treatment of diseases, conditions or disorders associated with LSD1.
A24. The compound or the pharmaceutical composition for use according to Clause A23, wherein the use is in the treatment of cancers, oncologic diseases, autoimmune disorders and/or inflammatory diseases; particularly wherein the use is in the treatment of diseases, conditions or disorders selected from the group consisting of: glioblastoma and acute myeloid leukemia (AML).
A25. The compound or pharmaceutical composition for use according to Clause A23 or Clause A24, wherein the use is in a method comprising administering the compound orally, topically, by inhalation, by intranasal administration, or systemically by intravenous, intraperitoneal, subcutaneous, or intramuscular injection; optionally wherein the use is in a method comprising administering the compound according to formula (I) in combination with one or more additional therapeutic agent.

## Claims

1. A compound of formula (I), or a pharmaceutically acceptable salt, solvate, prodrug, or pharmaceutically active metabolite thereof: 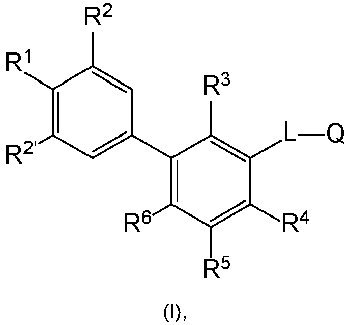 wherein
R¹ is nitro or cyano;
R² and R^{2'} are each independently selected from C₁₋₃ alkyl, hydrogen or halogen
R³ is selected from hydrogen, C₁₋₃ alkyl, C₁₋₃ haloalkyl, C₃-C₆ cycloalkyl, C₃-C₆ cycloalkenyl, C₃-C₆ heterocycloalkyl, C₄-C₅ heterocycloalkenyl, C₃-C₅ heterocycloalkyl C₁-C₃ alkyl, C₅-C₆ aryl, C₃-C₆ heteroaryl, C₁₋C₄ alkoxyl, C₃-C₆ cycloalkyl-C₁-C₃ alkoxyl, C₁₋C₄ haloalkoxyl, C₁-C₃ haloalkyl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, cyano, wherein each alkyl, cycloalkyl, cycloalkenyl, heterocycloalkyl, heterocycloalkenyl, heterocycloalkyl alkyl, aryl, heteroaryl, alkoxyl, cycloalkyl alkoxyl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, amino;
R⁴ is selected from hydrogen or halogen;
R⁵ is selected from the group consisting of hydrogen, C₁₋₃ alkyl, C₃-C₆ cycloalkyl, C₃-C₆ cycloalkenyl, C₃-C₆ aryl, C₃-C₆ heteroaryl, halogen, amino, alkylamino, dialkylamino, C₂-C₆ cyclic amine, C₂-C₅ heterocyclic amine, C₃-C₆ unsaturated or aromatic cyclic amine, C₃-C₅ unsaturated or aromatic heterocyclic amine, wherein each alkyl, cycloalkyl, cycloalkenyl, aryl, heteroaryl, alkylamino, dialkylamino, cyclic amine, heterocyclic amine, unsaturated or aromatic cyclic amine, and unsaturated or aromatic heterocyclic amine is optionally substituted with 1 to 3 substituents selected from the group consisting of C₁-C₃ alkyl, C₁-C₃ alkoxyl, C₃-C₆ cycloalkyl, hydroxyl, halogen, or amino;
R⁶ is selected from the group consisting of hydrogen, C₁₋C₄ alkoxyl, C₁₋C₄ alkoxyl, amino, alkylamino, aminoalkyl, dialkylamino, C₈-C₁₁ spirocycloalkyl, C₅-C₁₀ heterospirocycloalkyl, C₅-C₁₁ monocyclic or bicyclic aryl, monocyclic or bicyclic heteroaryl, C₂-C₆ cyclic amine, C₂-C₅ heterocyclic amine, C₃-C₆ unsaturated or aromatic cyclic amine, C₃-C₅ unsaturated or aromatic heterocyclic amine, C₇₋C₁₀ spirocyclic amine, or C₄-C₉ heterospirocyclic amine, wherein each is optionally substituted with 1 to 3 substituents selected from the group consisting of hydroxyl, amino, C₁-C₆ alkyl, C₁-C₆ alkoxyl, halogen, C₁-C₃ haloalkyl, optionally substituted C₃-C₆ cycloalkyl, optionally substituted C₂-C₅ heterocycloalkyl, or optionally substituted C₃-C₆ heteroaryl, wherein said optionally substituted cycloalkyl, heterocycloalkyl and heteroaryl may be substituted with 1 or 2 substituents selected from the group consisting of C₁-C₃ alkyl, C₁-C₃ alkoxyl, hydroxyl, halogen or amine;
L is a linker selected from the group consisting of a bond, -CH₂-, -CH₂-CH₂-, -CH(CH₃)-, -C(CH₃)₂-, -C≡C-, -CH₂-O-, -C(O)-, -O-, -O-CH₂-, -NH-CH₂-, -N(CH₃)-CH₂-, --NH-C(O)-, -N(CH₃)-C(O)-, -CH₂-NH-CH₂-
Q is a monocyclic or bicyclic ring system selected from the group consisting of C₅-C₉ cycloalkyl, C₄-C₈ heterocycloalkyl, C₈-C₁₁ spirocycloalkyl, C₅-C₁₀ heterospirocycloalkyl, C₃-C₅ cyclic amine, C₃-C₅ heterocyclic amine, C₇₋C₁₀ spirocyclic amine, or C₄-C₉ heterospirocyclic amine, wherein each cycloalkyl or heterocycloalkyl group may be saturated or unsaturated, said bicyclic ring may be a fused or bridged bicycle, and wherein Q is optionally substituted with 1 to 3 R⁷, wherein each R⁷ is independently selected from hydrogen, C₁-C₆ alkyl, C₁-C₆ haloalkyl, C₁-C₆ alkylcarbonyl, hydroxyl, oxo, halogen, C₁-C₆ alkylamine or amino.

2. The compound of formula (I) or a pharmaceutically acceptable salt, solvate, prodrug, or pharmaceutically active metabolite thereof according to Claim 1, wherein each heterocyclic or heteroaryl substituent may include from 1 to 4 heteroatoms independently selected from O, S and N .

3. The compound of formula (I) or a pharmaceutically acceptable salt, solvate, prodrug, or pharmaceutically active metabolite thereof according to Claim 1 or Claim 2, wherein:
(i) one of R² and R^{2'} is hydrogen and the other is F;
(ii) one of R² and R^{2'} is hydrogen and the other is CI; or
(iii) both R² and R^{2'} are hydrogen.

4. The compound of formula (I) or a pharmaceutically acceptable salt, solvate, prodrug, or pharmaceutically active metabolite thereof according to any of Claims 1 to 3, wherein:
(i) R³ is hydrogen or CI;
(ii) R³ is an optionally substituted 5 or 6 membered heteroaryl;
(iii) R³ is an optionally substituted 4 to 6 membered heterocycloalkyl; or
(iv) R³ is an optionally substituted 3 to 5 membered cycloalkyl;
wherein each hetero moiety may include 1 or 2 heteroatoms independently selected from O, S and N; and
wherein said optional substituents are independently selected from 1 or 2 substituents of the group consisting of methyl, F and Cl.

5. The compound of formula (I) or a pharmaceutically acceptable salt, solvate, prodrug, or pharmaceutically active metabolite thereof according to any of Claims 1 to 4, wherein:
(i) R⁴ is hydrogen;
(ii) R⁴ is F and R⁵ and R⁶ are each hydrogen;
(iii) R⁴ is F and R⁵ and R⁶ are each hydrogen; or
(iii) R⁴, R⁵ and R⁶ are all hydrogen.

6. The compound of formula (I) or a pharmaceutically acceptable salt, solvate, prodrug, or pharmaceutically active metabolite thereof according to any of Claims 1 to 5, wherein:
(i) R⁵ is hydrogen;
(ii) R⁵ is F;
(iii) R⁵ is a 4 or 5 membered heterocycloalkyl;
(iv) R⁵ is C₁-C₃ alkyl;
(v) R⁵ is 3, 4 or 5 membered cycloalkyl
(vi) R⁵ is optionally substituted C₃-C₆ cycloalkyl, C₃-C₆ cycloalkenyl, C₃-C₆ heterocycloalkyl, C₃-C₆ aryl or C₃-C₆ heteroaryl.

7. The compound of formula (I) or a pharmaceutically acceptable salt, solvate, prodrug, or pharmaceutically active metabolite thereof according to any of Claims 1 to 6, wherein:
(i) R⁶ is hydrogen;
(ii) R⁶ is F or Cl;
(iii) R⁶ is an optionally substituted 4 or 5 membered heterocycloalkyl;
(iv) R⁶ is an optionally substituted 8, 9 or 10 membered bicyclic or spirocyclic heterocycloalkyl;
(v) R⁶ is an optionally substituted 5 or 6 membered heteroaryl;
(vi) R⁶ is an optionally substituted 4, 5 or 6 membered cycloalkyl or spirocycloalkyl;
(vii) R⁶ is a C₁-C₃ haloalkoxy; or
(viii) R⁶ is a C₂-C₈ alkylamine or dialkylamine
wherein each hetero moiety may include 1 or 2 heteroatoms independently selected from O, S and N; and
wherein said optional substituents are independently selected from 1 or 2 substituents from the group consisting of C₁-C₃ alkyl, C₁-C₃ haloalkyl, C₁-C₃ alkoxyl, F, Cl and hydroxyl.

8. The compound of formula (I) or a pharmaceutically acceptable salt, solvate, prodrug, or pharmaceutically active metabolite thereof according to any of Claims 1 to 7, wherein L is selected from -CH₂- or -CH₂-CH₂-.

9. The compound of formula (I) or a pharmaceutically acceptable salt, solvate, prodrug, or pharmaceutically active metabolite thereof according to any of Claims 1 to 8, wherein Q is selected from the group consisting of optionally substituted 5 to 7 membered heterocycloalkyl and optionally substituted 4 to 7 membered mono or fused or bridged bicycloalkylamine;
wherein each heterocyclic moiety may include 1 or 2 heteroatoms independently selected from O and N; and
wherein said optional substituents are independently selected from 1 to 3 substituents from the group consisting of C₁-C₃ alkyl, C₁-C₃ haloalkyl, C₁-C₃ alkoxyl, C₁-C₃ alkylamine, C₁-C₃ dialkylamine, C₁-C₃ carbonyl, amine, amino-C₁-C₃ alkyl, oxo, F, Cl and hydroxyl.

10. The compound of formula (I) or a pharmaceutically acceptable salt, solvate, prodrug, or pharmaceutically active metabolite thereof according to any of Claims 1 to 9, wherein:
(i) R⁷ is independently selected from C₁-C₃ alkyl, C₁-C₃ haloalkyl, hydroxyl, oxo, F, C₁-C₃ alkylamino, C₁-C₃ dialkylamino or amino;
(ii) R⁷ is independently selected from methyl, ethyl, amine and F;
wherein there are 1, 2 or 3 R⁷ groups.

11. The compound of formula (I) or a pharmaceutically acceptable salt, solvate, prodrug, or pharmaceutically active metabolite thereof according to any of Claims 1 to 10, wherein R³ is selected from the group consisting of the following structures:

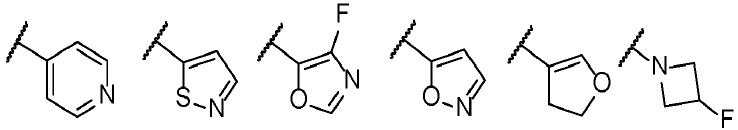

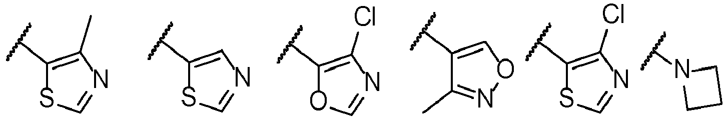

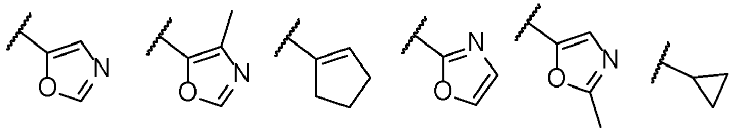

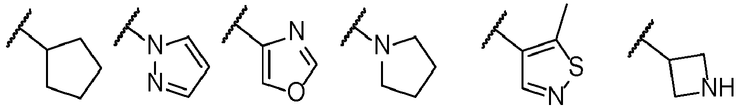

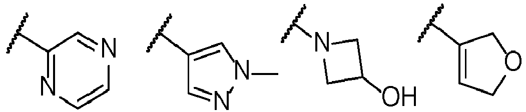

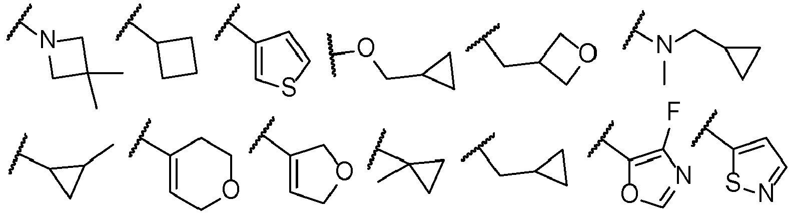

12. The compound of formula (I) or a pharmaceutically acceptable salt, solvate, prodrug, or pharmaceutically active metabolite thereof according to any of Claims 1 to 11, wherein R⁵ is selected from the group consisting of hydrogen and the following structures:

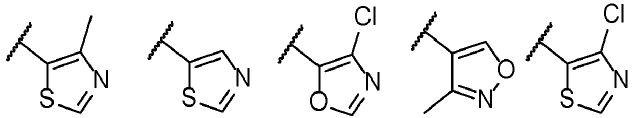

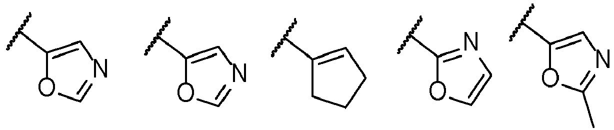

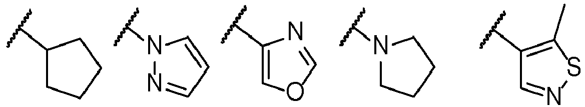

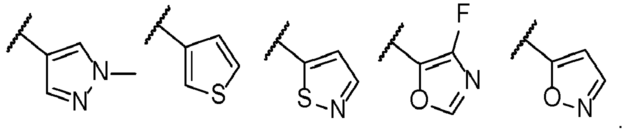

13. The compound of formula (I) or a pharmaceutically acceptable salt, solvate, prodrug, or pharmaceutically active metabolite thereof according to any of Claims 1 to 12, wherein R⁶ is selected from the group consisting of hydrogen and the following structures:

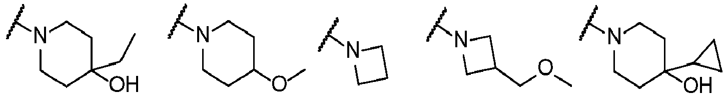

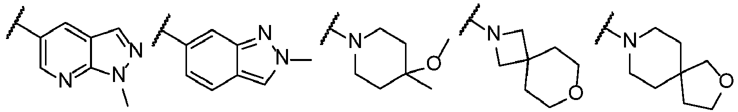

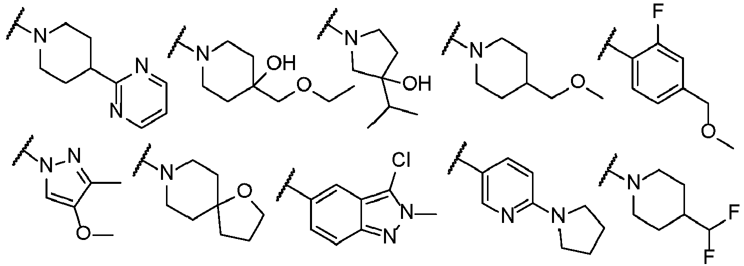

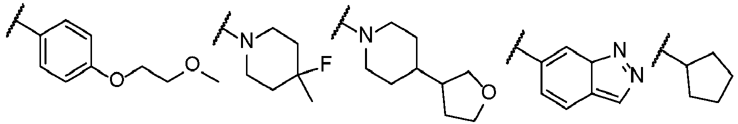

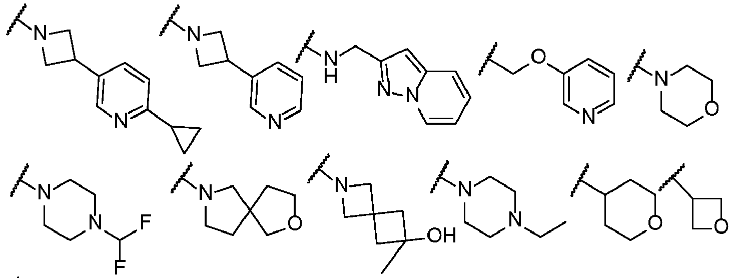

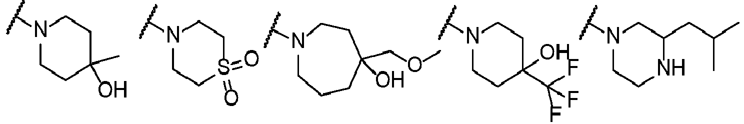

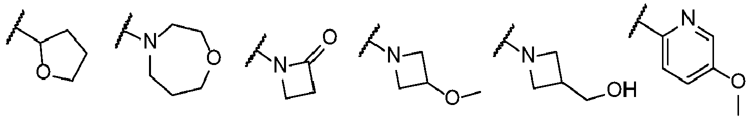

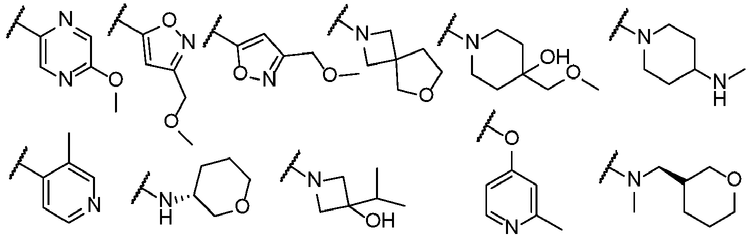

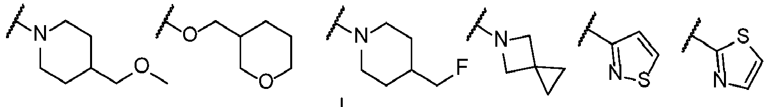

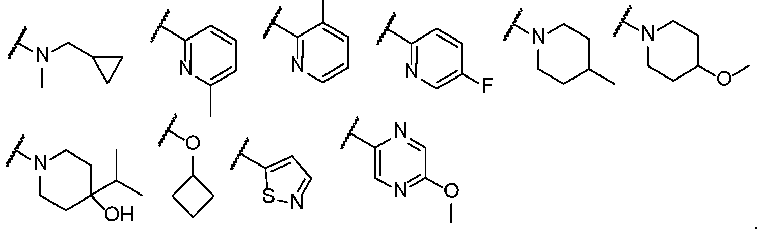

14. The compound of formula (I) or a pharmaceutically acceptable salt, solvate, prodrug, or pharmaceutically active metabolite thereof according to any of Claims 1 to 13, wherein Q is selected from the group consisting of the following structures:

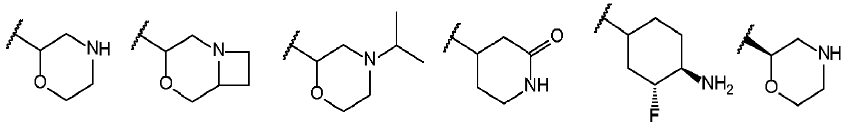

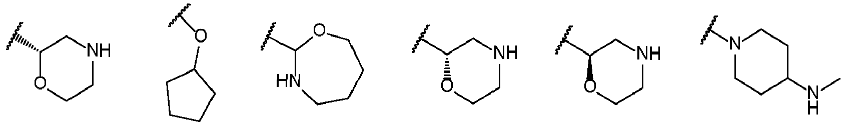

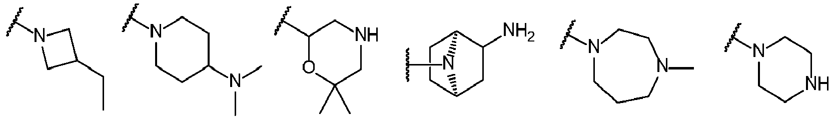

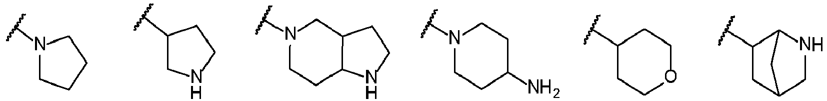

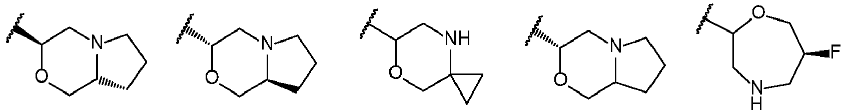

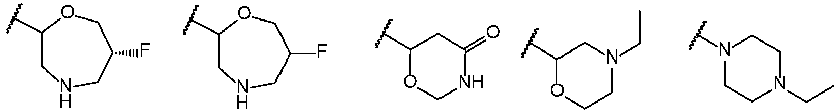

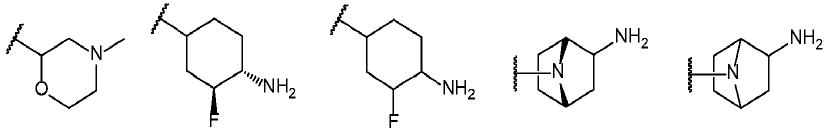

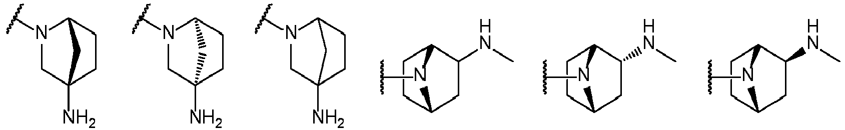

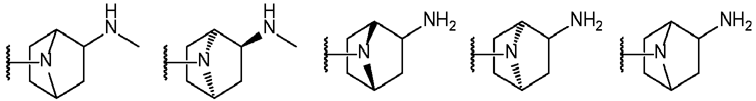

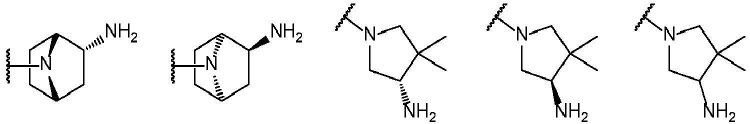

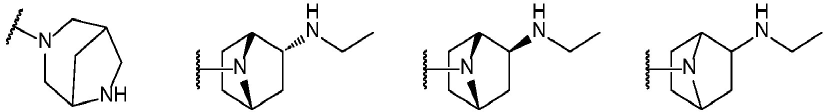

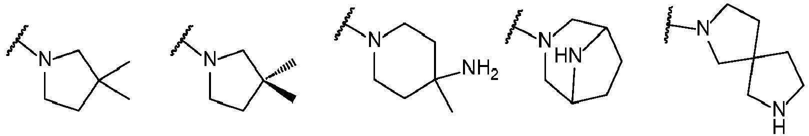

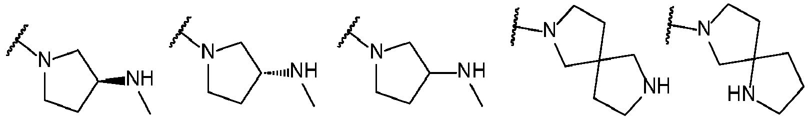

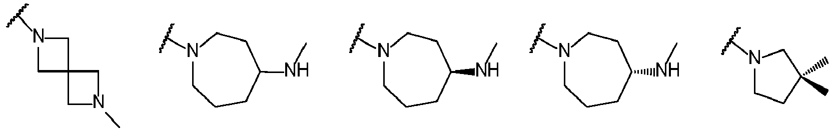

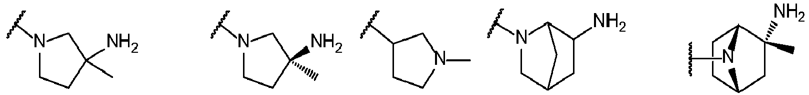

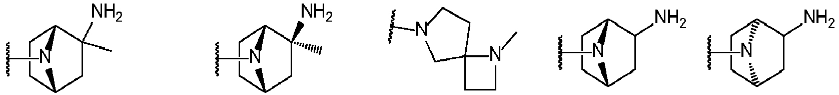

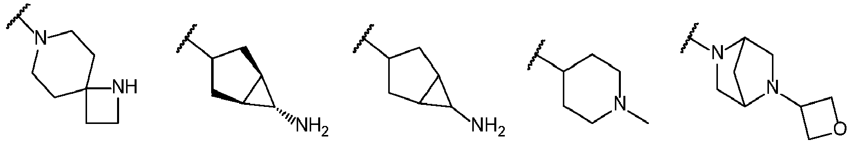

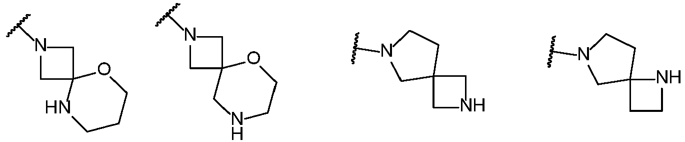

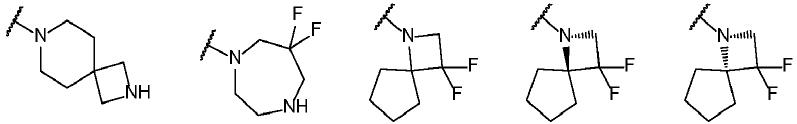

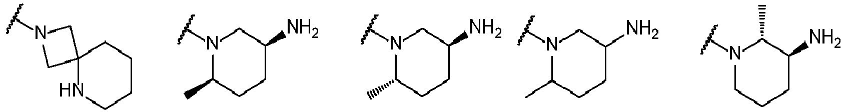

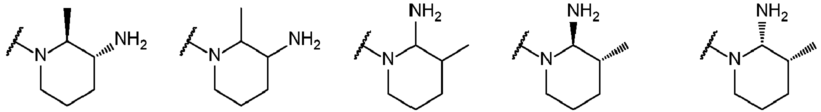

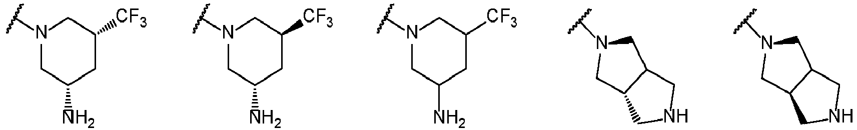

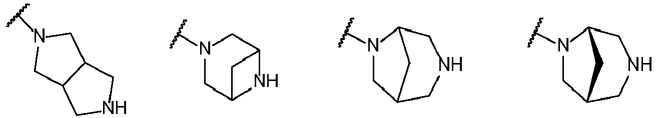

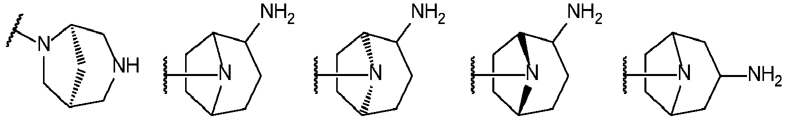

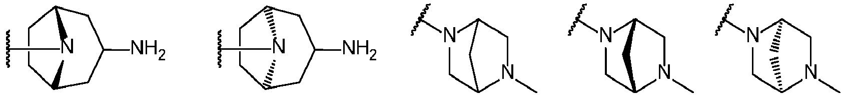

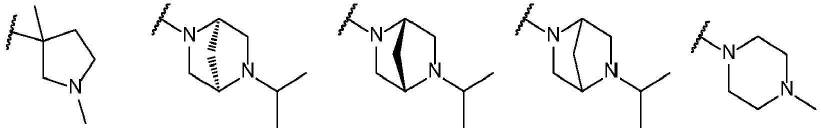

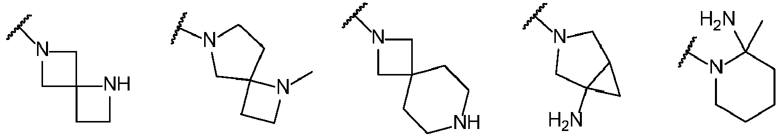

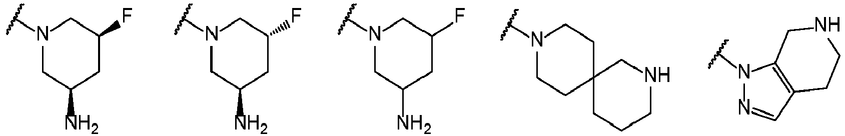

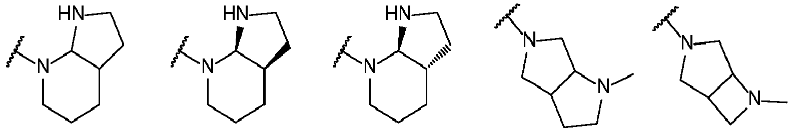

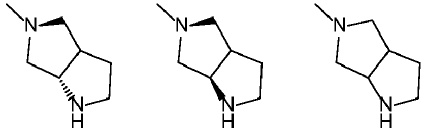

15. A compound selected from any one of Examples 1 to 351.
